# EUROPEAN PATENT SPECIFICATION

(11) **EP 4 190 779 B1**
(45) Date of publication and mention of the grant of the patent: **02.09.2026**
(21) Application number: 21850588.1
(22) Date of filing: 21.07.2021
(51) Int. Cl.: C09K 11/06, C07D 209/86, C07D 405/14, C07D 409/14, H10K 85/60, H10K 50/11, H10K 50/12, H10K 85/30

(54) **HETEROCYCLIC COMPOUND, ORGANIC LIGHT-EMITTING DEVICE COMPRISING SAME, AND COMPOSITION FOR ORGANIC LAYER OF ORGANIC LIGHT-EMITTING DEVICE**
HETEROCYCLISCHE VERBINDUNG, ORGANISCHE LICHTEMITTIERENDE VORRICHTUNG DAMIT UND ZUSAMMENSETZUNG FÜR ORGANISCHE SCHICHT EINER ORGANISCHEN LICHTEMITTIERENDEN VORRICHTUNG
COMPOSÉ HÉTÉROCYCLIQUE, DISPOSITIF ÉLECTROLUMINESCENT ORGANIQUE LE COMPRENANT, ET COMPOSITION POUR COUCHE ORGANIQUE DE DISPOSITIF ÉLECTROLUMINESCENT ORGANIQUE

(30) Priority: 27.07.2020 KR 20200093407
(43) Date of publication of application: 07.06.2023
(73) Proprietor: LT Materials Co., Ltd., Yongin-si, Gyeonggi-do 17118 (KR)
(72) Inventor: NO, Young-Seok, Yongin-si, Gyeonggi-do 17118 (KR); PARK, Geon-Yu, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/009383
(87) International publication number: WO 2022/025515

(56) References cited:
- WO-A1-2021/037401
- CN-A- 109 956 939
- KR-A- 20140 035 737
- KR-A- 20170 102 000
- KR-A- 20190 128 684
- KR-A- 20200 082 020
- US-A1- 2020 212 309

## Description

### [Technical Field]

The present specification relates to a heterocyclic compound, an organic light emitting device comprising the same, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US 4356429 A discloses an organic electroluminescent cell comprising a luminescent zone between two electrodes, wherein a hole-injecting zone comprising a porphyrinic compound is disposed between the luminescent zone and the anode electrode.

US 2020 212309 A1 discloses an organic light emitting diode comprising a first electrode; a second electrode facing the first electrode; and an emitting material layer. The emitting material layer includes a p-type host, a n-type host and a phosphorescent dopant and is positioned between the first electrode and the second electrode, wherein a first energy level of a HOMO of the p-type host is equal to or lower than a second energy level of a HOMO of the n-type host, and a difference between an energy level of a singlet state of the n-type host and an energy level of a triplet state of the n-type host is greater than 0.3 eV and smaller than 0.5 eV.

KR 2017 0102000 A discloses an aromatic heterocyclic derivative and an organic electroluminescent device using the same, which is reported to have a high luminous efficiency, a long emission life, and a small change over time even in use at a high temperature. Further, a display device and a lighting device provided with the organic electroluminescent device is disclosed. The aromatic heterocyclic derivative therein is an aromatic heterocyclic derivative having a specific structure including a carbazole ring.

KR 2020 0082020 A discloses an organic light emitting diode and an organic light emitting device including the same. A light emitting material layer includes a biscarbazole moiety and a dibenzofuran moiety or a dibenzothiophene moiety connected to the biscarbazole moiety. The light emitting material layer further includes a p-type host in which an aryl group is substituted at position 1 of the dibenzofuran moiety or the dibenzothiophene moiety and an n-type host as a triazine derivative. The luminous efficiency and lifetime of the organic light emitting diode and the organic light emitting device including the same are reported to be increased.

CN 109 956 939 A discloses an azacarbazole derivative being characterized by having a specific structural formula. In addition, an organic electroluminescent device containing the azacarbazole derivative is disclosed.

KR 2014 0035737 A discloses an organic electroluminescence compound and an organic electroluminescence diode containing the same. The organic electroluminescence compound, according to the disclosure, is excellent in light emitting properties and can be used to manufacture an organic light emitting diode (OLED) having enhanced energy efficiency, by reducing the driving voltage of the diode.

KR 2019 0128684 A discloses dibenzofuran and dibenzothiophene derivatives, in particular for use as triplet matrix materials in organic electroluminescent devices. The disclosure also relates to processes for the preparation of the compounds and to electronic devices comprising them.

WO 2021 037401 A1 discloses dibenzofuran derivatives which are substituted with electron-poor heteroaryl groups, and electronic devices, in particular organic electroluminescent devices containing said compounds as triplet matrix materials.

### [Disclosure]

### [Technical Problem]

The present disclosure is directed to providing a heterocyclic compound, and an organic light emitting device comprising the same.

### [Technical Solution]

One embodiment of the present application provides a heterocyclic compound represented by any one of the following Chemical Formulae 1-2 to 1-4: 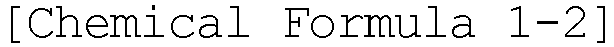 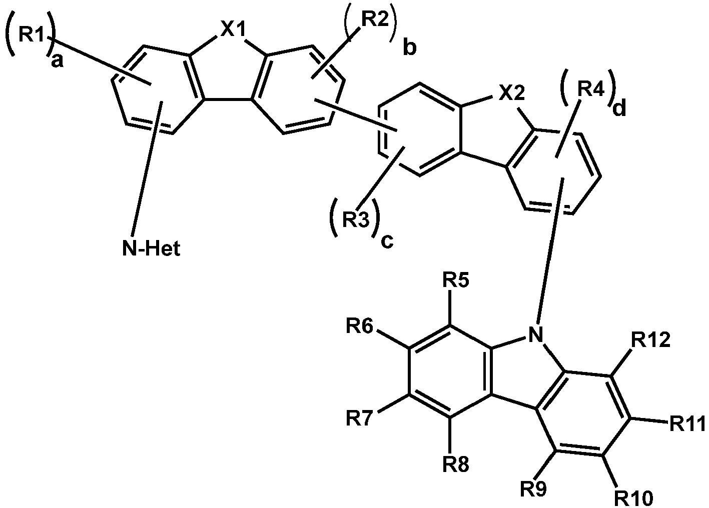 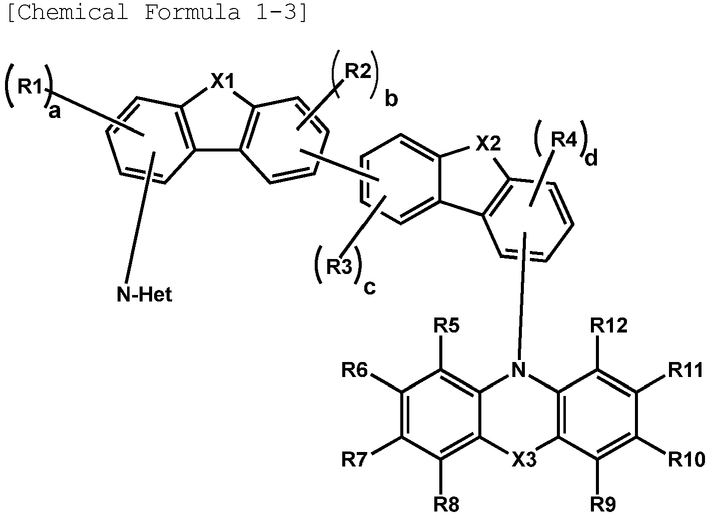 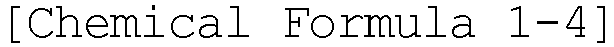 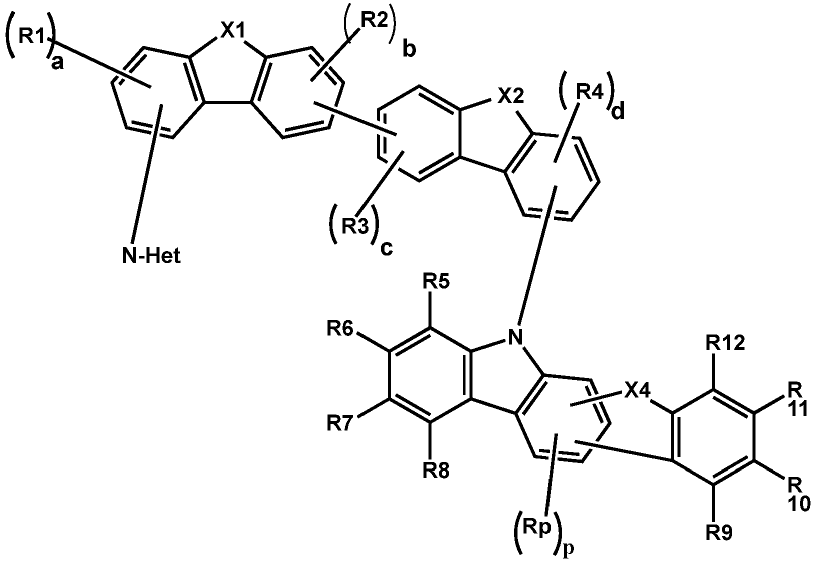 in Chemical Formulae 1-2 to 1-4,
X1 and X2 are the same as or different from each other, and each independently O; or S;
R1 to R4 are the same as or different from each other, and each independently hydrogen; deuterium; a substituted or unsubstituted aryl group having 6 to 60 carbon atoms; or a substituted or unsubstituted heteroaryl group having 6 to 60 carbon atoms, a to d are each an integer of 0 to 3, and when a to d are each 2 or greater, substituents in the parentheses are the same as each other;
N-Het is a group represented by any one of the following Chemical Formulae 2 to 4;
X3 and X4 are each independently O; S; CRaRb; or NRc, Ra and Rb are the same as or different from each other and each independently a substituted or unsubstituted alkyl group having 1 to 10 carbon atoms; or a substituted or unsubstituted aryl group having 6 to 20 carbon atoms, and Rc is a substituted or unsubstituted aryl group having 6 to 20 carbon atoms;
R5 to R12 and Rp are the same as or different from each other, and each independently hydrogen; deuterium; a halogen group; a cyano group; a substituted or unsubstituted aryl group having 6 to 20 carbon atoms; or a substituted or unsubstituted heteroaryl group having 2 to 20 carbon atoms;
p is an integer of 0 to 2, and when p is 2, substituents in the parentheses are the same as each other; 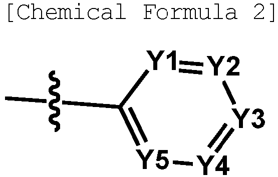 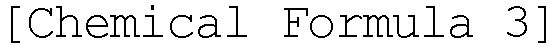 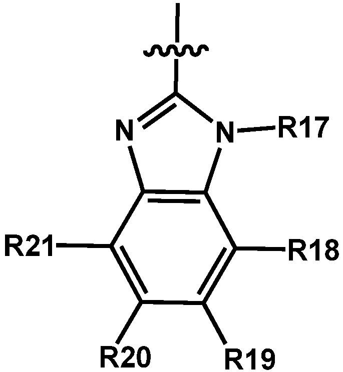 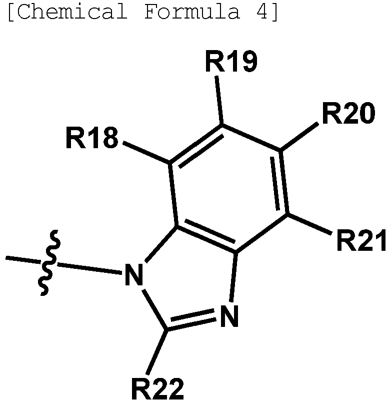 in Chemical Formulae 2 to 4,
   Y1 is CR12' or N, Y2 is CR13' or N, Y3 is CR14' or N, Y4 is CR15' or N, Y5 is CR16' or N, and at least one of Y1 to Y5 is N; and
   R12' to R16' and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring; 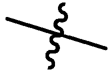
   is a site linked to any one of the following Chemical Formulae 1-2 to 1-4; and
   No. 3 position of 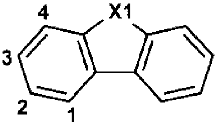 is substituted with N-Het and
   No. 3 position of 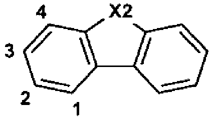 is substituted with any one of 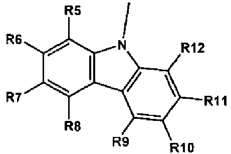 of Chemical Formula 1-2, 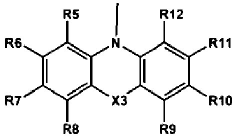 of Chemical Formula 1-3 and 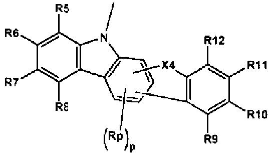 of Chemical Formula 1-4.

Another embodiment of the present application provides a heterocyclic compound represented by any one of the following compounds:

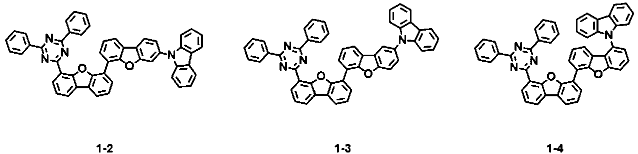

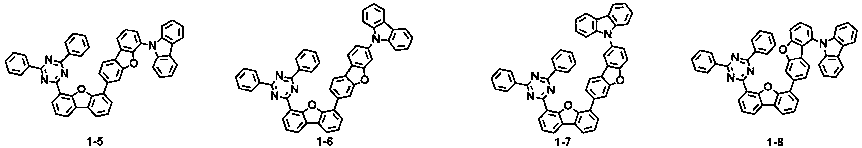

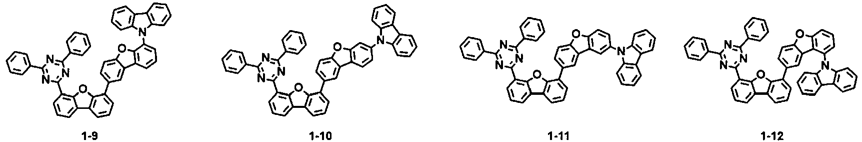

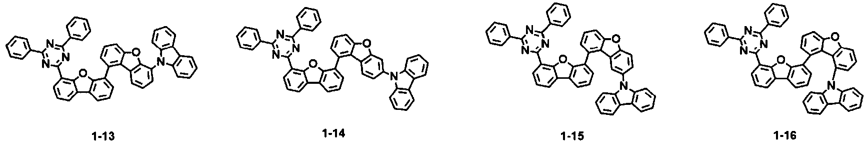

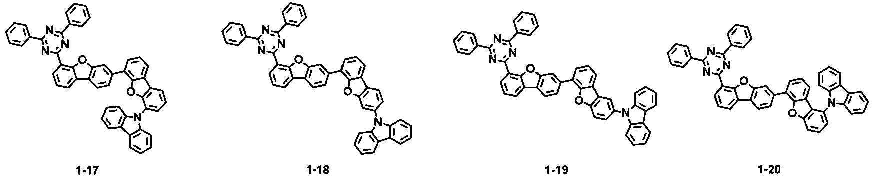

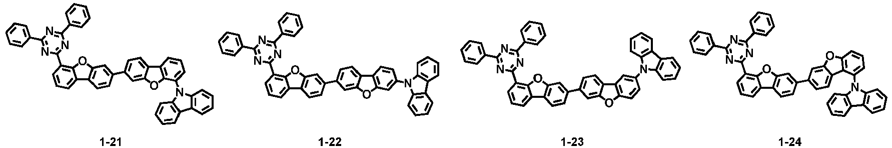

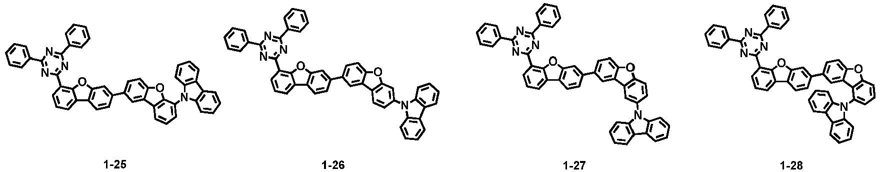

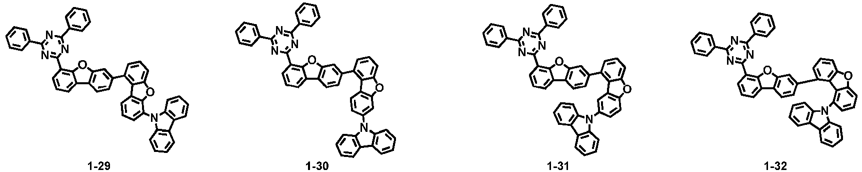

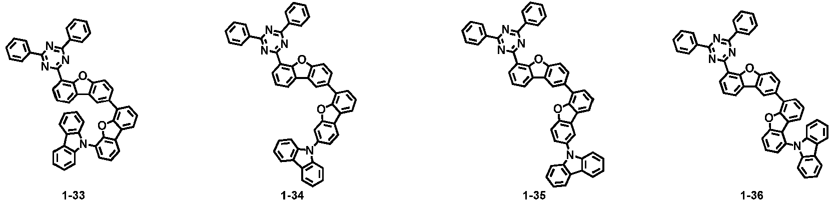

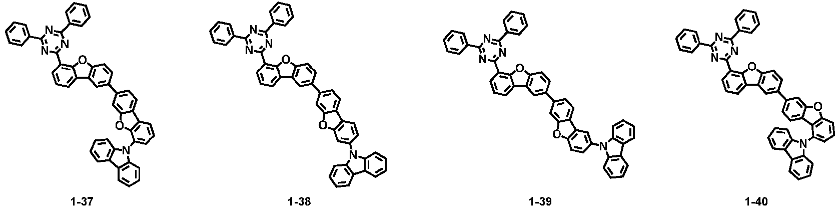

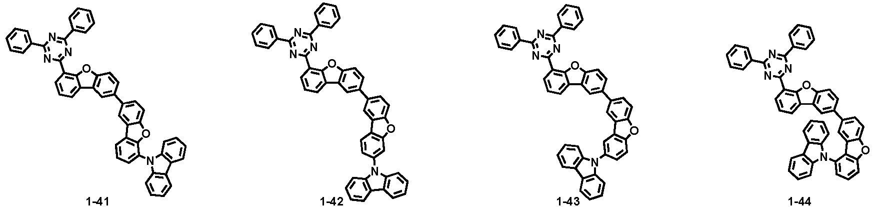

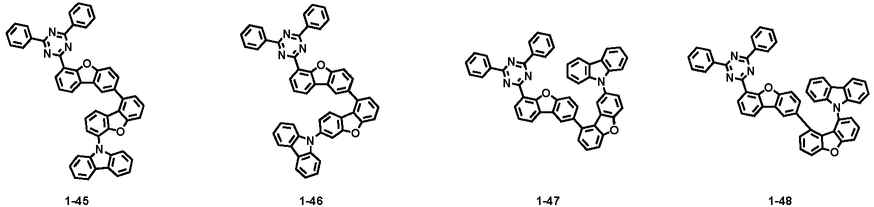

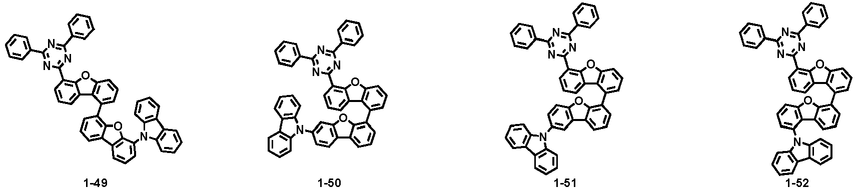

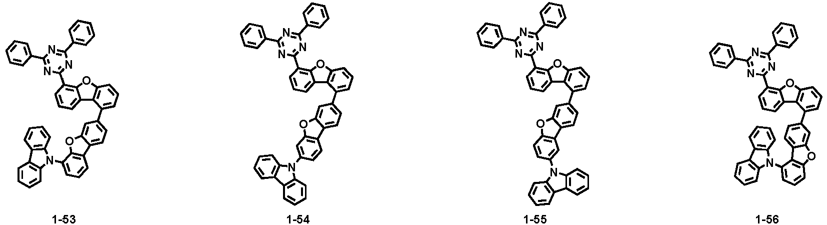

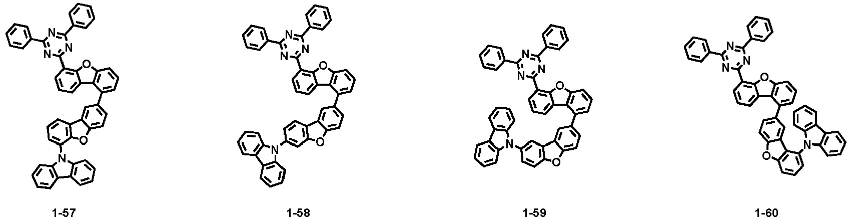

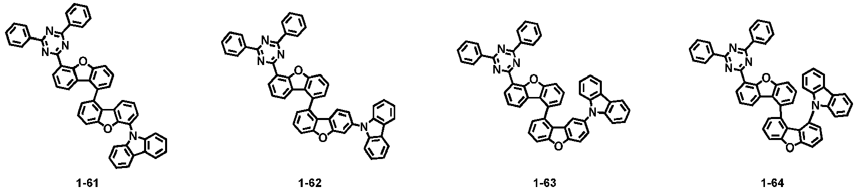

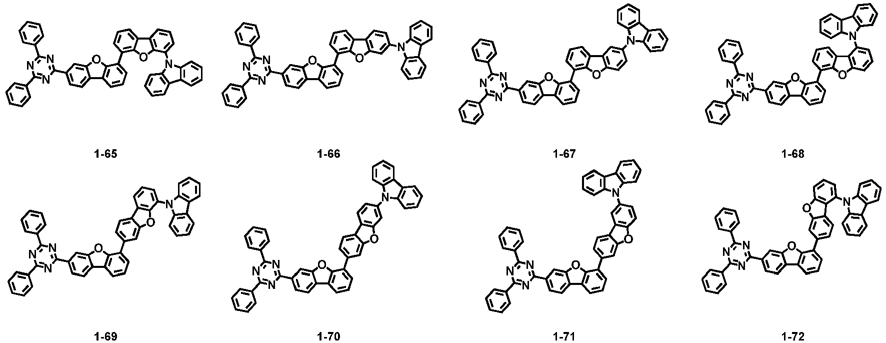

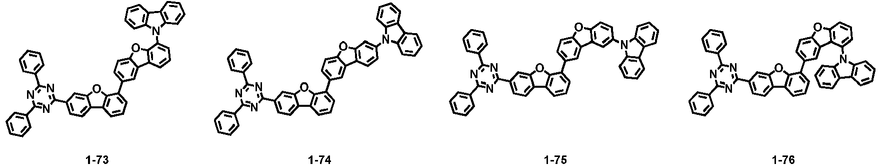

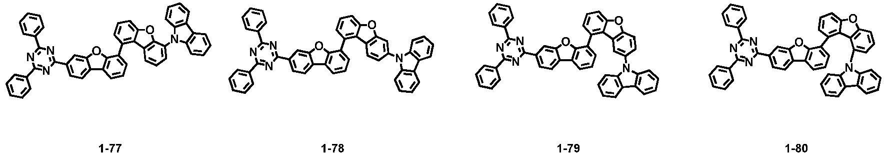

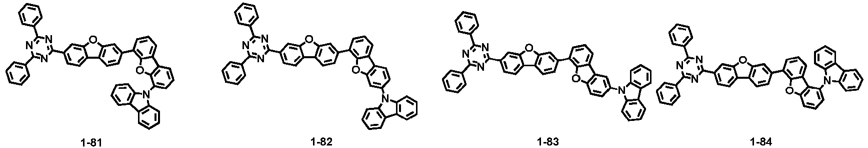

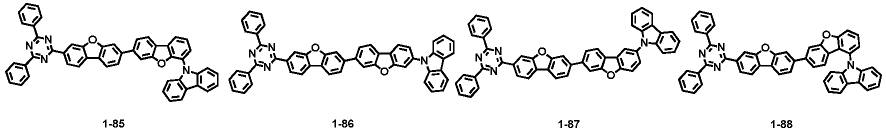

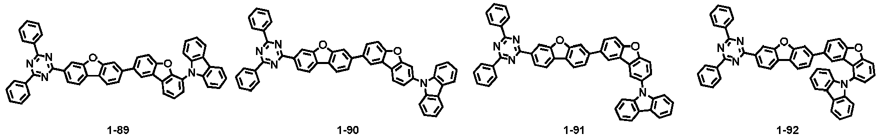

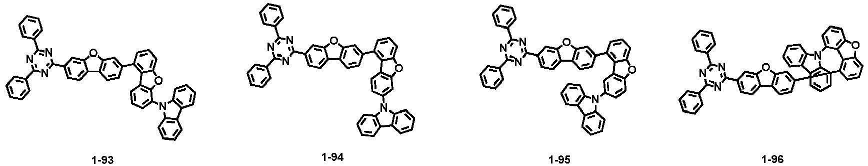

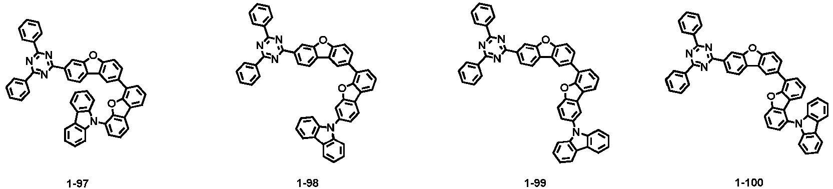

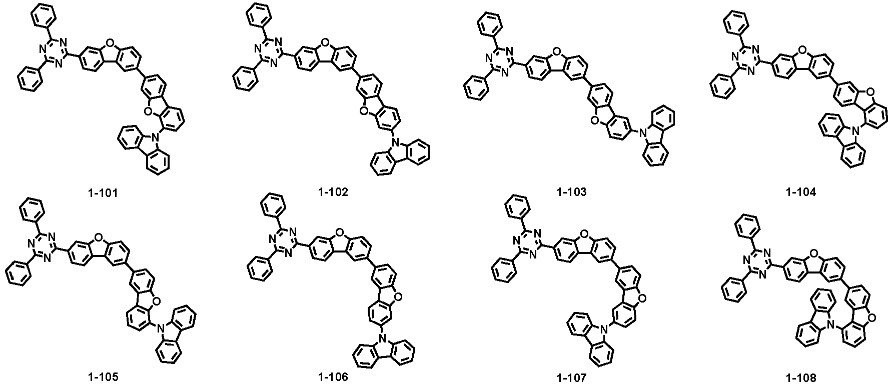

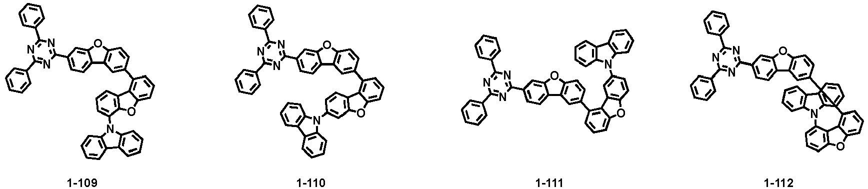

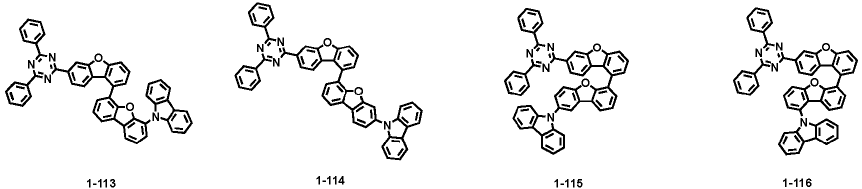

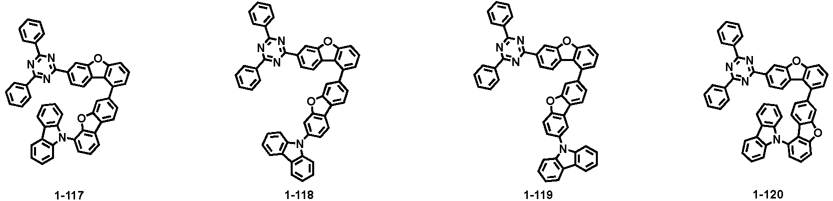

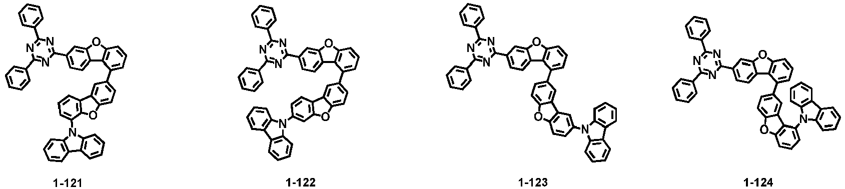

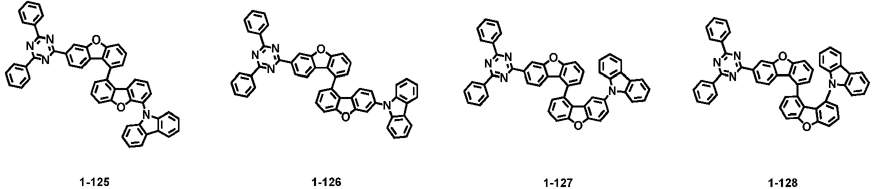

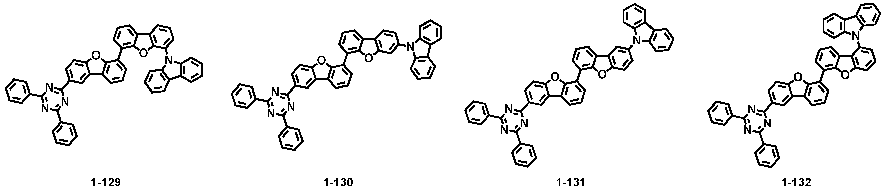

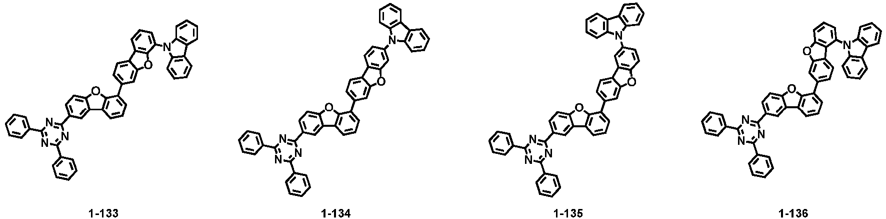

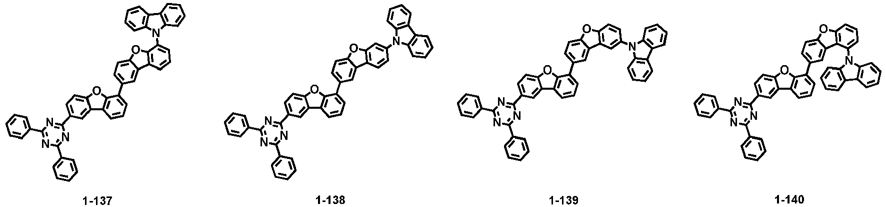

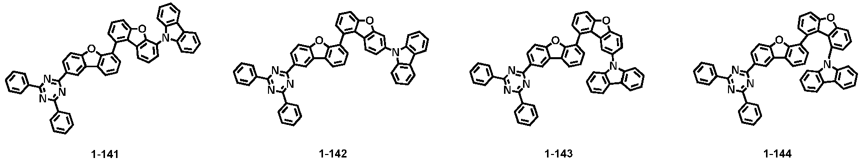

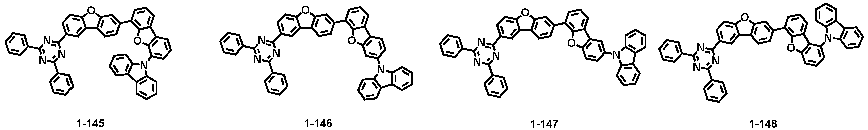

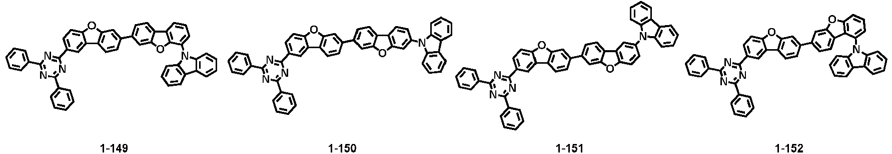

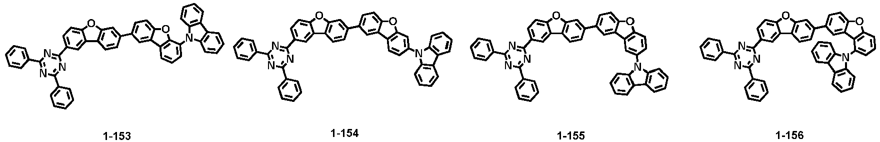

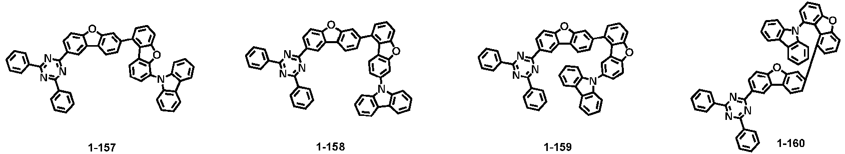

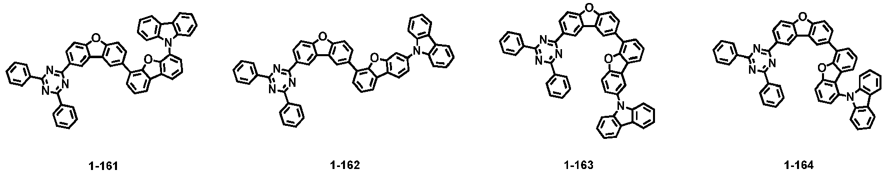

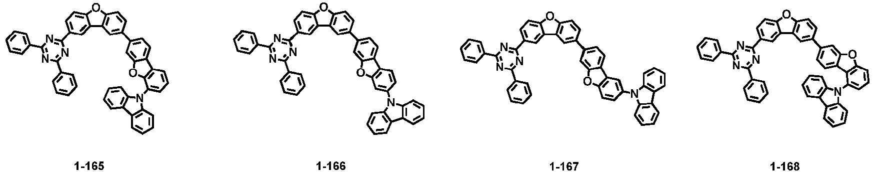

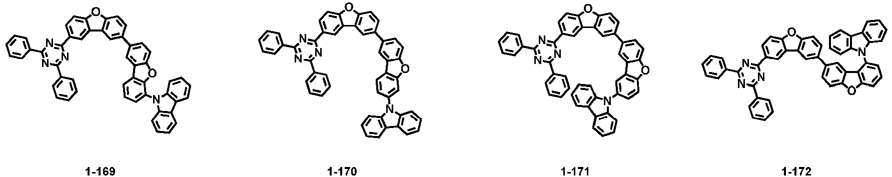

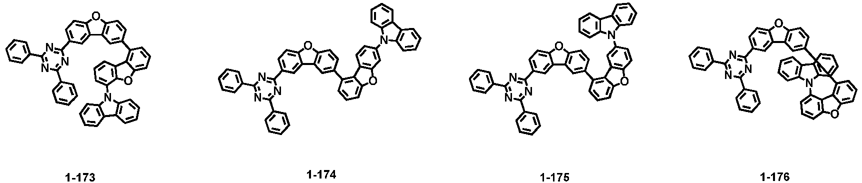

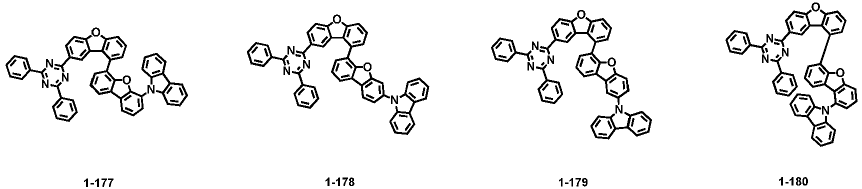

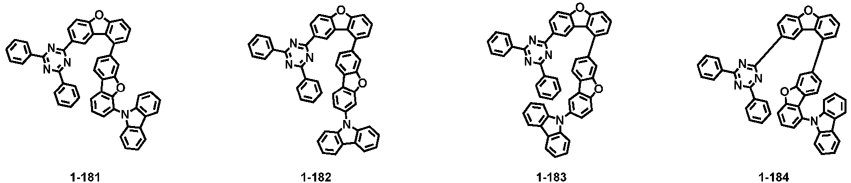

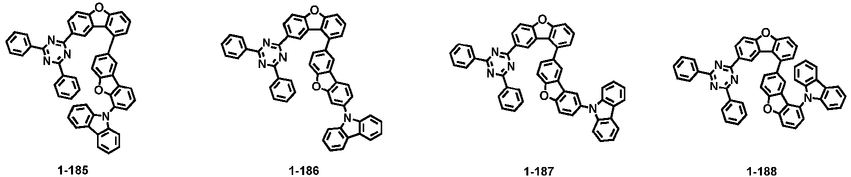

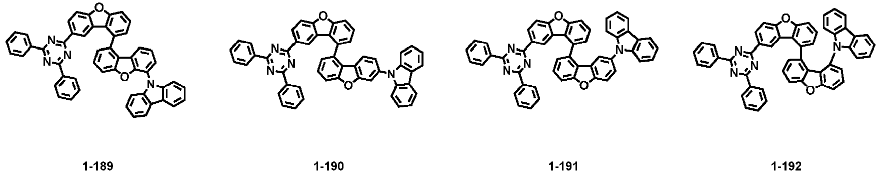

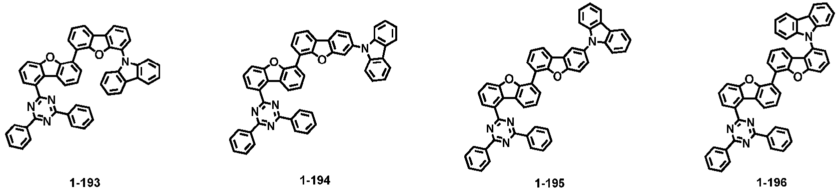

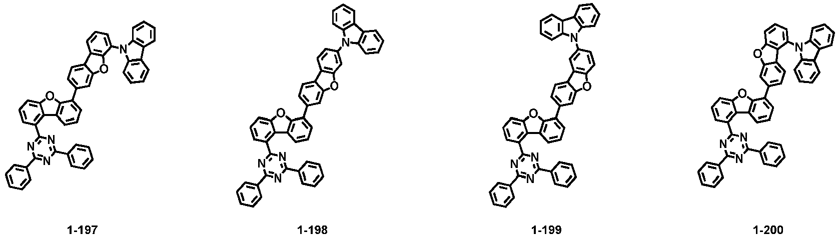

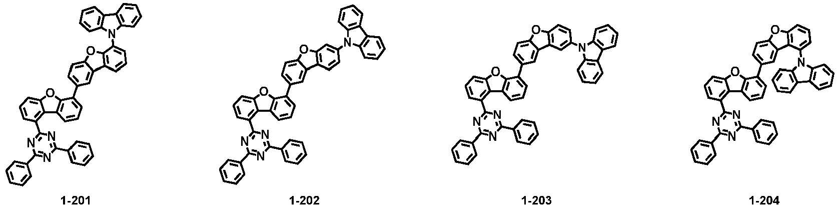

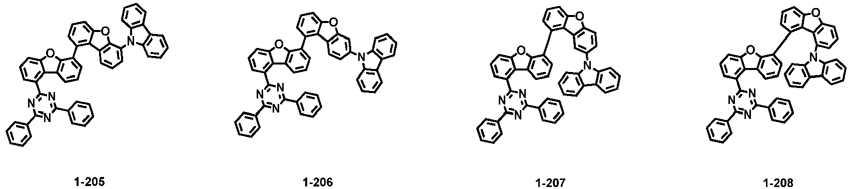

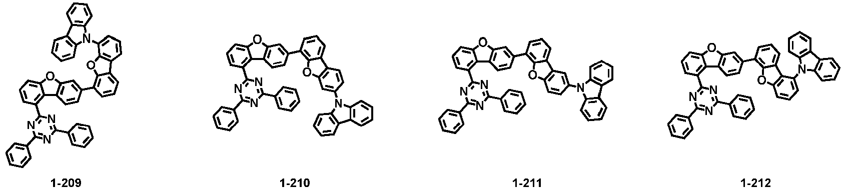

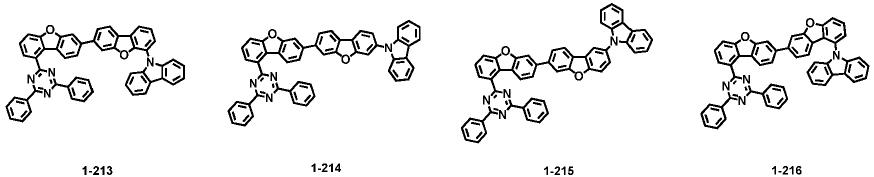

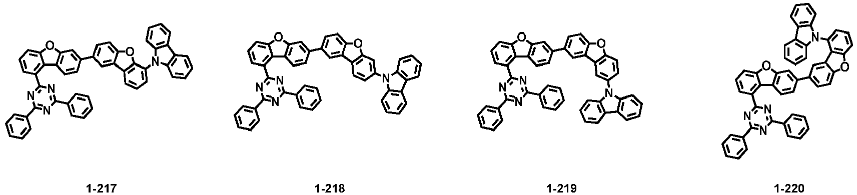

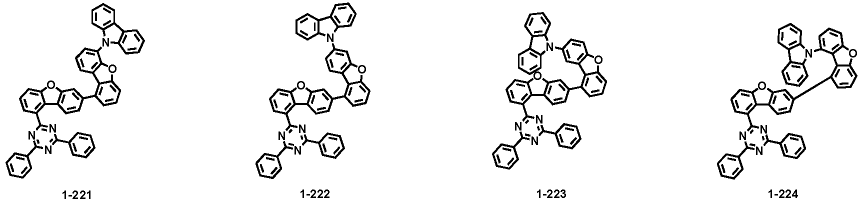

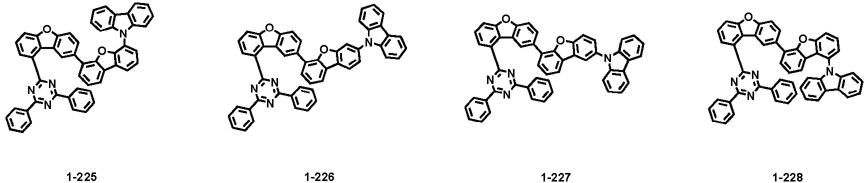

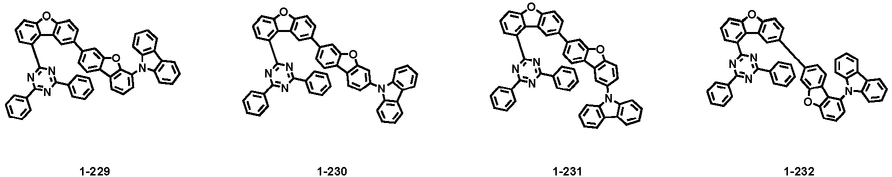

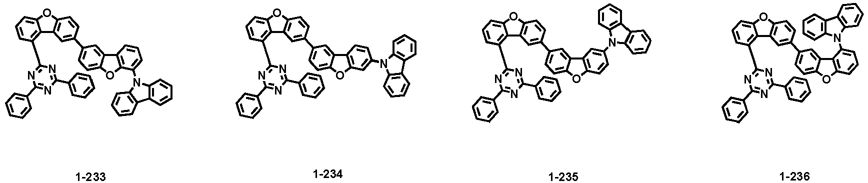

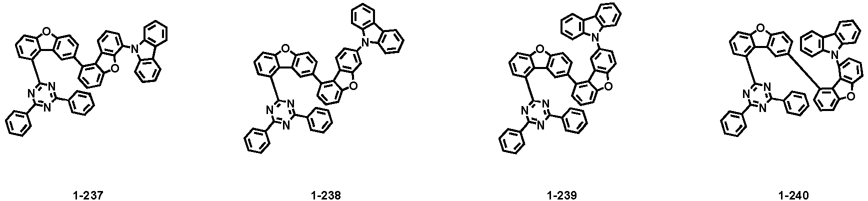

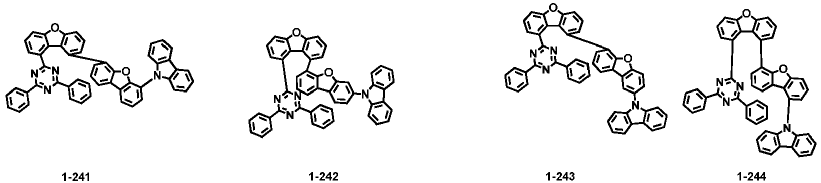

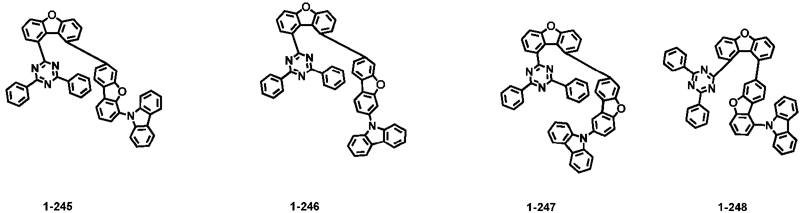

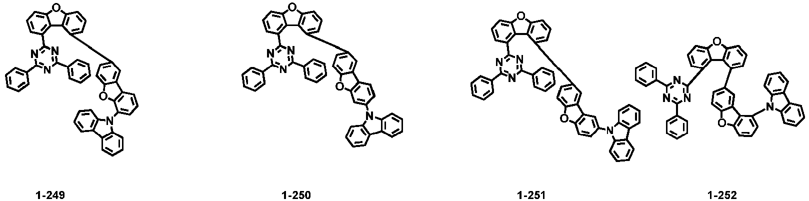

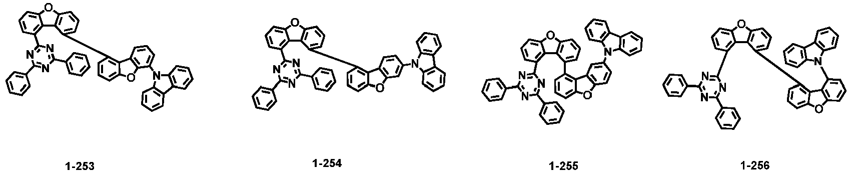

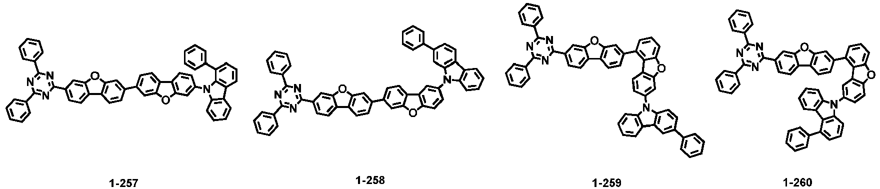

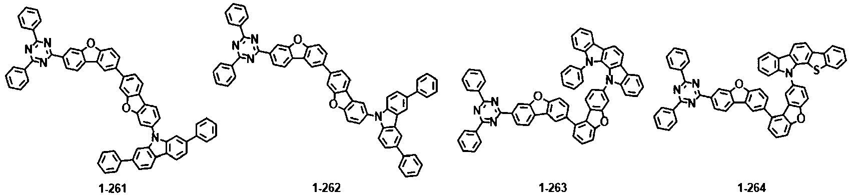

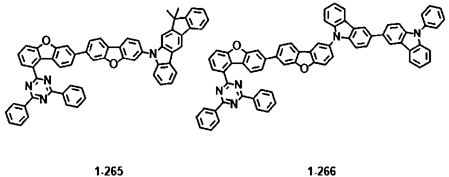

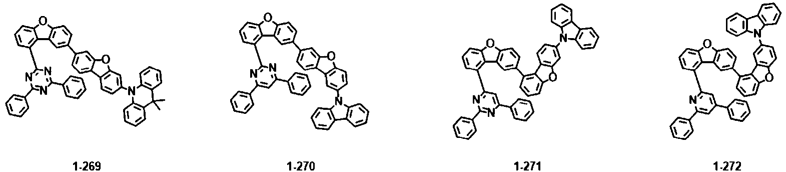

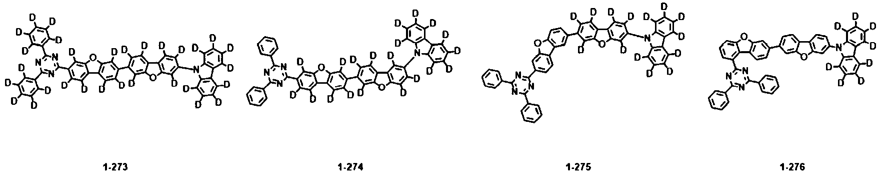

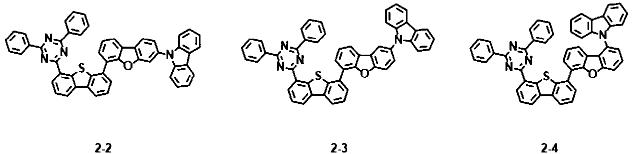

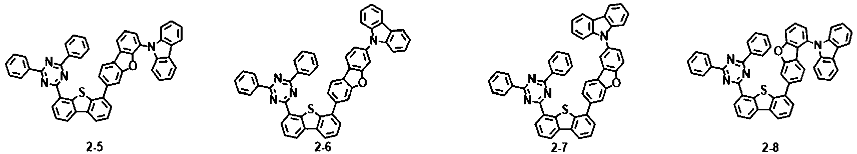

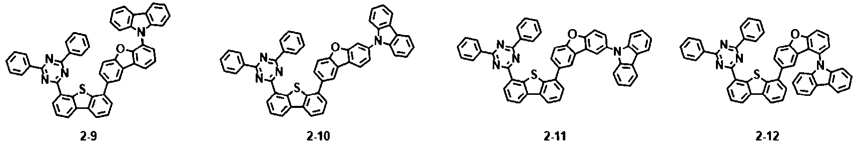

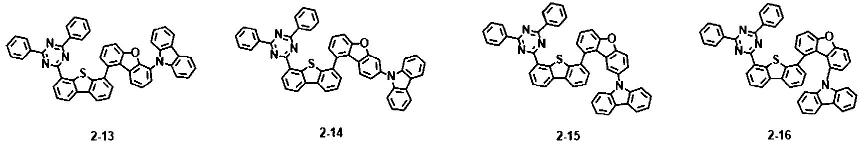

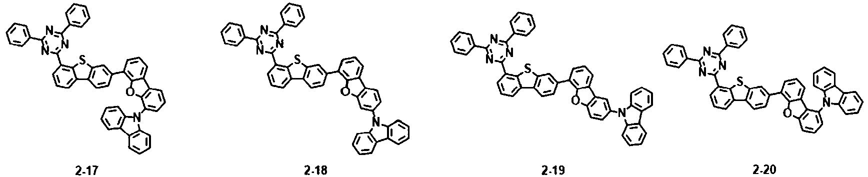

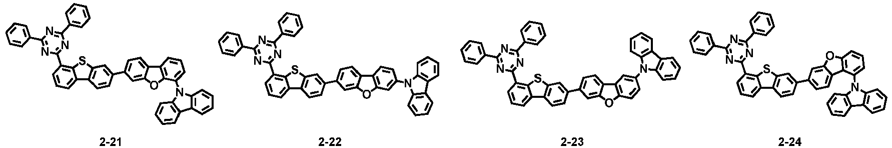

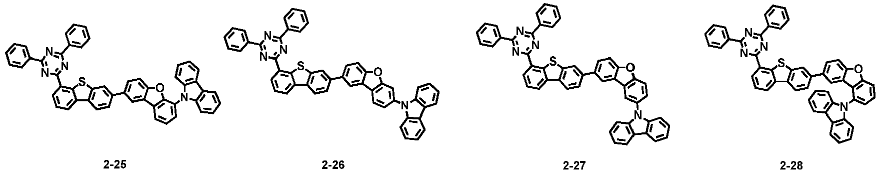

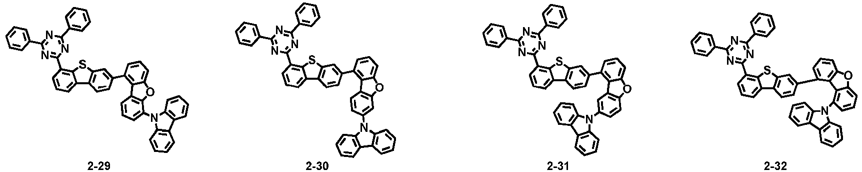

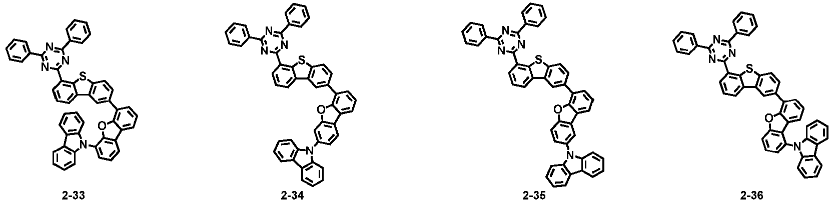

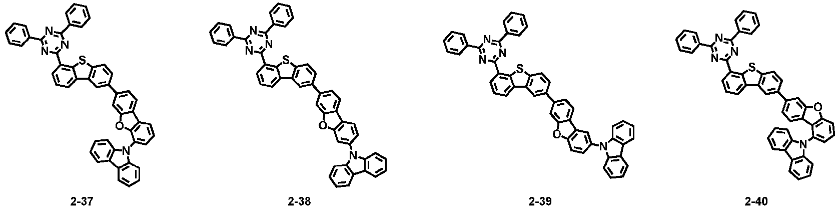

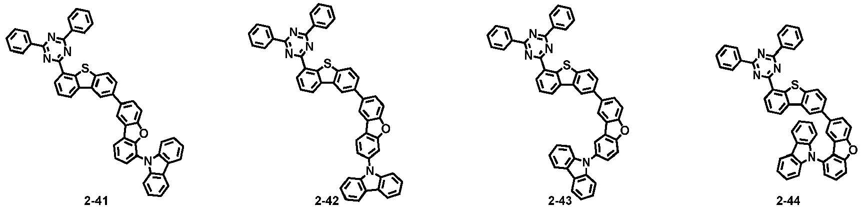

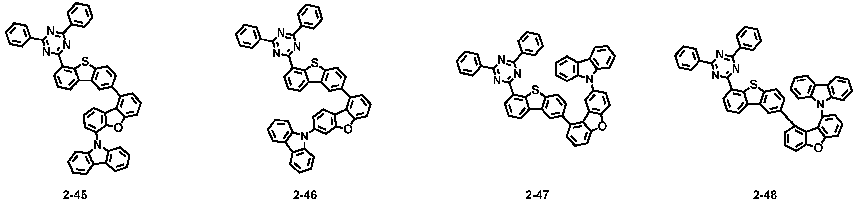

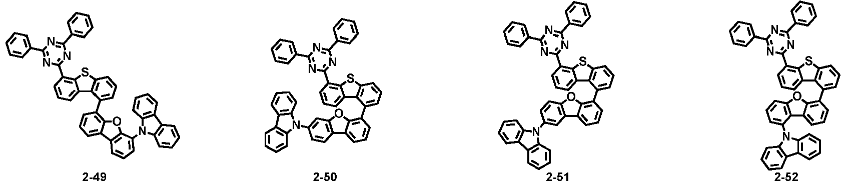

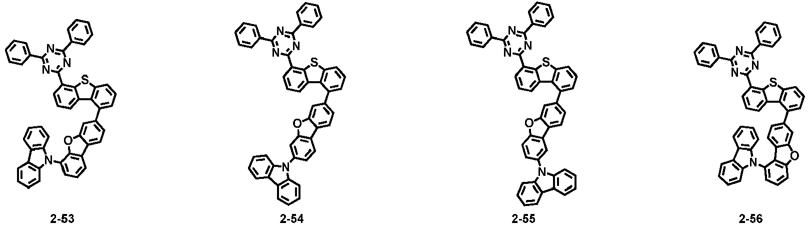

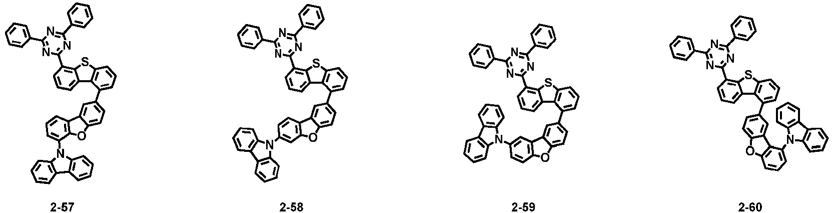

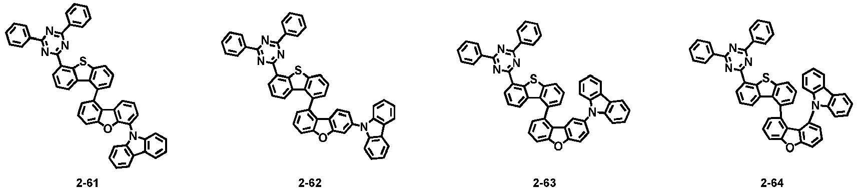

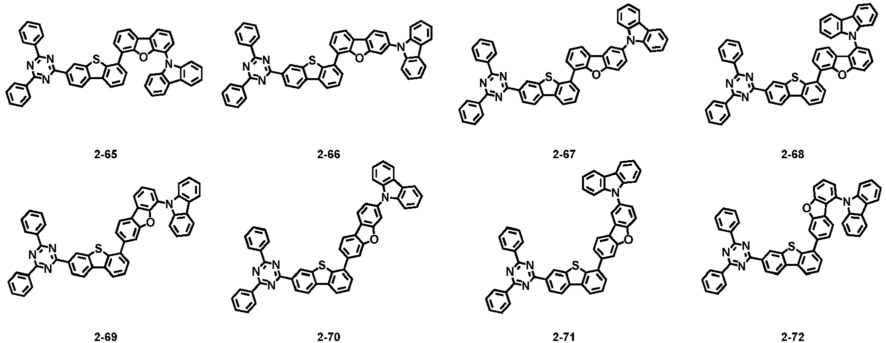

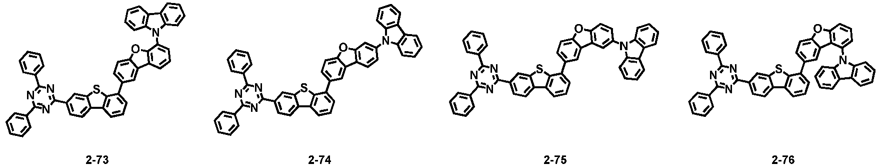

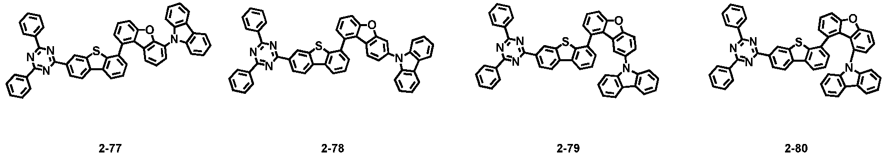

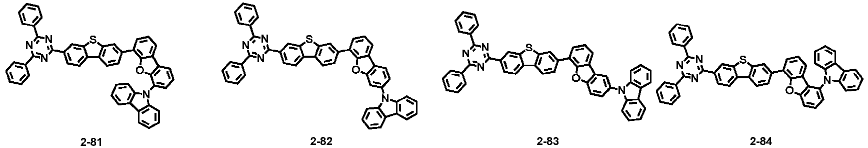

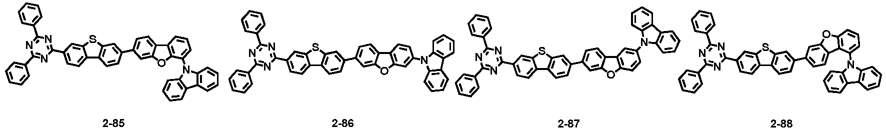

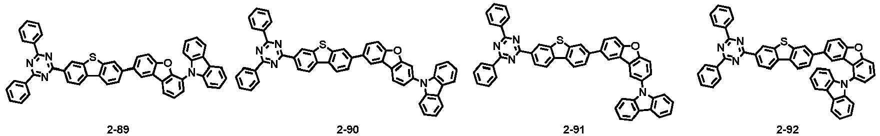

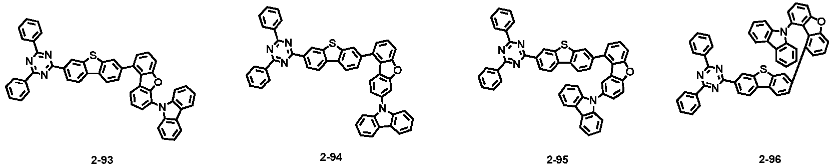

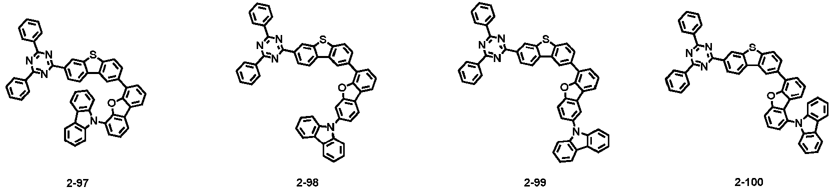

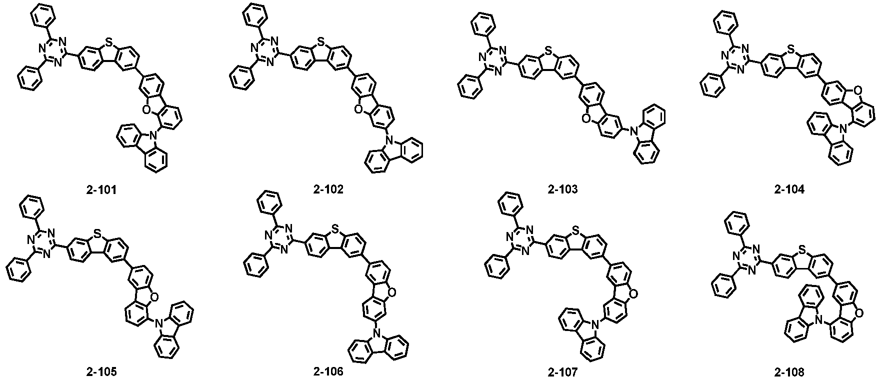

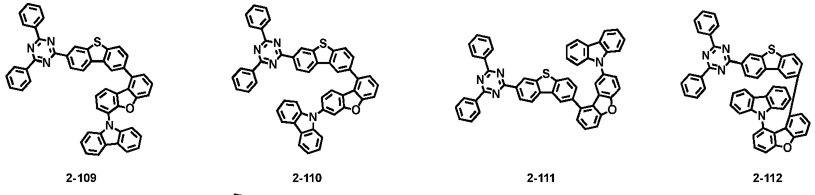

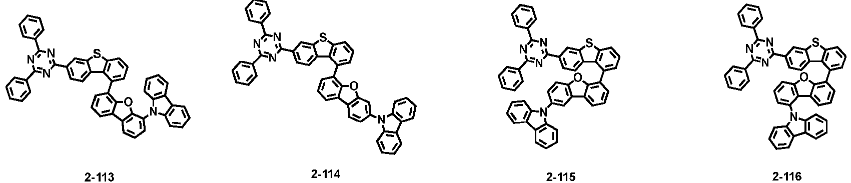

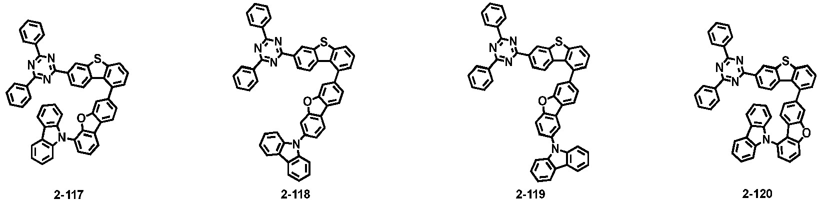

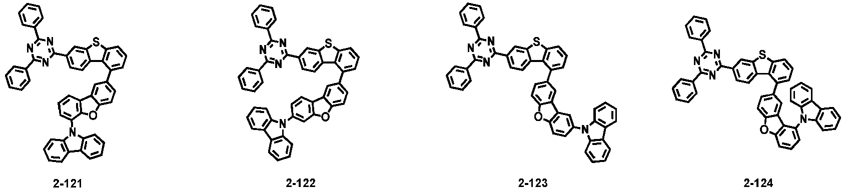

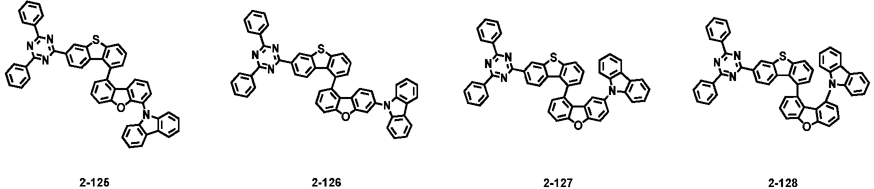

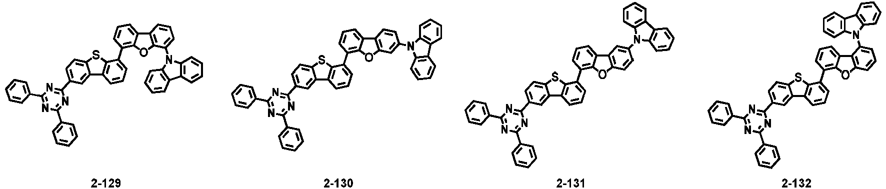

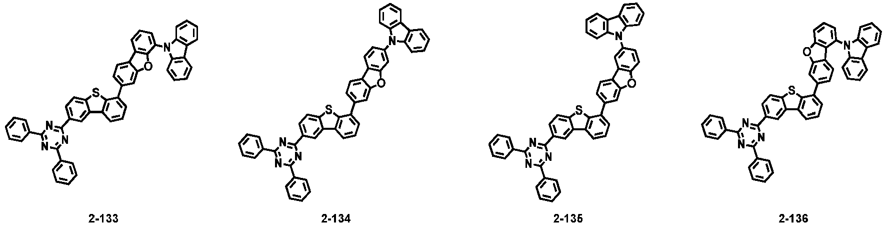

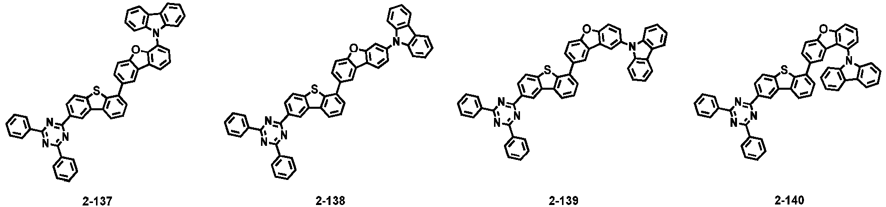

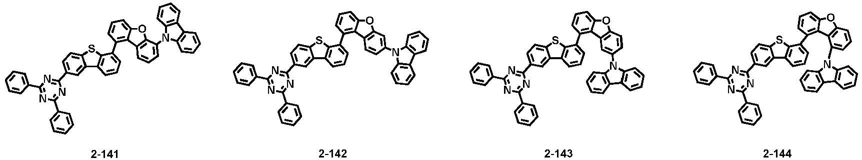

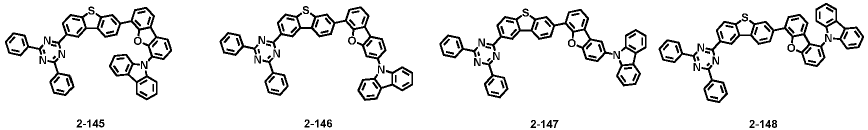

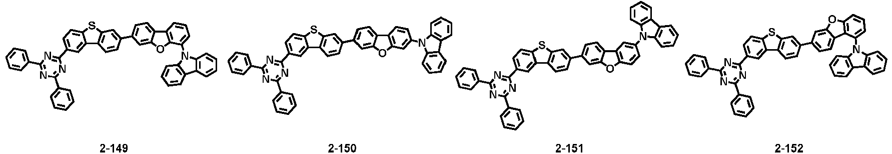

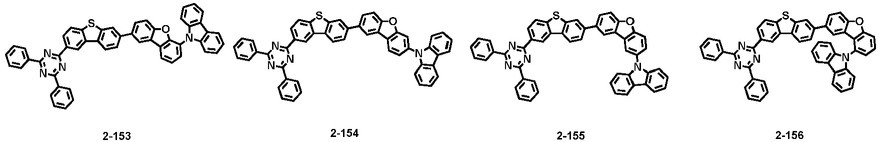

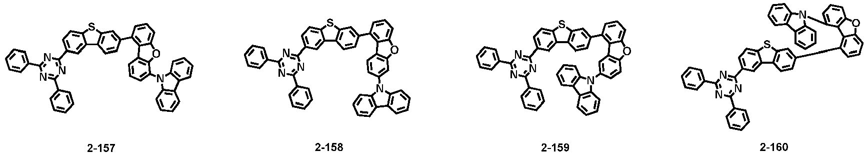

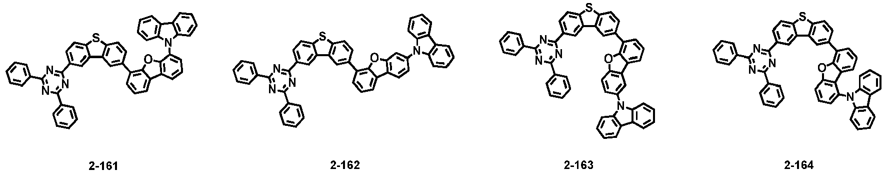

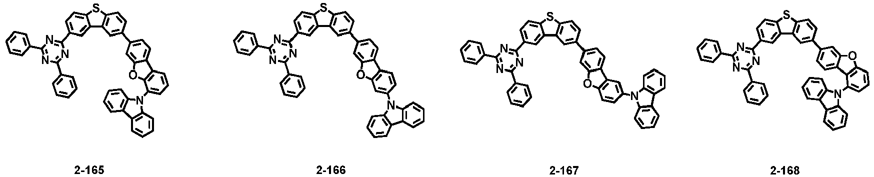

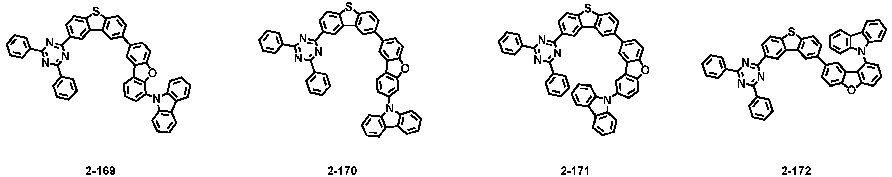

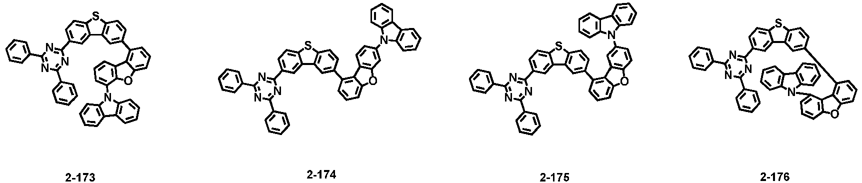

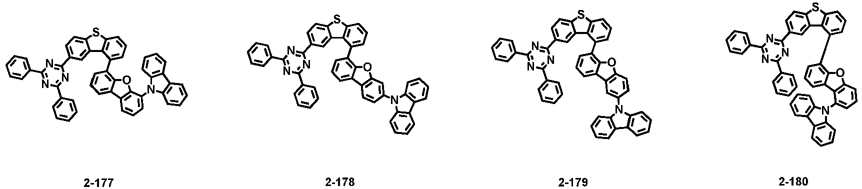

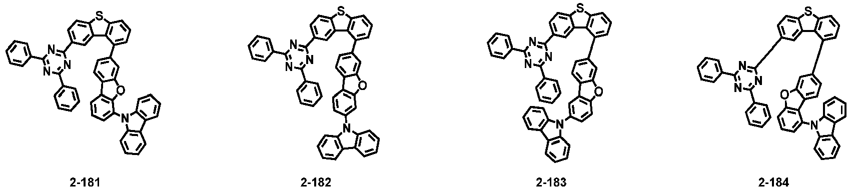

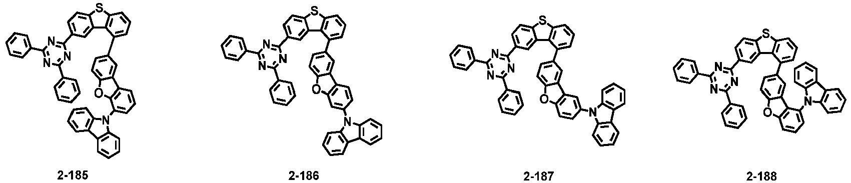

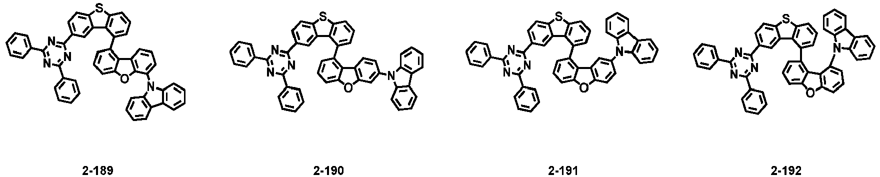

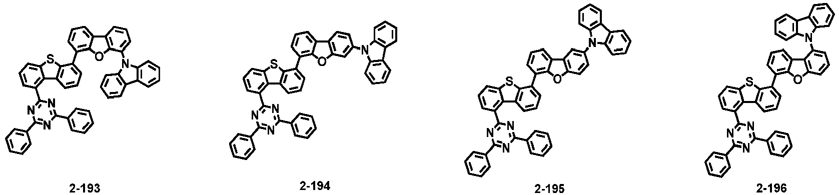

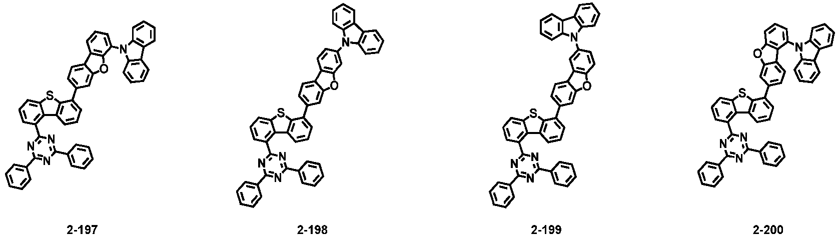

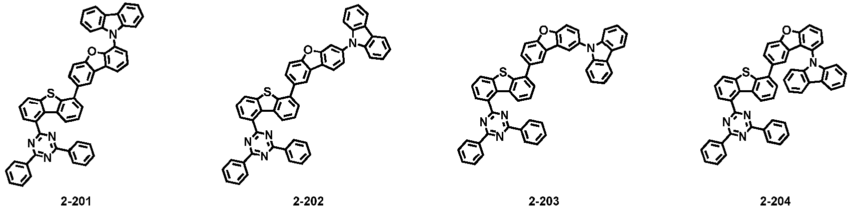

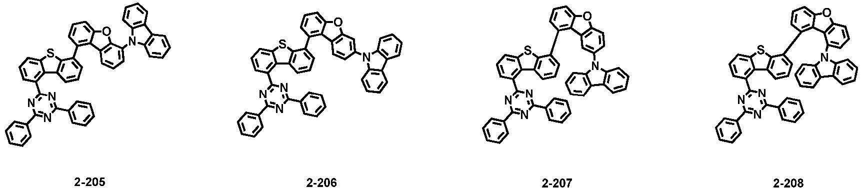

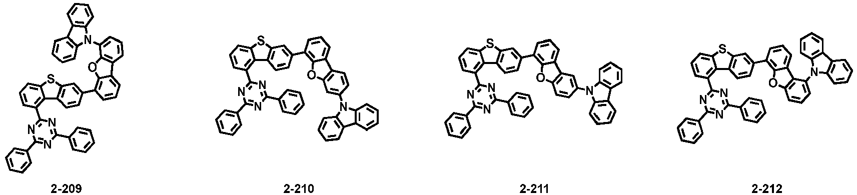

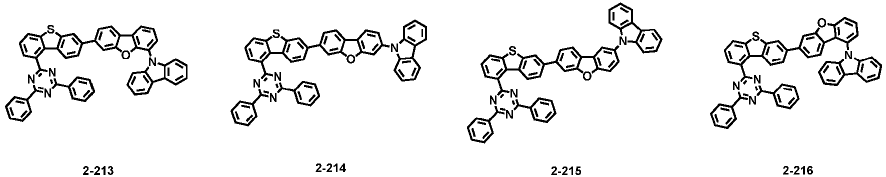

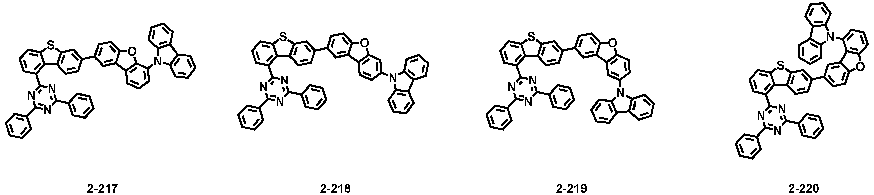

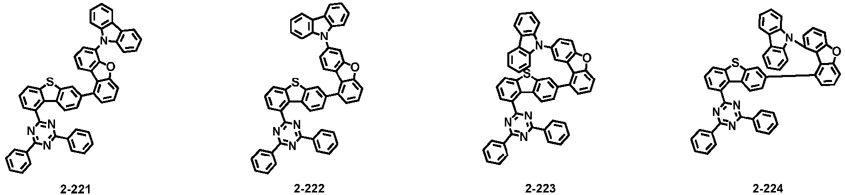

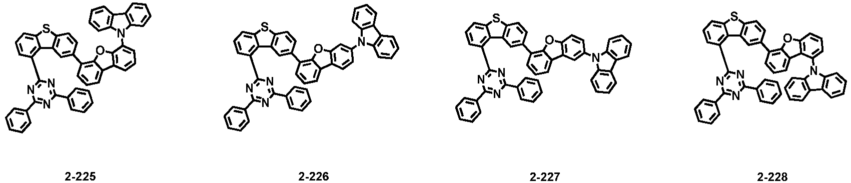

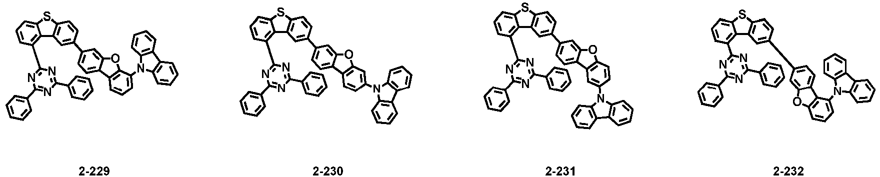

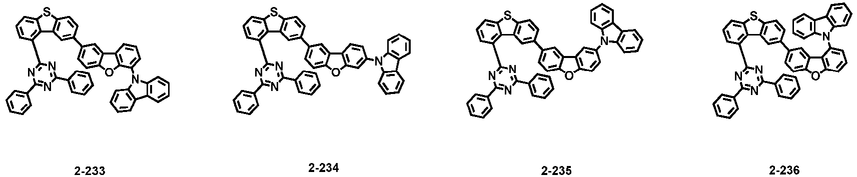

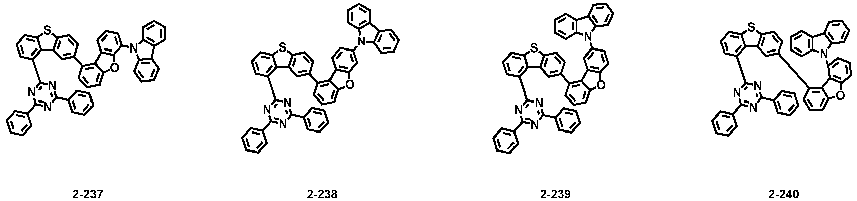

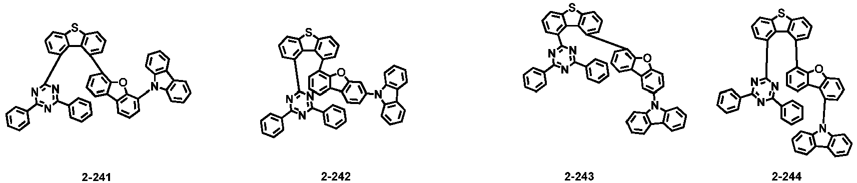

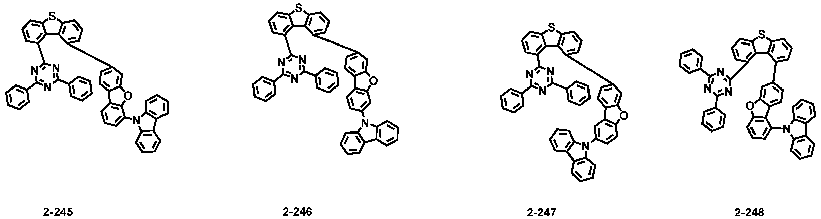

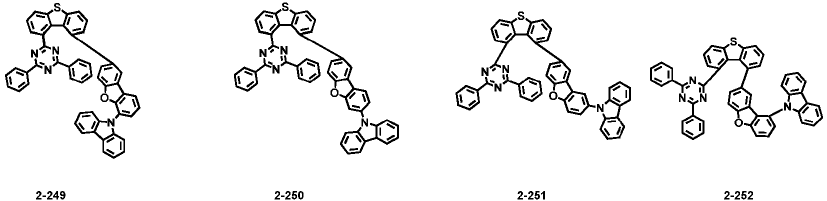

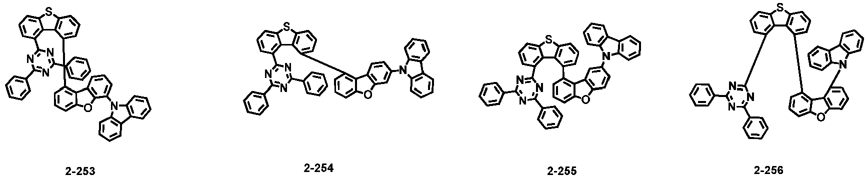

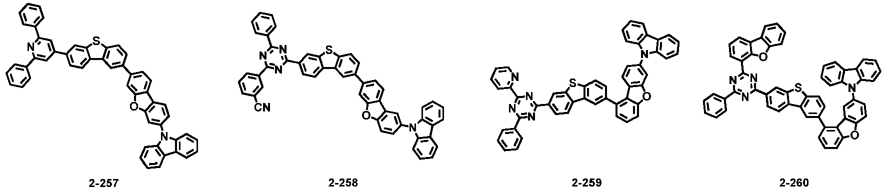

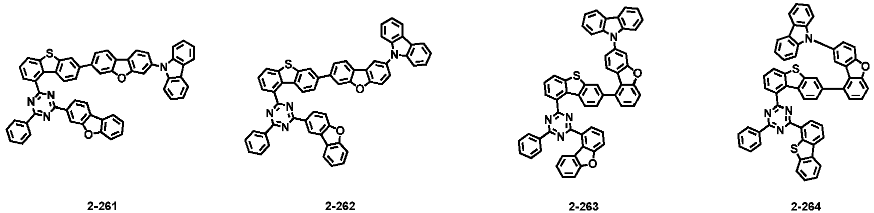

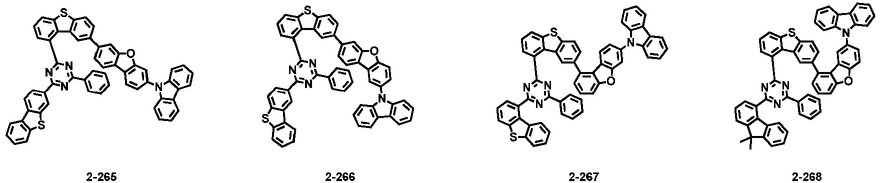

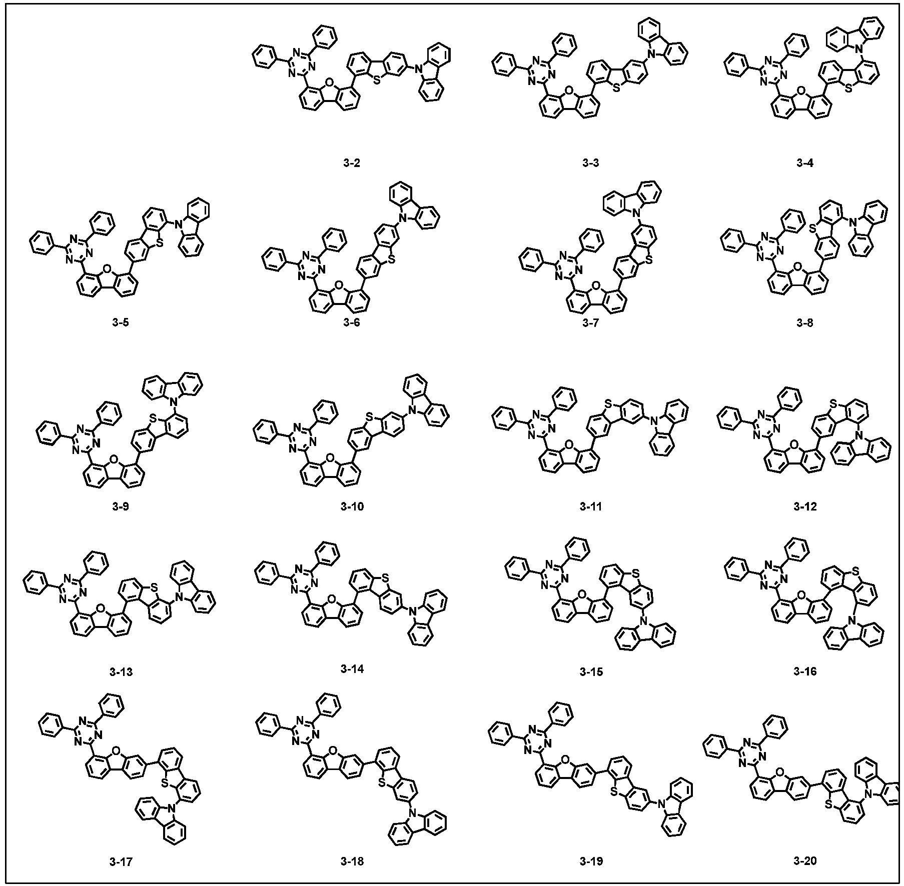

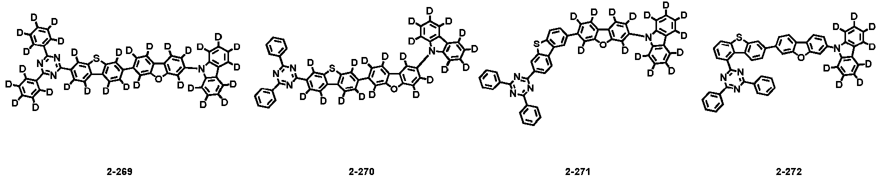

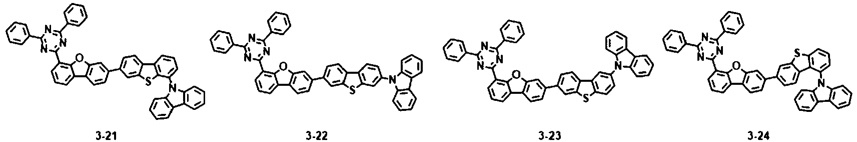

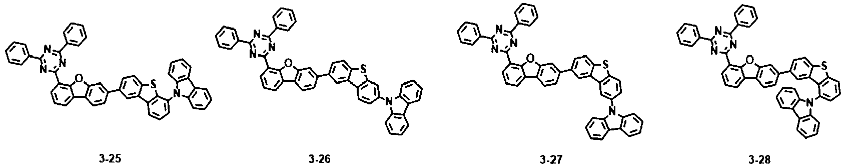

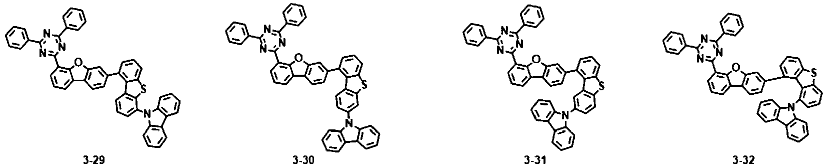

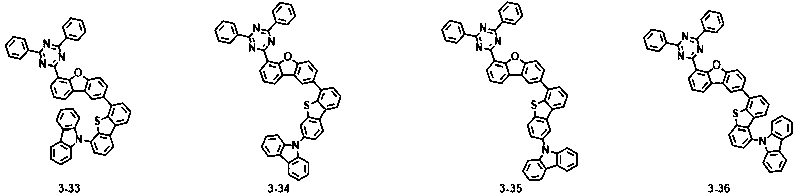

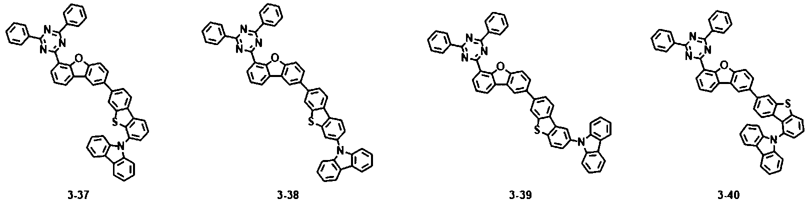

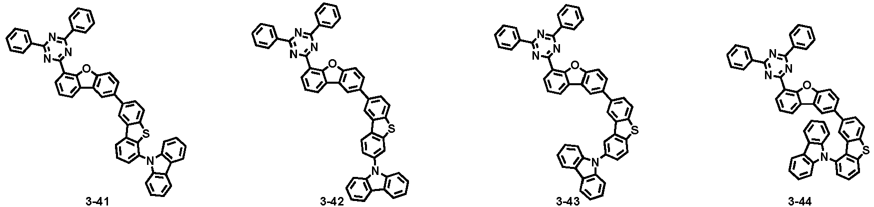

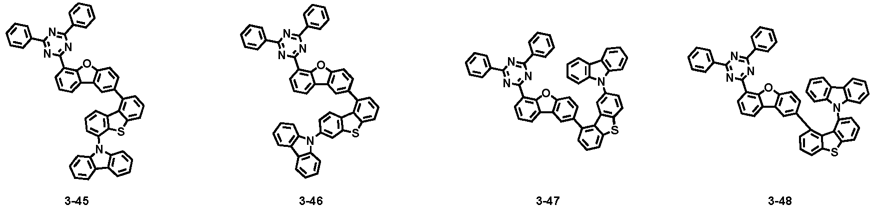

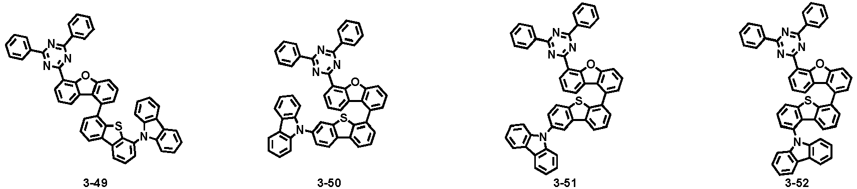

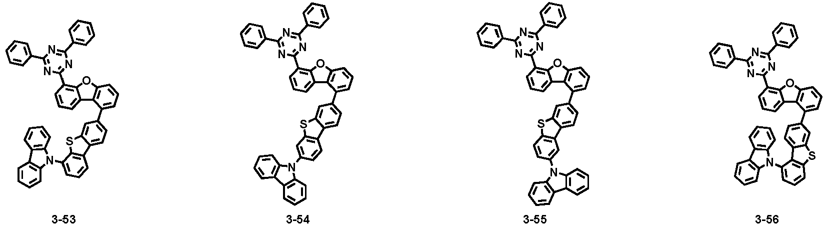

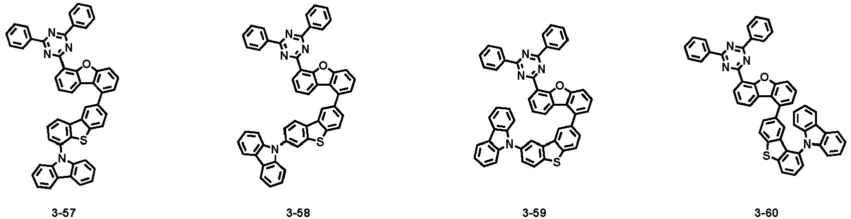

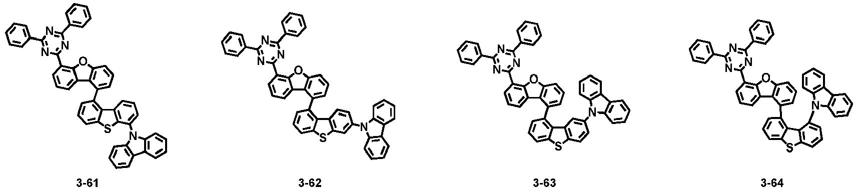

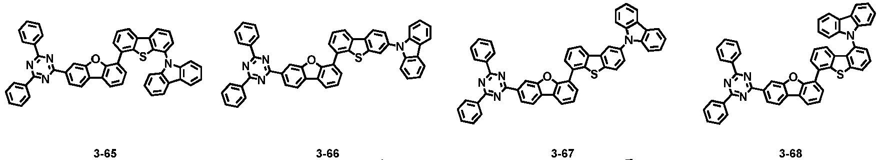

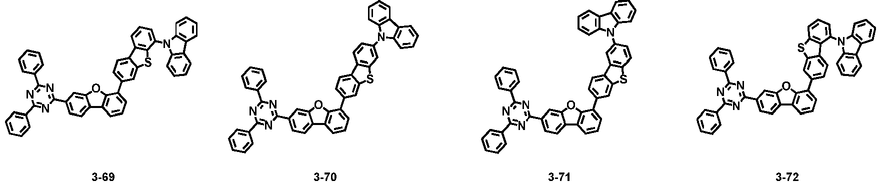

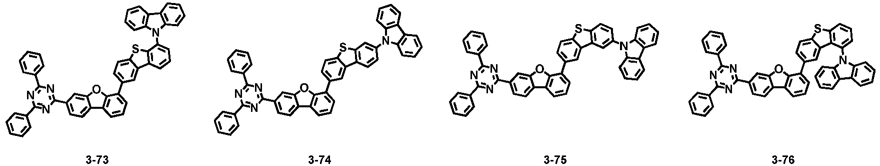

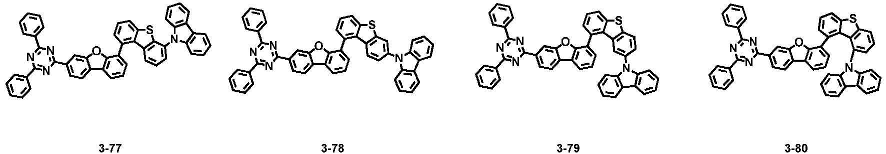

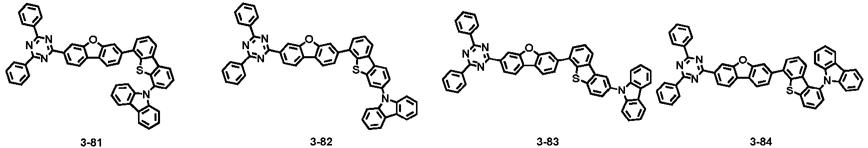

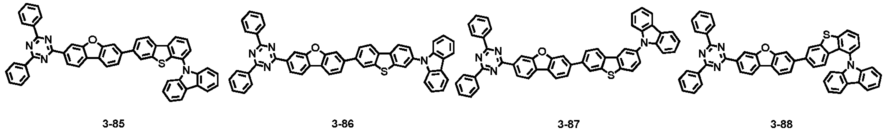

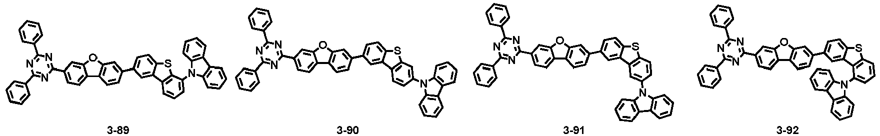

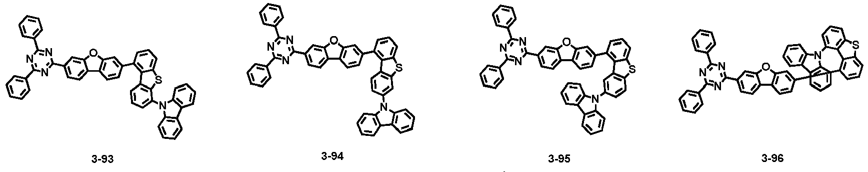

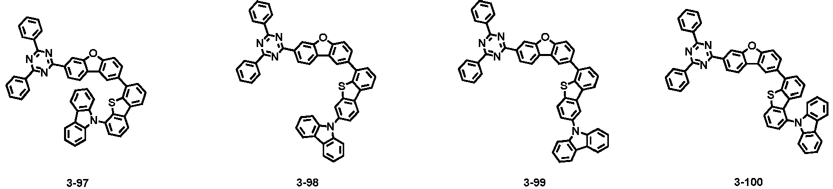

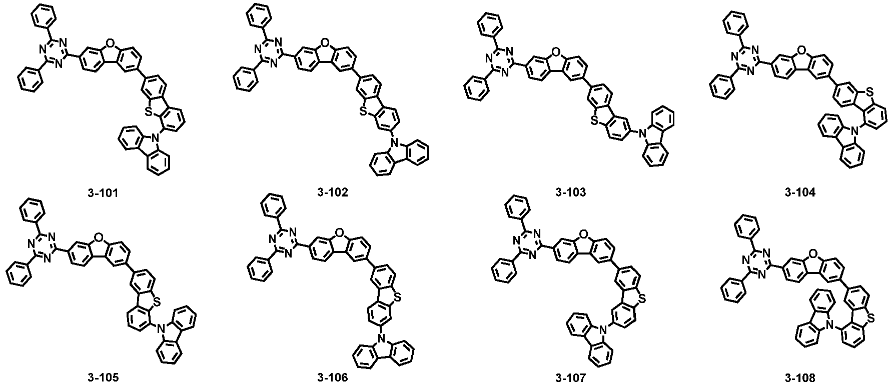

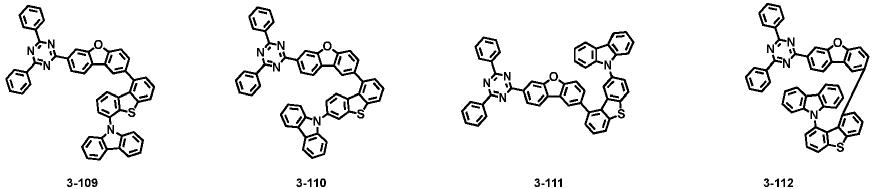

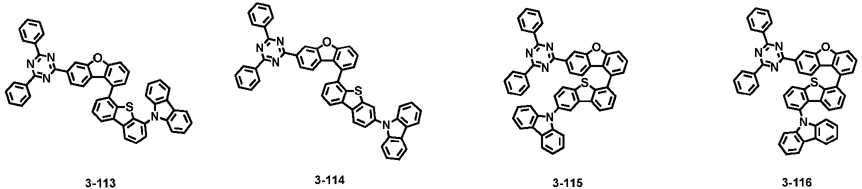

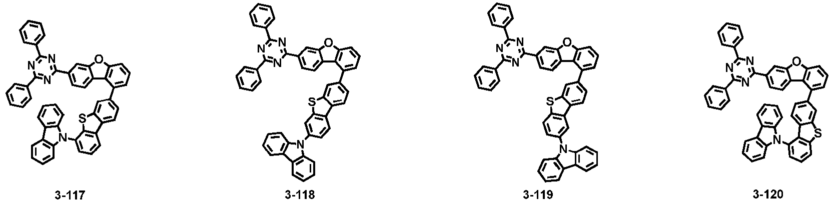

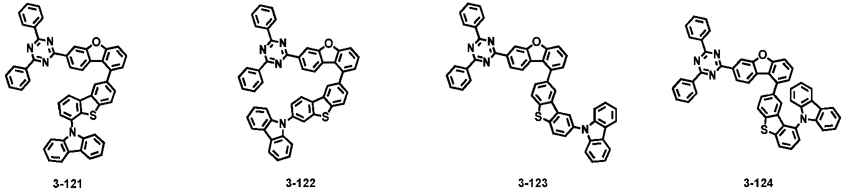

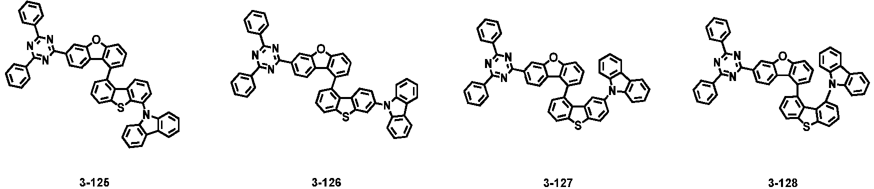

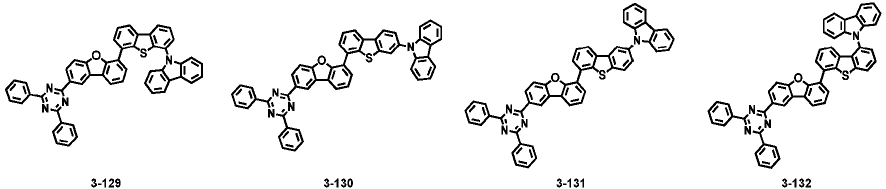

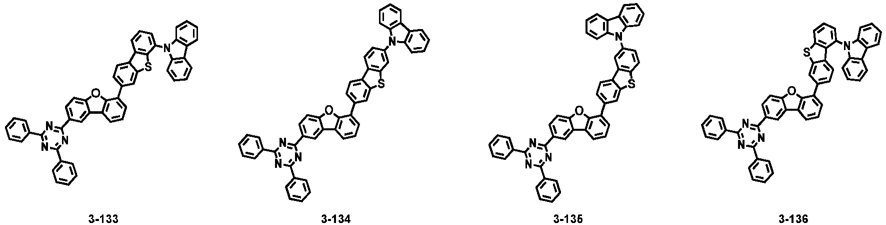

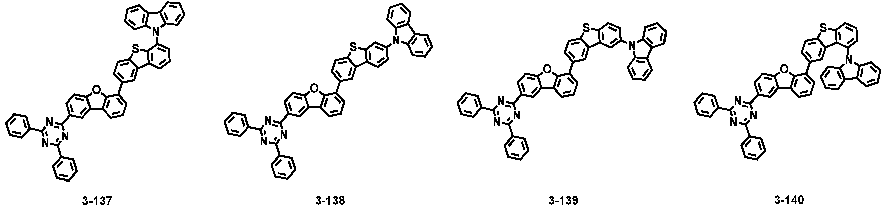

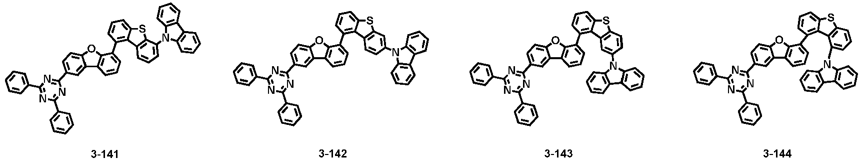

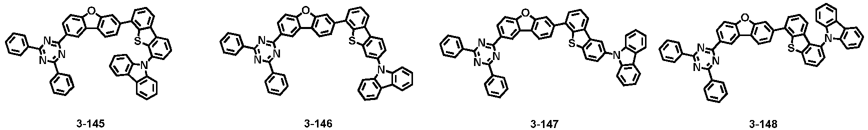

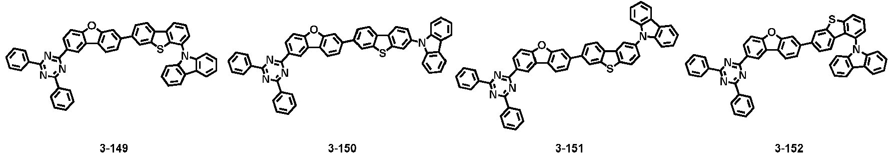

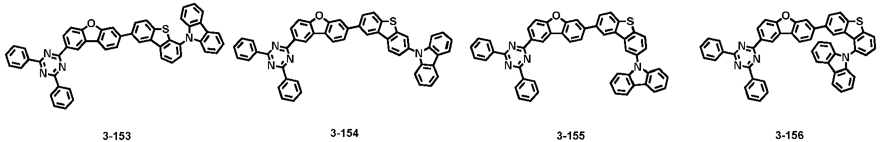

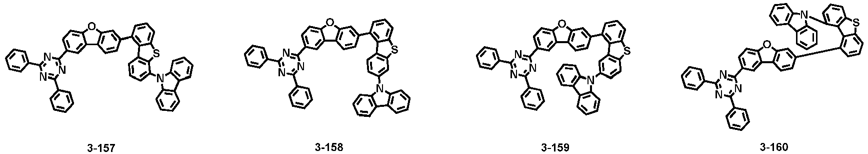

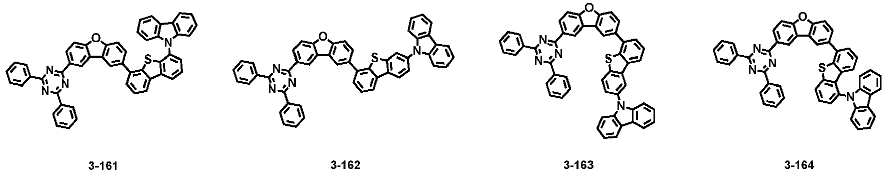

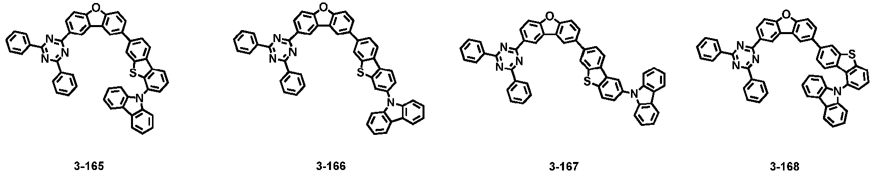

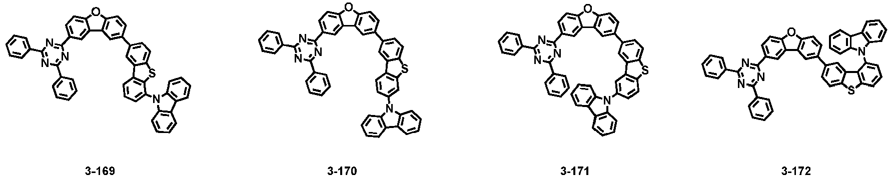

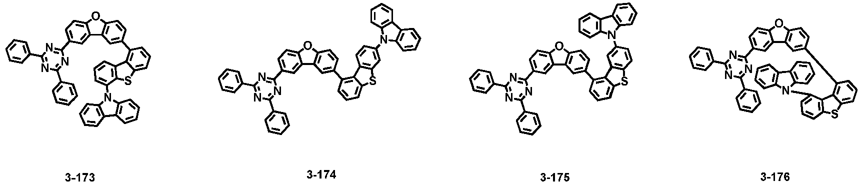

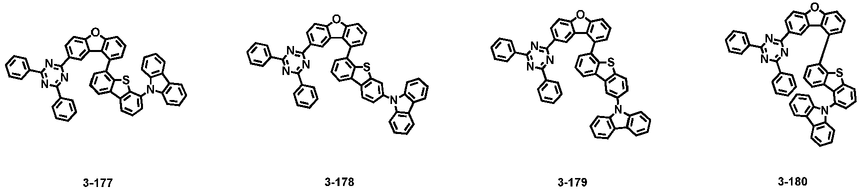

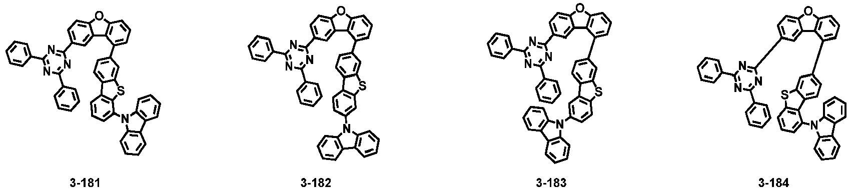

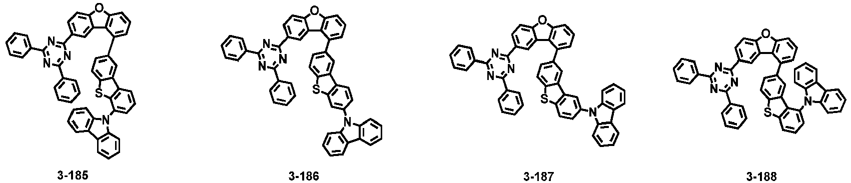

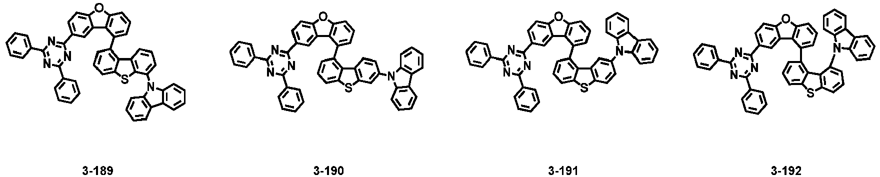

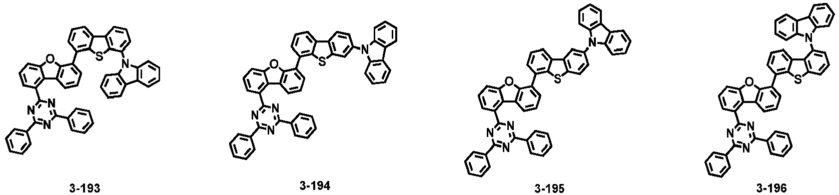

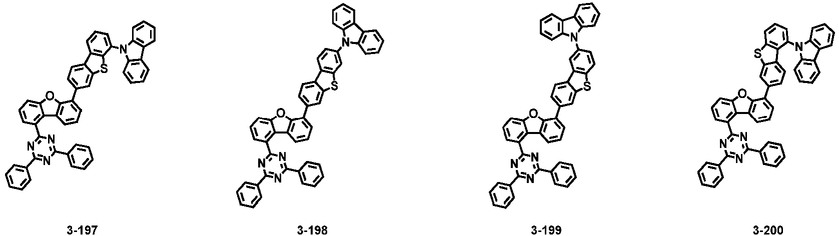

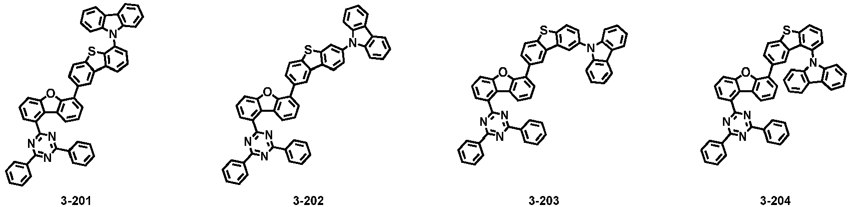

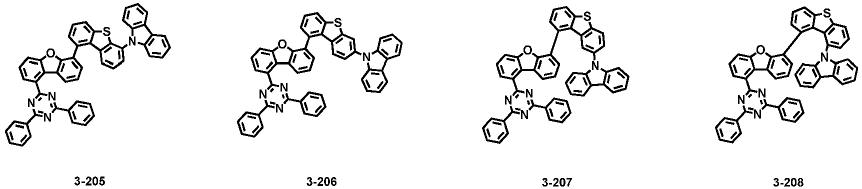

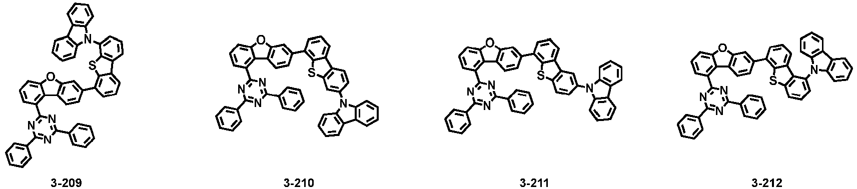

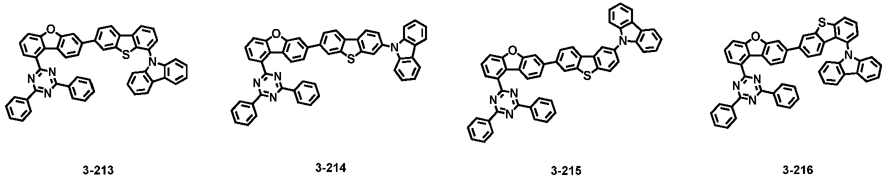

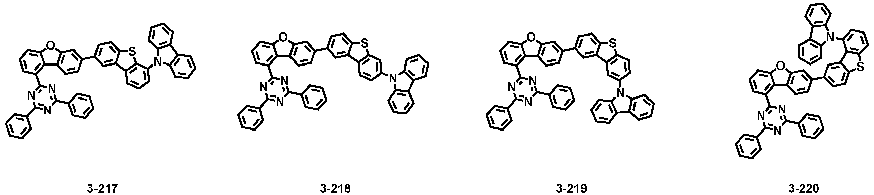

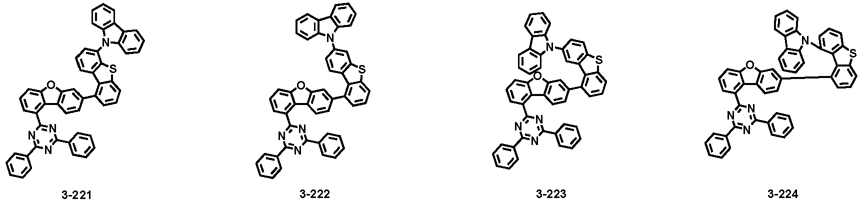

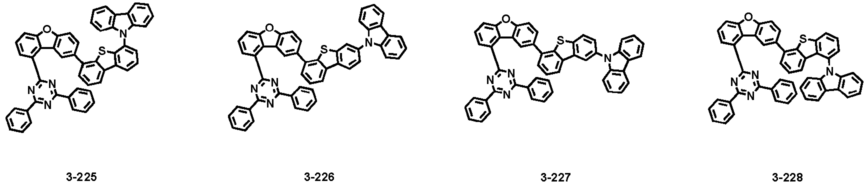

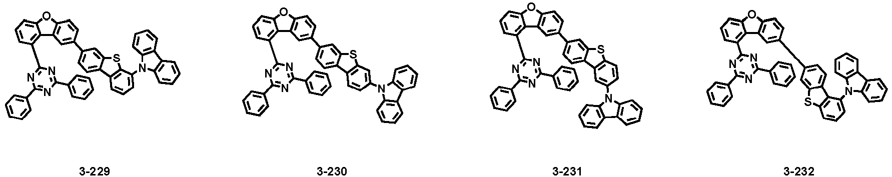

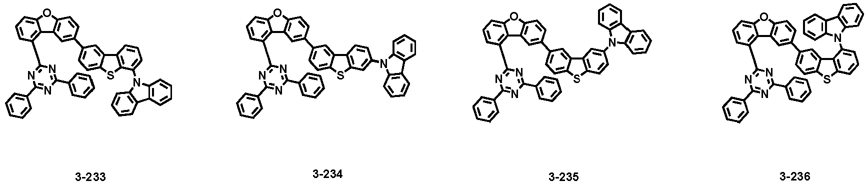

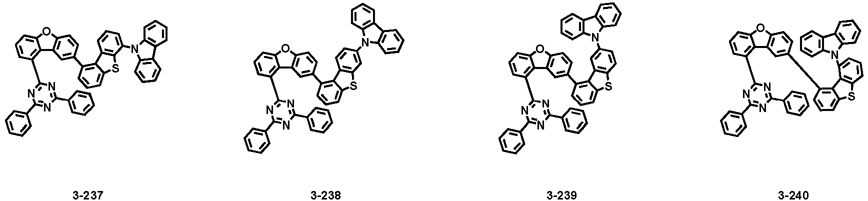

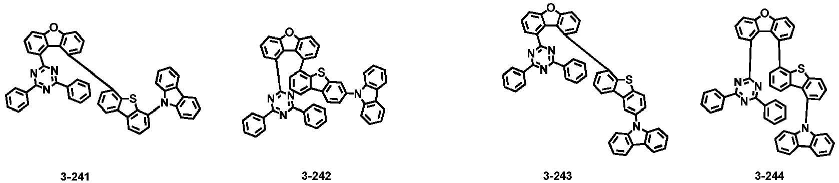

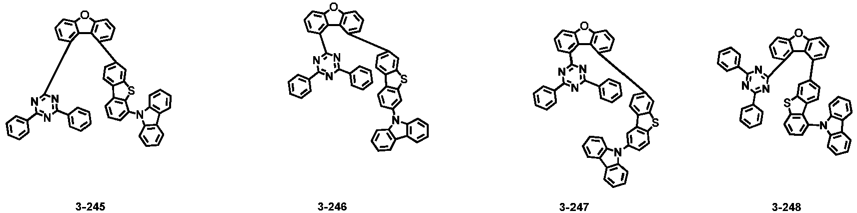

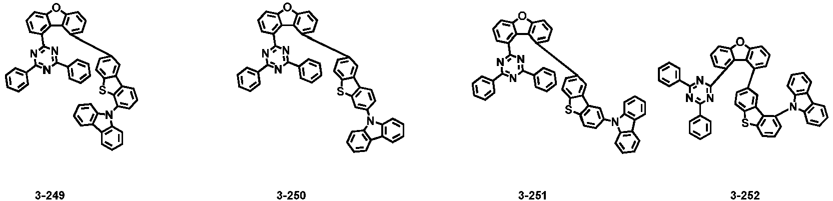

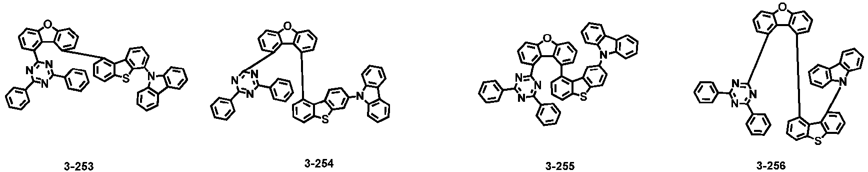

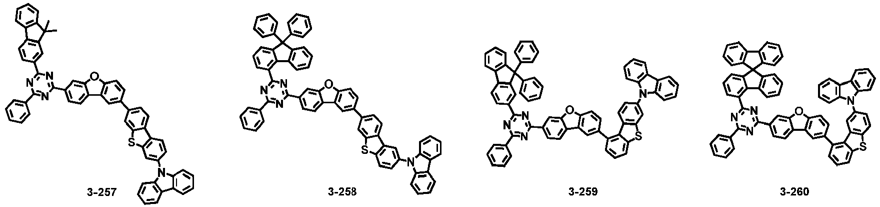

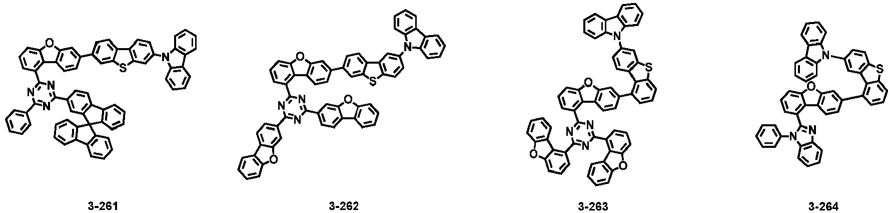

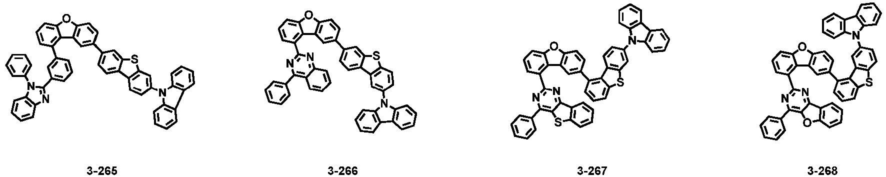

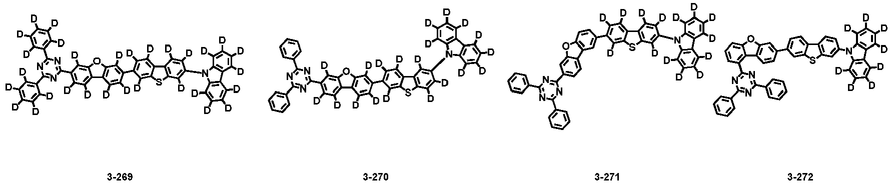

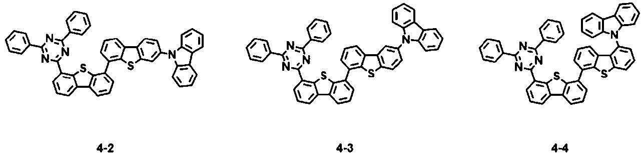

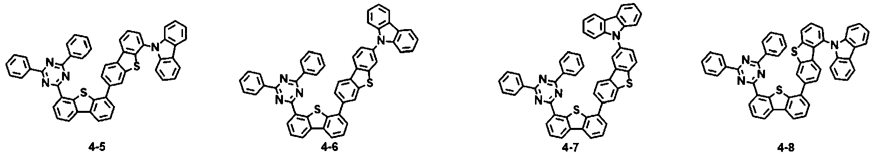

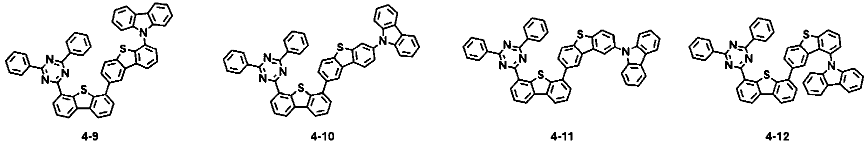

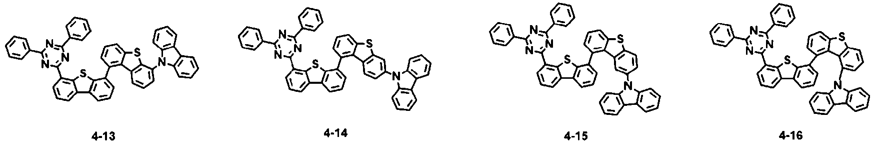

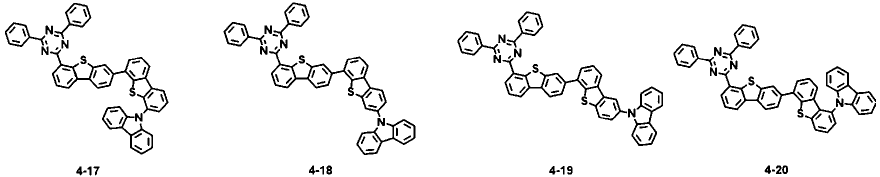

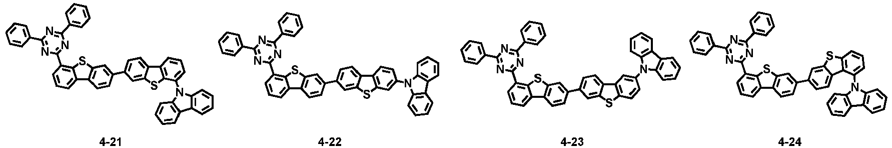

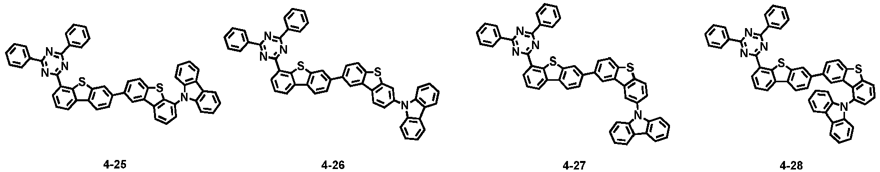

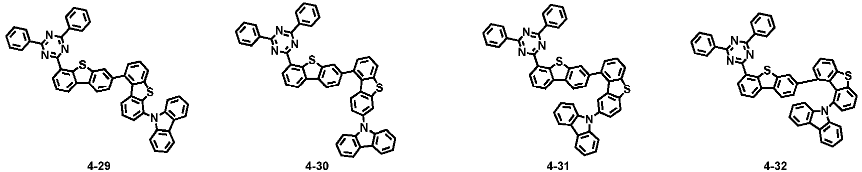

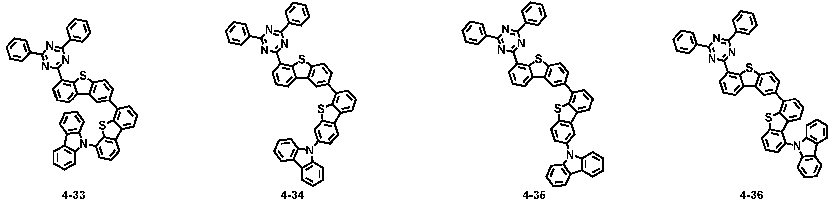

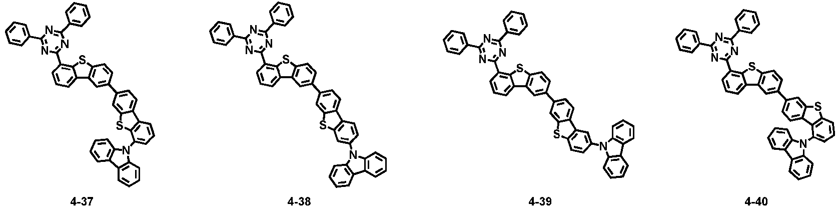

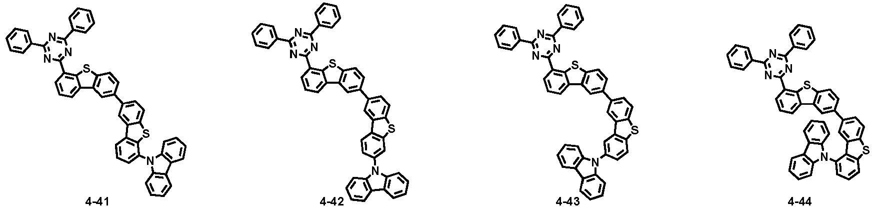

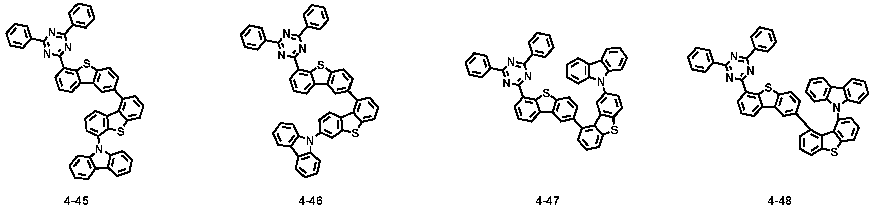

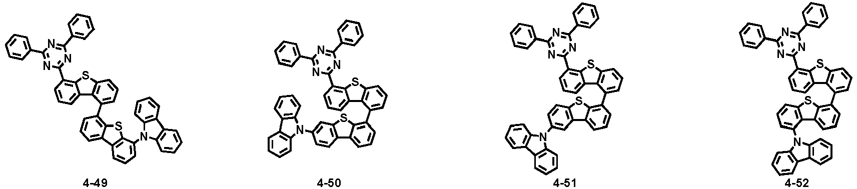

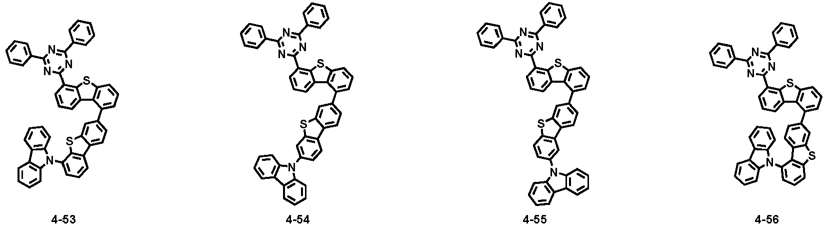

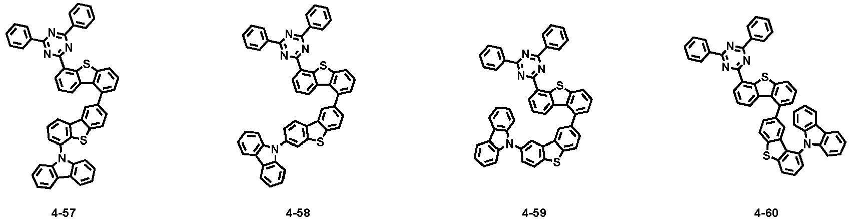

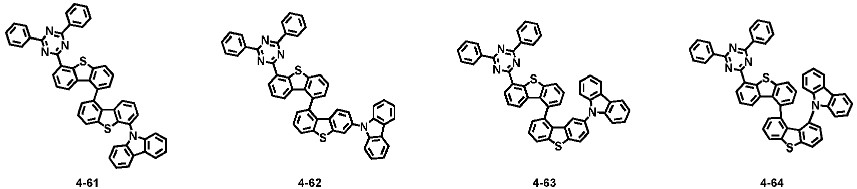

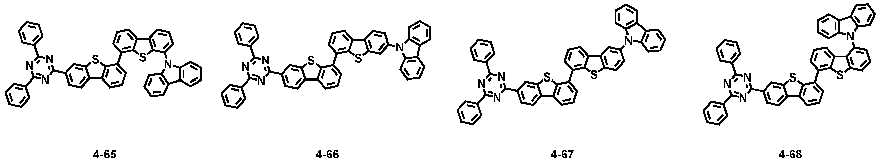

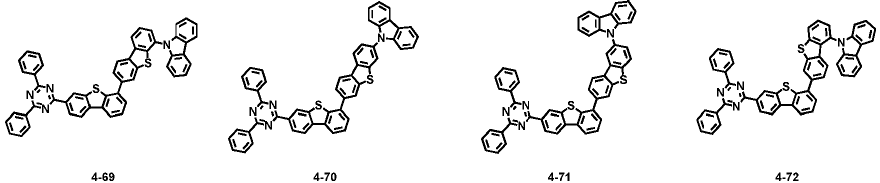

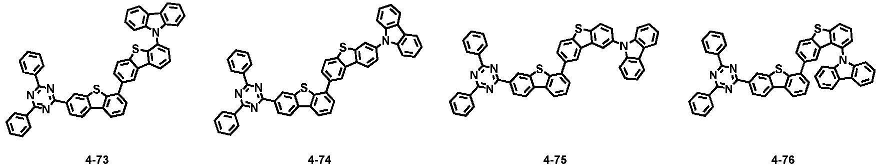

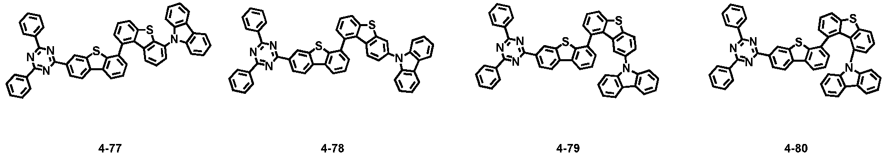

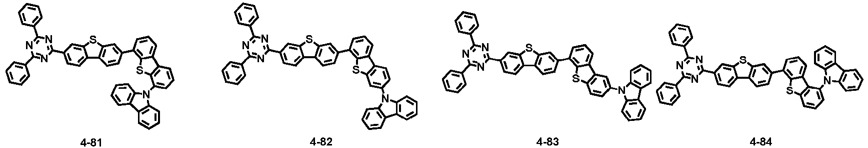

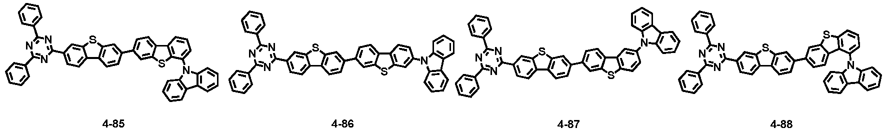

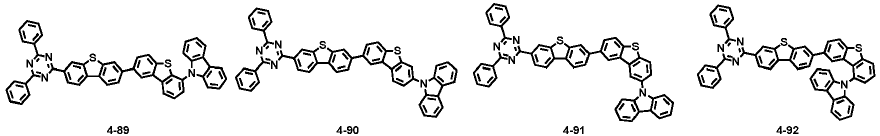

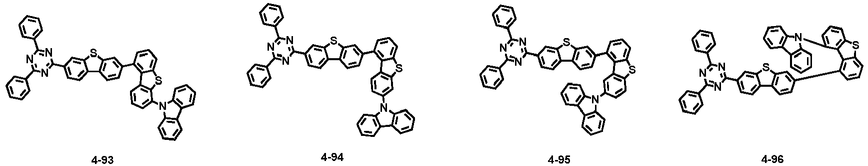

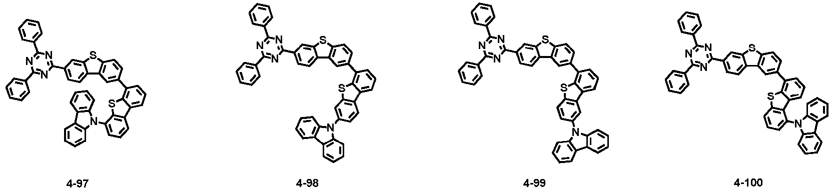

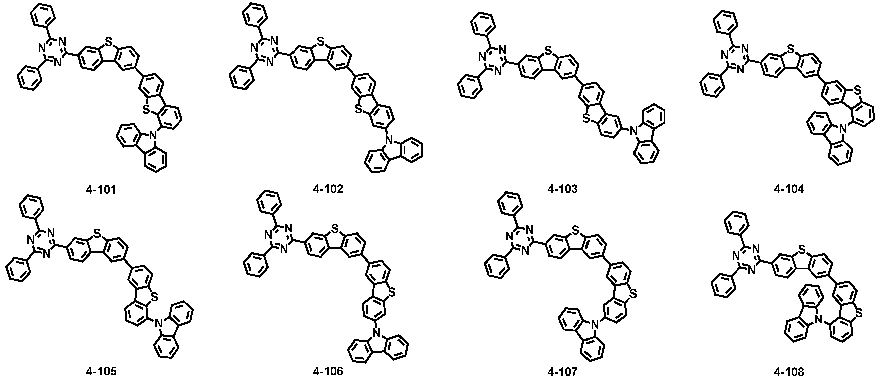

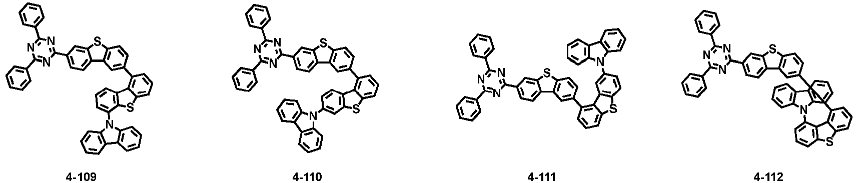

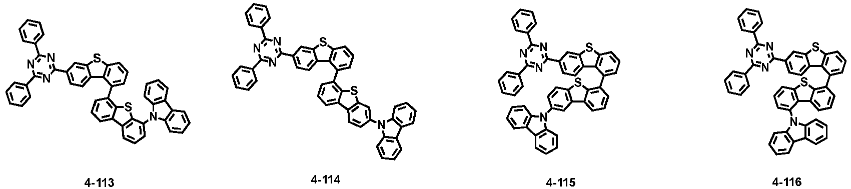

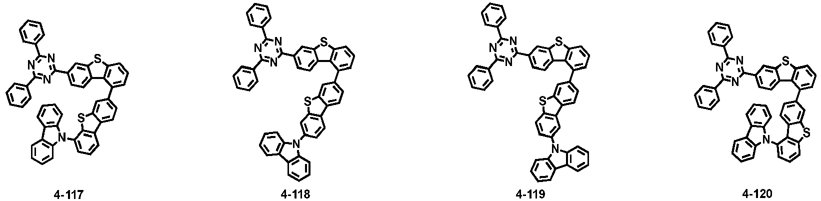

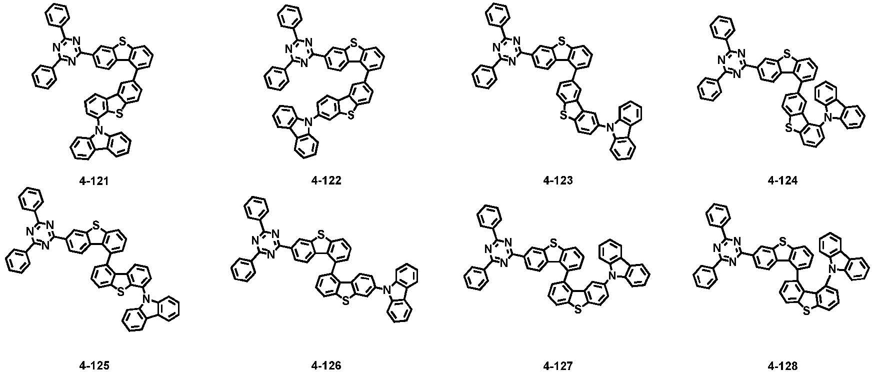

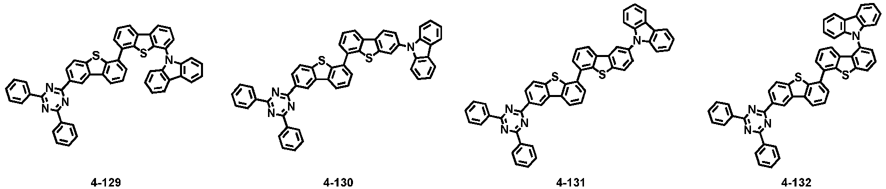

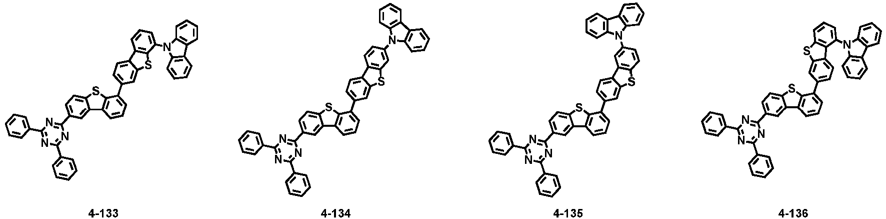

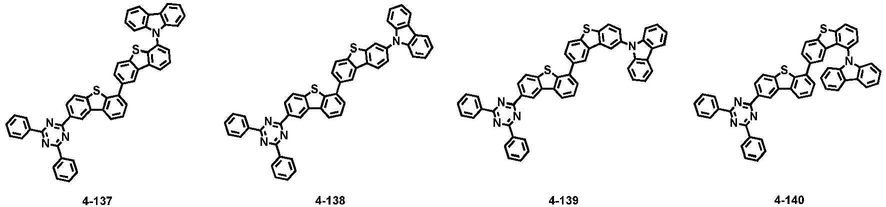

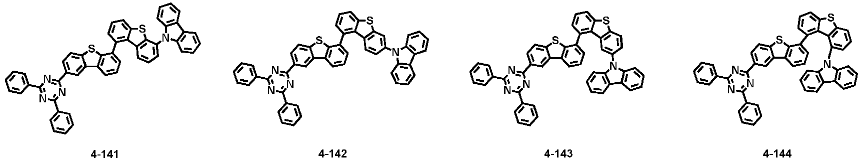

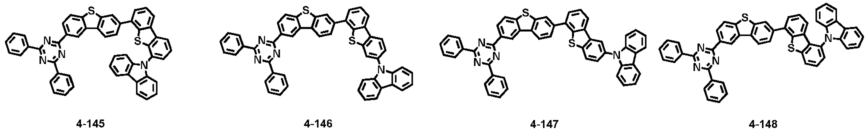

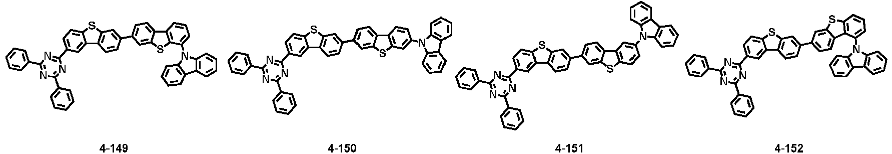

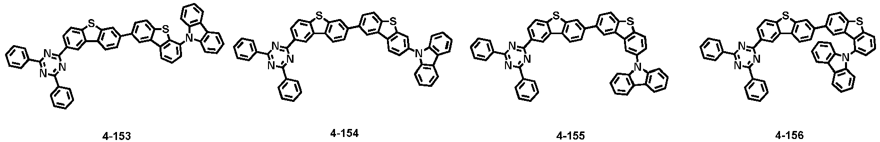

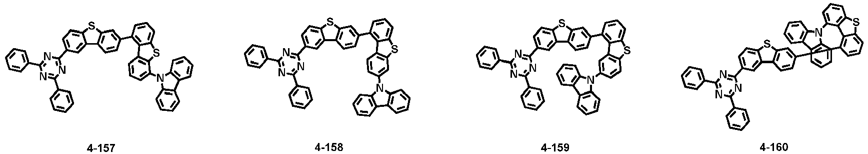

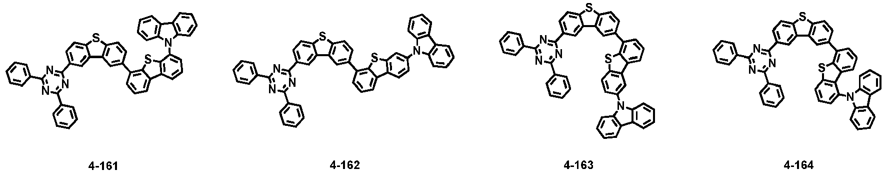

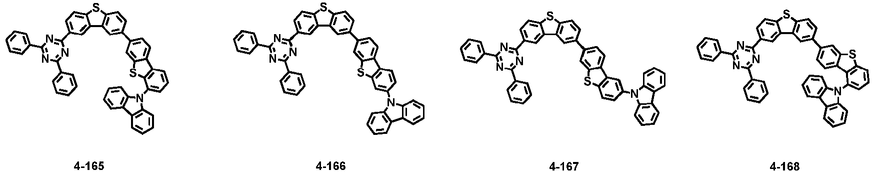

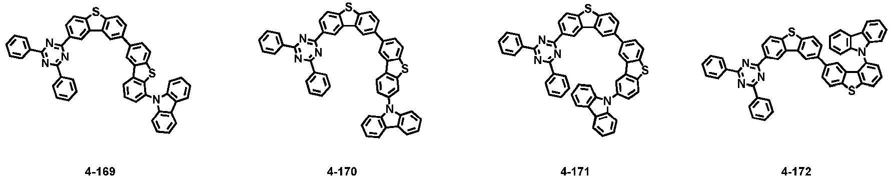

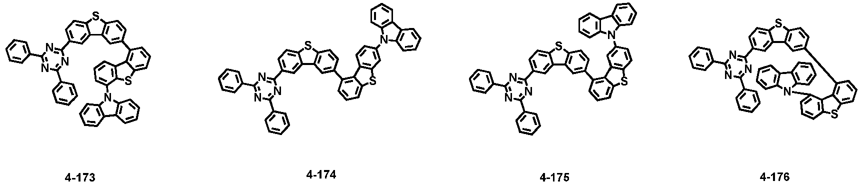

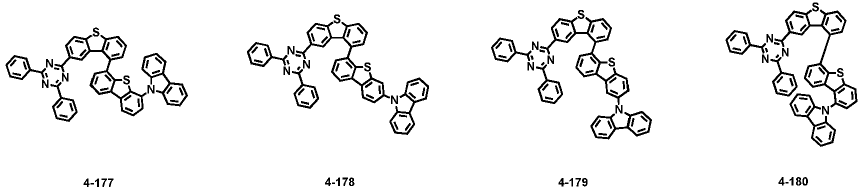

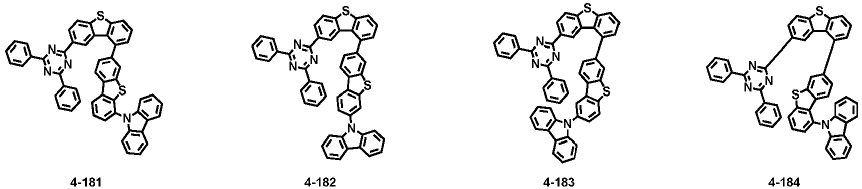

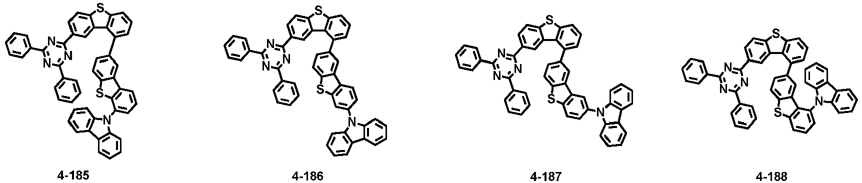

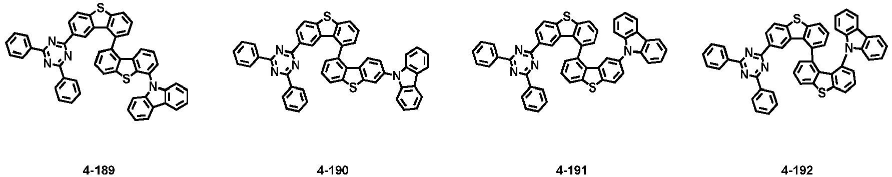

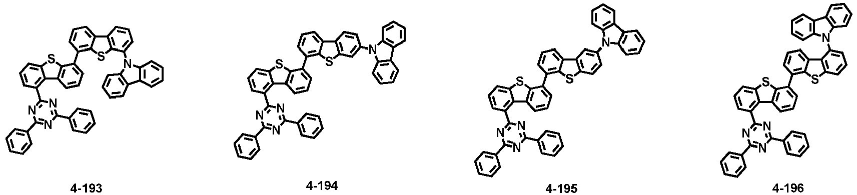

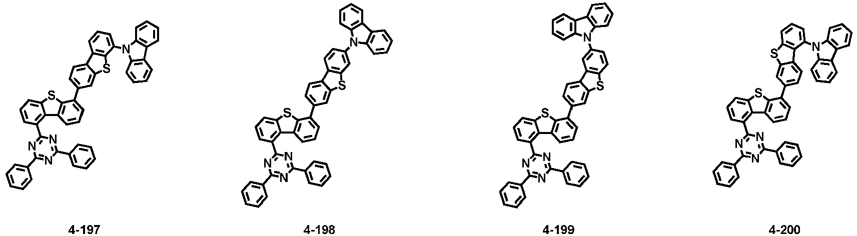

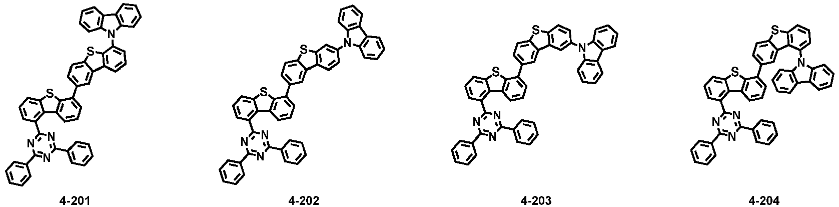

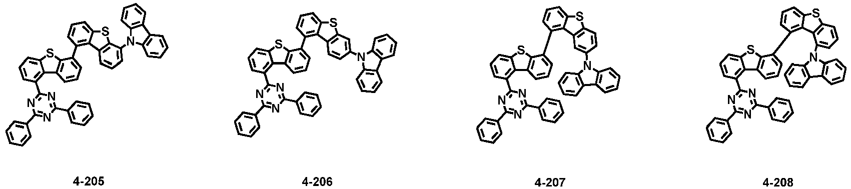

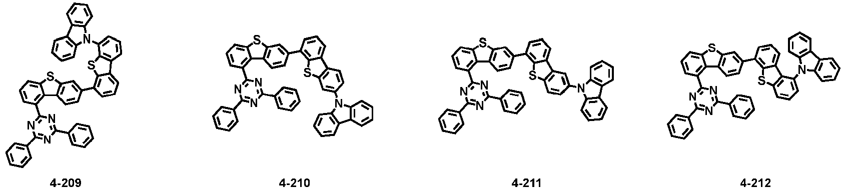

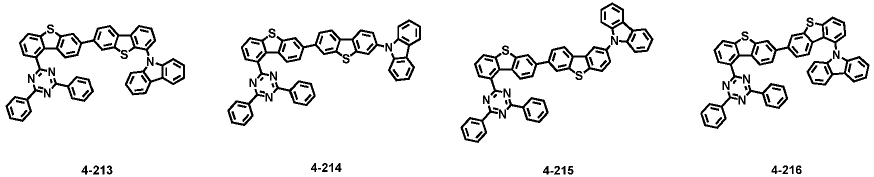

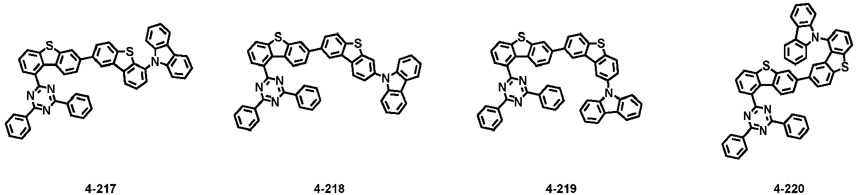

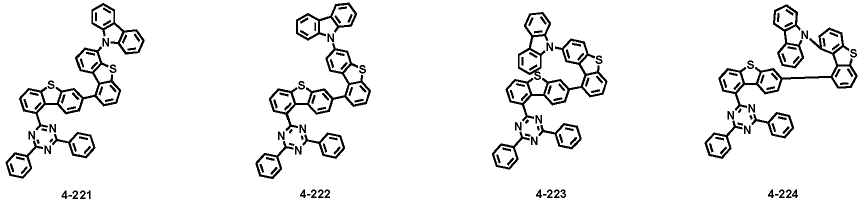

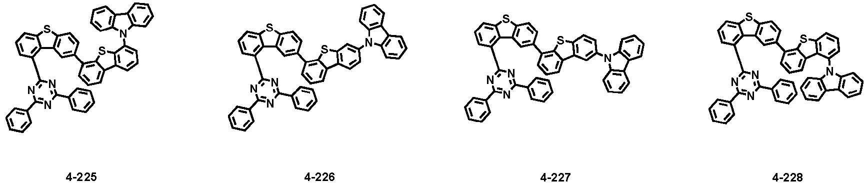

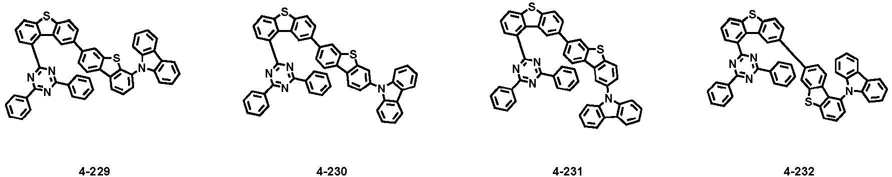

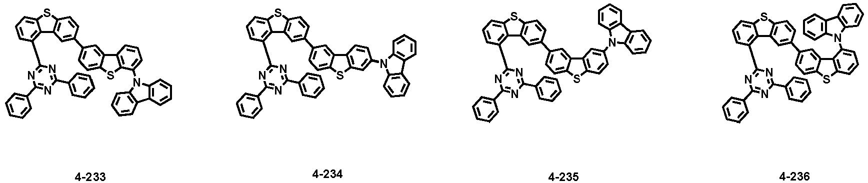

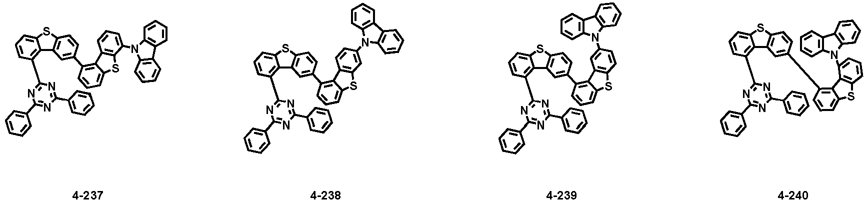

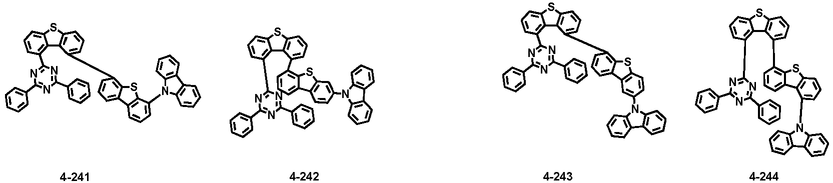

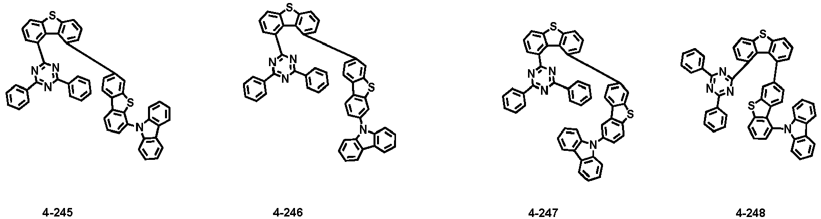

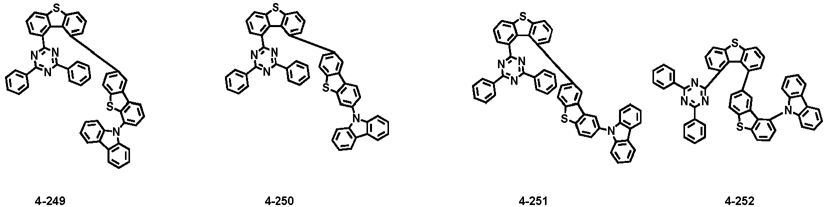

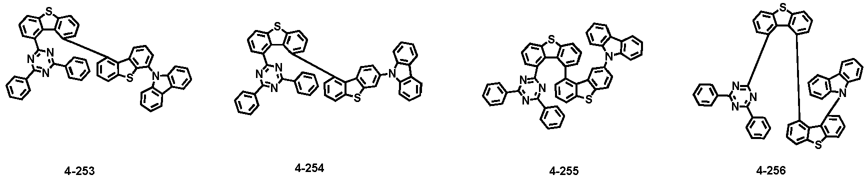

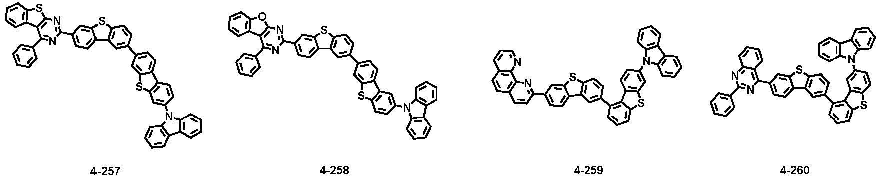

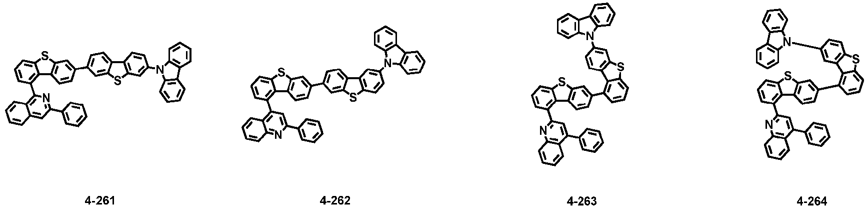

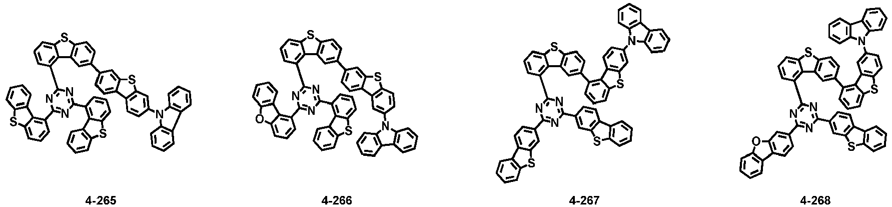

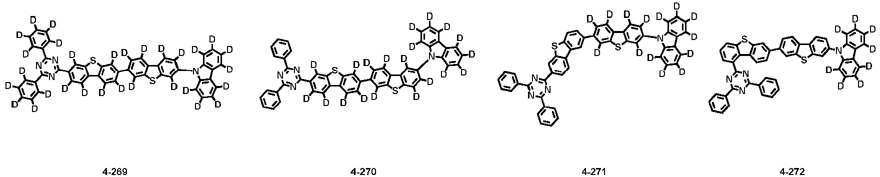

Another embodiment of the present application provides an organic light emitting device comprising a first electrode, a second electrode, and one or more organic material layers provided between the first electrode and the second electrode, wherein one or more layers of the organic material layers comprise the heterocyclic compound represented above.

Another embodiment of the present application provides a composition for an organic material layer of an organic light emitting device, the composition comprising the heterocyclic compound represented by any one of Chemical Formulae 1-2 to 1-4 and a heterocyclic compound represented by the following Chemical Formula 13 or 14: 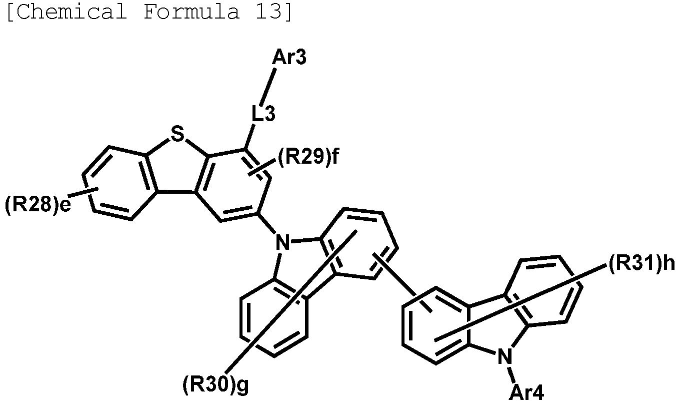 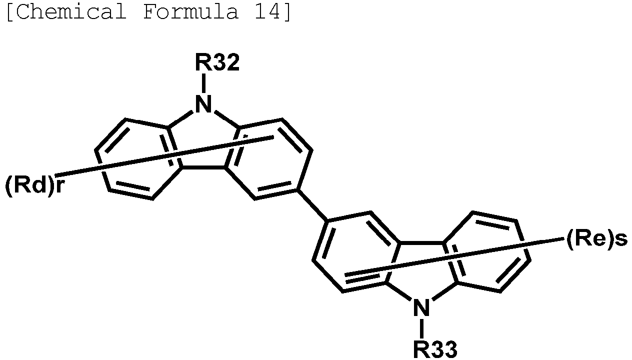 wherein, in Chemical Formulae 13 and 14,
L3 is a direct bond; a substituted or unsubstituted arylene group having 6 to 60 carbon atoms; or a substituted or unsubstituted heteroarylene group having 2 to 60 carbon atoms;
R28 to R31, Rd and Re are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aromatic hydrocarbon ring having 6 to 60 carbon atoms or a substituted or unsubstituted heteroring having 2 to 60 carbon atoms, r and s are an integer of 0 to 7, and when r is 2 or greater, Rds are the same as or different from each other, when s is 2 or greater, Res are the same as or different from each other, e is an integer of 0 to 4, g and h are each an integer of 0 to 7, f is an integer of 0 to 2, and when e is 2 or greater, R28s are the same as or different from each other, when f is an integer of 2, R29s are the same as or different from each other, when g is 2 or greater, R30s are the same as or different from each other, and when h is 2 or greater, R31s are the same as or different from each other;
Ar3 is a substituted or unsubstituted aryl group having 6 to 60 carbon atoms; or a substituted or unsubstituted heteroaryl group having 2 to 60 carbon atoms;
Ar4 is a substituted or unsubstituted aryl group having 6 to 60 carbon atoms; or a substituted or unsubstituted heteroaryl group having 2 to 60 carbon atoms; and
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms.

Further embodiments are disclosed in the dependent claims.

### [Advantageous Effects]

A heterocyclic compound according to one embodiment of the present application can be used as a material of an organic material layer of an organic light emitting device. The heterocyclic compound can be used as a material of a hole injection layer, a hole transfer layer, a light emitting layer, an electron transfer layer, an electron injection layer, a charge generation layer and the like in an organic light emitting device. Particularly, the heterocyclic compound represented by any one of the Chemical Formulae 1-2 to 1-4 or the heterocyclic compound represented by any one of the compounds listed above can be used as a material of a light emitting layer of an organic light emitting device. In addition, using the heterocyclic compound represented by any one of the Chemical Formulae 1-2 to 1-4 or the heterocyclic compound represented by any one of the compounds listed above in an organic light emitting device is capable of lowering a driving voltage of the device, enhancing light efficiency, and enhancing lifetime properties of the device by thermal stability of the compound.

### [Description of Drawings]

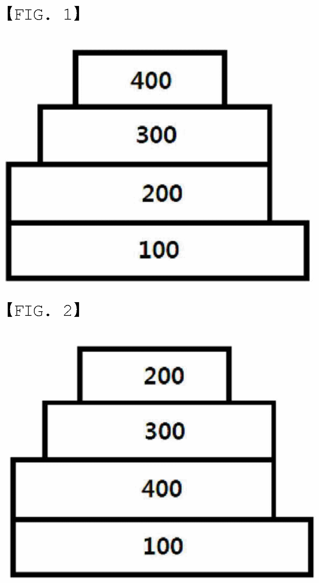

FIG. 1 to FIG. 3 are diagrams each schematically illustrating a lamination structure of an organic light emitting device according to one embodiment of the present application.

### [Mode for Disclosure]

Hereinafter, the present application will be described in detail.

One embodiment of the present application provides a heterocyclic compound represented by any one of the following Chemical Formulae 1-2 to 1-4: 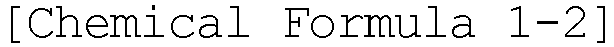 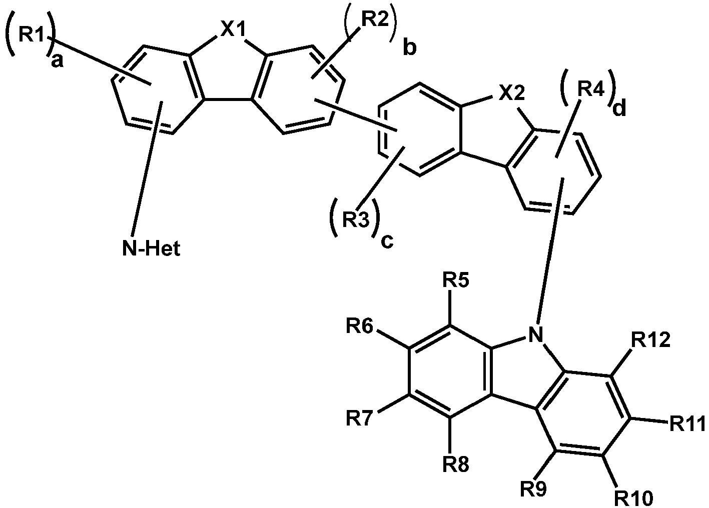 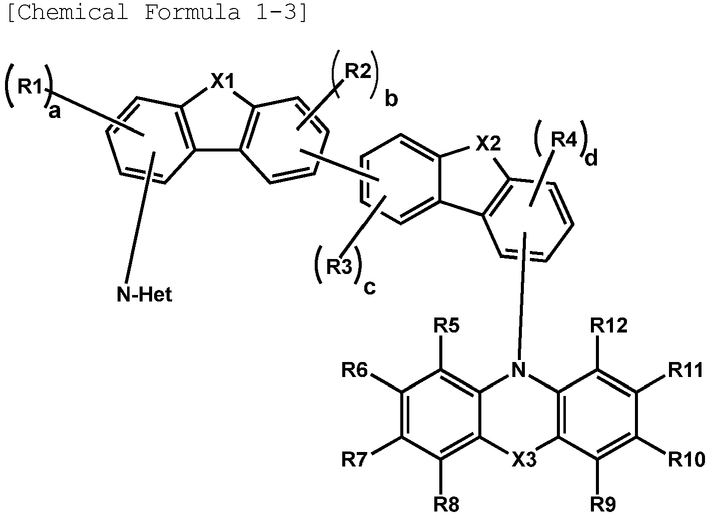 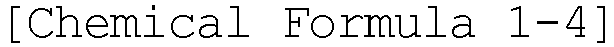 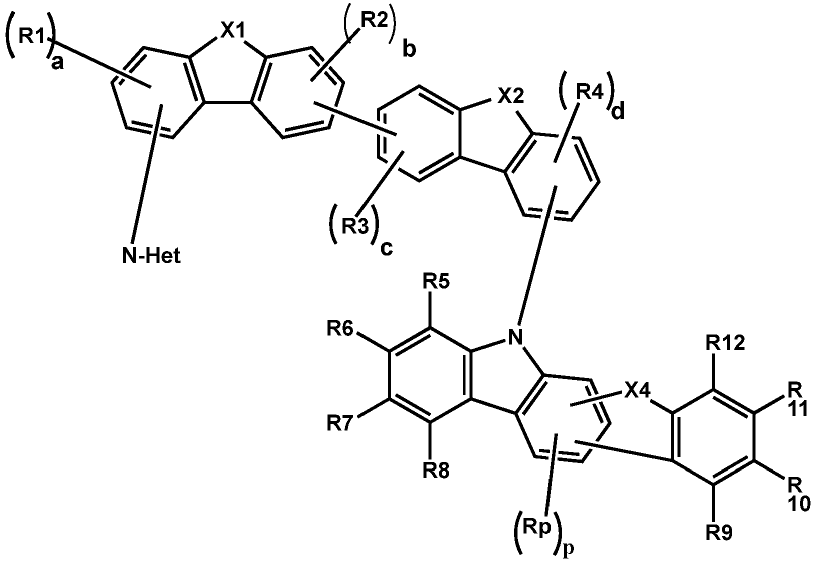 in Chemical Formulae 1-2 to 1-4,
X1 and X2 are the same as or different from each other, and each independently O; or S;
R1 to R4 are the same as or different from each other, and each independently hydrogen; deuterium; a substituted or unsubstituted aryl group having 6 to 60 carbon atoms; or a substituted or unsubstituted heteroaryl group having 6 to 60 carbon atoms, a to d are each an integer of 0 to 3, and when a to d are each 2 or greater, substituents in the parentheses are the same as each other;
N-Het is a group represented by any one of the following Chemical Formulae 2 to 4;
X3 and X4 are each independently O; S; CRaRb; or NRc, Ra and Rb are the same as or different from each other and each independently a substituted or unsubstituted alkyl group having 1 to 10 carbon atoms; or a substituted or unsubstituted aryl group having 6 to 20 carbon atoms, and Rc is a substituted or unsubstituted aryl group having 6 to 20 carbon atoms;
R5 to R12 and Rp are the same as or different from each other, and each independently hydrogen; deuterium; a halogen group; a cyano group; a substituted or unsubstituted aryl group having 6 to 20 carbon atoms; or a substituted or unsubstituted heteroaryl group having 2 to 20 carbon atoms;
p is an integer of 0 to 2, and when p is 2, substituents in the parentheses are the same as each other; 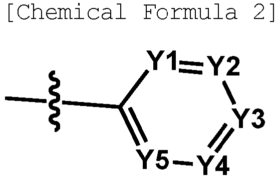 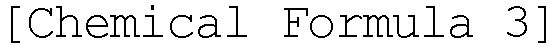 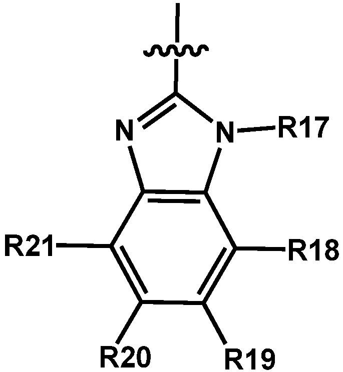 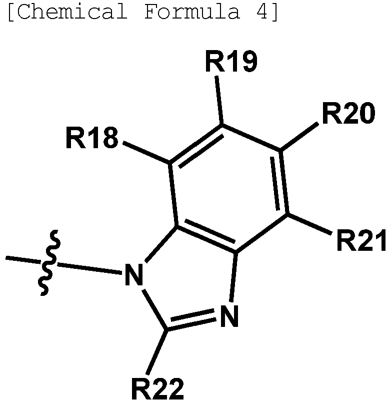 in Chemical Formulae 2 to 4,
   Y1 is CR12' or N, Y2 is CR13' or N, Y3 is CR14' or N, Y4 is CR15' or N, Y5 is CR16' or N, and at least one of Y1 to Y5 is N; and
   R12' to R16' and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring; 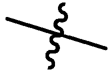
   is a site linked to any one of the following Chemical Formulae 1-2 to 1-4; and
   No. 3 position of 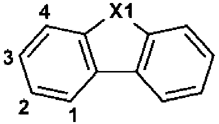 is substituted with N-Het and No. 3 position of 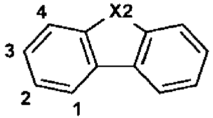 is substituted with any one of 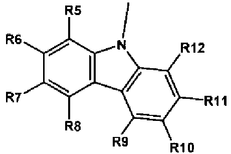 of Chemical Formula 1-2, 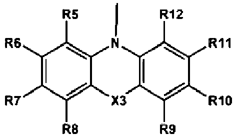 of Chemical Formula 1-3 and 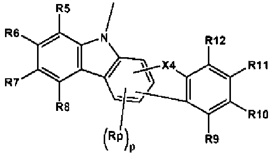 of Chemical Formula 1-4.

The compound represented by any one of Chemical Formulae 1-2 to 1-4 is substituted with carbazole or amine having a strong ability to push electrons and thereby has a high HOMO (highest occupied molecular orbital) level, and accordingly, is capable of helping with hole transfer when used as a material, particularly a dopant material, of a light emitting layer of an organic light emitting device.

In addition, the compound represented by any one of Chemical Formulae 1-2 to 1-4 has a structure in which LUMO (lowest unoccupied molecular orbital) is expanded and is thereby capable of enhancing electron mobility and stabilizing electrons, and as a result, device efficiency and lifetime may be enhanced when using the compound represented by any one of Chemical Formulae 1-2 to 1-4 in an organic light emitting device.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 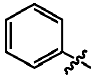 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

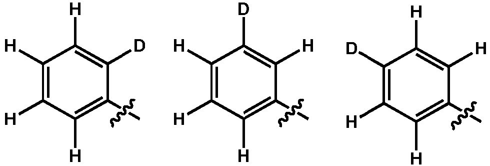

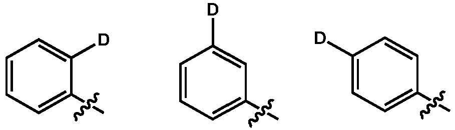

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

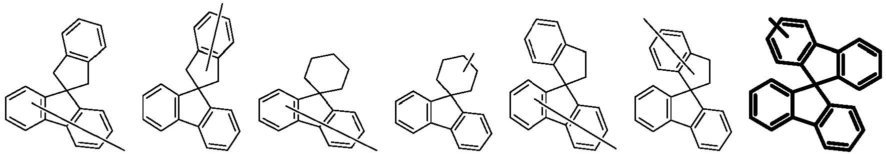

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c] [1,2,5]thiadiazolyl group, 5,10-dihydrobenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto. In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

The heterocyclic compound according to one embodiment of the present application is represented by any one of the Chemical Formulae 1-2 to 1-4. More specifically, by having a core structure and structural properties of the substituents as above, the heterocyclic compound represented by any one of the Chemical Formulae 1-2 to 1-4 may be used as a material of an organic material layer of an organic light emitting device.

In one embodiment of the present application, the heterocyclic compound represented by any one of the Chemical Formulae 1-2 to 1-4 may have a deuterium content of greater than 10% and less than or equal to 100%.

In one embodiment of the present application, X1 and X2 of any one of the Chemical Formulae 1-2 to 1-4 are the same as or different from each other, and each independently O; or S.

In another embodiment, X1 and X2 are O.

In another embodiment, X1 and X2 are S.

In another embodiment, X1 is O and X2 is S in any one of the Chemical Formulae 1-2 to 1-4.

In another embodiment, X1 is S and X2 is O in any one of the Chemical Formulae 1-2 to 1-4.

In one embodiment of the present application, R1 to R4 in any one of the Chemical Formulae 1-2 to 1-4 are the same as or different from each other, and may be each independently hydrogen; deuterium; a substituted or unsubstituted aryl group having 6 to 60 carbon atoms; or a substituted or unsubstituted heteroaryl group having 6 to 60 carbon atoms.

In another embodiment, R1 to R4 are hydrogen; or deuterium.

In another embodiment, R1 to R4 are hydrogen.

In another embodiment, R1 to R4 are deuterium.

In one embodiment of the present application, a to d of in any one of the Chemical Formulae 1-2 to 1-4 are each an integer of 0 to 3, and when a to d are each 2 or greater, substituents in the parentheses are the same as each other.

In another embodiment, a is 0.

In another embodiment, a is 1.

In another embodiment, a is 2.

In another embodiment, a is 3.

In one embodiment of the present application, when a is 2 or greater, substituents in the parentheses are the same as each other.

In another embodiment, b is 0.

In another embodiment, b is 1.

In another embodiment, b is 2.

In another embodiment, b is 3.

In one embodiment of the present application, when b is 2 or greater, substituents in the parentheses are the same as each other.

In another embodiment, c is 0.

In another embodiment, c is 1.

In another embodiment, c is 2.

In another embodiment, c is 3.

In one embodiment of the present application, when c is 2 or greater, substituents in the parentheses are the same as each other.

In another embodiment, d is 0.

In another embodiment, d is 1.

In another embodiment, d is 2.

In another embodiment, d is 3.

In one embodiment of the present application, when d is 2 or greater, substituents in the parentheses are the same as each other.

In one embodiment of the present application, the heterocyclic compound is represented by any one of the following Chemical Formulae 1-2 to 1-4. 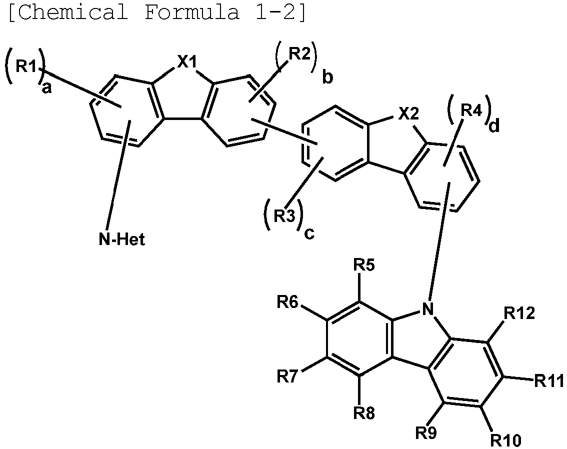 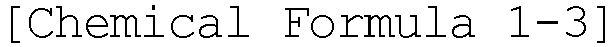 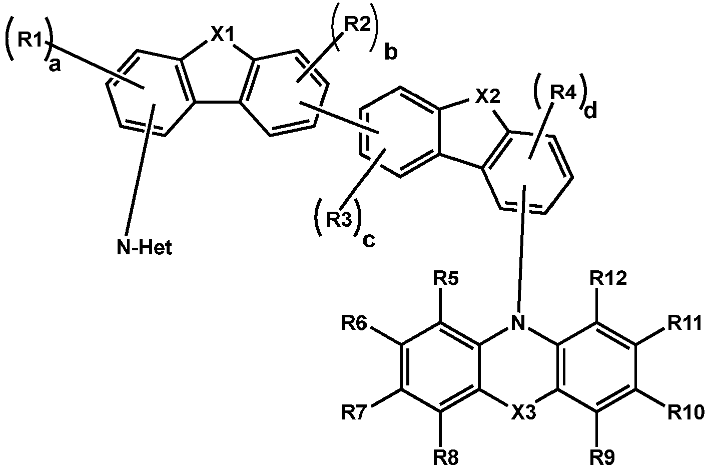 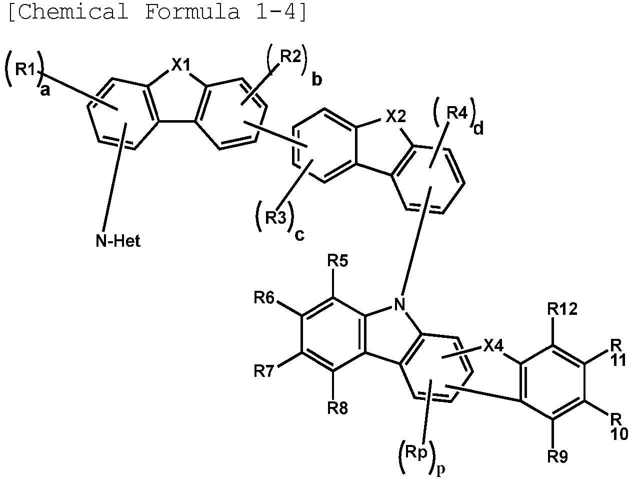 In Chemical Formulae 1-2 to 1-4,
X3 and X4 are each independently O; S; CRaRb; or NRc, Ra and Rb are the same as or different from each other and each independently a substituted or unsubstituted alkyl group having 1 to 10 carbon atoms; or a substituted or unsubstituted aryl group having 6 to 20 carbon atoms, and Rc is a substituted or unsubstituted aryl group having 6 to 20 carbon atoms,
R5 to R12 and Rp are the same as or different from each other, and each independently hydrogen; deuterium; a halogen group; a cyano group; a substituted or unsubstituted aryl group having 6 to 20 carbon atoms; or a substituted or unsubstituted heteroaryl group having 2 to 20 carbon atoms, p is an integer of 0 to 2, and when p is 2, substituents in the parentheses are the same as each other, and
X1, X2, R1 to R4, N-Het and a to d have the same definitions as above.

In one embodiment of the present application, R5 to R12 and Rp are the same as or different from each other, and each independently hydrogen; deuterium; a halogen group; a cyano group; a substituted or unsubstituted aryl group having 6 to 20 carbon atoms; or a substituted or unsubstituted heteroaryl group having 2 to 20 carbon atoms, p is an integer of 0 to 2, and when p is 2, substituents in the parentheses may be the same as each other.

In one embodiment of the present application, R5 to R12 and Rp are the same as or different from each other, and each independently hydrogen; deuterium; a halogen group; a cyano group; an aryl group having 6 to 20 carbon atoms unsubstituted or substituted with deuterium; or a heteroaryl group having 2 to 20 carbon atoms unsubstituted or substituted with deuterium, p is an integer of 0 to 2, and when p is 2, substituents in the parentheses may be the same as each other.

In one embodiment of the present application, R5 to R12 and Rp are the same as or different from each other, and each independently hydrogen; or deuterium, p is an integer of 0 to 2, and when p is 2, substituents in the parentheses may be the same as each other.

In one embodiment of the present application, R5 to R12 and Rp are hydrogen.

In one embodiment of the present application, R5 to R12 and Rp are deuterium.

In one embodiment of the present application, X3 and X4 are each independently O; S; CRaRb; or NRc, Ra and Rb are the same as or different from each other and may be each independently a substituted or unsubstituted alkyl group having 1 to 10 carbon atoms; or a substituted or unsubstituted aryl group having 6 to 20 carbon atoms, and Rc may be a substituted or unsubstituted aryl group having 6 to 20 carbon atoms.

In one embodiment of the present application, X3 and X4 are O; S; CRaRb; or NRc, and Rc may be a phenyl group.

In one embodiment of the present application, X3 is O.

In one embodiment of the present application, X3 is S.

In one embodiment of the present application, X3 is CRaRb, and Ra and Rb are the same as or different from each other and may be each independently a substituted or unsubstituted alkyl group having 1 to 10 carbon atoms; or a substituted or unsubstituted aryl group having 6 to 20 carbon atoms.

In one embodiment of the present application, Ra and Rb are the same as or different from each other, and each independently a substituted or unsubstituted methyl group; or a substituted or unsubstituted phenyl group.

In one embodiment of the present application, X3 is NRc, and Rc is a phenyl group.

In one embodiment of the present application, X4 is O.

In one embodiment of the present application, X4 is S.

In one embodiment of the present application, X4 is CRaRb, and Ra and Rb are the same as or different from each other and may be each independently a substituted or unsubstituted alkyl group having 1 to 10 carbon atoms; or a substituted or unsubstituted aryl group having 6 to 20 carbon atoms.

In one embodiment of the present application, Ra and Rb are the same as or different from each other, and each independently a substituted or unsubstituted methyl group; or a substituted or unsubstituted phenyl group.

In one embodiment of the present application, X4 is NRc, and Rc is a phenyl group.

N-Het is a group represented by any one of the following Chemical Formulae 2 to 4. 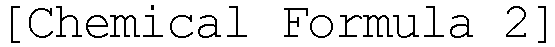 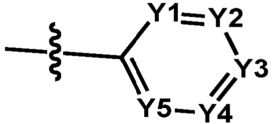 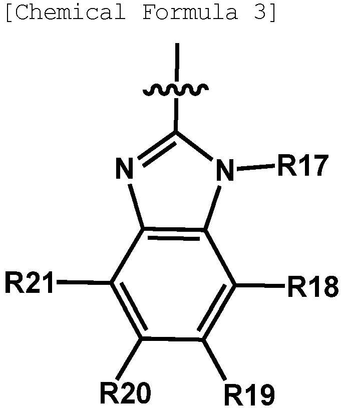 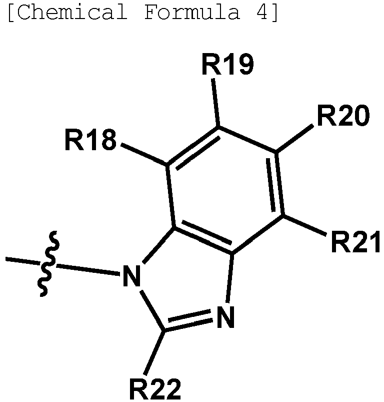 In Chemical Formulae 2 to 4,
Y1 is CR12 or N, Y2 is CR13 or N, Y3 is CR14 or N, Y4 is CR15 or N, Y5 is CR16 or N, and at least one of Y1 to Y5 is N,
R12 to R16 and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring, and 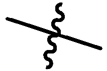 is a site linked to any one of Chemical Formulae 1-2 to 1-4.

In one embodiment of the present application, Chemical Formula 2 may be represented by one of the following Chemical Formulae 5 and 6.

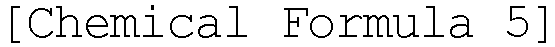

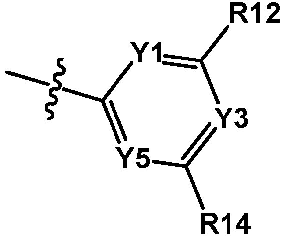

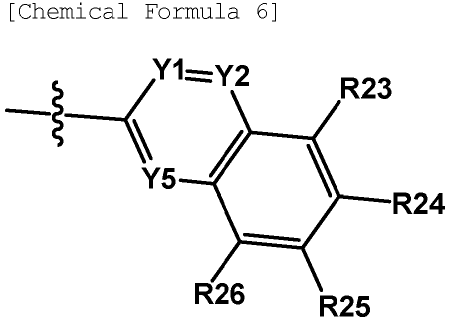

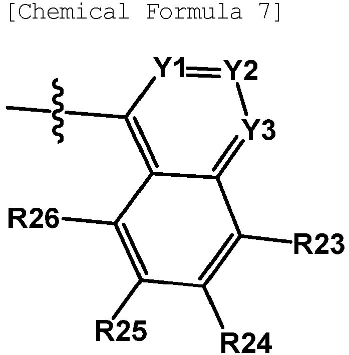

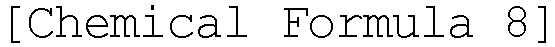

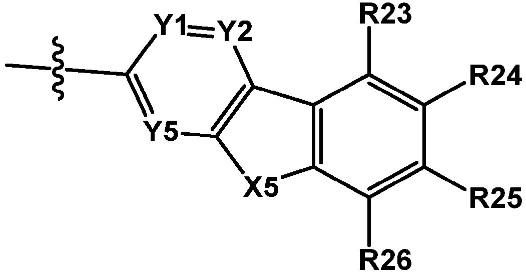

In Chemical Formula 5, one or more of Y1, Y3 and Y5 are N, and the rest have the same definitions as in Chemical Formula 5 and Chemical Formula 8,
in Chemical Formula 6, one or more of Y1, Y2 and Y5 are N, and the rest have the same definitions as in Chemical Formula 5,
in Chemical Formula 7, one or more of Y1 to Y3 are N, and the rest have the same definitions as in Chemical Formula 5,
in Chemical Formula 8, one or more of Y1, Y2 and Y5 are N, and the rest have the same definitions as in Chemical Formula 5,
X5 is O; or S,
R12, R14 and R23 to R26 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 6 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring.

In one embodiment of the present application, Chemical Formula 5 may be selected from among structural formulae of the following Group A.

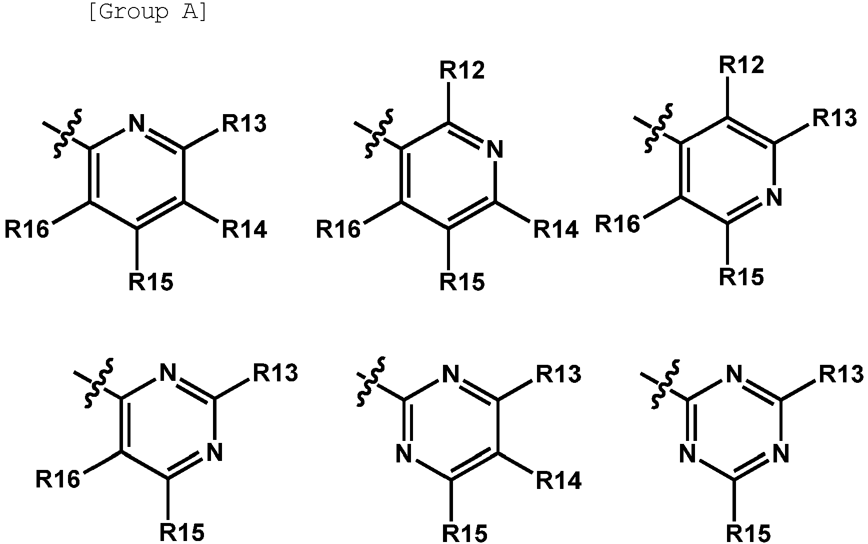

Substituents of the structural formulae of Group A have the same definitions as in Chemical Formula 5.

In one embodiment of the present application, Chemical Formula 6 may be represented by the following Chemical Formula 9.

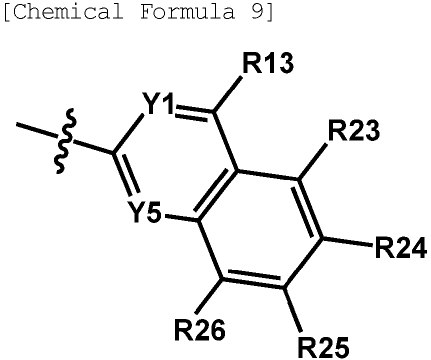

Substituents of Chemical Formula 9 have the same definitions as in Chemical Formula 6.

In one embodiment of the present application, Chemical Formula 7 may be represented by the following Chemical Formula 10.

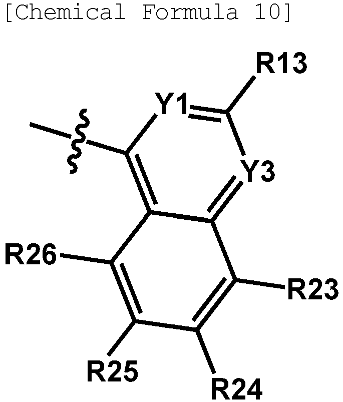

Substituents of Chemical Formula 10 have the same definitions as in Chemical Formula 7.

In one embodiment of the present application, Chemical Formula 6 may be represented by the following Chemical Formula 11. 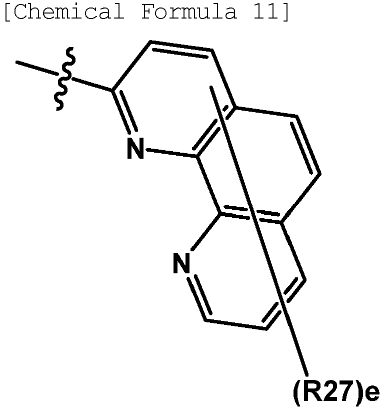 In Chemical Formula 11,
R27s are the same as or different from each other, and selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring, e is an integer of 0 to 7, and when e is 2 or greater, R27s are the same as or different from each other.

In one embodiment of the present application, Chemical Formula 8 may be represented by the following Chemical Formula 12.

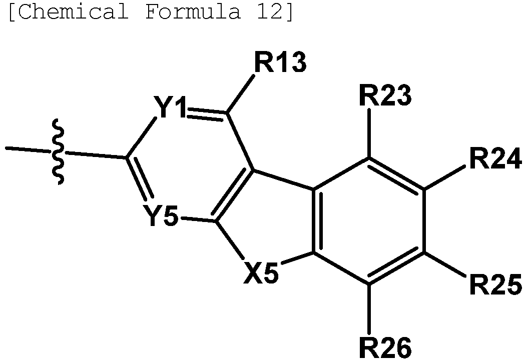

Substituents of Chemical Formula 12 have the same definitions as in Chemical Formula 8.

According to one embodiment of the present application, a bonding position where 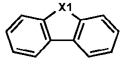 of any one of the Chemical Formulae 1-2 to 1-4 bonds to N-Het and 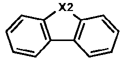 of any one of the Chemical Formulae 1-2 to 1-4 is as shown in the following Group B.

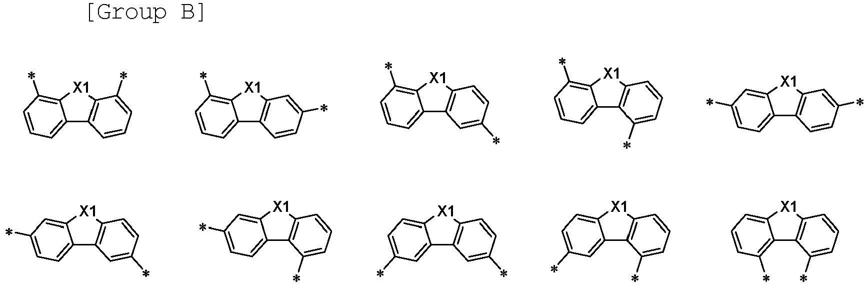

In Group B, * is a position where N-Het or 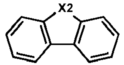 bonds, and X₁, X₂ and N-Het have the same definitions as in any one of the Chemical Formulae 1-2 to 1-4.

According to one embodiment of the present application, a bonding position where of 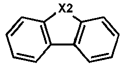 Chemical Formula 1 bonds to 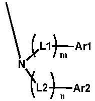 of Chemical Formula 1 and 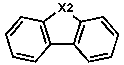 of Chemical Formula 1 is as shown in the following Group C.

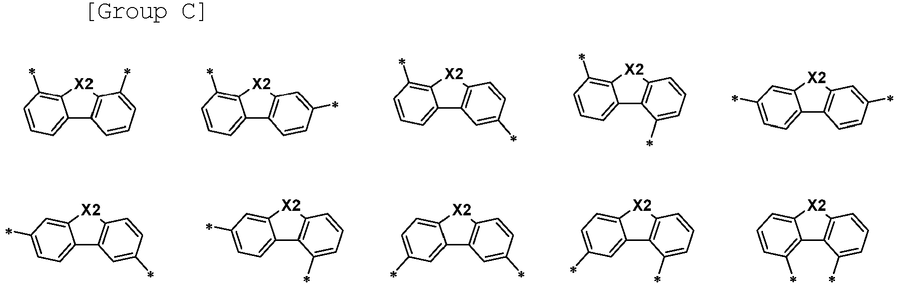

In Group C, * is a position where 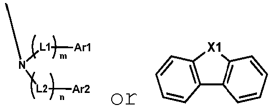 bonds, and X₁, X₂ and 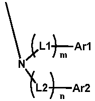 have the same definitions as in Chemical Formula 1.

More specifically, when a No. 3 position of 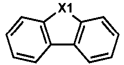 is substituted with N-Het and a No. 3 position of 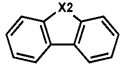 is substituted with 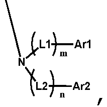 mobility and stability of holes and electrons may be further enhanced, and therefore, a lifetime of an organic light emitting device may be further enhanced when having the above-described bonding positions.

In the present specification, the position of substitution means positions indicated by the numbers in the following Chemical Formulae A and B.

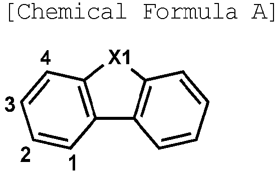

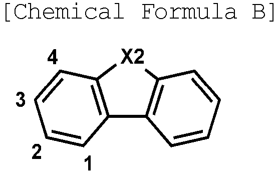

In Chemical Formulae A and B, X₁ and X₂ have the same definitions as in any one of the Chemical Formulae 1-2 to 1-4, and each number means a position substituted with a substituent.

Accordingly, in the following Chemical Formula C, it may be described that the No. 4 position of 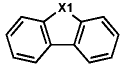 is substituted with a triazine group, and the No. 4 position of 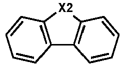 is substituted with a carbazole group.

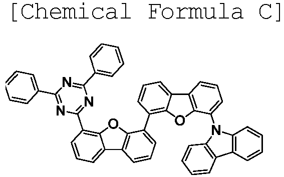

Another embodiment of the present application provides a heterocyclic compound represented by any one of the following compounds:

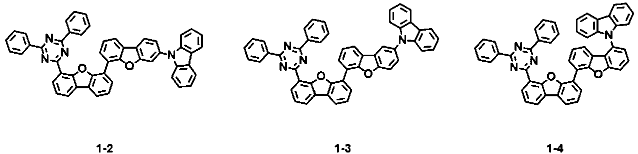

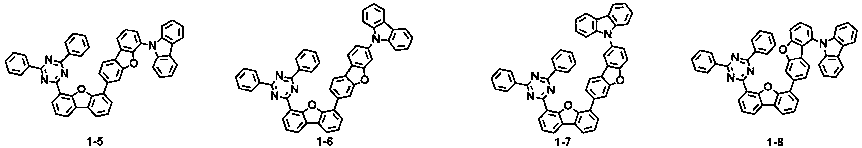

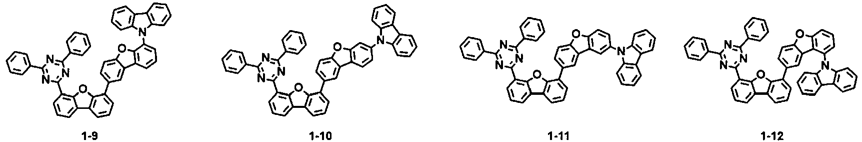

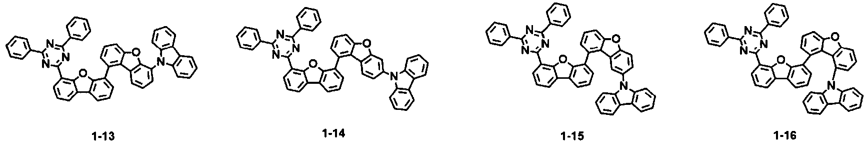

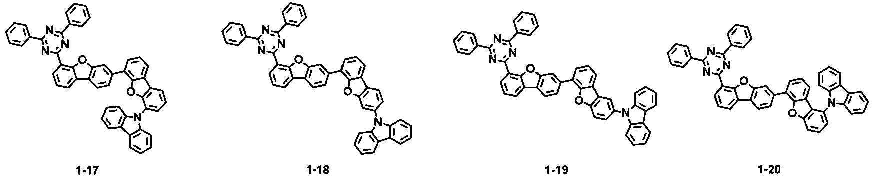

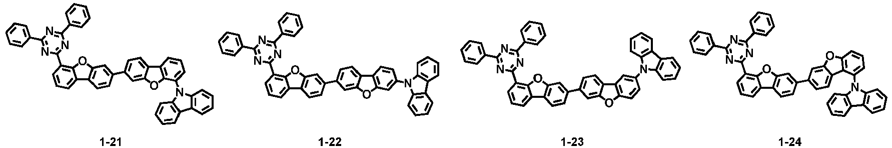

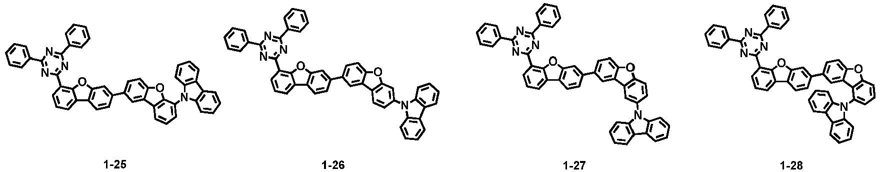

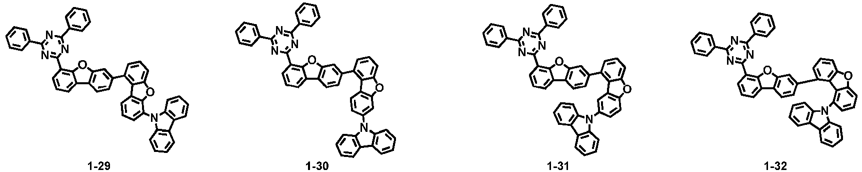

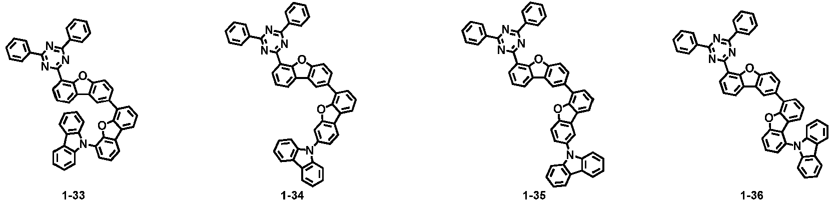

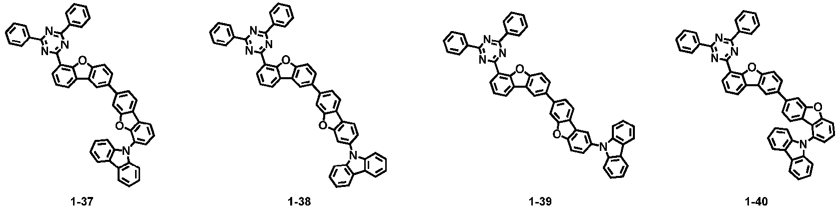

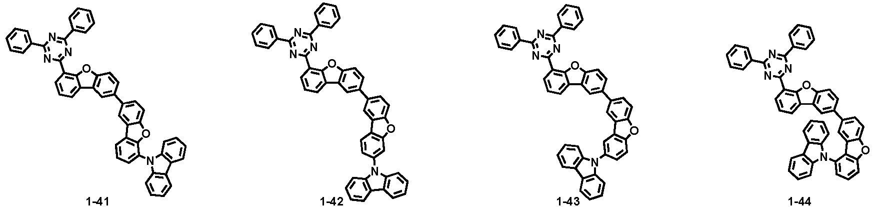

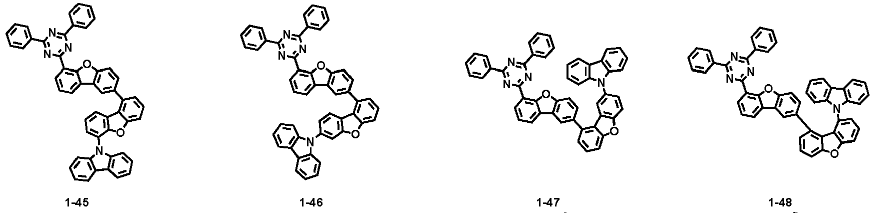

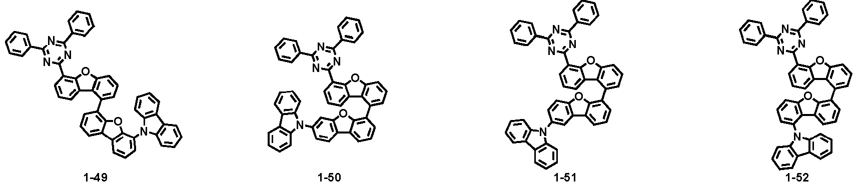

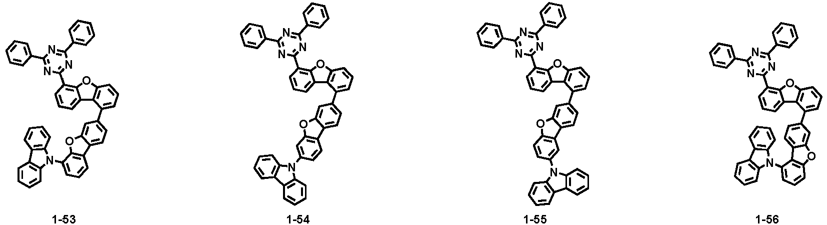

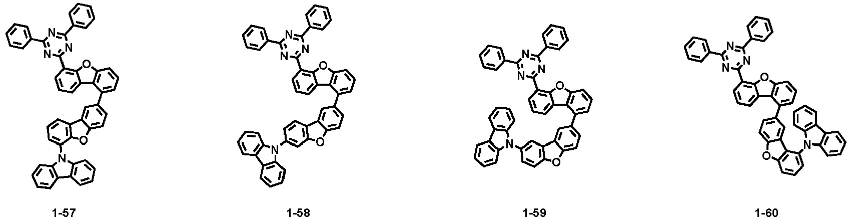

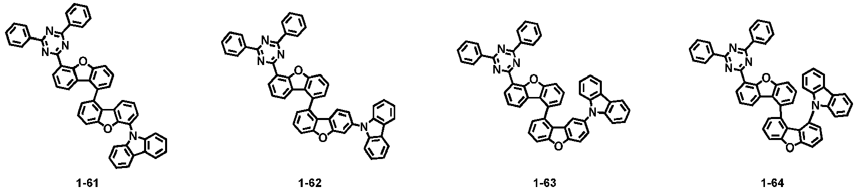

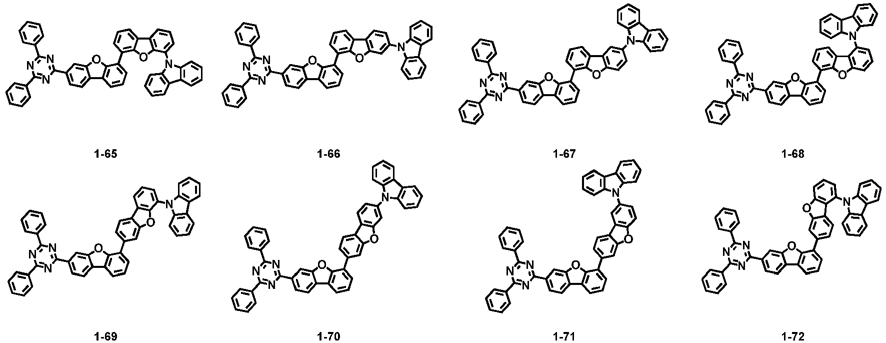

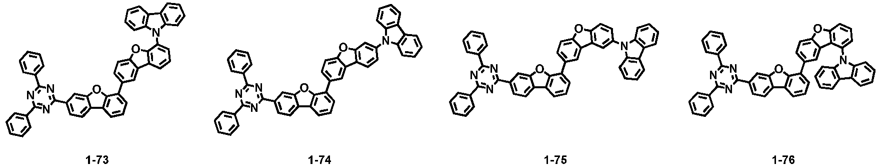

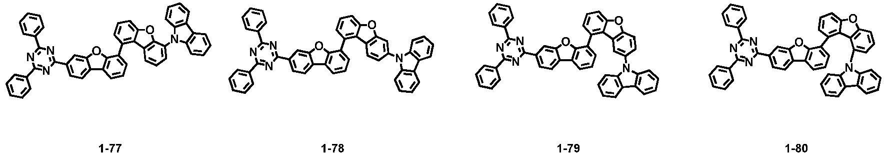

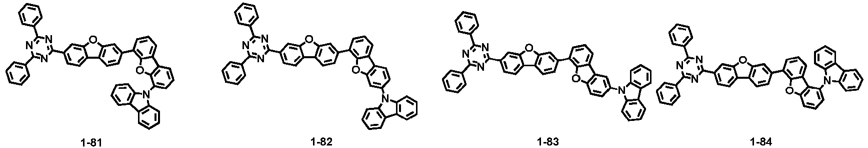

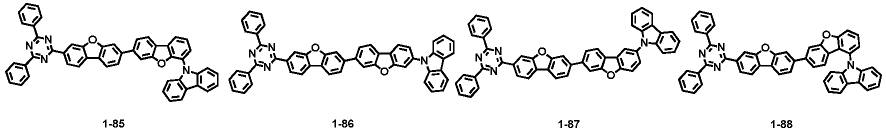

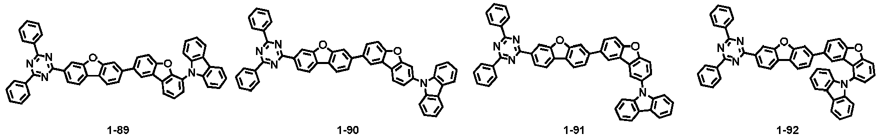

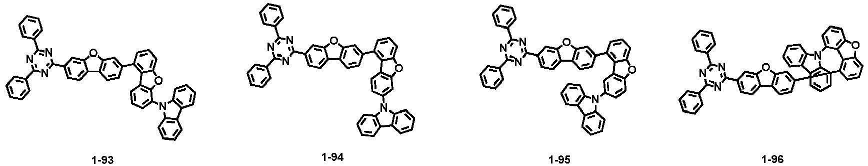

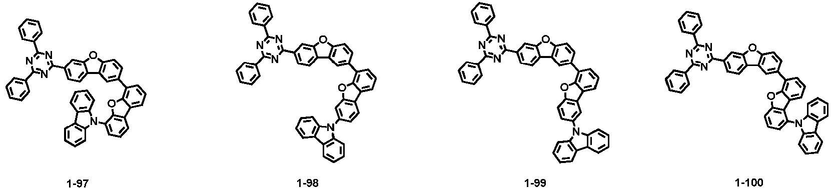

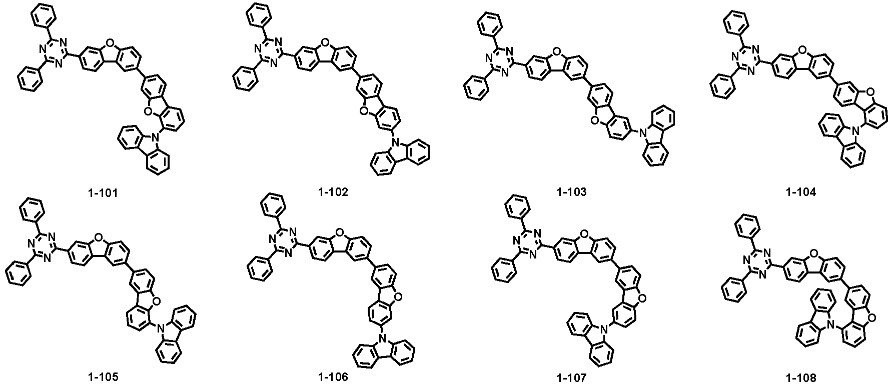

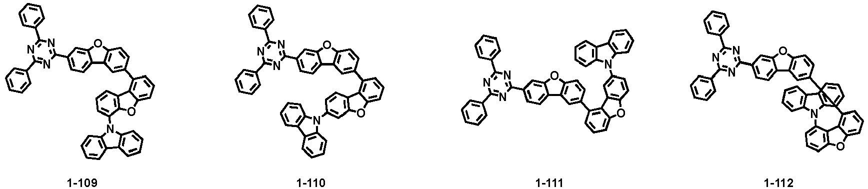

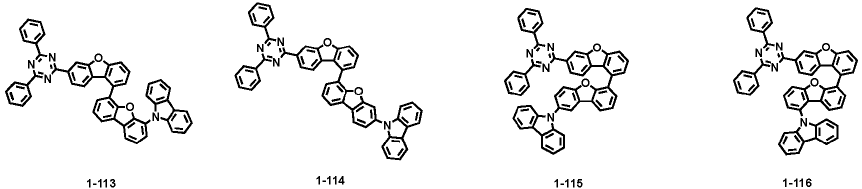

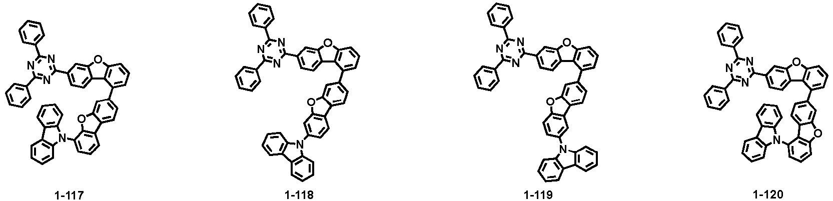

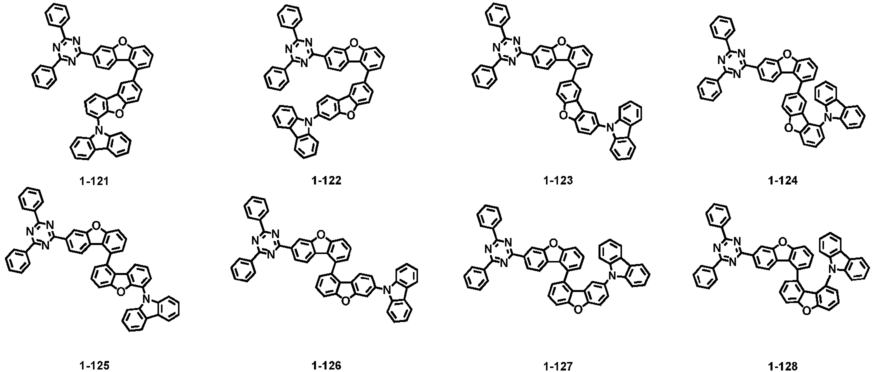

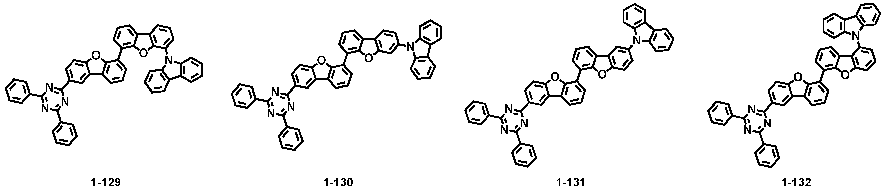

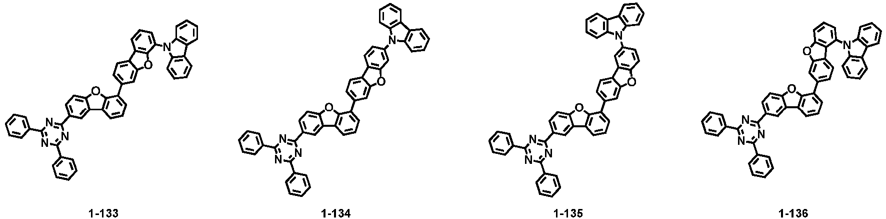

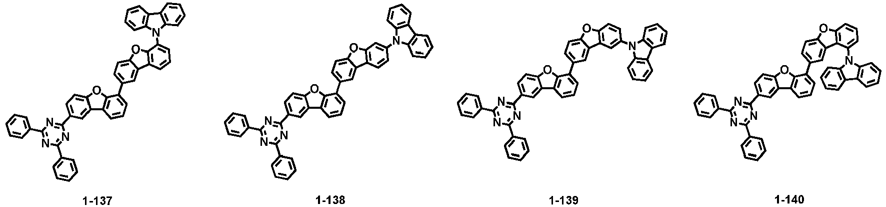

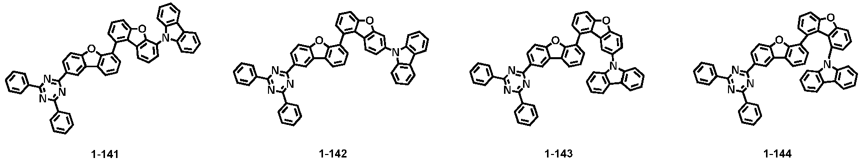

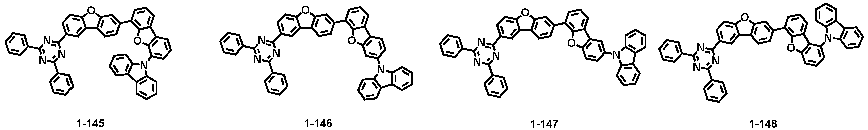

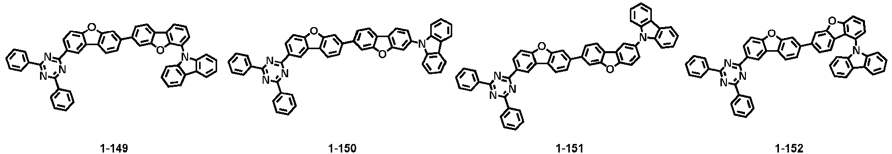

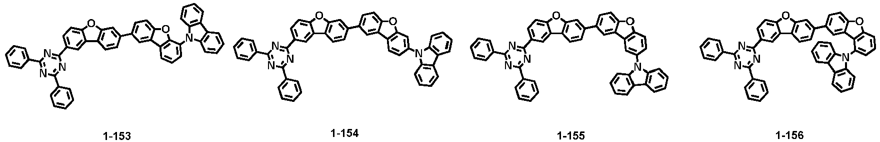

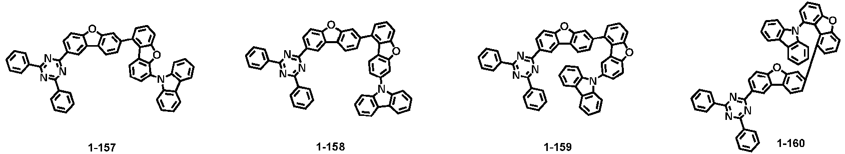

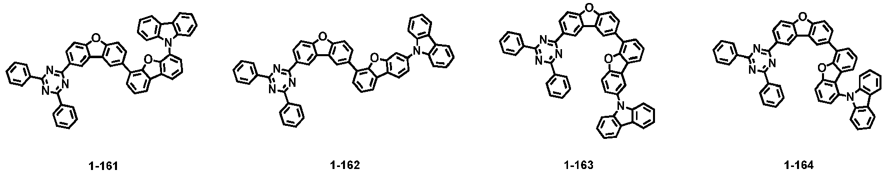

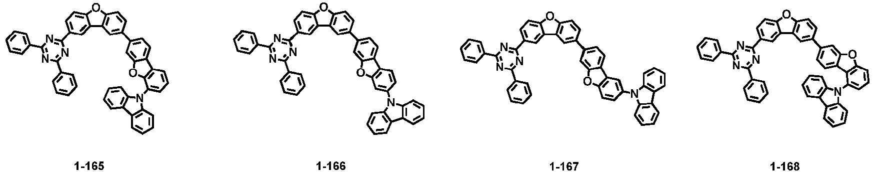

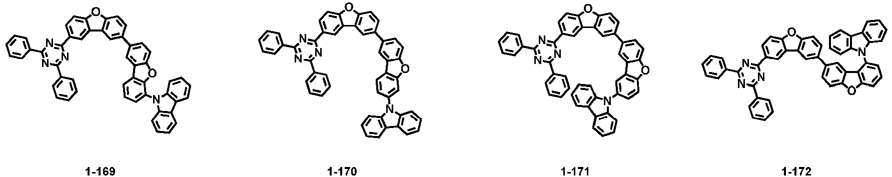

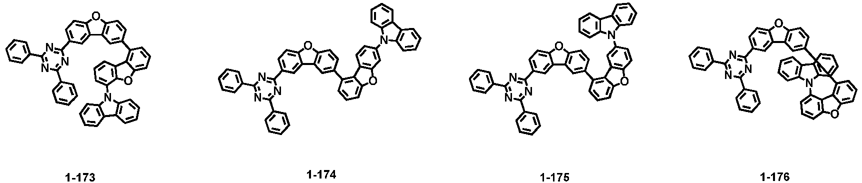

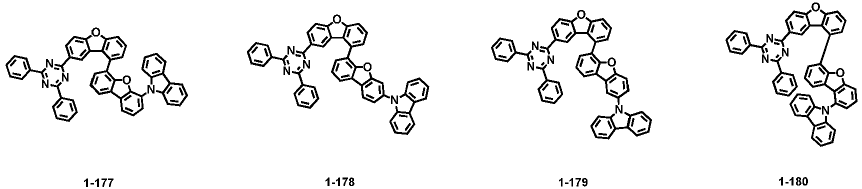

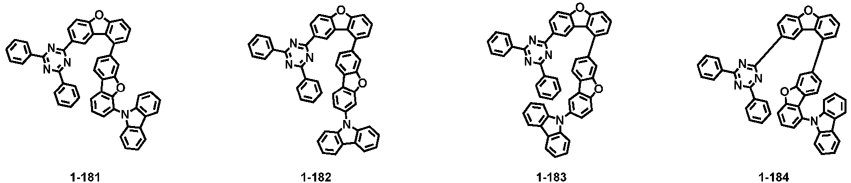

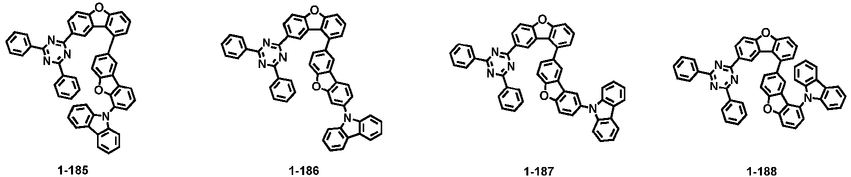

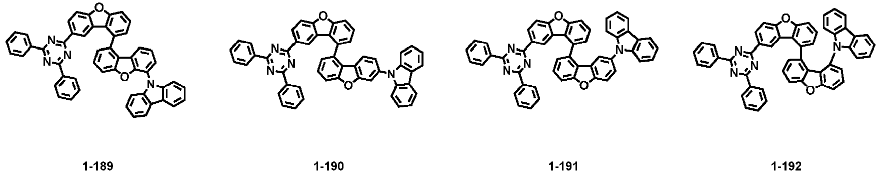

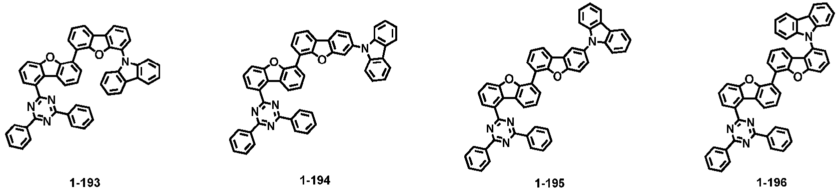

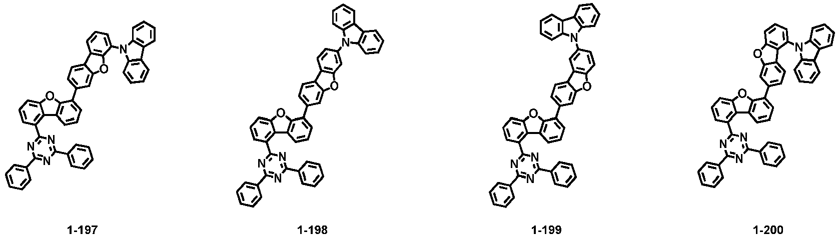

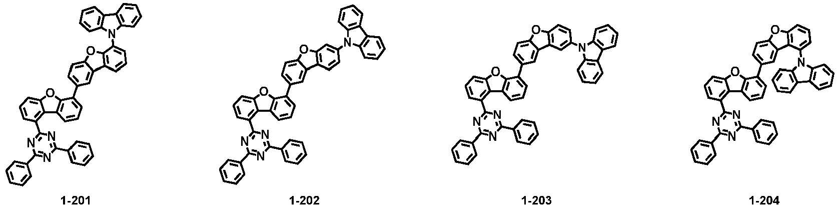

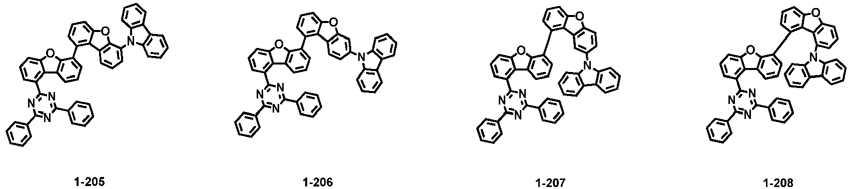

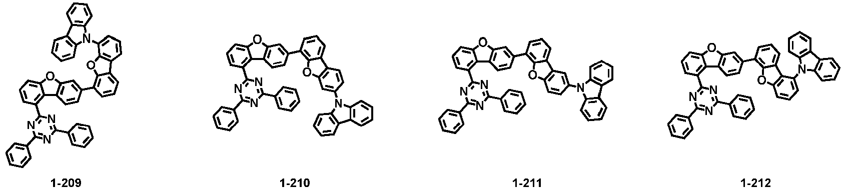

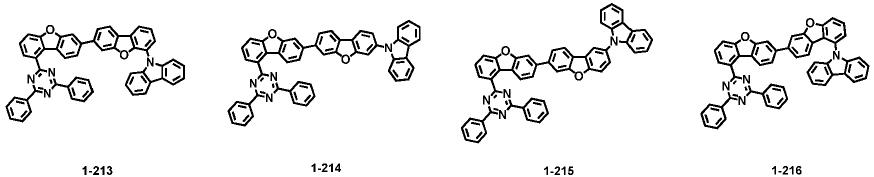

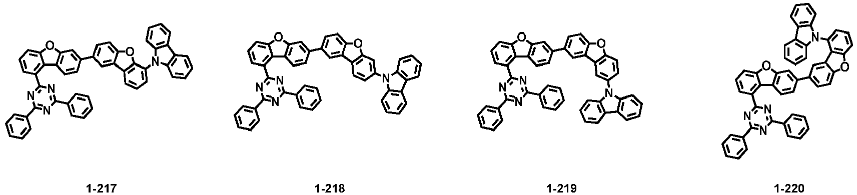

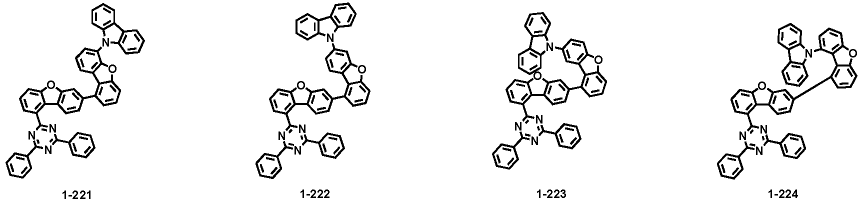

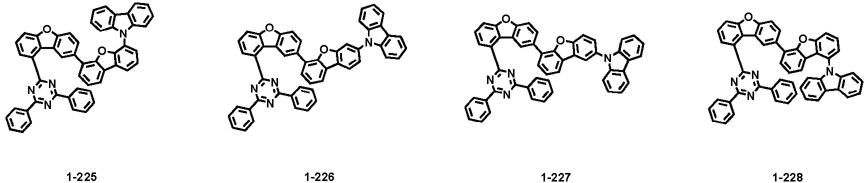

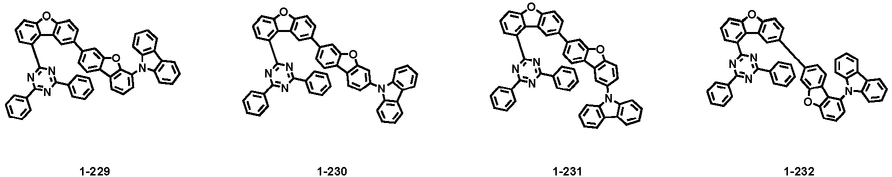

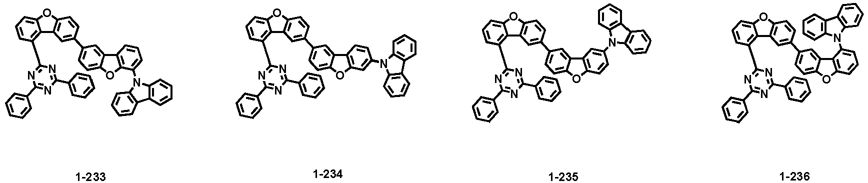

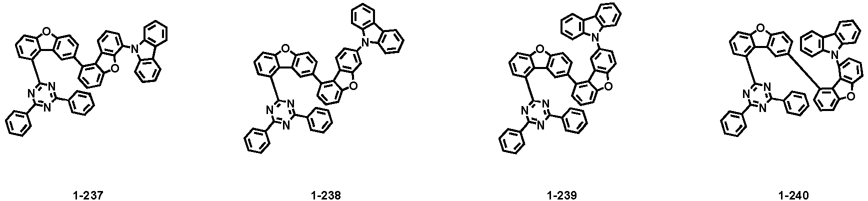

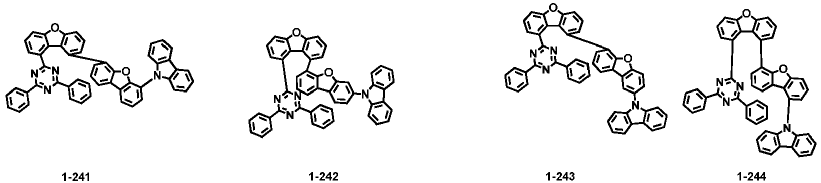

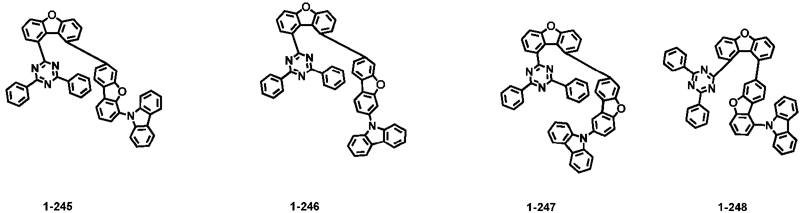

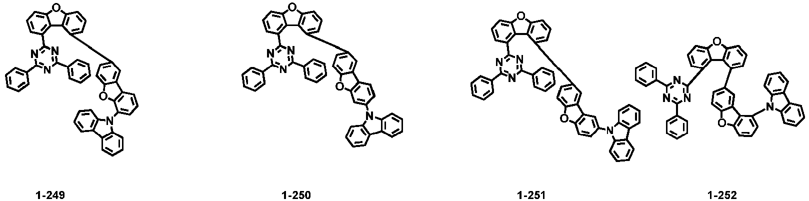

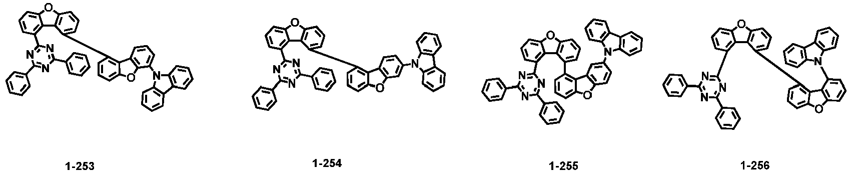

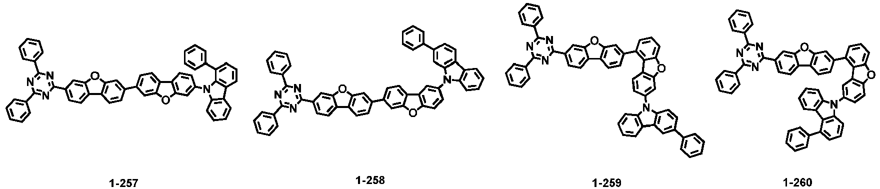

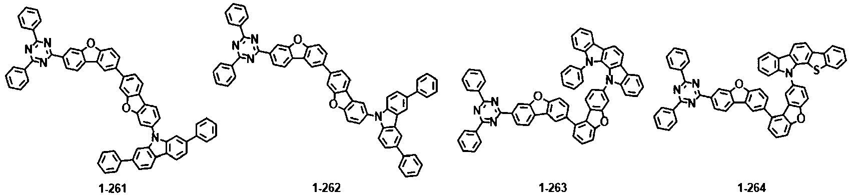

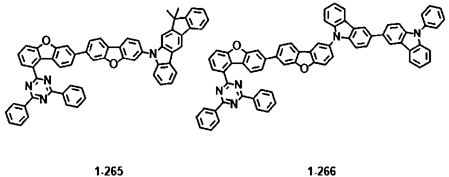

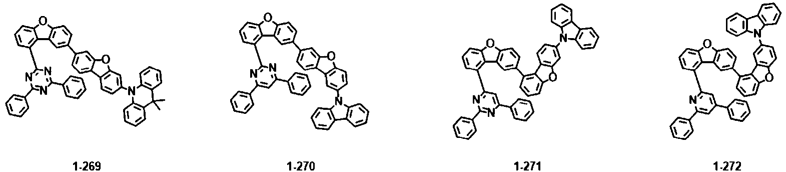

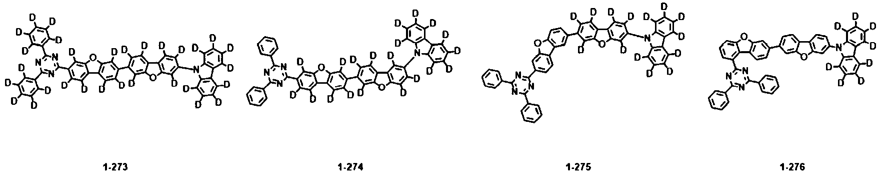

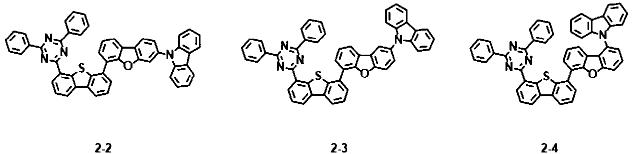

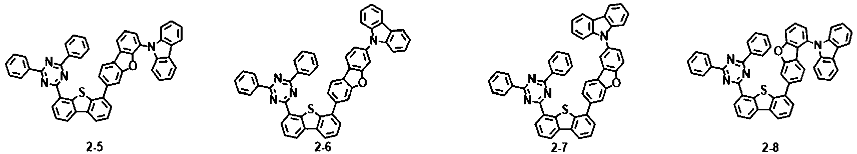

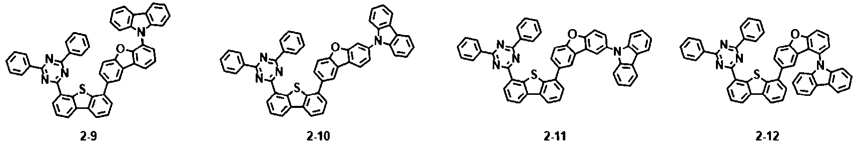

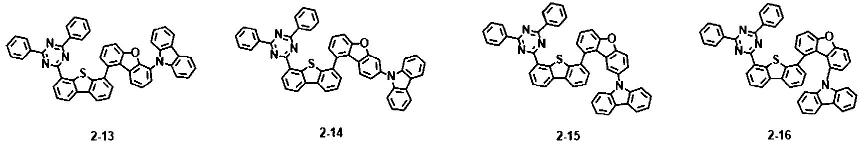

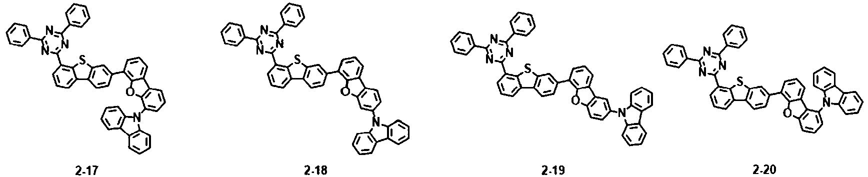

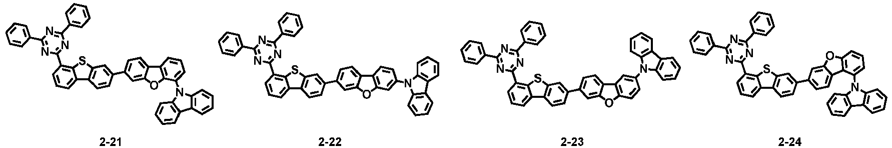

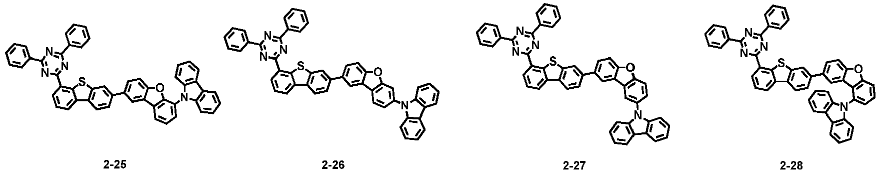

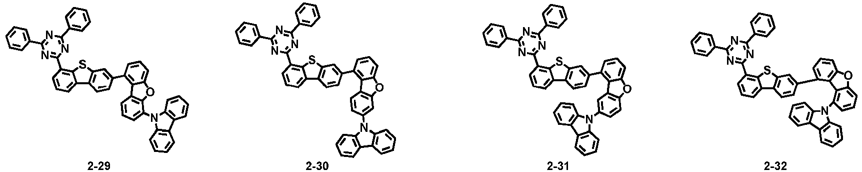

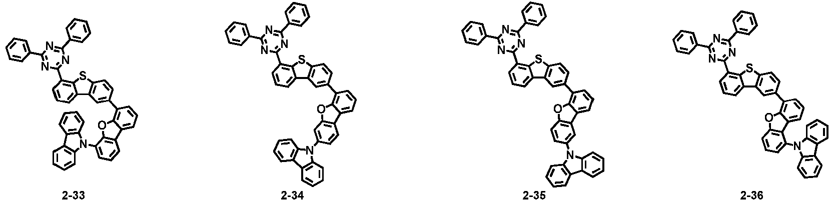

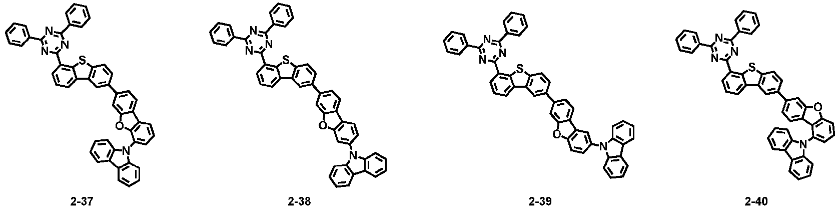

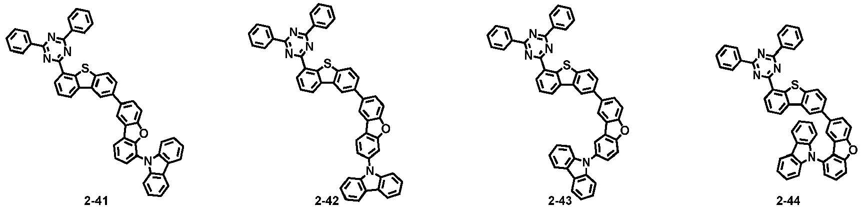

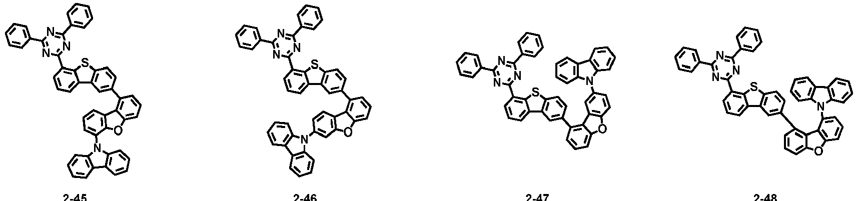

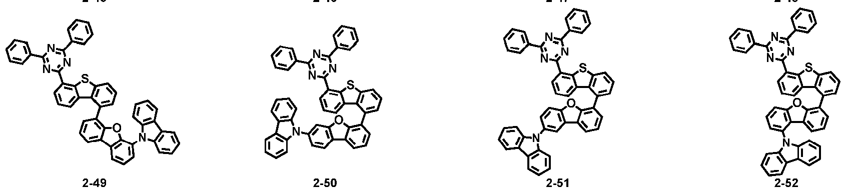

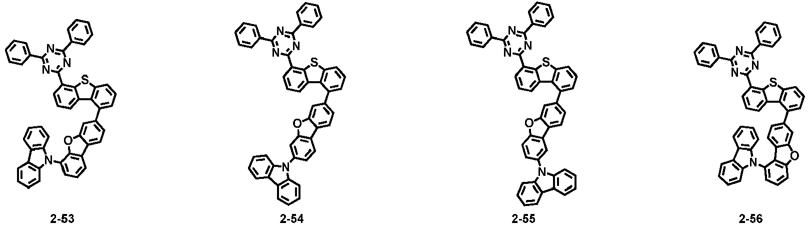

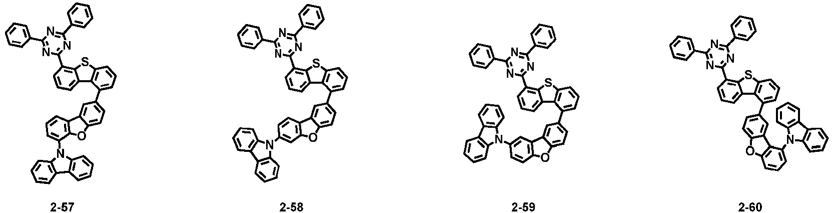

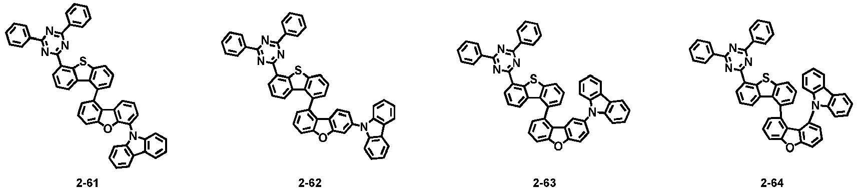

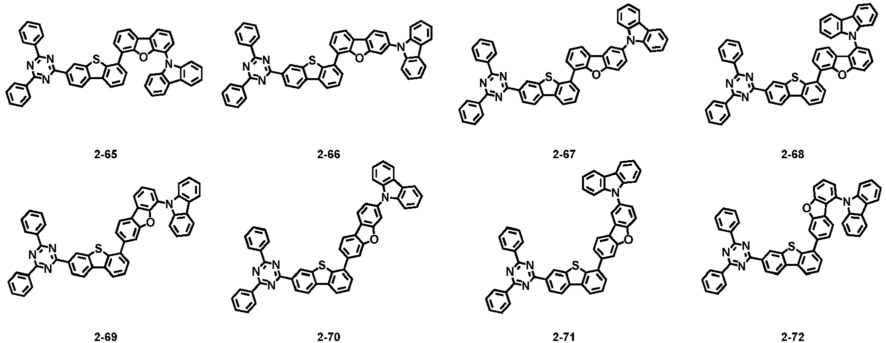

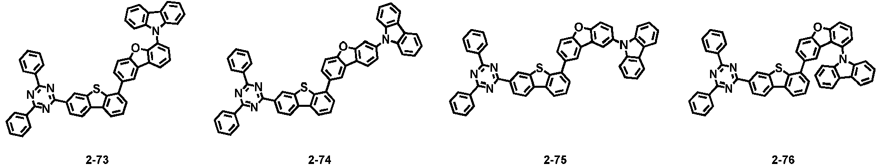

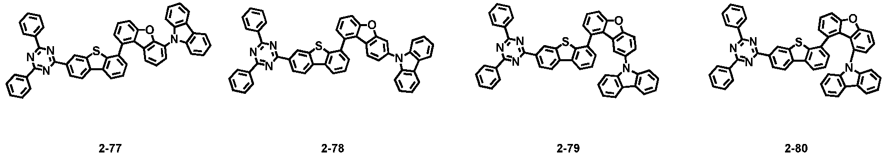

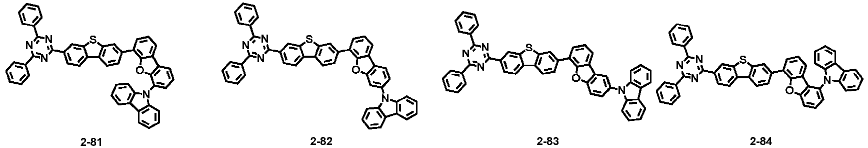

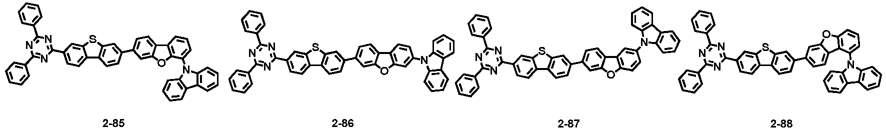

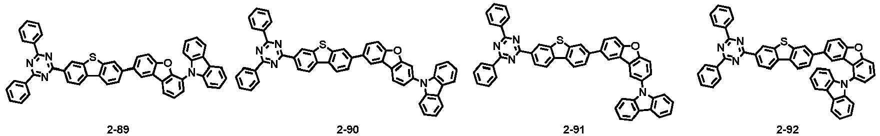

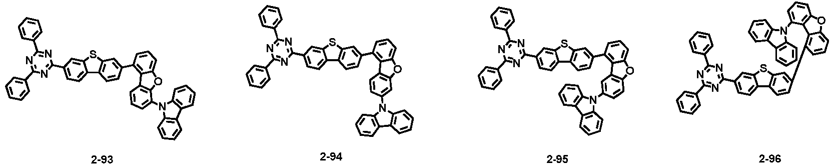

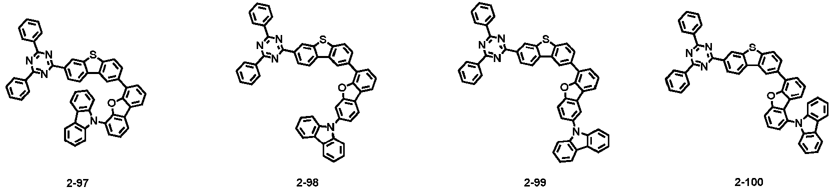

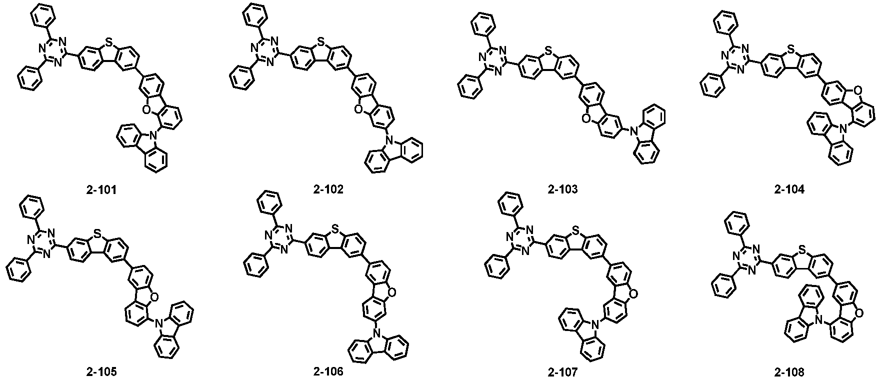

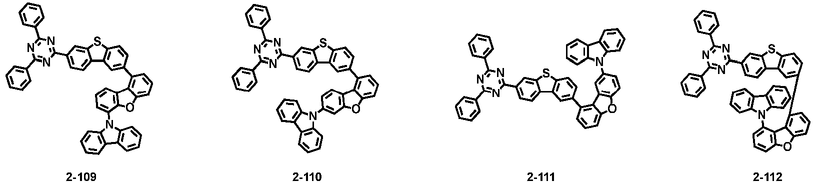

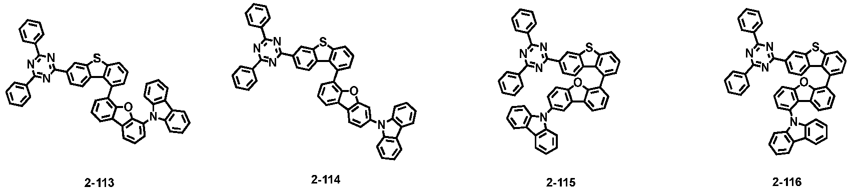

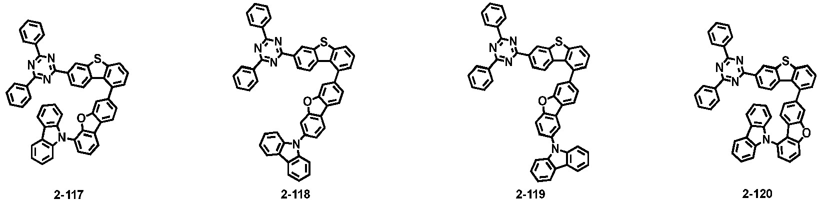

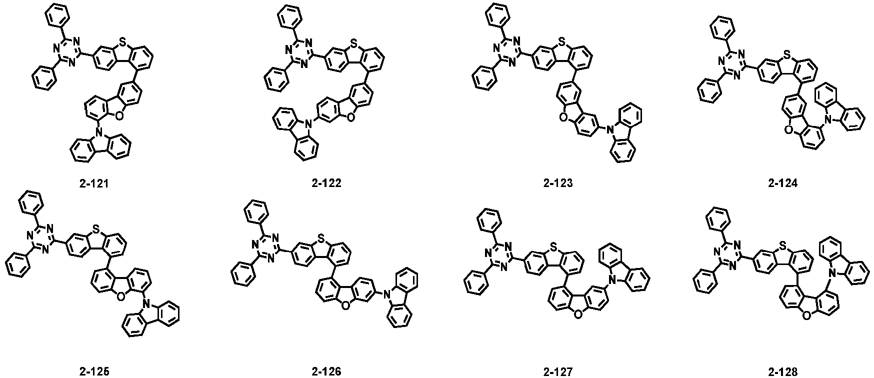

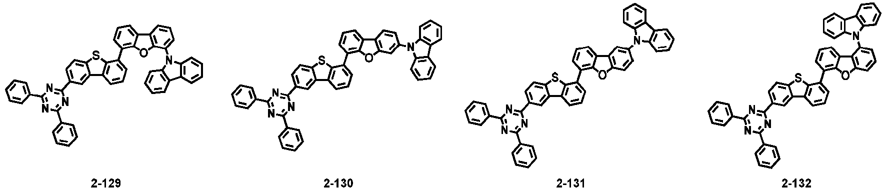

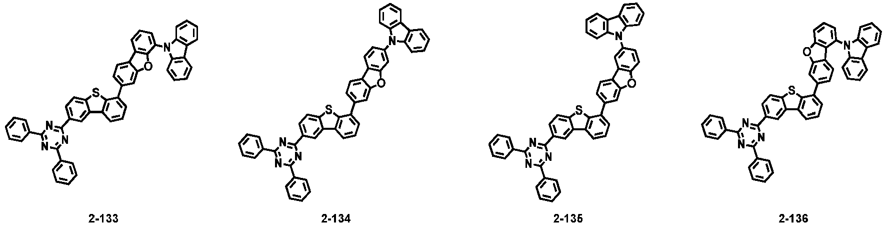

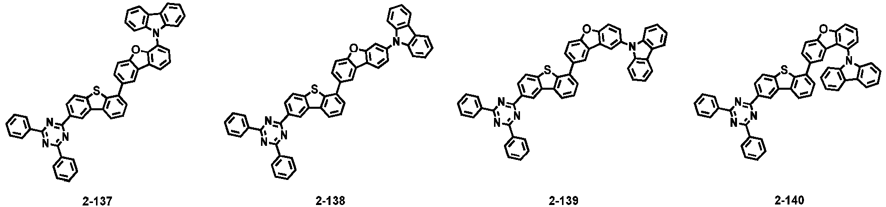

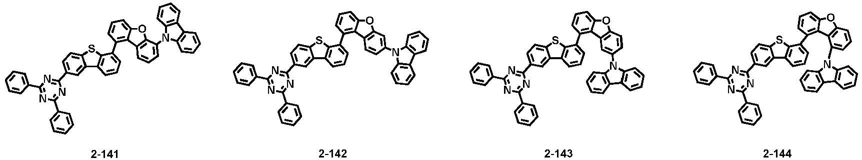

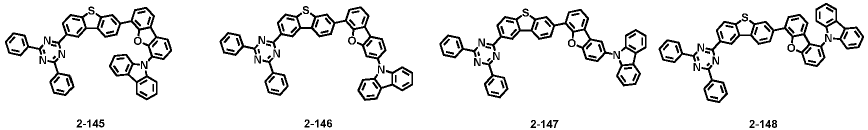

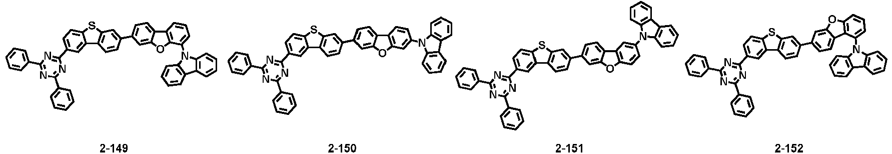

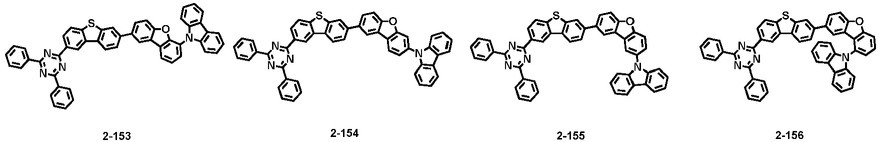

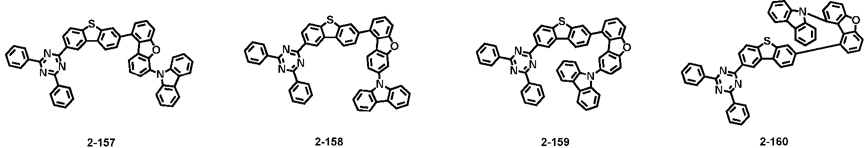

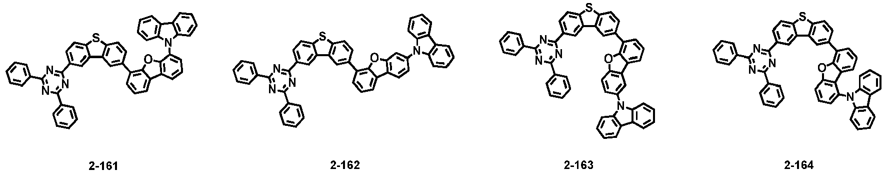

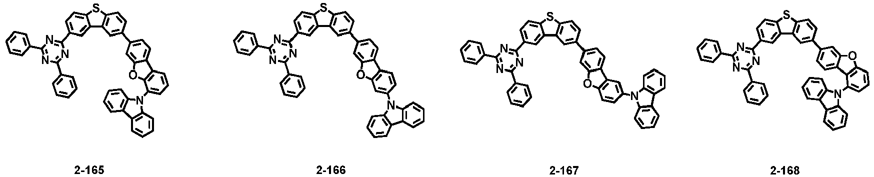

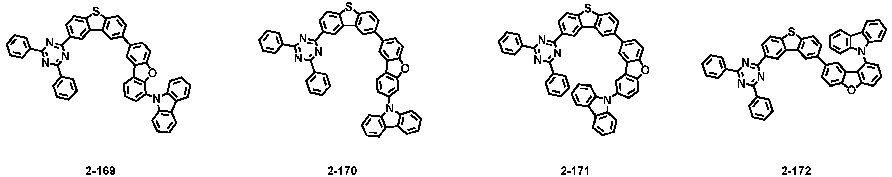

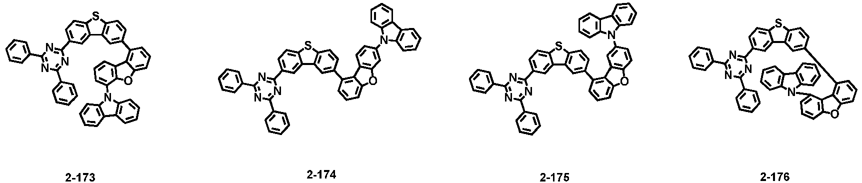

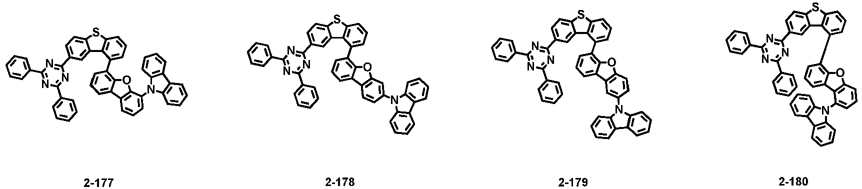

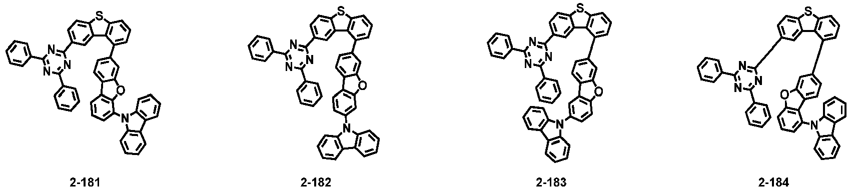

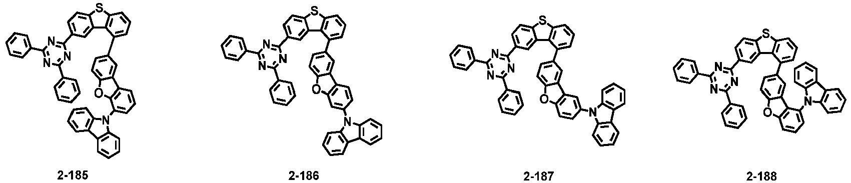

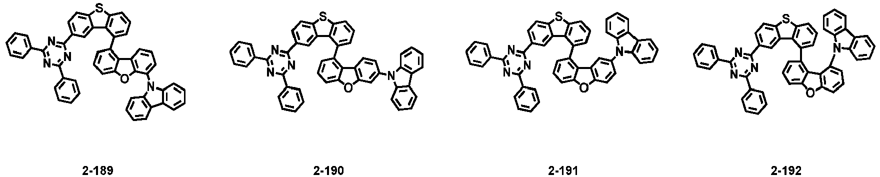

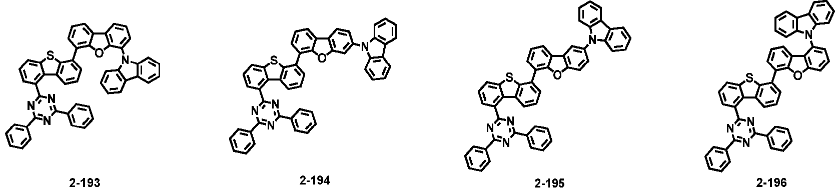

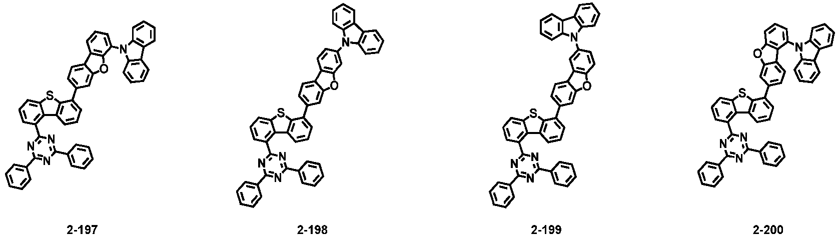

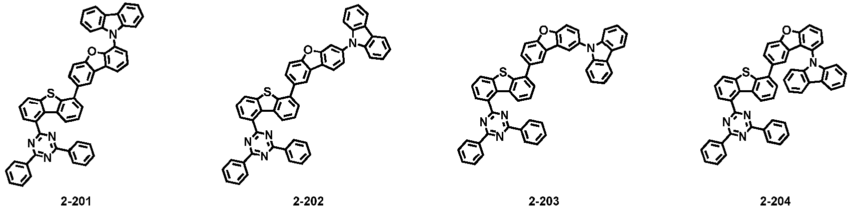

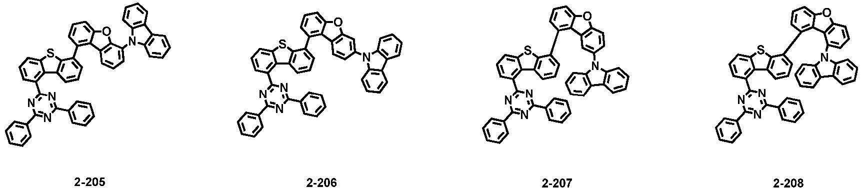

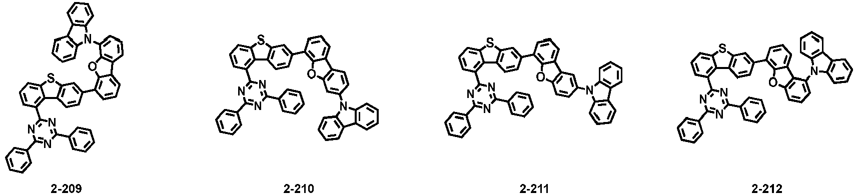

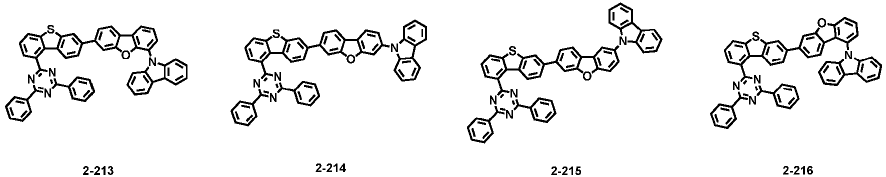

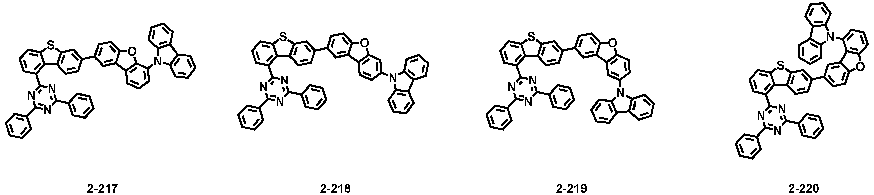

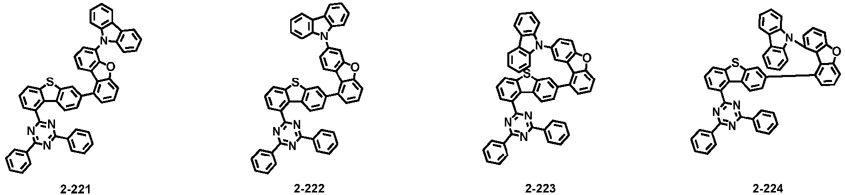

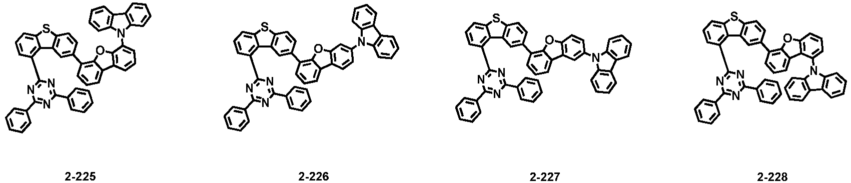

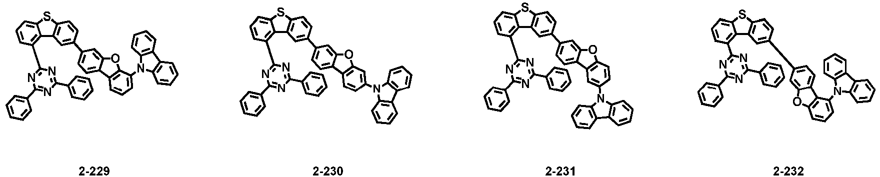

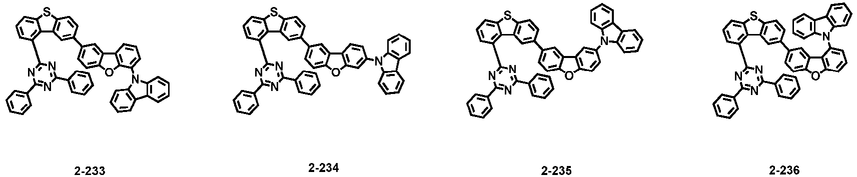

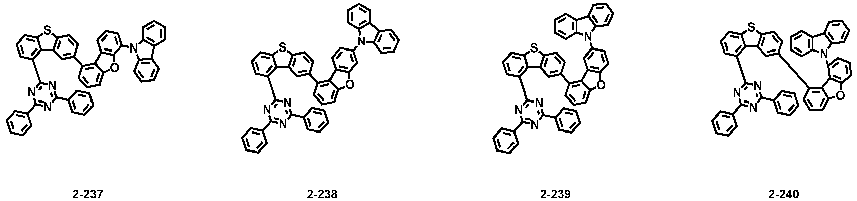

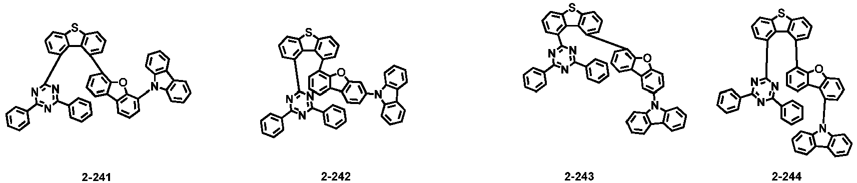

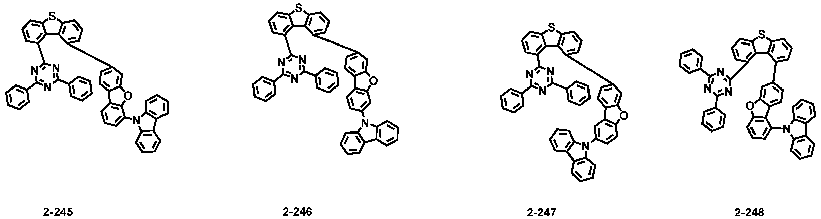

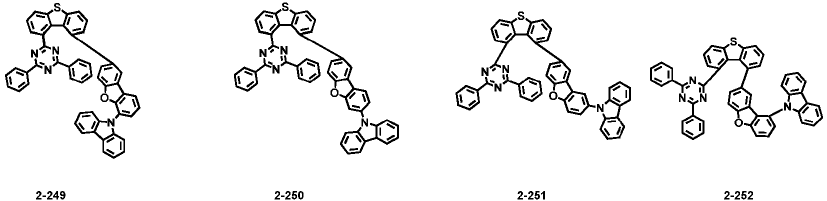

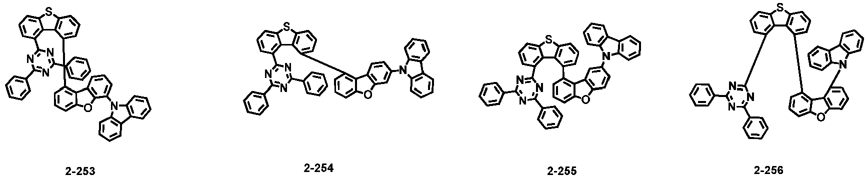

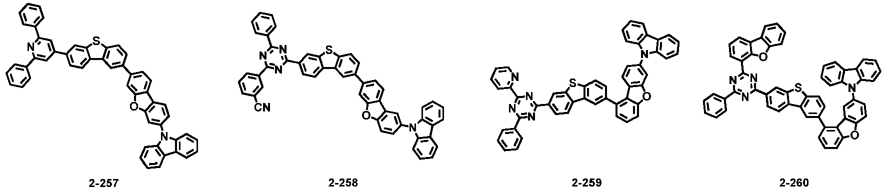

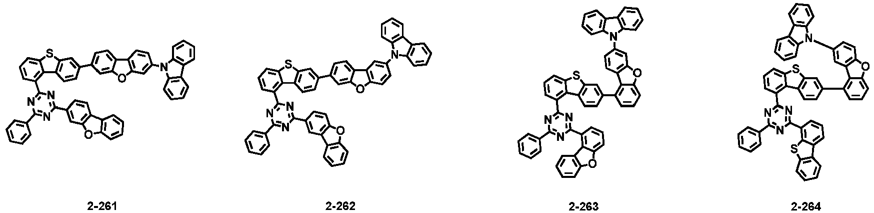

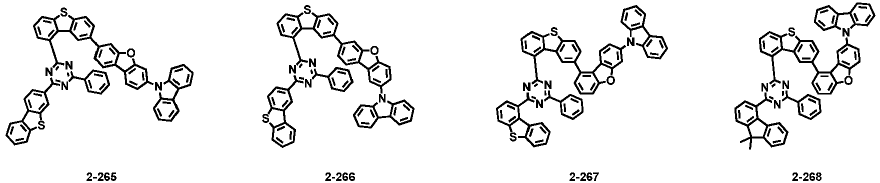

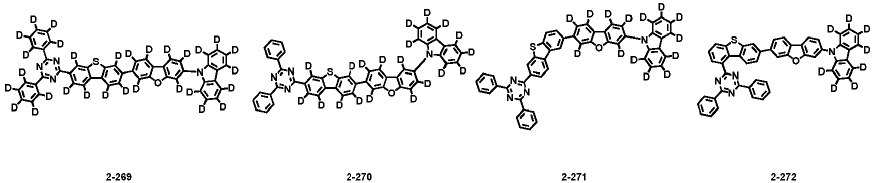

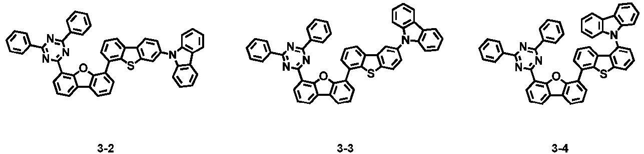

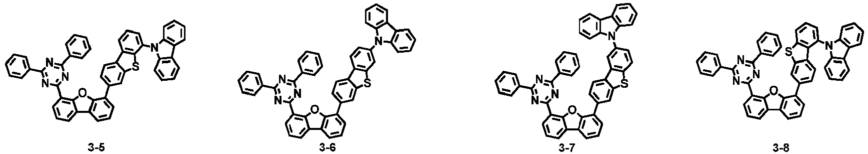

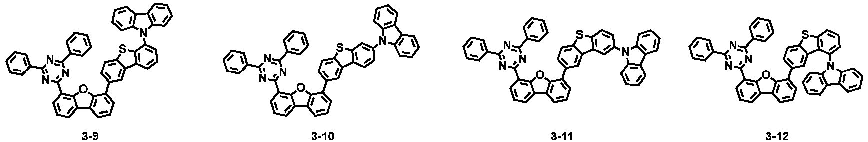

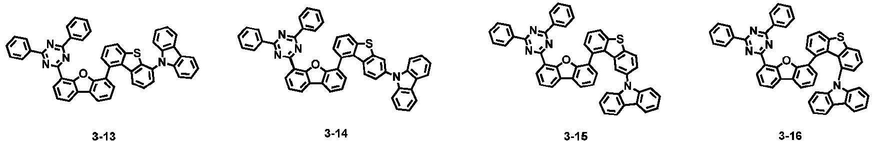

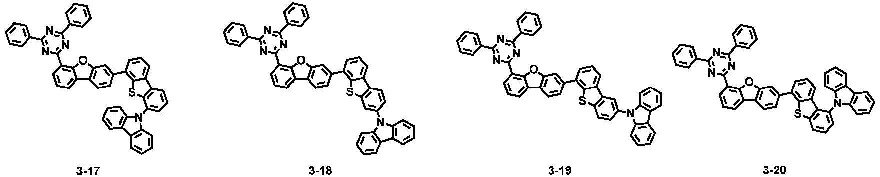

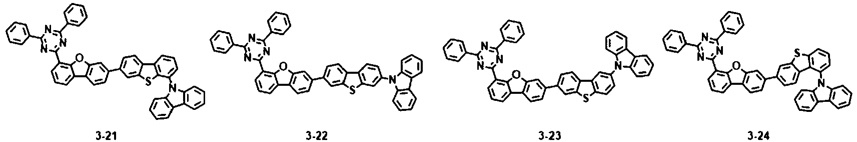

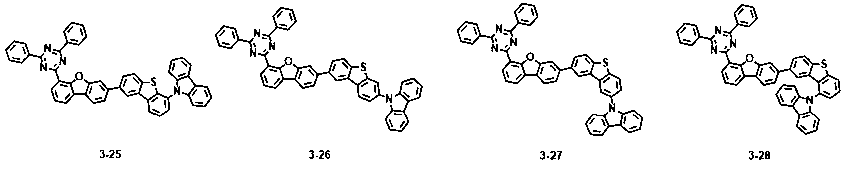

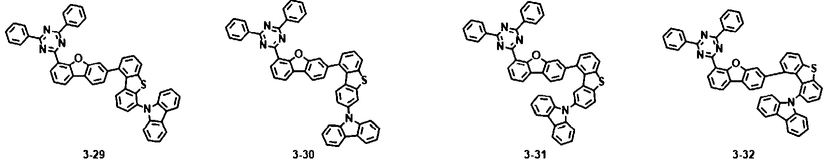

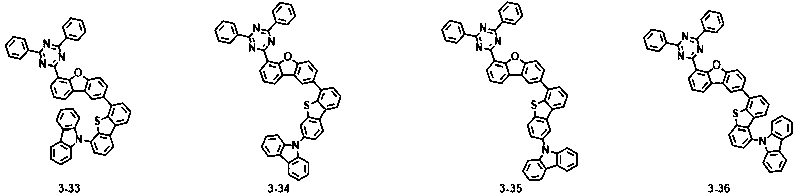

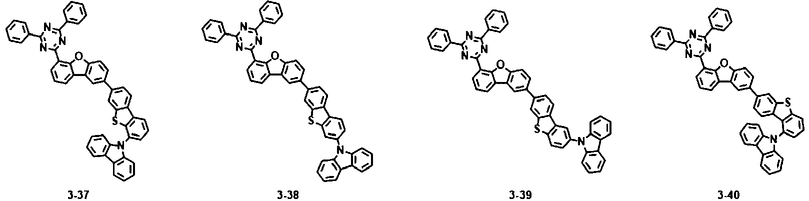

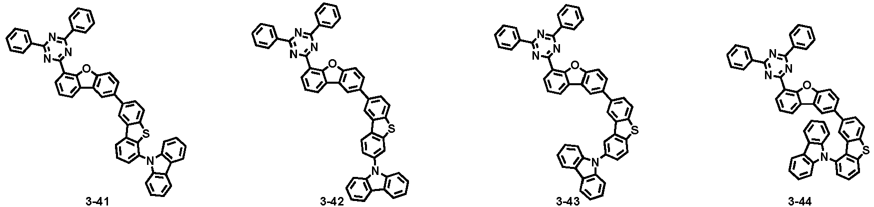

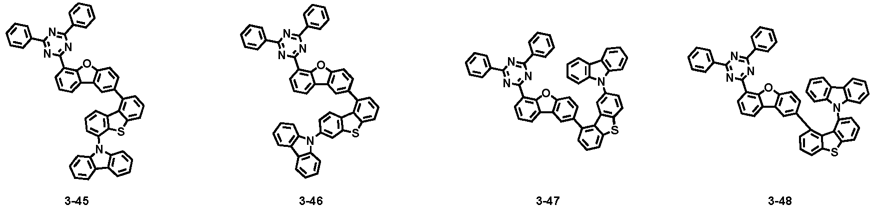

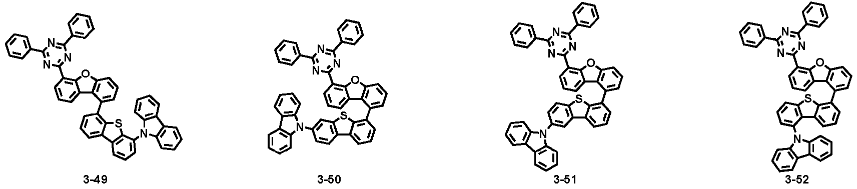

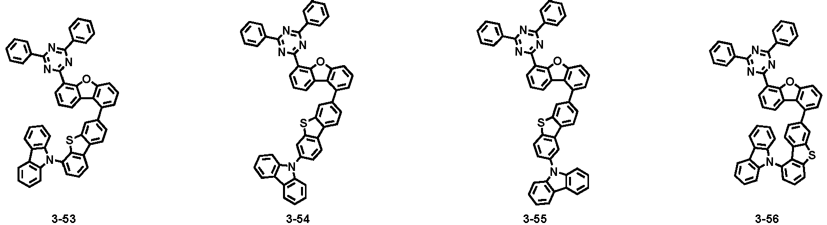

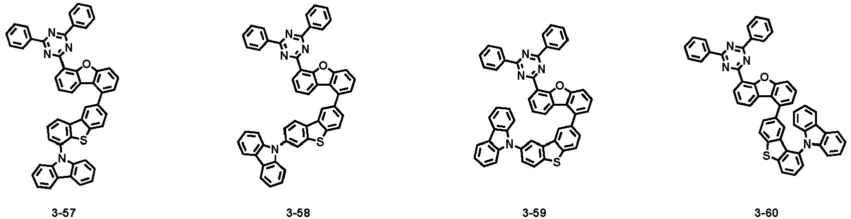

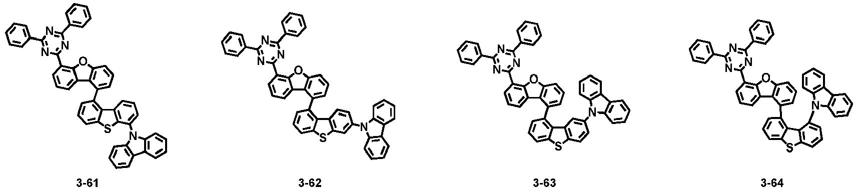

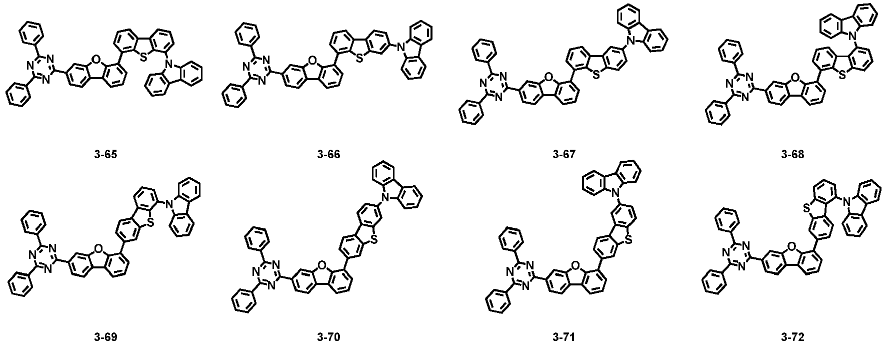

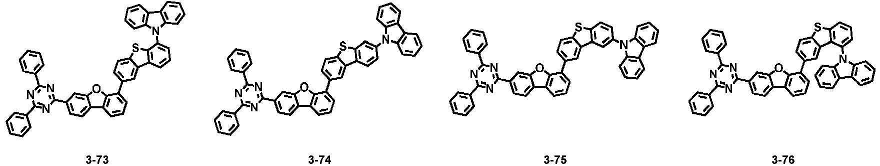

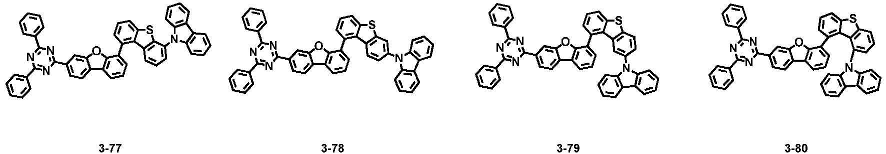

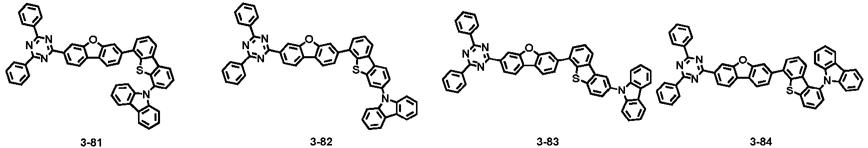

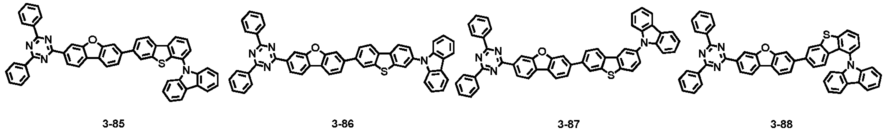

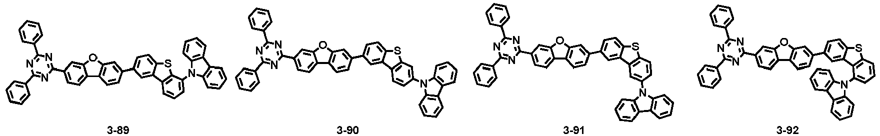

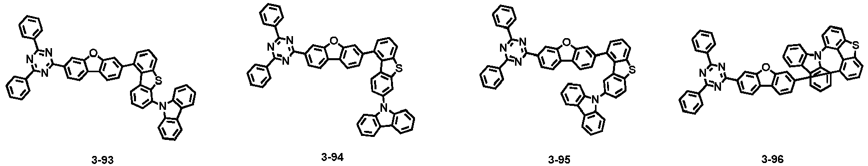

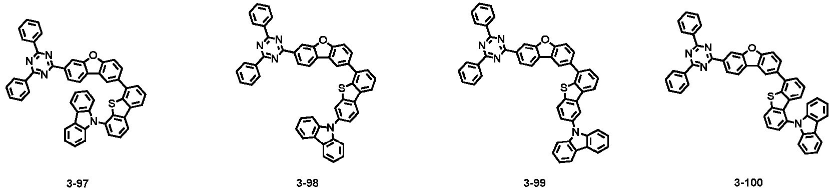

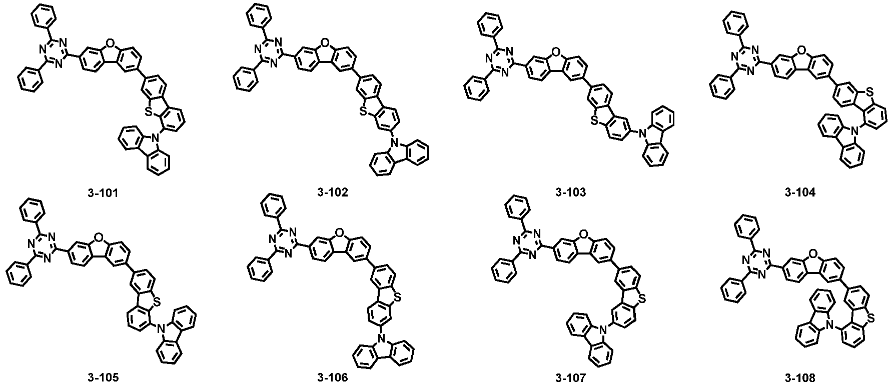

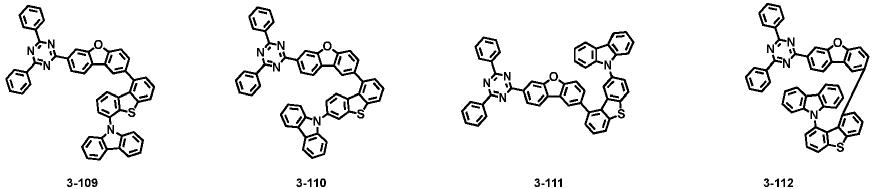

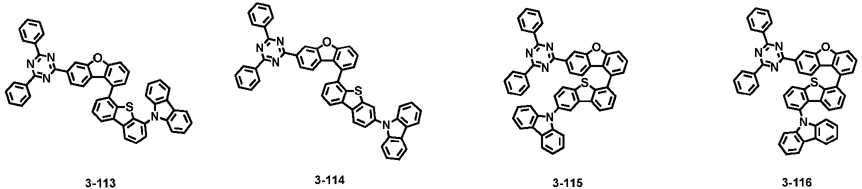

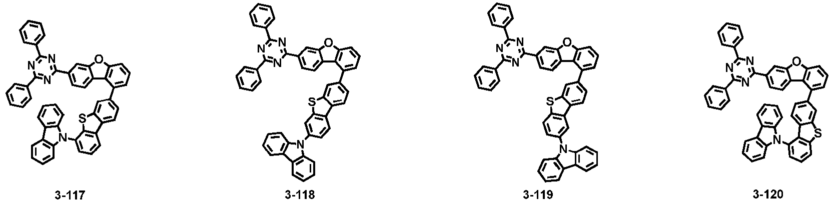

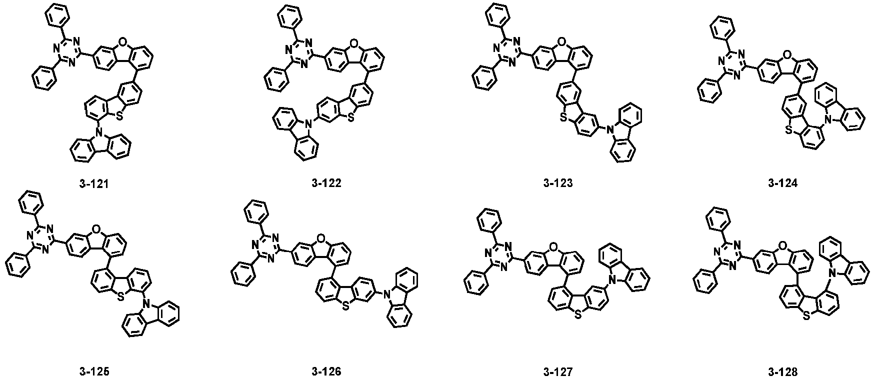

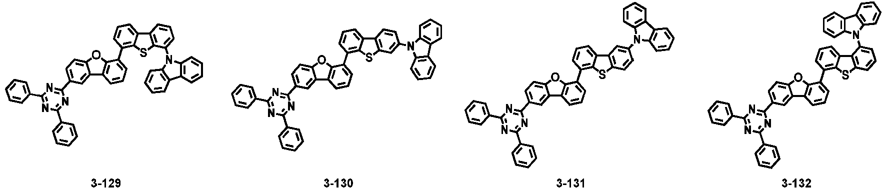

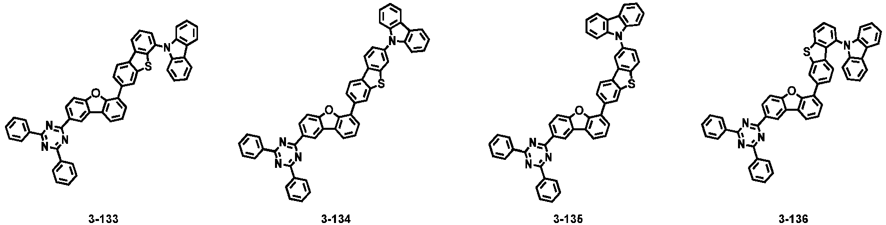

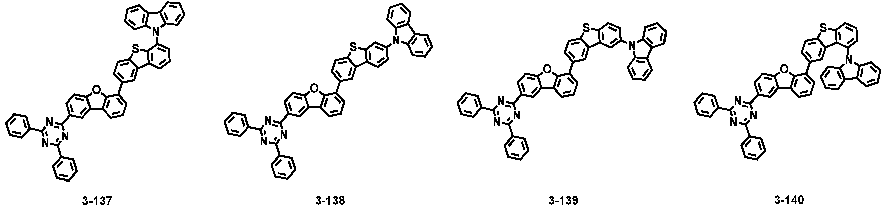

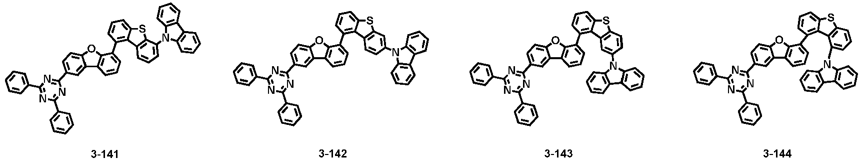

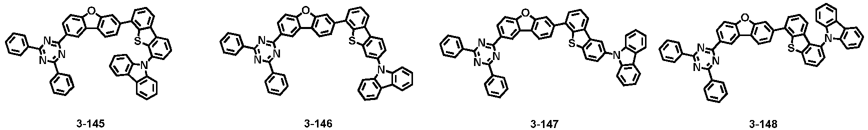

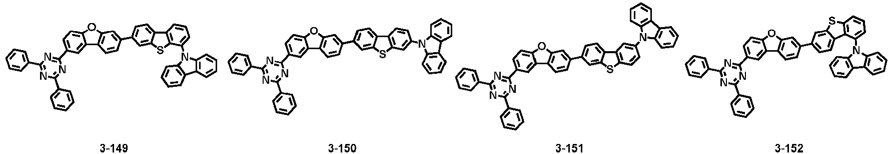

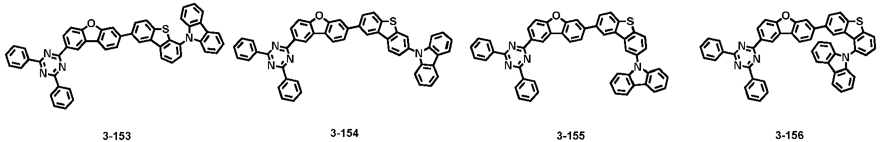

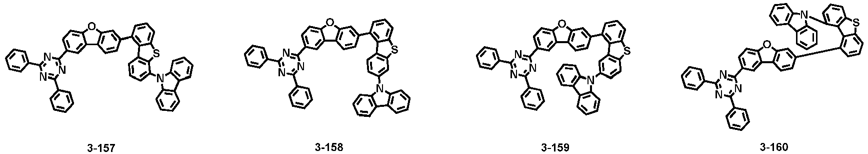

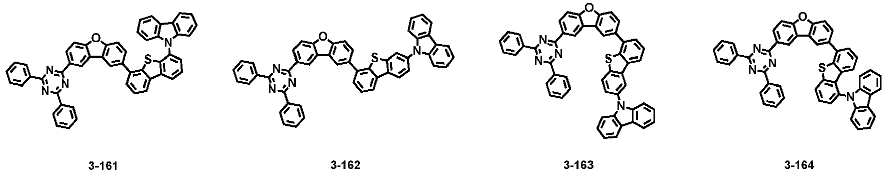

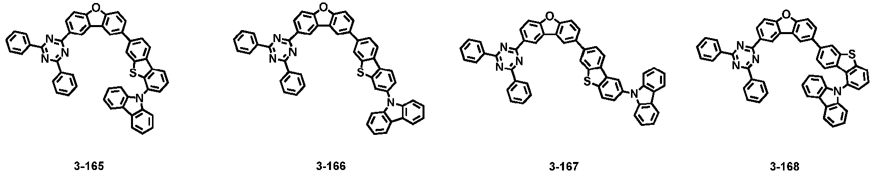

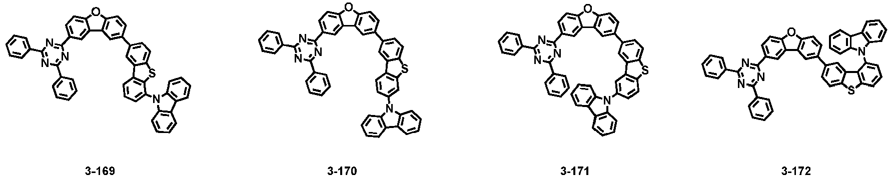

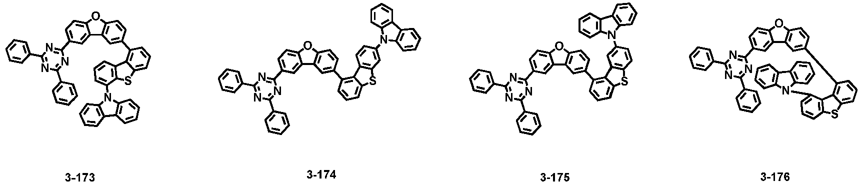

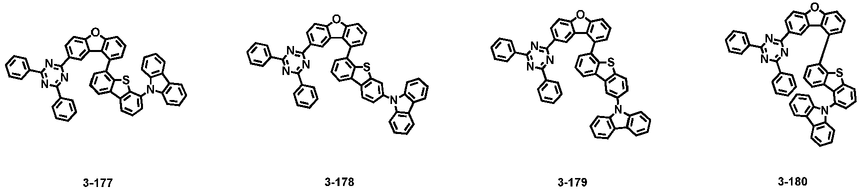

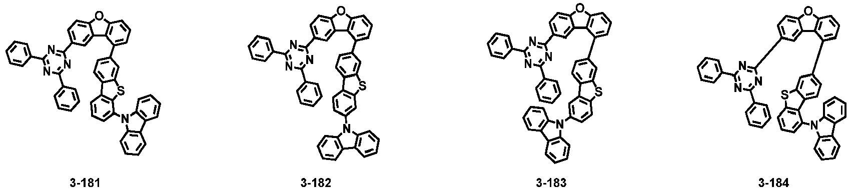

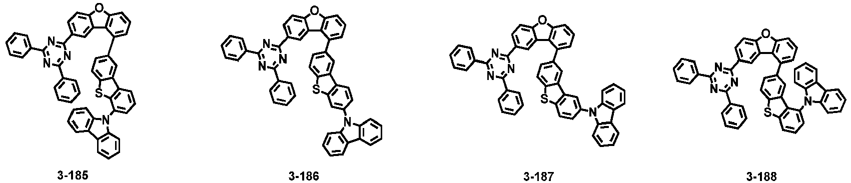

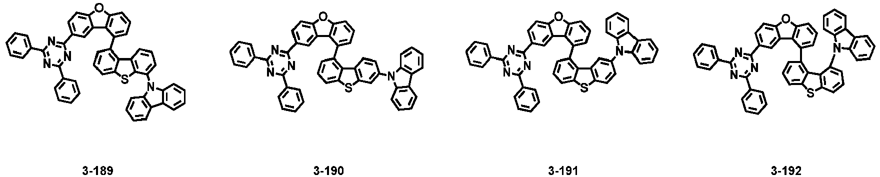

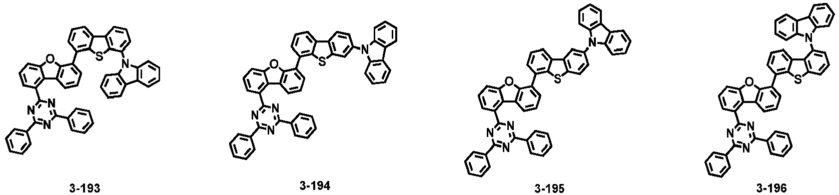

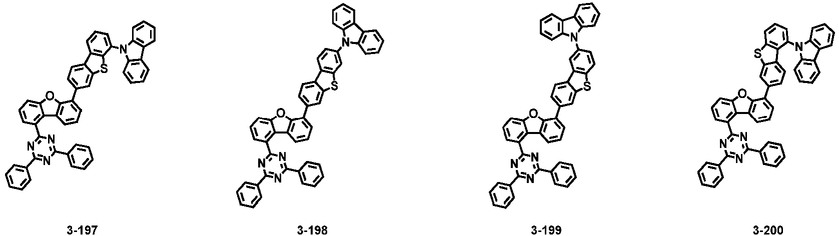

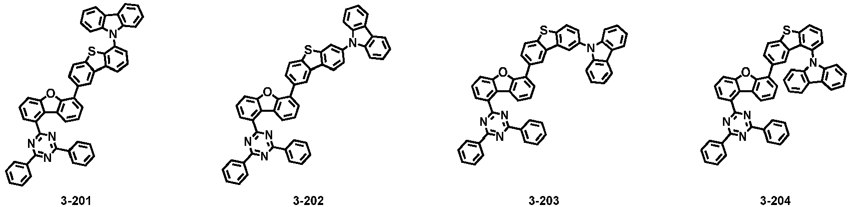

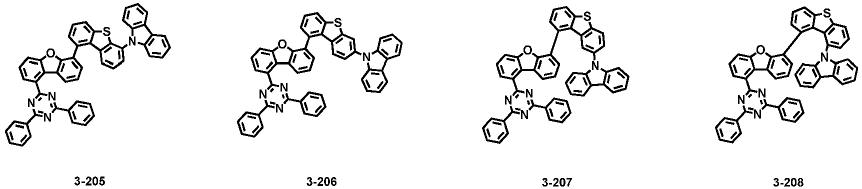

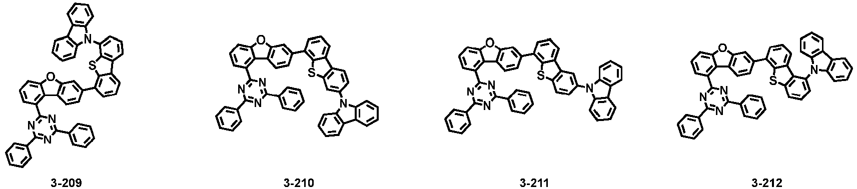

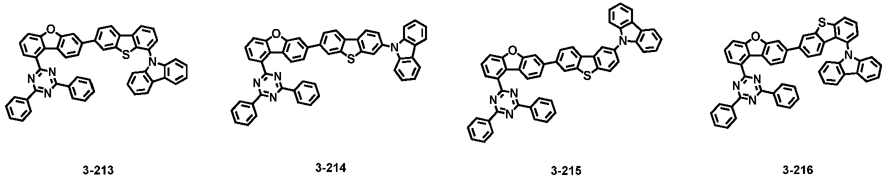

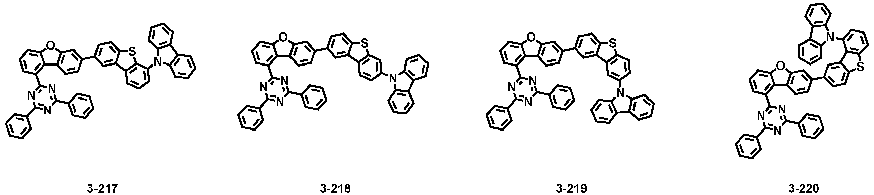

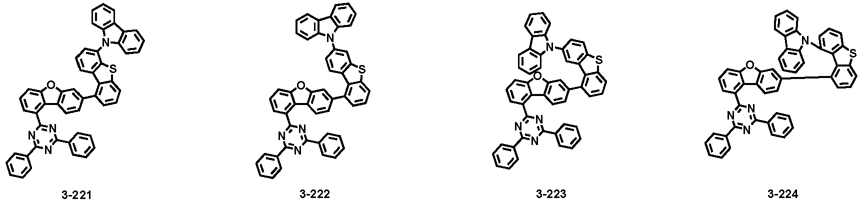

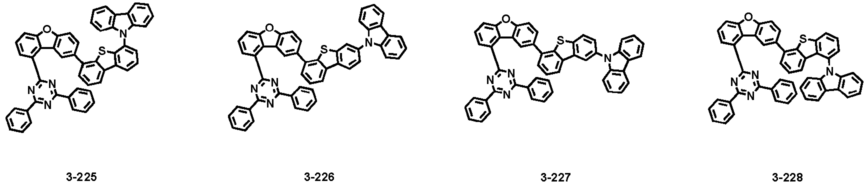

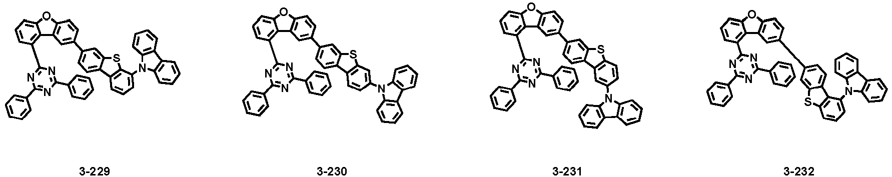

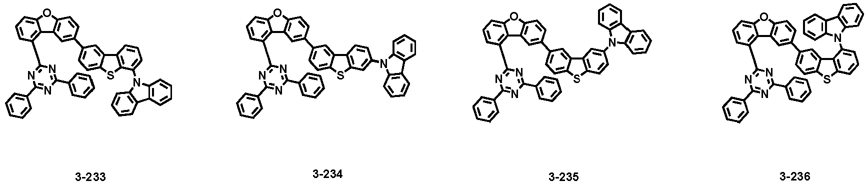

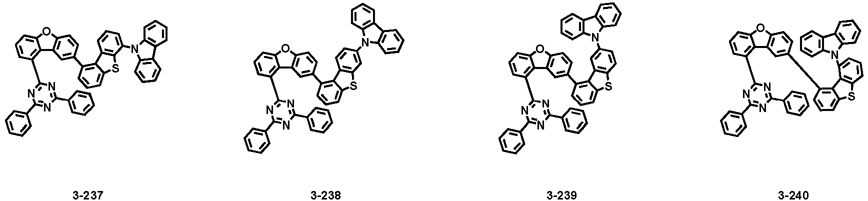

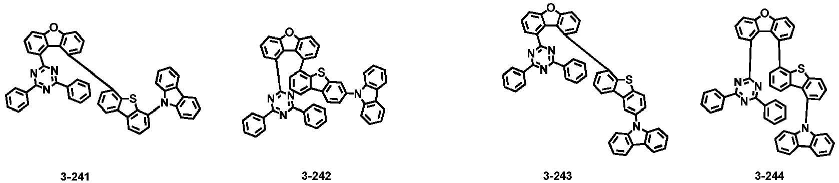

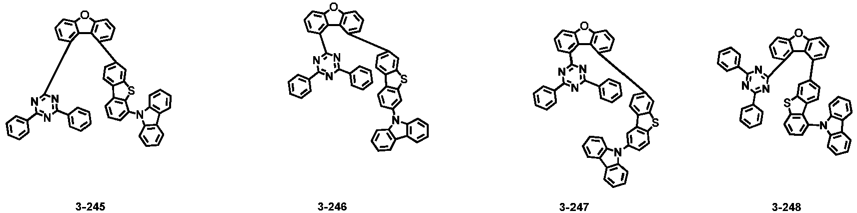

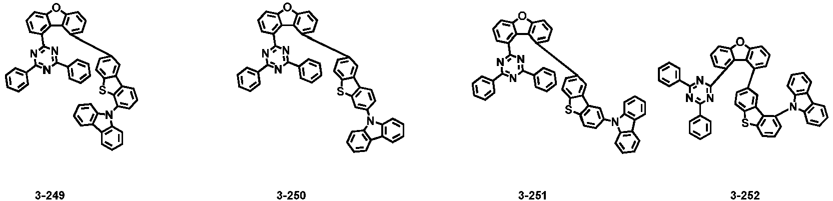

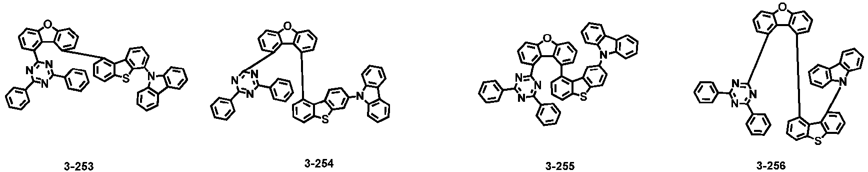

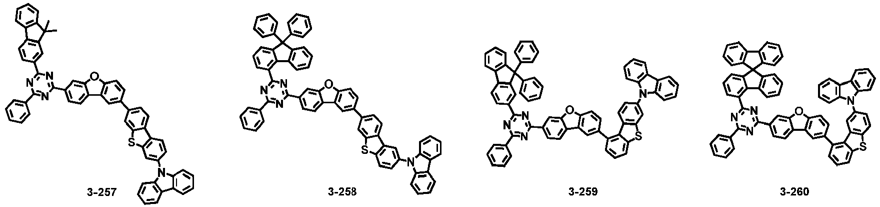

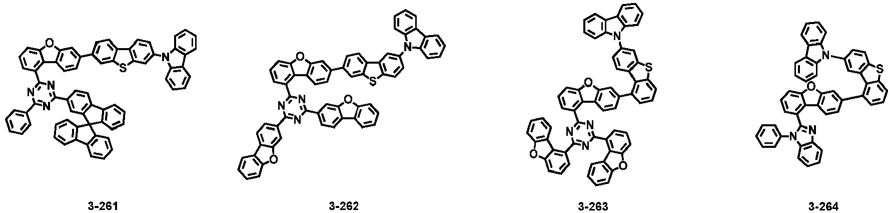

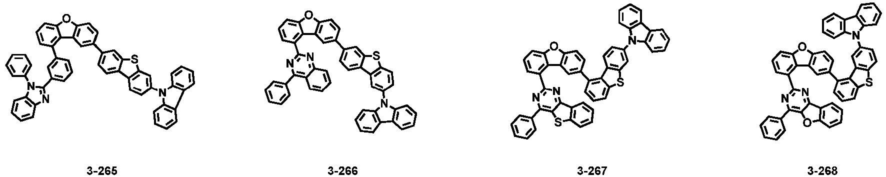

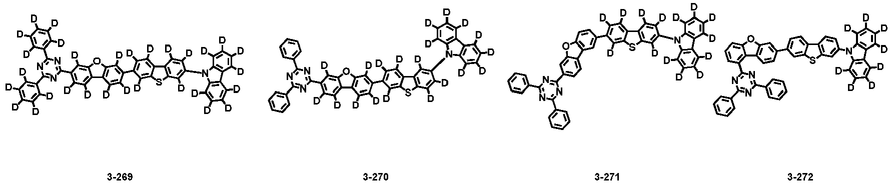

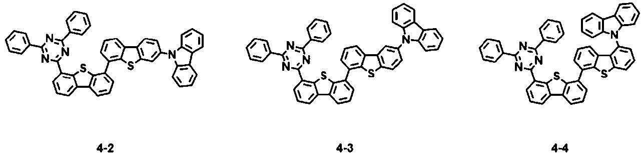

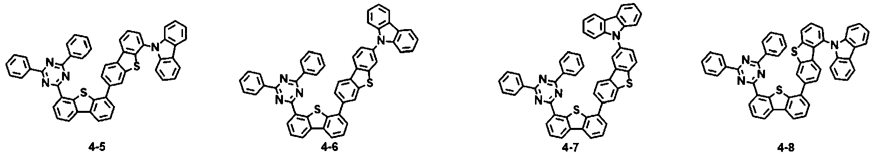

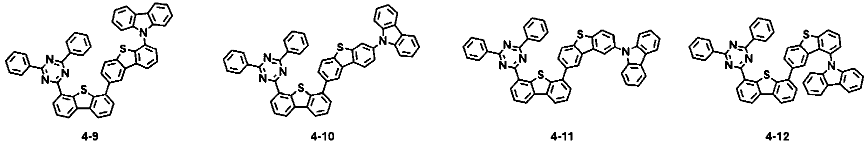

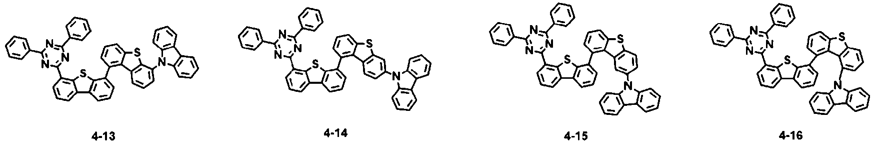

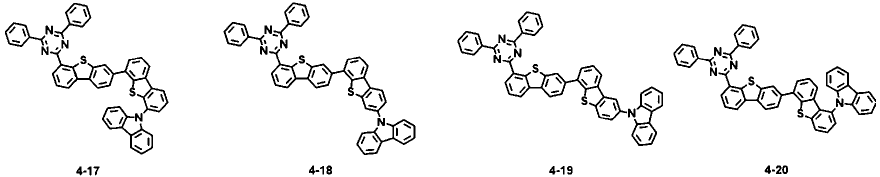

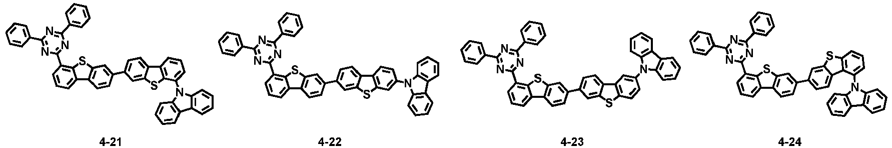

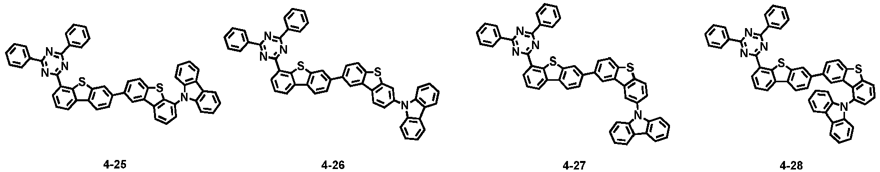

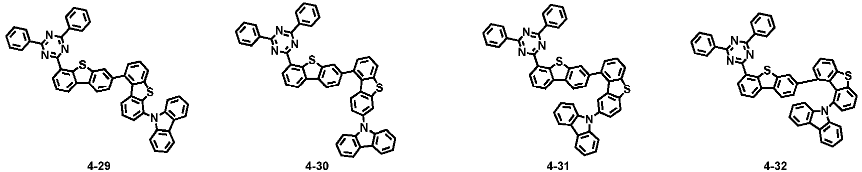

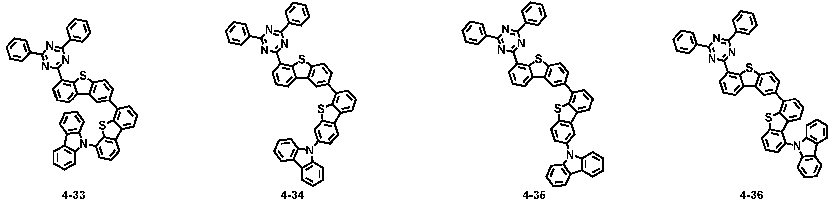

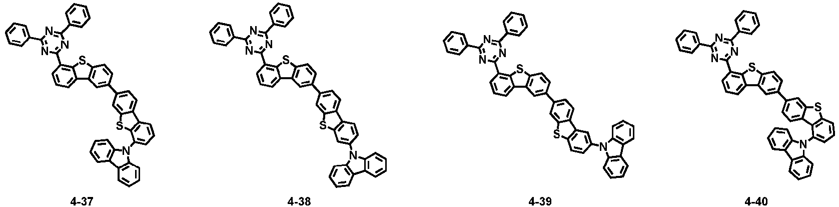

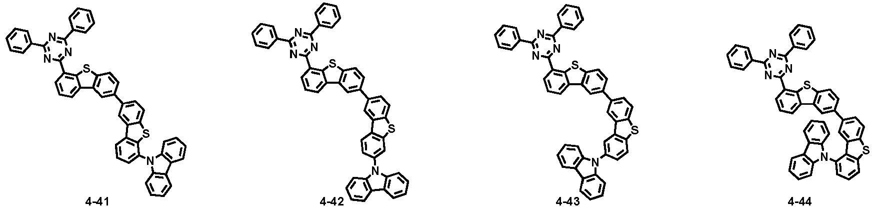

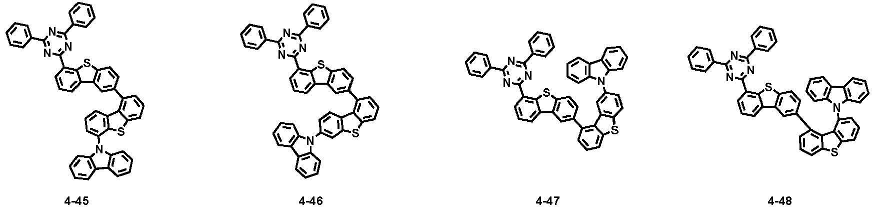

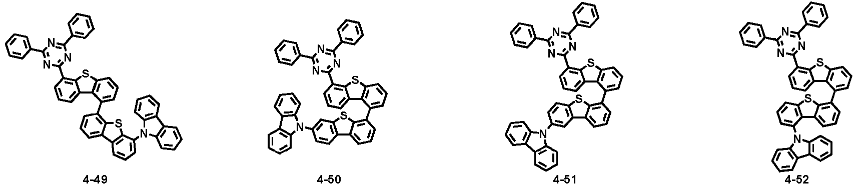

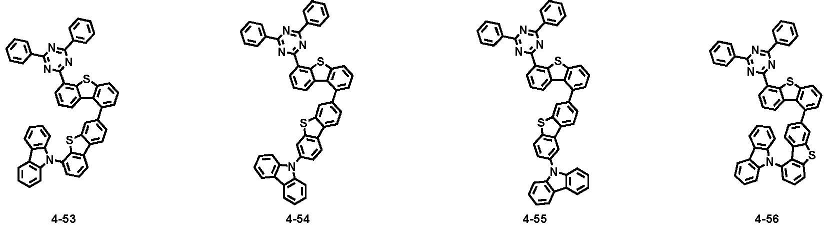

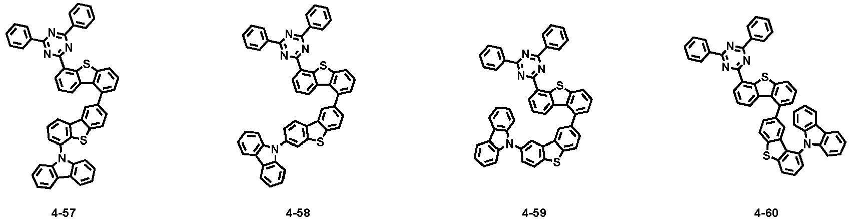

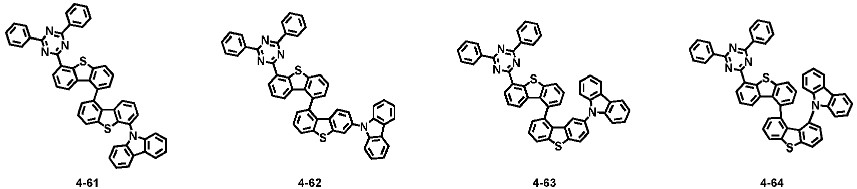

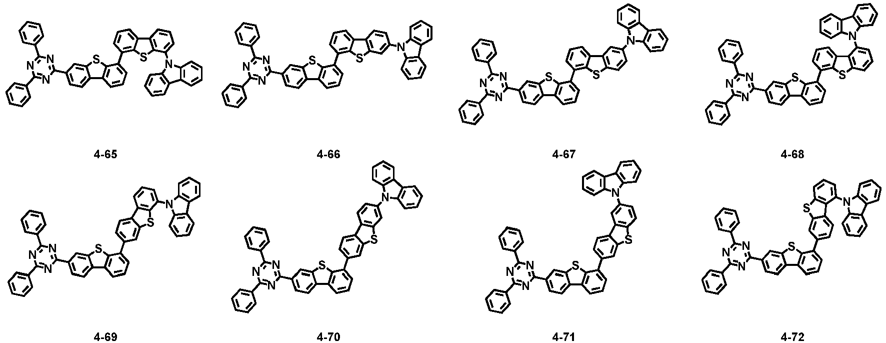

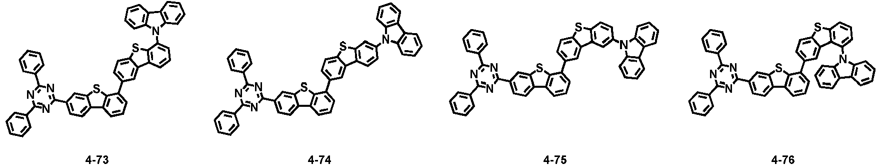

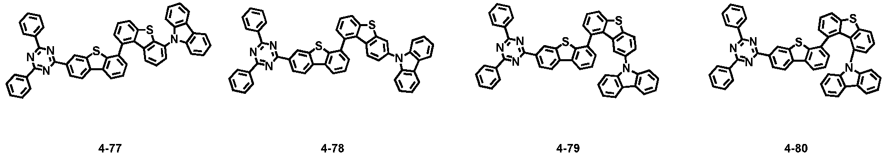

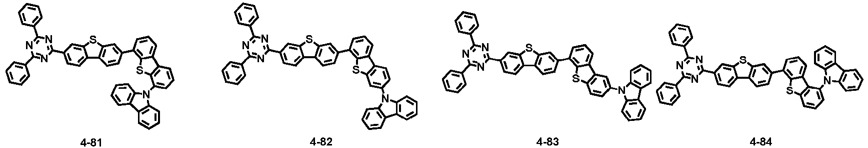

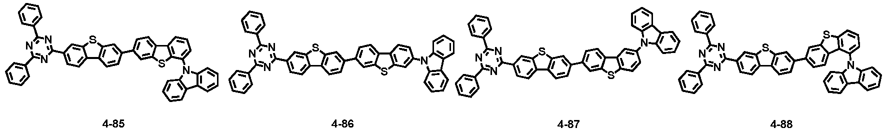

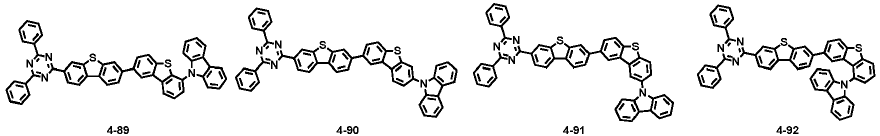

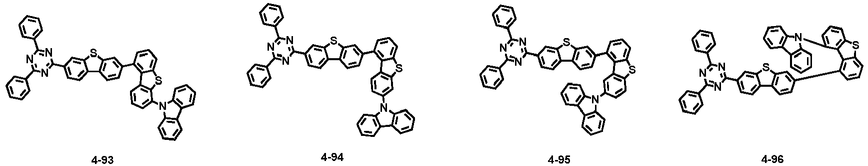

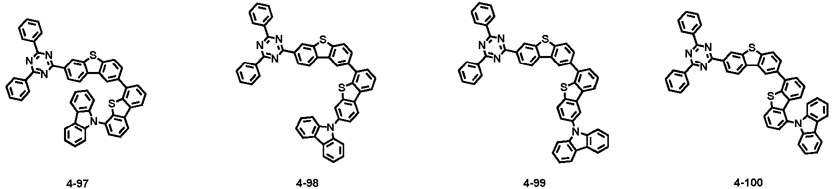

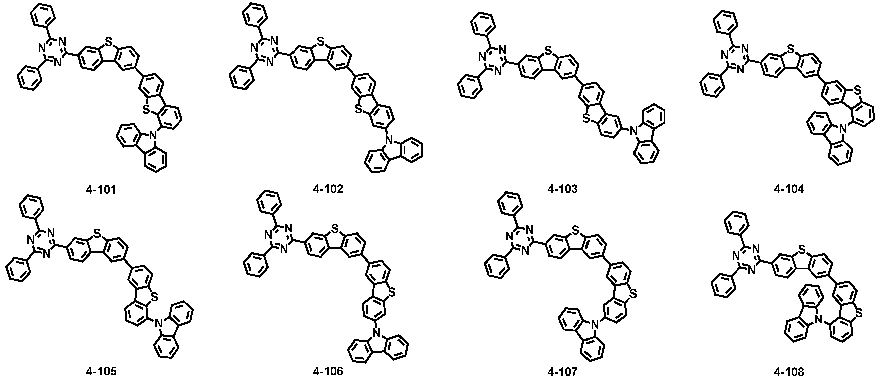

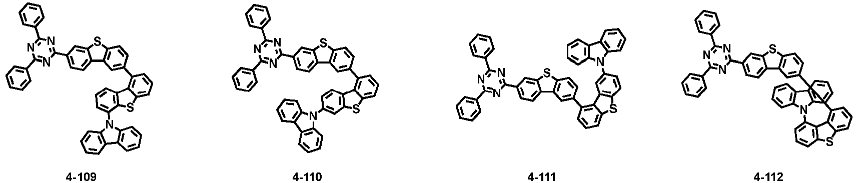

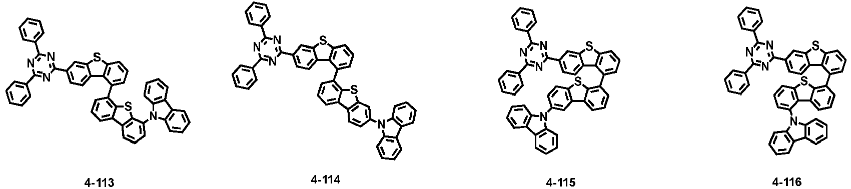

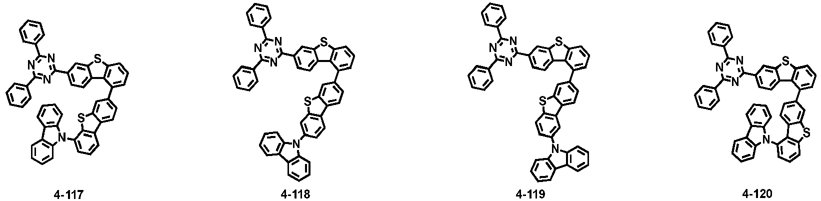

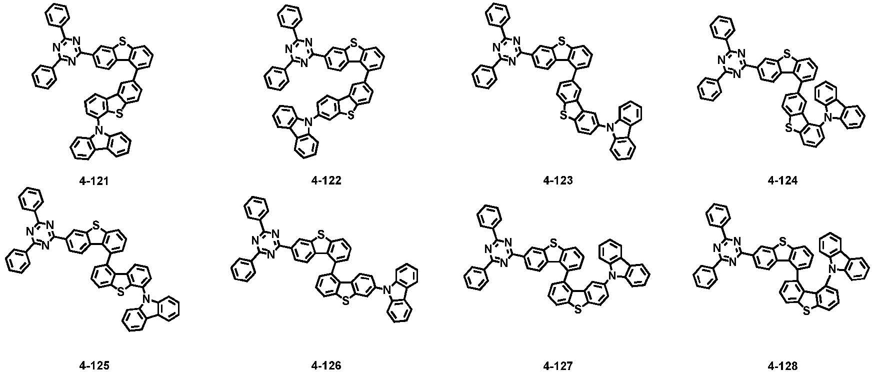

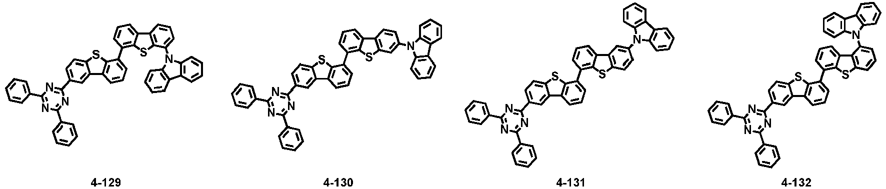

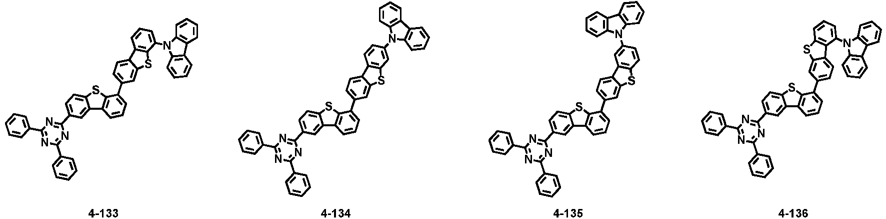

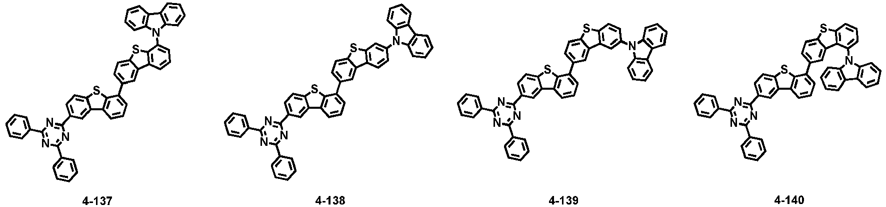

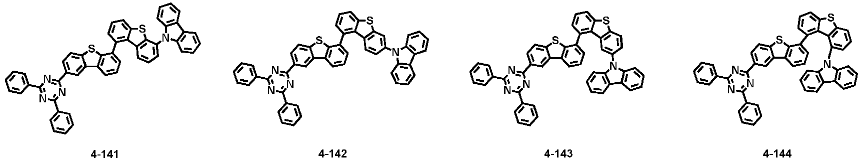

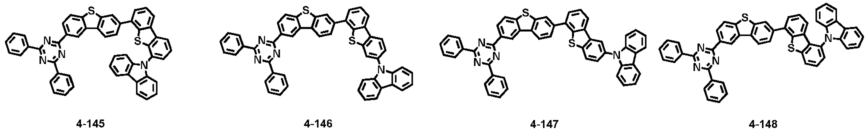

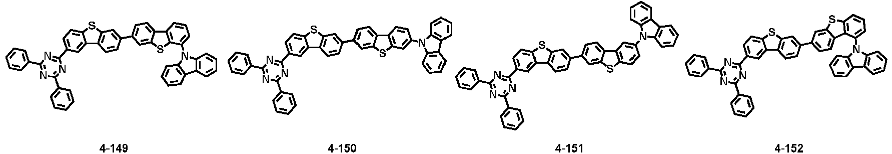

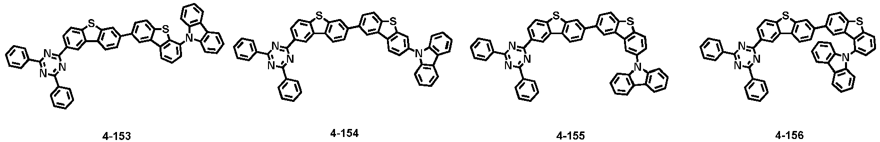

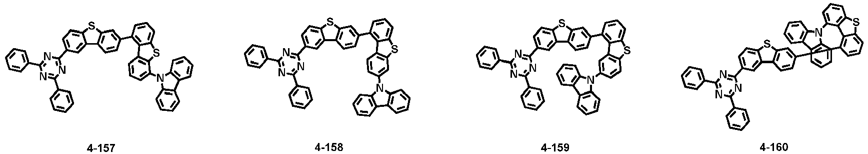

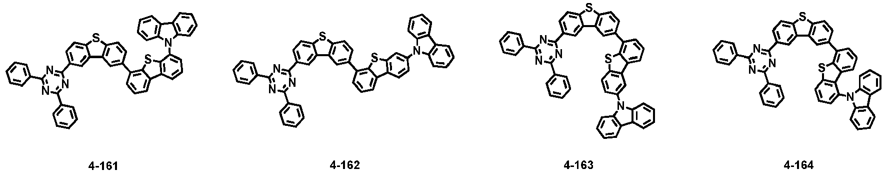

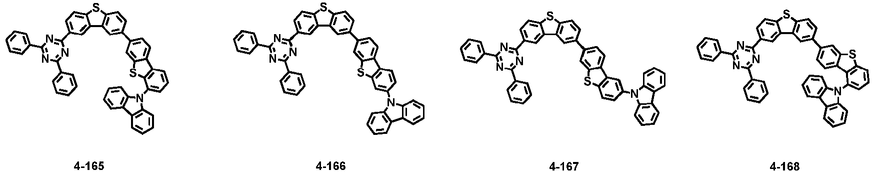

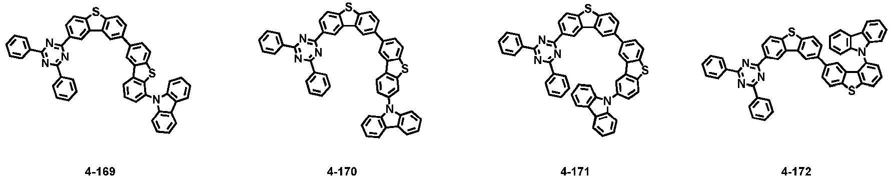

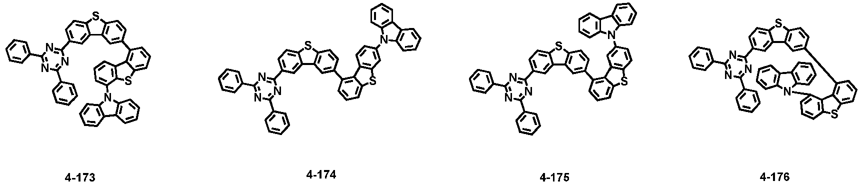

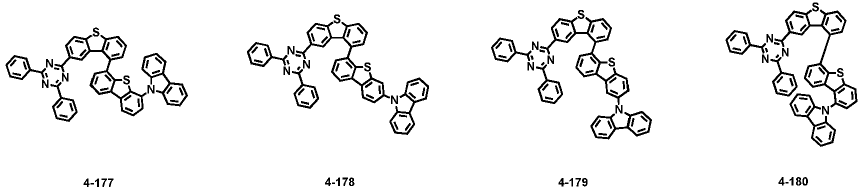

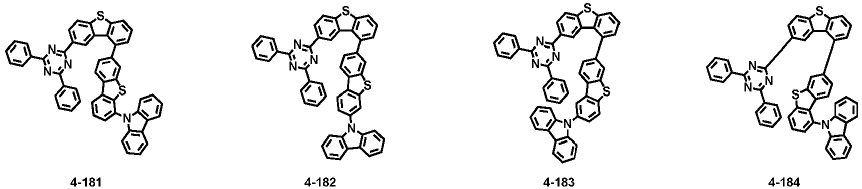

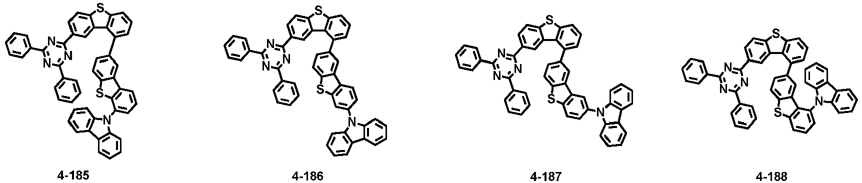

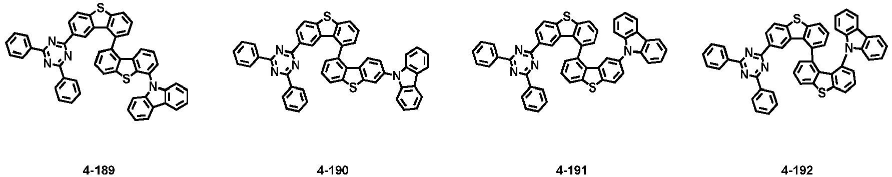

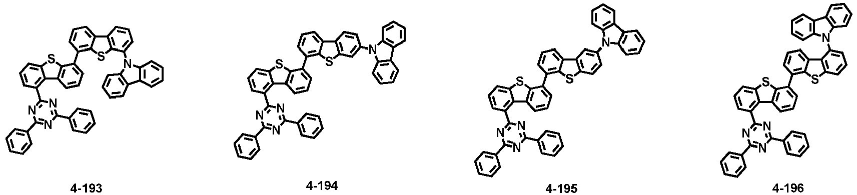

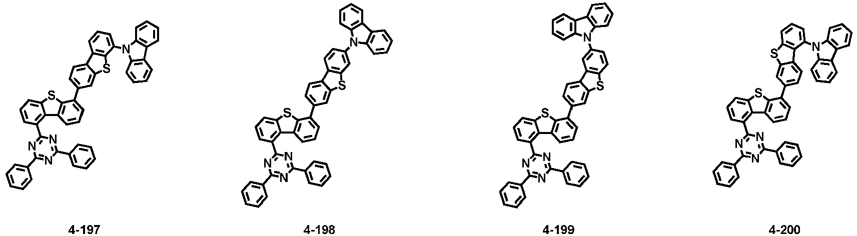

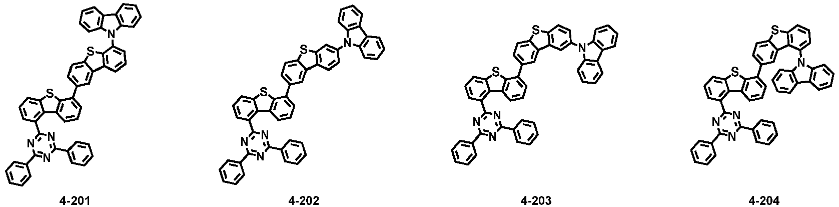

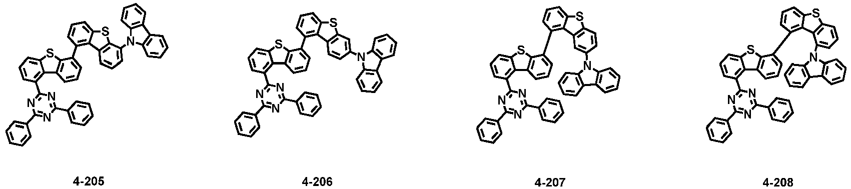

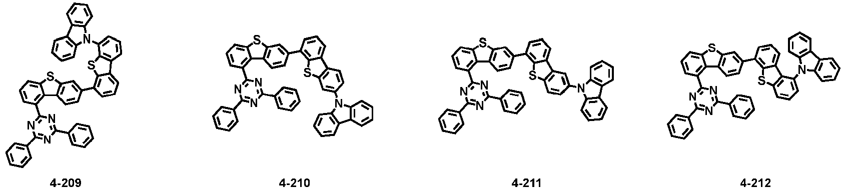

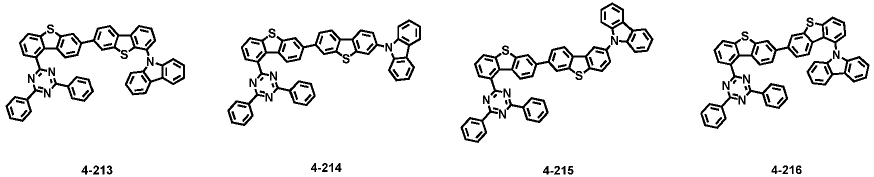

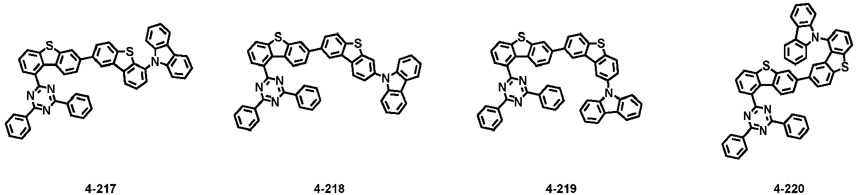

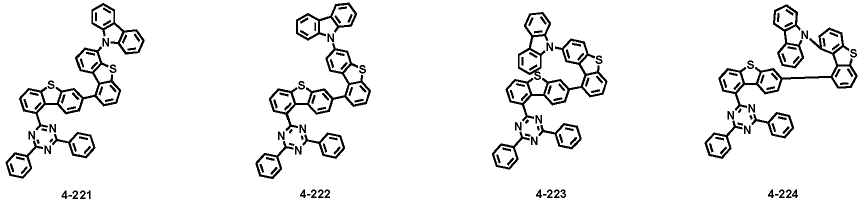

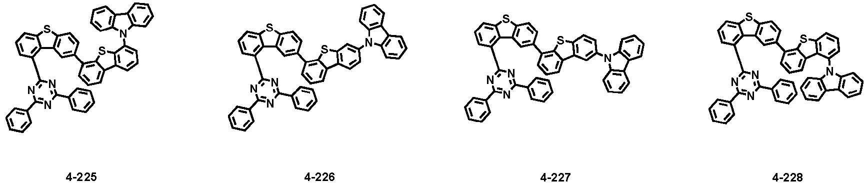

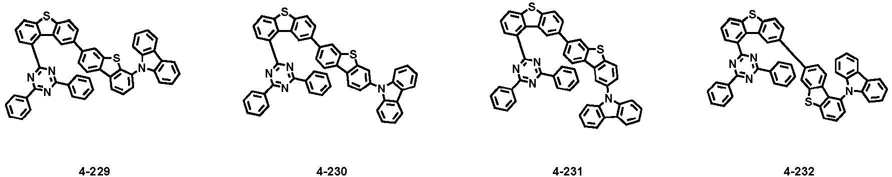

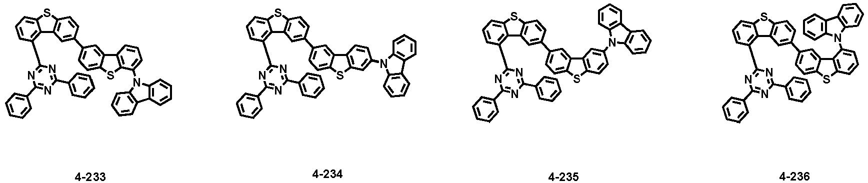

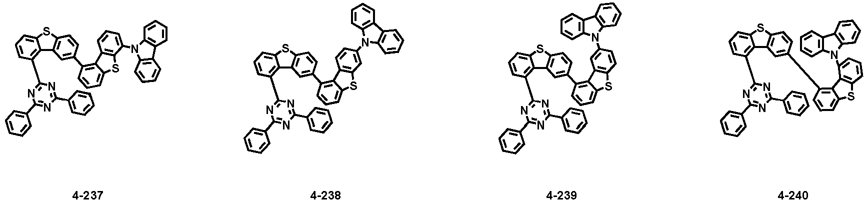

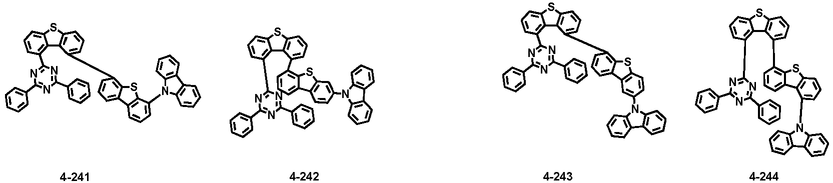

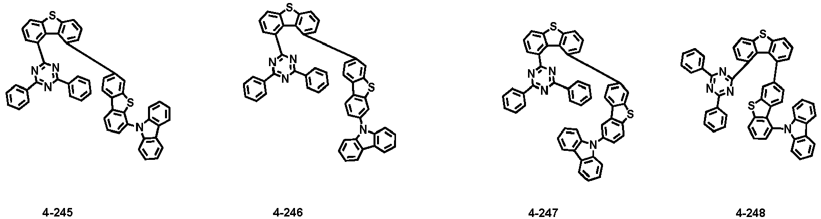

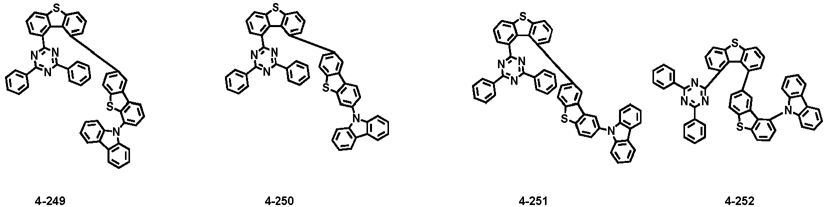

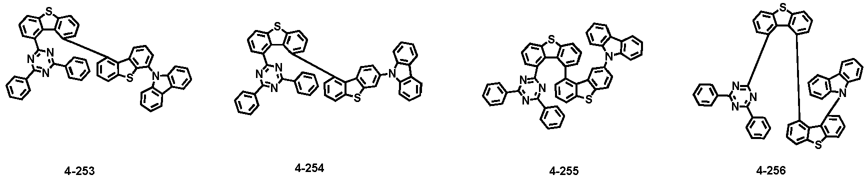

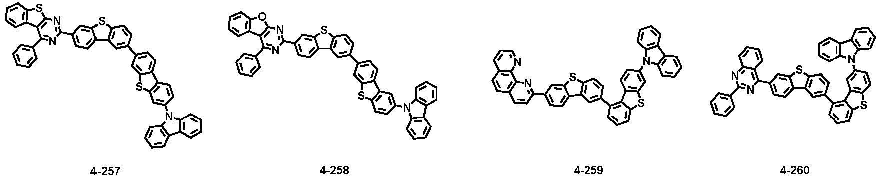

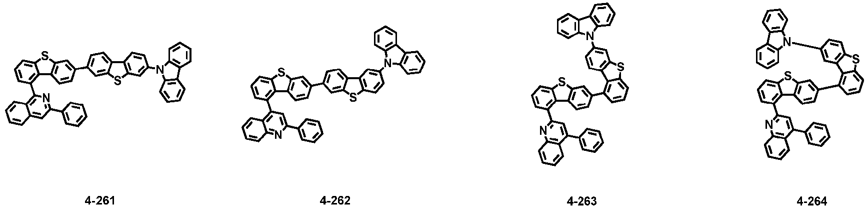

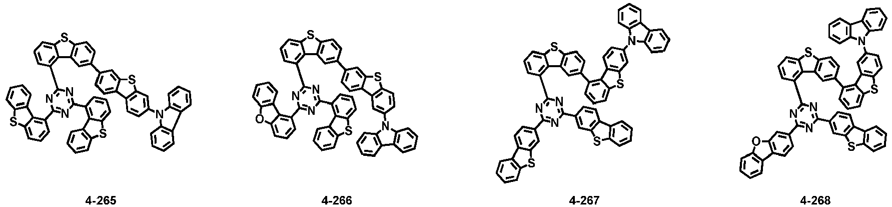

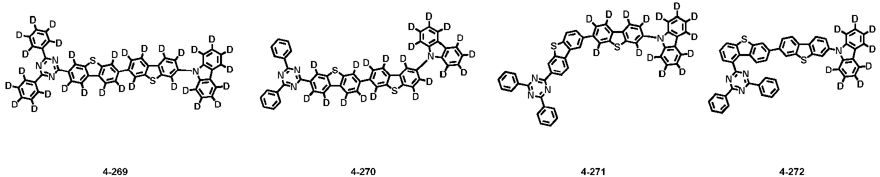

In the compound represented by any one of the Chemical Formulae 1-2 to 1-4 of the present application comprising the specific examples or represented by any one of the specific examples above, orbital disconnection occurs around N of carbazole, and the HOMO orbital is generally localized to the carbazole. Being localized means that electron cloud is relatively focused on carbazole.

When more widely delocalizing the HOMO orbital as above, hole mobility and stability may increase.

In addition, dibenzofuran or dibenzothiophene of the compound represented by any one of the Chemical Formulae 1-2 to 1-4 of the present application comprising the specific examples or represented by any one of the specific examples above corresponds to a group affecting the LUMO orbital of the heterocyclic group comprising N.

As a result, the LUMO level of the compound may be adjusted depending on the position of substitution of the heterocyclic group comprising N substituting dibenzofuran or dibenzothiophene. This means that the compound represented by any one of the Chemical Formulae 1-2 to 1-4 or represented by any one of the specific examples aboveof the present application may be used as a material of an organic light emitting device, particularly a material of a light emitting layer, and more specifically a host material by properly adjusting the LUMO level depending on dopant and electron transfer layer (ETL) materials in the organic light emitting device. In other words, it means that, when dopant and electron transfer layer materials change, a host material needs to be changed depending on the charge balance.

Specifically, when using a compound with a low LUMO level as a material of a light emitting layer, electrons may be readily injected from a host to a dopant, and when using a compound with a high LUMO level as a material of a light emitting layer, electrons may be readily injected from an electron transfer layer (ETL) to a host.

In addition, dibenzofuran or dibenzothiophene substituted with amine or carbazole as in the compound represented by any one of the Chemical Formulae 1-2 to 1-4 of the present application comprising the specific examples or represented by any one of the specific examples above may be affected by the LUMO orbital widely spread in the heterocyclic group comprising N to have an influence on enhancement in the electron mobility and stability.

In addition, when the compound represented by any one of the Chemical Formulae 1-2 to 1-4 of the present application comprising the specific examples or represented by any one of the specific examples above has a disconnected structure depending on the position of substitution of amine or carbazole, the HOMO orbital of carbazole or amine is affected, and the HOMO level may be adjusted. This means that the compound represented by any one of the Chemical Formulae 1-2 to 1-4 of the present application or represented by any one of the specific examples above may be used as a material of an organic light emitting device, particularly a material of a light emitting layer, and more specifically a host material by properly adjusting the HOMO level depending on dopant and electron transfer layer (ETL) materials. In other words, it means that, when dopant and electron transfer layer materials change, a host material needs to be changed depending on the charge balance.

In addition, by introducing various substituents to the structure of any one of the Chemical Formulae 1-2 to 1-4, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of any one of the Chemical Formulae 1-2 to 1-4, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the heterocyclic compound of any one of the Chemical Formulae 1-2 to 1-4 or any one of the specific examples above may be prepared. More specifically, the heterocyclic compound according to one embodiment of the present application may be prepared based on preparation examples to describe later.

Another embodiment of the present application provides an organic light emitting device comprising the heterocyclic compound represented by any one of the Chemical Formulae 1-2 to 1-4 or the heterocyclic compound represented by any one of the above compounds. The "organic light emitting device" may be expressed in terms such as an "organic light emitting diode", an "OLED", an "OLED device" and an "organic electroluminescent device".

The heterocyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises a first electrode, a second electrode, and one or more organic material layers provided between the first electrode and the second electrode, and one or more layers of the organic material layers comprise the heterocyclic compound represented by any one of the Chemical Formulae 1-2 to 1-4 or the heterocyclic compound represented by any one of the above compounds. When comprising the heterocyclic compound represented by any one of the Chemical Formulae 1-2 to 1-4 or the heterocyclic compound represented by any one of the above compounds in the organic material layer, the organic light emitting device has superior light emission efficiency and lifetime.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to any one of the Chemical Formulae 1-2 to 1-4 or the heterocyclic compound represented by any one of the above compounds may be used as a material of the blue organic light emitting device.

In one embodiment of the present application, the organic light emitting device may be a green organic light emitting device, and the heterocyclic compound according to any one of the Chemical Formulae 1-2 to 1-4 or the heterocyclic compound represented by any one of the above compounds may be used as a material of the green organic light emitting device.

In one embodiment of the present application, the organic light emitting device may be a white organic light emitting device, and the heterocyclic compound according to any one of the Chemical Formulae 1-2 to 1-4 or the heterocyclic compound represented by any one of the above compoundsmay be used as a material of the white organic light emitting device.

In one embodiment of the present application, the organic light emitting device may be a red organic light emitting device, and the heterocyclic compound according to any one of the Chemical Formulae 1-2 to 1-4 or the heterocyclic compound represented by any one of the above compoundsmay be used as a material of the red organic light emitting device.

In addition, the organic material layer comprises one or more light emitting layers, and the light emitting layer comprises the heterocyclic compound represented by any one of the Chemical Formulae 1-2 to 1-4 or the heterocyclic compound represented by any one of the above compounds. When comprising the heterocyclic compound represented by any one of the Chemical Formulae 1-2 to 1-4 or the heterocyclic compound represented by any one of the above compoundsin the light emitting layer among the organic material layers, the organic light emitting device has more superior light emission efficiency and lifetime.

In addition, in the organic light emitting device of the present application, the organic material layer comprises the heterocyclic compound represented by any one of the Chemical Formulae 1-2 to 1-4 or the heterocyclic compound represented by any one of the above compoundsas a first compound, and may further comprise a heterocyclic compound represented by the following Chemical Formula 13 or 14 as a second compound. 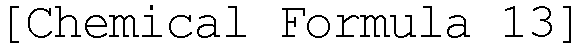 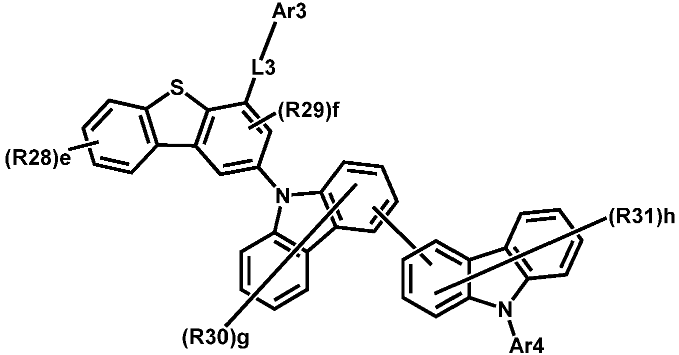 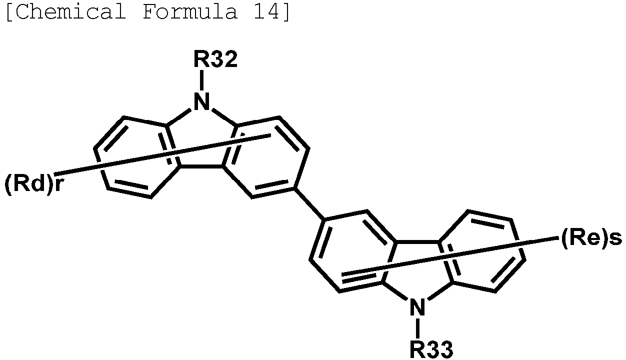 In Chemical Formulae 13 and 14,
L3 is a direct bond; a substituted or unsubstituted arylene group having 6 to 60 carbon atoms; or a substituted or unsubstituted heteroarylene group having 2 to 60 carbon atoms;
R28 to R31, Rd and Re are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aromatic hydrocarbon ring having 6 to 60 carbon atoms or a substituted or unsubstituted heteroring having 2 to 60 carbon atoms, r and s are an integer of 0 to 7, and when r is 2 or greater, Rds are the same as or different from each other, when s is 2 or greater, Res are the same as or different from each other, e is an integer of 0 to 4, g and h are each an integer of 0 to 7, f is an integer of 0 to 2, and when e is 2 or greater, R28s are the same as or different from each other, when f is an integer of 2, R29s are the same as or different from each other, when g is 2 or greater, R30s are the same as or different from each other, and when h is 2 or greater, R31s are the same as or different from each other,
Ar3 is a substituted or unsubstituted aryl group having 6 to 60 carbon atoms; or a substituted or unsubstituted heteroaryl group having 2 to 60 carbon atoms,
Ar4 is a substituted or unsubstituted aryl group having 6 to 60 carbon atoms; or a substituted or unsubstituted heteroaryl group having 2 to 60 carbon atoms, and
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms.

In one embodiment of the present application, the heterocyclic compound represented by Chemical Formula 13 may have a deuterium content of greater than 10% and less than or equal to 100%.

In one embodiment of the present application, the heterocyclic compound represented by Chemical Formula 14 may have a deuterium content of greater than 10% and less than or equal to 100%.

In one embodiment of the present application, L3 may be a direct bond; a substituted or unsubstituted arylene group having 6 to 40 carbon atoms; or a substituted or unsubstituted heteroarylene group having 2 to 40 carbon atoms.

In one embodiment of the present application, L3 may be a direct bond; a substituted or unsubstituted arylene group having 6 to 20 carbon atoms; or a substituted or unsubstituted heteroarylene group having 2 to 20 carbon atoms.

In another embodiment, L3 is a direct bond; or a substituted or unsubstituted phenylene group.

In one embodiment of the present application, R28 to R31 are hydrogen.

In one embodiment of the present application, R28 to R31 are deuterium.

In one embodiment of the present application, Ar3 may be a substituted or unsubstituted aryl group having 6 to 60 carbon atoms; or a substituted or unsubstituted heteroaryl group having 2 to 60 carbon atoms.

In one embodiment of the present application, Ar3 may be a substituted or unsubstituted aryl group having 6 to 40 carbon atoms; or a substituted or unsubstituted heteroaryl group having 2 to 40 carbon atoms.

In one embodiment of the present application, Ar3 may be a substituted or unsubstituted aryl group having 6 to 20 carbon atoms; or a substituted or unsubstituted heteroaryl group having 2 to 20 carbon atoms.

In another embodiment, Ar3 may be a substituted or unsubstituted phenyl group; a substituted or unsubstituted biphenyl group; a substituted or unsubstituted fluorene group; a substituted or unsubstituted dibenzofuran group; or a substituted or unsubstituted dibenzothiophene group.

In another embodiment, Ar3 may be a phenyl group; a biphenyl group; a fluorene group substituted with one or more selected from the group consisting of an alkyl group having 1 to 10 carbon atoms; a dibenzofuran group; or a dibenzothiophene group.

In one embodiment of the present application, Ar4 may be a substituted or unsubstituted aryl group having 6 to 60 carbon atoms; or a substituted or unsubstituted heteroaryl group having 2 to 60 carbon atoms.

In one embodiment of the present application, Ar4 may be a substituted or unsubstituted aryl group having 6 to 40 carbon atoms; or a substituted or unsubstituted heteroaryl group having 2 to 40 carbon atoms.

In one embodiment of the present application, Ar4 may be a substituted or unsubstituted aryl group having 6 to 20 carbon atoms; or a substituted or unsubstituted heteroaryl group having 2 to 20 carbon atoms.

In another embodiment, Ar4 is a substituted or unsubstituted phenyl group.

In another embodiment, Ar4 is a phenyl group.

According to one embodiment of the present application, Chemical Formula 13 may be represented by any one of the following compounds, but is not limited thereto.

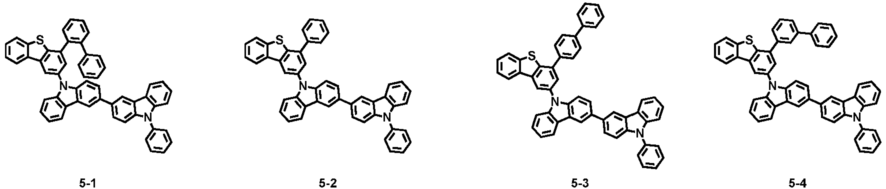

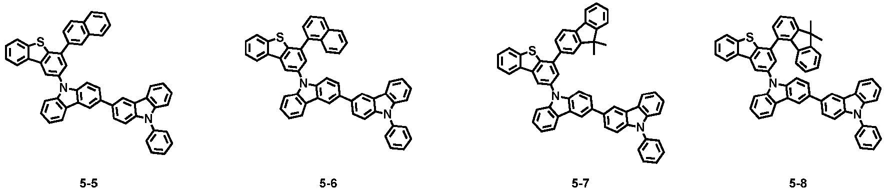

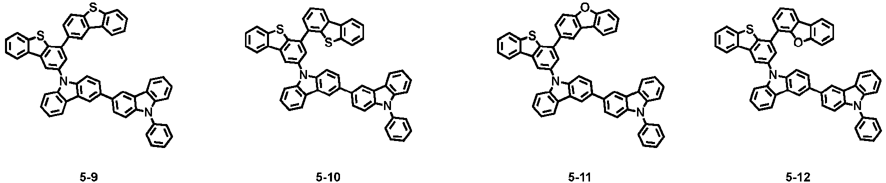

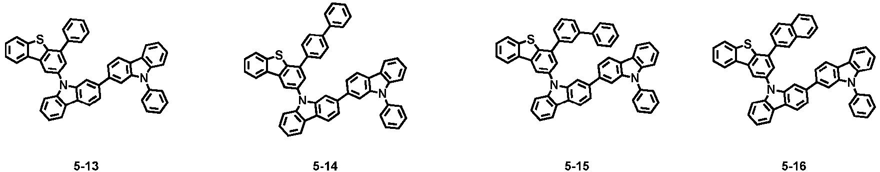

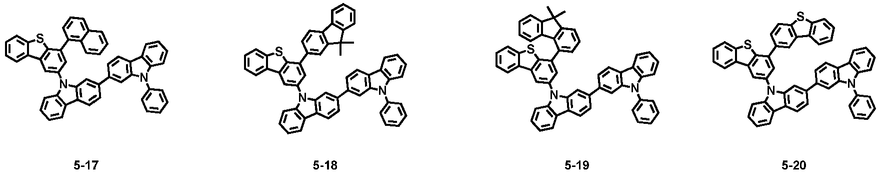

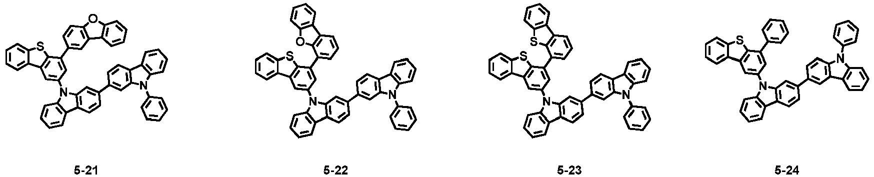

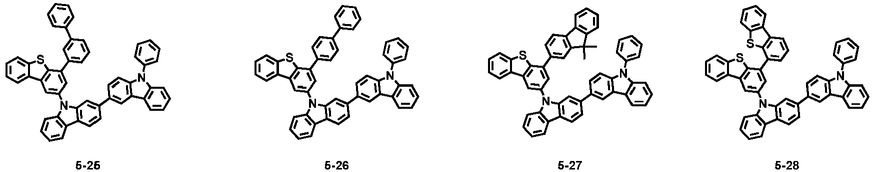

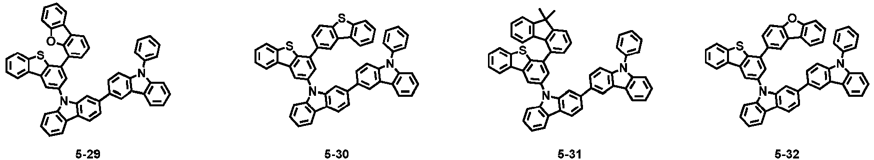

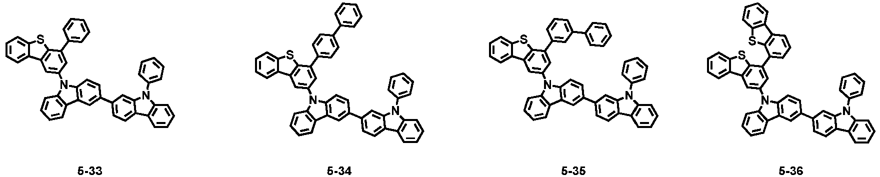

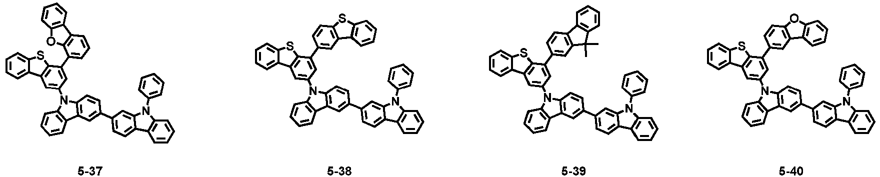

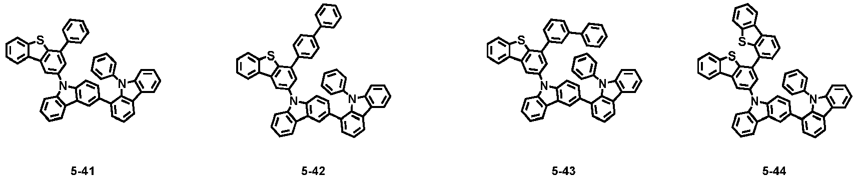

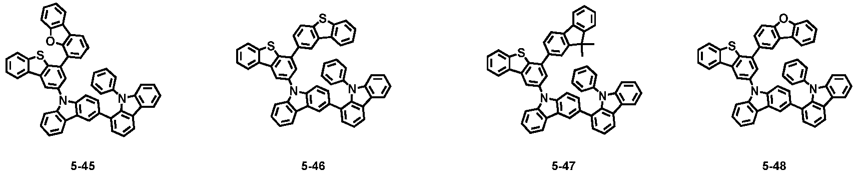

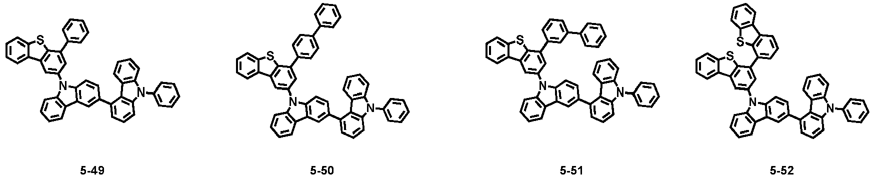

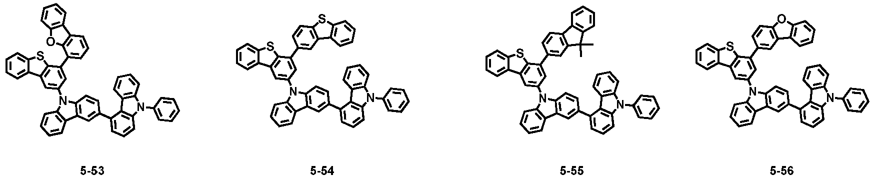

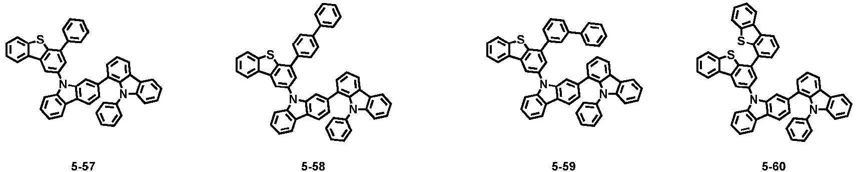

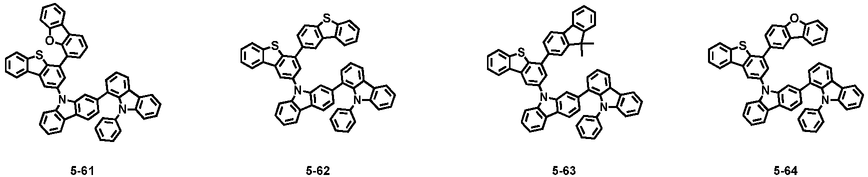

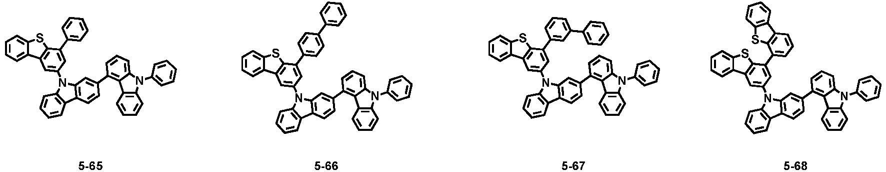

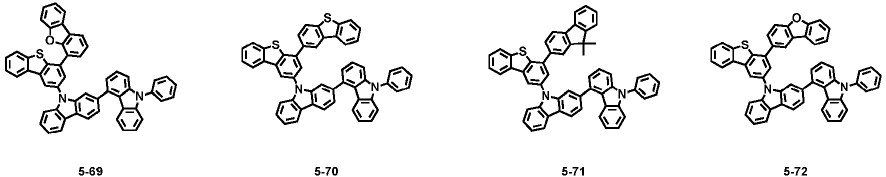

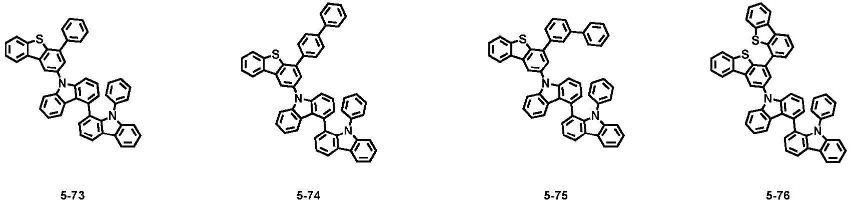

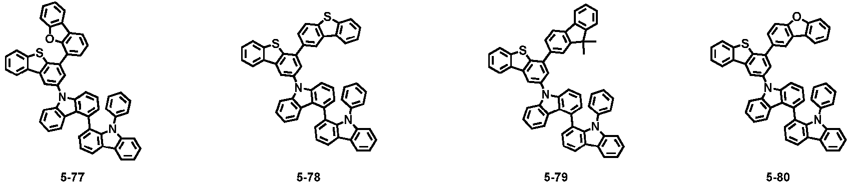

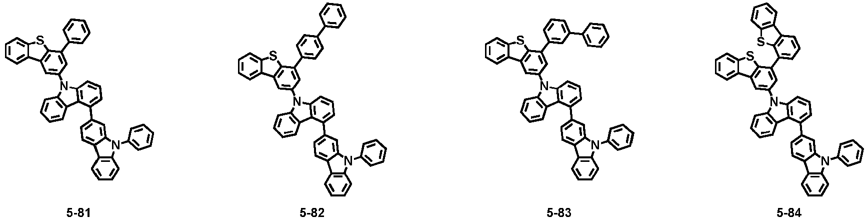

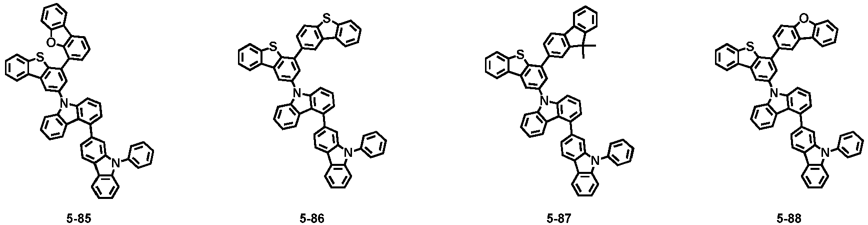

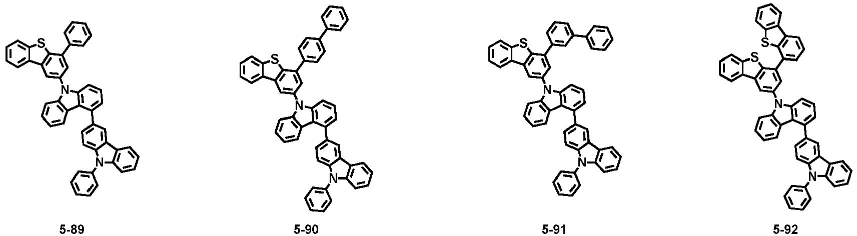

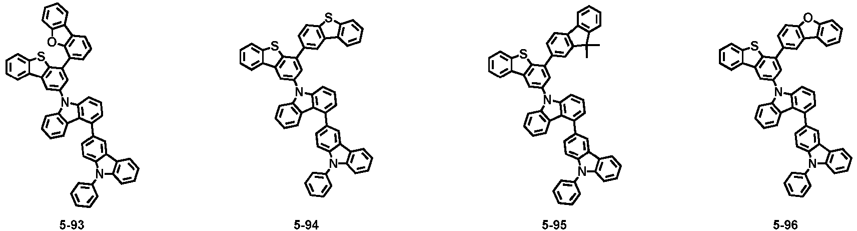

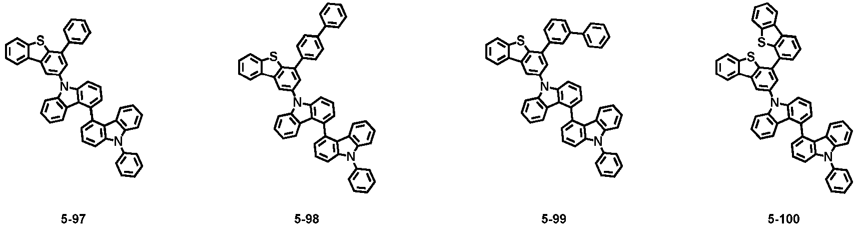

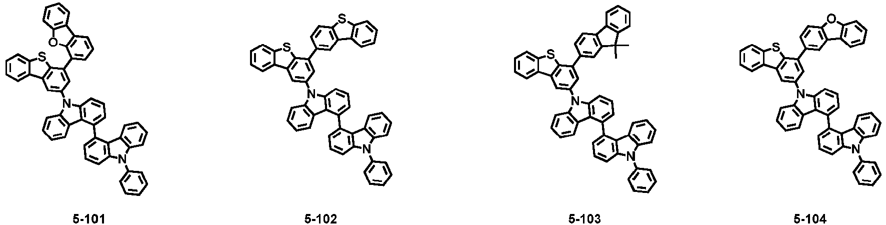

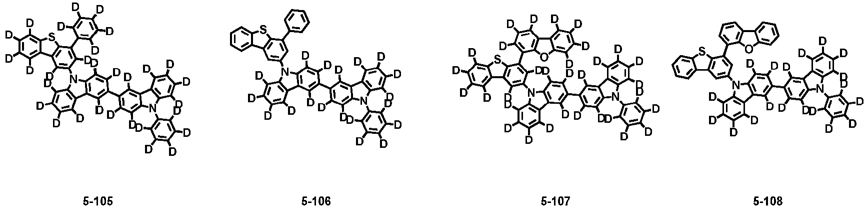

In one embodiment of the present application, Rd and Re are the same as or different from each other, and may be each independently hydrogen; deuterium; or a substituted or unsubstituted aryl group having 6 to 60 carbon atoms.

In one embodiment of the present application, Rd and Re are the same as or different from each other, and may be each independently hydrogen; deuterium; or a substituted or unsubstituted aryl group having 6 to 40 carbon atoms.

In one embodiment of the present application, Rd and Re are the same as or different from each other, and may be each independently hydrogen; deuterium; or a substituted or unsubstituted aryl group having 6 to 20 carbon atoms.

In one embodiment of the present application, Rd and Re are the same as or different from each other, and may be each independently hydrogen; deuterium; a substituted or unsubstituted phenyl group; or a substituted or unsubstituted biphenyl group.

In one embodiment of the present application, Rd and Re are the same as or different from each other, and may be each independently hydrogen; deuterium; a phenyl group; or a biphenyl group.

In one embodiment of the present application, Rd and Re are the same as or different from each other, and may be each independently hydrogen; or deuterium.

In one embodiment of the present application, Rd and Re are hydrogen.

In one embodiment of the present application, Rd and Re are deuterium.

In another embodiment, r is an integer of 0 to 7, and when r is 2 or greater, Rds are the same as or different from each other, and two or more Rds adjacent to each other may bond to each other to form a substituted or unsubstituted aliphatic or aromatic hydrocarbon ring or heteroring.

In one embodiment of the present application, R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms.

In one embodiment of the present application, R32 and R33 are the same as or different from each other, and each independently a substituted or unsubstituted silyl group; a substituted or unsubstituted aryl group having 6 to 40 carbon atoms; or a substituted or unsubstituted heteroaryl group having 2 to 40 carbon atoms.

In one embodiment of the present application, R32 and R33 are the same as or different from each other, and may be each independently a substituted or unsubstituted silyl group; a substituted or unsubstituted phenyl group; a substituted or unsubstituted biphenyl group; a substituted or unsubstituted terphenyl group; a substituted or unsubstituted triphenyl group; a substituted or unsubstituted naphthyl group; a substituted or unsubstituted dibenzothiophene group; or a substituted or unsubstituted dibenzofuran group.

According to one embodiment of the present application, Chemical Formula 14 may be represented by any one of the following compounds, but is not limited thereto.

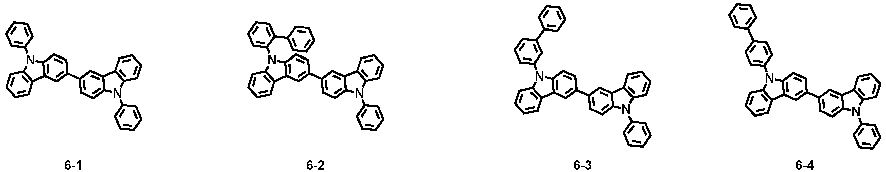

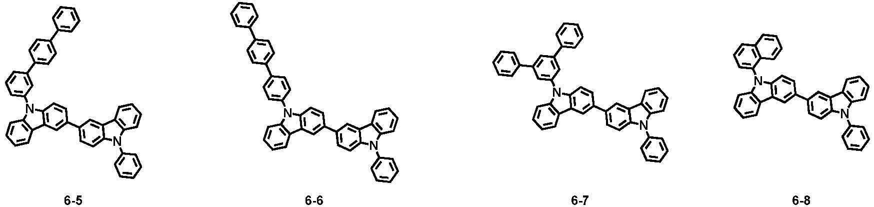

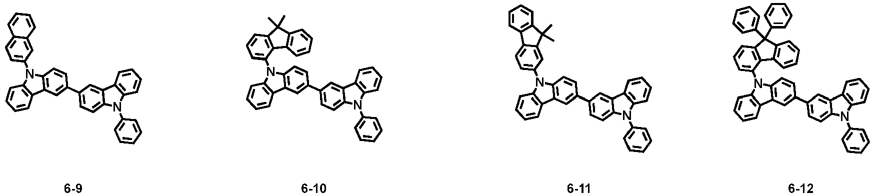

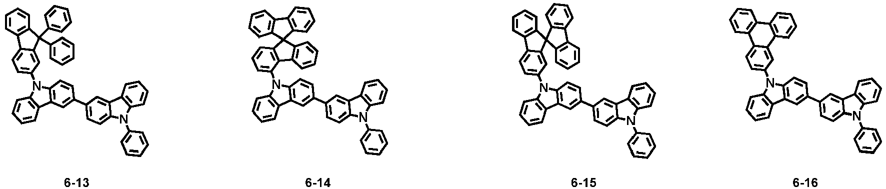

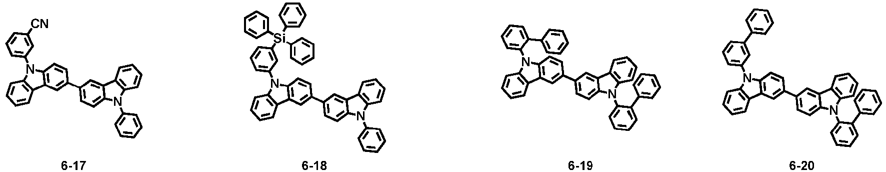

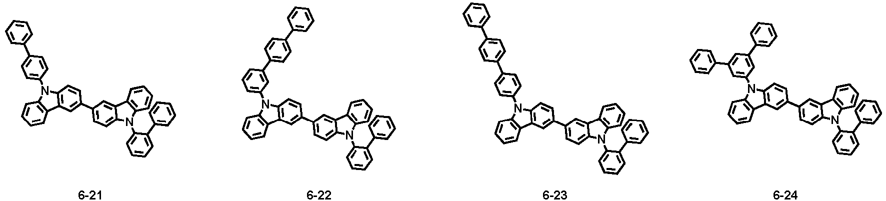

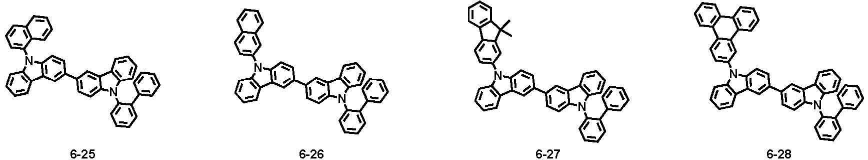

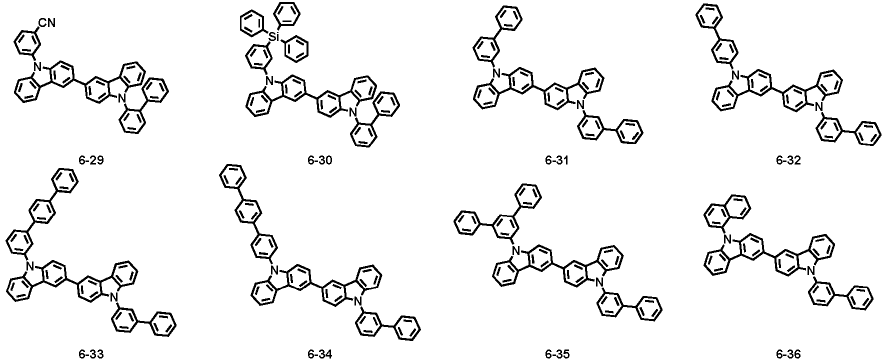

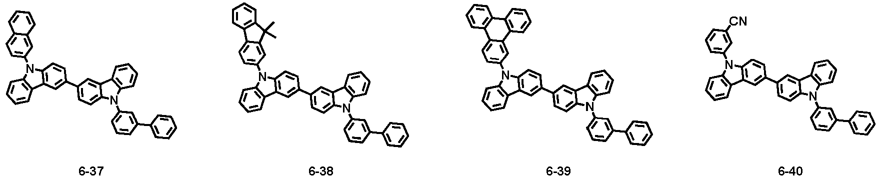

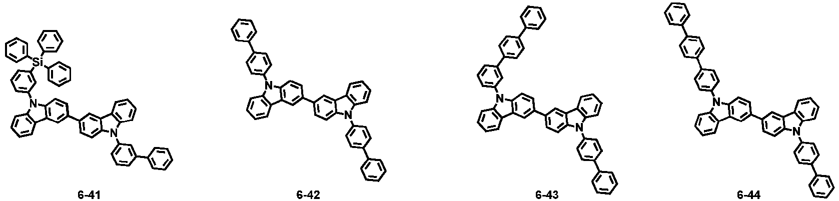

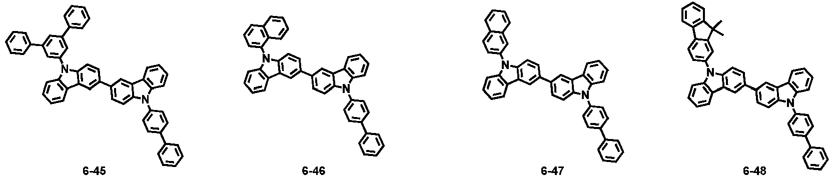

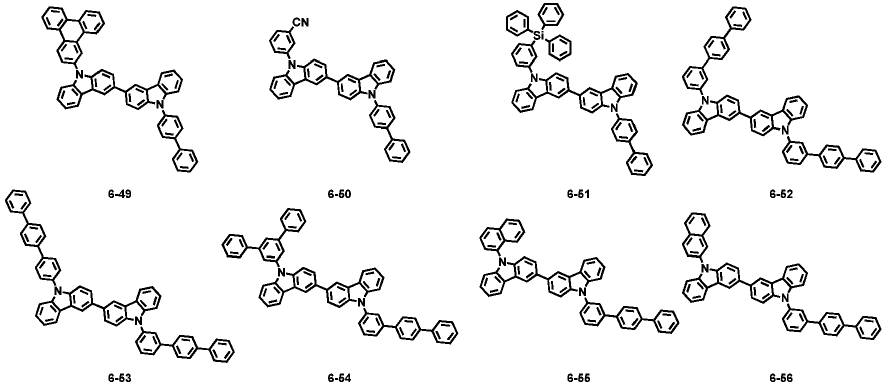

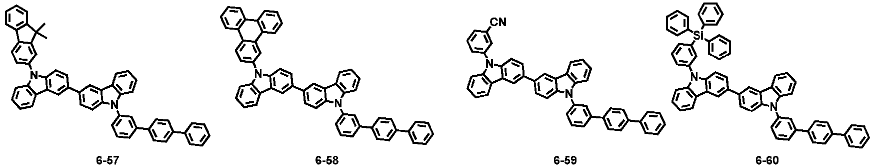

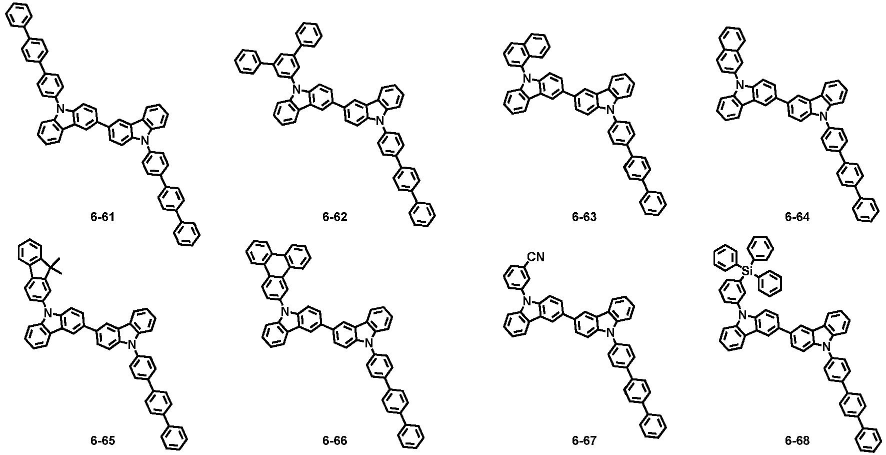

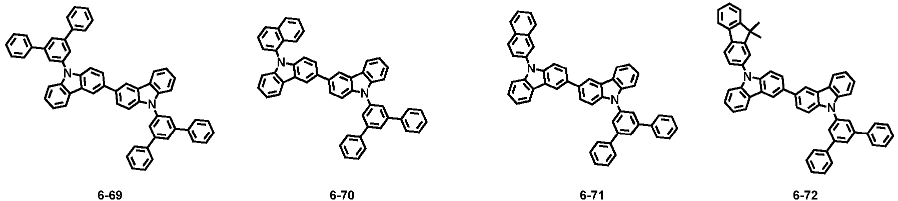

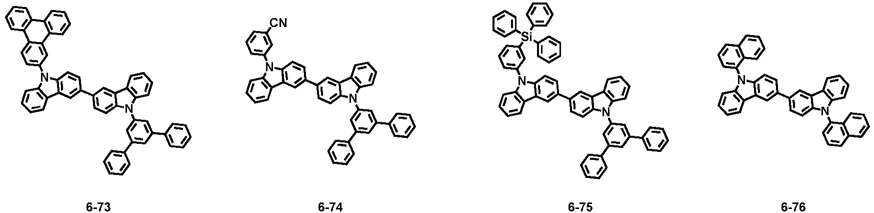

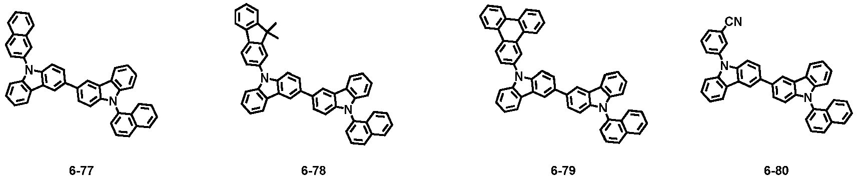

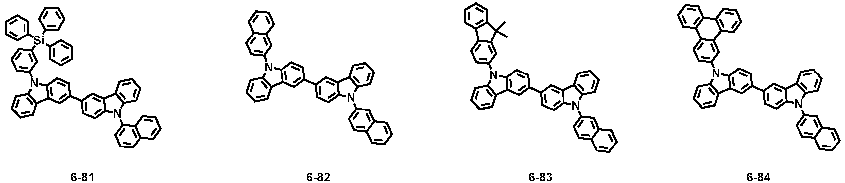

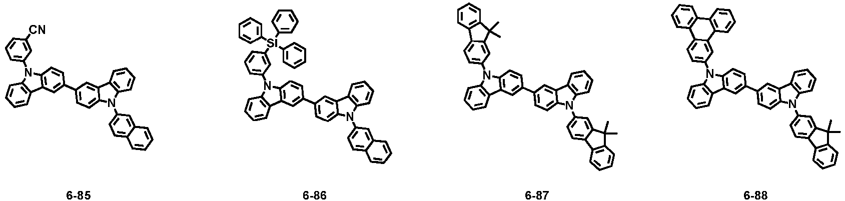

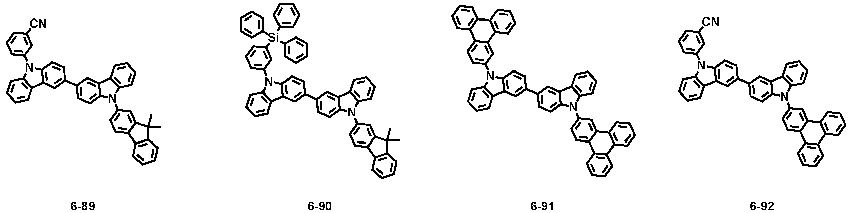

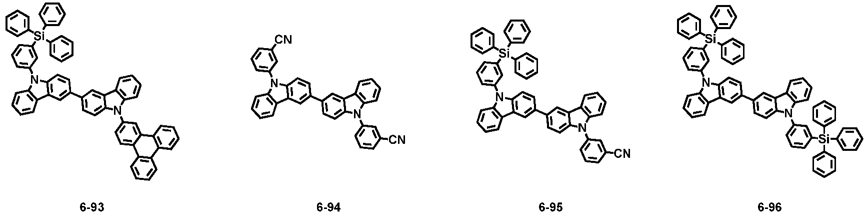

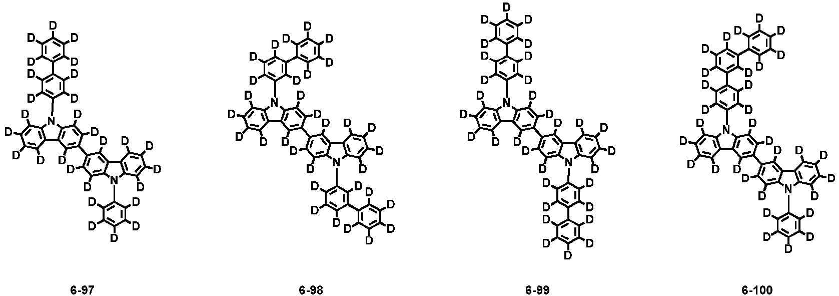

In addition, the organic material layer comprises one or more light emitting layers, and the light emitting layer comprises the heterocyclic compound represented by any one of Chemical Formula 1-2 to 1-4 or the heterocyclic compound represented by any one of the above compounds as a first compound, and further comprises the heterocyclic compound represented by Chemical Formula 13 or 14 as a second compound. When comprising the heterocyclic compound represented by any one of Chemical Formula 1-2 to 1-4 or the heterocyclic compound represented by any one of the above compounds in the light emitting layer among the organic material layers, the organic light emitting device has more superior light emission efficiency and lifetime.

In addition, by introducing various substituents to the structure of Chemical Formula 13 or 14, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 13 or 14, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the heterocyclic compound of Chemical Formula 13 or 14 may be prepared. More specifically, the heterocyclic compound according to one embodiment of the present application may be prepared based on preparation examples to describe later.

In addition, the organic material layer comprises one or more light emitting layers, and the light emitting layer further comprises one of the heterocyclic compounds represented by any one of Chemical Formulae 1-2 to 1-4 or the heterocyclic compound represented by any one of the above compounds and the heterocyclic compound represented by Chemical Formula 13. When comprising one of the heterocyclic compounds represented by any one of Chemical Formulae 1-2 to 1-4 or the heterocyclic compound represented by any one of the above compounds and the heterocyclic compound represented by Chemical Formula 13 in the light emitting layer among the organic material layers, the organic light emitting device has more superior light emission efficiency and lifetime due to an exciplex phenomenon.

In addition, the organic material layer comprises one or more light emitting layers, and the light emitting layer further comprises one of the heterocyclic compounds represented by any one of Chemical Formulae 1-2 to 1-4 or the heterocyclic compound represented by any one of the above compounds and the heterocyclic compound represented by Chemical Formula 14. When comprising one of the heterocyclic compounds represented by any one of Chemical Formulae 1-2 to 1-4 or the heterocyclic compound represented by any one of the above compounds and the heterocyclic compound represented by Chemical Formula 14 in the light emitting layer among the organic material layers, the organic light emitting device has more superior light emission efficiency and lifetime due to an exciplex phenomenon.

In the organic light emitting device of the present application, the organic material layer comprises a light emitting layer, and the light emitting layer may comprise the heterocyclic compound as a host material of a light emitting material.

In the organic light emitting device of the present application, the light emitting layer may comprise two or more host materials, and at least one of the host materials may comprise the heterocyclic compound as a host material of a light emitting material.

In the organic light emitting device of the present application, two or more host materials may be pre-mixed and used as the light emitting layer, and at least one of the two or more host materials may comprise the heterocyclic compound as a host material of a light emitting material.

The pre-mixing means placing and mixing two or more host materials of the light emitting layer in one source of supply before depositing on the organic material layer.

In the organic light emitting device of the present application, the light emitting layer may comprise two or more host materials, and the two or more host materials may each comprise one or more p-type host materials and n-type host materials, and at least one of the host materials may comprise the heterocyclic compound as a host material of a light emitting material. In this case, the organic light emitting device may have superior driving, efficiency and lifetime.

The organic light emitting device of the present disclosure may further comprise one, two or more layers selected from the group consisting of a light emitting layer, a hole injection layer, a hole transfer layer, an electron injection layer, an electron transfer layer, a hole auxiliary layer and a hole blocking layer.

The organic light emitting device according to one embodiment of the present application may be manufactured using common organic light emitting device manufacturing methods and materials except that the organic material layer is formed using the heterocyclic compound described above.

In addition, another embodiment of the present application provides a composition for an organic material layer of an organic light emitting device, the composition comprising the heterocyclic compound represented by any one of Chemical Formula 1-2 to 1-4 and the heterocyclic compound represented by Chemical Formula 13 or 14.

In another embodiment of the present application, the heterocyclic compound represented by any one of Chemical Formula 1-2 to 1-4:the heterocyclic compound represented by Chemical Formula 13 or 14 may have a weight ratio of 1:10 to 10:1, 1:8 to 8:1, 1:5 to 5:1 or 1:2 to 2:1 in the composition, however, the weight ratio is not limited thereto.

Specific descriptions on the heterocyclic compound represented by any one of Chemical Formula 1-2 to 1-4 and the compound represented by Chemical Formula 13 or 14 are the same as the descriptions provided above.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

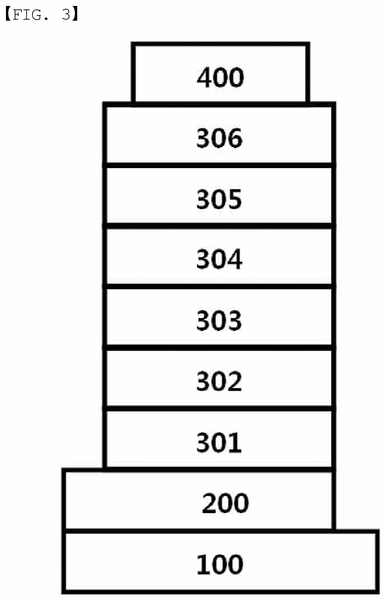

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

In the organic light emitting device according to one embodiment of the present application, materials other than the heterocyclic compound of any one of Chemical Formulae 1-2 to 1-4 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino) phenyl] benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Target Compound 1-86

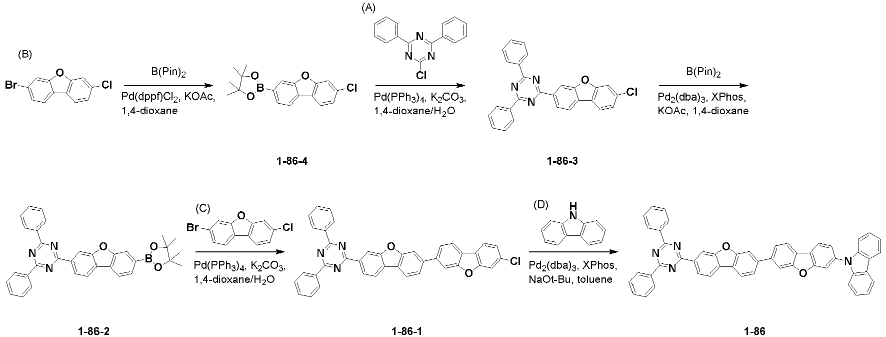

### 1) Preparation of Compound 1-86-4

3-Bromo-7-chlorodibenzo[b,d]furan (B) (16.8 g, 59.8 mM), B(Pin)₂ (22.8 g, 89.7 mM), Pd(dppf)Cl₃ (2.2 g, 3.0 mM) and KOAc (17.6 g, 179.4 mM) were dissolved in 1,4-dioxane (300 mL), and refluxed for 12 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:Hex=1:2) to obtain Compound 1-86-4 (35.8 g, 82%). The Hex means hexane.

### 2) Preparation of Compound 1-86-3

Compound 1-86-4 (35.8 g, 49.0 mM), 2-chloro-4,6-diphenyl-1,3,5-triazine (A) (15.7 g, 58.8 mM), Pd(PPh₃)₄ (2.8 g, 2.4 mM), and K₂CO₃ (13.5 g, 98.0 mM) were dissolved in 1,4-dioxane/H₂O (600 mL/120 mL), and refluxed for 12 hours. After the reaction was completed, the result was extracted by introducing distilled water and dichloromethane (DCM) thereto at room temperature, and after drying the organic layer with MgSO₄, the solvent was removed using a rotary evaporator. The reaction material was recrystallized with methanol to obtain Compound 1-86-3 (18.2 g, 86%).

### 3) Preparation of Compound 1-86-2

Compound 1-86-3 (18.2 g, 41.9 mM), B(Pin)₂ (16.0 g, 62.8 mM), Pd₂(dba)₃ (1.9 g, 2.1 mM), XPhos (2.0 g, 4.2 mM) and KOAc (12.3 g, 125.7 mM) were dissolved in 1,4-dioxane (300 mL), and refluxed for 12 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:Hex=1:2) to obtain Compound 1-86-2 (17.8 g, 81%).

### 4) Preparation of Compound 1-86-1

Compound 1-86-2 (17.8 g, 33.9 mM), 3-bromo-7-chlorodibenzo[b,d]furan (C) (11.4 g, 40.7 mM), Pd(PPh₃)₄ (2.0 g, 1.7 mM) and K₂CO₃ (9.3 g, 67.8 mM) were dissolved in 1,4-dioxane/H₂O (300 mL/60 mL), and refluxed for 12 hours. After the reaction was completed, the result was extracted by introducing distilled water and dichloromethane (DCM) thereto at room temperature, and after drying the organic layer with MgSO₄, the solvent was removed using a rotary evaporator. The reaction material was recrystallized with methanol to obtain Compound 1-86-1 (17.7 g, 87%).

### 5) Preparation of Target Compound 1-86

Compound 1-86-1 (17.7 g, 29.5 mM), 9H-carbazole (D) (5.9 g, 35.4 mM), Pd₂(dba)₃ (1.4 g, 1.5 mM), XPhos (1.4 g, 3.0 mM) and NaOt-Bu (5.7 g, 59.0 mM) were dissolved in 1,4-dioxane (300 mL), and refluxed for 12 hours. After the reaction was completed, the result was extracted by introducing distilled water and dichloromethane (DCM) thereto at room temperature, and after drying the organic layer with MgSO₄, the solvent was removed using a rotary evaporator. The reaction material was recrystallized with methanol to obtain target Compound 1-86 (19.2 g, 89%).

Target Compounds of the following Table 1 were synthesized in the same manner as in Preparation Example 1 except that Intermediate A of the following Table 1 was used instead of 2-chloro-4,6-diphenyl-1,3,5-triazine in Preparation Example 1-2), Intermediate B was used instead of 3-bromo-7-chlorodibenzo[b,d]furan in Preparation Example 1-1), Intermediate C was used instead of 3-bromo-7-chlorodibenzo[b,d]furan in Preparation Example 1-4), and Intermediate D was used instead of 9H-carbazole in Preparation Example 1-5).

The following example compounds 1-1 and 1-267 are not according to the invention and are present for illustration purposes only.[Table 1]

| Compound No. | Intermediate A | Intermediate B | Intermediate C | Intermediate D | Target Compound |
|---|---|---|---|---|---|
| 1-1 (not according to the invention) | 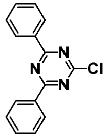 | 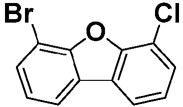 | | 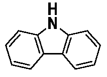 | 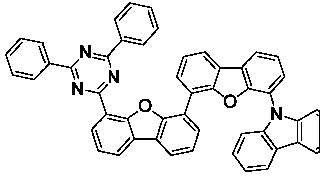 |
| 1-87 | | 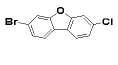 | 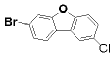 | | 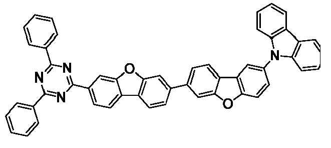 |
| 1-94 | | | 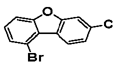 | | 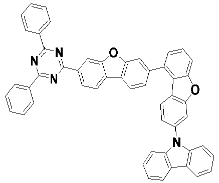 |
| 1-95 | | | 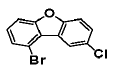 | | 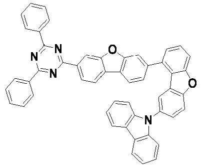 |
| 1-102 | | 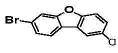 | 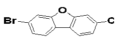 | | 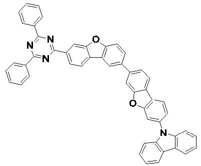 |
| 1-103 | | | 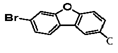 | | 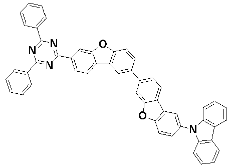 |
| 1-110 | | | 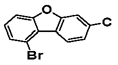 | | 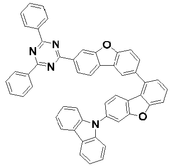 |
| 1-111 | | | 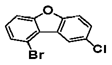 | | 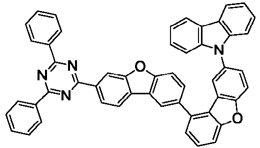 |
| 1-214 | | 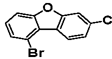 | 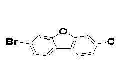 | | 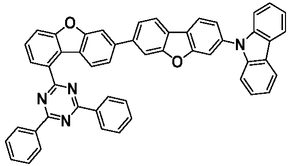 |
| 1-215 | | | 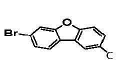 | | 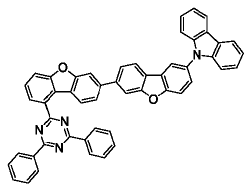 |
| 1-222 | | | 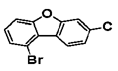 | | 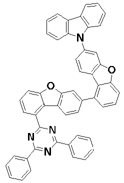 |
| 1-223 | | | 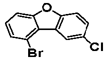 | | 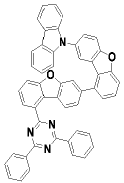 |
| 1-230 | | 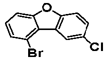 | 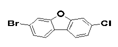 | | 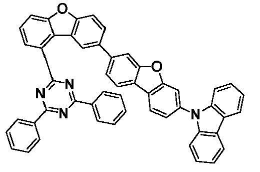 |
| 1-231 | | | 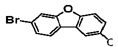 | | 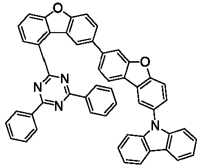 |
| 1-238 | | | 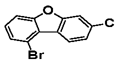 | | 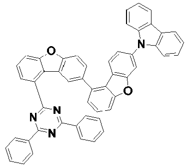 |
| 1-239 | | | 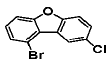 | | 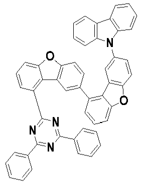 |
| 1-259 | | 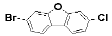 | 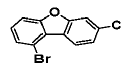 | 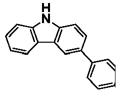 | 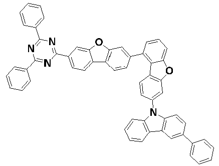 |
| 1-263 | | 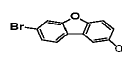 | 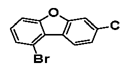 | 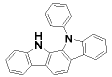 | 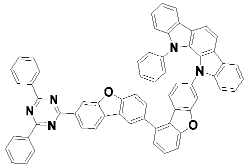 |
| 1-264 | | | 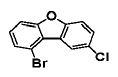 | 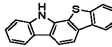 | 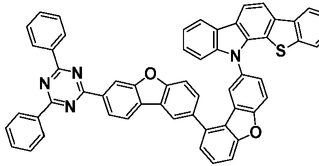 |
| 1-267 (not according to the invention) | | 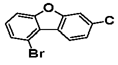 | 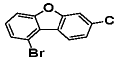 | 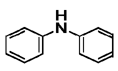 | 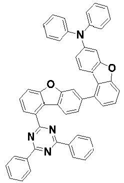 |
| 1-270 | 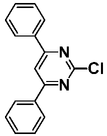 | 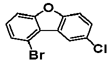 | 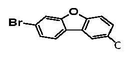 | 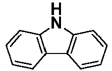 | 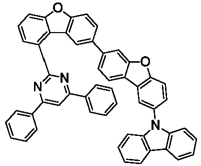 |
| 2-11 | 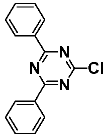 | 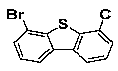 | 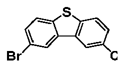 | | 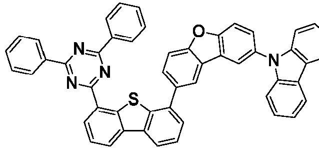 |
| 2-86 | | 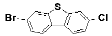 | 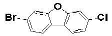 | | 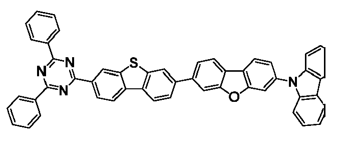 |
| 2-87 | | | 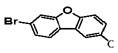 | | 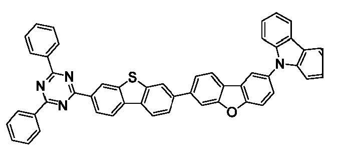 |
| 3-102 | | 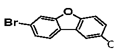 | 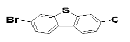 | | 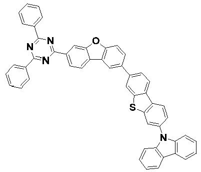 |
| 3-214 | | 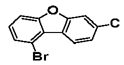 | | | 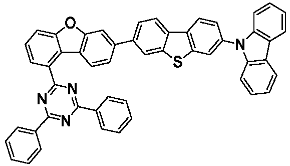 |
| 4-230 | | 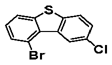 | | | 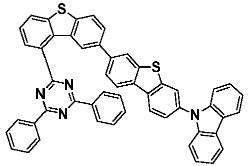 |
| 4-239 | | | 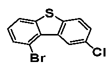 | | 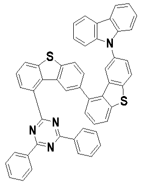 |

### <Preparation Example 2> Preparation of Target Compound 5-2

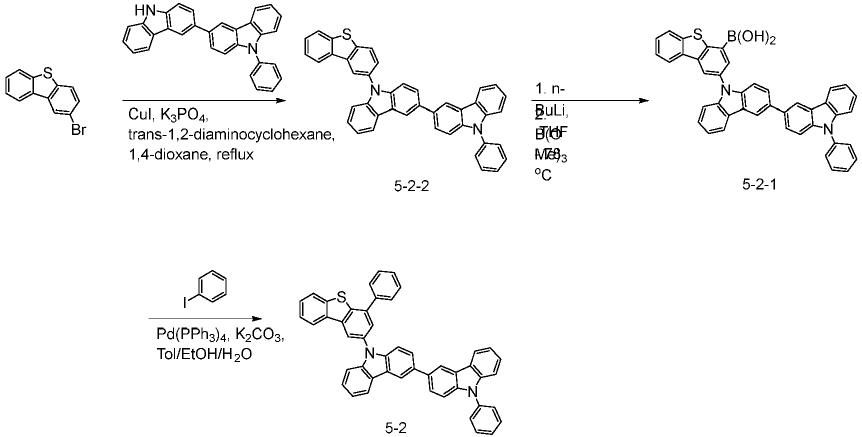

### 1) Preparation of Compound 5-2-2

2-Bromodibenzo[b,d]thiophene (4.2 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) were dissolved in 1,4-dioxane (100 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:Hex=1:3), and recrystallized with methanol to obtain Compound 5-2-2 (7.9 g, 85%).

### 2) Preparation of Compound 5-2-1

To a mixture solution of Compound 5-2-1 (8.4 g, 14.3 mMol) and tetrahydrofuran (THF) (100 mL), 2.5 M n-BuLi (7.4 mL, 18.6 mmol) was added dropwise at -78°C, and the result was stirred for 1 hour at room temperature. Trimethyl borate (4.8 mL, 42.9 mmol) was added dropwise to the reaction mixture, and the result was stirred for 2 hours at room temperature. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:MeOH=100:3), and recrystallized with dichloromethane (DCM) to obtain Compound 5-2-1 (3.9 g, 70%). The MeOH means methanol.

### 3) Preparation of Target Compound 5-2

Compound 5-2-1 (6.7 g, 10.5 mM), iodobenzene (2.1 g, 10.5 mM), Pd(PPh₃)₄ (606 mg, 0.52 mM) and K₂CO₃ (2.9 g, 21.0 mM) were dissolved in toluene/ethanol/water (toluene/EtOH/H₂O) (100 mL/20 mL/20 mL), and refluxed for 12 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 5-2 (4.9 g, 70%).

### <Preparation Example 3> Preparation of Target Compound 5-3

Target Compound 5-3 (83%) was obtained in the same manner as Preparation of Compound 5-2 of Preparation Example 2 except that 4-iodo-1,1'-biphenyl was used instead of iodobenzene.

### <Preparation Example 4> Preparation of Target Compound 5-12

Target Compound 5-12 (80%) was obtained in the same manner as Preparation of Compound 5-2 of Preparation Example 2 except that 4-iododibenzo[b,d]furan was used instead of iodobenzene.

### <Preparation Example 5> Preparation of Target Compound 6-3

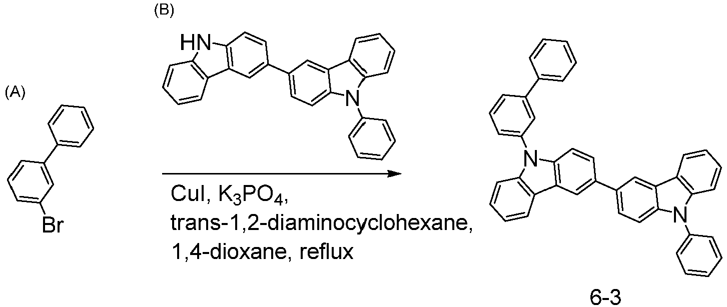

### 1) Preparation of Target Compound 6-3

3-Bromo-1,1'-biphenyl (A) (3.7 g, 15.8 mM), 9-phenyl-9H,9'H-3,3'-bicarbazole (B) (6.5 g, 15.8 mM), CuI (3.0 g, 15.8 mM), trans-1,2-diaminocyclohexane (1.9 mL, 15.8 mM) and K₃PO₄ (3.3 g, 31.6 mM) were dissolved in 1,4-dioxane (100 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:Hex=1:3), and recrystallized with methanol (MeOH) to obtain target Compound 6-3 (7.5 g, 85%).

Target Compounds were synthesized in the same manner as in Preparation Example 5 except that Intermediate A of the following Table 2 was used instead of 3-bromo-1,1'-biphenyl, and Intermediate B of the following Table 2 was used instead of 9-phenyl-9H,9'H-3,3'-bicarbazole.

**[Table 2]**

| Compound No. | Intermediate A | Intermediate B | Target Compound |
|---|---|---|---|
| 6-4 | 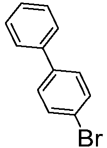 | 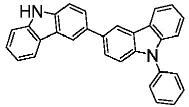 | 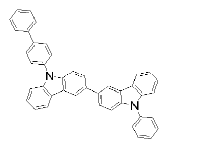 |
| 6-7 | 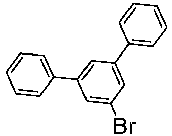 | | 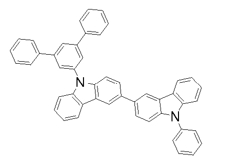 |
| 6-9 | 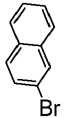 | | 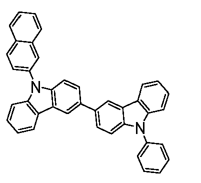 |
| 6-31 | 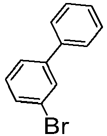 | 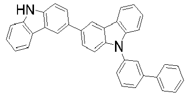 | 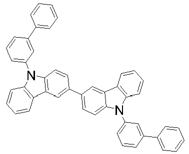 |
| 6-32 | 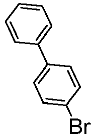 | | 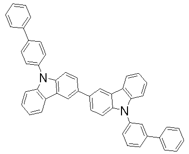 |
| 6-42 | 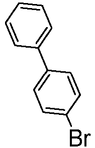 | 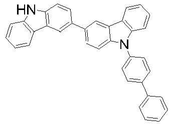 | 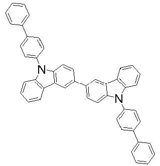 |

### <Preparation Example 6> Preparation of Target Compound 1-273

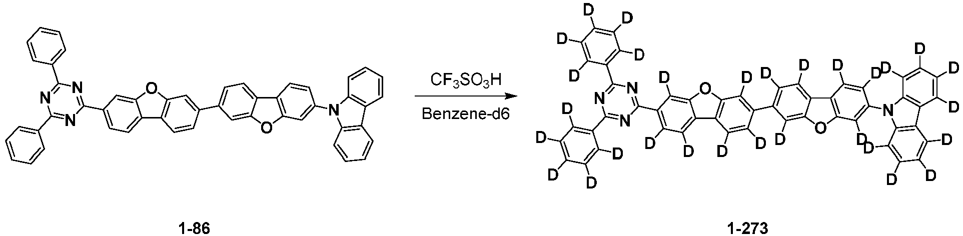

### 1) Preparation of Target Compound 1-273

Compound 1-86 (7.7 g, 10.5 mM) was dissolved in triflic acid (23 mL, 262.5 mM) and benzene-d6 (70 mL), and refluxed at 50°C. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 1-273 (6.2 g, 78%).

### <Preparation Example 7> Preparation of Target Compound 5-105

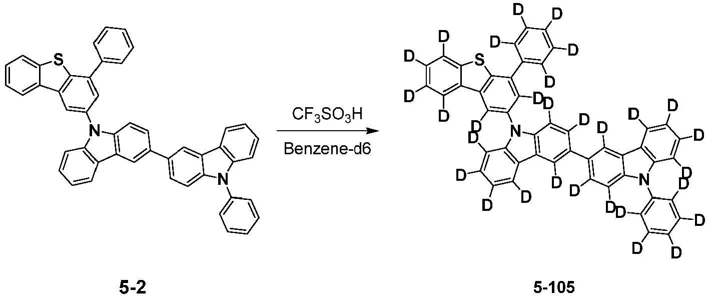

### 1) Preparation of Target Compound 5-105

Target Compound 5-105 (80%) was obtained in the same manner as in Preparation of Compound 1-273 of Preparation Example 6 except that Compound 5-2 was used instead of Compound 1-86.

### <Preparation Example 8> Preparation of Target Compound 6-99

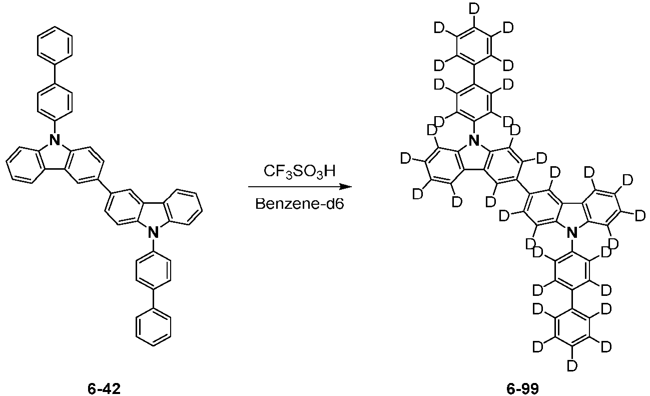

### 1) Preparation of Target Compound 6-99

Target Compound 6-99 (79%) was obtained in the same manner as in Preparation of Compound 1-273 of Preparation Example 6 except that Compound 6-42 was used instead of Compound 1-86.

Compounds other than the compounds described in Preparation Example 1 to Preparation Example 8 and Table 1 and Table 2 were also prepared in the same manner as in the methods described in the preparation examples described above.

Synthesis identification results of the prepared compounds are shown in the following Table 3 and Table 4. The following Table 3 shows measurement values of ¹H NMR (CDCl₃, 200 Mz), and the following Table 4 shows measurement values of FD-mass spectrometry (FD-MS: field desorption mass spectrometry).

The following example compounds 1-1 and 1-267 are not according to the invention and are present for illustration purposes only.

**[Table 3]**

| Compound No. | ¹H NMR (CDCl₃, 200 Mz) |
|---|---|
| 1-1 (not according to the invention) | δ=8.55 (1H, d), 8.36 (4H, m), 8.19 (1H, d), 8.08-8.02 (5H, m), 7.94-7.88 (3H, m), 7.58-7.46 (12H, m), 7.35-7.31 (2H, m), 7.20-7.16 (2H, m) |
| 1-86 | δ=8.55 (1H, d), 8.36 (4H, m), 8.19 (1H, d), 8.03-7.94 (5H, m), 7.82-7.76 (6H, m), 7.58-7.50 (9H, m), 7.35 (1H, t), 7.25-7.16 (3H, m) |
| 1-87 | δ=8.55 (1H, d), 8.36 (4H, m), 8.19 (1H, d), 8.03 (3H, d), 7.94 (1H, d), 7.82-7.74 (7H, m), 7.61-7.50 (9H, m), 7.35-7.31 (2H, m), 7.20-7.16 (2H, m) |
| 1-94 | δ=8.55 (1H, d), 8.36 (4H, m), 8.19 (1H, d), 8.03-7.94 (4H, m), 7.82-7.76 (5H, m), 7.69 (1H, m), 7.58-7.50 (10H, m), 7.35 (1H, t), 7.25-7.16 (3H, m) |
| 1-95 | δ=8.55 (1H, d), 8.36 (4H, m), 8.19 (1H, d), 8.03 (2H, m), 7.94 (1H, d), 7.82-7.69 (7H, m), 7.61-7.50 (10H, m), 7.35-7.31 (2H, m), 7.20-7.16 (2H, m) |
| 1-102 | δ=8.55 (1H, d), 8.36 (4H, m), 8.19 (1H, d), 8.03-7.76 (11H, m), 7.58-7.50 (9H, m), 7.35 (1H, t), 7.25-7.16 (3H, m) |
| 1-103 | δ=8.55 (1H, d), 8.36 (4H, m), 8.19 (1H, d), 8.03 (2H, d), 7.94-7.74 (9H, m), 7.61-7.50 (9H, m), 7.35-7.31 (2H, m), 7.20-7.16 (2H, m) |
| 1-110 | δ=8.55 (1H, d), 8.36 (4H, m), 8.19 (1H, d), 8.03-7.76 (9H, m), 7.69 (1H, d), 7.58-7.50 (10H, m), 7.35 (1H, t), 7.25-7.16 (3H, m) |
| 1-111 | δ=8.55 (1H, d), 8.36 (4H, m), 8.19 (1H, d), 8.03 (1H, d), 7.94-7.69 (9H, m), 7.61-7.50 (10H, m), 7.35-7.31 (2H, m), 7.20-7.16 (2H, m) |
| 1-214 | δ=8.55 (1H, d), 8.36 (4H, m), 8.19 (1H, d), 8.03-7.94 (4H, m), 7.82-7.76 (5H, m), 7.69 (1H, m), 7.58-7.50 (10H, m), 7.35 (1H, t), 7.25-7.16 (3H, m) |
| 1-215 | δ=8.55 (1H, d), 8.36 (4H, m), 8.19 (1H, d), 8.03 (2H, d), 7.94 (1H, d), 7.82-7.69 (7H, m), 7.58-7.50 (10H, m), 7.35-7.31 (2H, m), 7.20-7.16 (2H, m) |
| 1-222 | δ=8.55 (1H, d), 8.36 (4H, m), 8.19 (1H, d), 8.03-7.94 (3H, m), 7.82-7.76 (4H, m), 7.69 (2H, d), 7.58-7.50 (11H, m), 7.35 (1H, t), 7.25-7.16 (3H, m) |
| 1-223 | δ=8.55 (1H, d), 8.36 (4H, m), 8.19 (1H, d), 8.03 (1H, d), 7.94 (1H, d), 7.82-7.57 (7H, m), 7.61-7.50 (11H, m), 7.35-7.31 (2H, m), 7.20-7.16 (2H, m) |
| 1-230 | δ=8.55 (1H, d), 8.36 (4H, m), 8.19 (1H, d), 8.03-7.76 (9H, m), 7.69 (1H, d), 7.58-7.50 (10H, m), 7.35 (1H, m), 7.25-7.16 (3H, m) |
| 1-231 | δ=8.55 (1H, d), 8.36 (4H, m), 8.19 (1H, d), 8.03 (1H, d), 7.94-7.69 (9H, m), 7.59 -7.50 (10H, m), 7.35-7.31 (2H, m), 7.20-7.16 (2H, m) |
| 1-238 | δ=8.55 (1H, d), 8.36 (4H, m), 8.19 (1H, d), 7.98-7.79 (7H, m), 7.69 (2H, m), 7.58-7.50 (10H, m), 7.35 (1H, m), 7.25-7.16 (3H, m) |
| 1-239 | δ=8.55 (1H, d), 8.36 (4H, m), 8.19 (1H, d), 7.94-7.69 (9H, m), 7.61-7.50 (11H, m), 7.35-7.31 (2H, m), 7.20-7.16 (2H, m) |
| 1-259 | δ=8.55 (1H, d), 8.36 (4H, m), 8.03-7.89 (6H, m), 7.82-7.69 (9H, m), 7.57-7.35 (12H, m), 7.25 (1H, d), 7.16 (1H, d) |
| 1-263 | δ=8.55 (2H, d), 8.36 (4H, m), 8.12 (1H, d), 8.03-7.76 (10H, m), 7.69-7.50 (15H, m), 7.35 (2H, t), 7.25 (1H, d), 7.16 (2H, m) |
| 1-264 | δ=8.55 (1H, d), 8.45 (1H, d), 8.36 (4H, m), 8.05-8.03 (2H, m), 7.93-7.69 (10H, m), 7.61-7.49 (11H, m), 7.35-7.31 (2H, m), 7.16 (1H, m) |
| 1-267 (not according to the invention) | δ=8.36 (4H, m), 8.03 (2H, m), 7.82-7.76 (5H, m), 7.69 (2H, m), 7.57-7.50 (8H, m), 7.24 (4H, m), 7.08-7.00 (6H, m) |
| 1-270 | δ=8.55 (1H, d), 8.23 (1H, s), 8.19 (1H, d), 8.03 (1H, d), 7.94-7.69 (13H, m), 7.61-7.49 (10H, m), 7.35-7.31 (2H, m), 7.20-7.16 (2H, m) |
| 2-11 | δ=8.55 (3H, d), 8.36-8.32 (5H, m), 8.19 (1H, m), 7.94-7.70 (8H, m), 7.61-7.50 (9H, m), 7.35-7.31 (2H, m), 7.20-7.16 (2H, m) |
| 2-86 | δ=8.55 (1H, d), 8.36 (4H, m), 8.24-8.17 (6H, m), 8.03-7.94 (4H, m), 7.82-7.76 (2H, m), 7.58-7.50 (9H, m), 7.35 (1H, t), 7.25-7.16 (3H, m) |
| 2-87 | δ=8.55 (1H, d), 8.36 (4H, m), 8.24-8.17 (6H, m), 8.03 (1H, d), 7.94 (2H, d), 7.82-7.74 (3H, m), 7.61-7.50 (9H, m), 7.35-7.31 (2H, m), 7.20-7.16 (2H, m) |
| 3-102 | δ=8.55 (1H, d), 8.36 (4H, m), 8.24-8.17 (5H, m), 8.03-7.76 (8H, m), 7.58-7.45 (9H, m), 7.35 (1H, t), 7.20-7.16 (2H, m) |
| 3-214 | δ=8.55 (1H, d), 8.36 (4H, m), 8.24-8.17 (5H, m), 8.03-7.94 (3H, m), 7.82-7.76 (3H, m), 7.69 (1H, m), 7.58-7.45 (10H, m), 7.35 (1H, t), 7.20-7.16 (2H, m) |
| 4-230 | δ=8.55 (1H, d), 8.36 (4H, m), 8.24-8.12 (7H, m), 8.03-7.94 (5H, m), 7.68 (1H, t), 7.58-7.45 (9H, m), 7.35 (1H, m), 7.20-7.16 (2H, m) |
| 4-239 | δ=8.55 (1H, d), 8.36 (4H, m), 8.19-7.90 (11H, m), 7.68 (2H, t), 7.58-7.35 (10H, m), 7.20-7.16 (2H, m) |
| 5-2 | δ=8.55(1H, d), 8.45(1H, d), 8.30(1H, d), 8.19(1H, d), 8.13(1H, d), 8.00~7.89(6H, m), 7.77(2H, m), 7.62~7.35(15H, m), 7.20~7.16(2H, m) |
| 5-3 | δ=8.55(1H, d), 8.45(1H, d), 8.30(1H, d), 8.19(1H, d), 8.13(1H, d), 8.00~7.89(6H, m), 7.77-7.75(4H, m), 7.62~7.35(13H, m), 7.25~7.16(6H, m) |
| 5-12 | δ=8.55(1H, d), 8.45(1H, d), 8.30(1H, d), 8.19~7.89(11H, m), 7.77(2H, m), 7.62-7.31(14H, m), 7.20~7.16 (2H, m) |
| 6-3 | δ=8.55 (1H, d), 8.30 (1H, d), 8.21-8.13 (3H, m), 7.99-7.89 (4H, m), 7.77-7.35 (17H, m), 7.20-7.16 (2H, m) |
| 6-4 | δ=8.55 (1H, d), 8.30(1H, d), 8.19-8.13 (2H, m), 7.99-7.89(8H, m), 7.77-7.75 (3H, m), 7.62-7.35 (11H, m), 7.20-7.16 (2H, m) |
| 6-7 | δ=8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 6-9 | δ=8.55 (1H, d), 8.30 (1H, d), 8.19-8.13 (2H, m), 8.03-7.77 (9H, m), 7.62-7.50 (9H, m), 7.36-7.35 (2H, m), 7.20-7.16 (2H, m) |
| 6-31 | δ=8.55 (1H, d), 8.30 (1H, d), 8.21-8.13 (4H, m), 7.99-7.89 (4H, m), 7.77-7.35 (20H, m), 7.20-7.16 (2H, m) |
| 6-32 | δ=8.55 (1H, d), 8.30 (1H, d), 8.21-8.13 (3H, m), 7.99-7.89 (8H, m), 7.77-7.35 (17H, m), 7.20-7.16 (2H, m) |
| 6-42 | δ=8.55 (1H, d), 8.30 (1H, d), 8.19 (1H, d), 8.13 (1H, d), 7.99-7.89 (12H, m), 7.77-7.75 (5H, m), 7.58 (1H, d), 7.50-7.35 (8H, m), 7.20-7.16 (2H, m) |

**[Table 4]**

| Compound | FD-MS | Compound | FD-MS |
|---|---|---|---|
| 1-1 (not according to the invention) | m/z=730.24 (C₅₁H₃₀N₄O₂=730.83) | 1-86 | m/z=730.24 (C₅₁H₃₀N₄O₂=730.83) |
| 1-87 | m/z=730.24 (C₅₁H₃₀N₄O₂=730.83) | 1-94 | m/z=730.24 (C₅₁H₃₀N₄O₂=730.83) |
| 1-95 | m/z=730.24 (C₅₁H₃₀N₄O₂=730.83) | 1-102 | m/z=730.24 (C₅₁H₃₀N₄O₂=730.83) |
| 1-103 | m/z=730.24 (C₅₁H₃₀N₄O₂=730.83) | 1-110 | m/z=730.24 (C₅₁H₃₀N₄O₂=730.83) |
| 1-111 | m/z=730.24 (C₅₁H₃₀N₄O₂=730.83) | 1-214 | m/z=730.24 (C₅₁H₃₀N₄O₂=730.83) |
| 1-215 | m/z=730.24 (C₅₁H₃₀N₄O₂=730.83) | 1-222 | m/z=730.24 (C₅₁H₃₀N₄O₂=730.83) |
| 1-223 | m/z=730.24 (C₅₁H₃₀N₄O₂=730.83) | 1-230 | m/z=730.24 (C₅₁H₃₀N₄O₂=730.83) |
| 1-231 | m/z=730.24 (C₅₁H₃₀N₄O₂=730.83) | 1-238 | m/z=730.24 (C₅₁H₃₀N₄O₂=730.83) |
| 1-239 | m/z=730.24 (C₅₁H₃₀N₄O₂=730.83) | 1-259 | m/z=806.27 (C₅₇H₃₄N₄O₂=806.93) |
| 1-263 | m/z=895.29 (C₆₃H₃₇N₅O₂=896.02) | 1-264 | m/z=836.22 (C₅₇H₃₂N₄O₂S=836.97) |
| 1-267 (not according to the invention) | m/z=732.25 (C₅₁H₃₂N₄O₂=732.84) | 1-270 | m/z=729.24 (C₅₂H₃₁N₃O₂=729.84) |
| 1-273 | m/z=760.43 (C₅₁D₃₀N₄O₂=761.01) | 2-11 | m/z=746.21 (C₅₁H₃₀N₄OS=746.89) |
| 2-86 | m/z=746.21 (C₅₁H₃₀N₄OS=74 6. 89) | 2-87 | m/z=746.21 (C₅₁H₃₀N₄OS=746.89) |
| 3-102 | m/z=746.21 (C₅₁H₃₀N₄OS=74 6. 89) | 3-214 | m/z=746.21 (C₅₁H₃₀N₄OS=746.89) |
| 4-230 | m/z=762.19 (C₅₁H₃₀N₄S₂=762.95) | 4-239 | m/z=762.19 (C₅₁H₃₀N₄S₂=762.95) |
| 5-2 | m/z=666.21 (C₄₈H₃₀N₂S=666.84) | 5-3 | m/z=742.24 (C₅₄H₃₄N₂S=742.94) |
| 5-12 | m/z=756.22 (C₅₄H₃₂N₂OS=756.92) | 5-105 | m/z=696.40 (C₄₈D₃₀N₂S=697.03) |
| 6-3 | m/z=560.23 (C₄₂H₂₈N₂=560.70) | 6-4 | m/z=560.23 (C₄₂H₂₈N₂=560.70) |
| 6-7 | m/z=636.26 (C₄₈H₃₂N₂=636.80) | 6-9 | m/z=534.21 (C₄₀H₂₆N₂=534. 6 6) |
| 6-31 | m/z=636.26 (C₄₈H₃₂N₂=636.80) | 6-32 | m/z=636.26 (C₄₈H₃₂N₂=636.80) |
| 6-42 | m/z=636.26 (C₄₈H₃₂N₂=636.80) | 6-99 | m/z=668.46 (C₄₈D₃₂N₂=668.99) |

### <Experimental Example 1>

### (1) Manufacture of Organic Light Emitting Device

A glass substrate on which indium tin oxide (ITO) was coated as a thin film to a thickness of 150 nm (1,500 Å) was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, the compound represented by any of the Chemical Formulae 1-2 to 1-4 or represented by any of the specific examples aboveof the present disclosure described in the following Table 5 was deposited to 40 nm (400 Å) as a host, and a green phosphorescent dopant [Ir(ppy)₃] was doped by 7 wt% of the deposited thickness of the light emitting layer and deposited. After that, BCP (bathocuproine) was deposited to a thickness of 6 nm (60 Å) as a hole blocking layer, and Alq₃ was deposited to a thickness of 20 nm (200 Å) thereon as an electron transfer layer.

Lastly, an electron injection layer was formed on the electron transfer layer by depositing lithium fluoride (LiF) to a thickness of 1 nm (10 Å), and then a cathode was formed on the electron injection layer by depositing an aluminum (Al) cathode to a thickness of 120 nm (1,200 Å), and as a result, an organic light emitting device was manufactured (Examples 1 to 29 and Comparative Examples 1 to 8).

Meanwhile, all the organic compounds required to manufacture the organic light emitting device were vacuum sublimation purified under approximately 1.33 × 10⁻⁶ to 1.33 × 10⁻⁴ Pa (10⁻⁸ torr to 10⁻⁶ torr) for each material to be used in the organic light emitting device manufacture.

### (2) Driving Voltage and Light Emission Efficiency of Organic Light Emitting Device

For each of the organic light emitting devices of Examples 1 to 29 and Comparative Examples 1 to 8 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. Herein, T₉₀ means a lifetime (unit: h, time) taken to become 90% with respect to initial luminance.

Results of measuring driving voltage, light emission efficiency, color (EL color) and lifetime of the organic light emitting devices manufactured according to the present disclosure are as shown in the following Table 5.

The following Examples 1 and 21 are not according to the invention and are present for illustration purposes only.

**[Table 5]**

| | Light Emitting Layer Compound | Driving Voltage (V) | Efficiency (cd/A) | Color (EL color) | Lifetime (T₉₀) |
|---|---|---|---|---|---|
| Example 1 (not according to the invention) | 1-1 | 4.91 | 69.8 | Green | 222 |
| Example 2 | 1-86 | 4.33 | 74.2 | | 405 |
| Example 3 | 1-87 | 4.35 | 79.2 | | 382 |
| Example 4 | 1-94 | 4.66 | 71.1 | | 347 |
| Example 5 | 1-95 | 4.69 | 70.9 | | 271 |
| Example 6 | 1-102 | 4.67 | 71.2 | | 390 |
| Example 7 | 1-103 | 4.66 | 71.2 | | 360 |
| Example 8 | 1-110 | 4.67 | 71.2 | | 277 |
| Example 9 | 1-111 | 4.36 | 78.9 | | 256 |
| Example 10 | 1-214 | 4.45 | 72.8 | | 370 |
| Example 11 | 1-215 | 4.32 | 71.5 | | 289 |
| Example 12 | 1-222 | 4.31 | 79.2 | | 261 |
| Example 13 | 1-223 | 4.38 | 76.4 | | 250 |
| Example 14 | 1-230 | 4.67 | 71.2 | | 356 |
| Example 15 | 1-231 | 4.66 | 71.1 | | 271 |
| Example 16 | 1-238 | 4.66 | 71.2 | | 251 |
| Example 17 | 1-239 | 4.41 | 75.8 | | 249 |
| Example 18 | 1-259 | 4.66 | 71.1 | | 352 |
| Example 19 | 1-263 | 4.35 | 79.2 | | 195 |
| Example 20 | 1-264 | 4.45 | 72.8 | | 198 |
| Example 21 (not according to the invention) | 1-267 | 4.67 | 71.2 | | 154 |
| Example 22 | 1-270 | 4.42 | 75.7 | | 200 |
| Example 23 | 1-273 | 4.53 | 74.9 | | 505 |
| Example 24 | 2-11 | 4.76 | 69.4 | | 185 |
| Example 25 | 2-86 | 4.36 | 78.9 | | 239 |
| Example 26 | 2-87 | 4.42 | 75.7 | | 237 |
| Example 27 | 3-102 | 4.33 | 75.2 | | 246 |
| Example 28 | 3-214 | 4.13 | 79.2 | | 242 |
| Example 29 | 4-230 | 4.41 | 75.8 | | 225 |
| Example 30 | 4-239 | 4.69 | 77.2 | | 221 |
| Comparative Example 1 | Ref. 1 | 5.66 | 45.8 | | 48 |
| Comparative Example 2 | Ref. 2 | 5.03 | 48.7 | | 59 |
| Comparative Example 3 | Ref. 3 | 5.47 | 41.2 | | 57 |
| Comparative Example 4 | Ref. 4 | 4.42 | 45.7 | | 47 |
| Comparative Example 5 | Ref. 5 | 5.33 | 68.2 | | 73 |
| Comparative Example 6 | Ref. 6 | 5.37 | 63.5 | | 82 |
| Comparative Example 7 | Ref. 7 | 5.82 | 60.8 | | 68 |
| Comparative Example 8 | Ref. 8 | 4.45 | 59.8 | | 78 |

In addition, Ref. 1 to Ref. 8, comparative compounds used in Comparative Examples 1 to 8, are as follows.

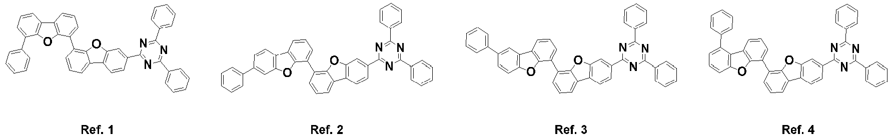

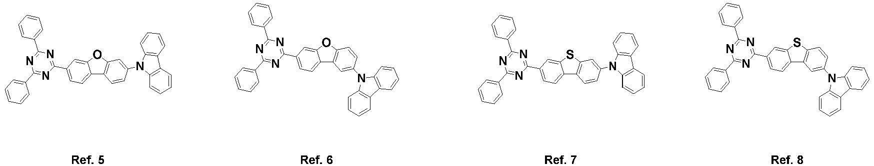

As seen from the results of Table 5, the organic light emitting devices of Examples 1 to 29 using the compound represented by any one of Chemical Formulae 1-2 to 1-4 or by any one of the specific examples aboveof the present disclosure, excluding Examples 1 and 21 which are not according to the invention and are present for illustration purposes only, as a light emitting layer material of the organic light emitting device had lower driving voltage, enhanced light emission efficiency and significantly improved lifetime compared to Comparative Examples 1 to 8 using the comparative compounds.

In this regard, HOMO orbitals and LUMO orbitals of the compound 1-1 and the comparative compounds Ref. 1 to Ref. 4 are as shown in the following Table 6. These compounds are not according to the invention and are present for illustration purposes only.

**[Table 6]**

| | Structural Formula | HOMO Orbital | LUMO Orbital |
|---|---|---|---|
| Ref.1 | 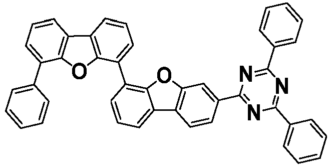 | 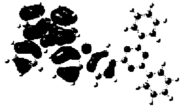 -5.78 | 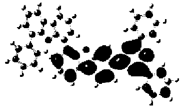 -1.96 |
| Ref.2 | 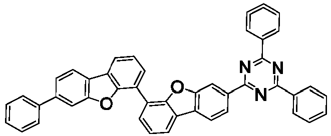 | 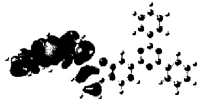 -5.74 | 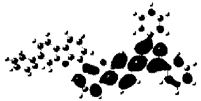 -1.96 |
| Ref.3 | 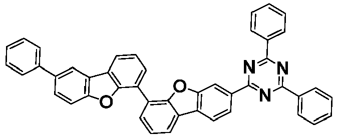 | 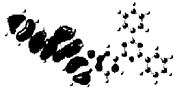 -5.73 | 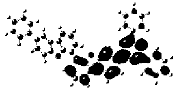 -1.96 |
| Ref.4 | 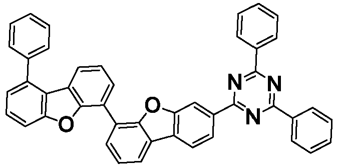 | 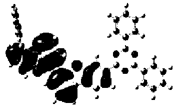 -5.78 | 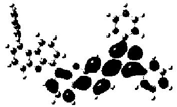 -1.94 |
| 1-1 (not accor ding to the inven tion) | 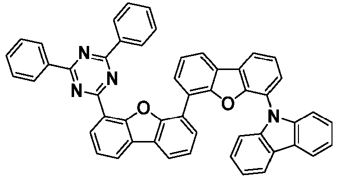 | 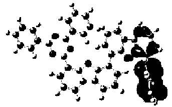 -5.40 | 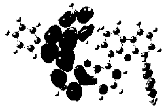 -1.84 |

As seen from Table 6, the HOMO orbitals of the comparative compounds Ref. 1, 2, 3 and 4 are delocalized to phenylene and dibenzofuran (DBF), and the HOMO levels are approximately -5.7. However, it is seen that Compound 1-1 is substituted with carbazole having a strong electron pushing ability, and the HOMO level is measured as -5.40. In other words, the compound has a higher HOMO level, and when using a compound represented by any one of the Chemical Formulae 1-2 to 1-4 of the present disclosure in the light emitting layer of the organic light emitting device, lifetime and efficiency may be enhanced in the electroluminescent device by facilitating hole injection from the host to the dopant.

In addition, HOMO orbitals and LUMO orbitals of the compound represented by Chemical Formulae 1-2of the present disclosure (Compound 1-86) and the comparative compounds Ref. 5 to Ref. 8 are as shown in the following Table 7.

**[Table 7]**

| | Structural Formula | HOMO Orbital | LUMO Orbital |
|---|---|---|---|
| Ref.5 | 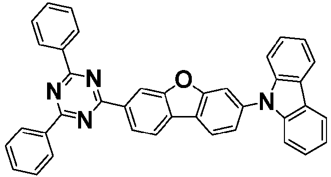 | 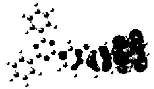 -5.38 | 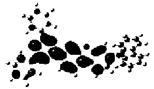 -2.06 |
| Ref.6 | 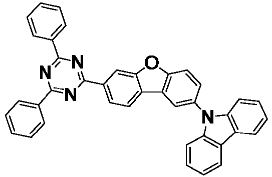 | 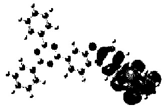 -5.36 | 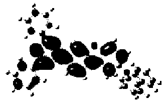 -2.08 |
| Ref.7 | 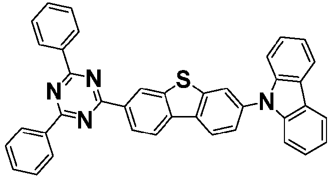 | 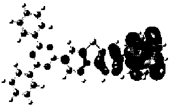 -5.39 | 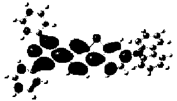 -2.08 |
| Ref.8 | 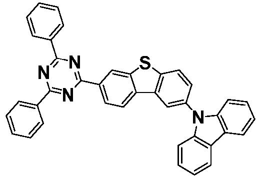 | 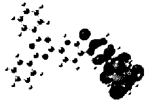 -5.37 | 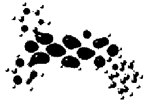 -2.09 |
| 1-86 | 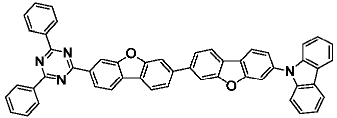 | 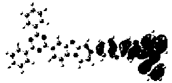 -5.34 | 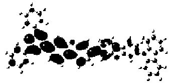 -2.07 |

As seen from Table 7, the LUMO orbitals of the comparative compounds Ref. 5, 6, 7 and 8 are delocalized from triazine to one heteroring. However, the LUMO orbital of Compound 1-86, the compound represented by Chemical Formulae 1-2 of the present disclosure, is delocalized from triazine to two heterorings. Due to such an expansion of the LUMO orbital, efficiency and lifetime of the organic light emitting device may be enhanced when using the compound represented any one of the Chemical Formulae 1-2 to 1-4 of the present disclosure in the light emitting layer of the organic light emitting device by enhancing electron mobility and stabilizing electrons.

In addition, HOMO orbitals and LUMO orbitals of Compound 1-1 (not according to the invention and present for illustration purposes only), Compound 2-11, Compound 1-86 and Compound 2-87, which are the compounds represented by any of the Chemical Formulae 1-2 to 1-4of the present disclosure, are as shown in the following Table 8.

**[Table 8]**

| | Structural Formula | HOMO Orbital | LUMO Orbital |
|---|---|---|---|
| 1-1 (not accord ing to the invent ion) | 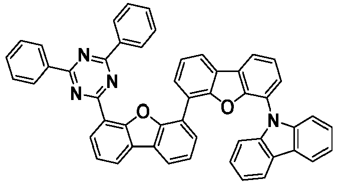 | 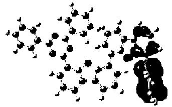 -5.40 | 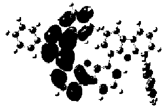 -1.84 |
| 2-11 | 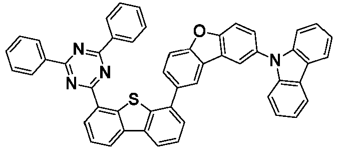 | 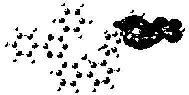 -5.34 | 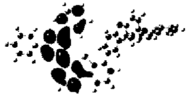 -1.95 |
| 1-86 | 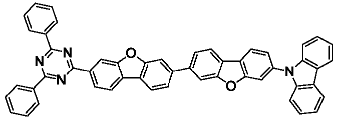 | 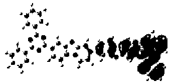 -5.34 | 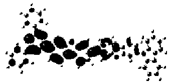 -2.07 |
| 2-87 | 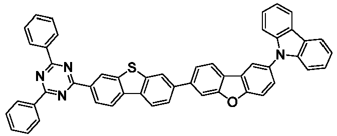 | 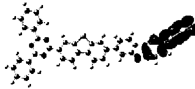 -5.35 | 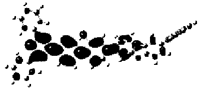 -2.10 |

Generally, a host in a light emitting layer is a layer to which both holes and electrons are injected, and efficiency and lifetime change depending on stability and mobility of holes and electrons.

The compound represented by any of the Chemical Formulae 1-2 to 1-4of the present disclosure is a compound formed with triazine that pulls electrons, carbazole that pushes electrons, and two heterorings connecting a donor and an acceptor.

The compound represented by any of the Chemical Formulae 1-2 to 1-4 may change mobility and stability of holes and electrons by changing a bonding position of the donor and the acceptor on the heteroring.

Specifically, carbazole or amine substituting at the end of the compound represented by any of the Chemical Formulae 1-2 to 1-4 has orbital disconnection around N, and accordingly, the HOMO orbital is generally localized to the carbazole or the amine. Herein, the HOMO orbital may be widely delocalized by changing the bonding position, which serves to increase hole mobility and stability.

In addition, in the compound represented by any of the Chemical Formulae 1-2 to 1-4, a heterocyclic group comprising N such as triazine is substituted with dibenzofuran or dibenzothiophene that may affect the LUMO orbital of the heterocyclic group comprising N.

As a result, the LUMO level of the compound may be adjusted depending on the position of substitution of the heterocyclic group comprising N substituted with dibenzofuran or dibenzothiophene. In other words, it means that the LUMO level may be adjusted depending on which position of the dibenzofuran or dibenzothiophene substituting the heterocyclic group comprising N such as triazine.

This means that, by properly adjusting the LUMO level depending on dopant and electron transfer layer (ETL) materials, the compound represented by any of the Chemical Formulae 1-2 to 1-4 of the present application may be used as a material of an organic light emitting device, particularly a material of a light emitting layer, and more specifically a host material. In other words, when changing dopant and electron transfer layer materials, a host material needs to be changed depending on the charge balance.

Specifically, when using a compound with a low LUMO level as a material of a light emitting layer, electrons may be readily injected from a host to a dopant, and when using a compound with a high LUMO level as a material of a light emitting layer, electrons may be readily injected from an electron transfer layer (ETL) to a host.

Particularly, in Compound 1-86, triazine bonds to the No. 3 position of dibenzofuran, and carbazole bonds to the No. 3 position of dibenzofuran, and the LUMO and HOMO orbitals are relatively widely delocalized to the heteroring. It may be identified that this enhances mobility and stability of holes and electrons resulting in an enhanced lifetime.

The above-described result may lead to a favorable result when electron mobility is fast from an electrode to a light emitting layer in an electroluminescent device. However, the result of a host in the light emitting layer may vary depending on the balance between holes and electrons in the device.

Generally, a charge balance between holes and electrons in a light emitting layer changes depending on the tendency of a phosphorescent dopant, and mobility of holes and electrons may be adjusted accordingly by either using a hole transfer auxiliary layer (G' HTL), a hole blocking layer (HBL) or an electron blocking layer (EBL), or doping to a hole transfer layer (HTL) or an electron transfer layer (ETL). Herein, a device lifetime may decrease when holes and electrons are over-accumulated in the light emitting layer.

When a compound having a structure disconnecting a heterocyclic group comprising N and carbazole or amine as the compound corresponding to the compound of the present application is used as a material of a light emitting layer, mobility of holes and electrons may be lowered to perform a role of decreasing the amounts of holes and electrons accumulated in the light emitting layer, and as a result, a device lifetime may be enhanced.

In addition, dibenzofuran or dibenzothiophene substituted with amine or carbazole as in the compound of the present application affects the LUMO orbital widely spread in the heterocyclic group comprising N, which may affect enhancement in the electron mobility and stability.

### <Experimental Example 2>

### (1) Manufacture of Organic Light Emitting Device

A glass substrate on which indium tin oxide (ITO) was coated as a thin film to a thickness of 150 nm (1,500 Å) was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of the compound represented by any of the Chemical Formulae 1-2 to 1-4 of the present disclosure (N-type) and one type of the compound represented by Chemical Formula 13 or 14 of the present disclosure (P-type), or the compound represented by any of the Chemical Formulae 1-2 to 1-4 of the present disclosure (N-type) and one type of the comparative compound (P-type) were pre-mixed as described in the following Table 9 and deposited to 40 nm (400 Å) in one source of supply as a host, and a green phosphorescent dopant [Ir(ppy)₃] was doped by an amount of 7 wt% of the deposited thickness of the light emitting layer and deposited. After that, BCP (bathocuproine) was deposited to 6 nm (60 Å) as a hole blocking layer, and Alq₃ was deposited to a thickness of 20 nm (200 Å) thereon as an electron transfer layer.

Lastly, an electron injection layer was formed on the electron transfer layer by depositing lithium fluoride (LiF) to a thickness of 1 nm (10 Å), and then a cathode was formed on the electron injection layer by depositing an aluminum (Al) cathode to a thickness of 120 nm (1,200 Å), and as a result, an organic light emitting device was manufactured (Examples 30 to 54 and Comparative Examples 9 to 20).

Meanwhile, all the organic compounds required to manufacture the OLED device were vacuum sublimation purified under approximately 1.33 × 10⁻⁶ to 1.33 × 10⁻⁴ Pa (10⁻⁸ torr to 10⁻⁶ torr) for each material to be used in the OLED manufacture.

### (2) Driving Voltage and Light Emission Efficiency of Organic Light Emitting Device

For each of the organic light emitting devices of Examples 30 to 54 and Comparative Examples 9 to 20 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. Herein, T₉₀ means a lifetime (unit: h, time) taken to become 90% with respect to initial luminance.

Results of measuring driving voltage, light emission efficiency, color coordinate (CIE) and lifetime of the organic light emitting devices manufactured according to the present disclosure are as shown in the following Table 9.

**[Table 9]**

| | Light Emitting Layer Compound | Ratio | Driving Voltage (V) | Efficiency (cd/A) | Color (EL Color) | Lifetime (T₉₀) |
|---|---|---|---|---|---|---|
| Example 31 | 1-86:5-2 | 1:8 | 4.73 | 54.2 | Green | 338 |
| Example 32 | | 1:5 | 4.71 | 57.2 | | 444 |
| Example 33 | | 1:2 | 4.33 | 75.2 | | 679 |
| Example 34 | | 1:1 | 4.48 | 70.2 | | 546 |
| Example 35 | | 2:1 | 4.69 | 69.2 | | 525 |
| Example 36 | | 5:1 | 4.32 | 68.3 | | 433 |
| Example 37 | | 8:1 | 4.21 | 67.0 | | 325 |
| Example 38 | 1-87:5-3 | 1:2 | 4.35 | 79.2 | | 599 |
| Example 39 | | 1:1 | 4.41 | 75.8 | | 591 |
| Example 40 | | 2:1 | 4.67 | 71.2 | | 540 |
| Example 41 | 1-94:5-12 | 1:2 | 4.38 | 76.4 | | 539 |
| Example 42 | | 1:1 | 4.45 | 72.8 | | 522 |
| Example 43 | | 2:1 | 4.66 | 71.1 | | 500 |
| Example 44 | 1-86:5-105 | 1:2 | 4.54 | 76.3 | | 779 |
| Example 45 | | 1:1 | 4.69 | 71.3 | | 646 |
| Example 46 | | 2:1 | 4.90 | 70.2 | | 625 |
| Example 47 | 1-102:6-4 | 1:2 | 4.33 | 75.2 | | 620 |
| Example 48 | | 1:1 | 4.48 | 70.2 | | 582 |
| Example 49 | | 2:1 | 4.69 | 69.2 | | 543 |
| Example 50 | 1-103:6-7 | 1:2 | 4.33 | 75.2 | | 569 |
| Example 51 | | 1:1 | 4.48 | 70.2 | | 543 |
| Example 52 | | 2:1 | 4.69 | 69.2 | | 518 |
| Example 53 | 1-214:6-31 | 1:2 | 4.31 | 79.2 | | 586 |
| Example 54 | | 1:1 | 4.42 | 75.7 | | 558 |
| Example 55 | | 2:1 | 4.66 | 71.1 | | 536 |
| Example 56 | 1-230:6-32 | 1:2 | 4.32 | 74.2 | | 562 |
| Example 57 | | 1:1 | 4.42 | 72.2 | | 553 |
| Example 58 | | 2:1 | 4.66 | 71.2 | | 528 |
| Example 59 | 2-86: 6-31 | 1:2 | 4.44 | 74.1 | | 409 |
| Example 60 | | 1:1 | 4.59 | 69.1 | | 371 |
| Example 61 | | 2:1 | 4.80 | 68.0 | | 332 |
| Example 62 | 3-102: 6-31 | 1:2 | 4.41 | 74.1 | | 358 |
| Example 63 | | 1:1 | 4.59 | 69.2 | | 332 |
| Example 64 | | 2:1 | 4.78 | 68.2 | | 307 |
| Example 65 | 4-230: 6-31 | 1:2 | 4.42 | 78.1 | | 275 |
| Example 66 | | 1:1 | 4.53 | 74.6 | | 246 |
| Example 67 | | 2:1 | 4.76 | 70.0 | | 225 |
| Example 68 | 4-230: 6-31 | 1:2 | 4.62 | 79.0 | | 376 |
| Example 69 | | 1:1 | 4.73 | 75.2 | | 343 |
| Example 70 | | 2:1 | 4.96 | 71.8 | | 321 |
| Comparative Example 9 | 1-86:Ref. 9 | 1:2 | 5.73 | 54.2 | | 316 |
| Comparative Example 10 | | 1:1 | 5.71 | 57.2 | | 225 |
| Comparative Example 11 | | 2:1 | 5.81 | 59.2 | | 133 |
| Comparative Example 12 | 1-86:Ref. 10 | 1:2 | 5.42 | 55.7 | | 399 |
| Comparative Example 13 | | 1:1 | 5.21 | 57.0 | | 311 |
| Comparative Example 14 | | 2:1 | 5.65 | 59.2 | | 240 |
| Comparative Example 15 | 1-86:Ref. 11 | 1:2 | 5.41 | 55.8 | | 464 |
| Comparative Example 16 | | 1:1 | 5.53 | 55.6 | | 331 |
| Comparative Example 17 | | 2:1 | 5.58 | 50.5 | | 310 |
| Comparative Example 18 | 1-86:Ref. 12 | 1:2 | 5.32 | 50.4 | | 138 |
| Comparative Example 19 | | 1:1 | 5.69 | 49.2 | | 79 |
| Comparative Example 20 | | 2:1 | 5.61 | 41.3 | | 44 |

In addition, Ref. 9 to Ref. 12, comparative compounds used in Comparative Examples 9 to 20, are as follows.

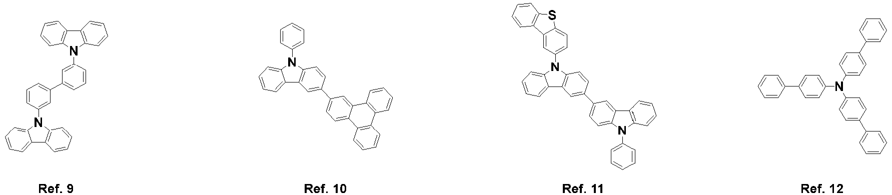

From the results of Table 9, effects of more superior efficiency and lifetime are obtained when including the compound represented by any of the Chemical Formulae 1-2 to 1-4of the present disclosure (N-type) and the compound represented by Chemical Formula 13 or 14 of the present disclosure (P-type) at the same time. Such results may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In the present disclosure, it was identified that excellent device properties were obtained when using the compound represented Chemical Formula 13 or 14 performing a donor role and the compound represented by any of the Chemical Formulae 1-2 to 1-4performing an acceptor role as a light emitting layer host.

In this regard, HOMO orbitals and LUMO orbitals of the compound represented by Chemical Formula 13 of the present disclosure (Compound 5-2), the compound represented by Chemical Formula 13 of the present disclosure (Compound 6-31), and the comparative compounds Ref. 9 to Ref. 12 are as shown in the following Table 10.

**[Table 10]**

| | Structural Formula | HOMO Orbital | LUMO Orbital |
|---|---|---|---|
| Ref. 9 | 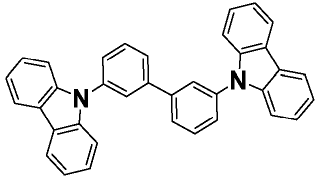 | 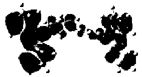 -5.40 | 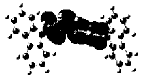 -1.19 |
| Ref. 10 | 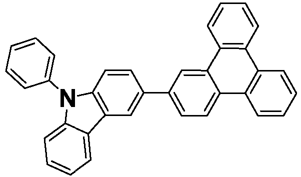 | 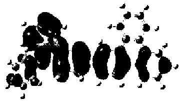 -5.21 | 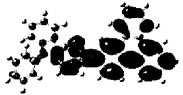 -1.03 |
| Ref. 11 | 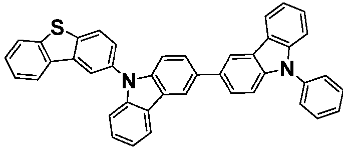 | 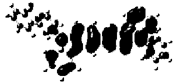 -4.97 | 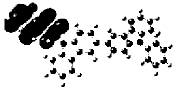 -1.20 |
| Ref. 12 | 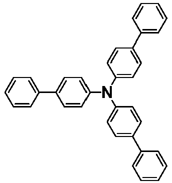 | 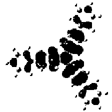 -4.87 | 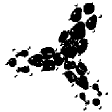 -0.91 |
| 5-2 | 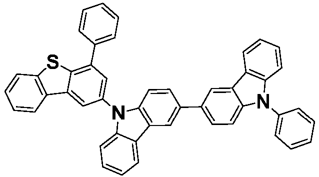 | 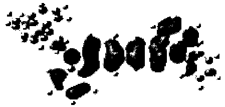 -4.96 | 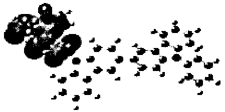 -1.17 |
| 6-31 | 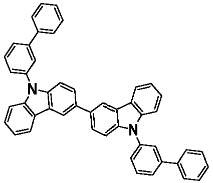 | 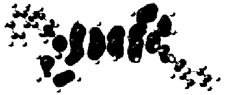 -4.96 | 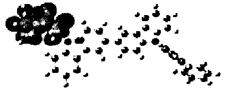 -1.00 |

As seem from Table 10, the HOMO orbital of the comparative compound Ref. 9 is localized to two carbazoles at the end resulting in low HOMO level and hole mobility. This causes an imbalance between holes and electrons in the device resulting in a decrease in the lifetime.

The HOMO orbital of the comparative compound Ref. 10 is delocalized to carbazole and triphenylene resulting in a higher HOMO level and enhanced hole mobility compared to Ref. 9, however, an imbalance in the device still exists.

When there is no aryl substituent at the No. 4 position of the dibenzothiophene (DBT) core as in the comparative compound Ref 11, molecular stability decreases since a protective substituent is not present at the No. 4 position of the dibenzothiophene (DBT) core that has favorable reactivity, and the LUMO orbital is localized to the dibenzothiophene (DBT) core compared to Compound 5-2, and as a result, the lifetime decreases by failing to effectively stabilize electrons.

Using amine as the p-type as in the comparative compound Ref. 12 causes an imbalance in the device due to too high HOMO level and fast hole mobility, and a decrease in the lifetime is identified.

The HOMO levels of Compound 5-2 represented by Chemical Formula 13 of the present disclosure and Compound 6-31 represented by Chemical Formula 14 of the present disclosure are widely delocalized to two carbazoles. When the compound represented by any of the Chemical Formulae 1-2 to 1-4 of the present disclosure with the compound represented by Chemical Formula 13 of the present disclosure or the compound represented by Chemical Formula 14 of the present disclosure are used at the same time in an organic light emitting device, HOMO level, and hole mobility and stability increase compared to basic carbazole, and a lifetime of the organic light emitting device may be enhanced. Particularly, the structure as Compound 5-2 effectively stabilizes electrons and enhances a lifetime of an organic light emitting device by introducing a dibenzothiophene (DBT) core and an aryl group considering electron stability.

### <Reference Numeral>

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

## Claims

1. A heterocyclic compound represented by any one of the following Chemical Formulae 1-2 to 1-4: 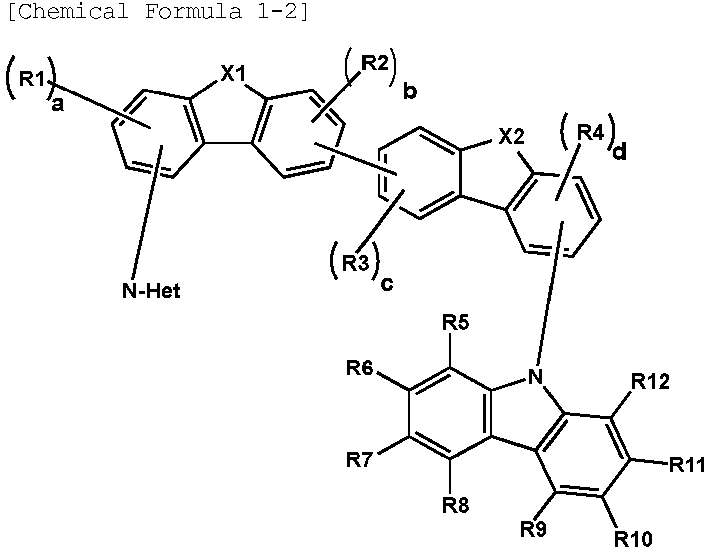 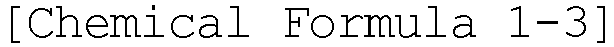 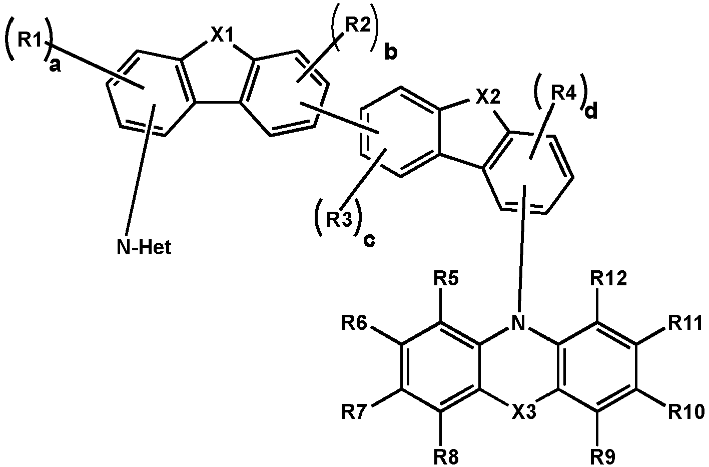 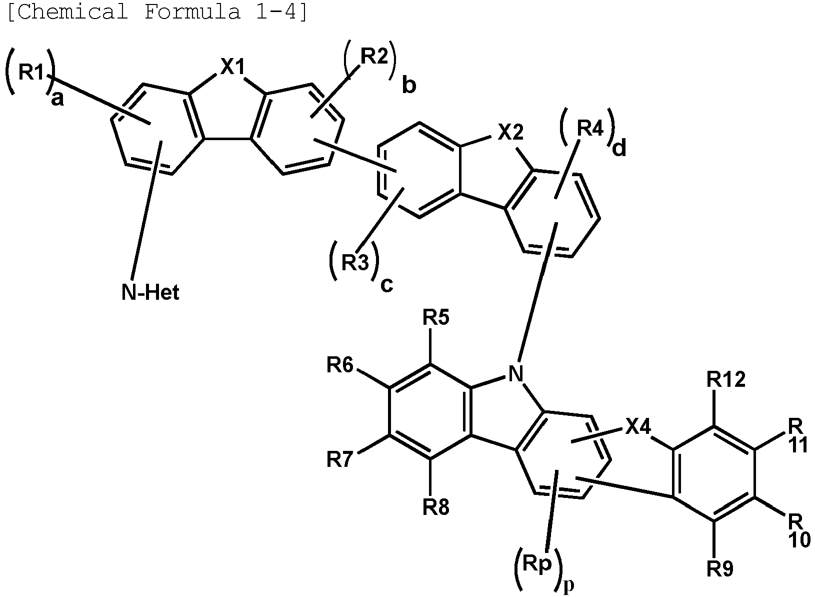 in Chemical Formulae 1-2 to 1-4,
X1 and X2 are the same as or different from each other, and each independently O; or S;
R1 to R4 are the same as or different from each other, and each independently hydrogen; deuterium; a substituted or unsubstituted aryl group having 6 to 60 carbon atoms; or a substituted or unsubstituted heteroaryl group having 6 to 60 carbon atoms, a to d are each an integer of 0 to 3, and when a to d are each 2 or greater, substituents in the parentheses are the same as each other;
N-Het is a group represented by any one of the following Chemical Formulae 2 to 4;
X3 and X4 are each independently O; S; CRaRb; or NRc, Ra and Rb are the same as or different from each other and each independently a substituted or unsubstituted alkyl group having 1 to 10 carbon atoms; or a substituted or unsubstituted aryl group having 6 to 20 carbon atoms, and Rc is a substituted or unsubstituted aryl group having 6 to 20 carbon atoms;
R5 to R12 and Rp are the same as or different from each other, and each independently hydrogen; deuterium; a halogen group; a cyano group; a substituted or unsubstituted aryl group having 6 to 20 carbon atoms; or a substituted or unsubstituted heteroaryl group having 2 to 20 carbon atoms;
p is an integer of 0 to 2, and when p is 2, substituents in the parentheses are the same as each other; 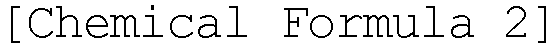 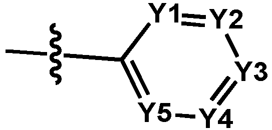 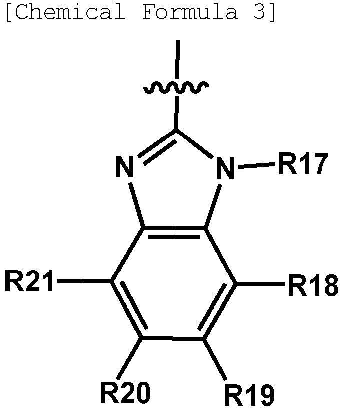 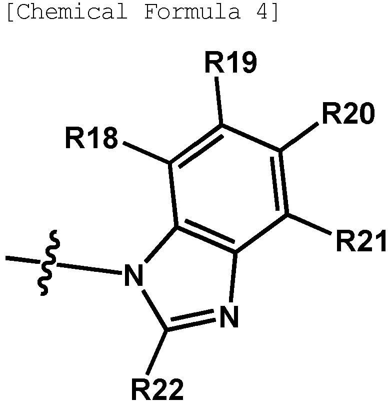 in Chemical Formulae 2 to 4,
Y1 is CR12' or N, Y2 is CR13' or N, Y3 is CR14' or N, Y4 is CR15' or N, Y5 is CR16' or N, and at least one of Y1 to Y5 is N; and
R12' to R16' and R17 to R22 are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 1 to 20 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aliphatic or aromatic hydrocarbon ring or heteroring; 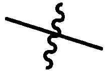
is a site linked to any one of the following Chemical Formulae 1-2 to 1-4; and
No. 3 position of 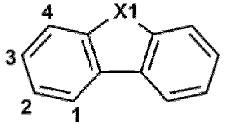 is substituted with N-Het and No. 3 position of 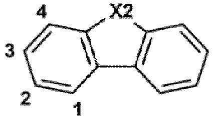 is substituted with any one of 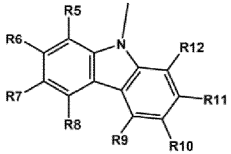 of Chemical Formula 1-2, 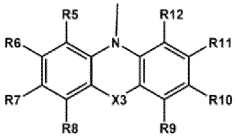 of Chemical Formula 1-3 and 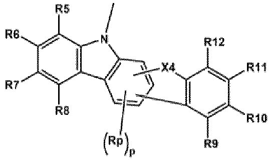 of Chemical Formula 1-4.

2. The heterocyclic compound of Claim 1, wherein the heterocyclic compound represented by any one of the following Chemical Formulae 1-2 to 1-4 has a deuterium content of 0%, or greater than 10% and less than or equal to 100%.

3. A heterocyclic compound represented by any one of the following compounds:

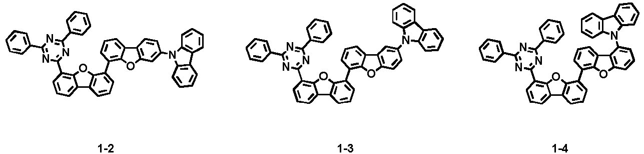

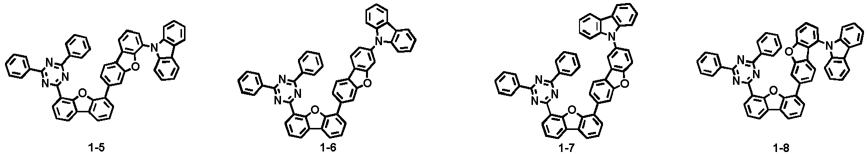

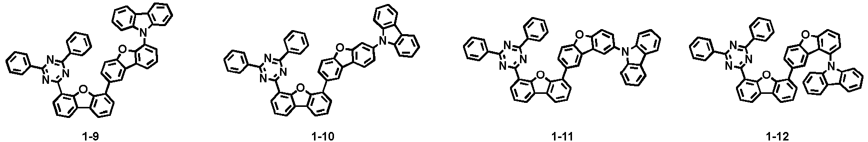

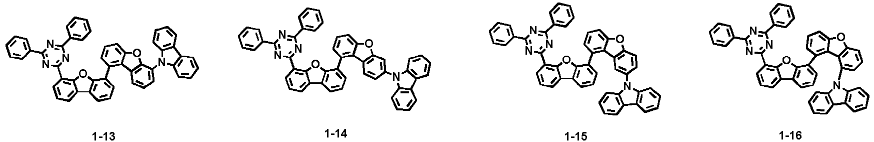

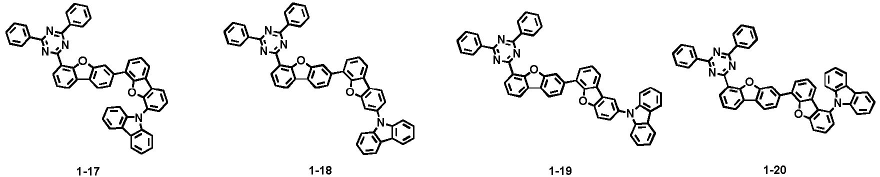

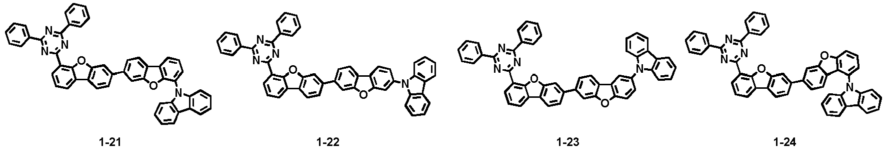

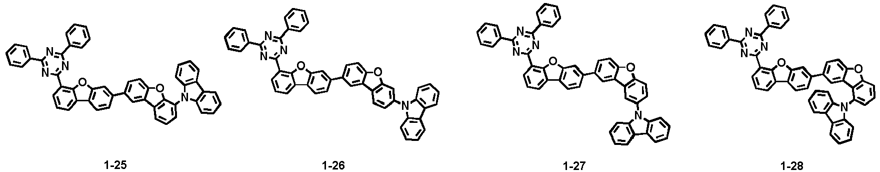

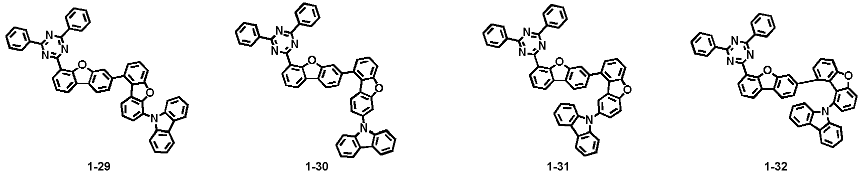

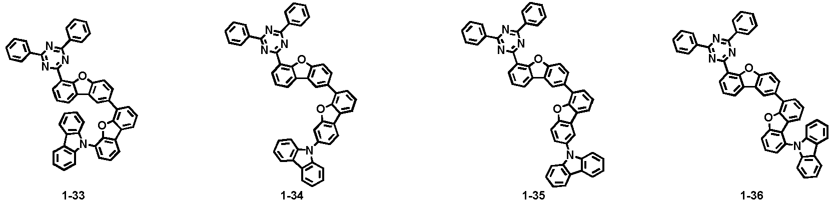

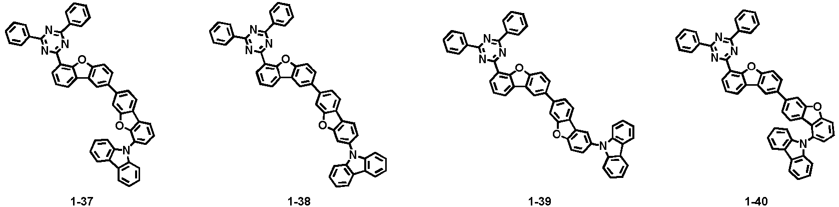

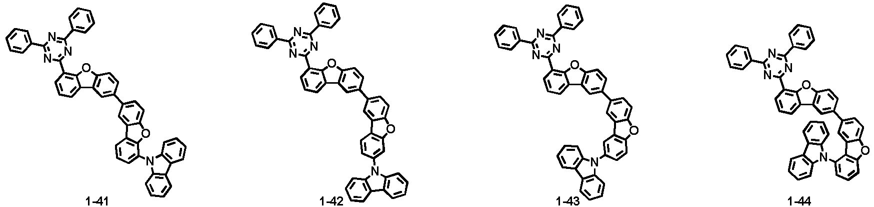

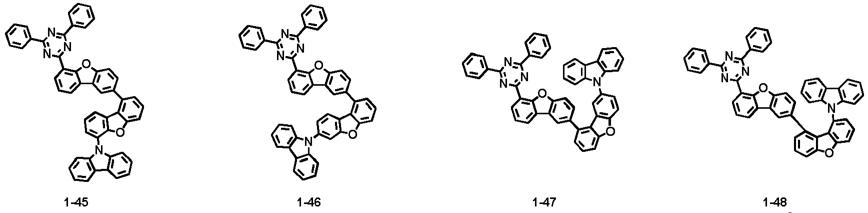

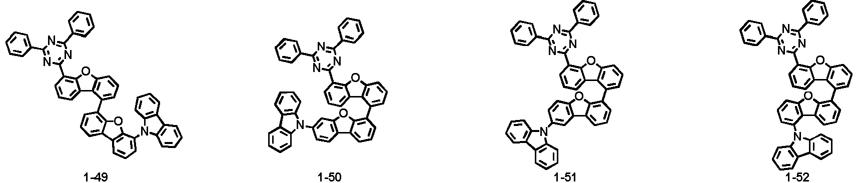

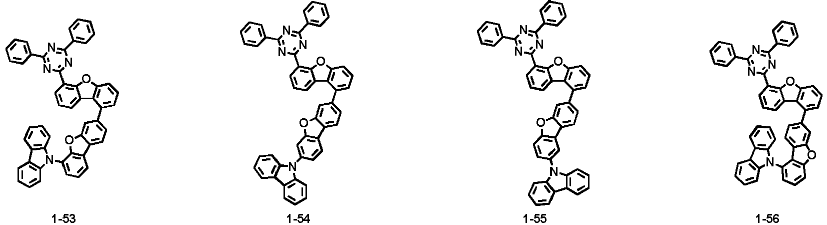

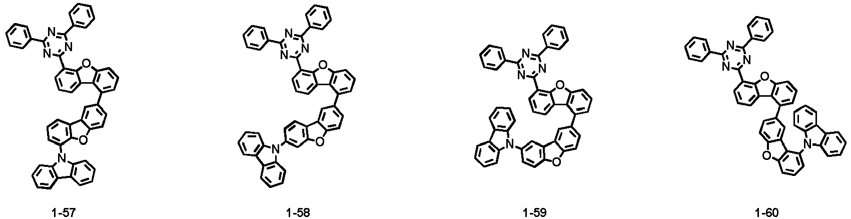

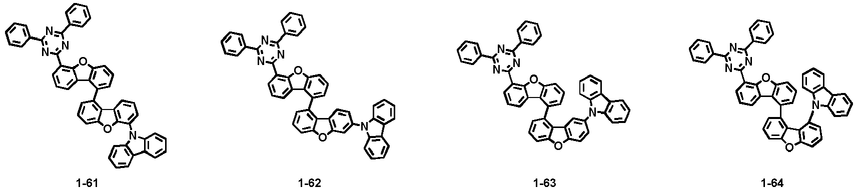

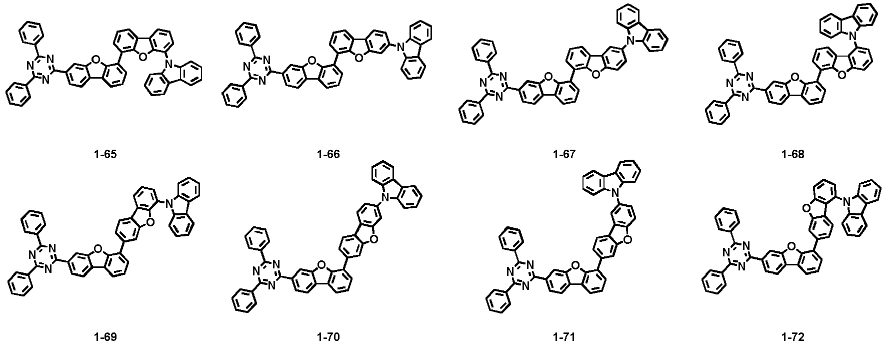

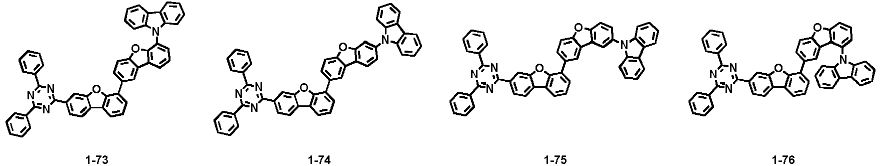

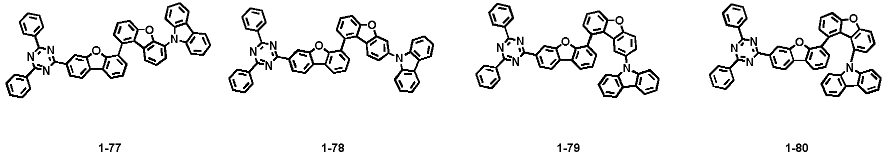

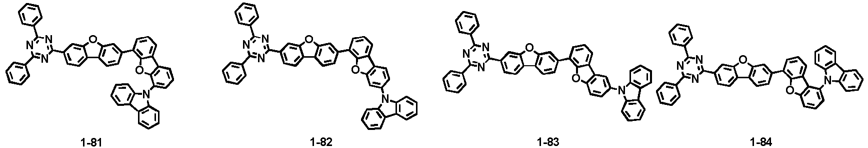

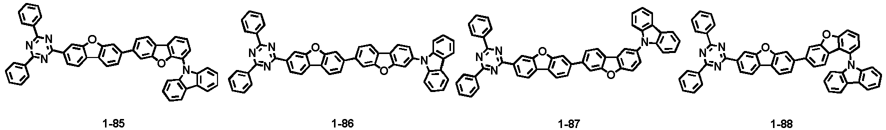

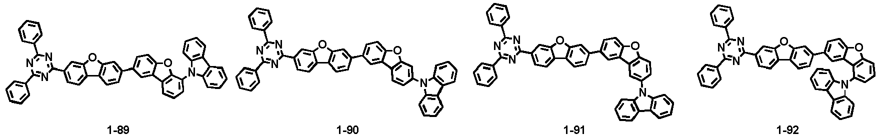

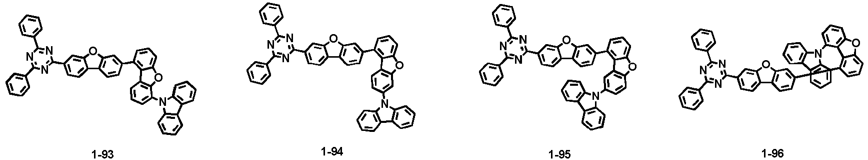

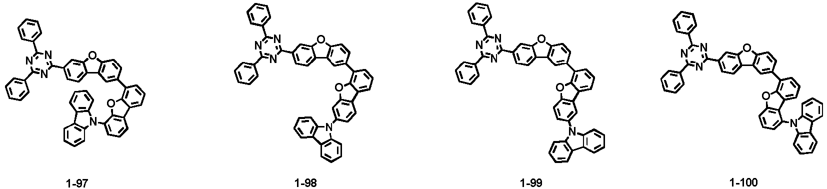

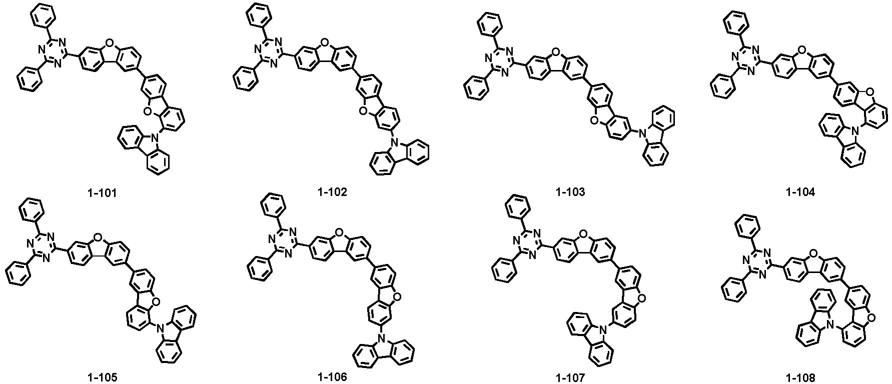

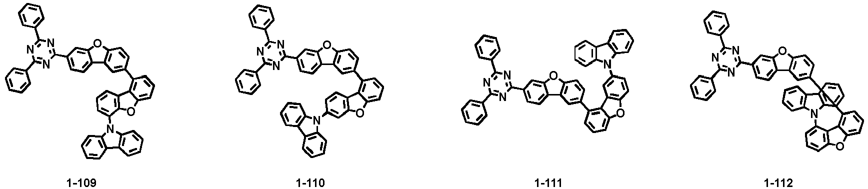

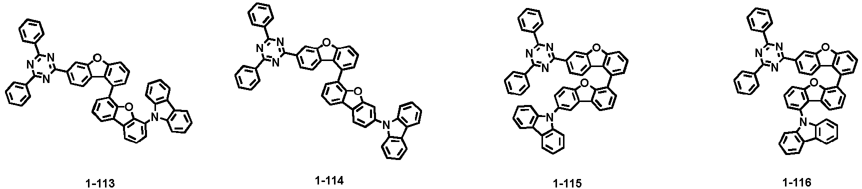

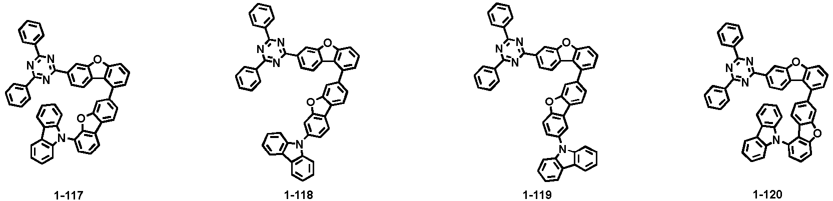

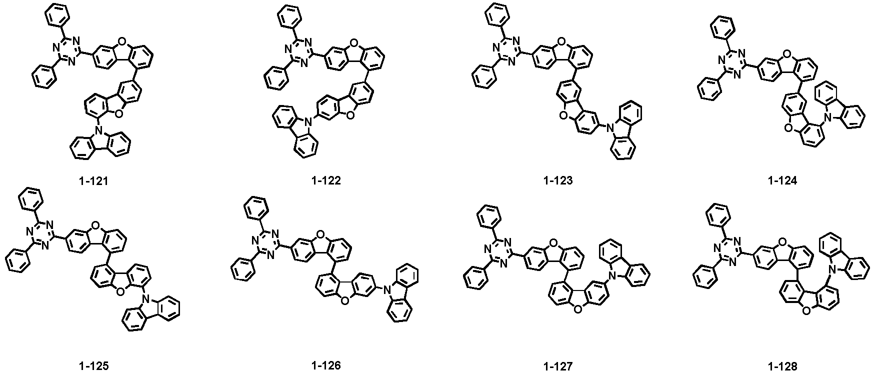

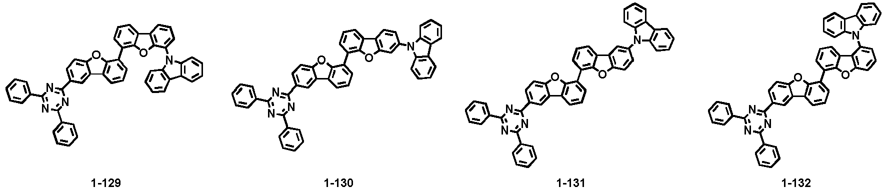

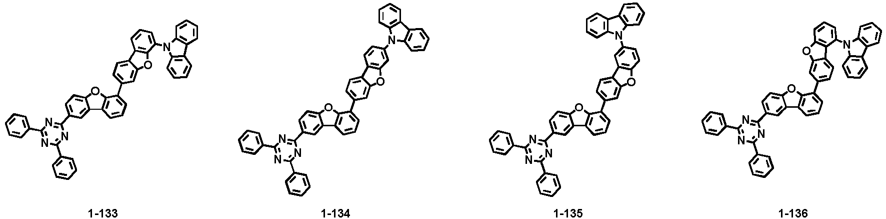

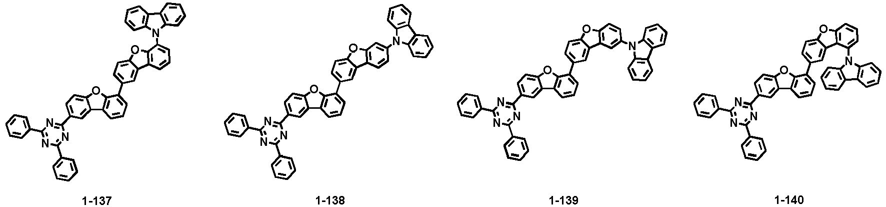

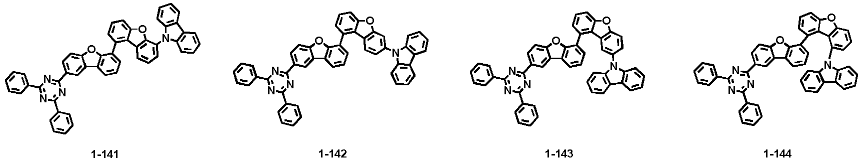

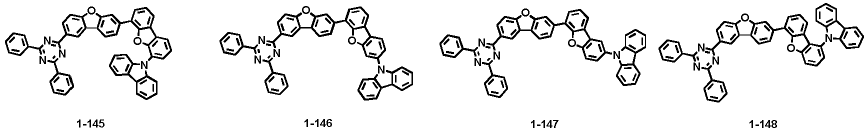

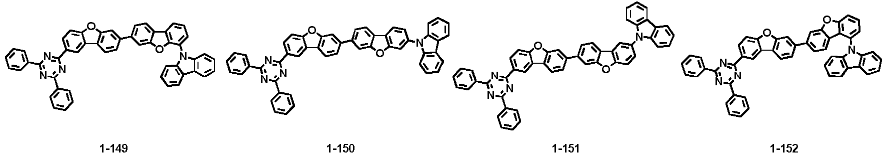

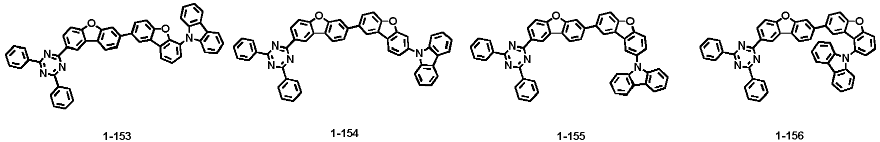

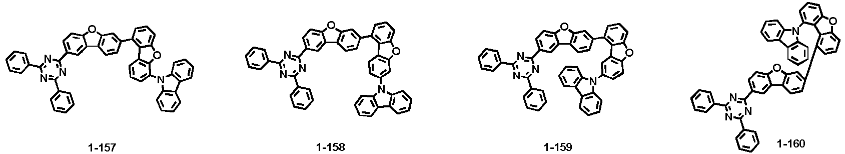

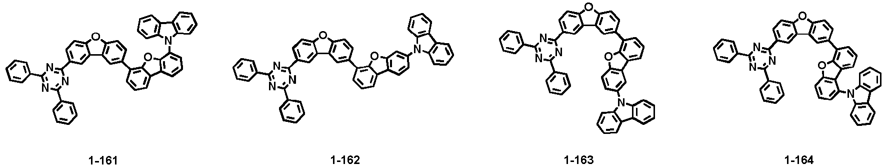

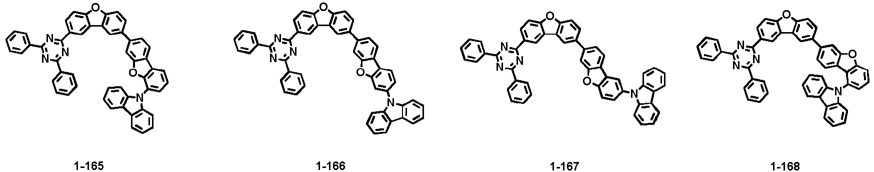

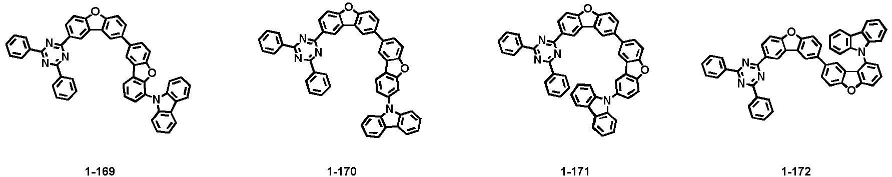

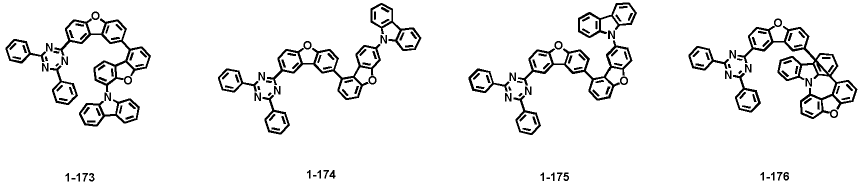

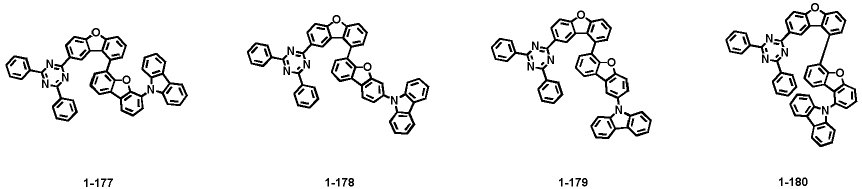

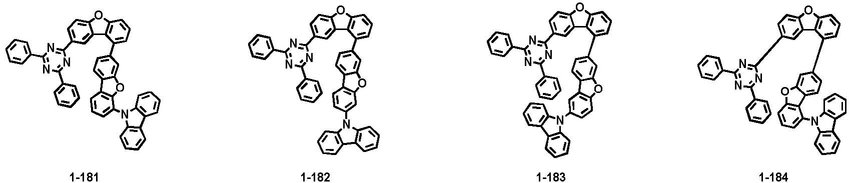

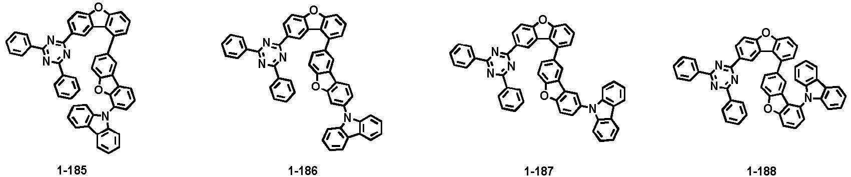

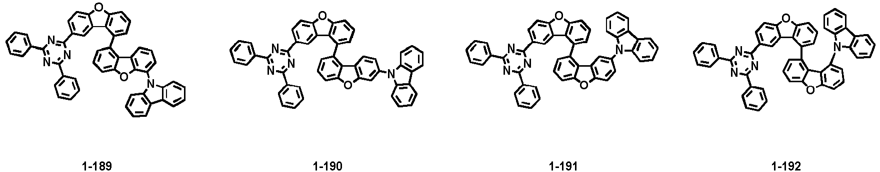

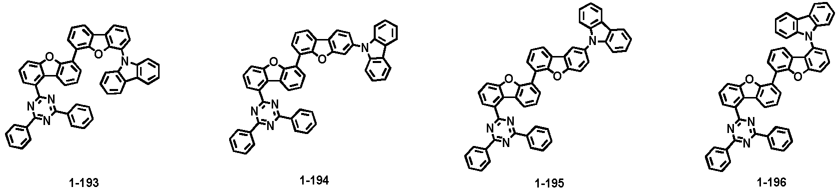

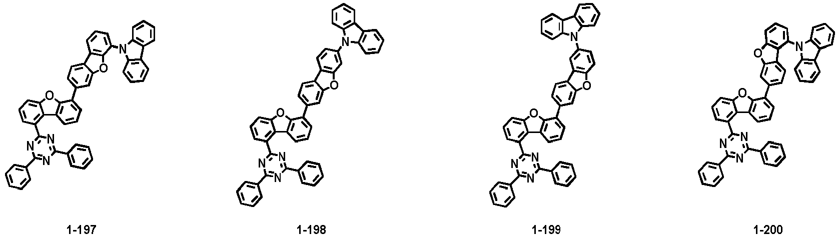

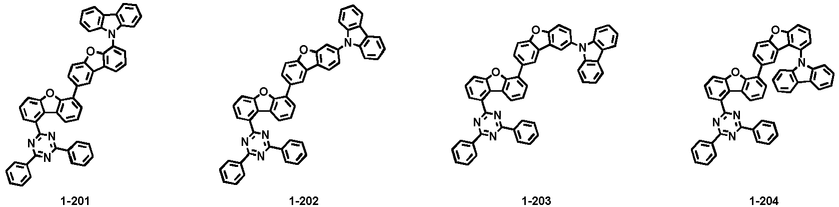

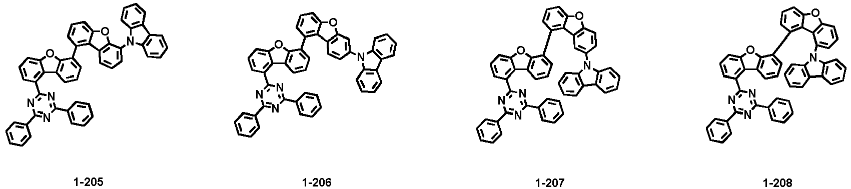

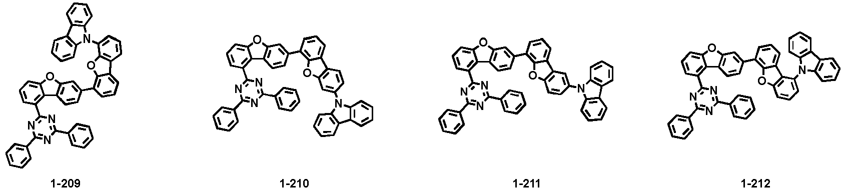

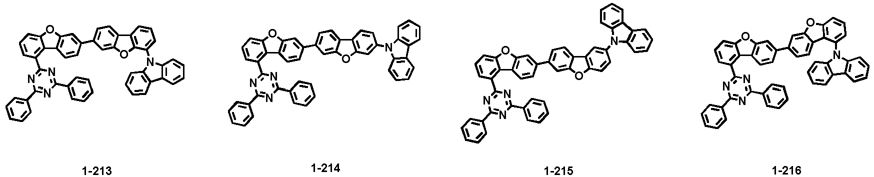

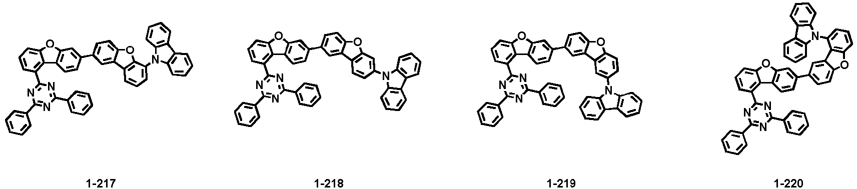

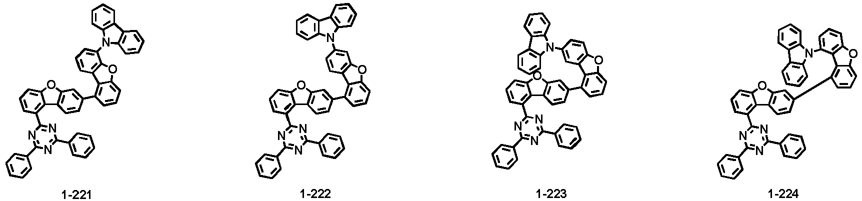

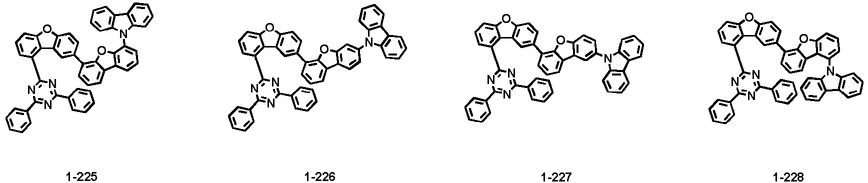

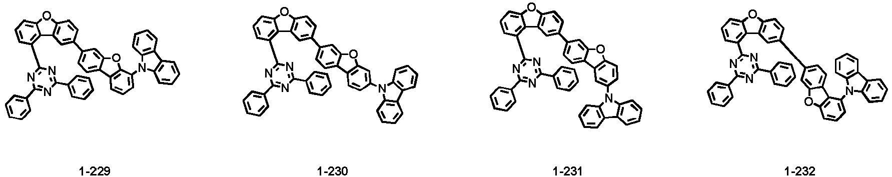

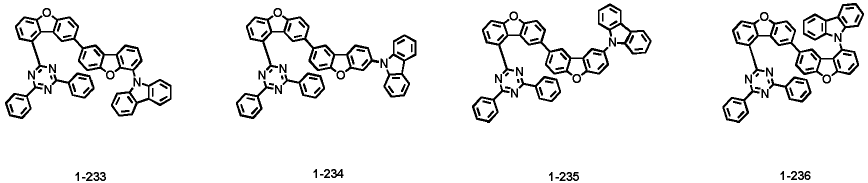

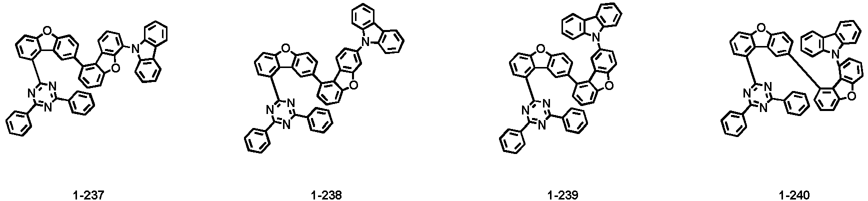

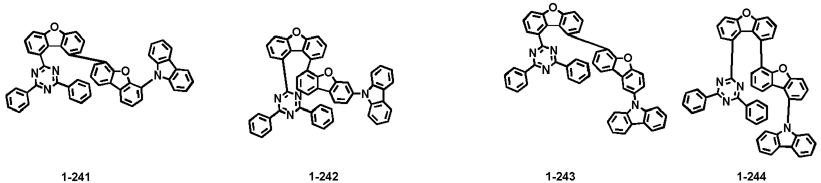

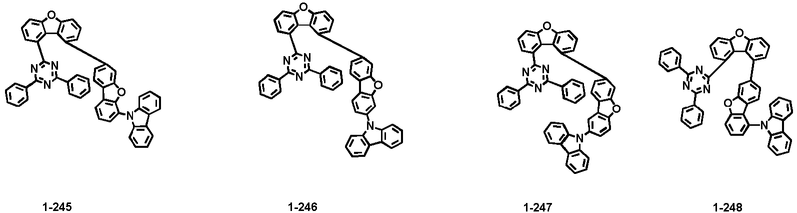

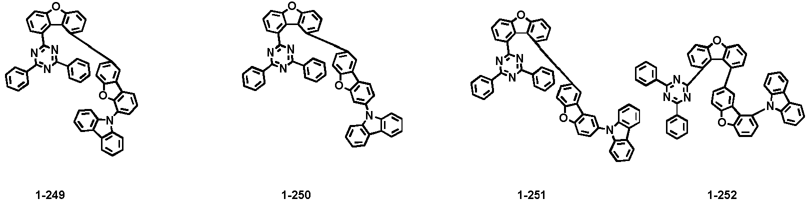

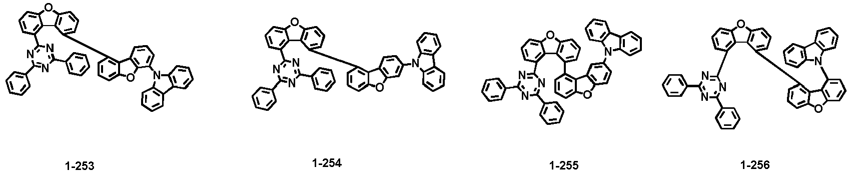

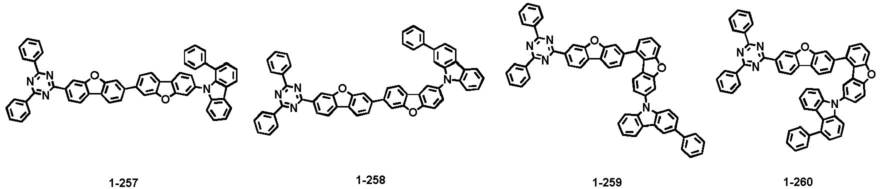

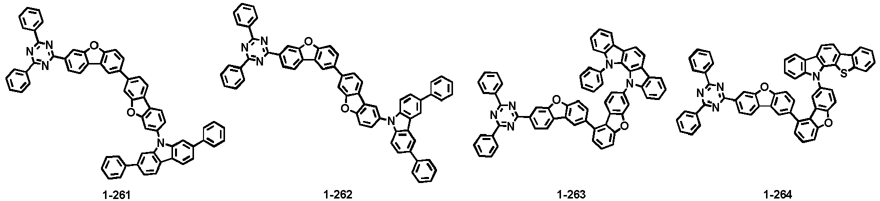

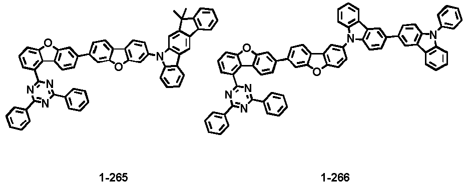

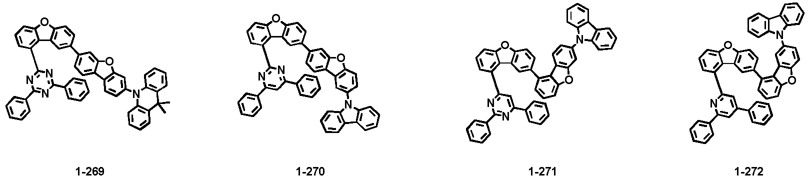

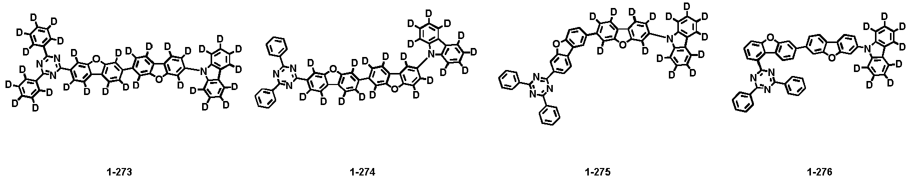

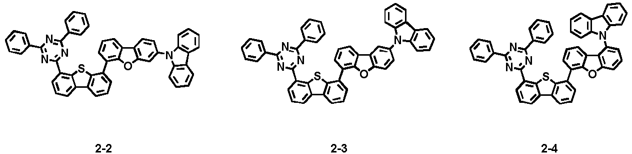

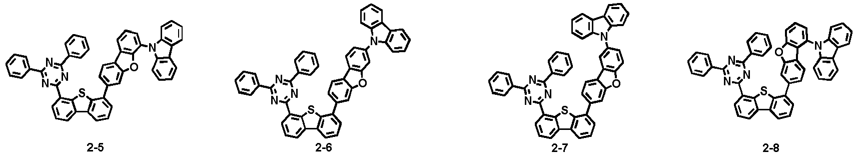

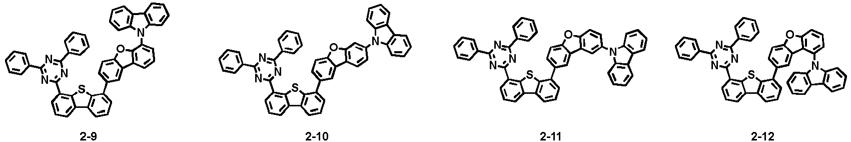

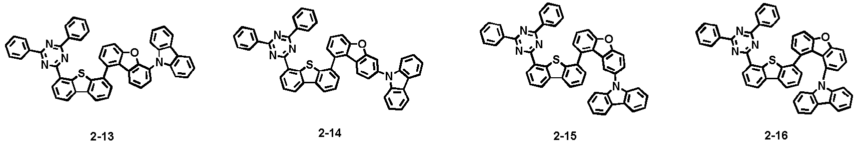

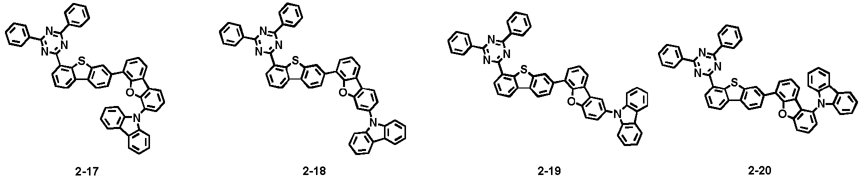

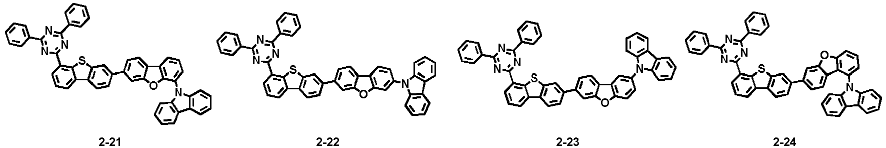

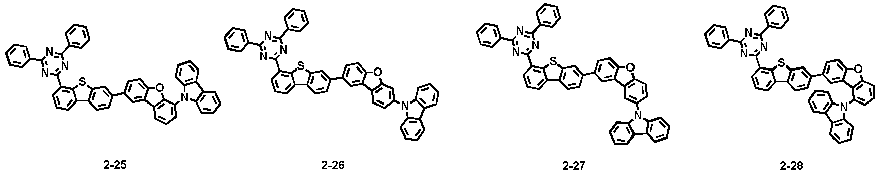

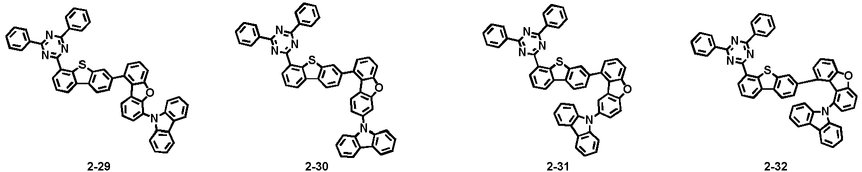

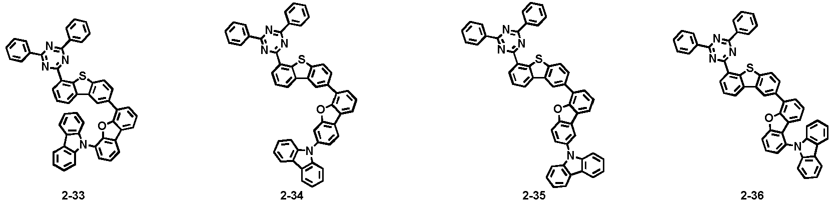

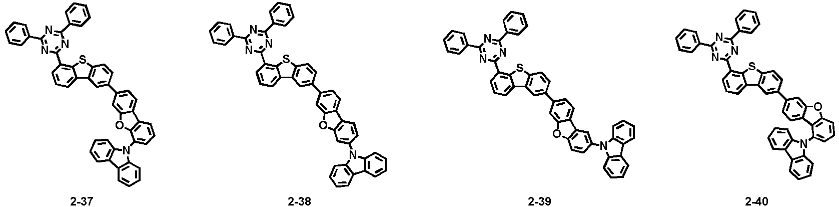

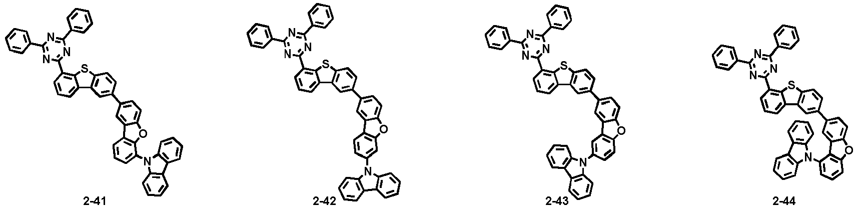

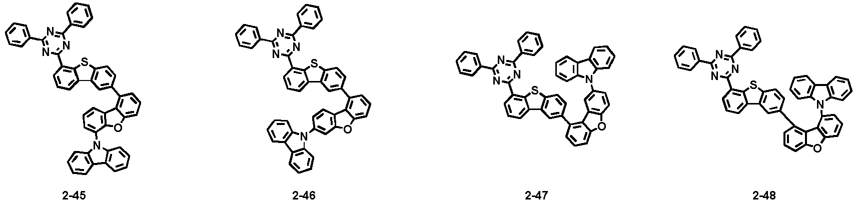

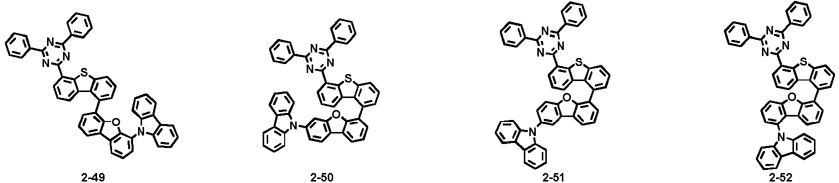

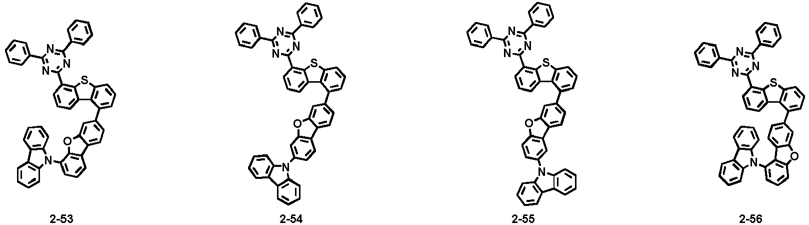

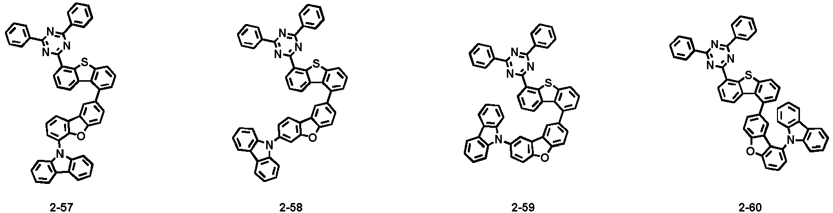

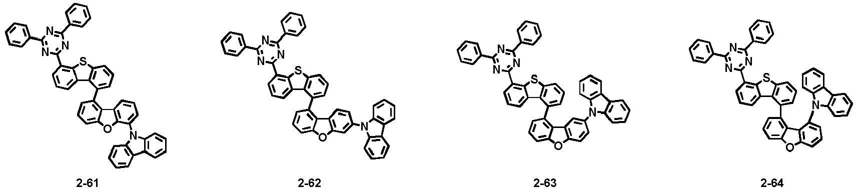

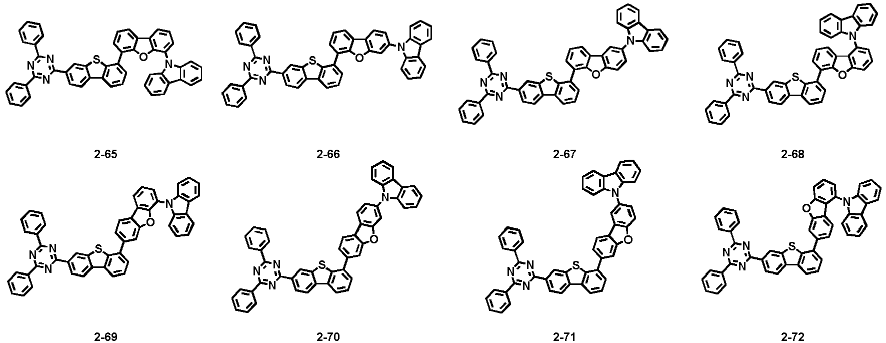

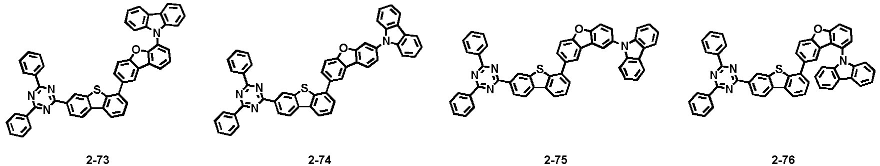

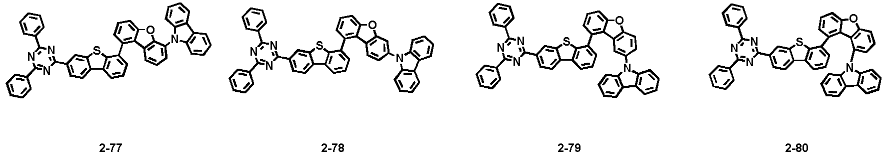

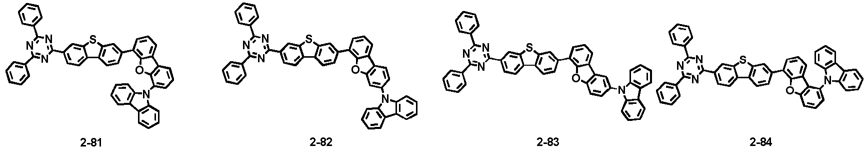

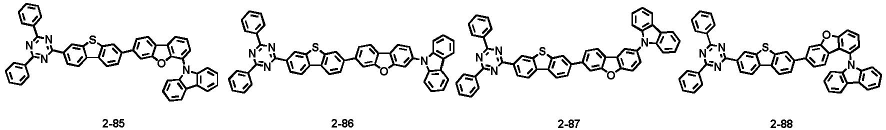

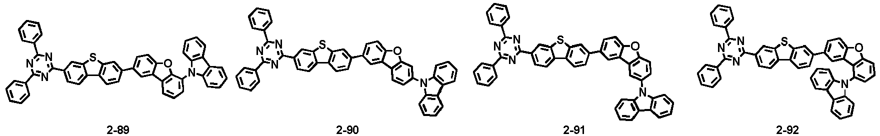

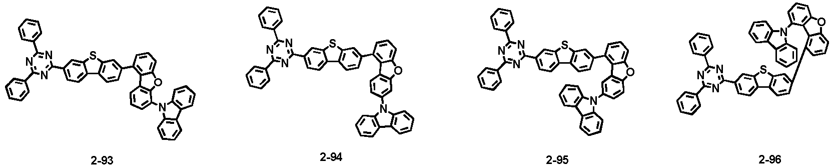

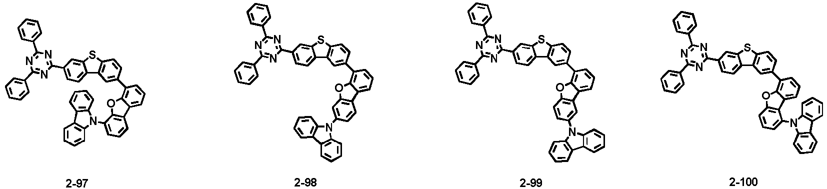

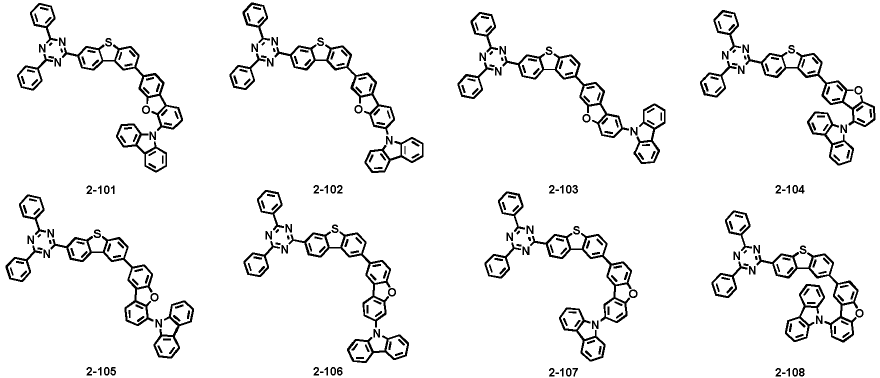

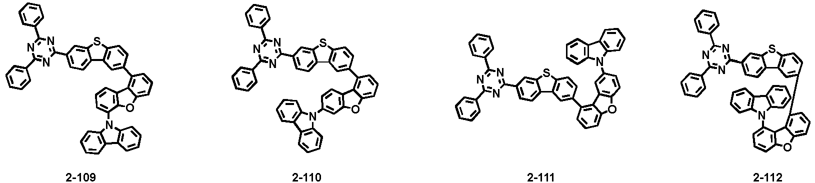

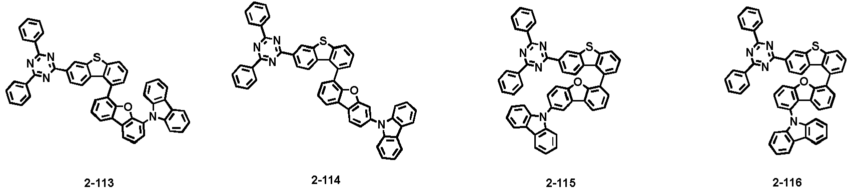

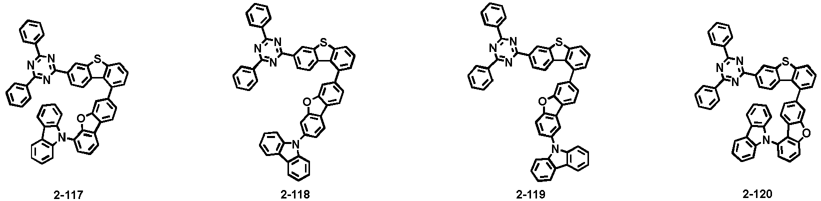

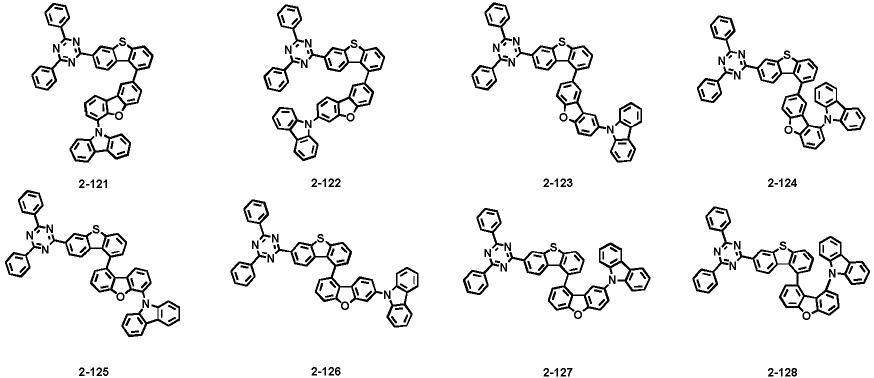

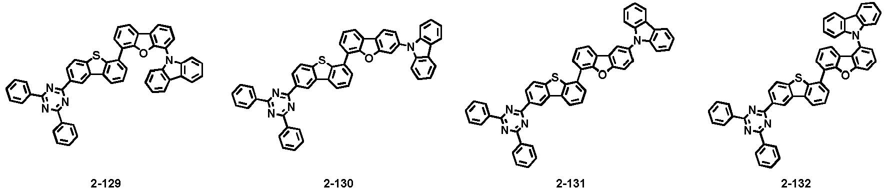

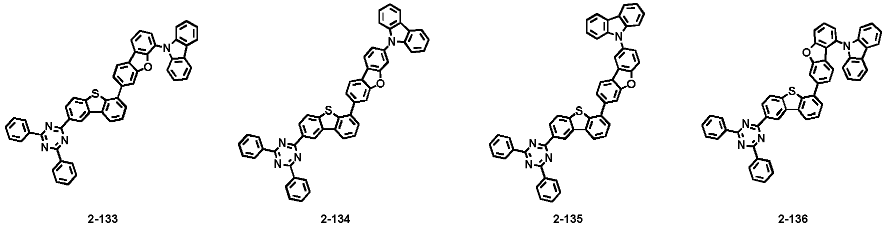

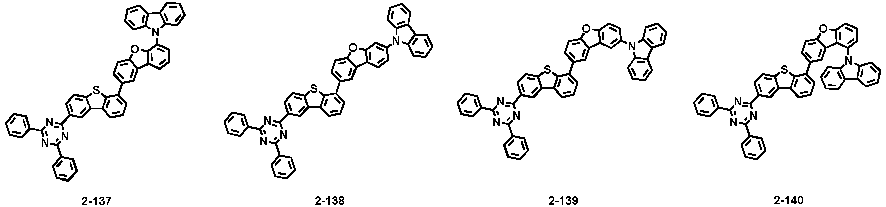

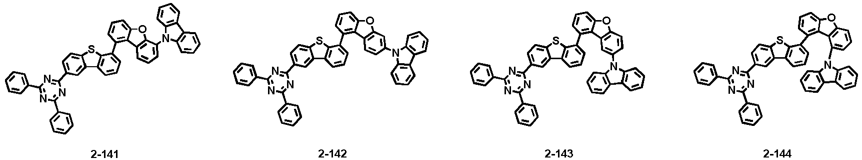

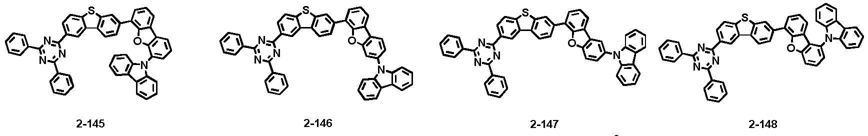

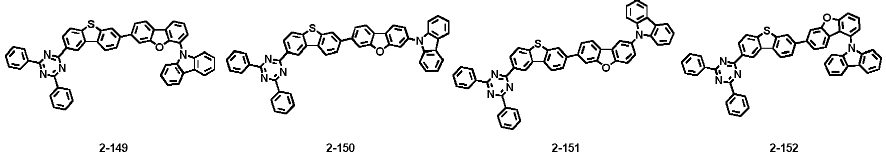

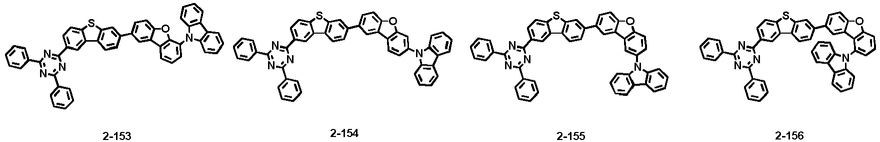

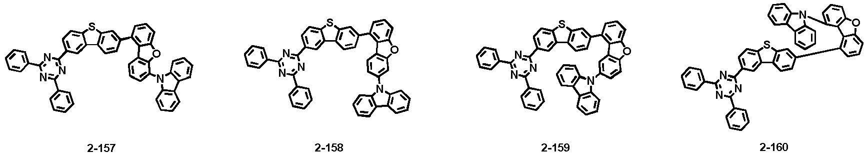

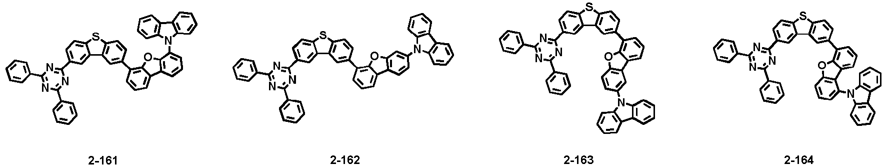

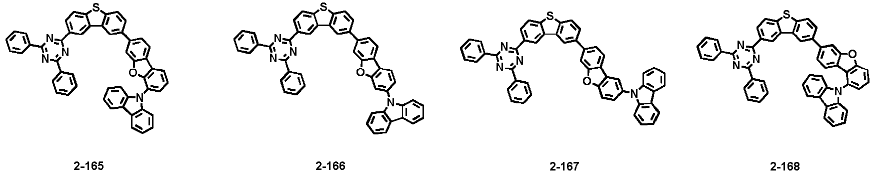

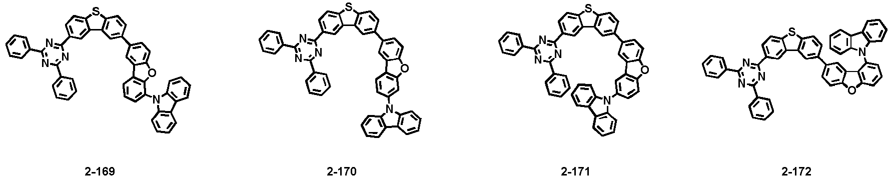

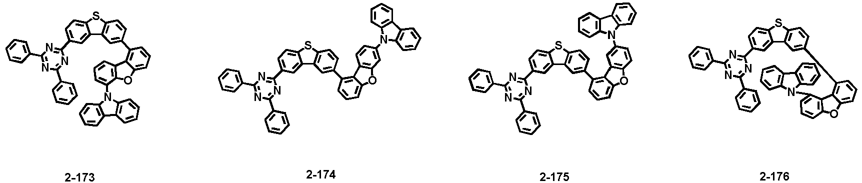

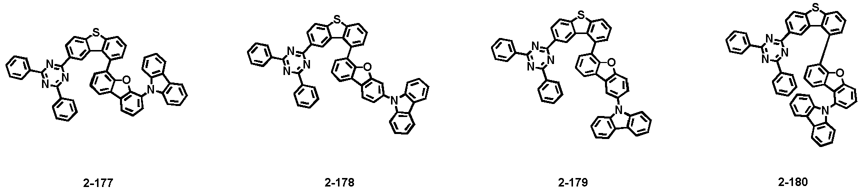

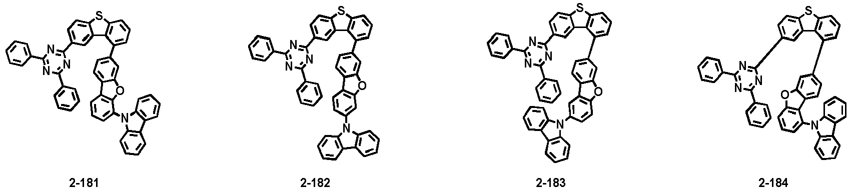

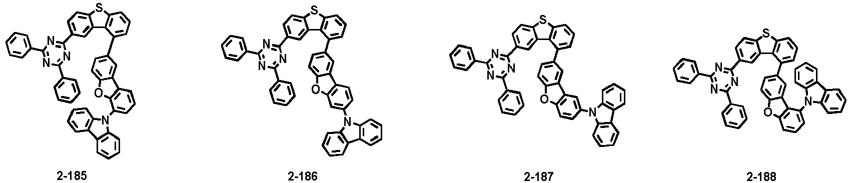

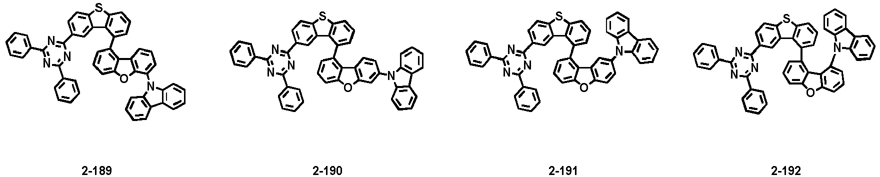

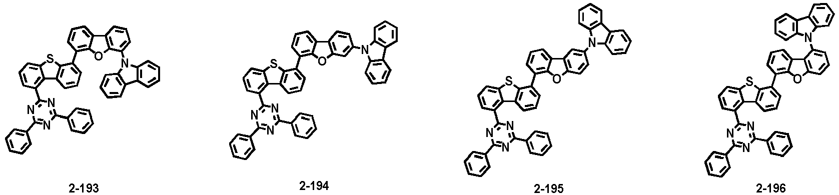

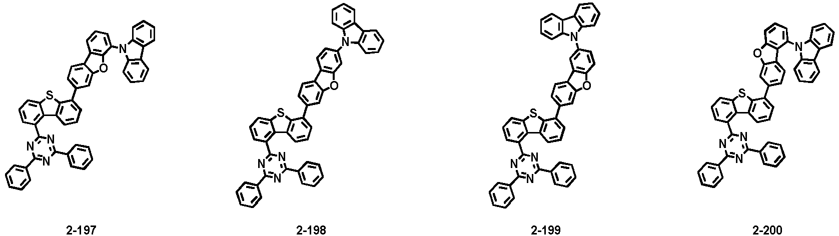

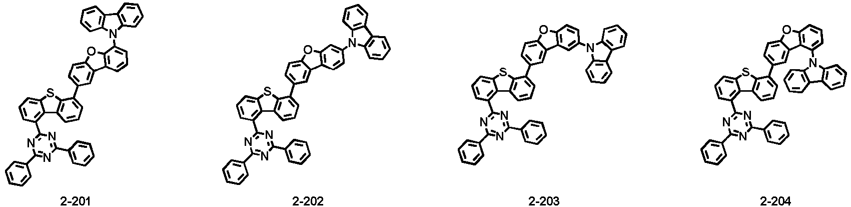

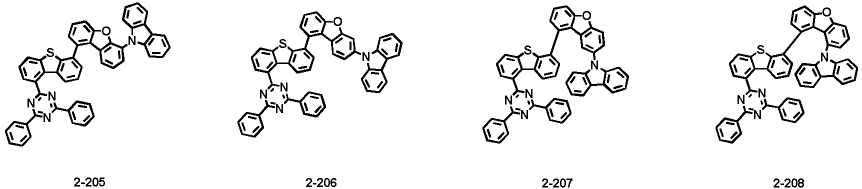

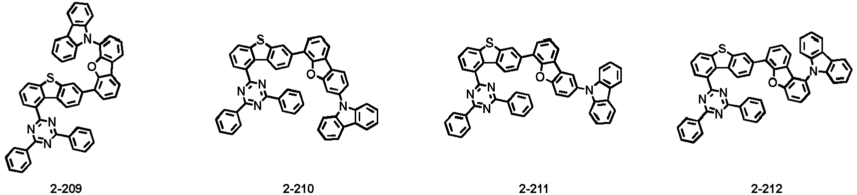

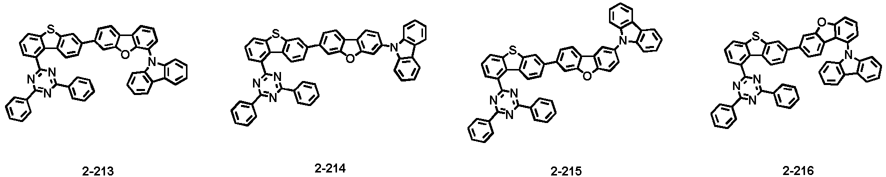

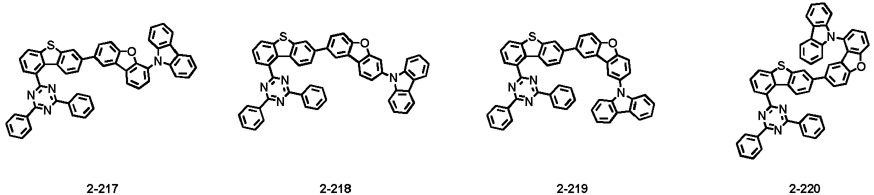

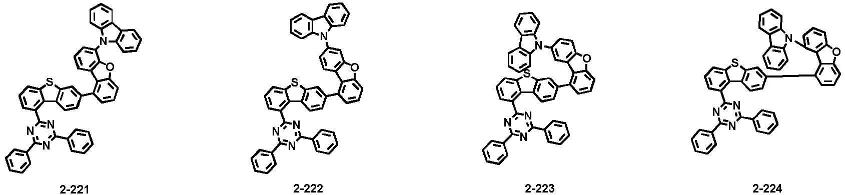

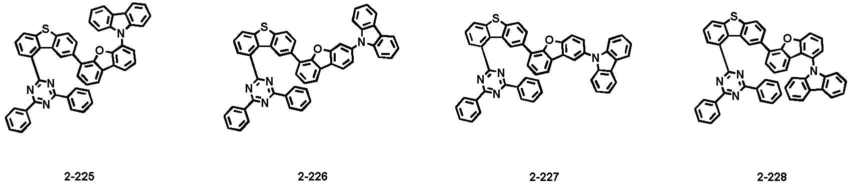

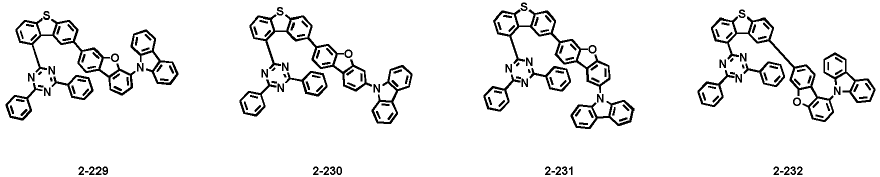

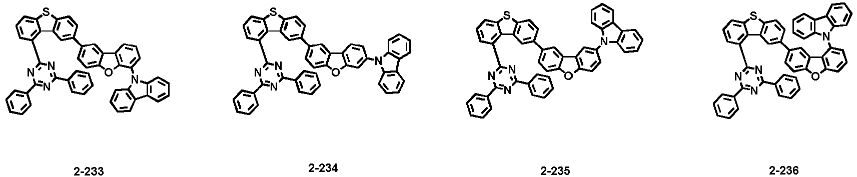

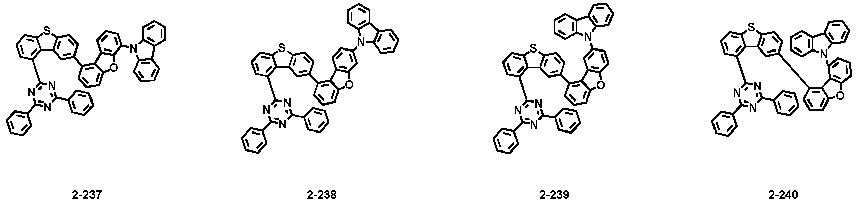

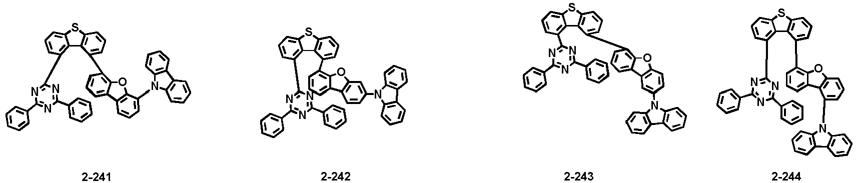

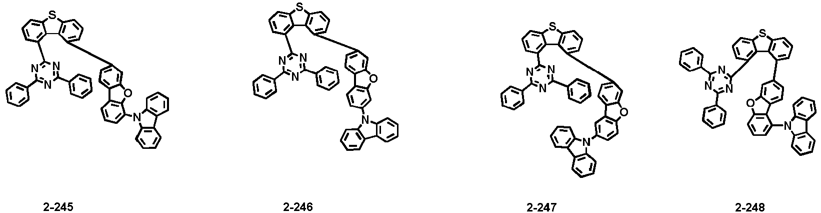

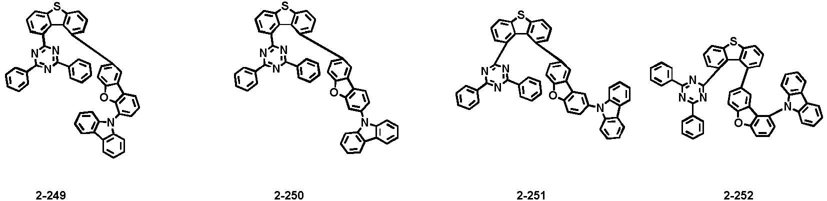

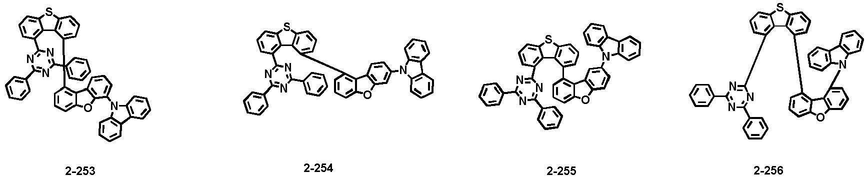

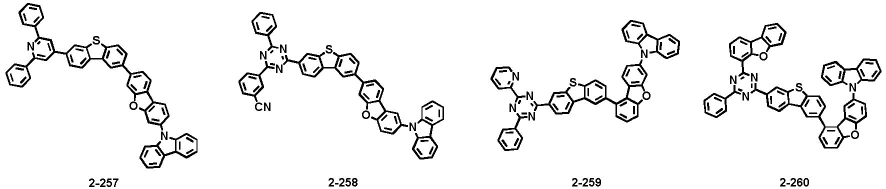

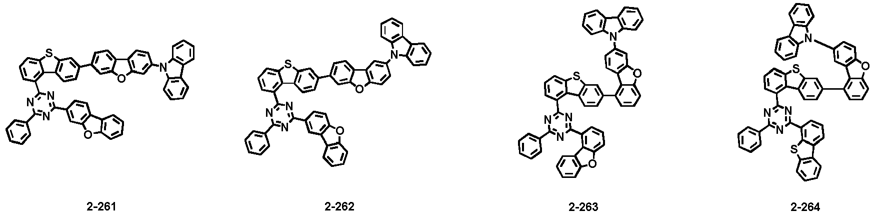

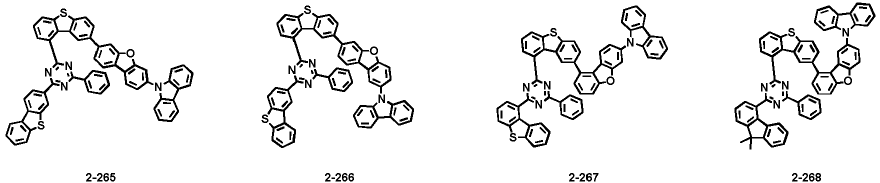

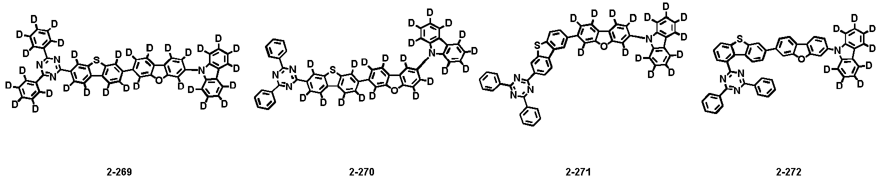

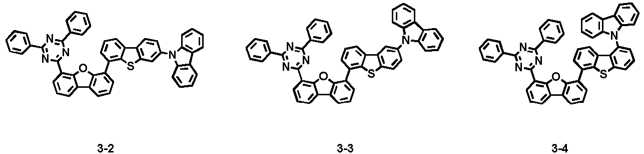

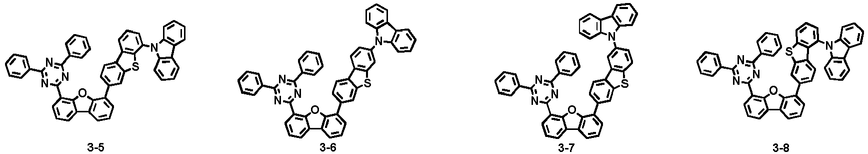

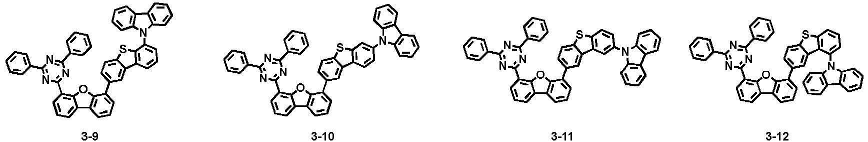

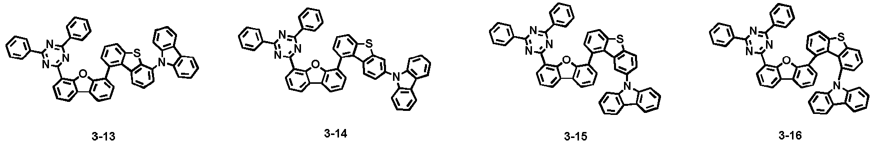

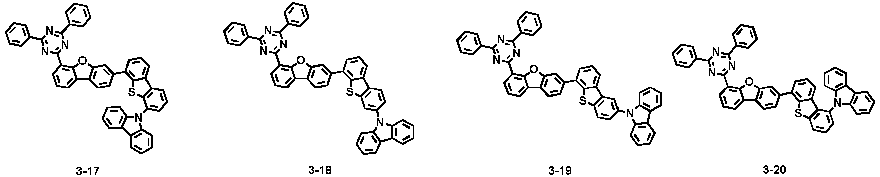

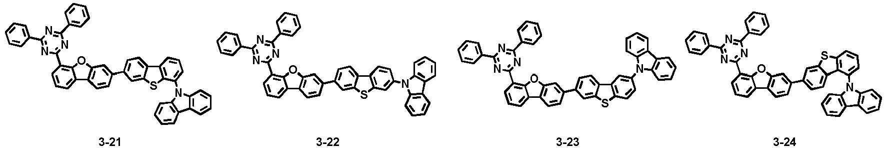

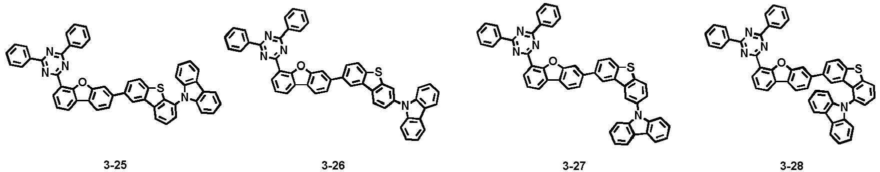

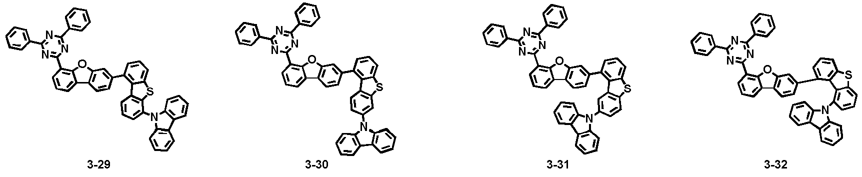

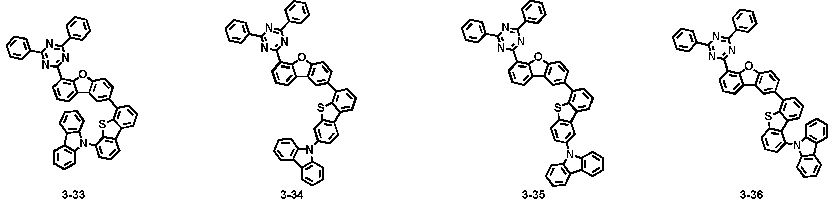

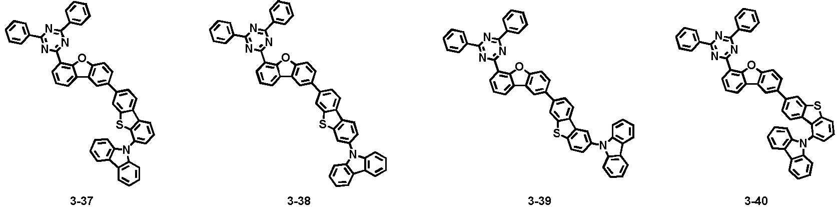

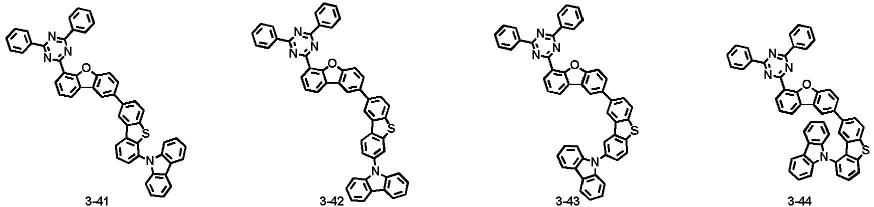

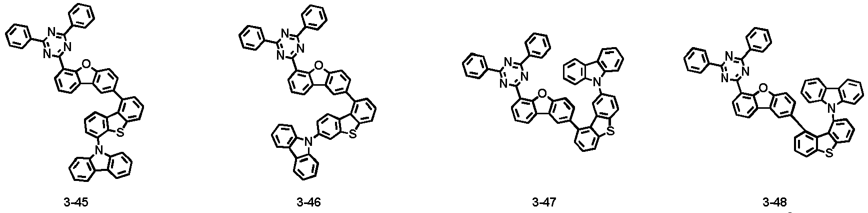

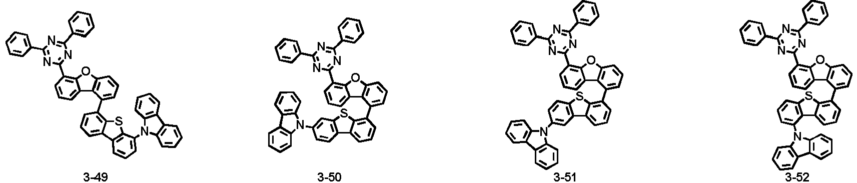

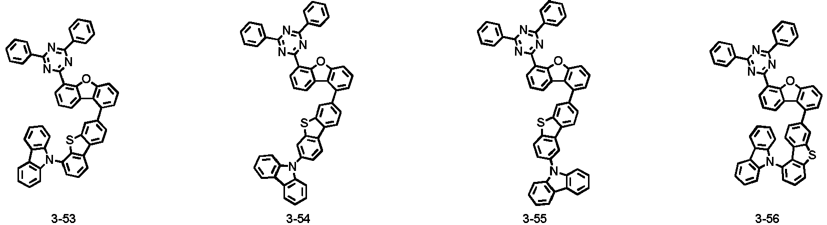

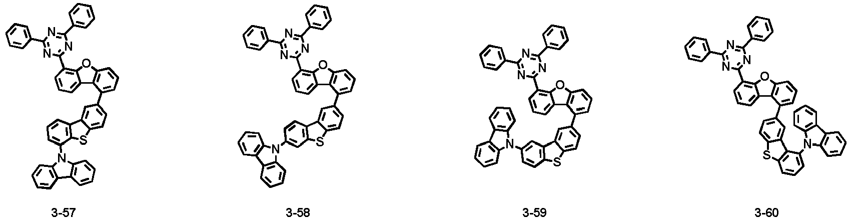

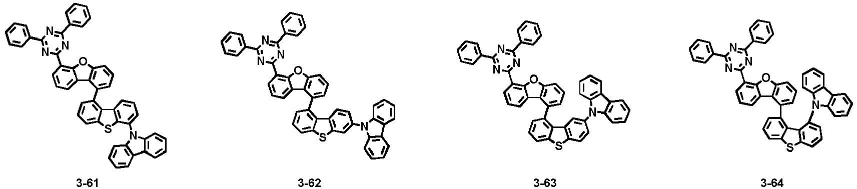

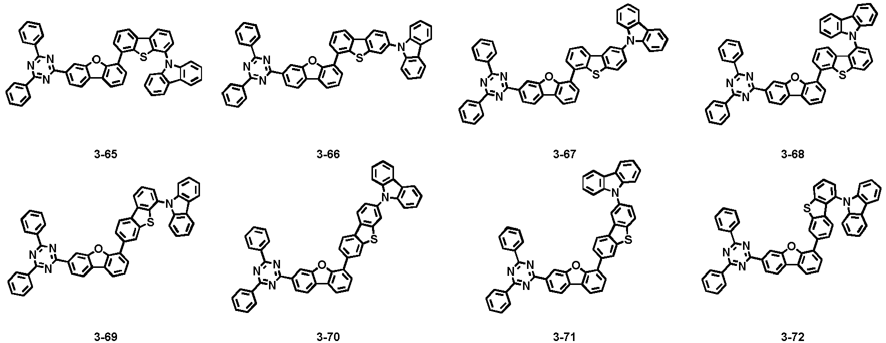

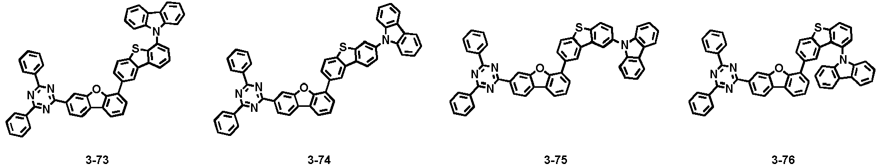

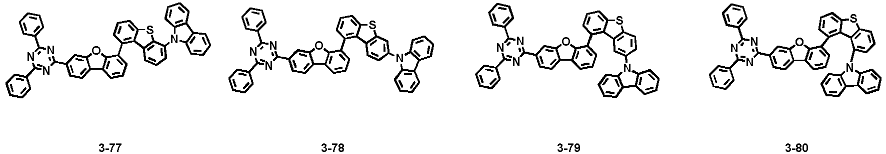

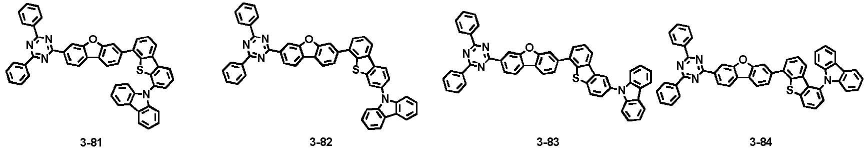

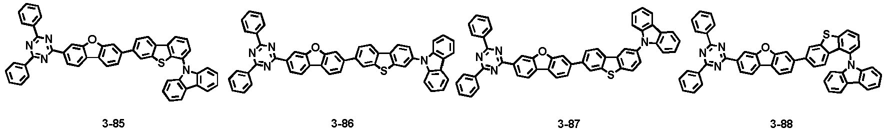

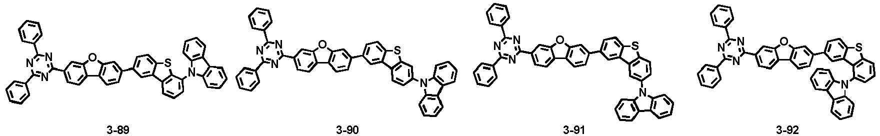

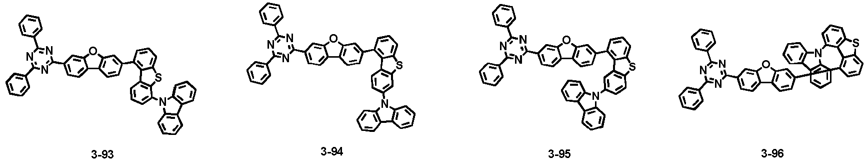

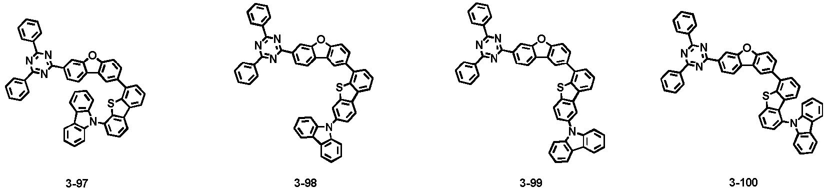

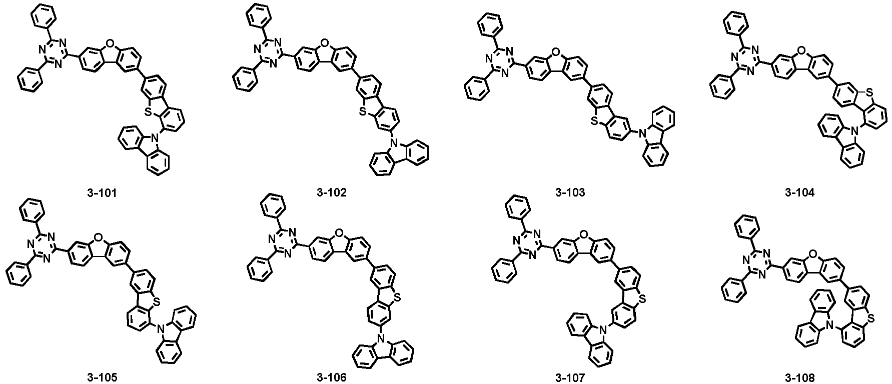

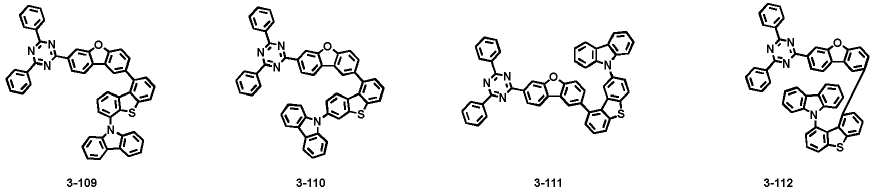

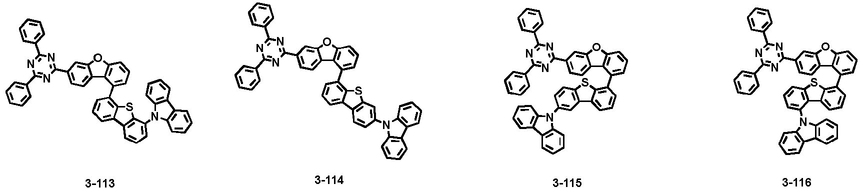

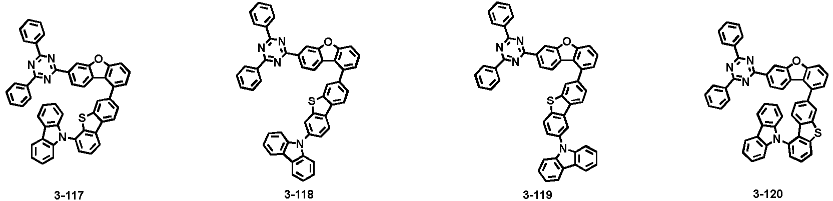

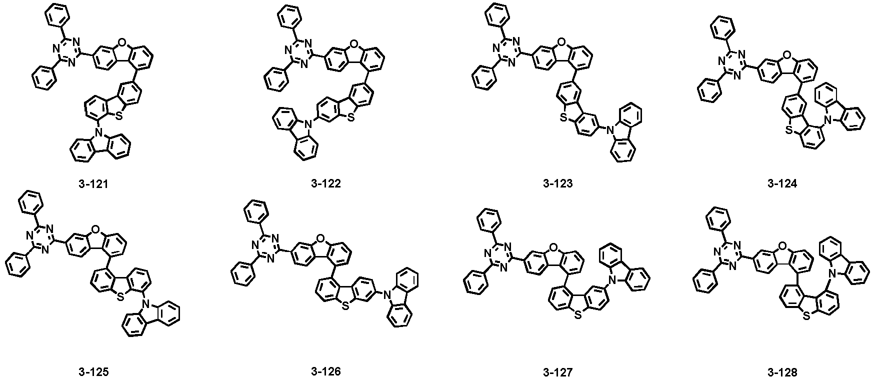

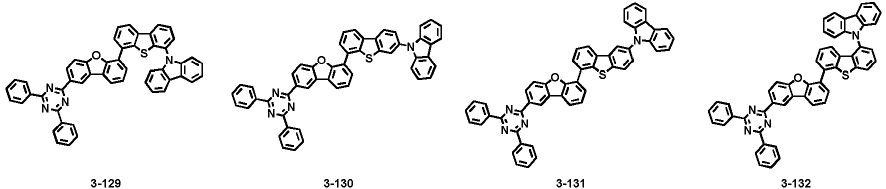

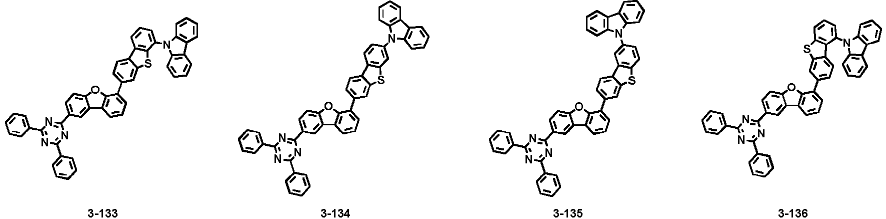

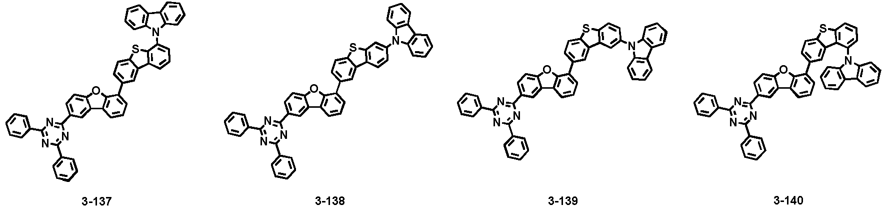

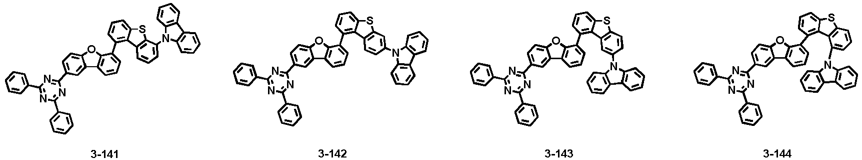

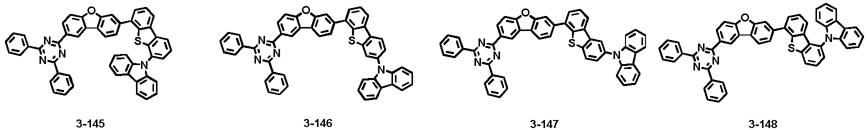

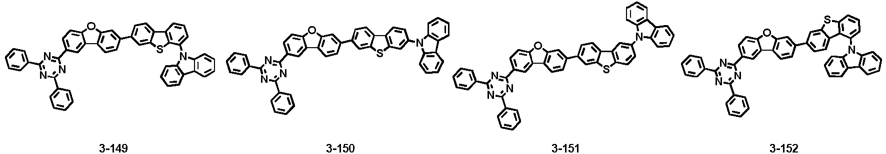

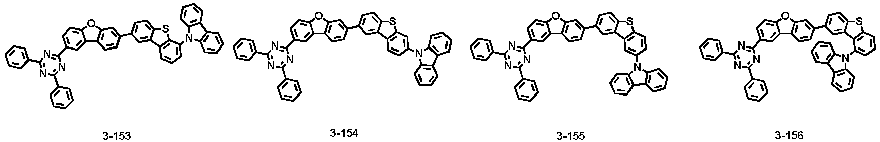

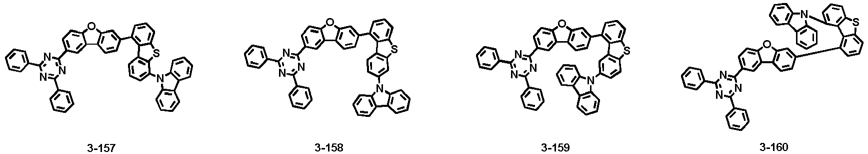

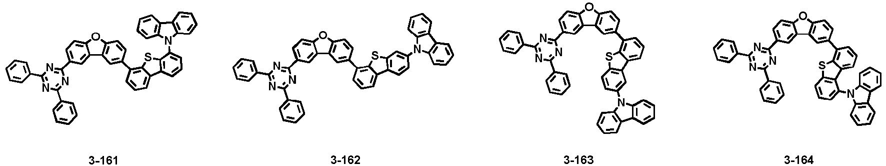

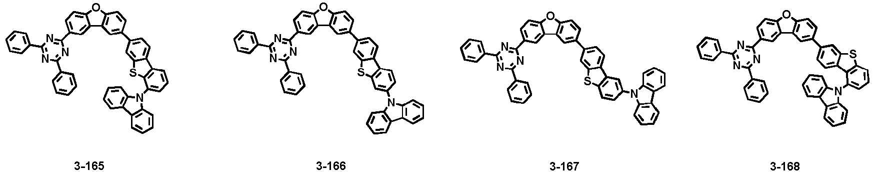

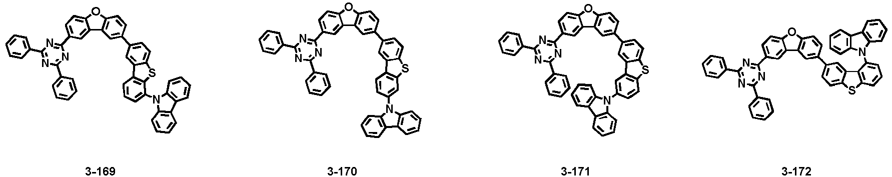

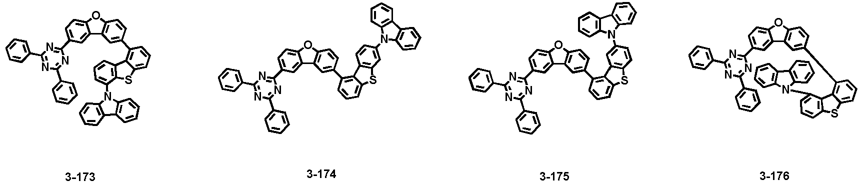

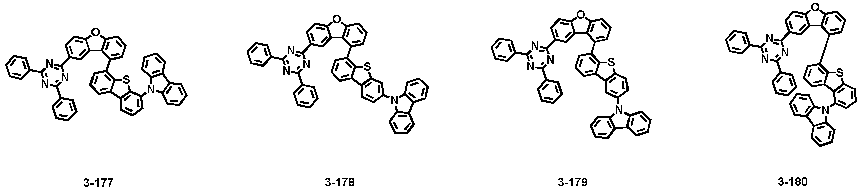

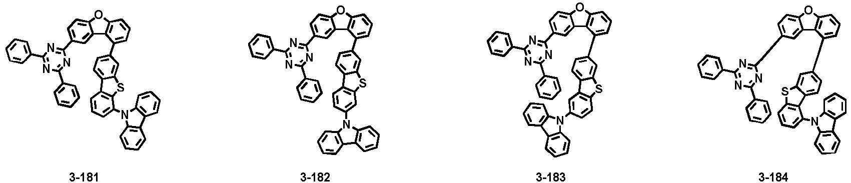

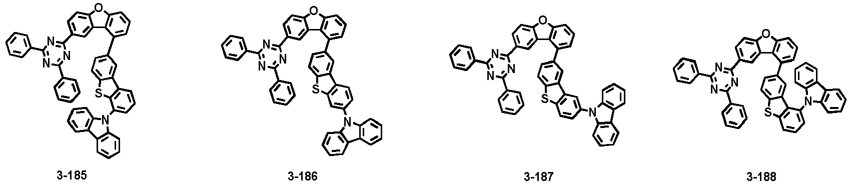

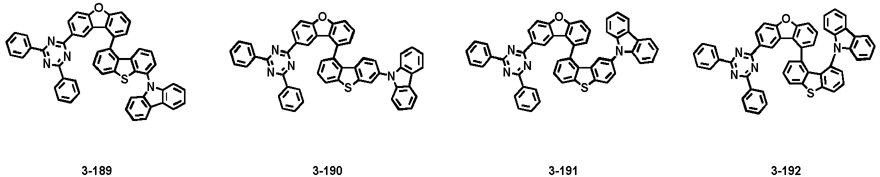

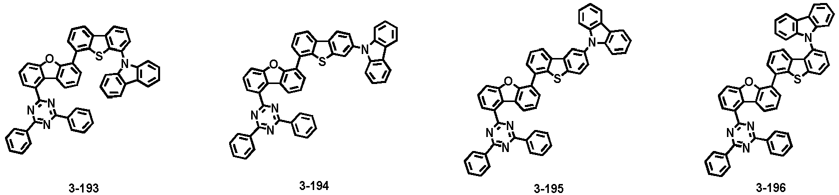

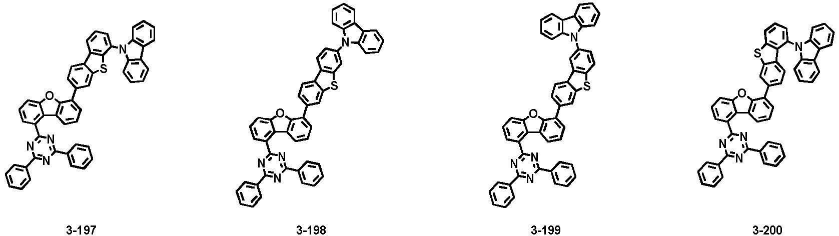

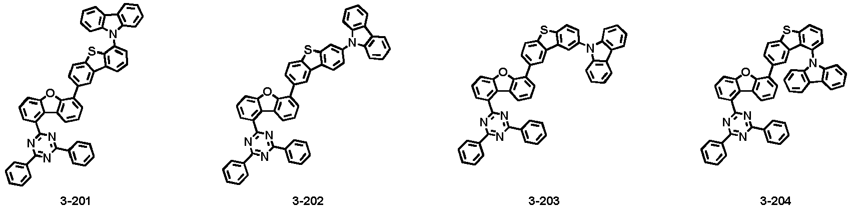

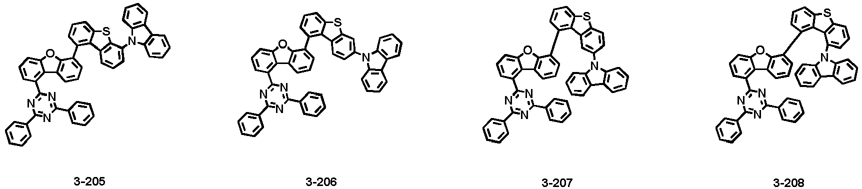

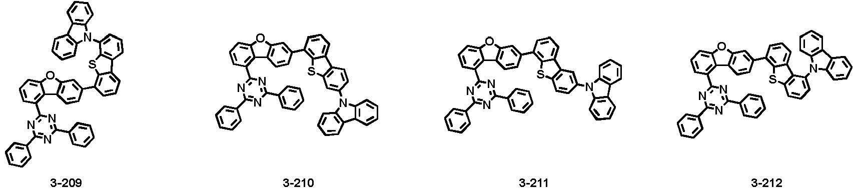

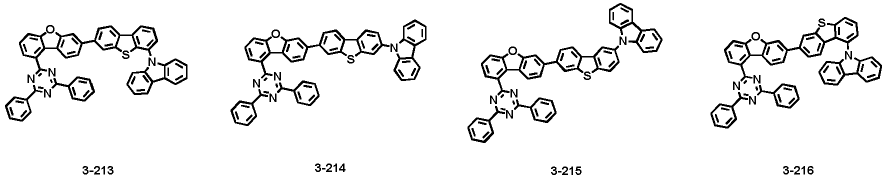

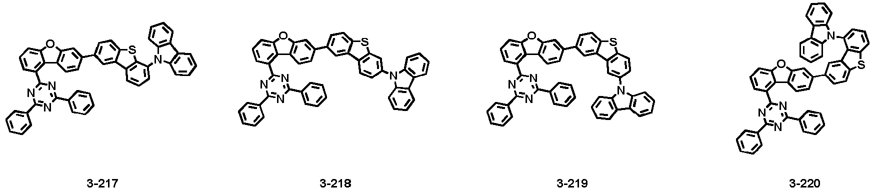

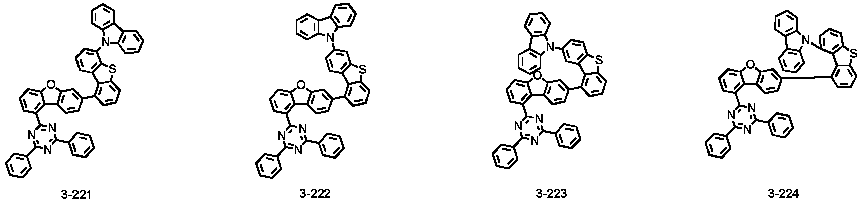

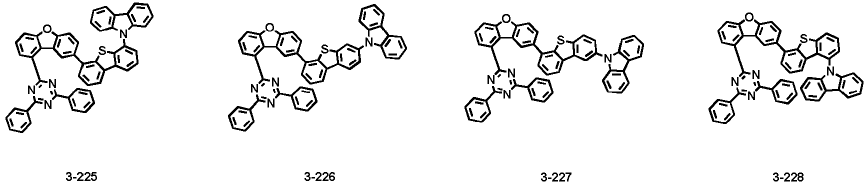

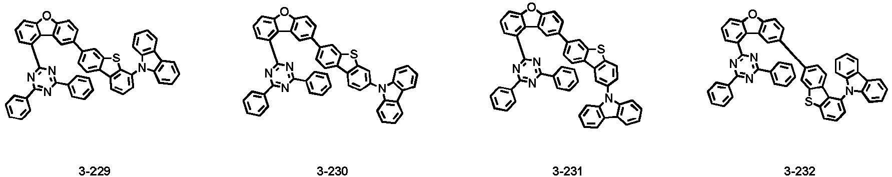

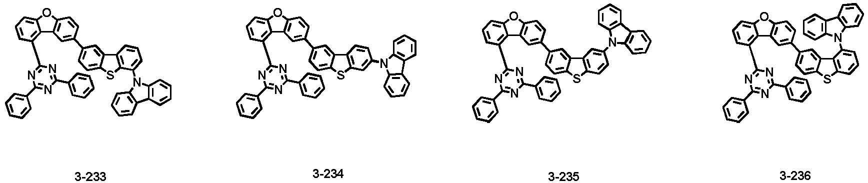

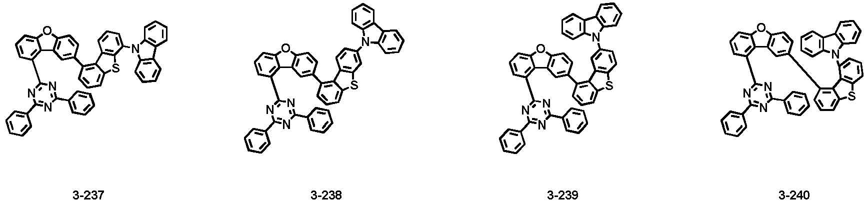

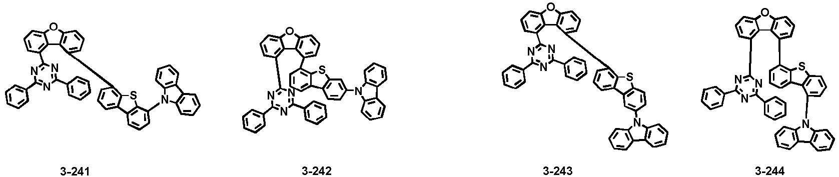

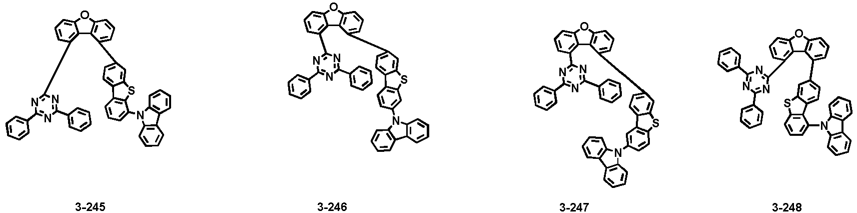

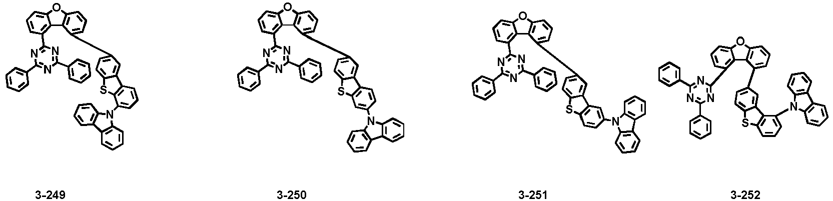

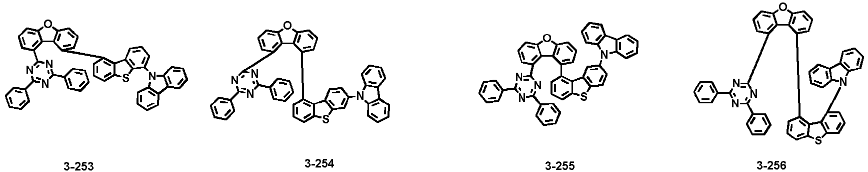

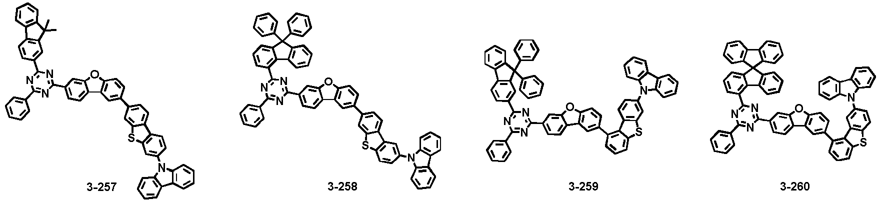

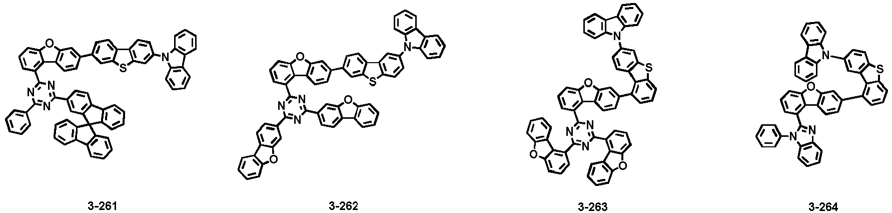

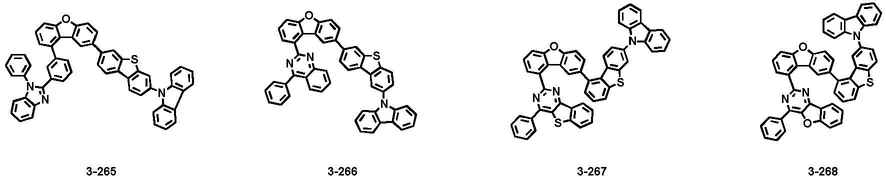

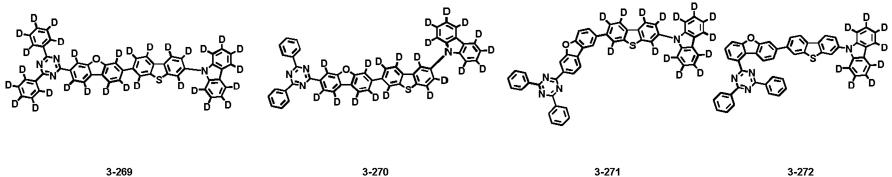

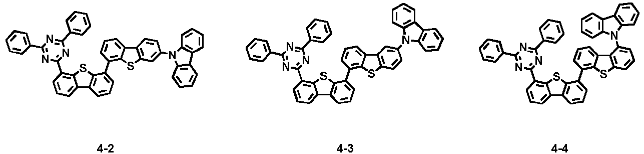

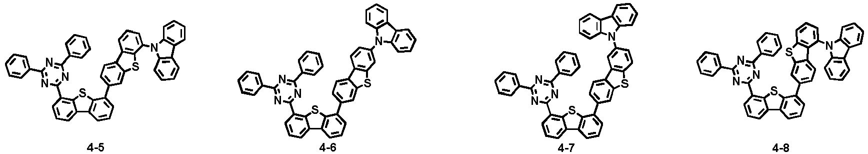

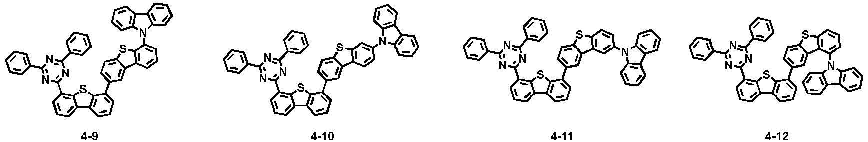

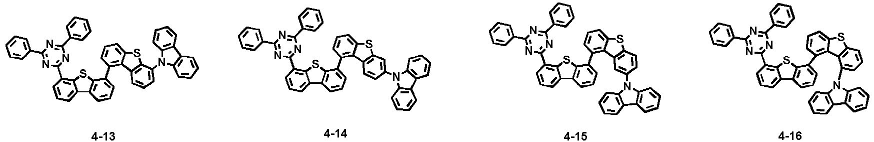

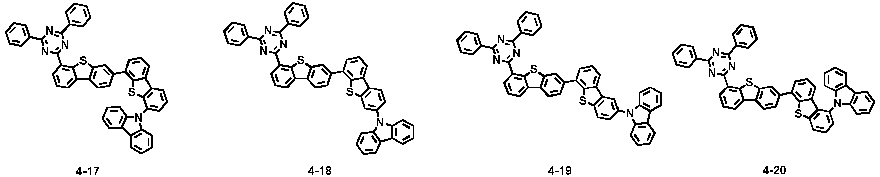

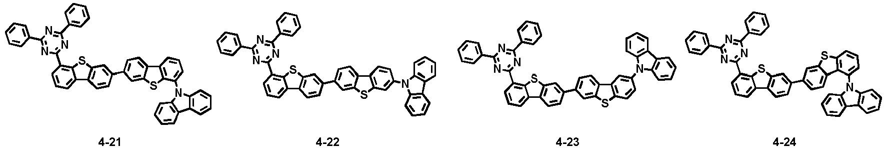

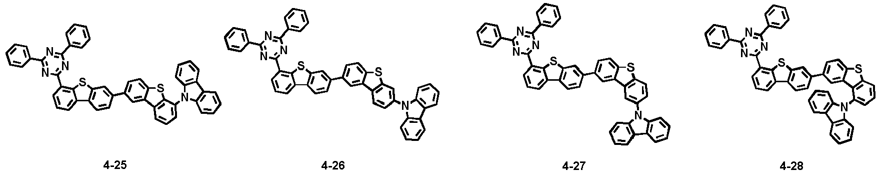

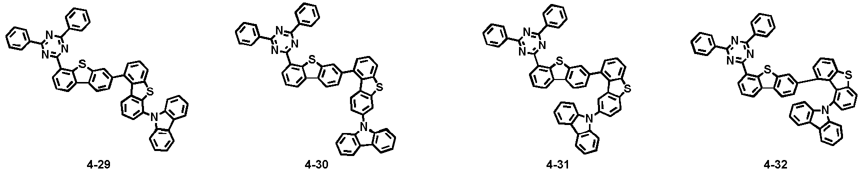

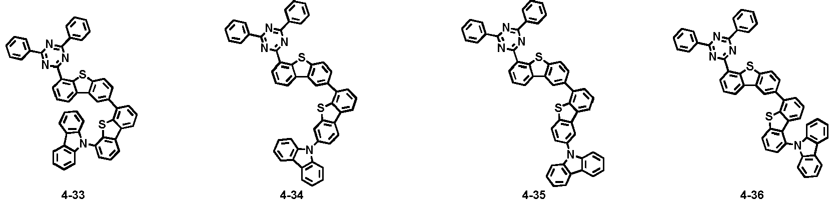

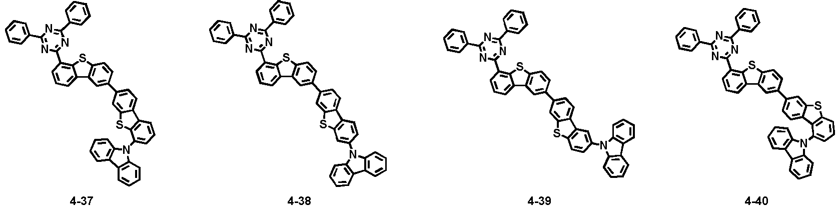

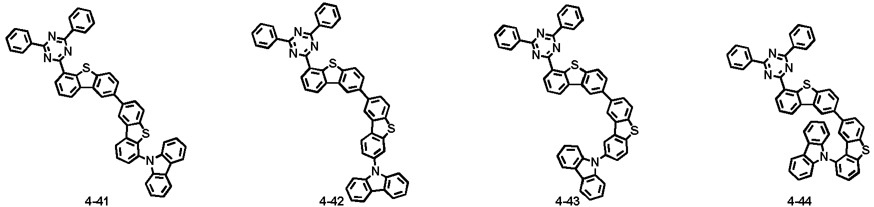

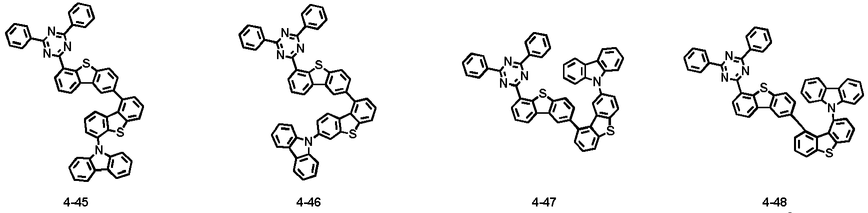

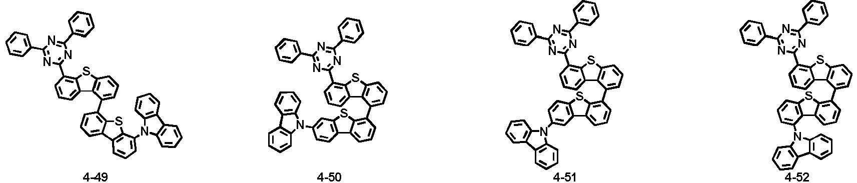

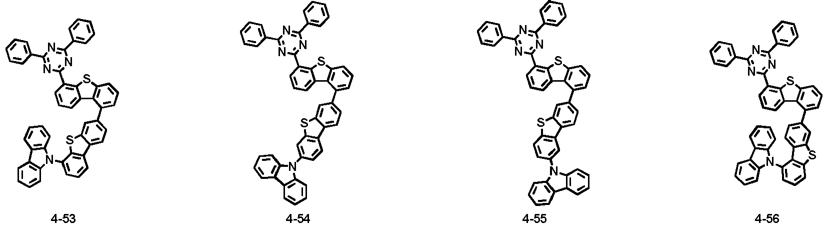

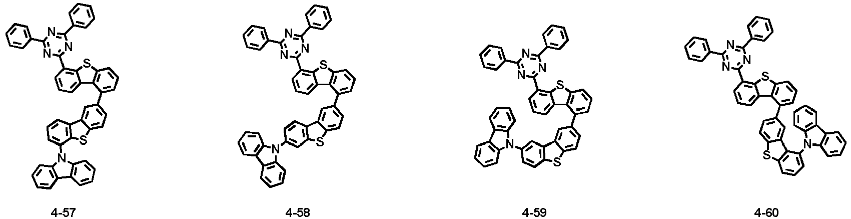

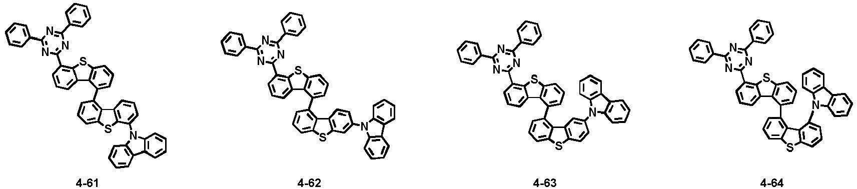

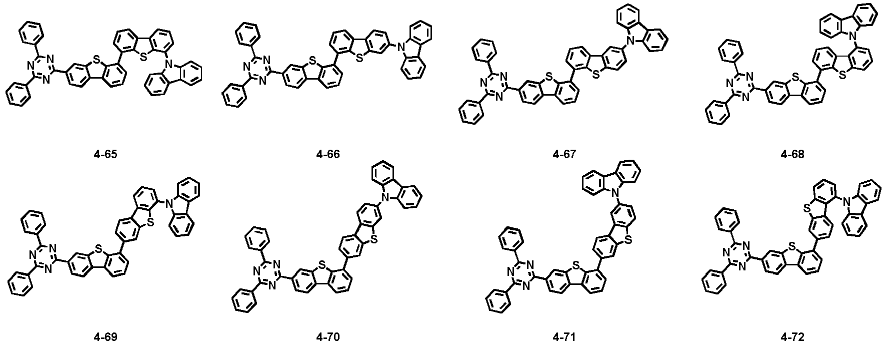

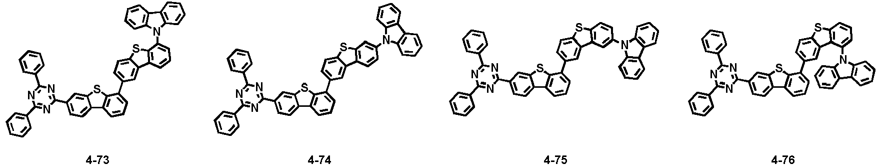

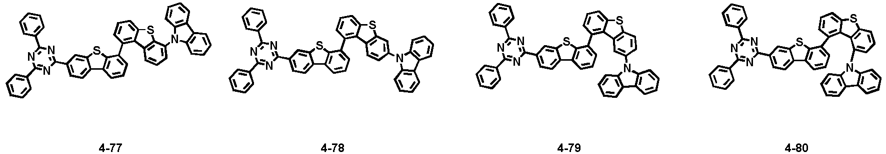

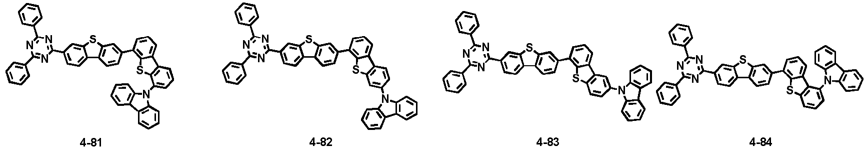

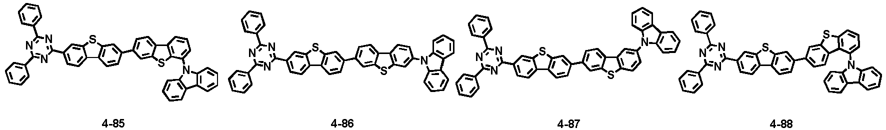

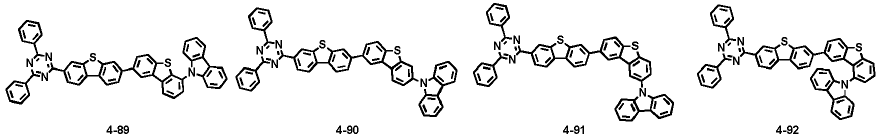

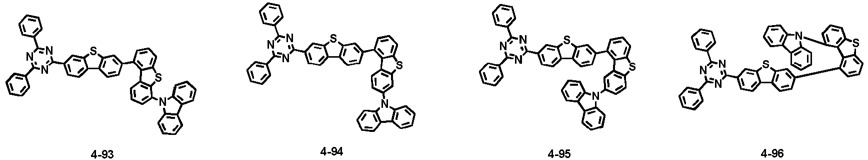

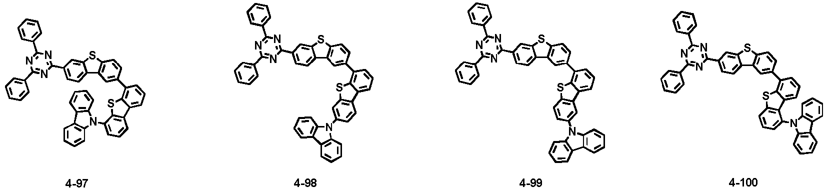

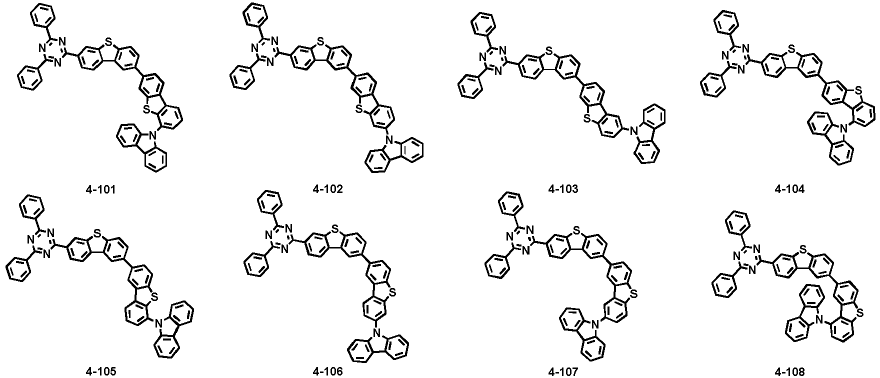

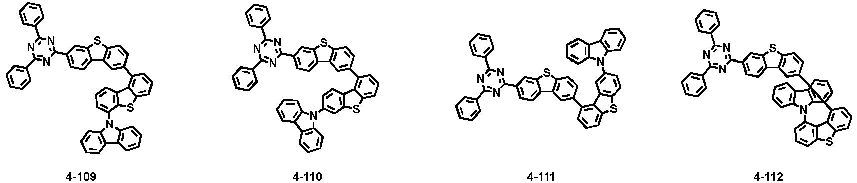

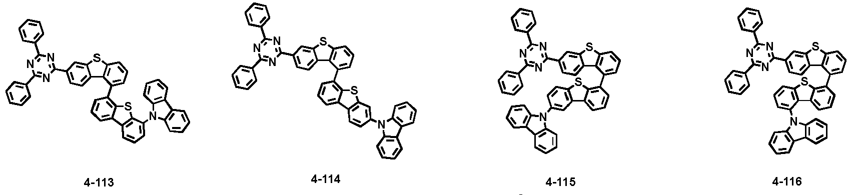

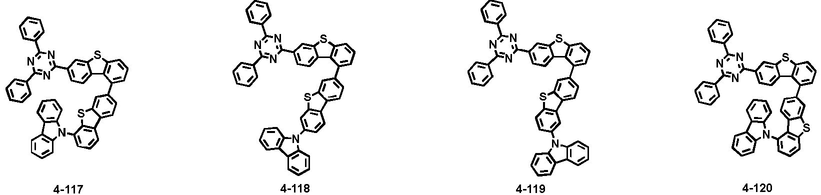

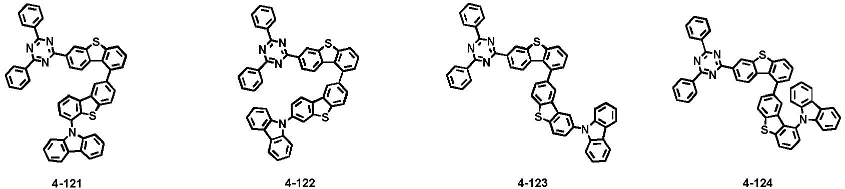

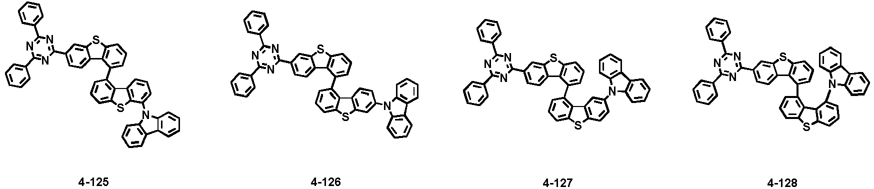

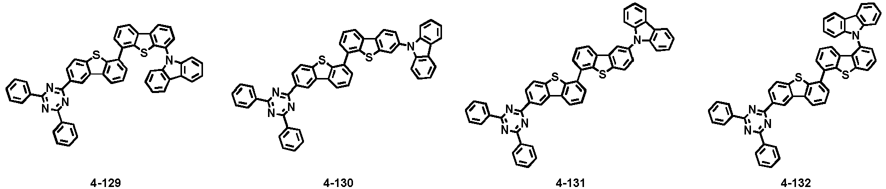

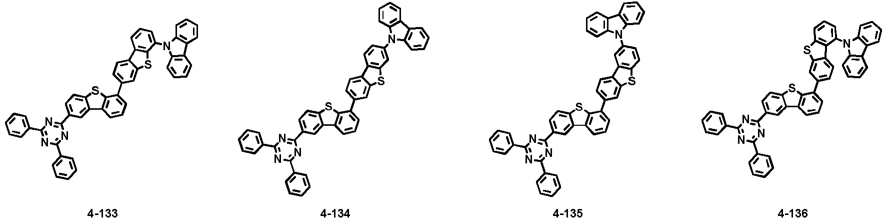

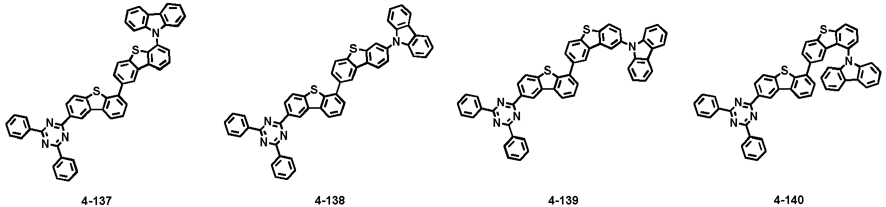

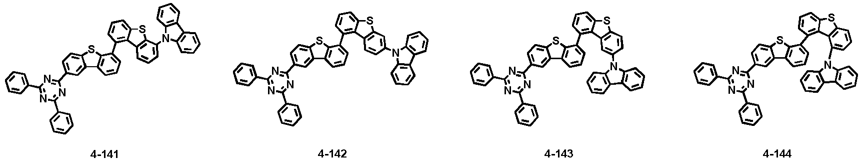

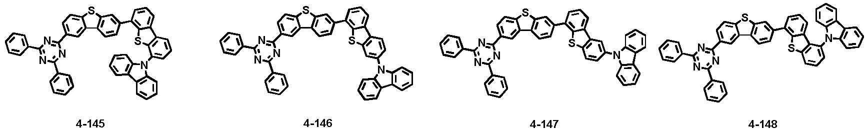

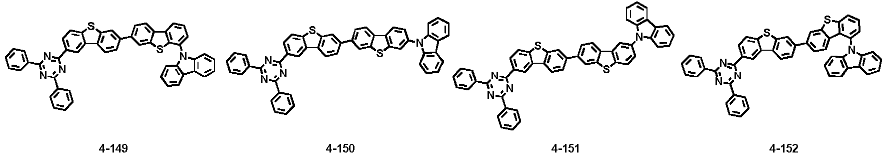

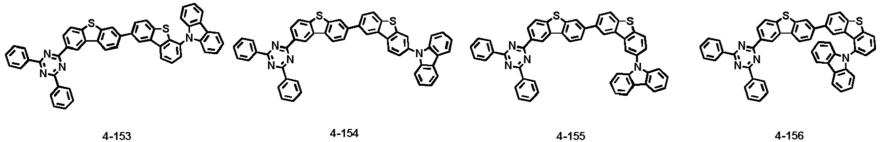

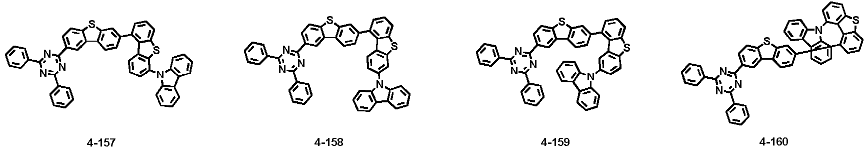

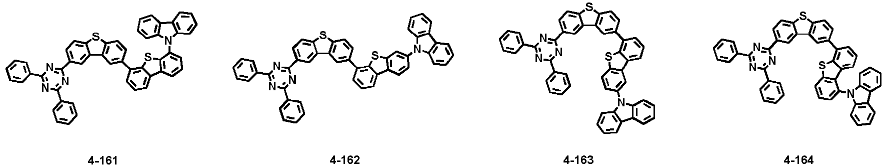

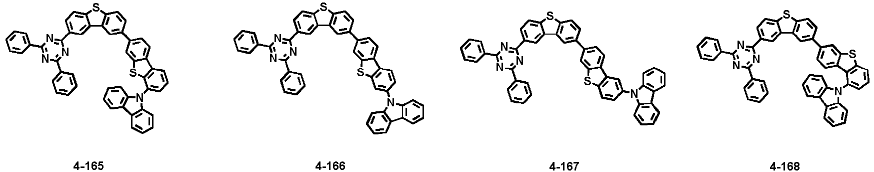

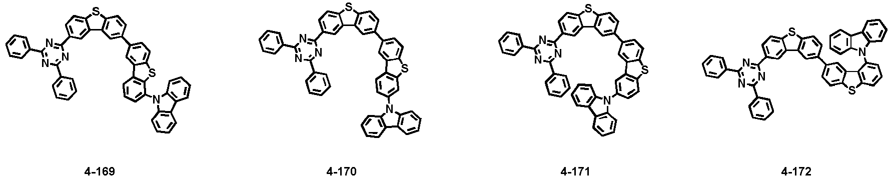

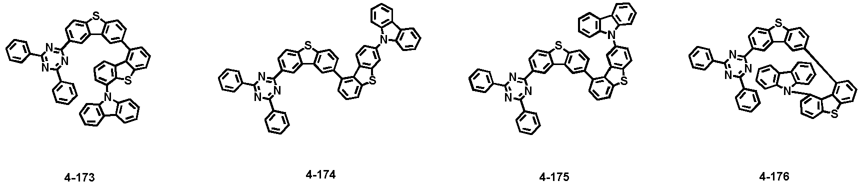

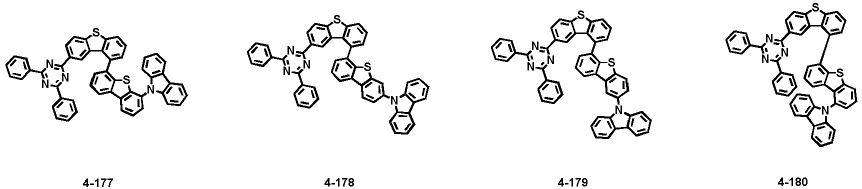

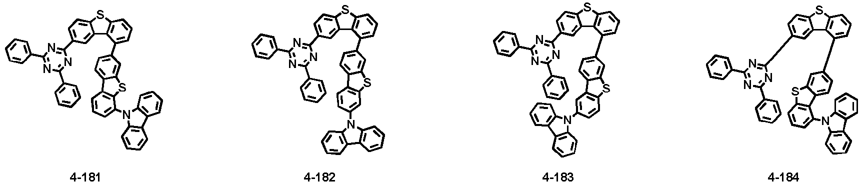

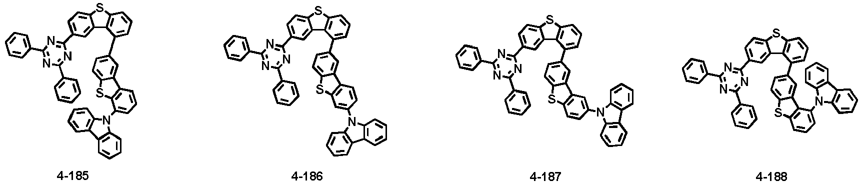

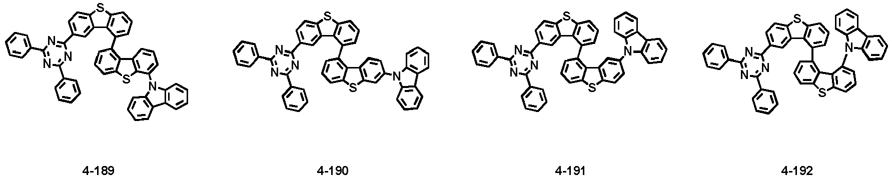

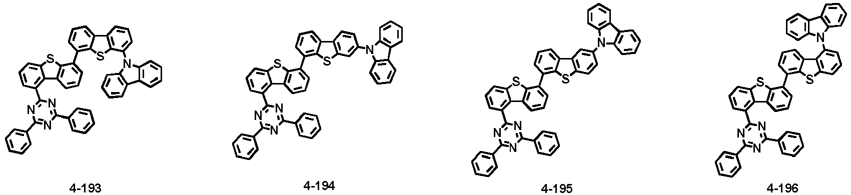

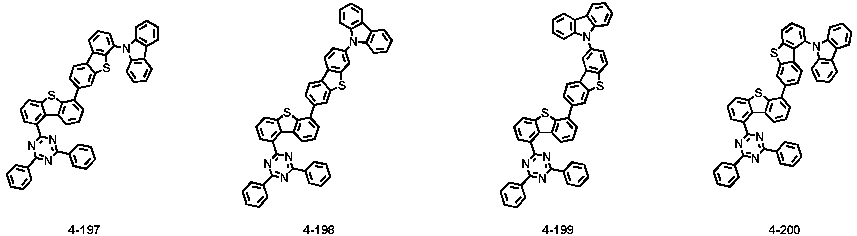

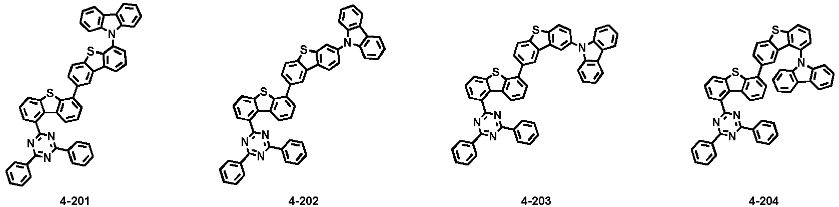

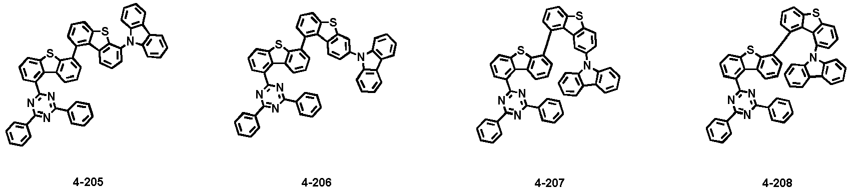

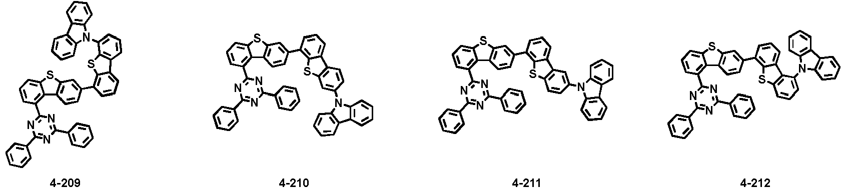

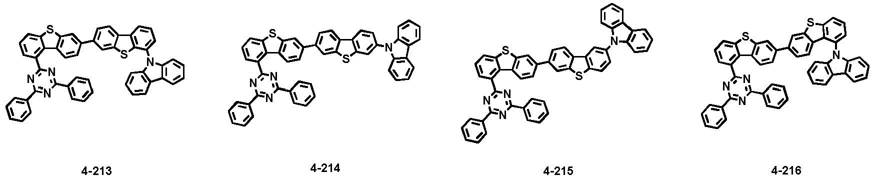

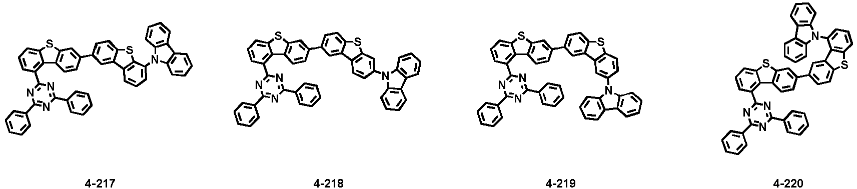

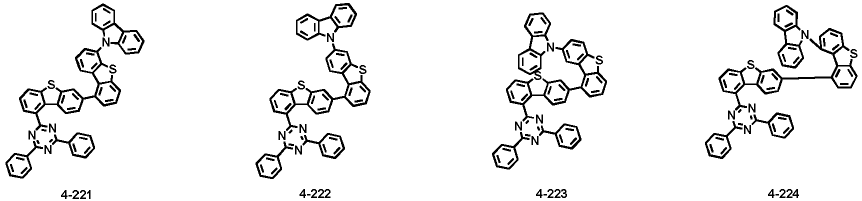

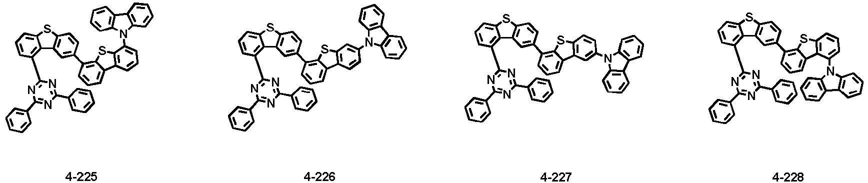

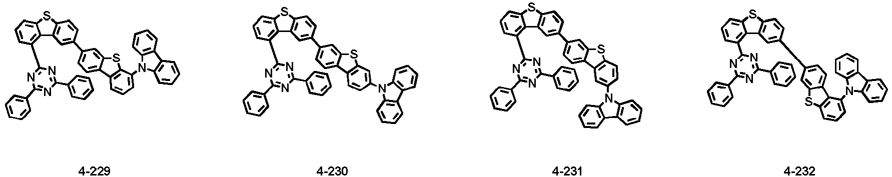

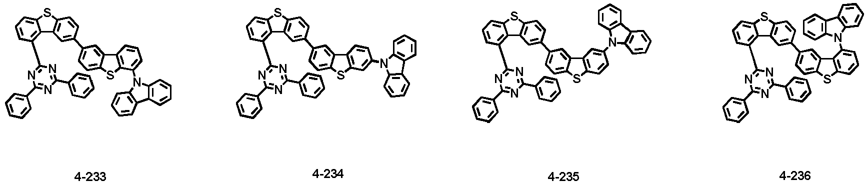

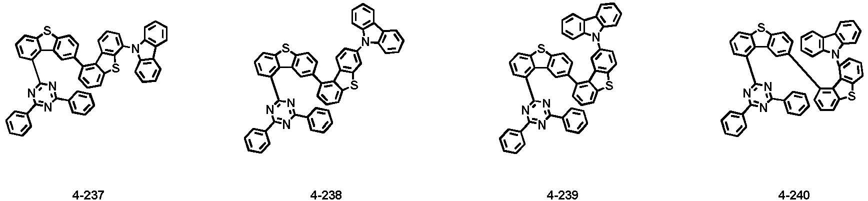

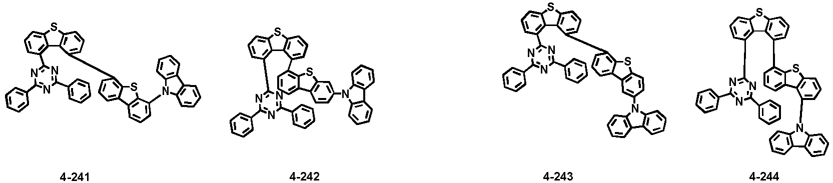

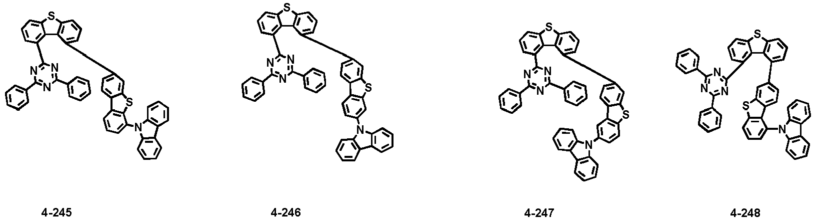

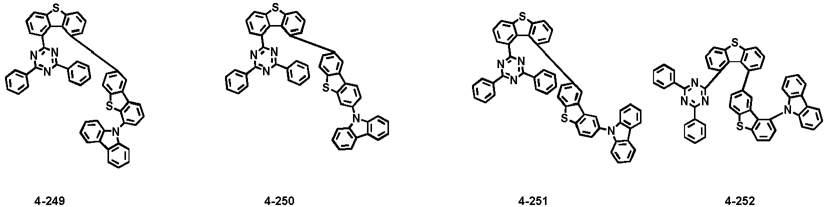

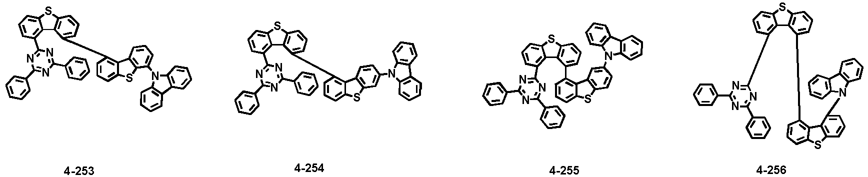

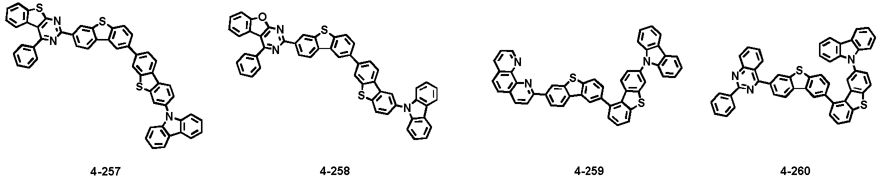

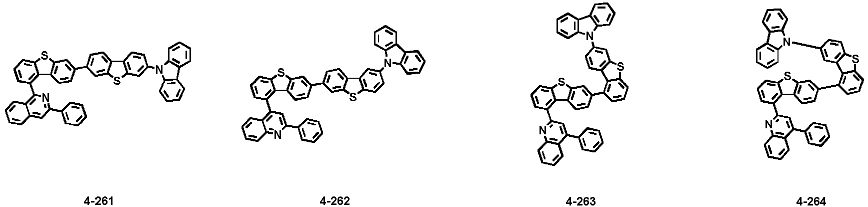

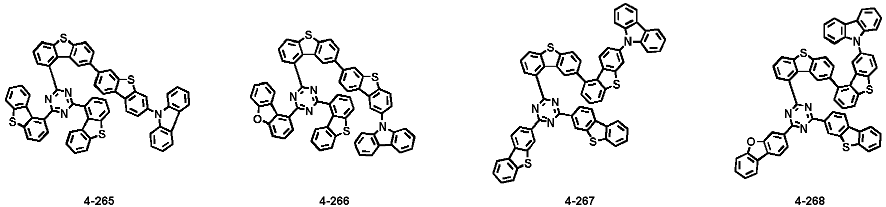

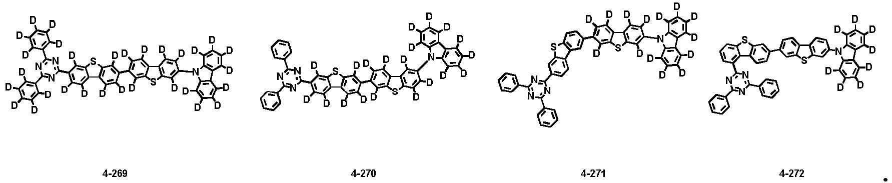

4. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 3.

5. The organic light emitting device of Claim 4, wherein the organic material layer comprises one or more light emitting layers, and the light emitting layer comprises the heterocyclic compound.

6. The organic light emitting device of Claim 5, wherein the light emitting layer comprises two or more host materials, and at least one of the host materials comprises the heterocyclic compound as a host material of a light emitting material.

7. The organic light emitting device of Claim 4, further comprising one, two or more layers selected from the group consisting of a light emitting layer, a hole injection layer, a hole transfer layer, an electron injection layer, an electron transfer layer, a hole auxiliary layer and a hole blocking layer.

8. The organic light emitting device of Claim 4, wherein the organic material layer comprises the heterocyclic compound of any one of Claims 1 to 3 as a first compound, and further comprises a heterocyclic compound represented by the following Chemical Formula 13 or 14 as a second compound: 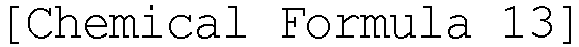 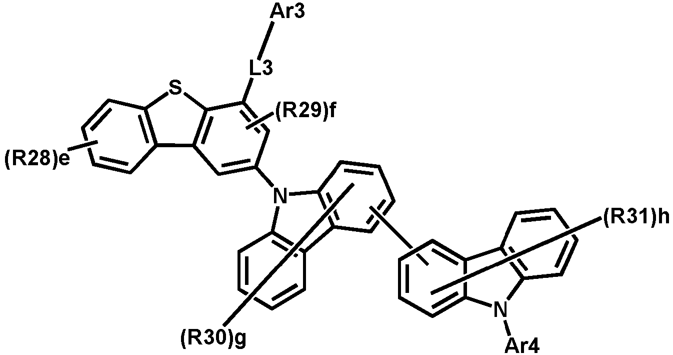 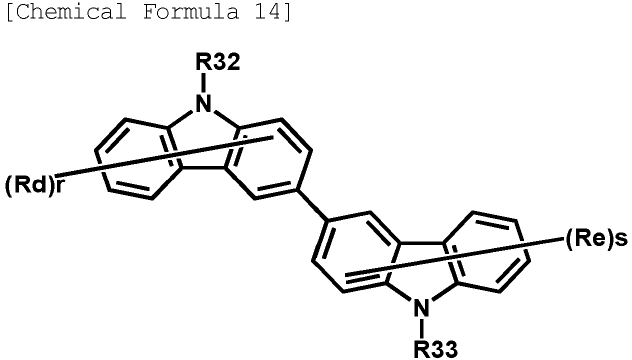 in Chemical Formulae 13 and 14,
L3 is a direct bond; a substituted or unsubstituted arylene group having 6 to 60 carbon atoms; or a substituted or unsubstituted heteroarylene group having 2 to 60 carbon atoms;
R28 to R31, Rd and Re are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aromatic hydrocarbon ring having 6 to 60 carbon atoms or a substituted or unsubstituted heteroring having 2 to 60 carbon atoms, r and s are an integer of 0 to 7, and when r is 2 or greater, Rds are the same as or different from each other, when s is 2 or greater, Res are the same as or different from each other, e is an integer of 0 to 4, g and h are each an integer of 0 to 7, f is an integer of 0 to 2, and when e is 2 or greater, R28s are the same as or different from each other, when f is an integer of 2, R29s are the same as or different from each other, when g is 2 or greater, R30s are the same as or different from each other, and when h is 2 or greater, R31s are the same as or different from each other;
Ar3 is a substituted or unsubstituted aryl group having 6 to 60 carbon atoms; or a substituted or unsubstituted heteroaryl group having 2 to 60 carbon atoms;
Ar4 is a substituted or unsubstituted aryl group having 6 to 60 carbon atoms; or a substituted or unsubstituted heteroaryl group having 2 to 60 carbon atoms; and
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms.

9. The organic light emitting device of Claim 8, wherein the heterocyclic compound represented by Chemical Formula 13 has a deuterium content of 0%, or greater than 10% and less than or equal to 100%.

10. The organic light emitting device of Claim 8, wherein the heterocyclic compound represented by Chemical Formula 14 has a deuterium of 0%%, or greater than 10% and less than or equal to 100%.

11. The organic light emitting device of Claim 8, wherein Chemical Formula 13 is represented by any one of the following compounds:

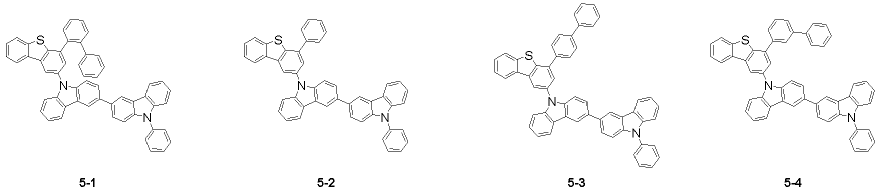

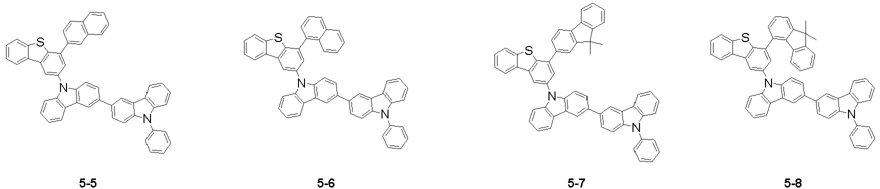

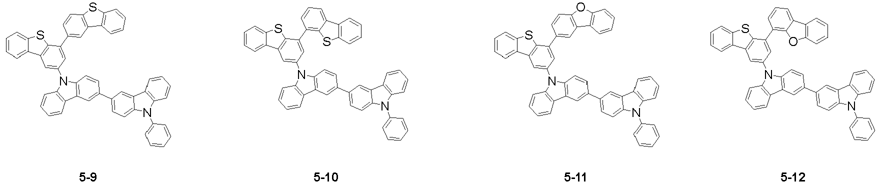

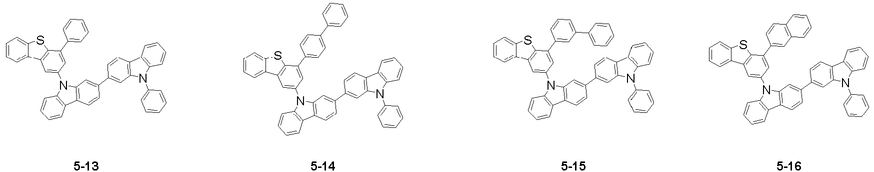

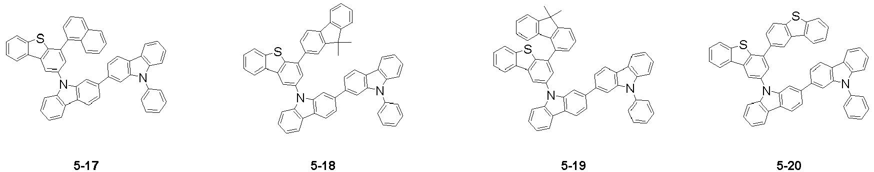

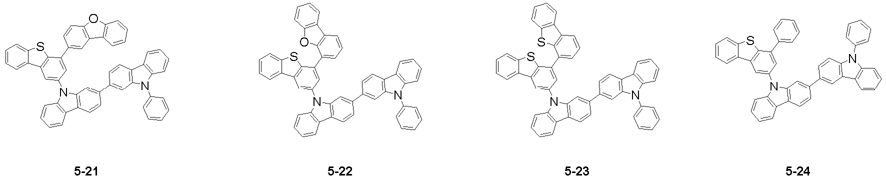

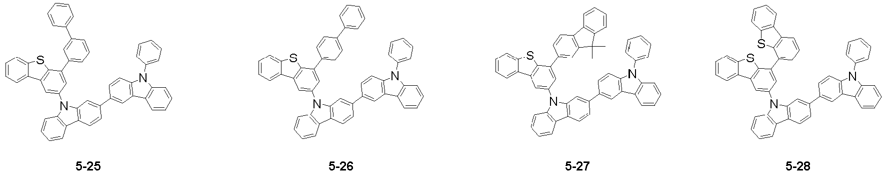

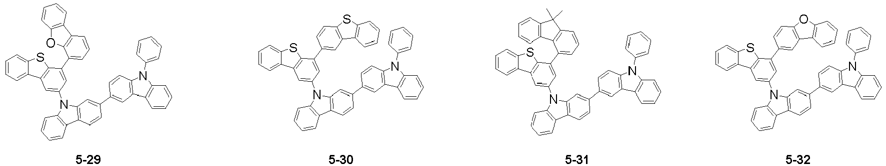

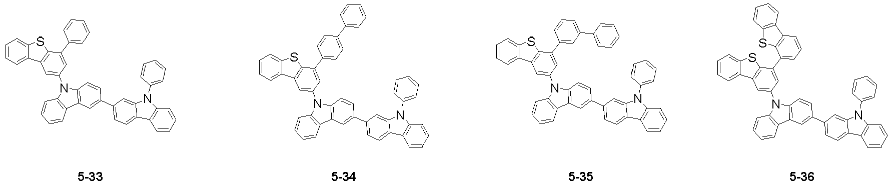

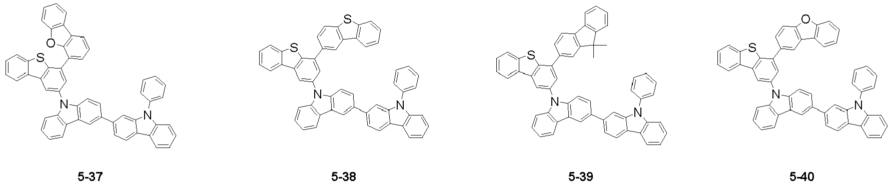

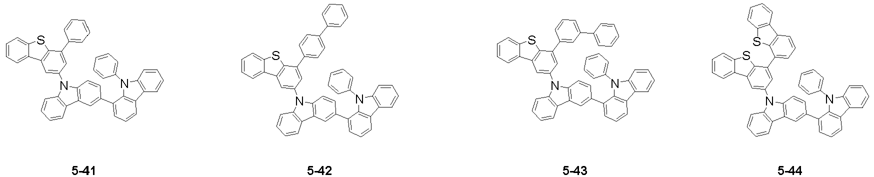

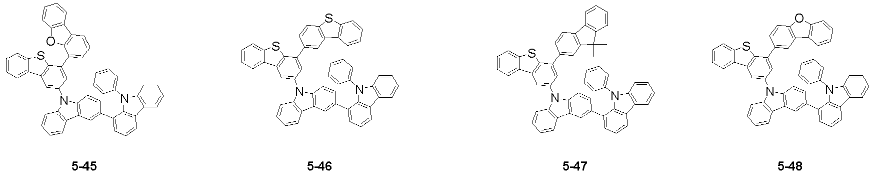

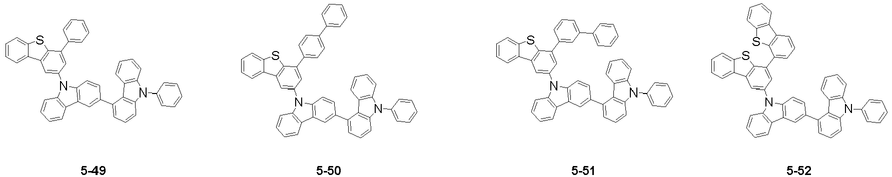

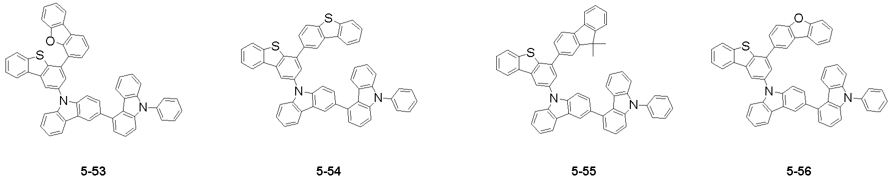

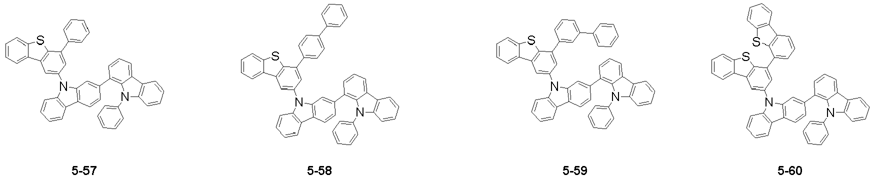

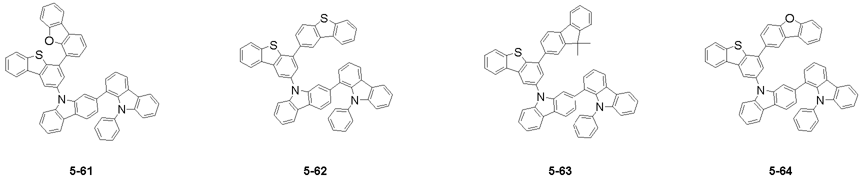

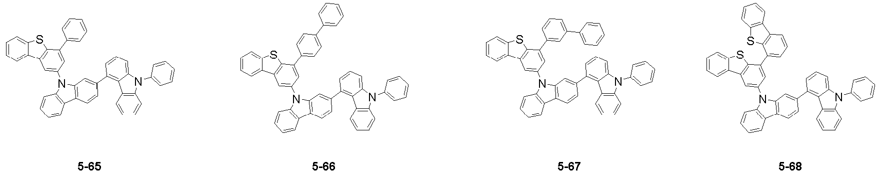

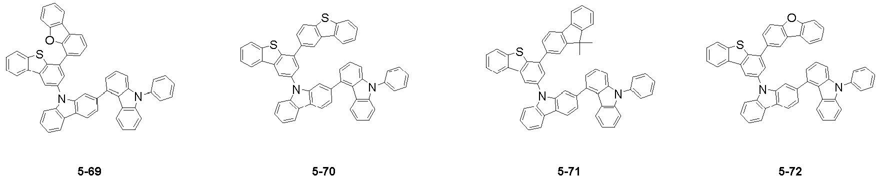

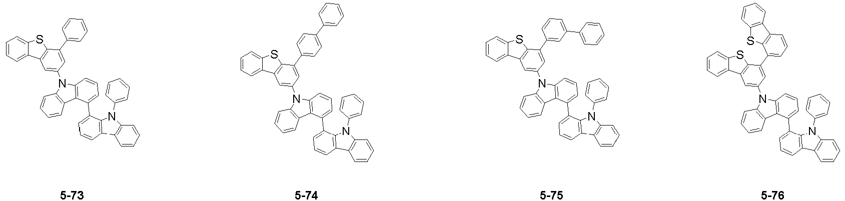

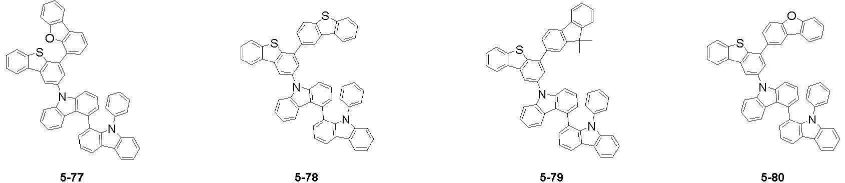

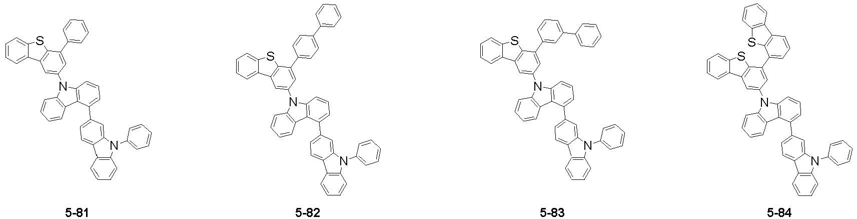

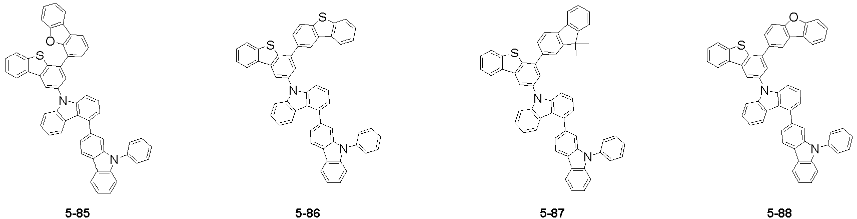

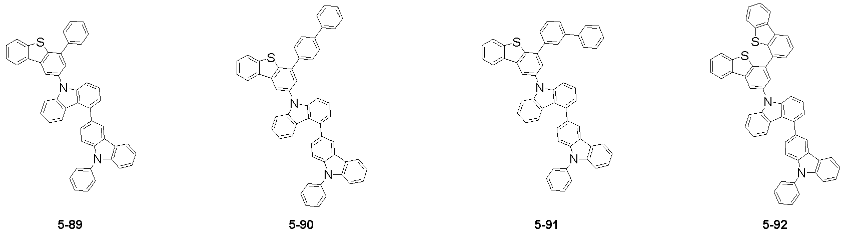

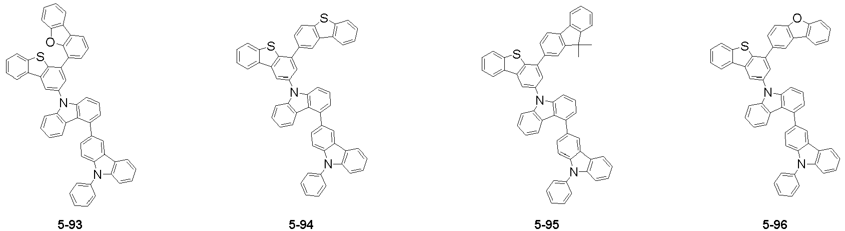

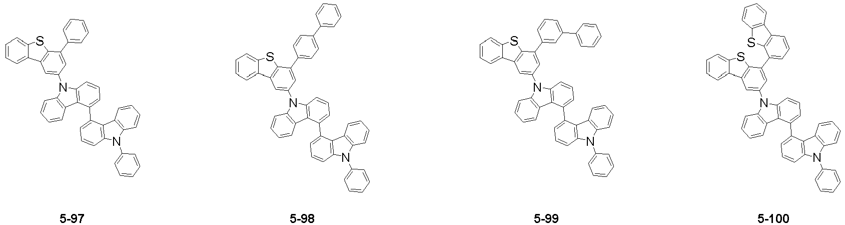

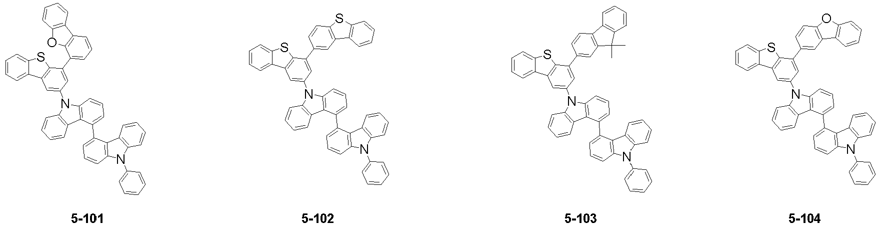

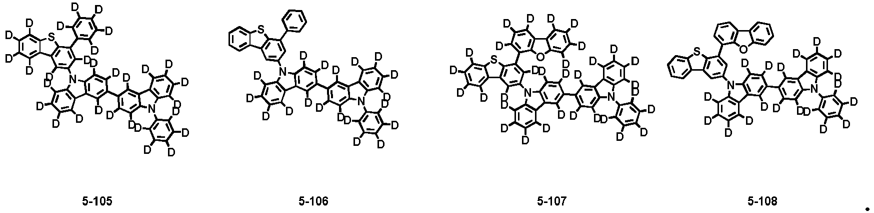

12. The organic light emitting device of Claim 9, wherein Chemical Formula 14 is represented by any one of the following compounds:

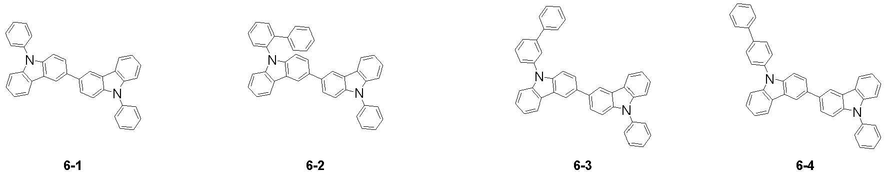

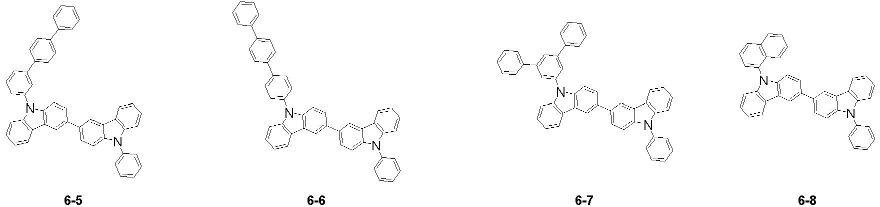

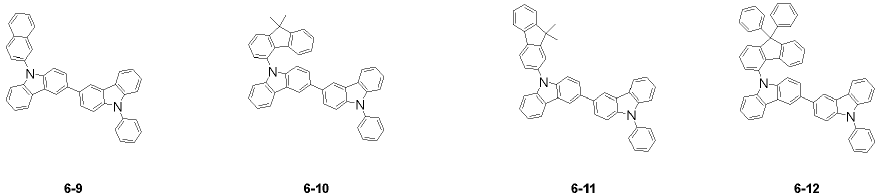

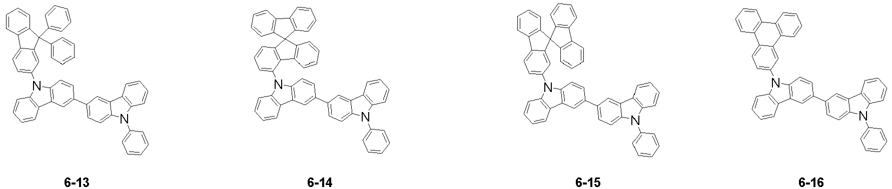

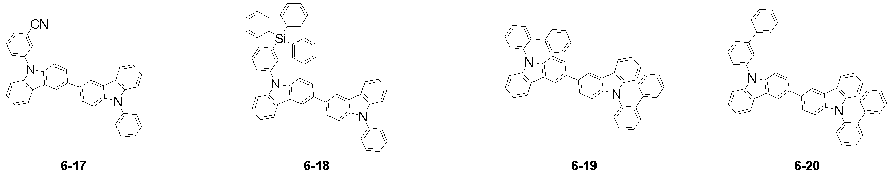

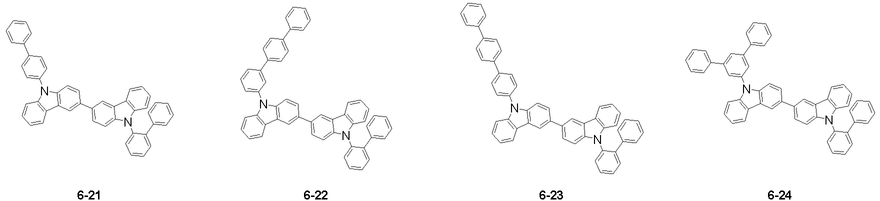

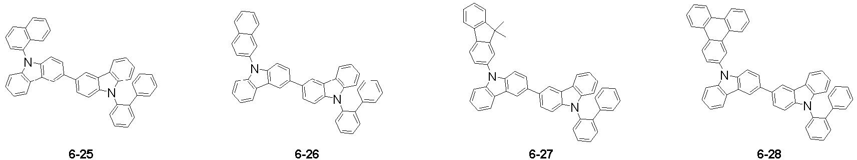

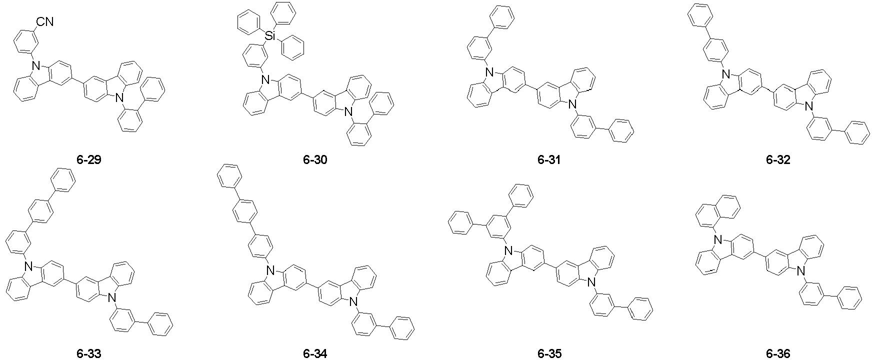

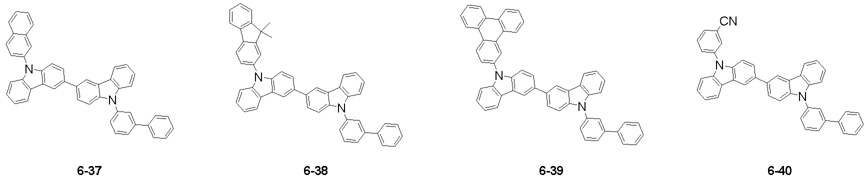

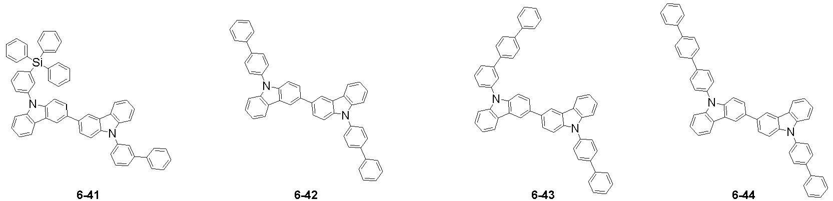

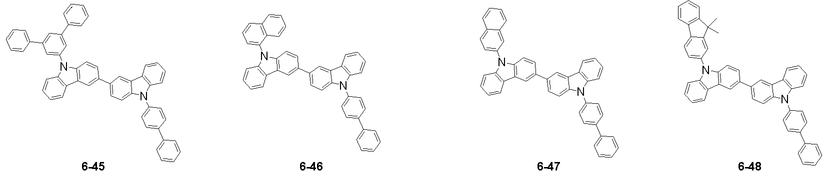

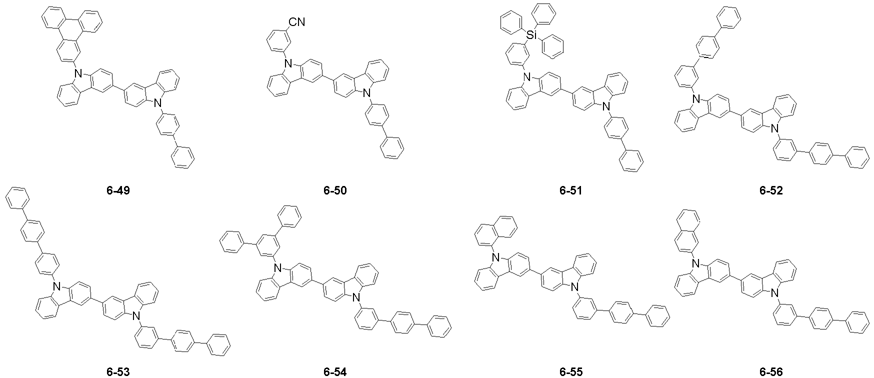

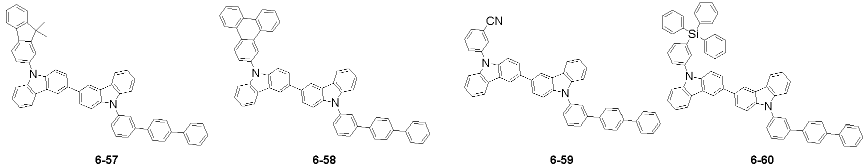

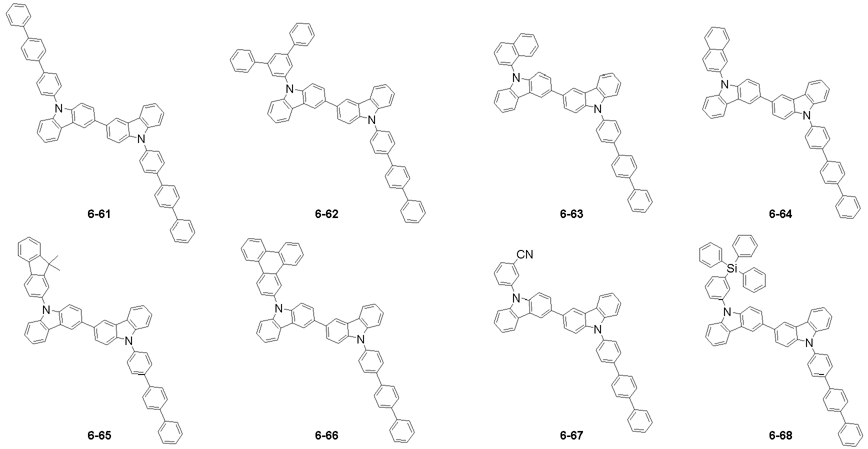

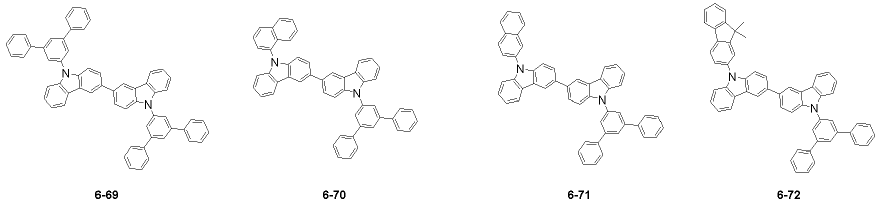

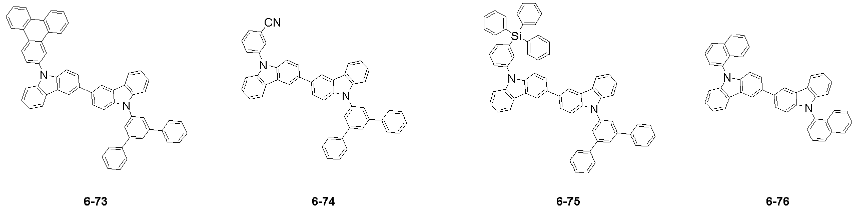

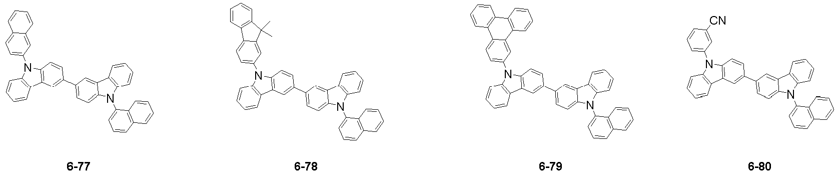

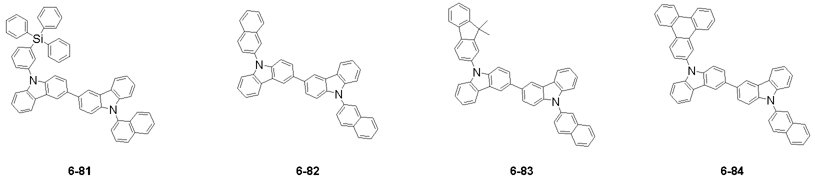

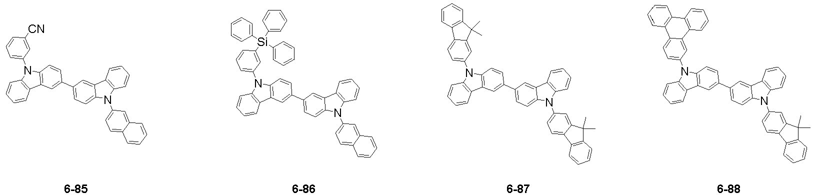

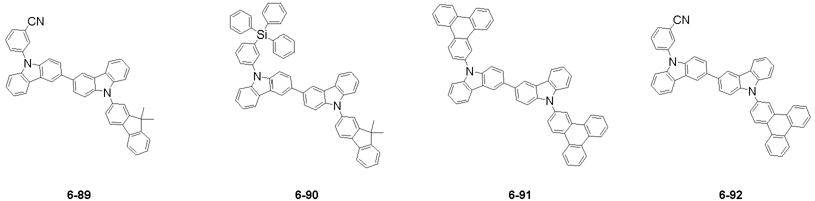

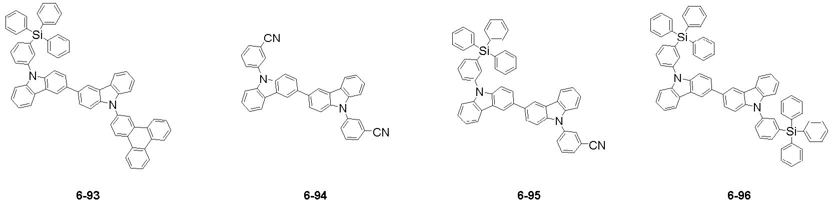

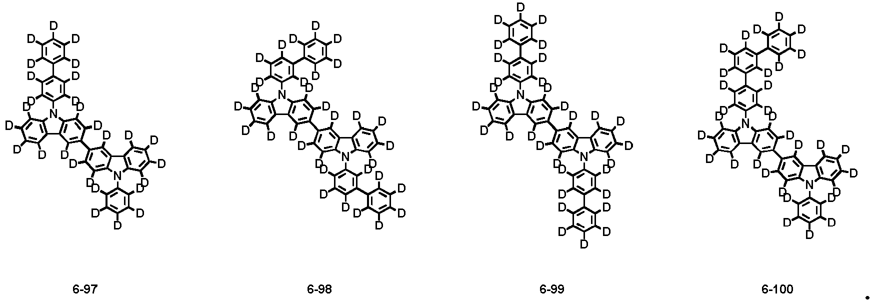

13. A composition for an organic material layer of an organic light emitting device, the composition comprising:
the heterocyclic compound represented by any one of Chemical Formula 1-2 to 1-4 of Claim 1; and
a heterocyclic compound represented by the following Chemical Formula 13 or 14: 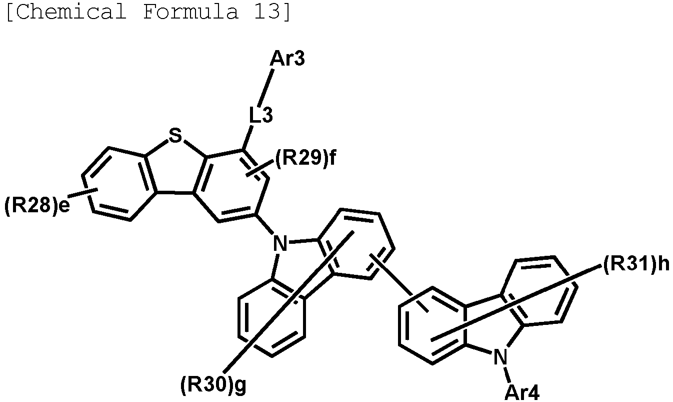 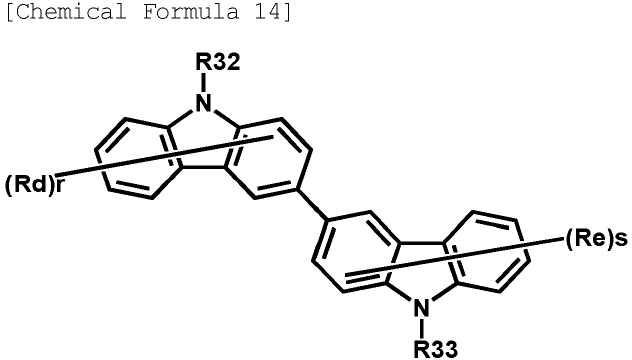 wherein, in Chemical Formulae 13 and 14,
L3 is a direct bond; a substituted or unsubstituted arylene group having 6 to 60 carbon atoms; or a substituted or unsubstituted heteroarylene group having 2 to 60 carbon atoms;
R28 to R31, Rd and Re are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, or two or more groups adjacent to each other bond to each other to form a substituted or unsubstituted aromatic hydrocarbon ring having 6 to 60 carbon atoms or a substituted or unsubstituted heteroring having 2 to 60 carbon atoms, r and s are an integer of 0 to 7, and when r is 2 or greater, Rds are the same as or different from each other, when s is 2 or greater, Res are the same as or different from each other, e is an integer of 0 to 4, g and h are each an integer of 0 to 7, f is an integer of 0 to 2, and when e is 2 or greater, R28s are the same as or different from each other, when f is an integer of 2, R29s are the same as or different from each other, when g is 2 or greater, R30s are the same as or different from each other, and when h is 2 or greater, R31s are the same as or different from each other;
Ar3 is a substituted or unsubstituted aryl group having 6 to 60 carbon atoms; or a substituted or unsubstituted heteroaryl group having 2 to 60 carbon atoms;
Ar4 is a substituted or unsubstituted aryl group having 6 to 60 carbon atoms; or a substituted or unsubstituted heteroaryl group having 2 to 60 carbon atoms; and
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms.

## Patentansprüche

1. Eine heterocyclische Verbindung, dargestellt durch eine der nachstehenden chemischen Formeln 1-2 bis 1-4: 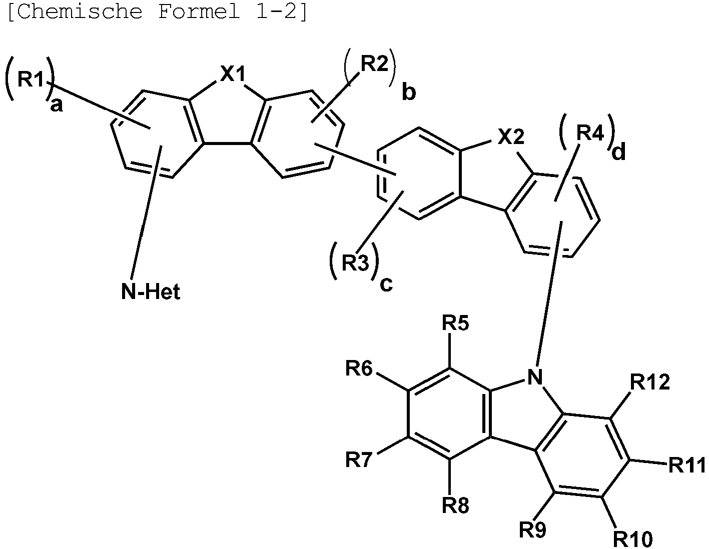 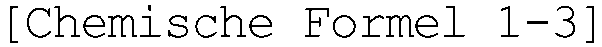 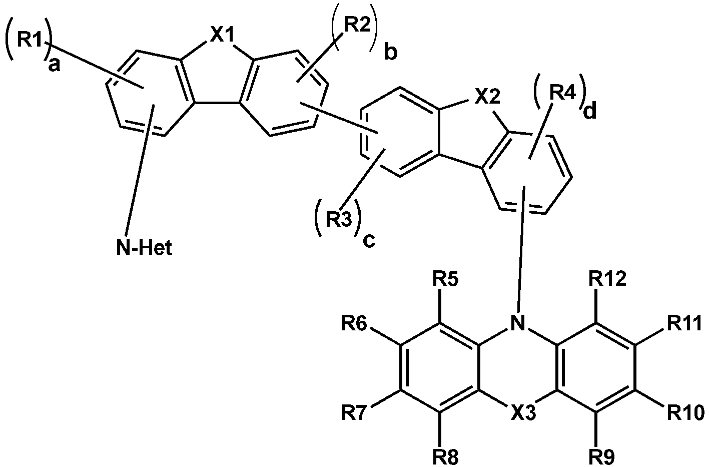 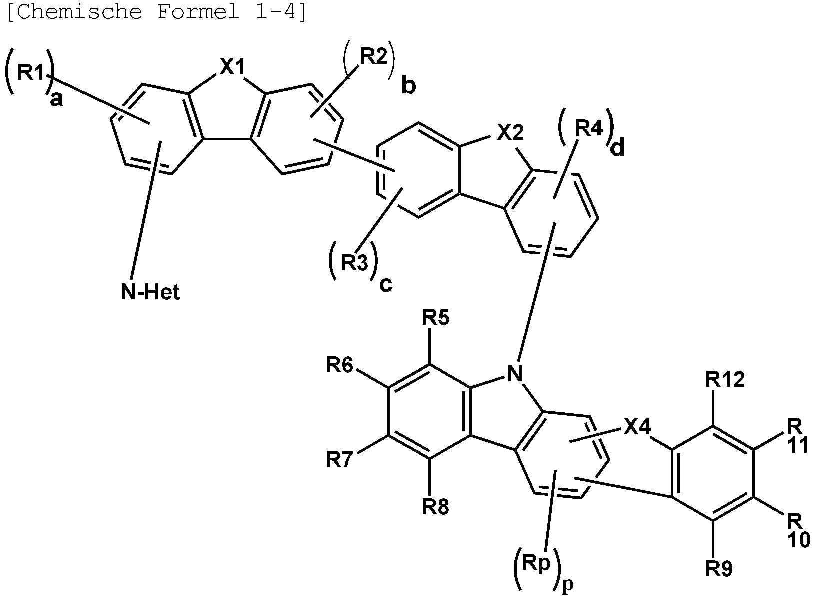 wobei in den chemischen Formeln 1-2 bis 1-4 gilt:
X1 und X2 sind gleich oder voneinander verschieden und jeweils unabhängig O; oder S;
R1 bis R4 sind gleich oder voneinander verschieden und jeweils unabhängig Wasserstoff, Deuterium, eine substituierte oder unsubstituierte Arylgruppe mit 6 bis 60 Kohlenstoffatomen oder eine substituierte oder unsubstituierte Heteroarylgruppe mit 6 bis 60 Kohlenstoffatomen; a bis d sind jeweils eine ganze Zahl von 0 bis 3, und wenn a bis d jeweils 2 oder größer sind, sind die in Klammern angegebenen Substituenten untereinander gleich;
N-Het ist eine Gruppe, die durch eine der folgenden Chemischen Formeln 2 bis 4 dargestellt wird.
X3 und X4 sind jeweils unabhängig O; S; CRaRb oder NRc; Ra und Rb sind gleich oder voneinander verschieden und jeweils unabhängig eine substituierte oder unsubstituierte Alkylgruppe mit 1 bis 10 Kohlenstoffatomen; oder eine substituierte oder unsubstituierte Arylgruppe mit 6 bis 20 Kohlenstoffatomen, und Rc ist eine substituierte oder unsubstituierte Arylgruppe mit 6 bis 20 Kohlenstoffatomen;
R5 bis R12 und Rp sind gleich oder voneinander verschieden und jeweils unabhängig Wasserstoff; Deuterium; eine Halogengruppe; eine Cyanogruppe; eine substituierte oder unsubstituierte Arylgruppe mit 6 bis 20 Kohlenstoffatomen oder eine substituierte oder unsubstituierte Heteroarylgruppe mit 2 bis 20 Kohlenstoffatomen;
p ist eine ganze Zahl von 0 bis 2, und wenn p 2 ist, sind die in Klammern angegebenen Substituenten untereinander gleich. 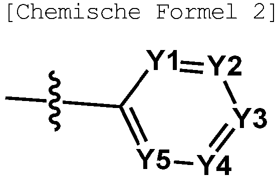 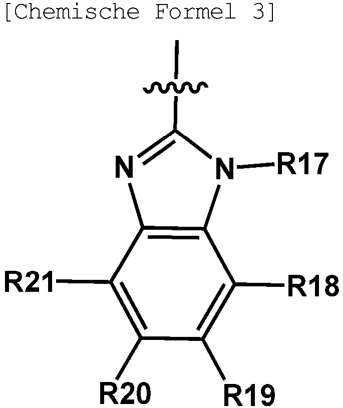 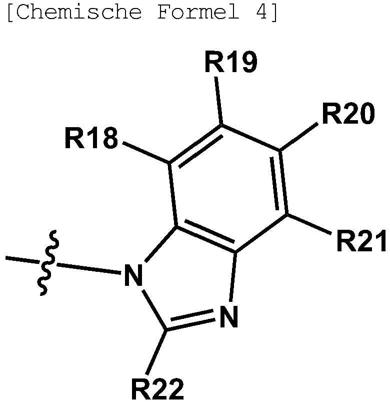 in den Chemischen Formeln 2 bis 4,
Y1 ist CR12' oder N, Y2 ist CR13' oder N, Y3 ist CR14' oder N, Y4 ist CR15' oder N, Y5 ist CR16' oder N, und mindestens eines von Y1 bis Y5 ist N; und
R12' bis R16' und R17 bis R22 sind gleich oder voneinander verschieden und jeweils unabhängig ausgewählt aus der Gruppe bestehend aus Wasserstoff; Deuterium; Halogen; einer Cyanogruppe; einer substituierten oder unsubstituierten Alkylgruppe mit 1 bis 60 Kohlenstoffatomen; einer substituierten oder unsubstituierten Alkenylgruppe mit 2 bis 60 Kohlenstoffatomen, einer substituierten oder unsubstituierten Alkynylgruppe mit 2 bis 60 Kohlenstoffatomen; einer substituierten oder unsubstituierten Alkoxygruppe mit 1 bis 20 Kohlenstoffatomen; einer substituierten oder unsubstituierten Cycloalkylgruppe mit 3 bis 60 Kohlenstoffatomen; einer substituierten oder unsubstituierten Heterocycloalkylgruppe mit 2 bis 60 Kohlenstoffatomen; einer substituierten oder unsubstituierten Arylgruppe mit 6 bis 60 Kohlenstoffatomen; einer substituierten oder unsubstituierten Heteroarylgruppe mit 2 bis 60 Kohlenstoffatomen; einer substituierten oder unsubstituierten Phosphinoxidgruppe; einer substituierten oder unsubstituierten Aminogruppe, oder zwei oder mehr benachbarte Gruppen verbinden sich miteinander und bilden einen substituierten oder unsubstituierten aliphatischen oder aromatischen Kohlenwasserstoffring oder Heteroring; 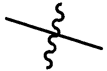
ist eine Position, die an eine der folgenden Chemischen Formeln 1-2 bis 1-4 gebunden ist; und
Position Nr. 3 von 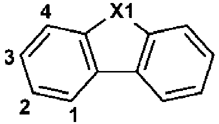 ist durch N-Het substituiert und Position Nr. 3 von 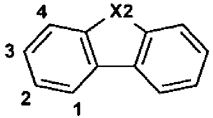 ist durch eines von 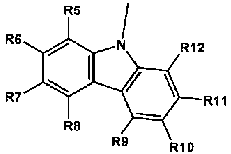 aus der Chemischen Formel 1-2, 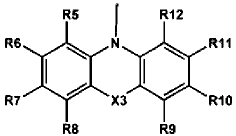 aus der Chemischen Formel 1-3 und 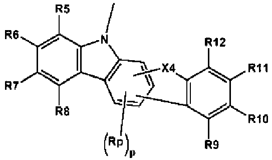 aus der Chemischen Formel 1-4 substituiert.

2. Die heterocyclische Verbindung nach Anspruch 1, wobei die heterocyclische Verbindung, die durch eine der folgenden chemischen Formeln 1-2 bis 1-4 dargestellt wird, einen Deuteriumgehalt von 0 %, oder größer als 10 % und kleiner oder gleich 100 % aufweist.

3. Eine heterocyclische Verbindung, die durch eine der folgenden Verbindungen dargestellt wird:

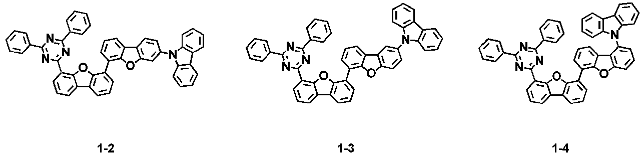

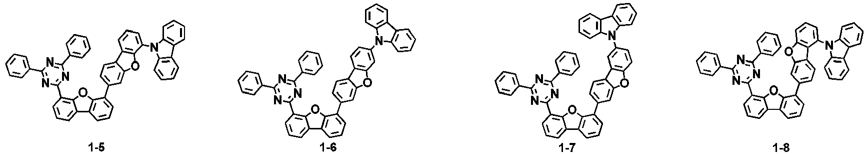

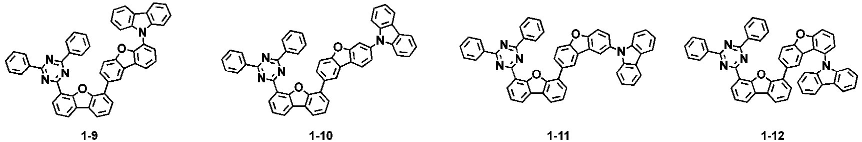

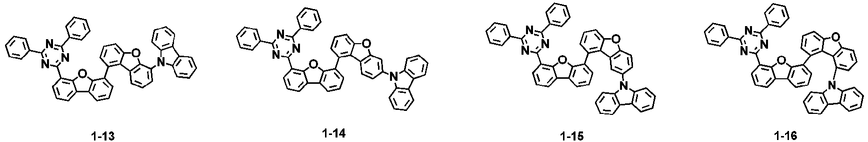

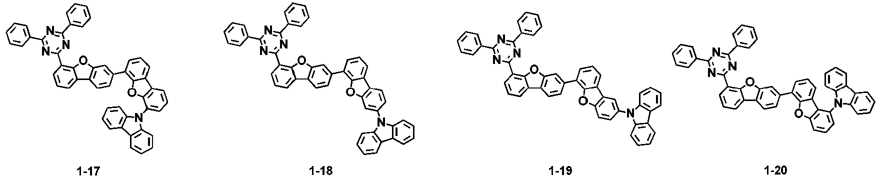

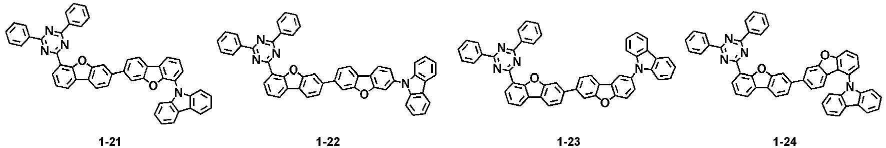

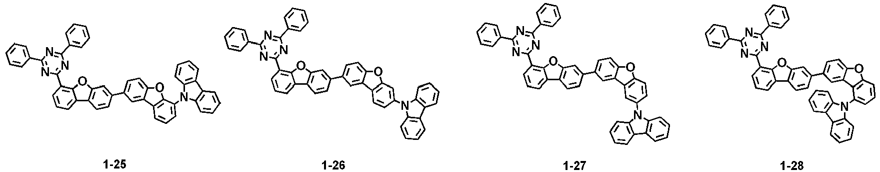

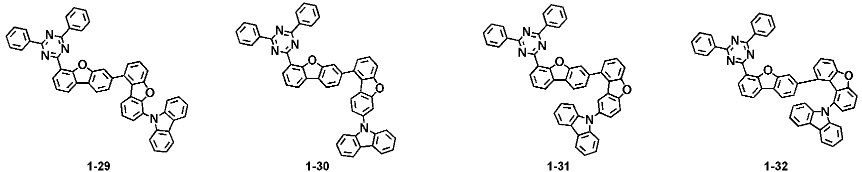

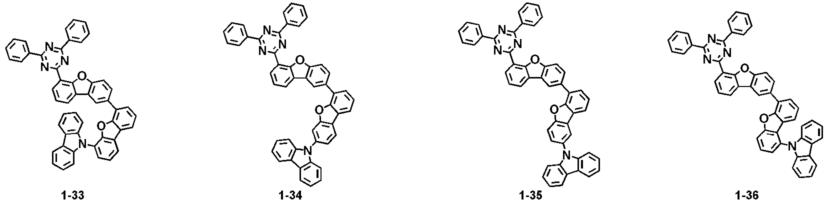

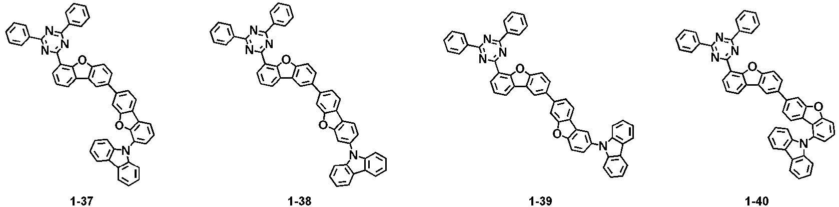

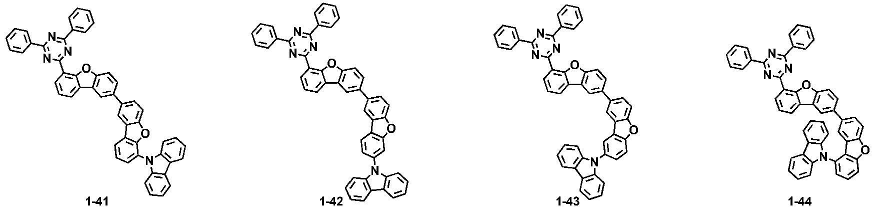

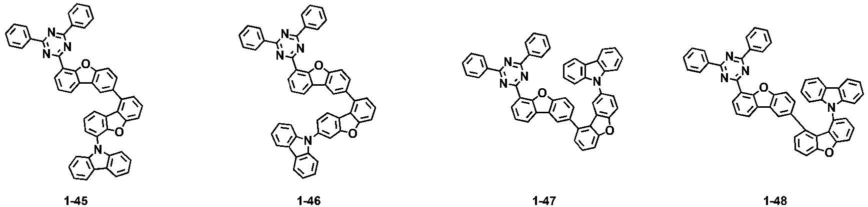

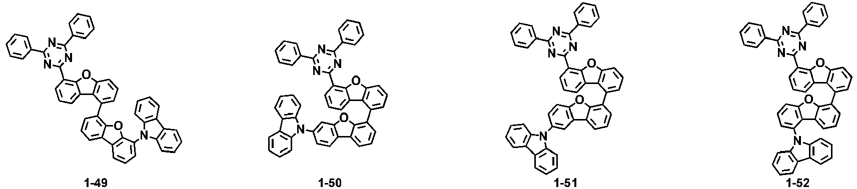

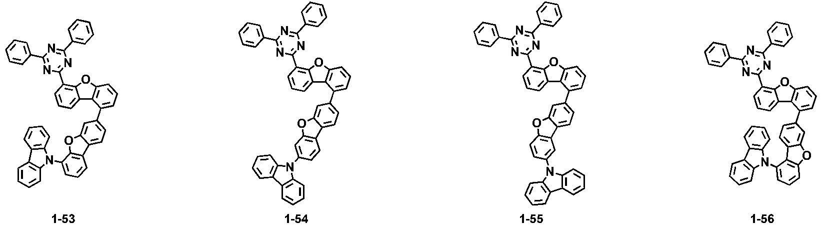

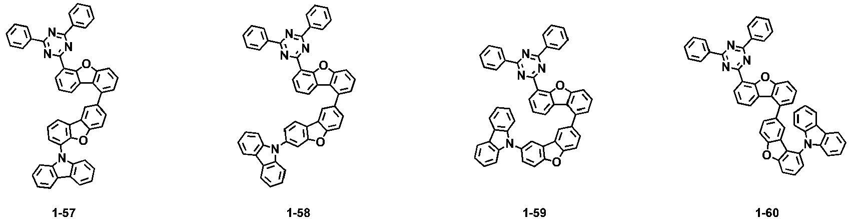

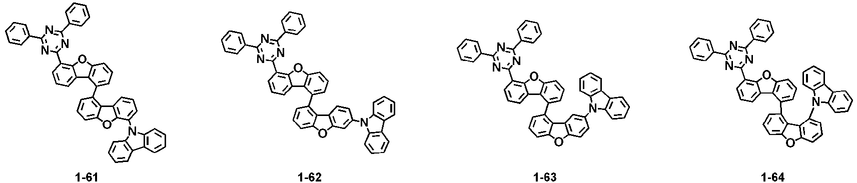

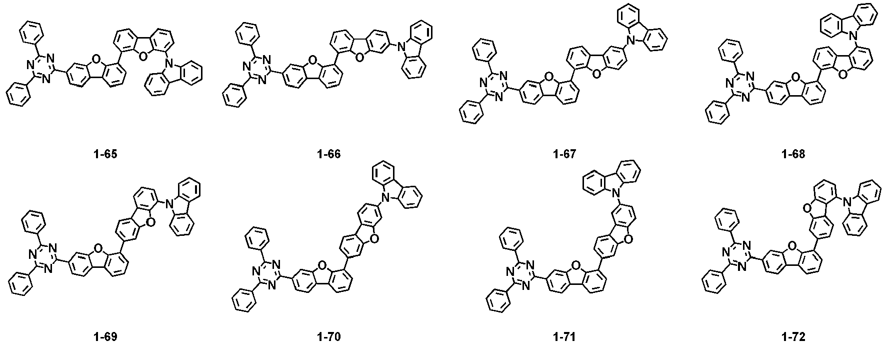

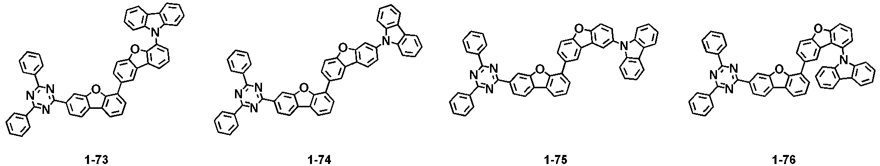

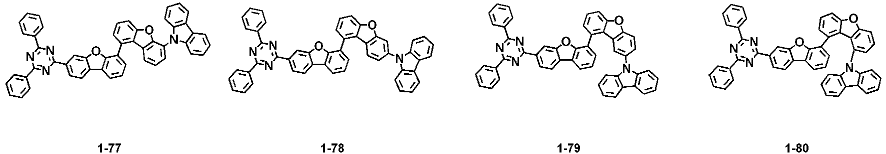

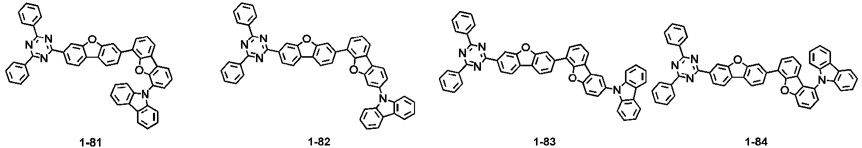

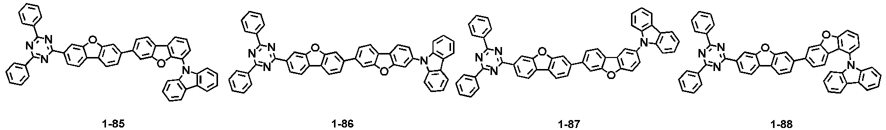

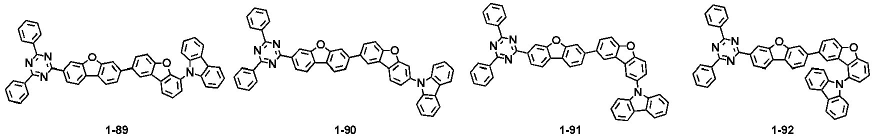

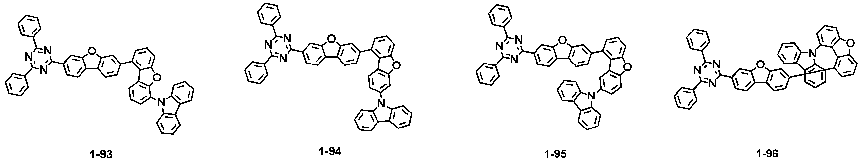

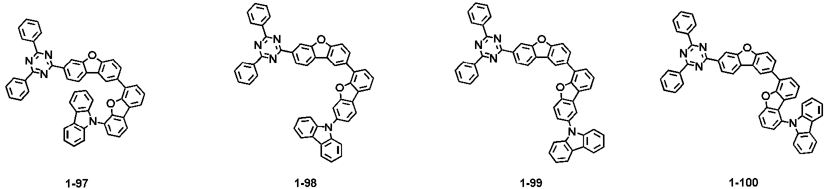

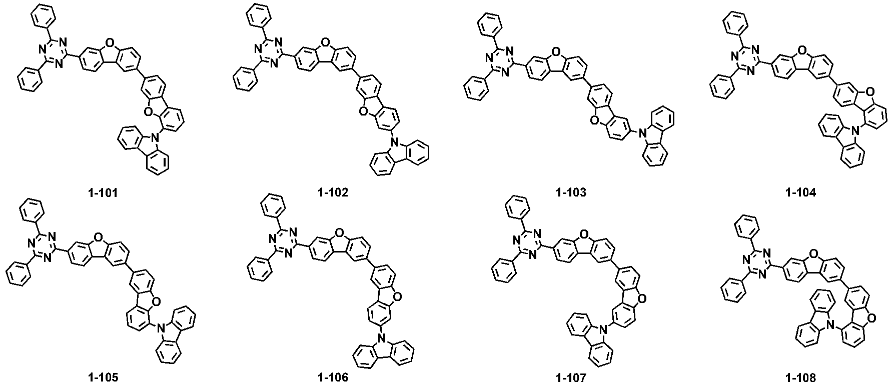

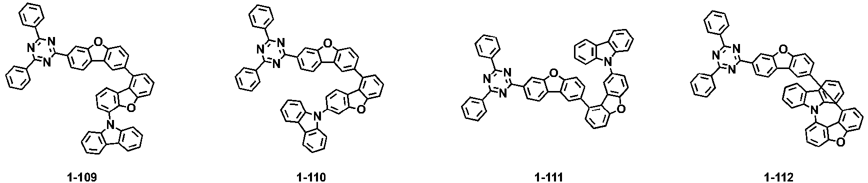

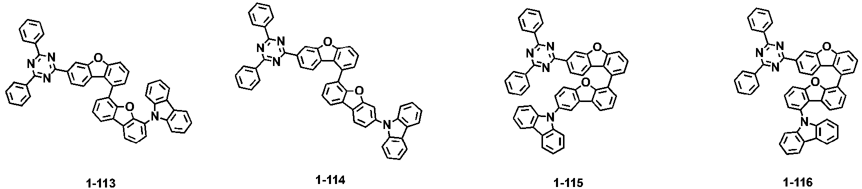

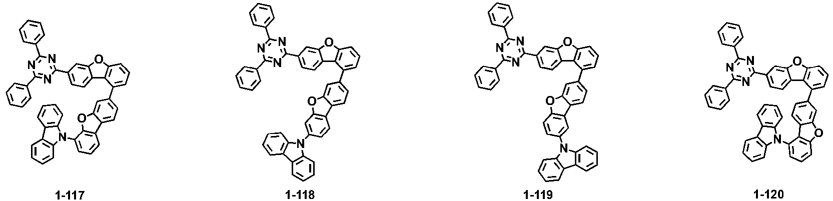

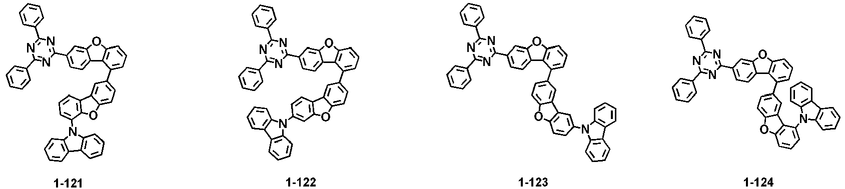

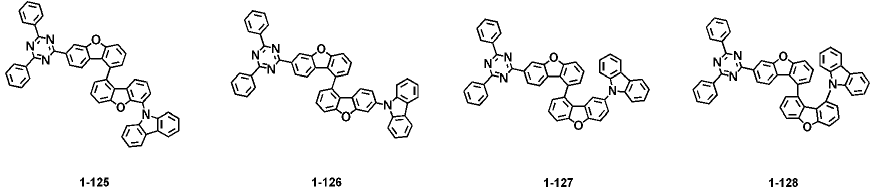

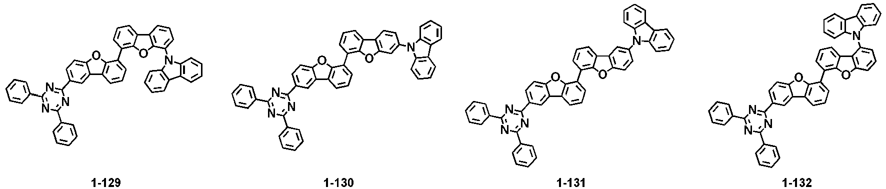

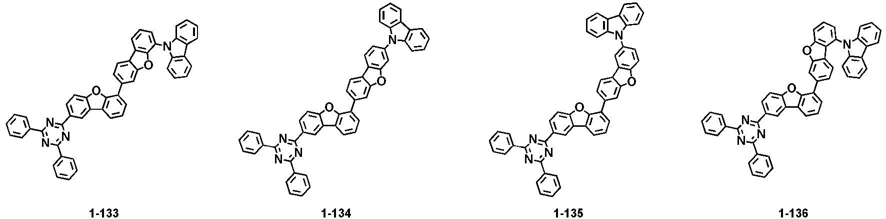

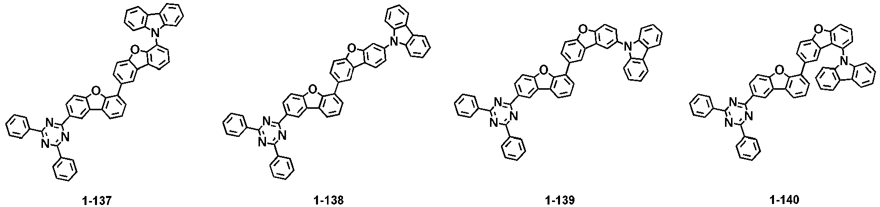

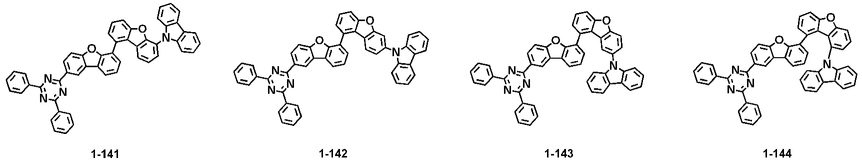

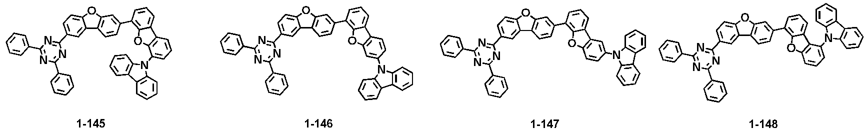

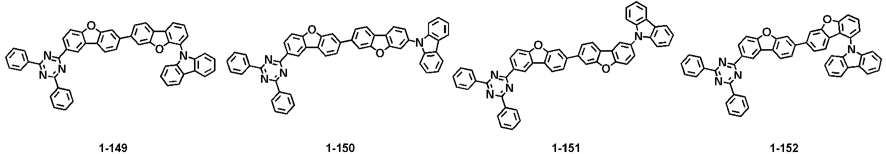

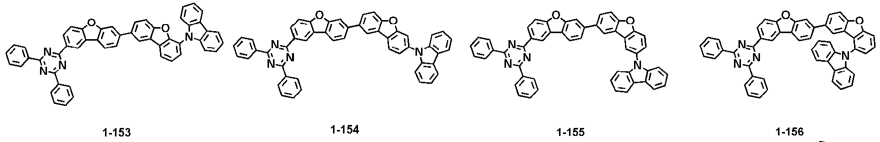

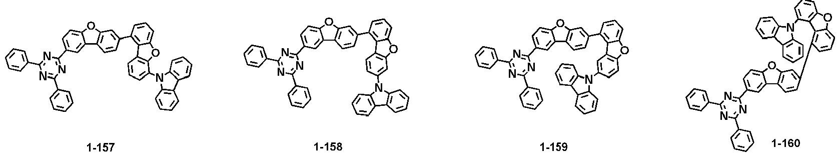

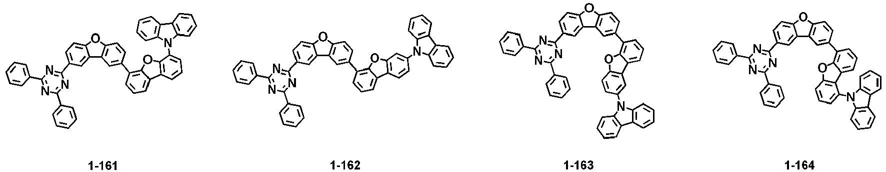

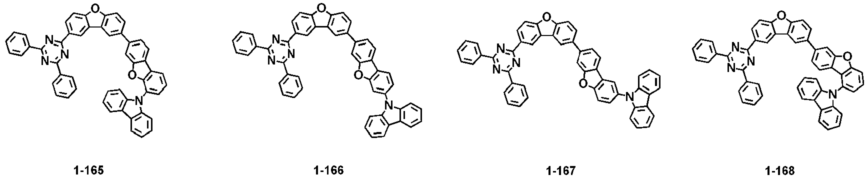

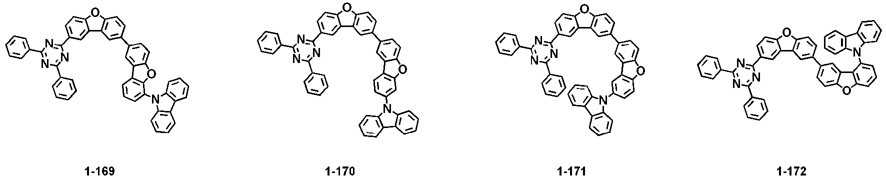

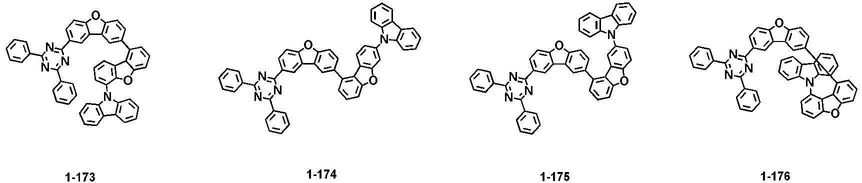

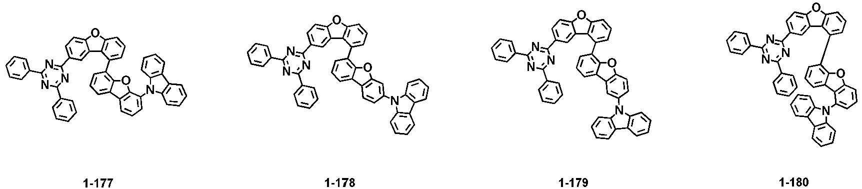

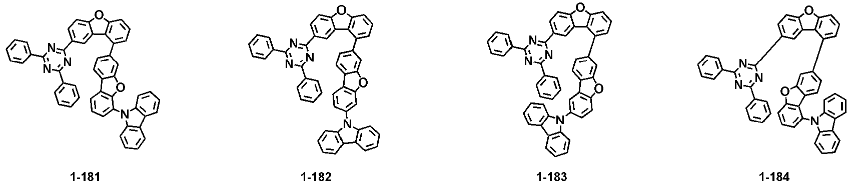

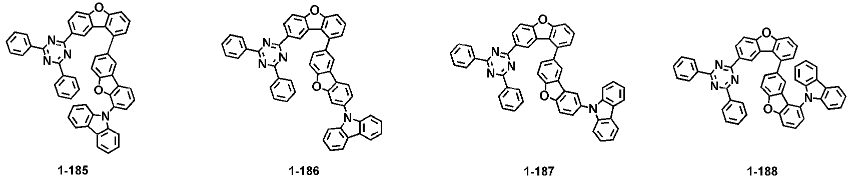

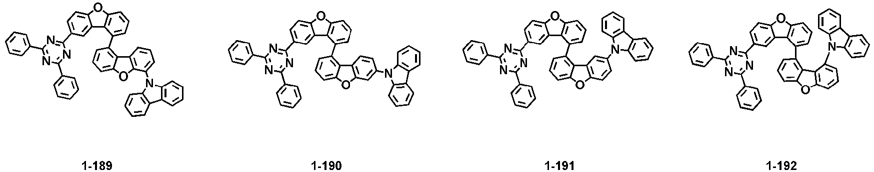

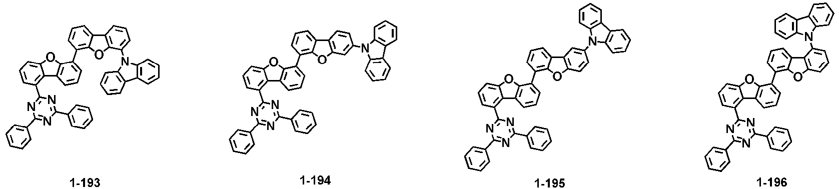

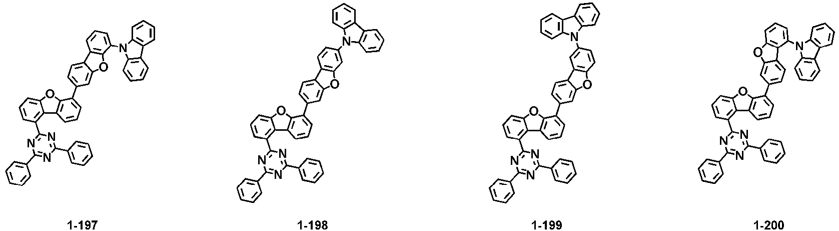

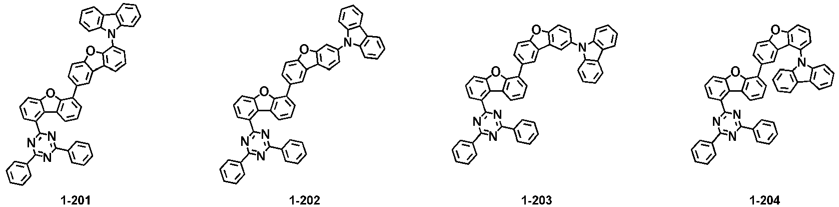

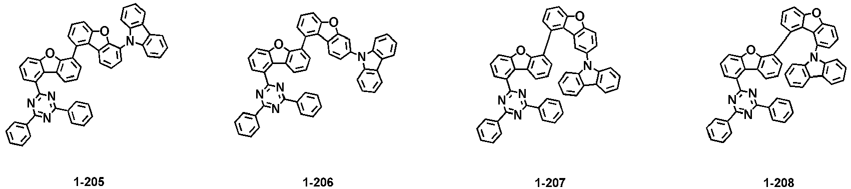

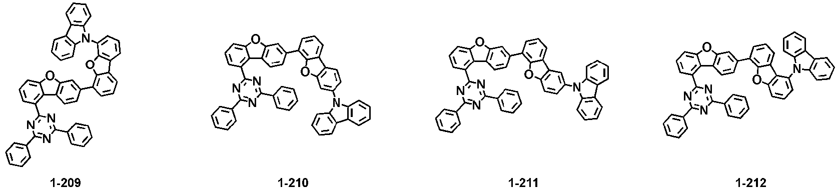

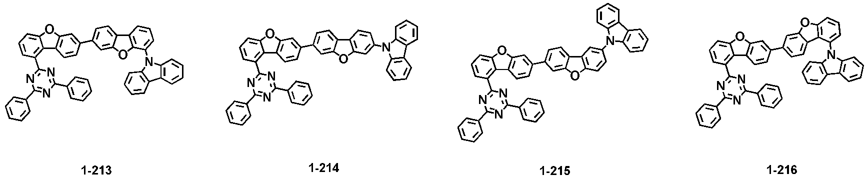

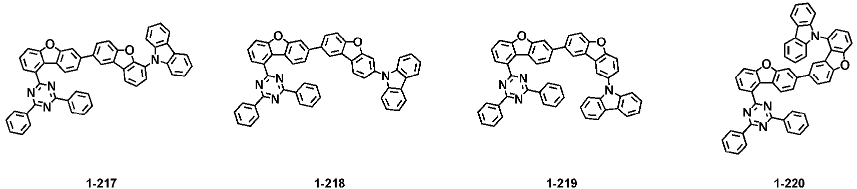

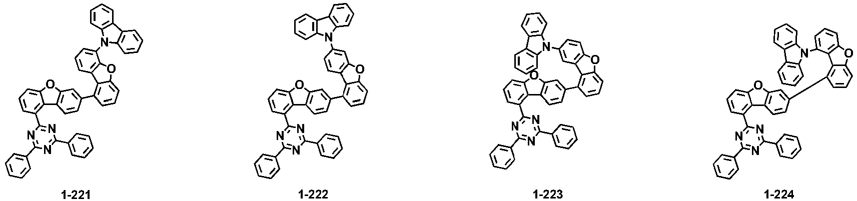

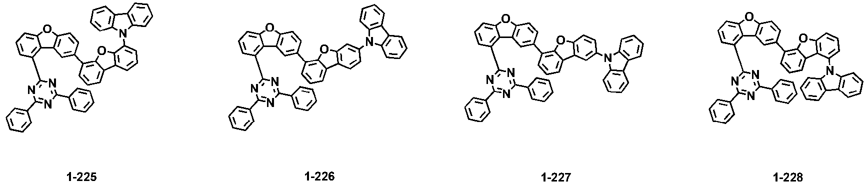

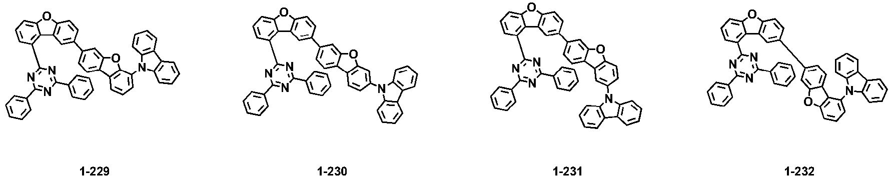

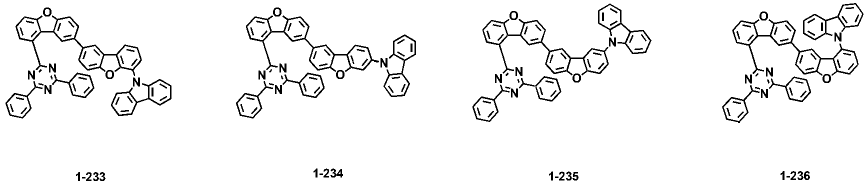

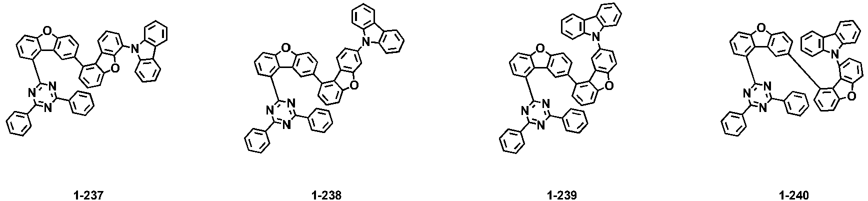

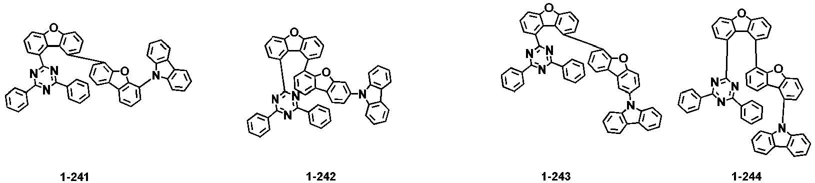

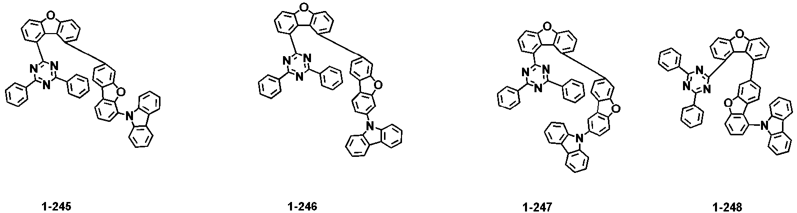

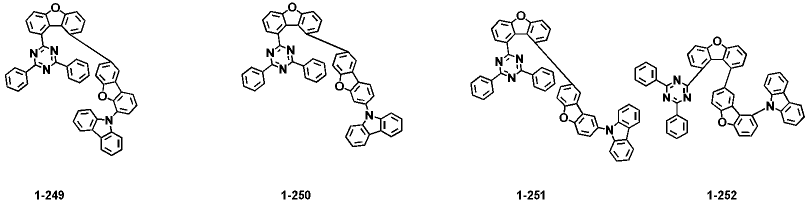

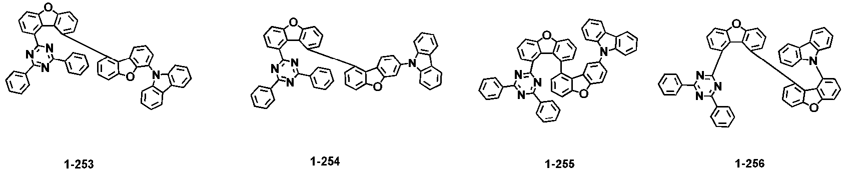

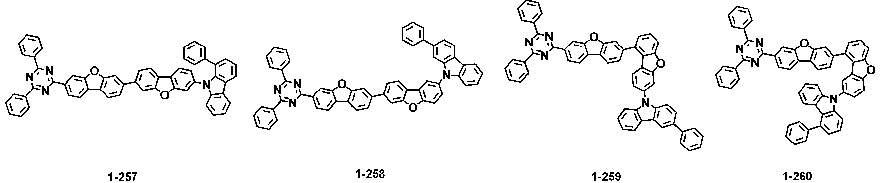

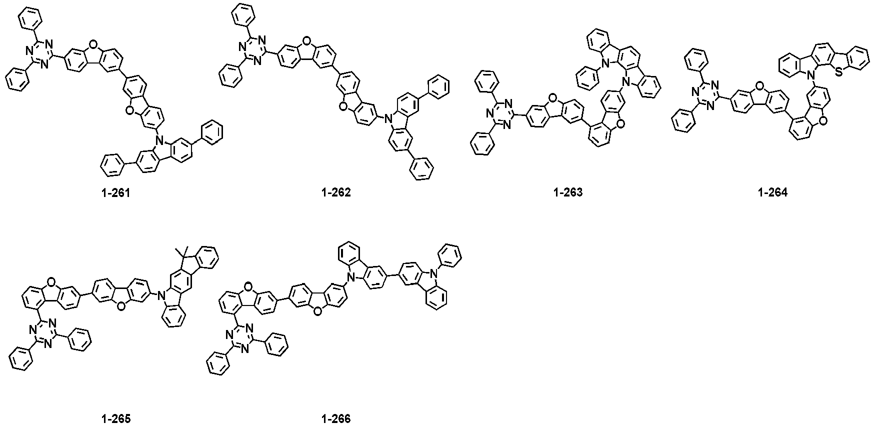

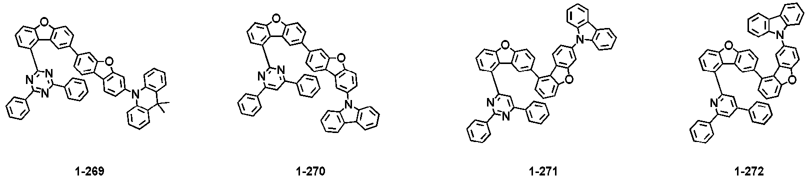

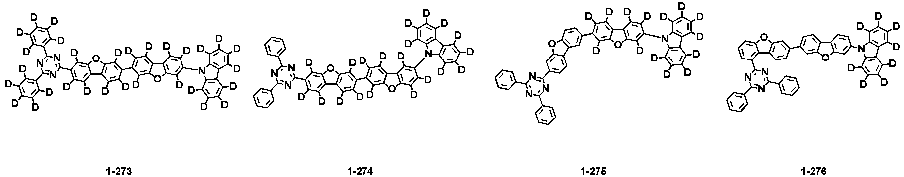

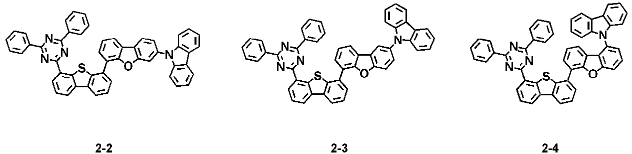

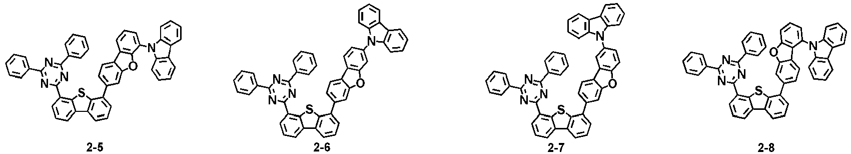

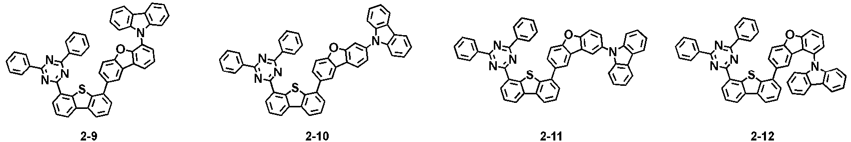

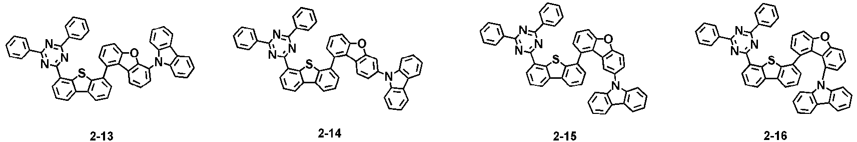

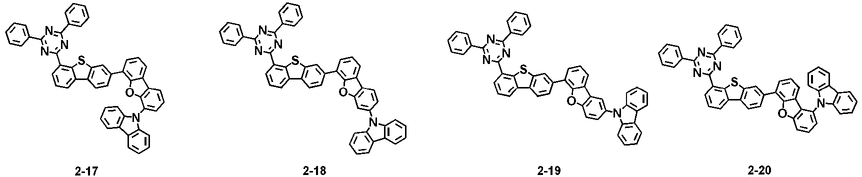

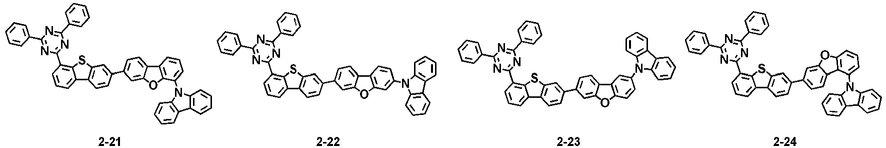

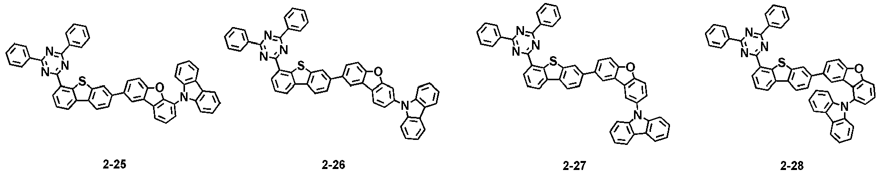

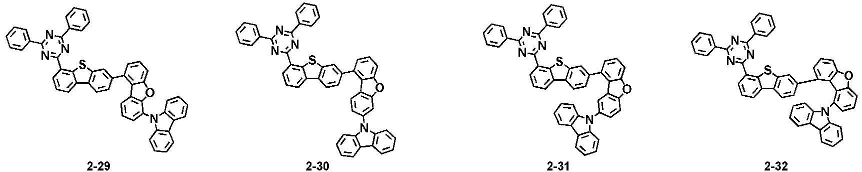

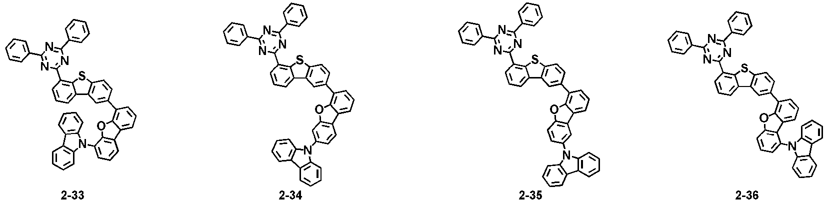

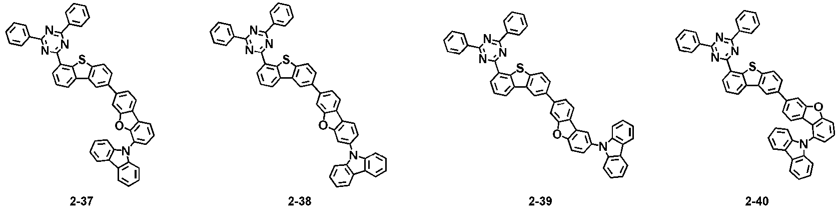

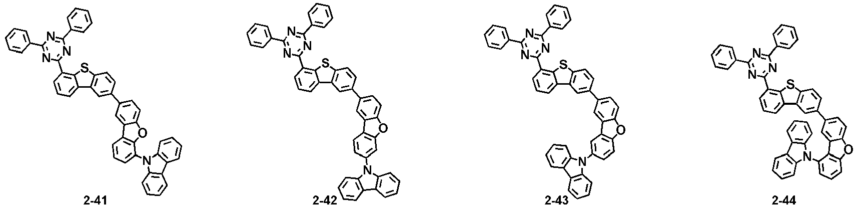

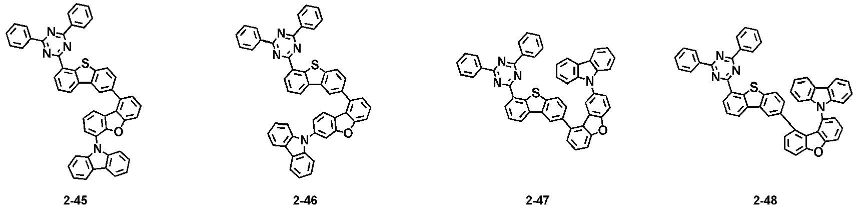

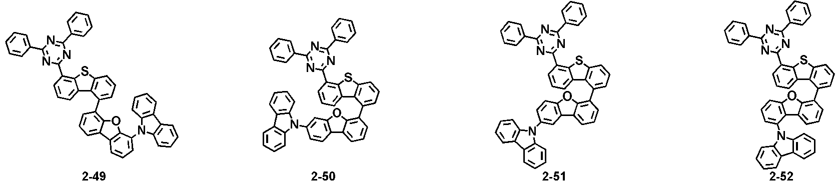

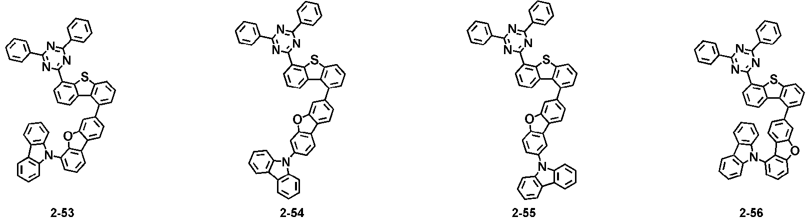

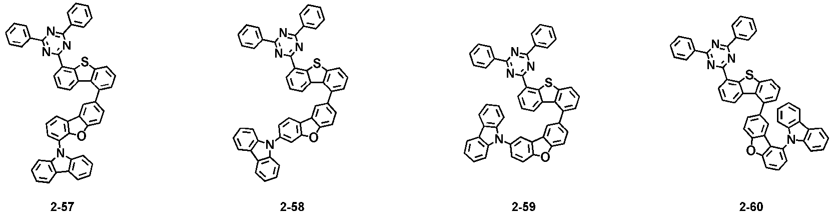

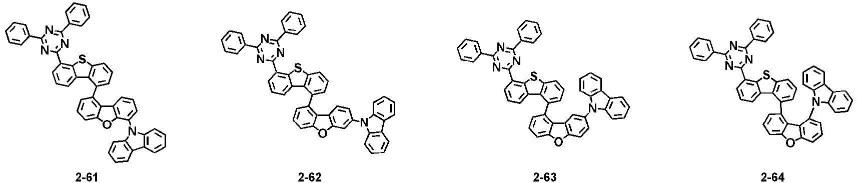

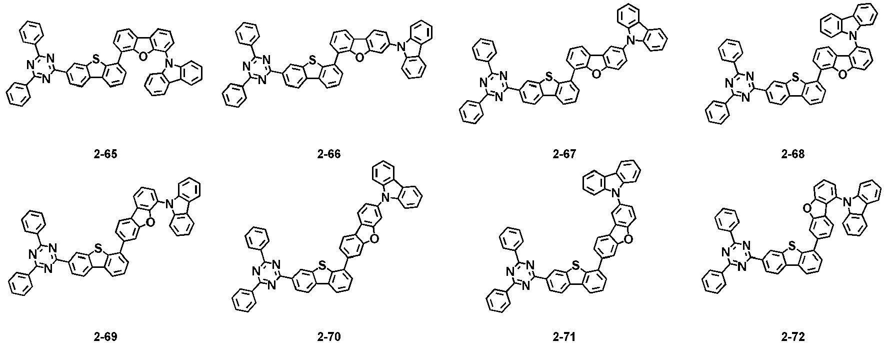

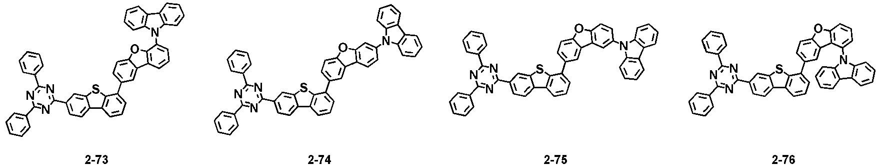

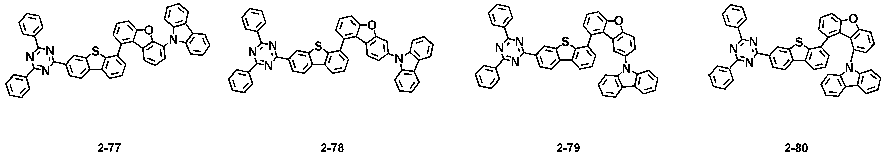

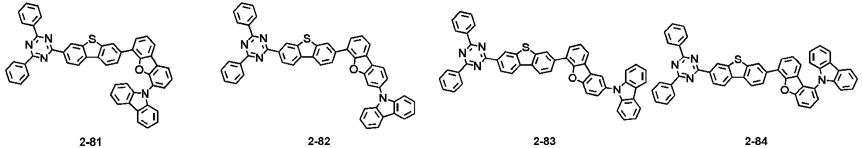

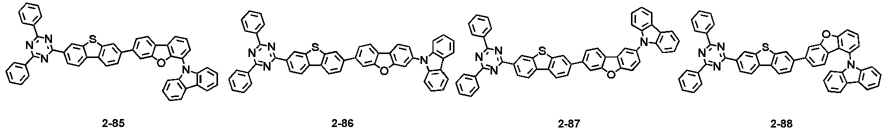

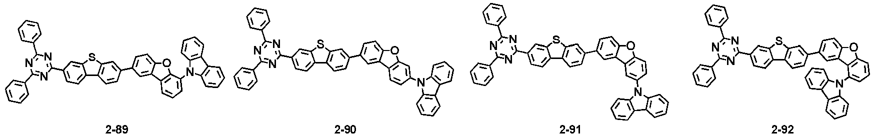

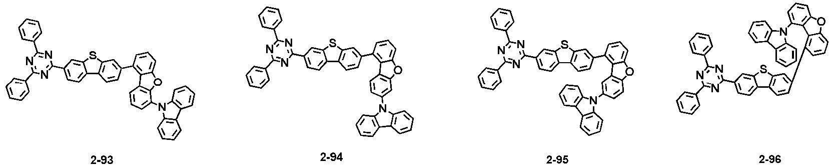

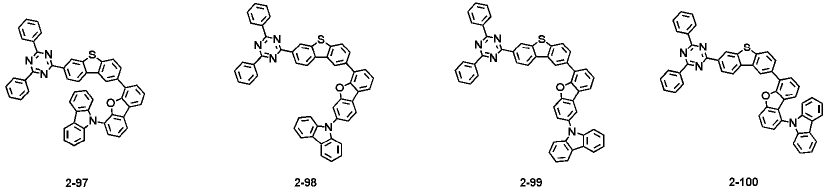

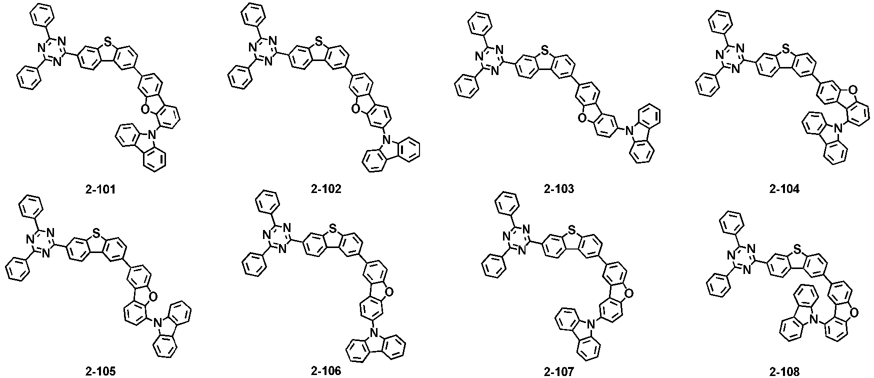

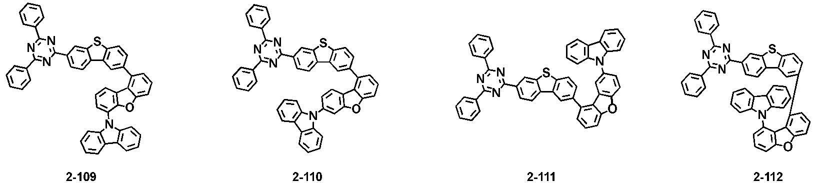

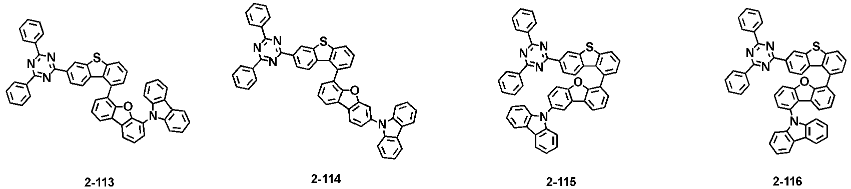

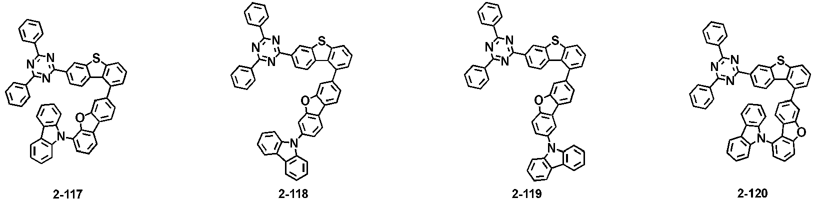

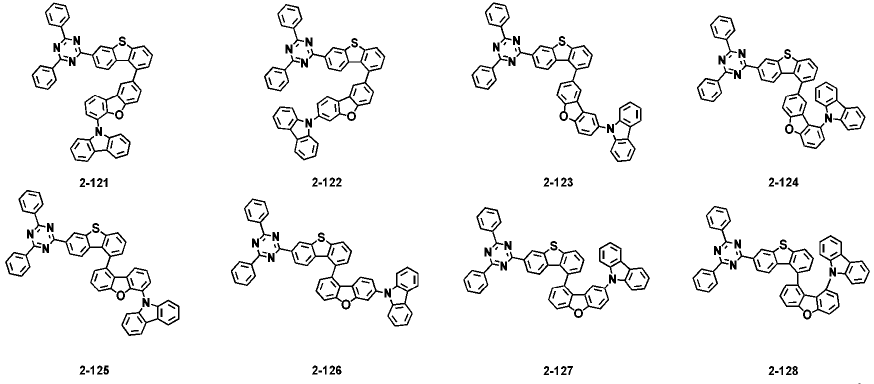

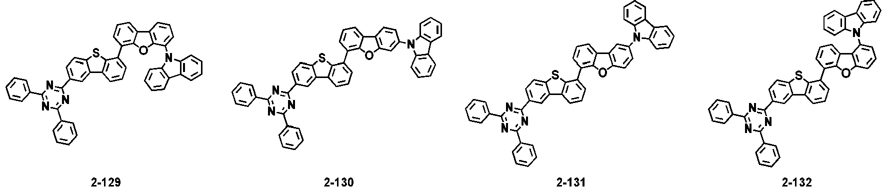

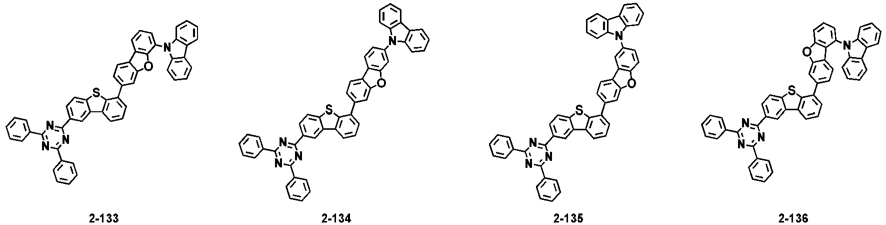

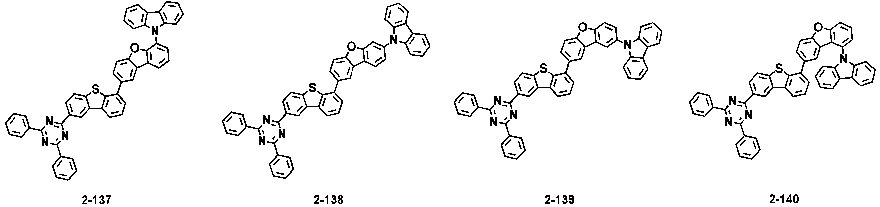

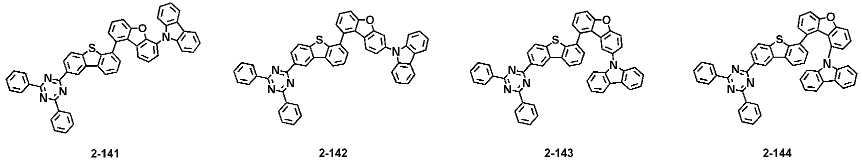

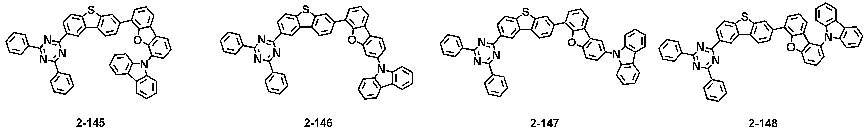

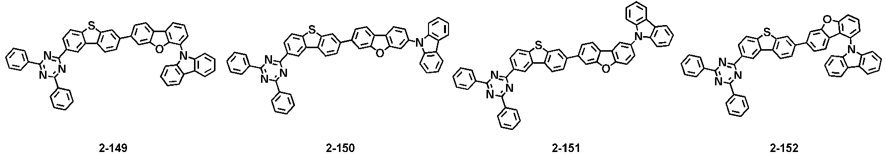

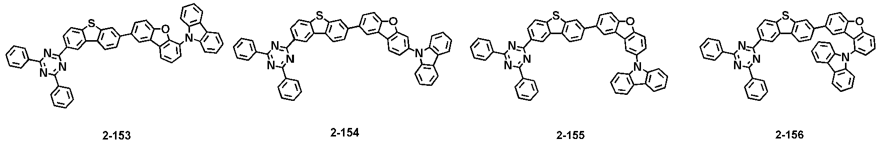

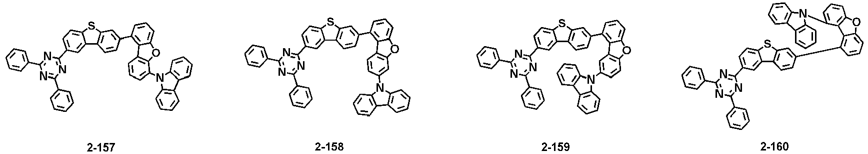

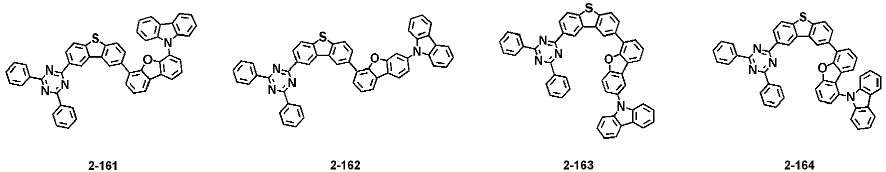

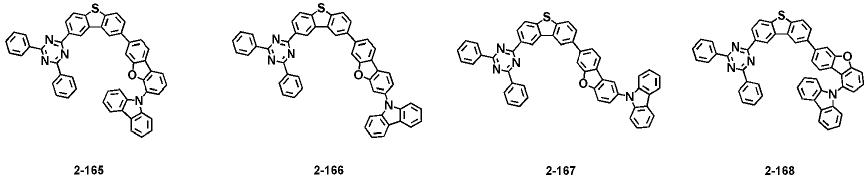

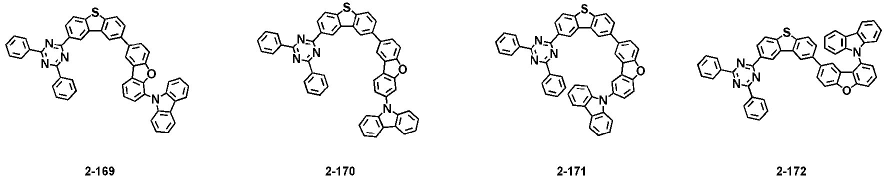

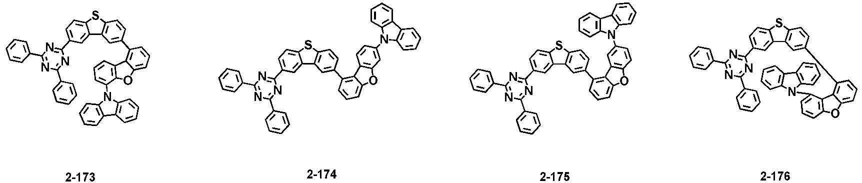

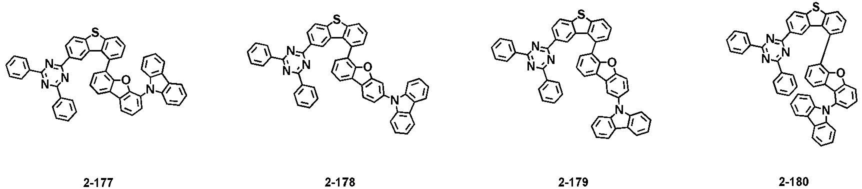

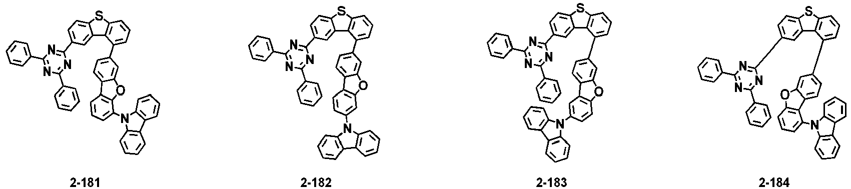

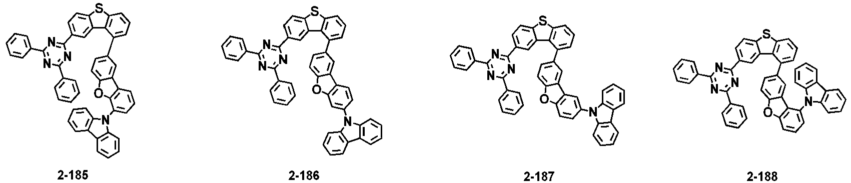

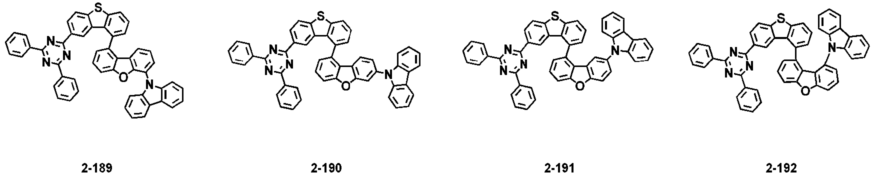

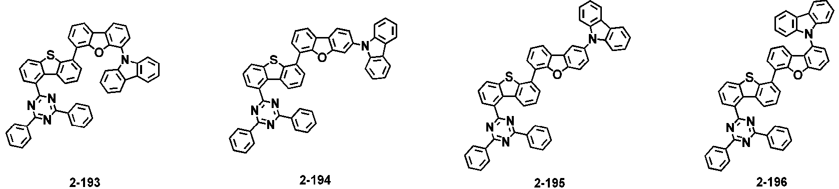

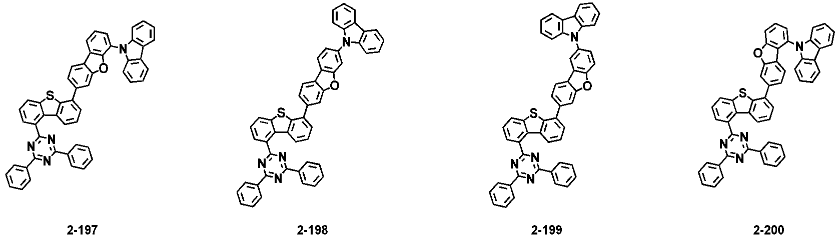

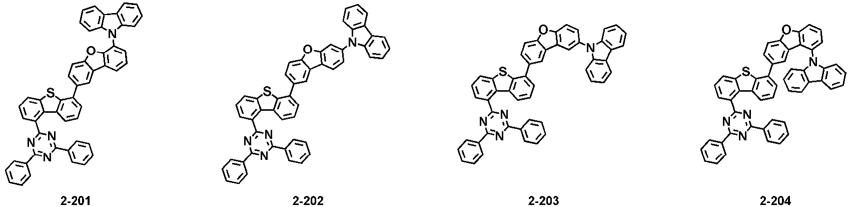

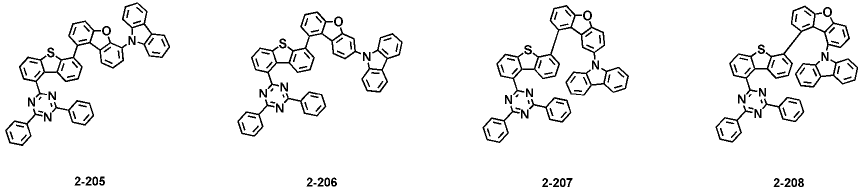

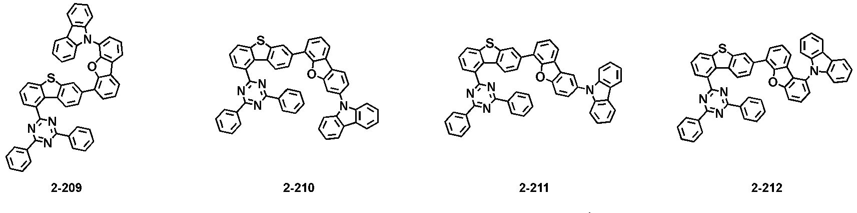

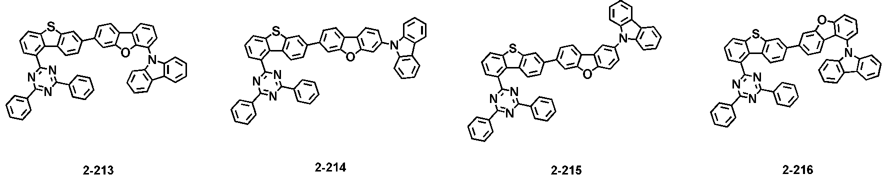

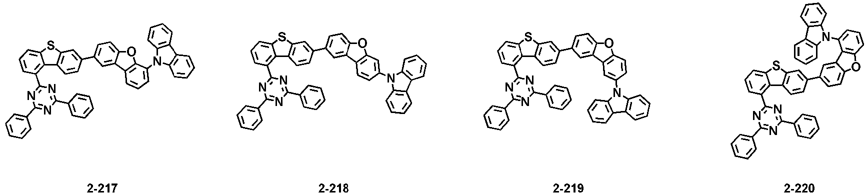

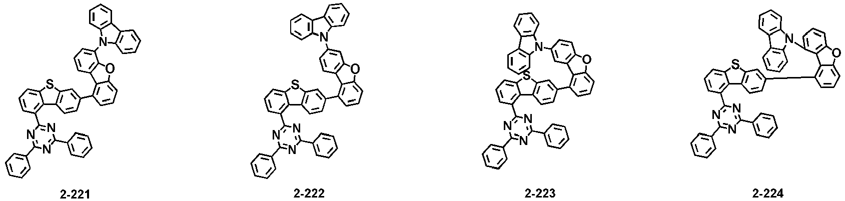

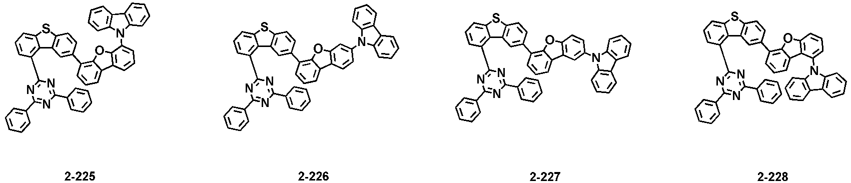

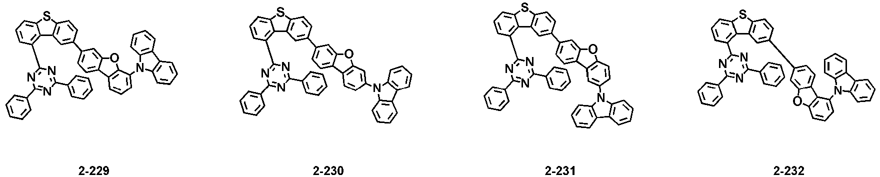

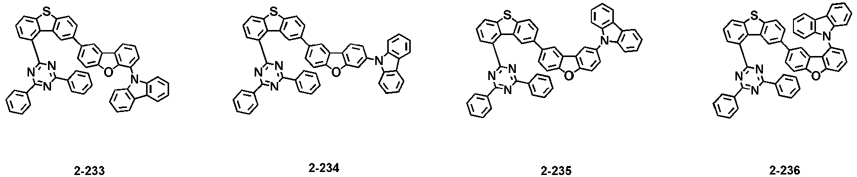

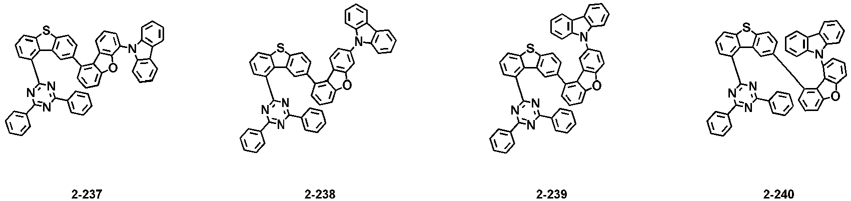

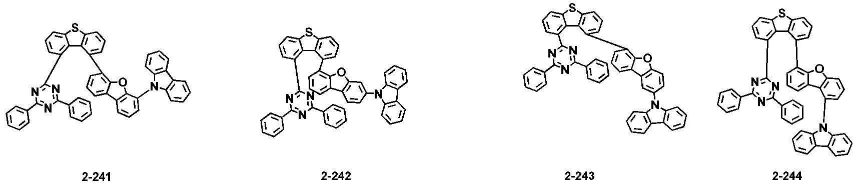

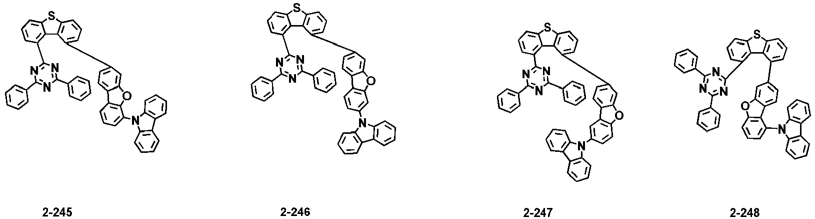

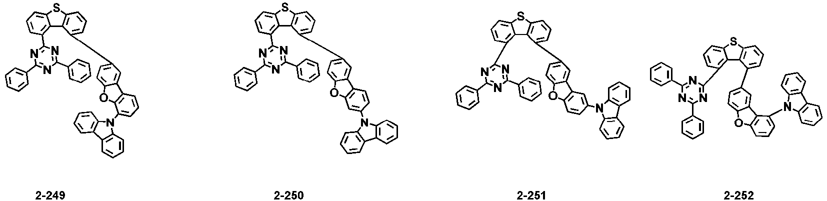

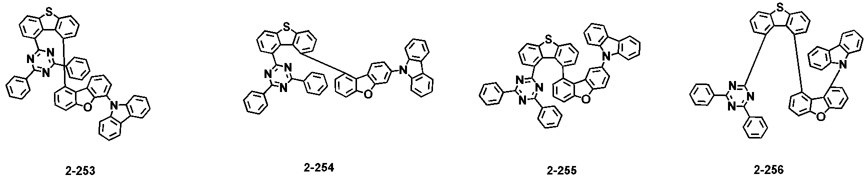

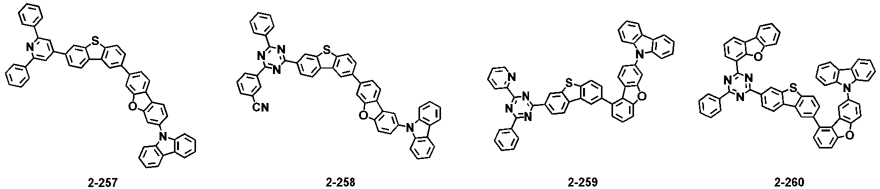

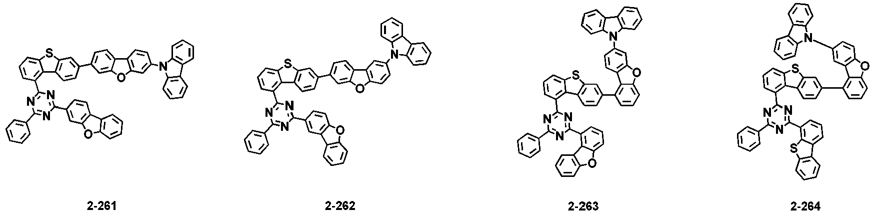

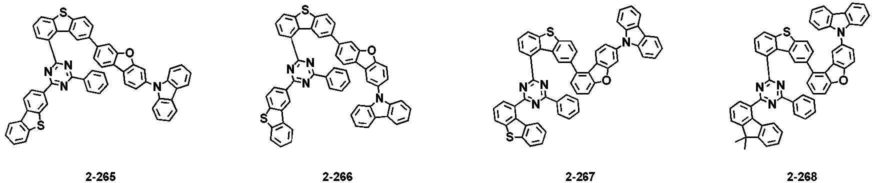

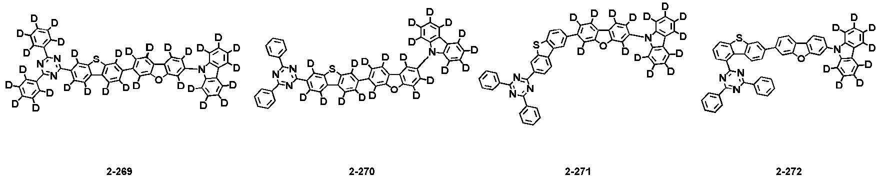

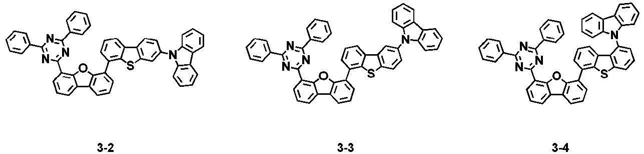

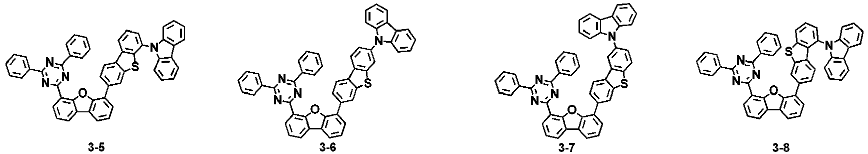

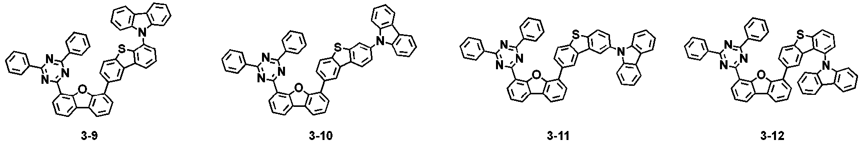

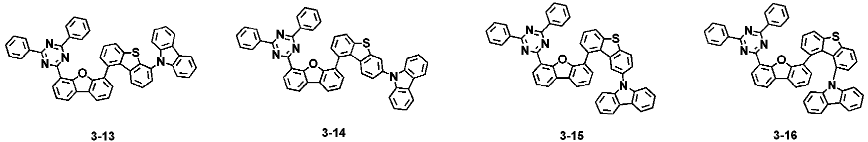

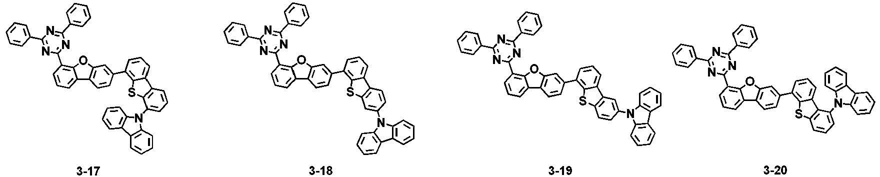

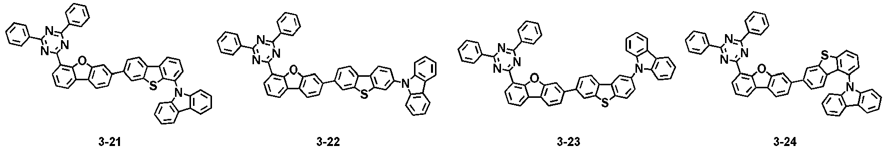

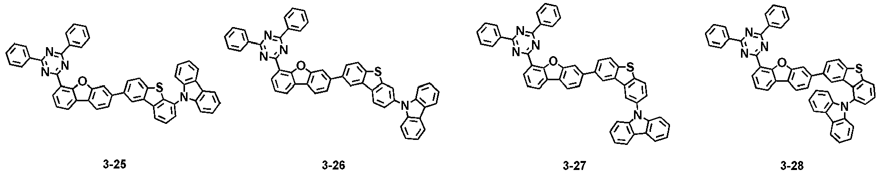

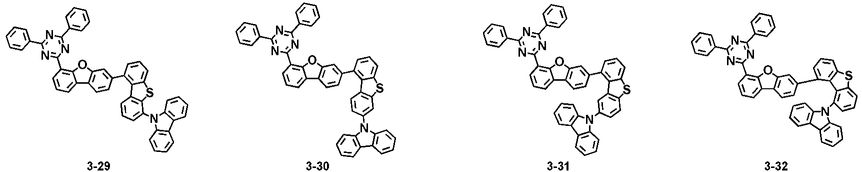

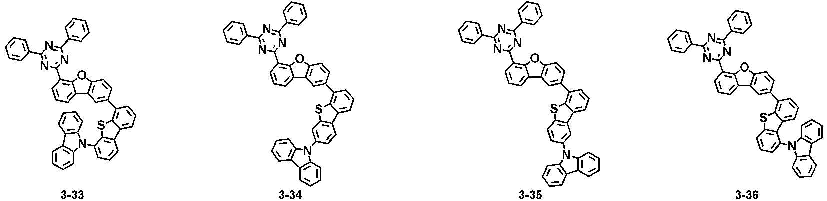

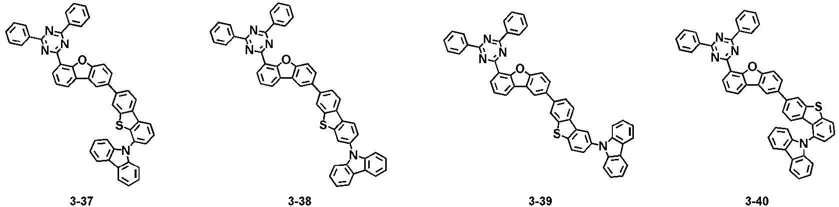

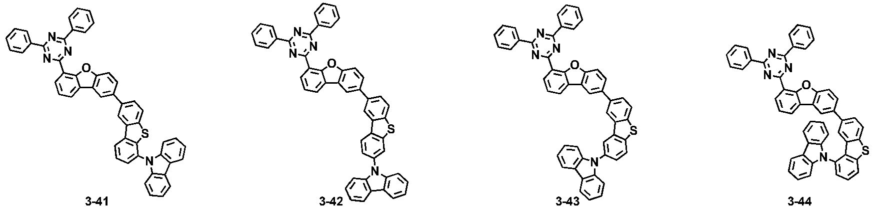

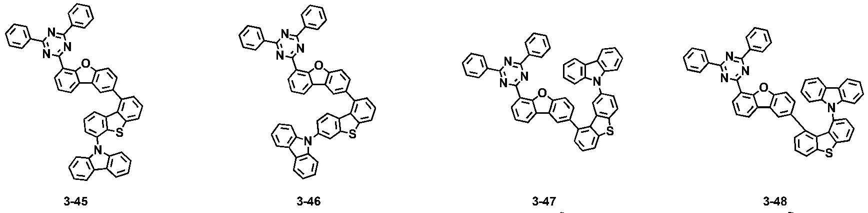

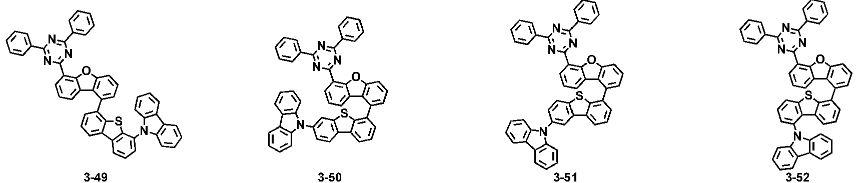

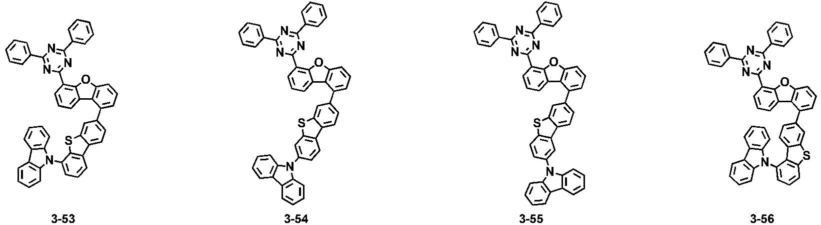

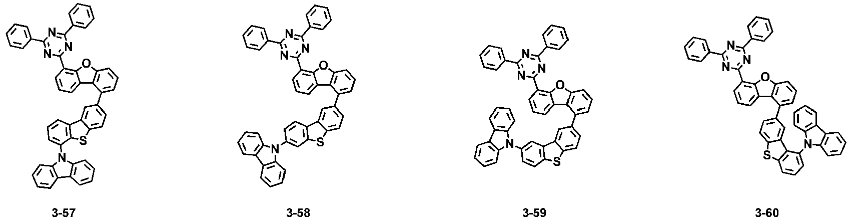

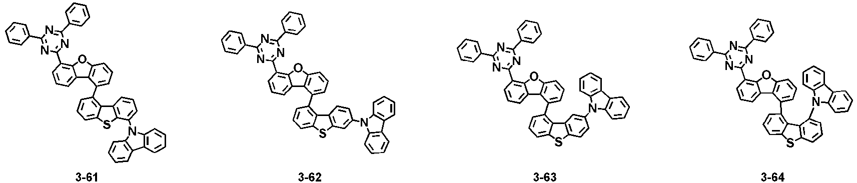

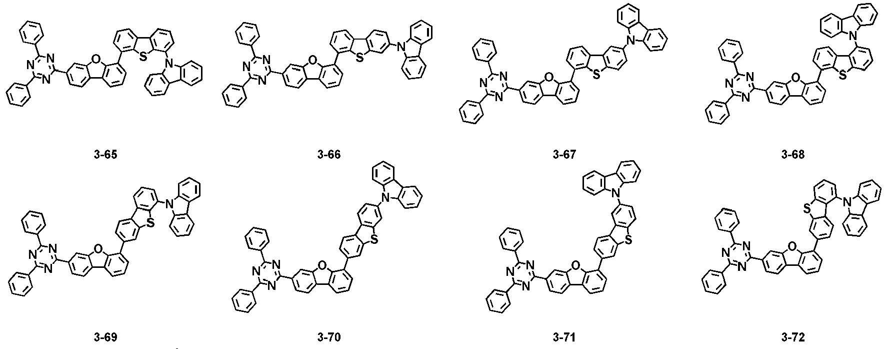

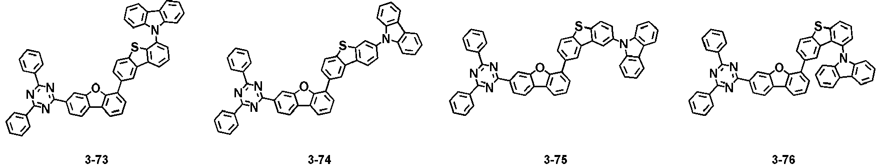

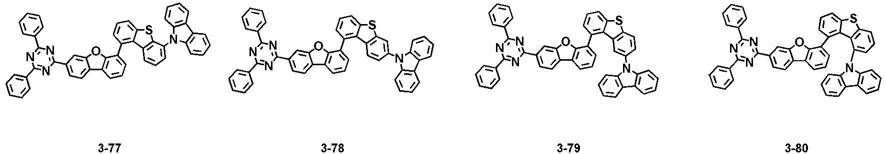

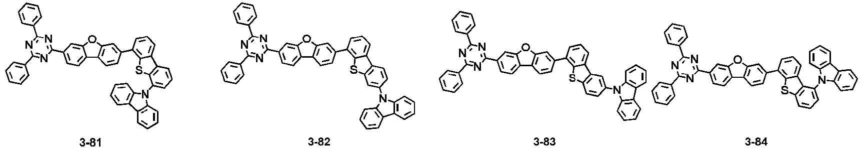

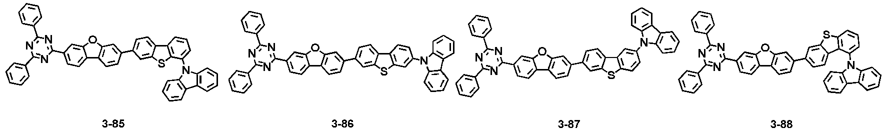

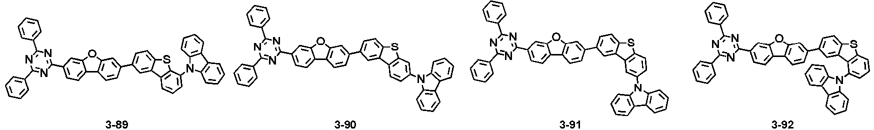

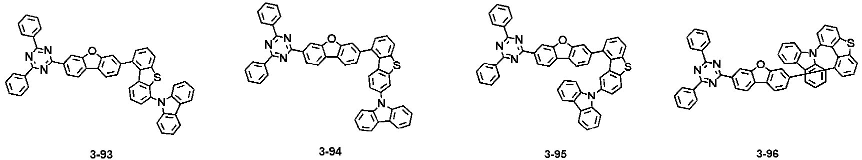

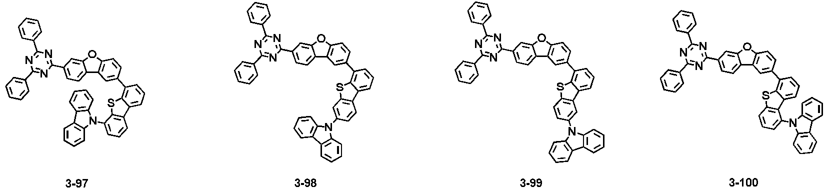

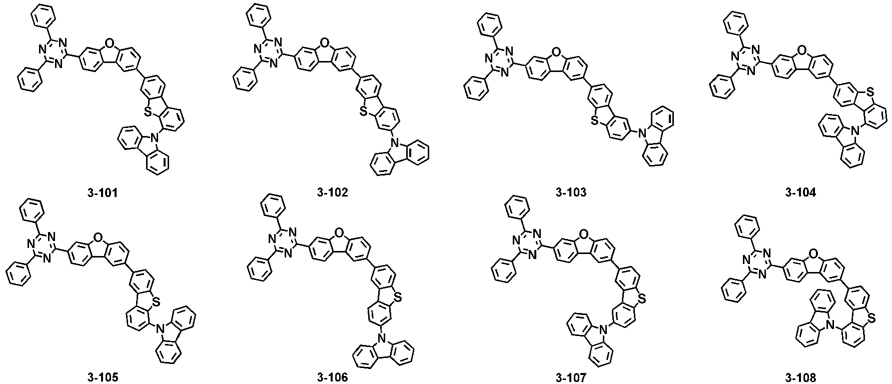

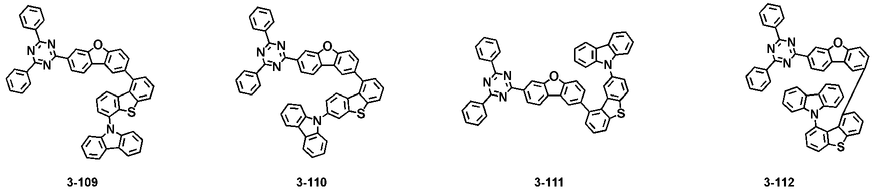

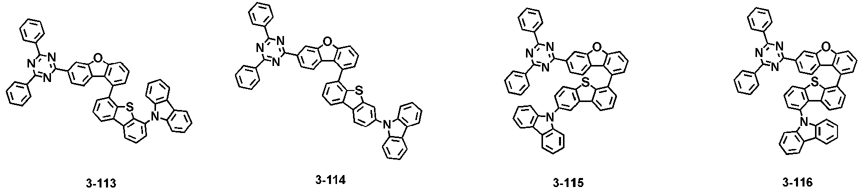

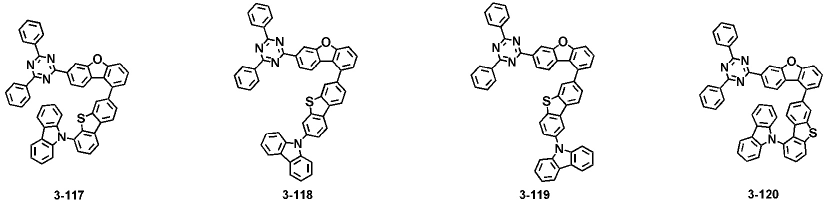

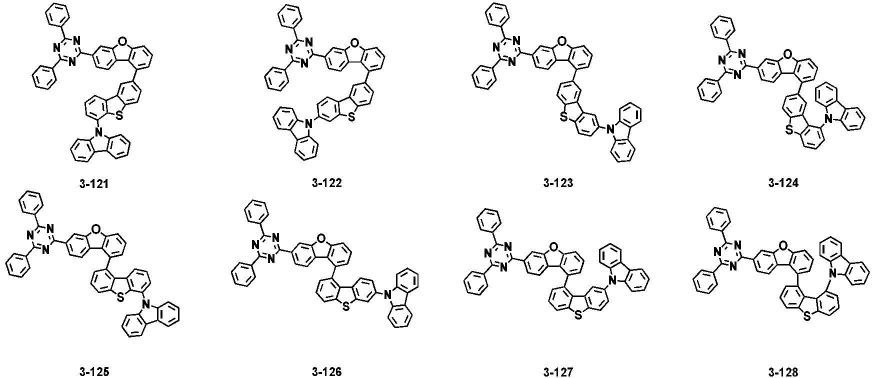

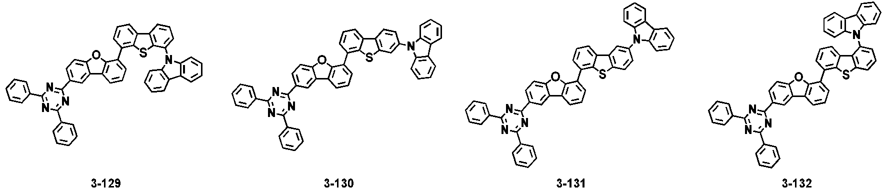

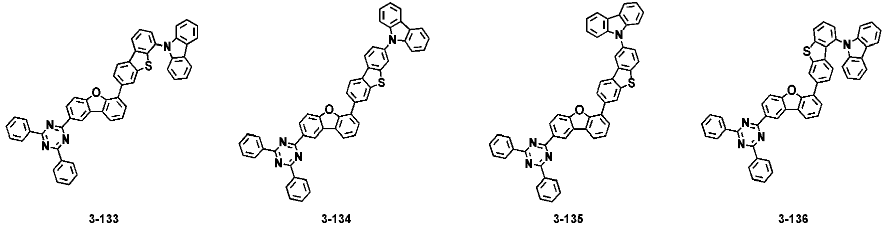

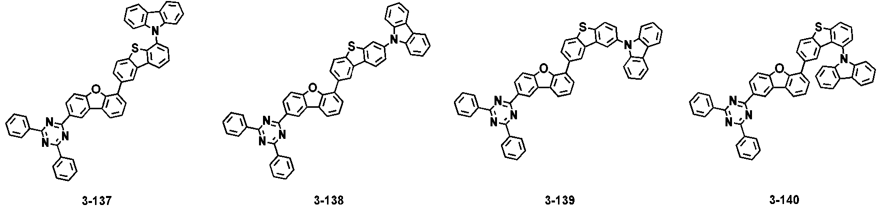

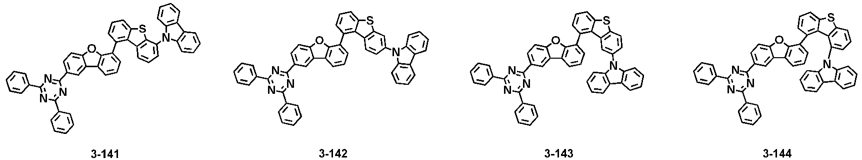

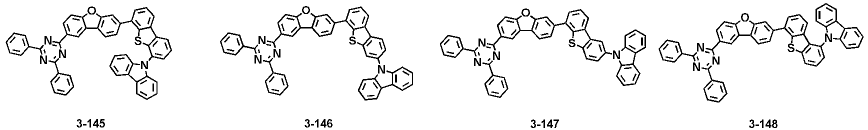

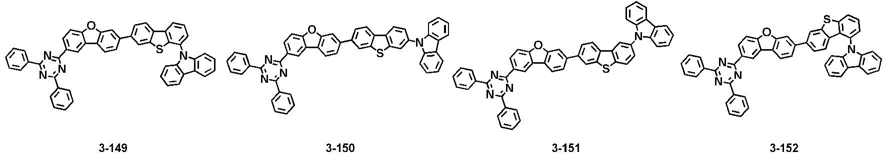

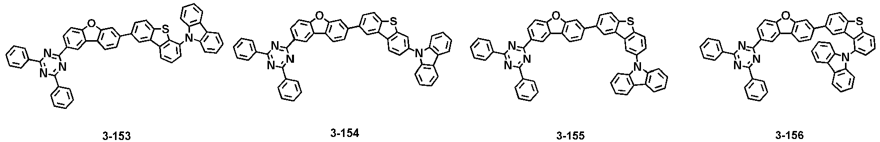

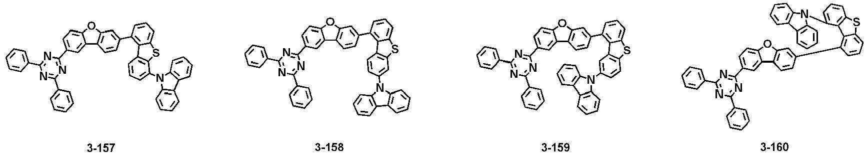

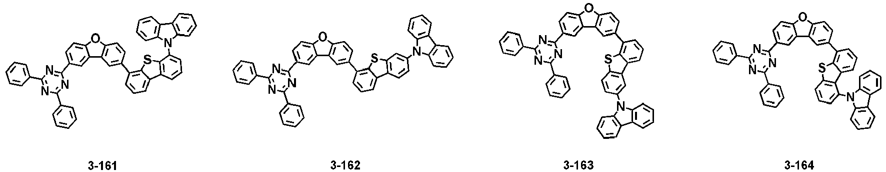

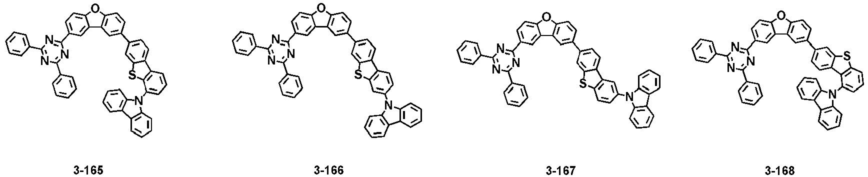

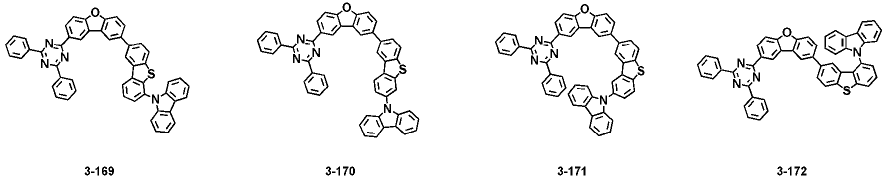

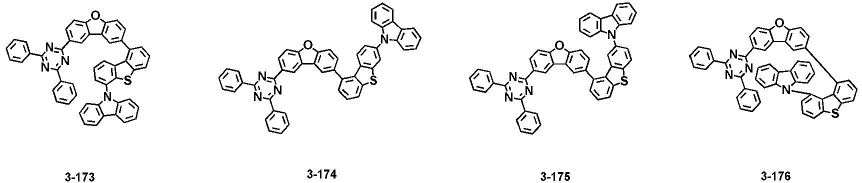

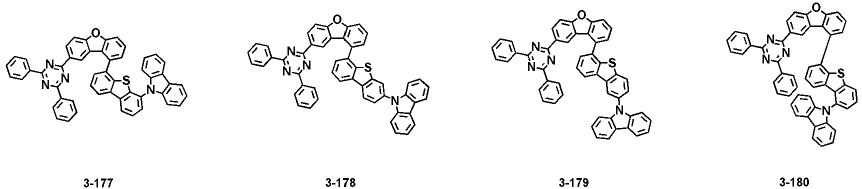

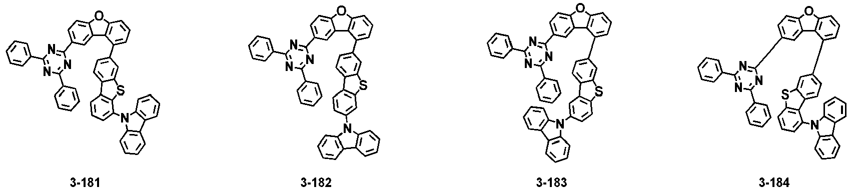

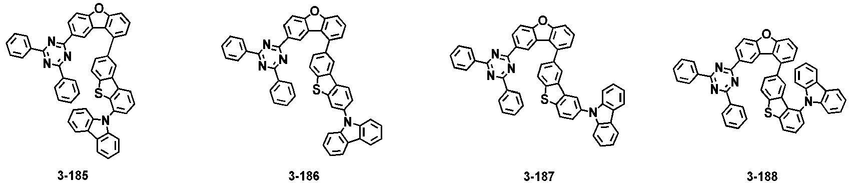

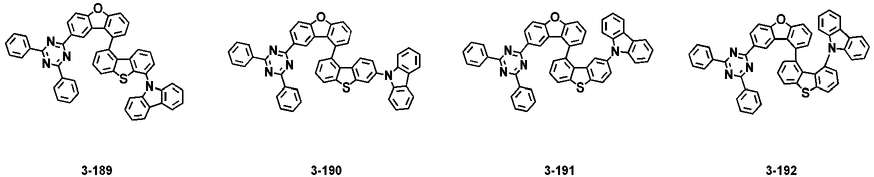

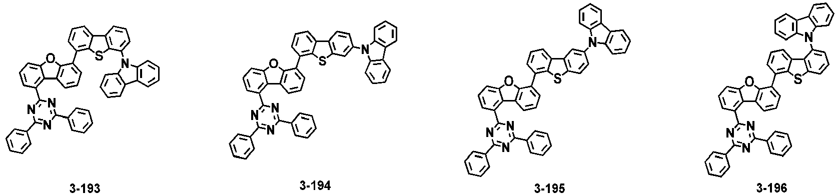

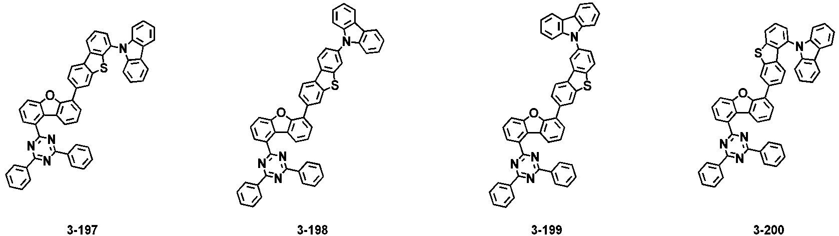

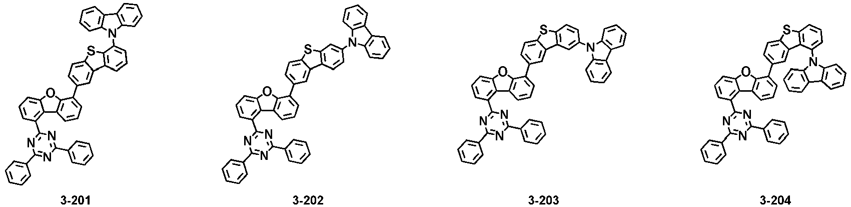

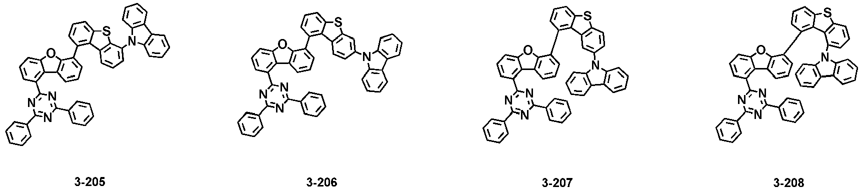

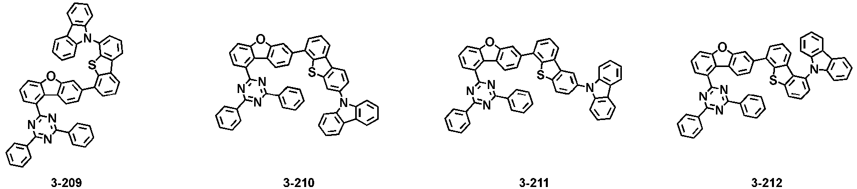

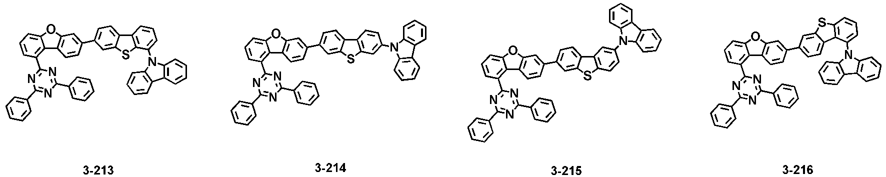

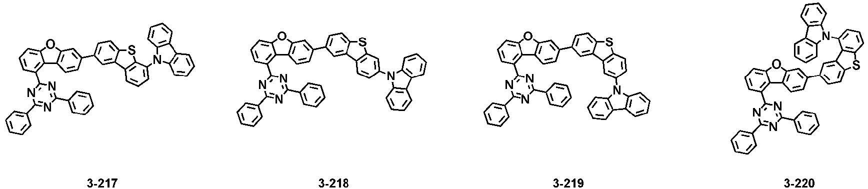

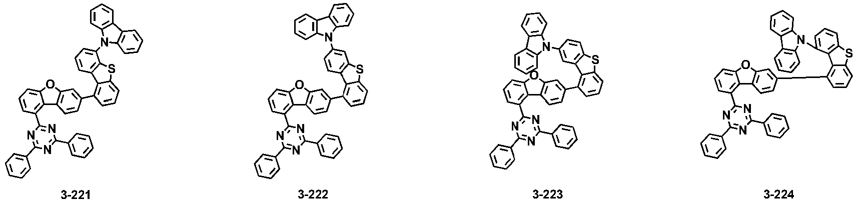

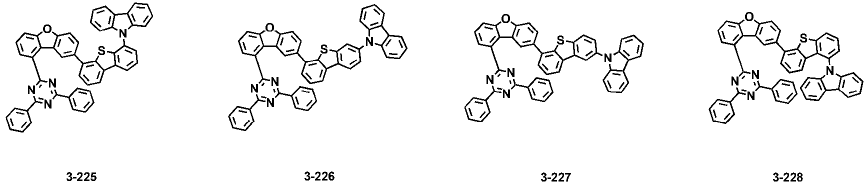

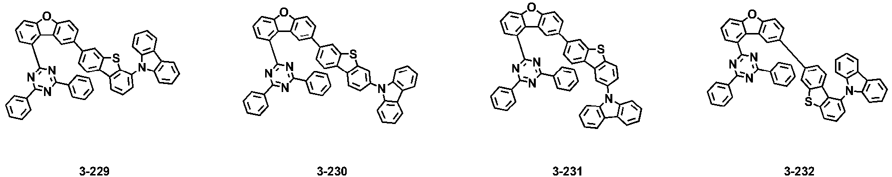

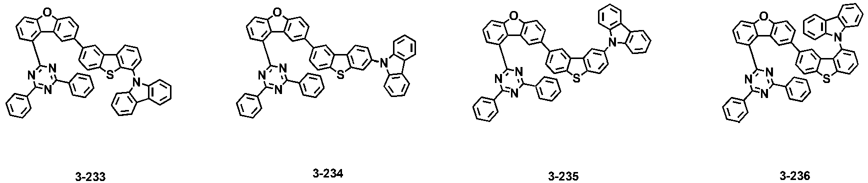

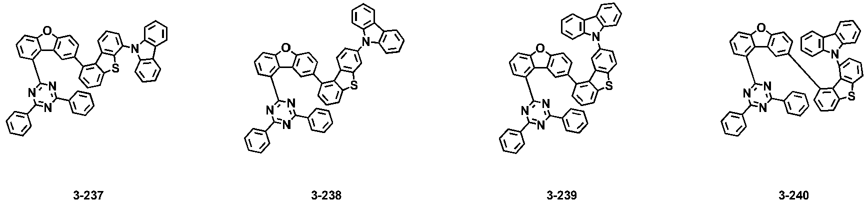

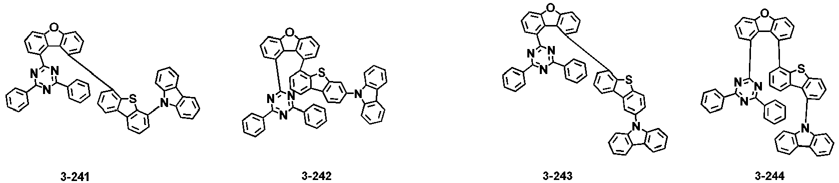

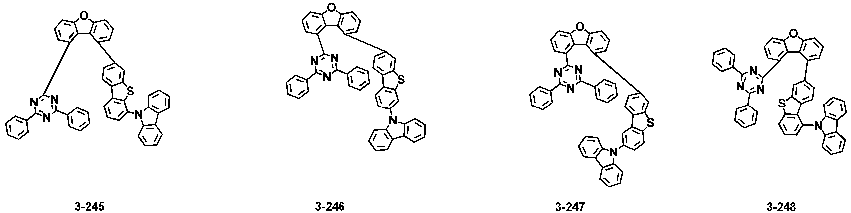

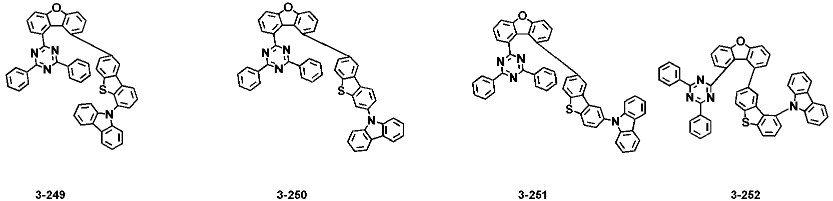

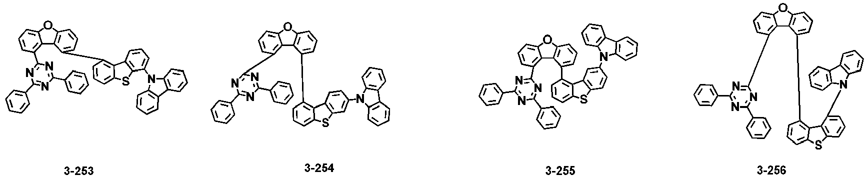

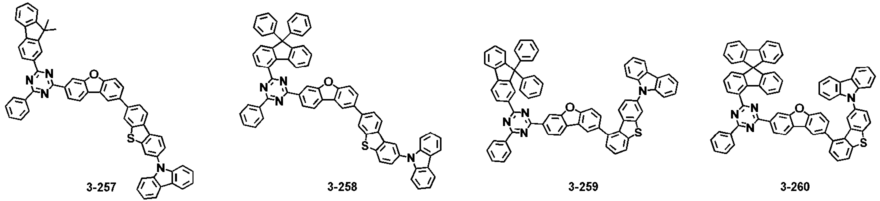

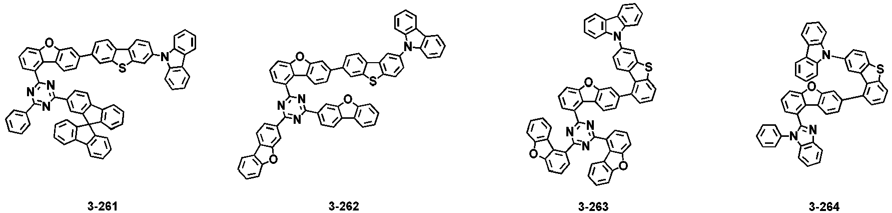

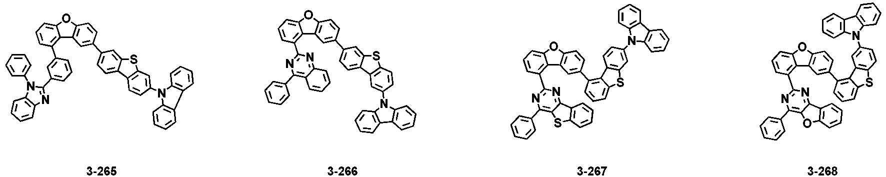

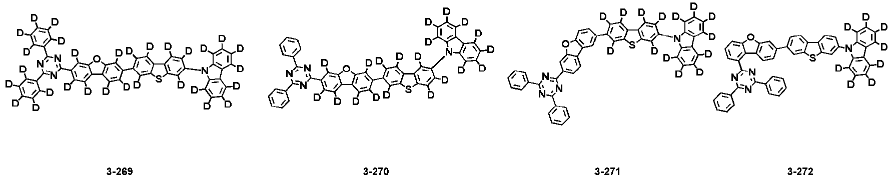

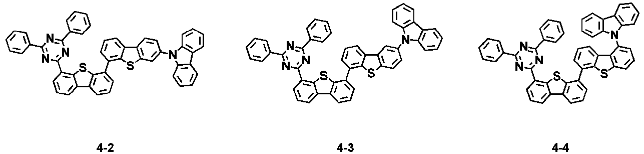

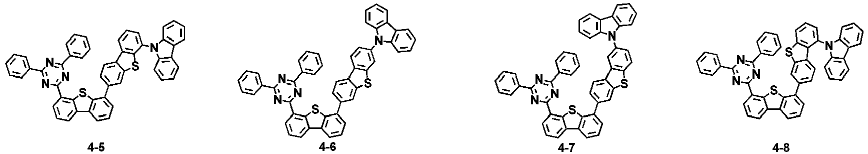

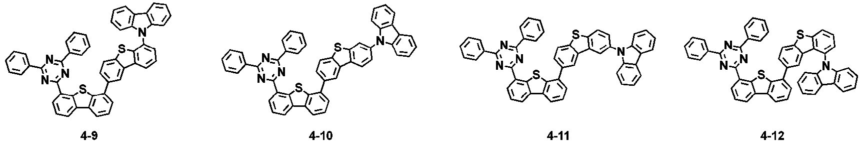

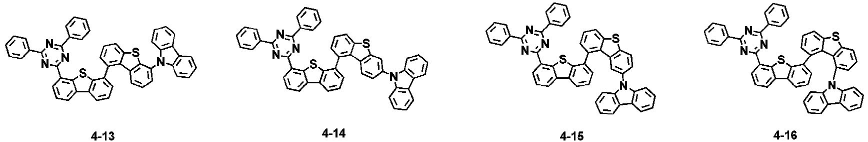

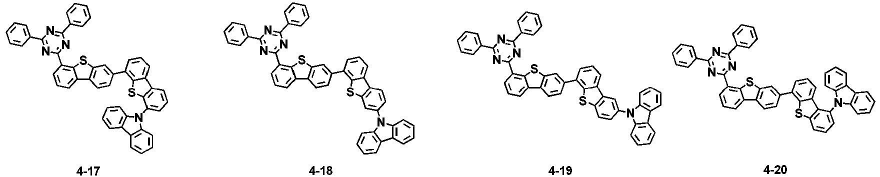

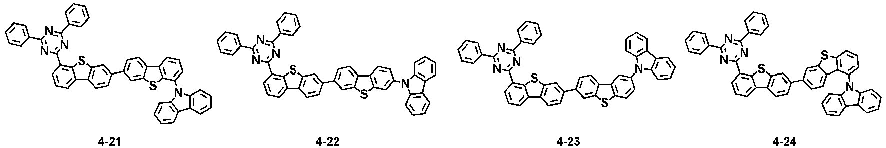

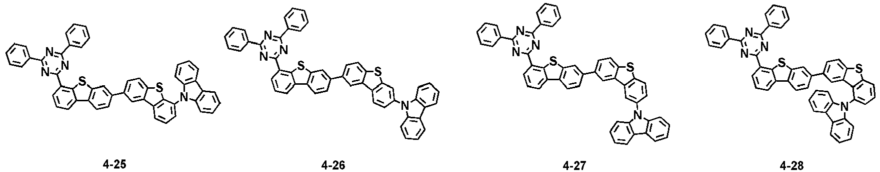

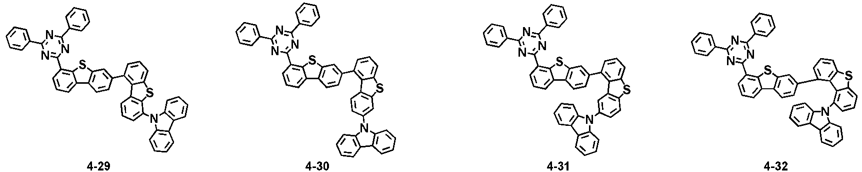

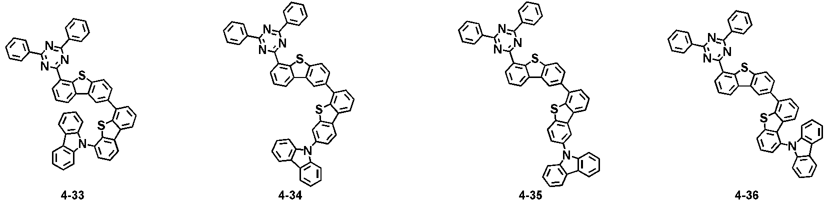

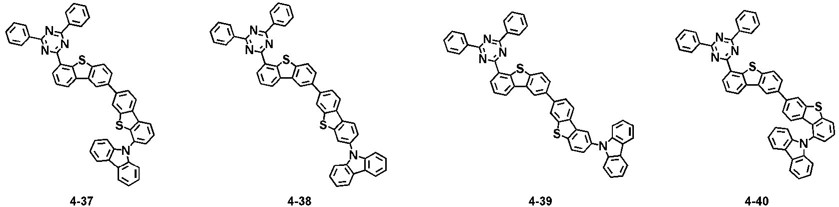

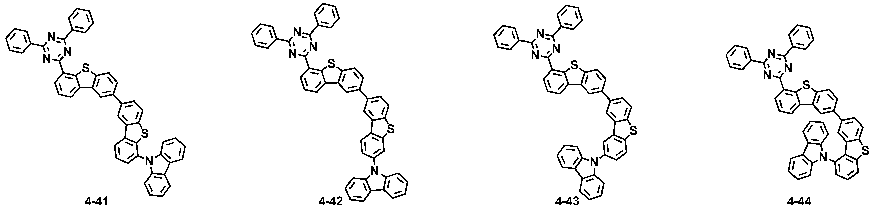

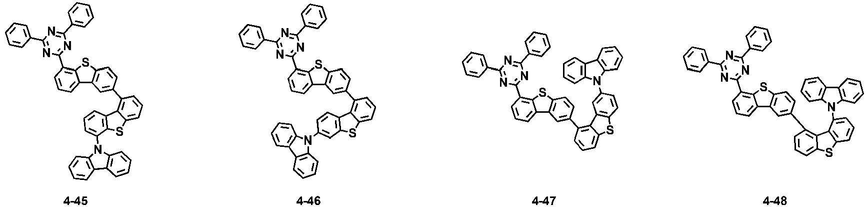

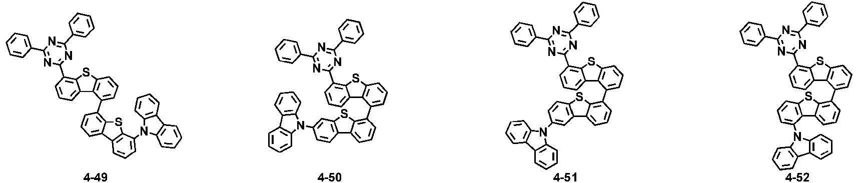

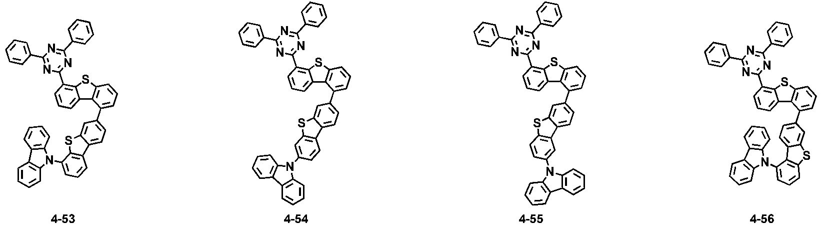

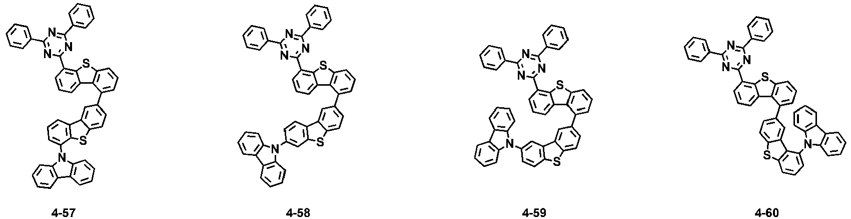

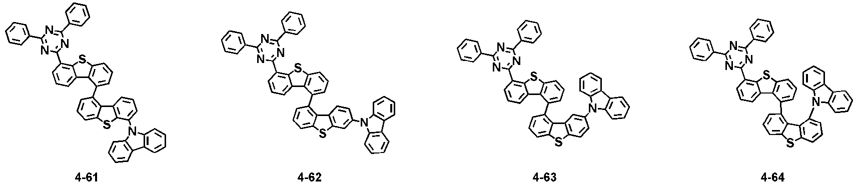

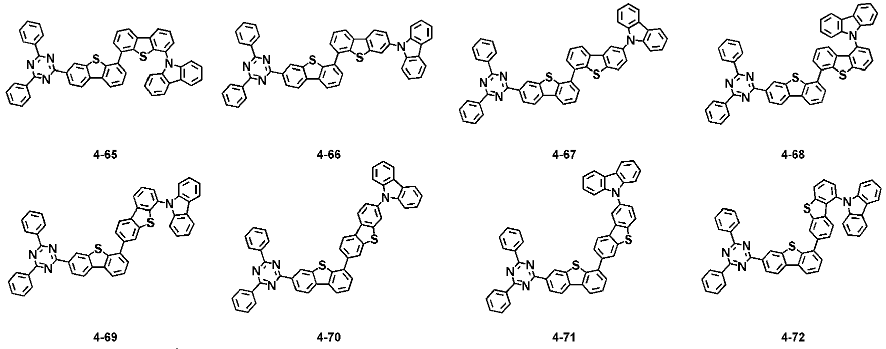

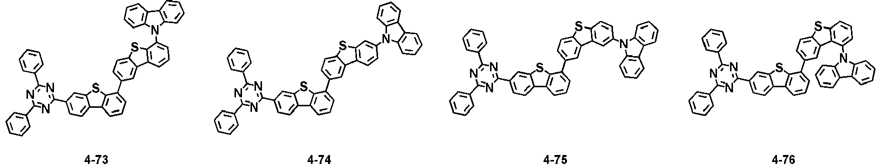

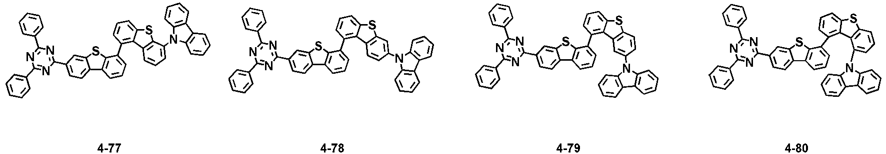

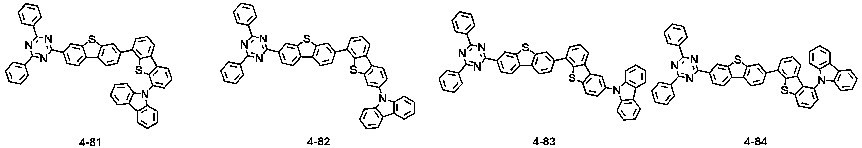

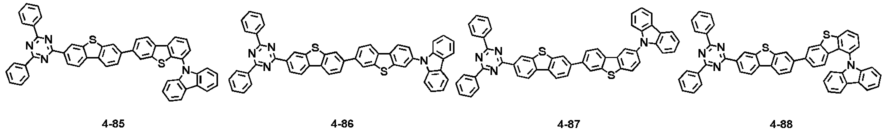

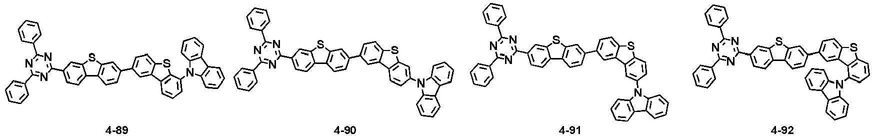

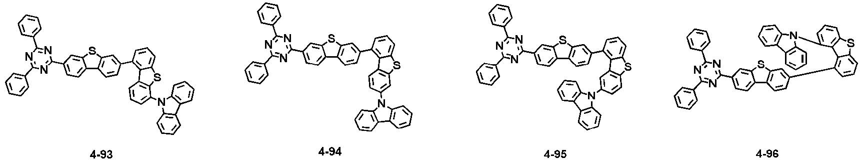

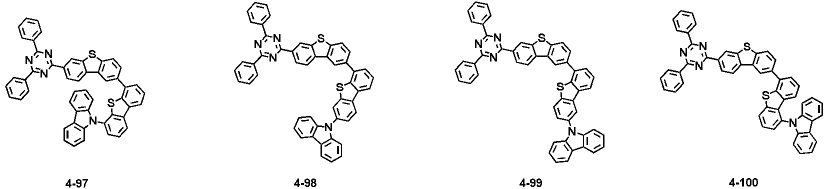

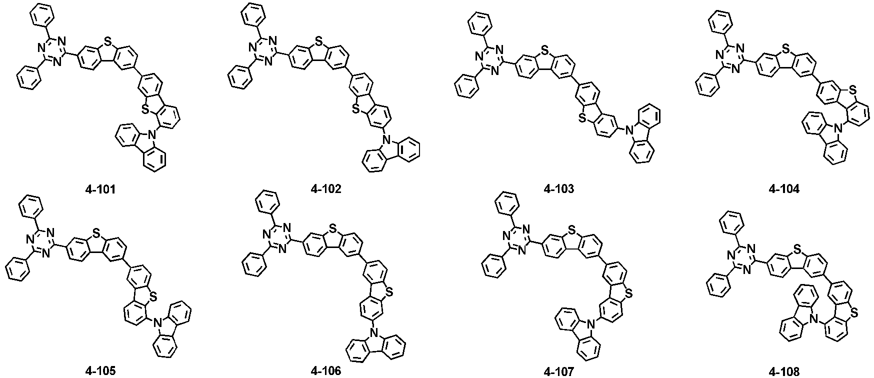

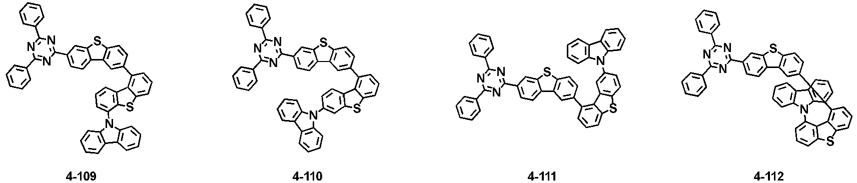

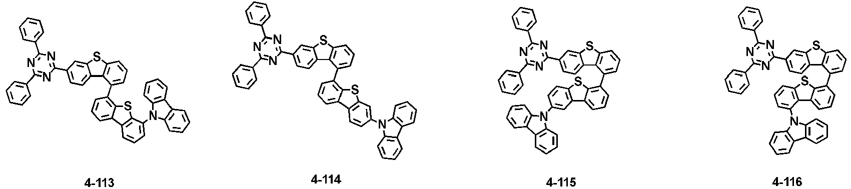

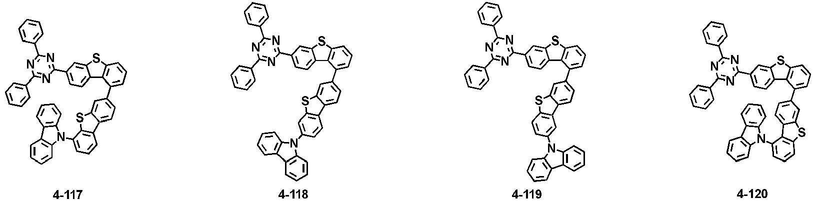

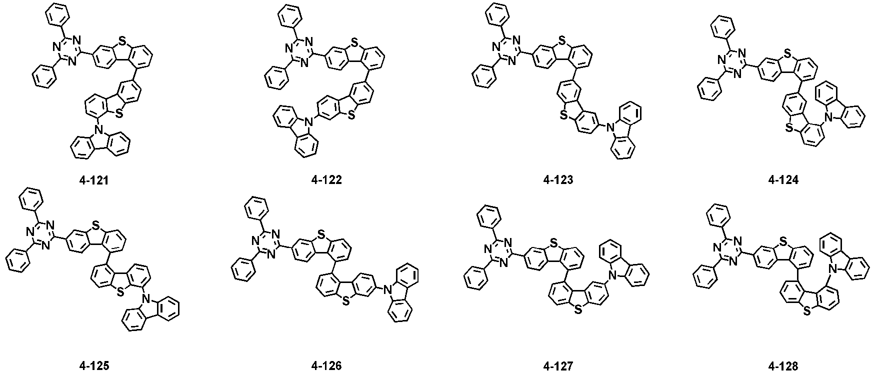

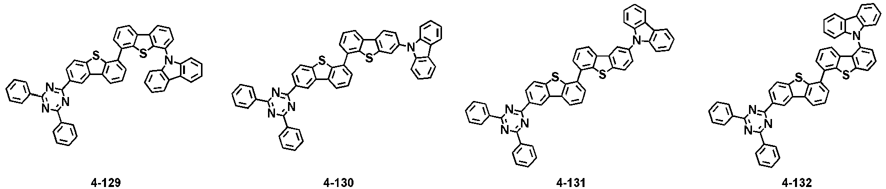

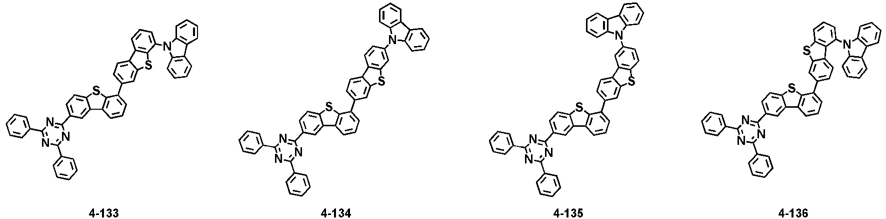

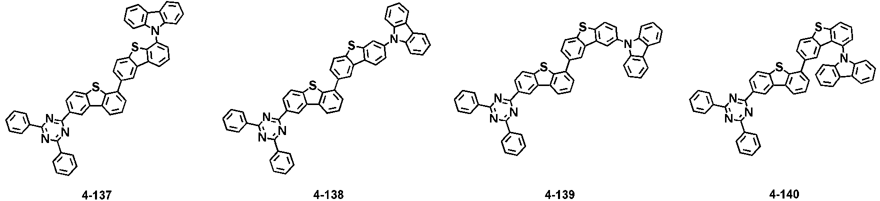

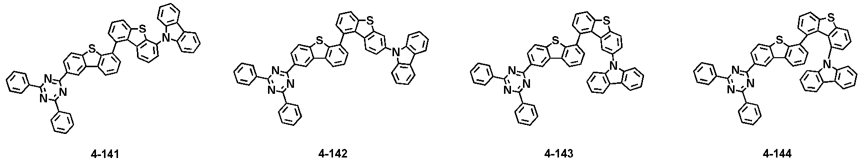

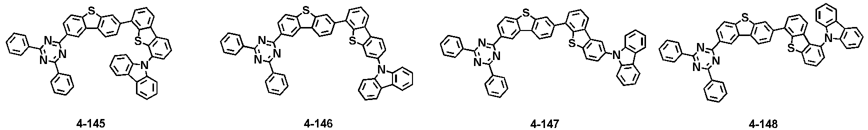

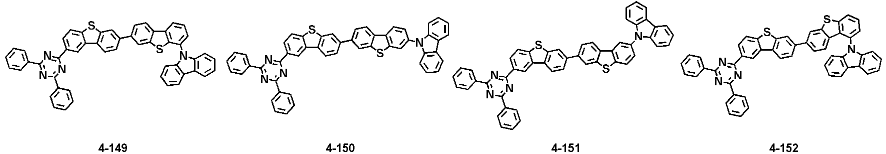

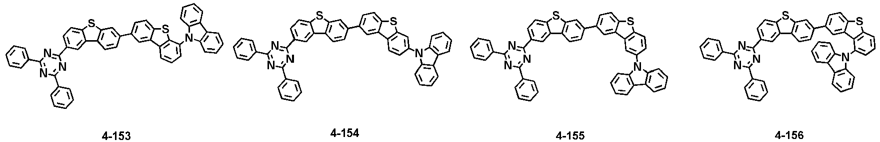

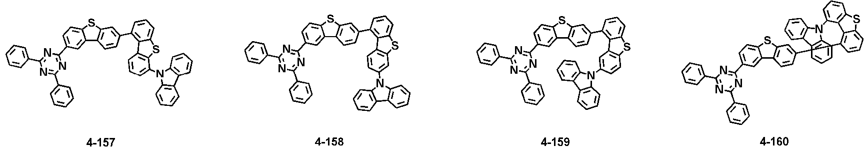

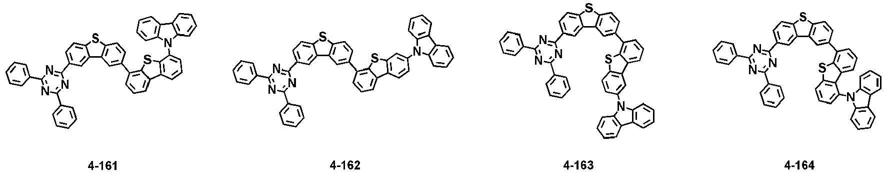

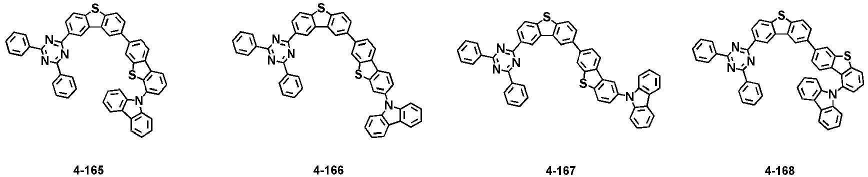

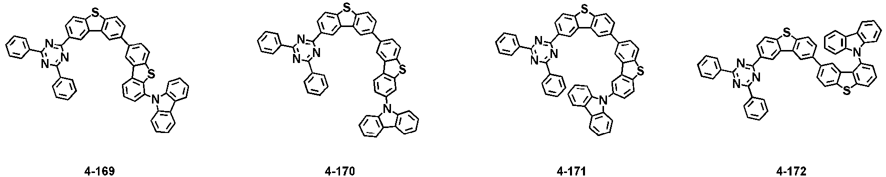

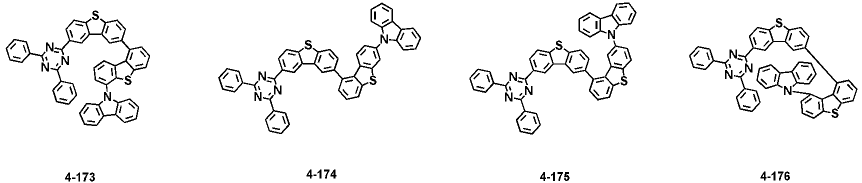

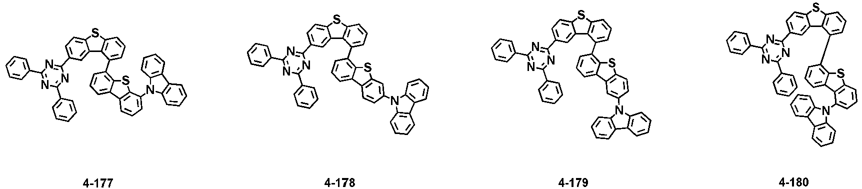

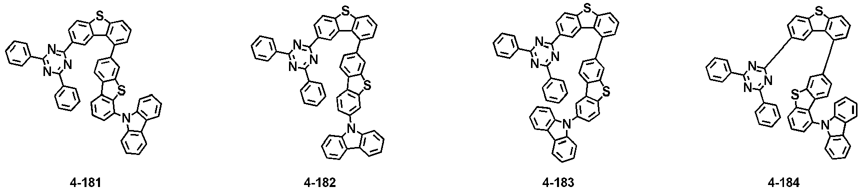

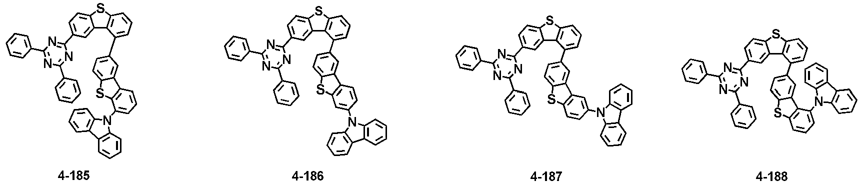

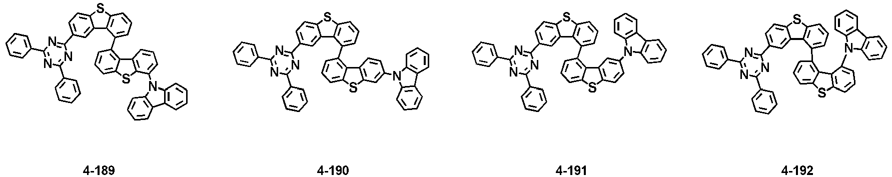

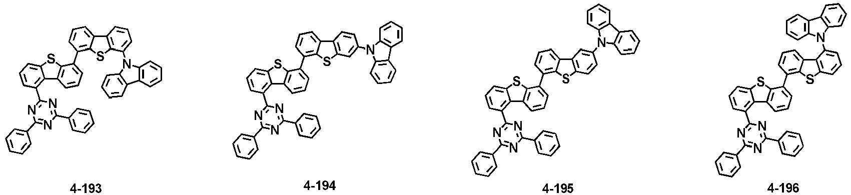

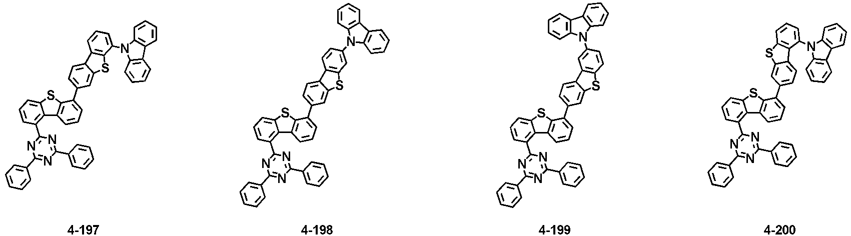

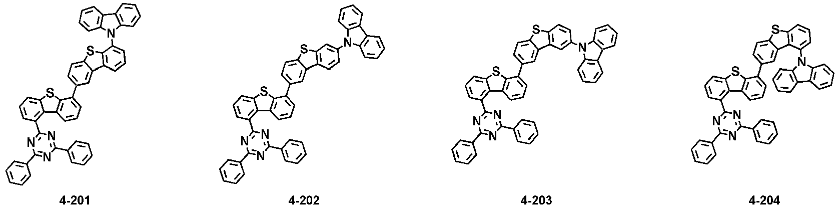

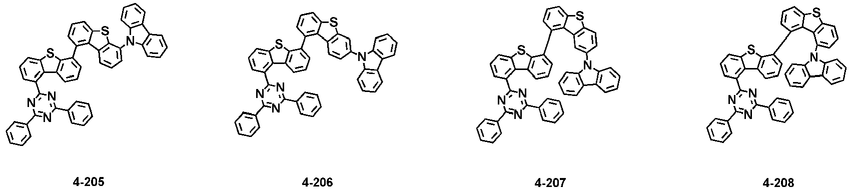

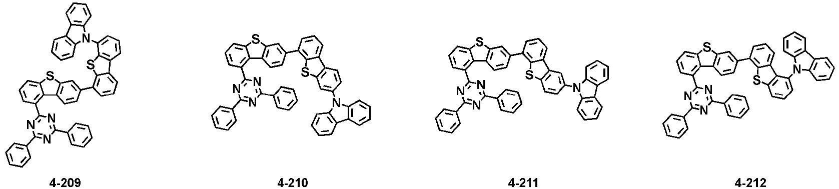

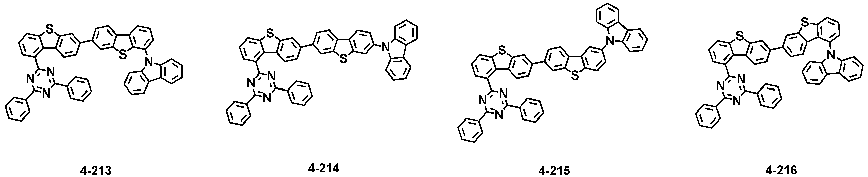

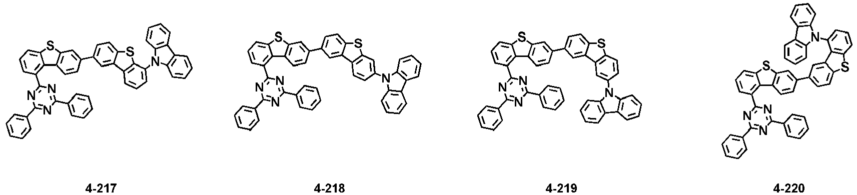

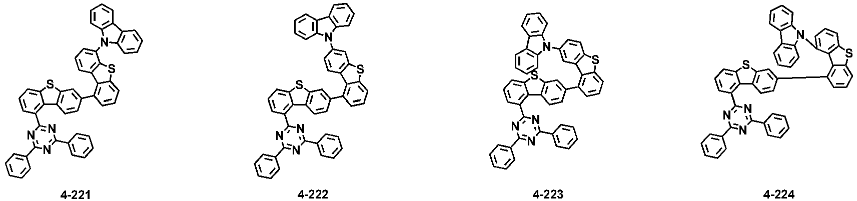

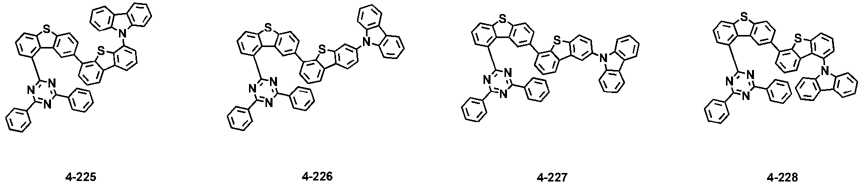

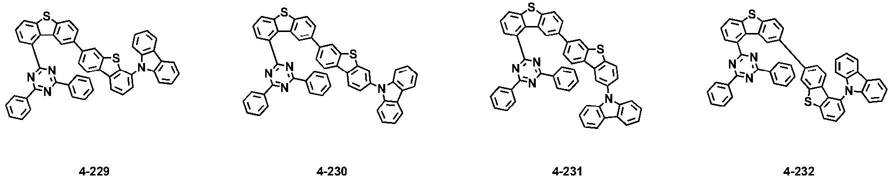

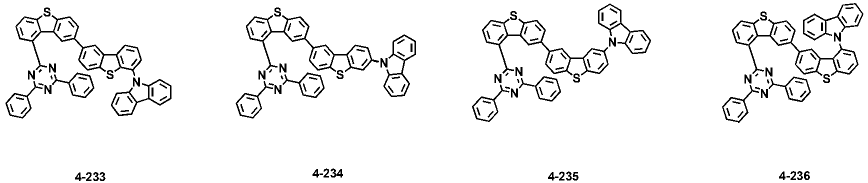

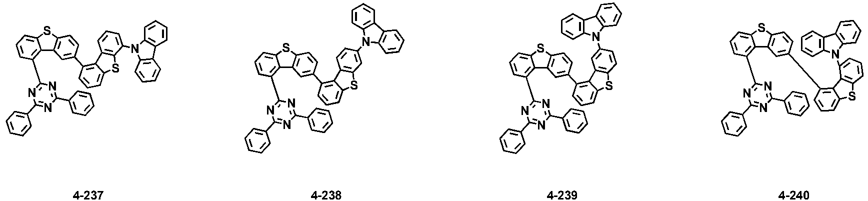

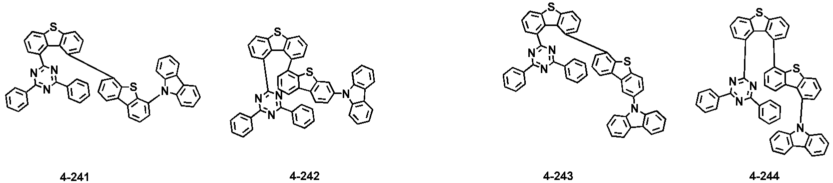

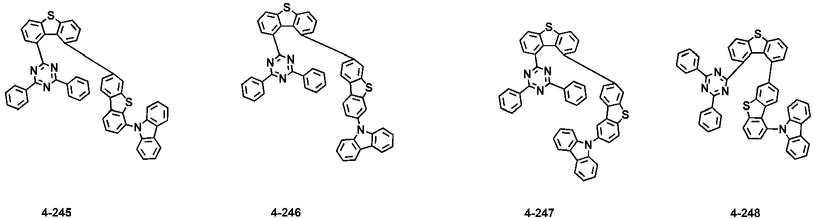

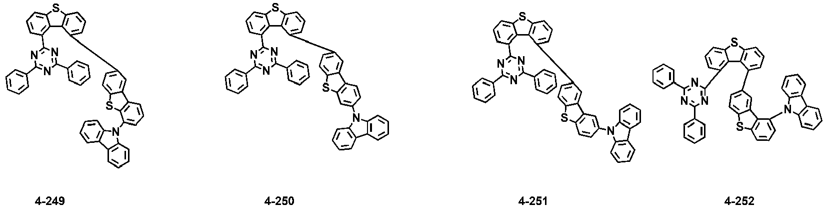

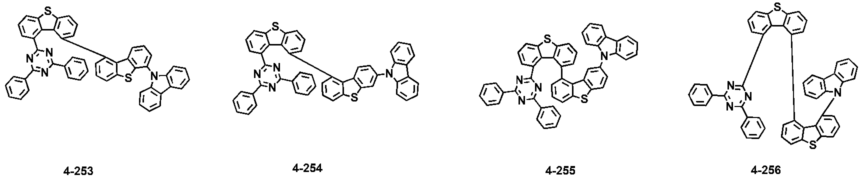

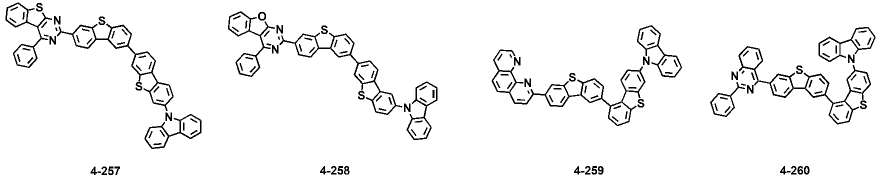

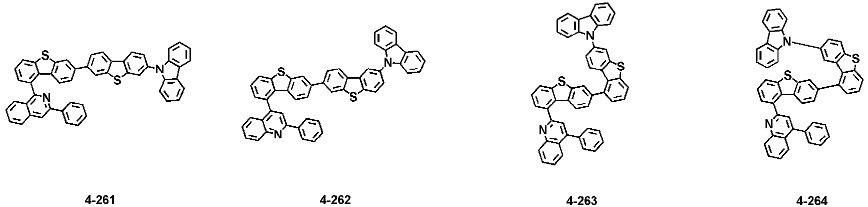

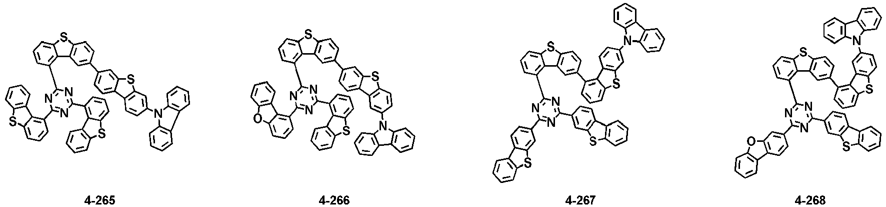

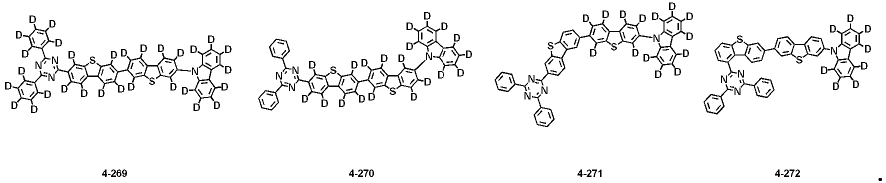

4. Eine organische lichtemittierende Vorrichtung, umfassend:
eine erste Elektrode;
eine zweite Elektrode; und
eine oder mehrere organische Materialschichten, die zwischen der ersten Elektrode und der zweiten Elektrode angeordnet sind,
wobei eine oder mehrere Schichten der organischen Materialschichten die heterocyclische Verbindung gemäß einem der Ansprüche 1 bis 3 umfassen.

5. Die organische lichtemittierende Vorrichtung nach Anspruch 4, wobei die organische Materialschicht eine oder mehrere lichtemittierende Schichten umfasst, und die lichtemittierende Schicht die heterocyclische Verbindung umfasst.

6. Die organische lichtemittierende Vorrichtung nach Anspruch 5, wobei die lichtemittierende Schicht zwei oder mehr Wirtsmaterialien umfasst, und mindestens eines der Wirtsmaterialien die heterocyclische Verbindung als Wirtsmaterial eines lichtemittierenden Materials umfasst.

7. Die organische lichtemittierende Vorrichtung nach Anspruch 4, ferner umfassend eine, zwei oder mehr Schichten, ausgewählt aus der Gruppe bestehend aus einer lichtemittierenden Schicht, einer Lochinjektionsschicht, einer Lochtransportschicht, einer Elektroneninjektionsschicht, einer Elektronentransportschicht, einer lochunterstützenden Schicht und einer Lochblockierschicht.

8. Die organische lichtemittierende Vorrichtung nach Anspruch 4, wobei die organische Materialschicht die heterocyclische Verbindung gemäß einem der Ansprüche 1 bis 3 als erste Verbindung umfasst, und ferner eine heterocyclische Verbindung, dargestellt durch die nachfolgende chemische Formel 13 oder 14, als zweite Verbindung umfasst: 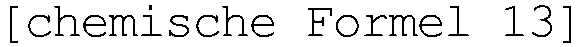 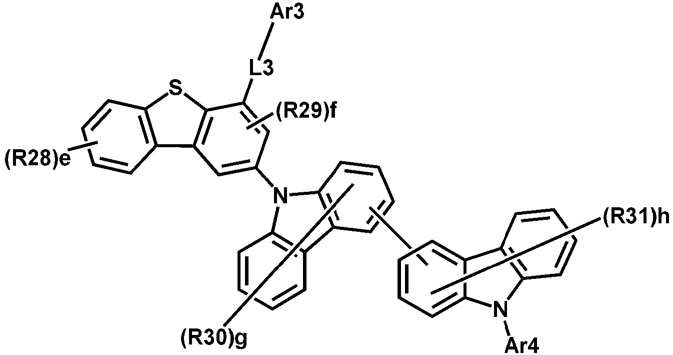 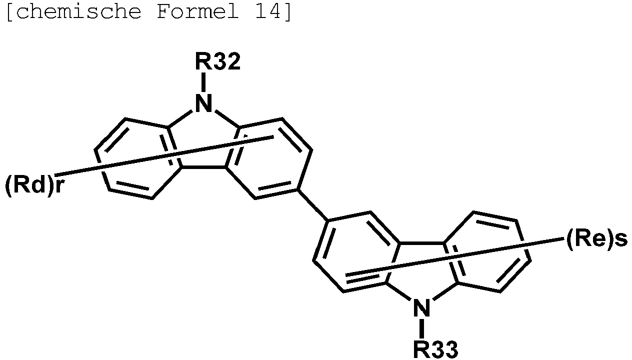 wobei in den chemischen Formeln 13 und 14 gilt:
L3 ist eine direkte Bindung; eine substituierte oder unsubstituierte Arylen-Gruppe mit 6 bis 60 Kohlenstoffatomen; oder eine substituierte oder unsubstituierte Heteroarylen-Gruppe mit 2 bis 60 Kohlenstoffatomen;
R28 bis R31, Rd und Re sind gleich oder verschieden und jeweils unabhängig ausgewählt aus der Gruppe bestehend aus Wasserstoff; Deuterium; Halogen; einer Cyano-Gruppe; einer substituierten oder unsubstituierten Alkylgruppe mit 1 bis 60 Kohlenstoffatomen; einer substituierten oder unsubstituierten Alkenylgruppe mit 2 bis 60 Kohlenstoffatomen; einer substituierten oder unsubstituierten Alkynylgruppe mit 2 bis 60 Kohlenstoffatomen; einer substituierten oder unsubstituierten Alkoxygruppe mit 2 bis 60 Kohlenstoffatomen; einer substituierten oder unsubstituierten Cycloalkylgruppe mit 3 bis 60 Kohlenstoffatomen; einer substituierten oder unsubstituierten Heterocycloalkylgruppe mit 2 bis 60 Kohlenstoffatomen; einer substituierten oder unsubstituierten Arylgruppe mit 6 bis 60 Kohlenstoffatomen; einer substituierten oder unsubstituierten Heteroarylgruppe mit 2 bis 60 Kohlenstoffatomen; einer substituierten oder unsubstituierten Phosphinoxidgruppe; und einer substituierten oder unsubstituierten Aminogruppe, oder zwei oder mehr benachbarte Gruppen sind miteinander verbunden, um einen substituierten oder unsubstituierten aromatischen Kohlenwasserstoffring mit 6 bis 60 Kohlenstoffatomen oder einen substituierten oder unsubstituierten Heteroring mit 2 bis 60 Kohlenstoffatomen zu bilden, r und s sind ganze Zahlen von 0 bis 7, und wenn r 2 oder größer ist, sind die Rds gleich oder verschieden voneinander, wenn s 2 oder größer ist, sind die Res gleich oder verschieden voneinander, e ist eine ganze Zahl von 0 bis 4, g und h sind jeweils ganze Zahlen von 0 bis 7, f ist eine ganze Zahl von 0 bis 2, und wenn e 2 oder größer ist, sind die R28s gleich oder verschieden voneinander, wenn f eine ganze Zahl von 2 ist, sind die R29s gleich oder verschieden voneinander, wenn g 2 oder größer ist, sind die R30s gleich oder verschieden voneinander, und wenn h 2 oder größer ist, sind die R31s gleich oder verschieden voneinander;
Ar3 ist eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen;
Ar4 ist eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen; und
R32 und R33 sind gleich oder verschieden und jeweils unabhängig eine substituierte oder unsubstituierte Silylgruppe; eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen.

9. Die organische lichtemittierende Vorrichtung nach Anspruch 8, wobei die durch die chemische Formel 13 dargestellte heterocyclische Verbindung einen Deuteriumgehalt von 0%, oder größer als 10% und kleiner oder gleich 100% aufweist.

10. Die organische lichtemittierende Vorrichtung nach Anspruch 8, wobei die durch die chemische Formel 14 dargestellte heterocyclische Verbindung einen Deuteriumgehalt von 0%, oder größer als 10% und kleiner oder gleich 100% aufweist.

11. Die organische lichtemittierende Vorrichtung nach Anspruch 8, wobei die chemische Formel 13 durch eine der folgenden Verbindungen dargestellt ist:

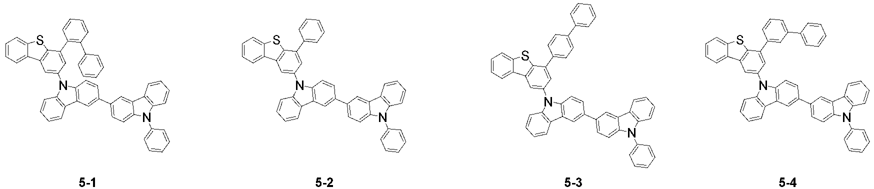

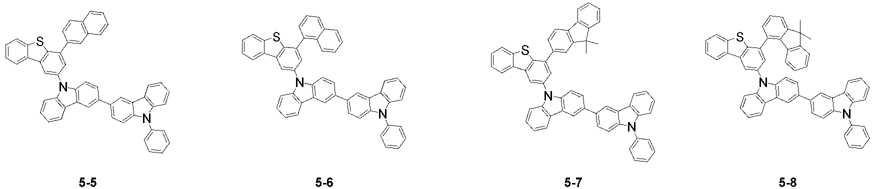

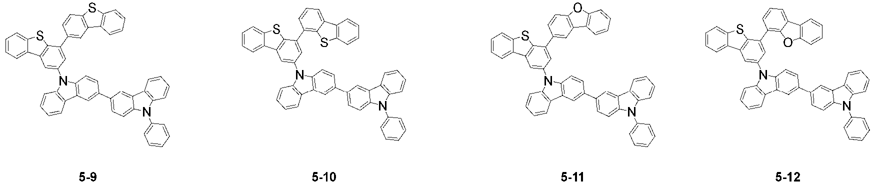

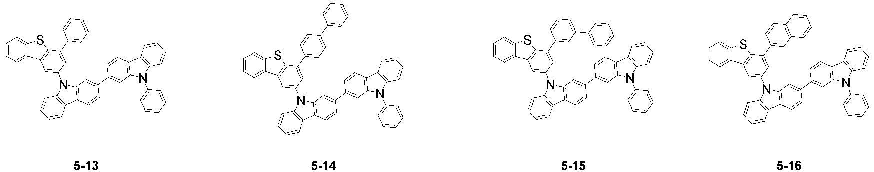

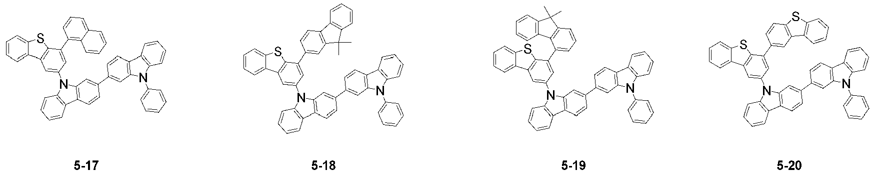

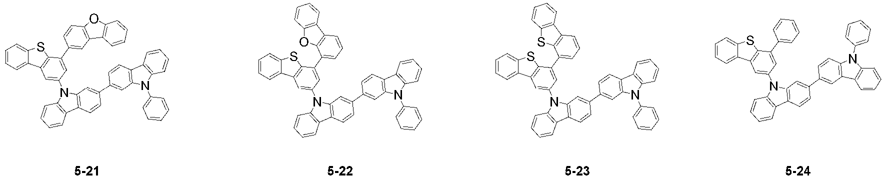

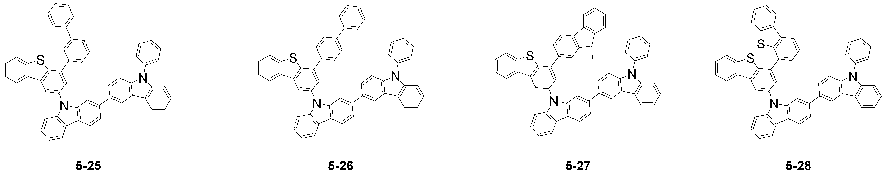

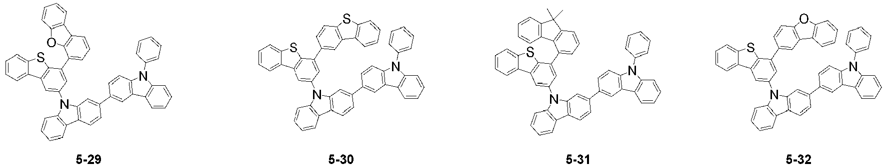

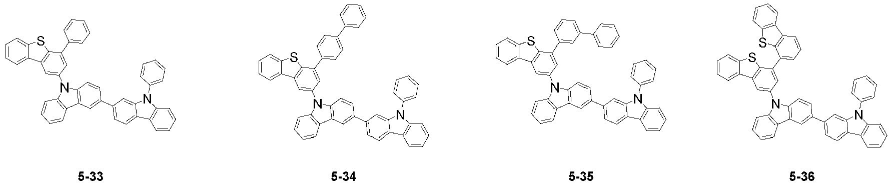

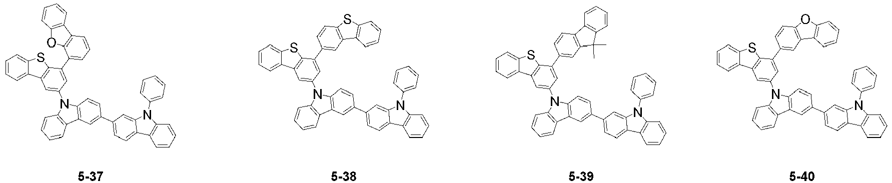

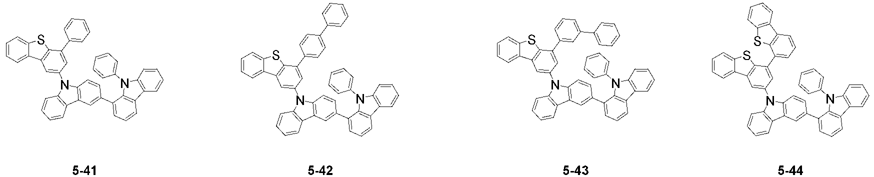

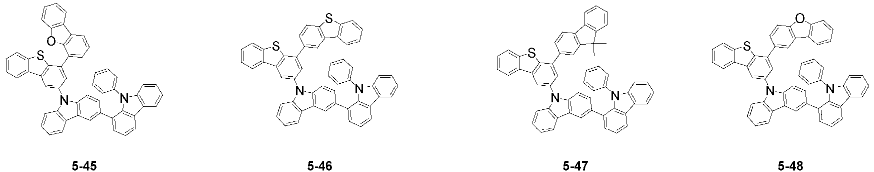

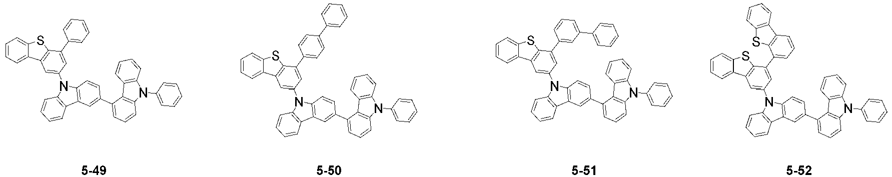

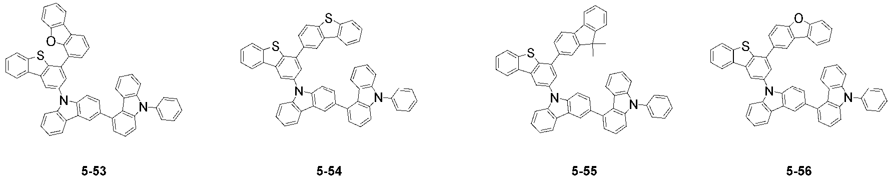

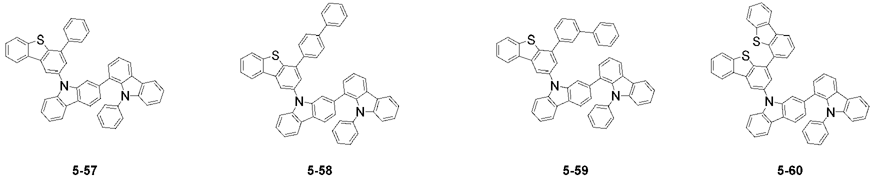

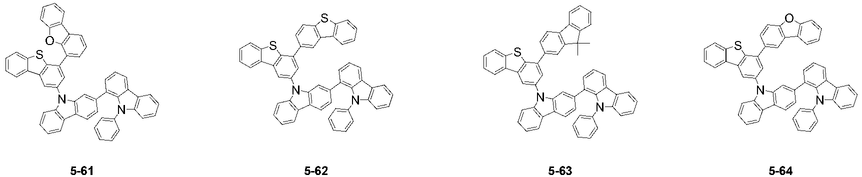

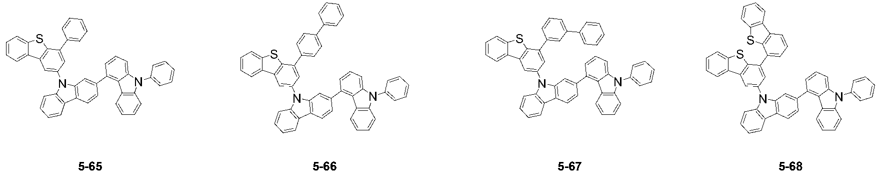

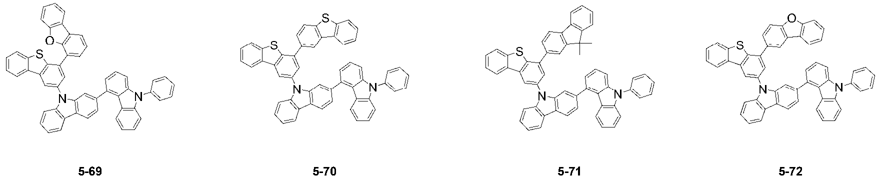

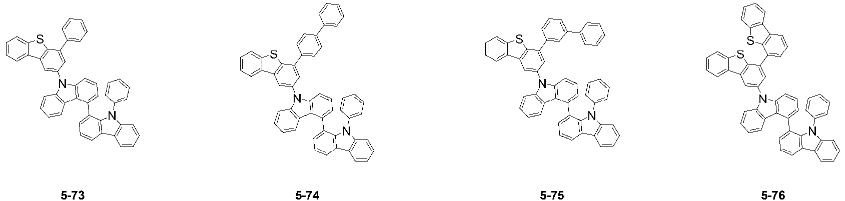

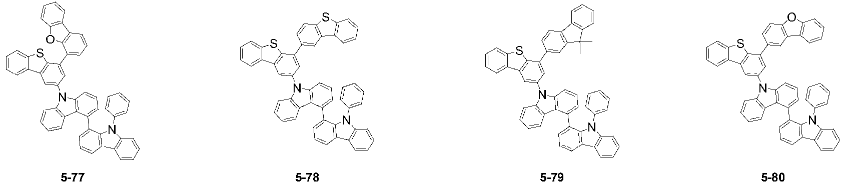

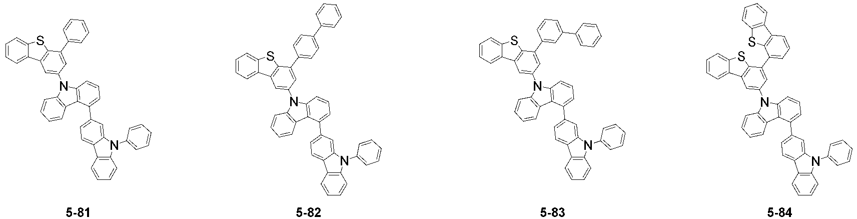

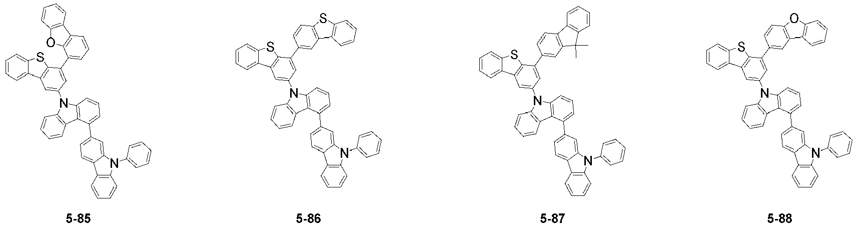

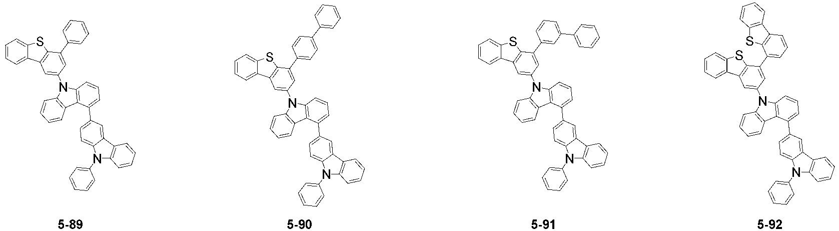

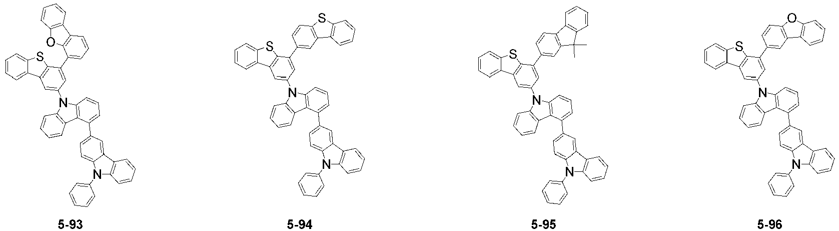

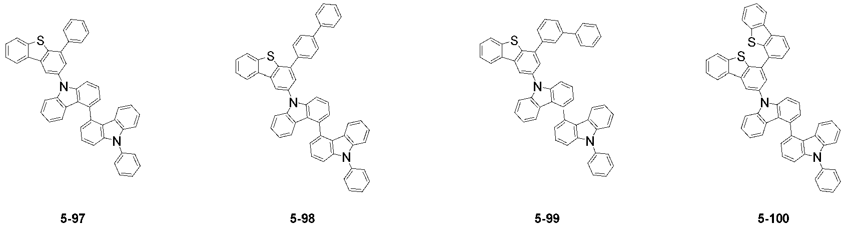

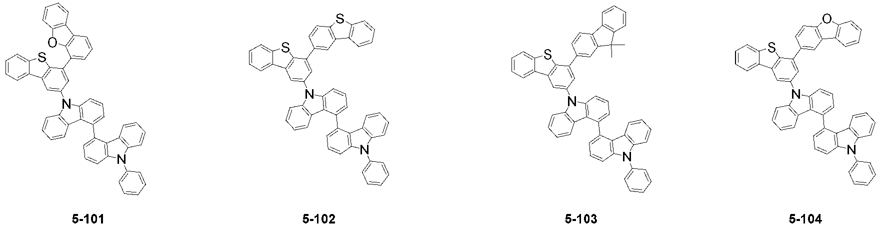

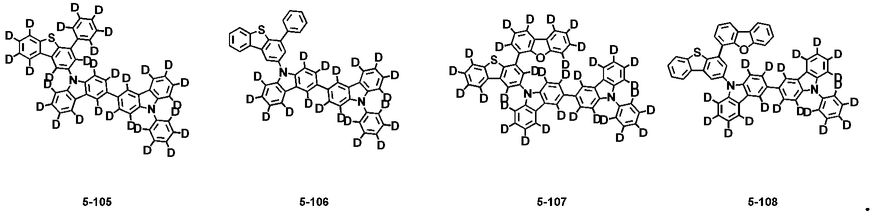

12. Die organische lichtemittierende Vorrichtung nach Anspruch 9, wobei die chemische Formel 14 durch eine der folgenden Verbindungen dargestellt ist:

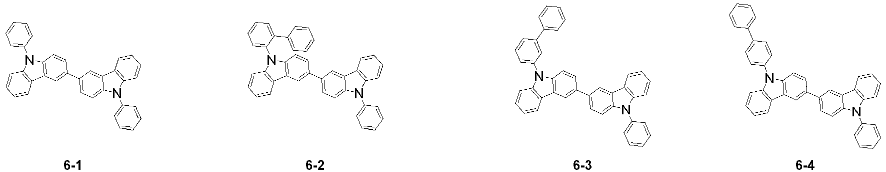

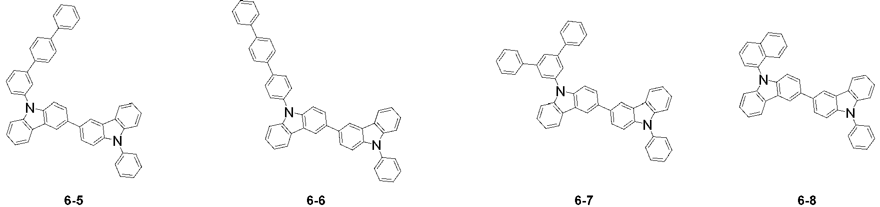

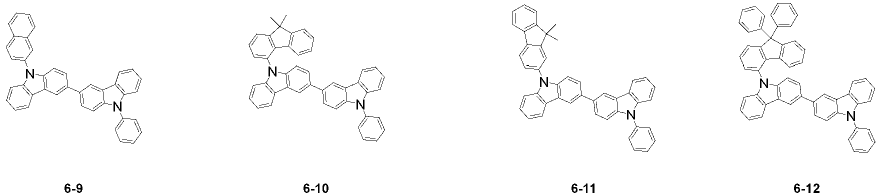

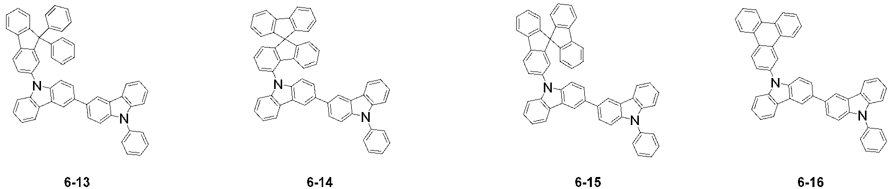

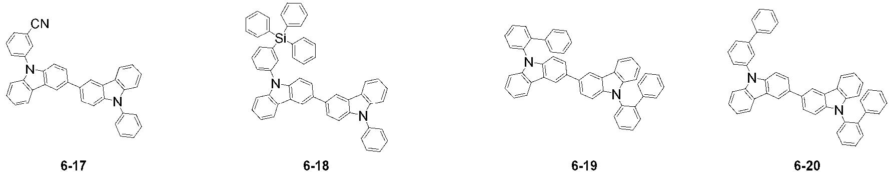

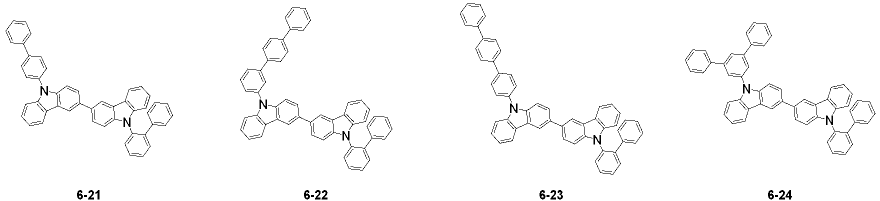

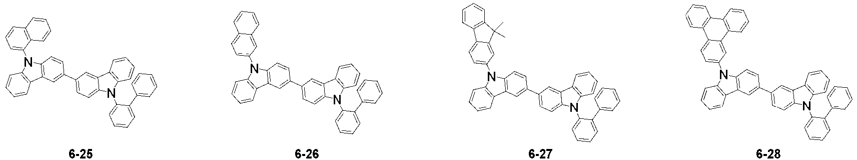

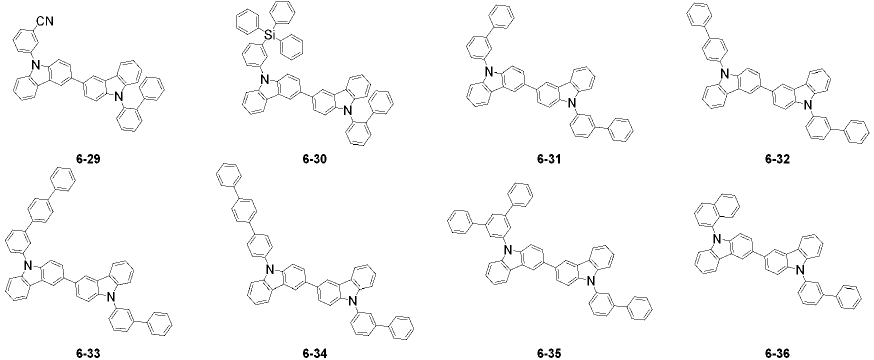

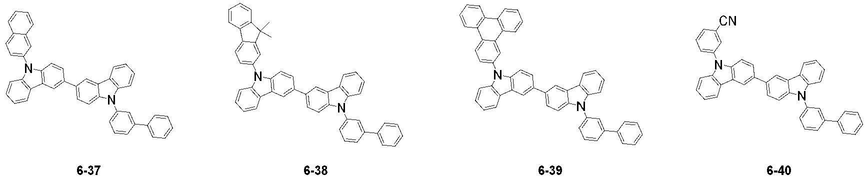

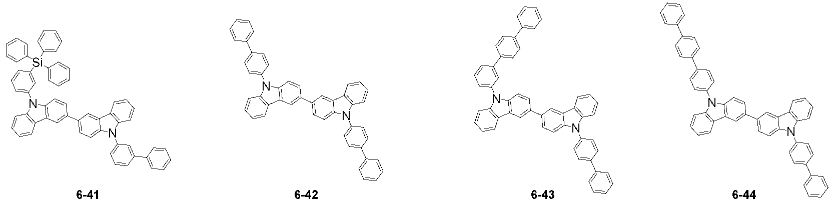

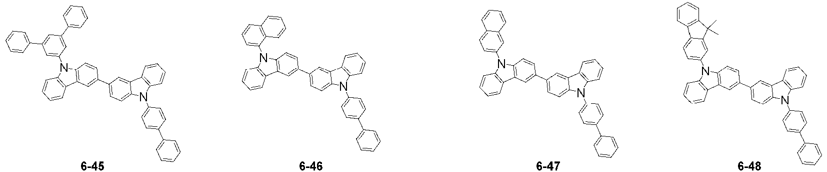

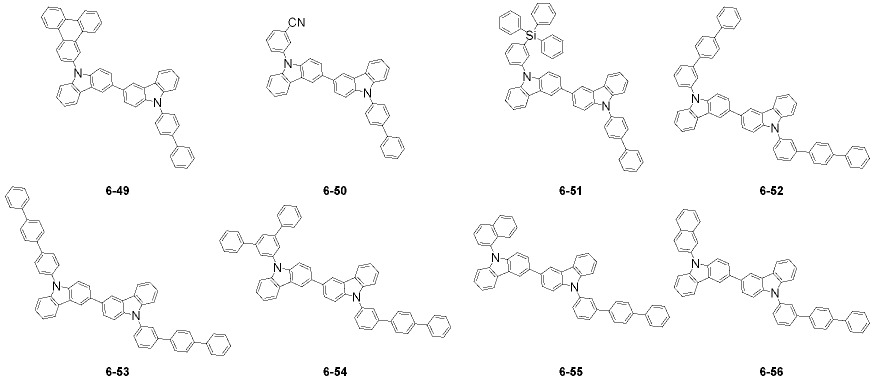

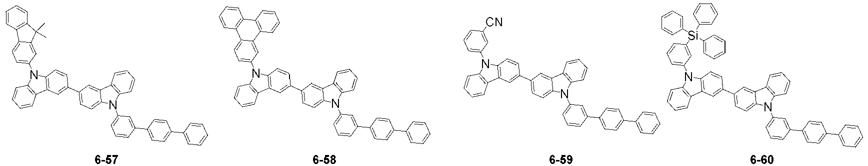

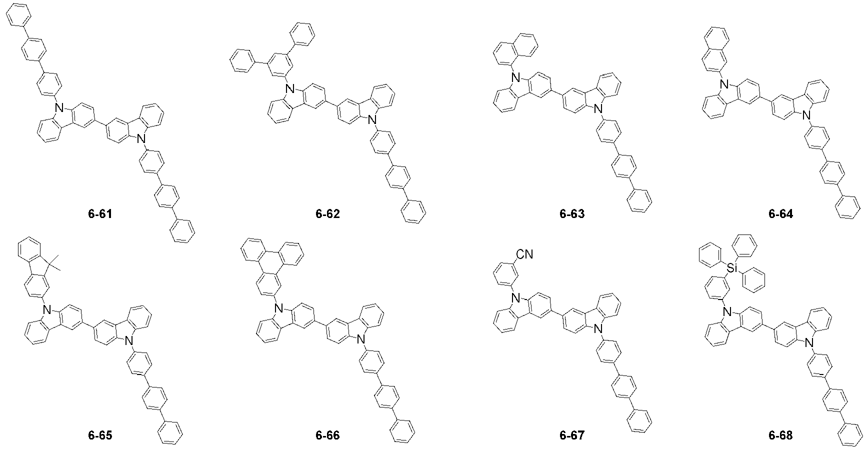

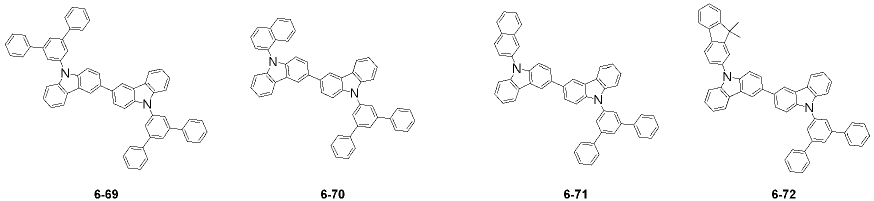

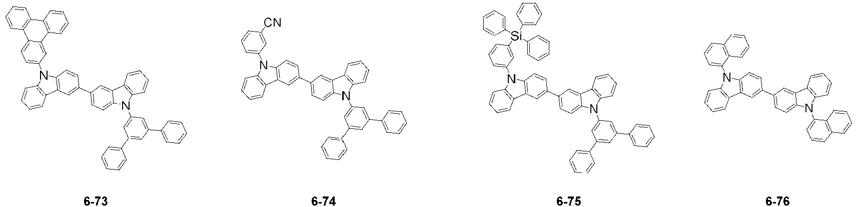

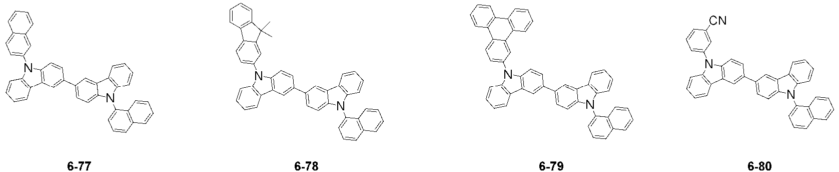

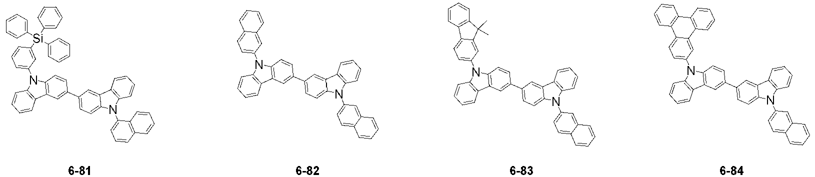

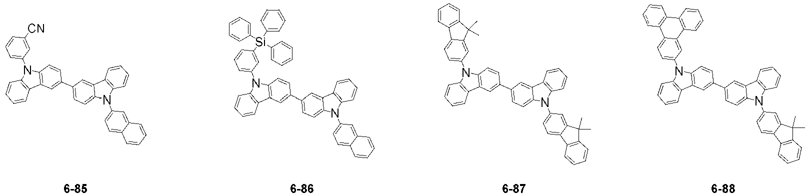

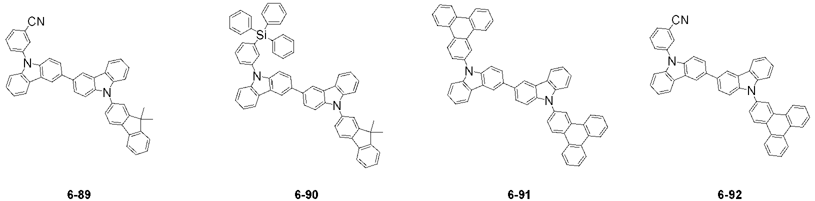

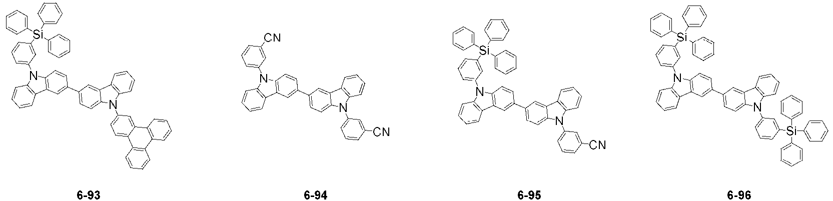

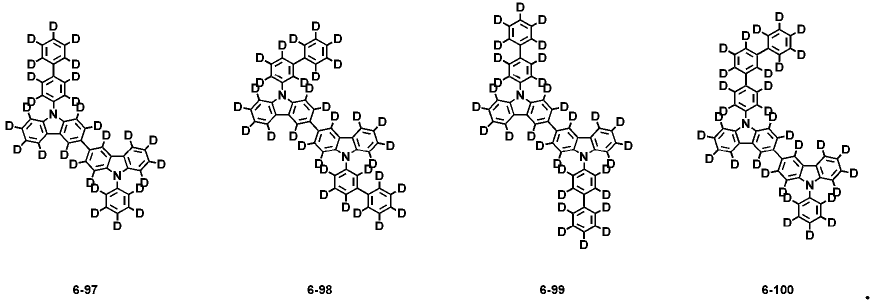

13. Eine Zusammensetzung für eine organische Materialschicht einer organischen lichtemittierenden Vorrichtung, wobei die Zusammensetzung umfasst:
die durch eine der chemischen Formeln 1-2 bis 1-4 gemäß Anspruch 1 dargestellte heterocyclische Verbindung; und
eine durch die nachfolgende chemische Formel 13 oder 14 dargestellte heterocyclische Verbindung: 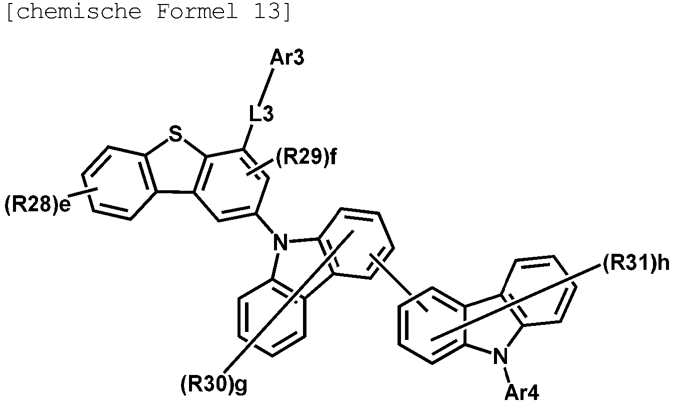 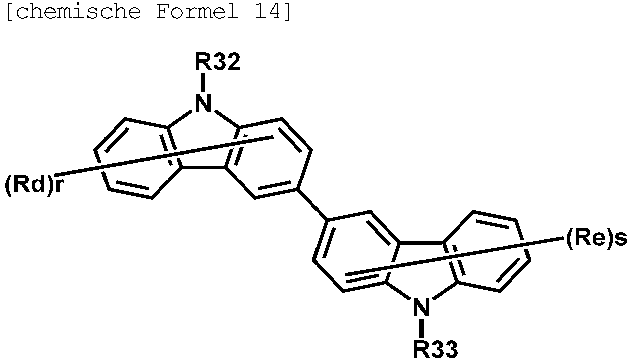 wobei in den chemischen Formeln 13 und 14 gilt:
L3 ist eine direkte Bindung; eine substituierte oder unsubstituierte Arylen-Gruppe mit 6 bis 60 Kohlenstoffatomen; oder eine substituierte oder unsubstituierte Heteroarylen-Gruppe mit 2 bis 60 Kohlenstoffatomen;
R28 bis R31, Rd und Re sind gleich oder verschieden und jeweils unabhängig ausgewählt aus der Gruppe bestehend aus Wasserstoff; Deuterium; Halogen; einer Cyano-Gruppe; einer substituierten oder unsubstituierten Alkylgruppe mit 1 bis 60 Kohlenstoffatomen; einer substituierten oder unsubstituierten Alkenylgruppe mit 2 bis 60 Kohlenstoffatomen; einer substituierten oder unsubstituierten Alkynylgruppe mit 2 bis 60 Kohlenstoffatomen; einer substituierten oder unsubstituierten Alkoxygruppe mit 2 bis 60 Kohlenstoffatomen; einer substituierten oder unsubstituierten Cycloalkylgruppe mit 3 bis 60 Kohlenstoffatomen; einer substituierten oder unsubstituierten Heterocycloalkylgruppe mit 2 bis 60 Kohlenstoffatomen; einer substituierten oder unsubstituierten Arylgruppe mit 6 bis 60 Kohlenstoffatomen; einer substituierten oder unsubstituierten Heteroarylgruppe mit 2 bis 60 Kohlenstoffatomen; einer substituierten oder unsubstituierten Phosphinoxidgruppe; und einer substituierten oder unsubstituierten Aminogruppe, oder zwei oder mehr benachbarte Gruppen sind miteinander verbunden, um einen substituierten oder unsubstituierten aromatischen Kohlenwasserstoffring mit 6 bis 60 Kohlenstoffatomen oder einen substituierten oder unsubstituierten Heteroring mit 2 bis 60 Kohlenstoffatomen zu bilden, r und s sind ganze Zahlen von 0 bis 7, und wenn r 2 oder größer ist, sind die Rds gleich oder verschieden voneinander, wenn s 2 oder größer ist, sind die Res gleich oder verschieden voneinander, e ist eine ganze Zahl von 0 bis 4, g und h sind jeweils ganze Zahlen von 0 bis 7, f ist eine ganze Zahl von 0 bis 2, und wenn e 2 oder größer ist, sind die R28s gleich oder verschieden voneinander, wenn f eine ganze Zahl von 2 ist, sind die R29s gleich oder verschieden voneinander, wenn g 2 oder größer ist, sind die R30s gleich oder verschieden voneinander, und wenn h 2 oder größer ist, sind die R31s gleich oder verschieden voneinander;
Ar3 ist eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen;
Ar4 ist eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen; und
R32 und R33 sind gleich oder verschieden und jeweils unabhängig eine substituierte oder unsubstituierte Silylgruppe; eine substituierte oder unsubstituierte Arylgruppe mit 6 bis 60 Kohlenstoffatomen; oder eine substituierte oder unsubstituierte Heteroarylgruppe mit 2 bis 60 Kohlenstoffatomen.

## Revendications

1. Composé hétérocyclique représenté par l'une quelconque des formules chimiques suivantes 1-2 à 1-4 : 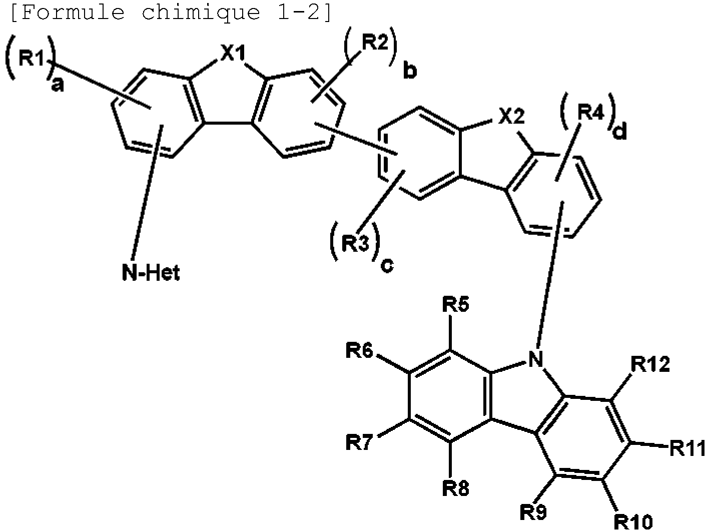 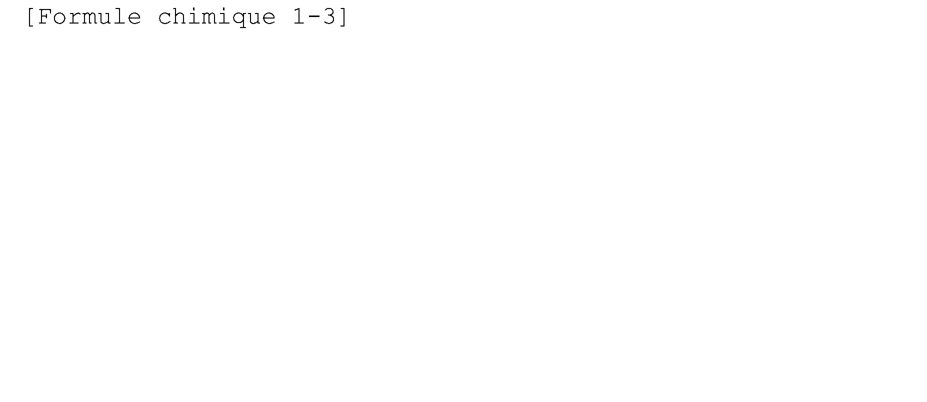 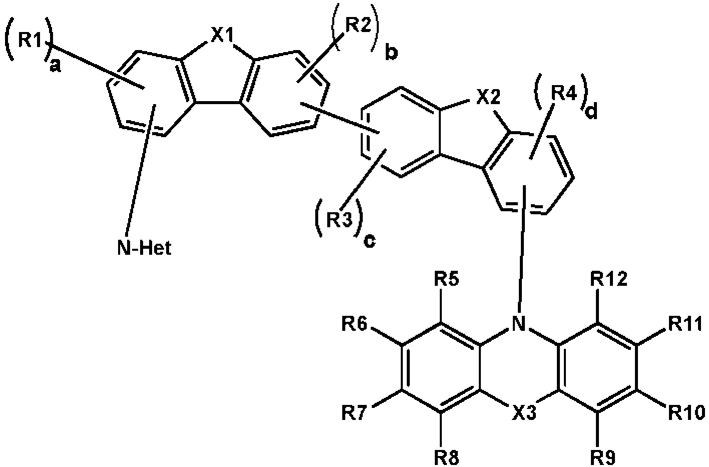 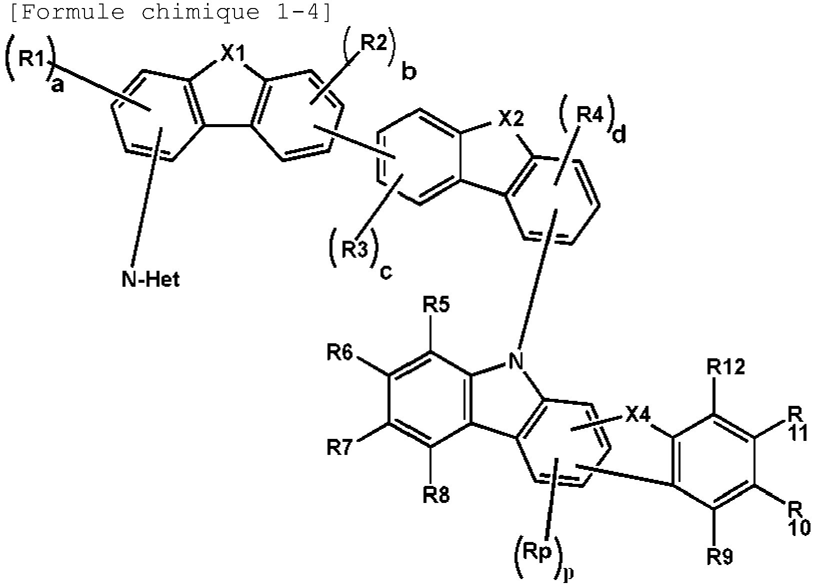 dans les formules chimiques 1-2 à 1-4,
X1 et X2 sont identiques ou différents l'un de l'autre, et chacun indépendamment O ; ou S ;
R1 à R4 sont identiques ou différents les uns des autres, et chacun indépendamment hydrogène ; deutérium ; un groupe aryle substitué ou non substitué ayant 6 à 60 atomes de carbone ; ou un groupe hétéroaryle substitué ou non substitué ayant 6 à 60 atomes de carbone, a à d sont chacun un entier de 0 à 3, et lorsque a à d sont chacun 2 ou plus, les substituants dans les parenthèses sont identiques les uns aux autres ;
N-Het est un groupe représenté par l'une quelconque des formules chimiques 2 à 4 suivantes ;
X3 et X4 sont chacun indépendamment O ; S ; CRaRb ; ou NRC, Ra et Rb sont identiques ou différents l'un de l'autre et chacun indépendamment un groupe alkyle substitué ou non substitué ayant 1 à 10 atomes de carbone ; ou un groupe aryle substitué ou non substitué ayant 6 à 20 atomes de carbone, et Rc est un groupe aryle substitué ou non substitué ayant 6 à 20 atomes de carbone ;
R5 à R12 et Rp sont identiques ou différents l'un de l'autre, et chacun indépendamment hydrogène ; deutérium ; un groupe halogène ; un groupe cyano ; un groupe aryle substitué ou non substitué ayant 6 à 20 atomes de carbone ; ou un groupe hétéroaryle substitué ou non substitué ayant 2 à 20 atomes de carbone ;
p est un entier de 0 à 2, et lorsque p est 2, les substituants entre parenthèses sont identiques les uns aux autres ; 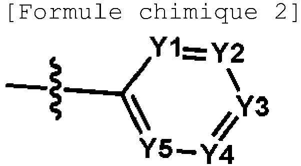 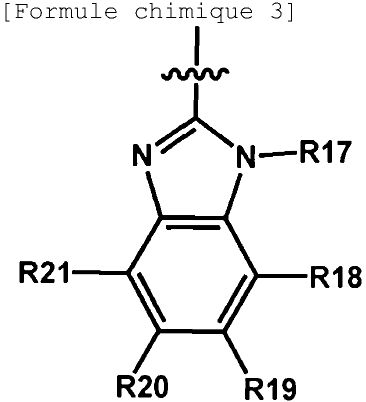 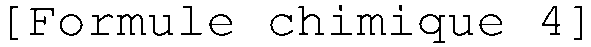 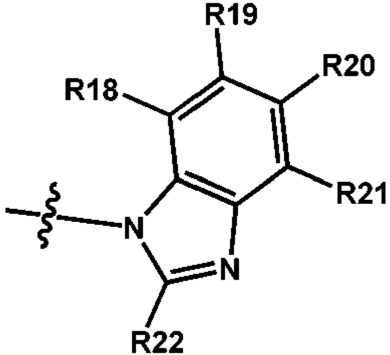 dans les formules chimiques 2 à 4,
Y1 est CR12' ou N, Y2 est CR13' ou N, Y3 est CR14' ou N, Y4 est CR15' ou N, Y5 est CR16' ou N, et au moins un parmi Y1 à Y5 est N ; et
R12' à R16' et R17 à R22 sont identiques ou différents les uns des autres, et chacun choisi indépendamment dans le groupe constitué d'hydrogène ; de deutérium ; d'halogène ; d'un groupe cyano ; d'un groupe alkyle substitué ou non substitué ayant 1 à 60 atomes de carbone ; d'un groupe alcényle substitué ou non substitué ayant 2 à 60 atomes de carbone ; d'un groupe alcynyle substitué ou non substitué ayant 2 à 60 atomes de carbone ; d'un groupe alcoxy substitué ou non substitué ayant 1 à 20 atomes de carbone ; d'un groupe cycloalkyle substitué ou non substitué ayant 3 à 60 atomes de carbone ; d'un groupe hétérocycloalkyle substitué ou non substitué ayant 2 à 60 atomes de carbone ; d'un groupe aryle substitué ou non substitué ayant 6 à 60 atomes de carbone ; d'un groupe hétéroaryle substitué ou non substitué ayant 2 à 60 atomes de carbone ; d'un groupe oxyde de phosphine substitué ou non substitué ; et d'un groupe amine substitué ou non substitué, ou de deux groupes, ou plus, adjacents, liés l'un à l'autre pour former un cycle ou hétérocycle hydrocarboné aliphatique ou aromatique substitué ou non substitué ; 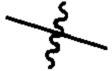
est un site lié à l'une quelconque des formules chimiques suivantes 1-2 à 1-4 ; et
la position n°3 de 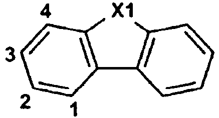 est substituée par N-Het et la position n°3 de 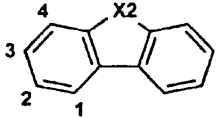 est substituée par l'un quelconque de 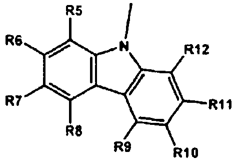 de formule chimique 1-2, 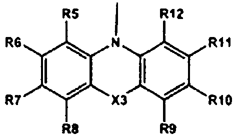 de formule chimique 1-3 et 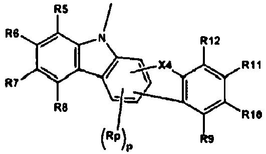 de formule chimique 1-4.

2. Composé hétérocyclique selon la revendication 1, dans lequel le composé hétérocyclique représenté par l'une quelconque des formules chimiques suivantes 1-2 à 1-4 a une teneur en deutérium de 0 %, ou supérieure à 10 % et inférieure ou égale à 100 %.

3. Composé hétérocyclique représenté par l'un quelconque des composés suivants :

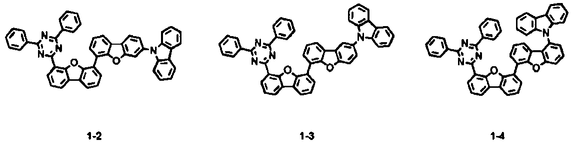

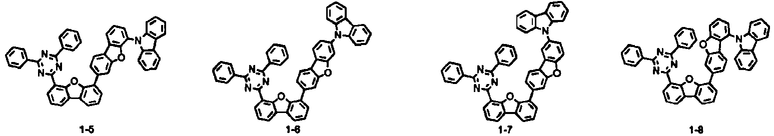

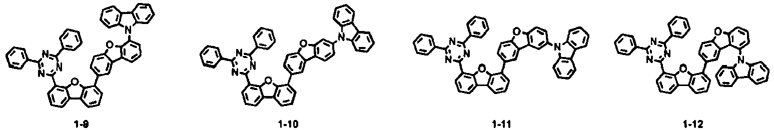

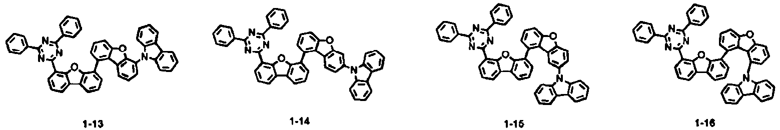

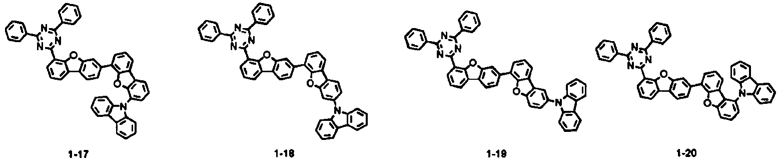

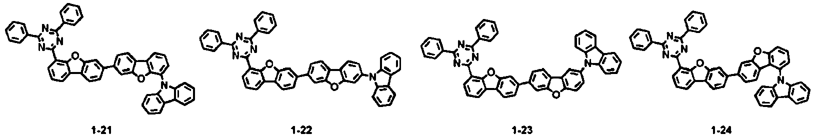

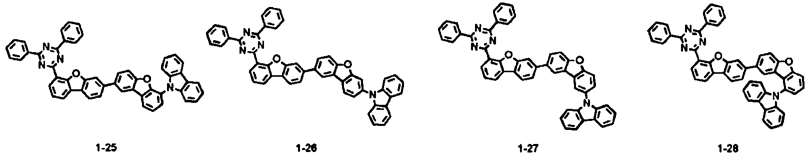

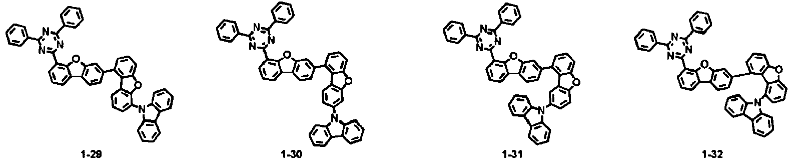

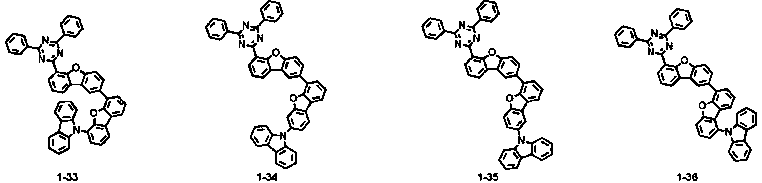

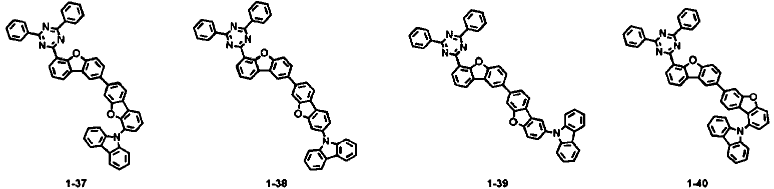

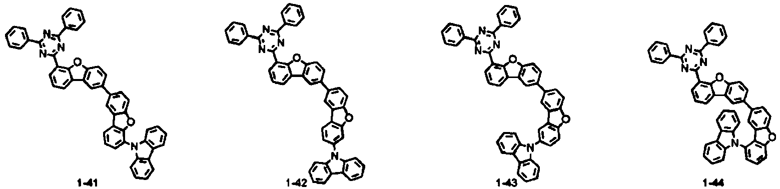

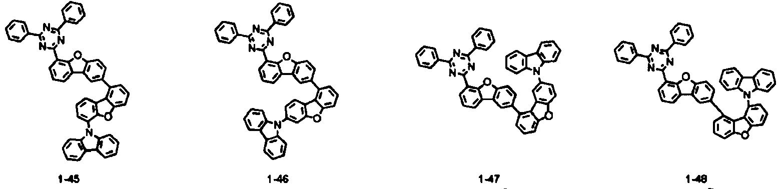

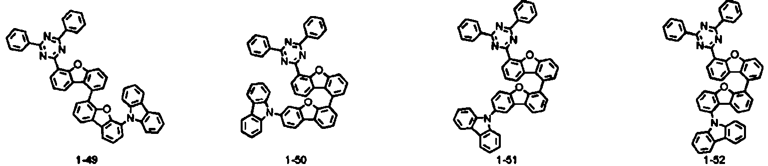

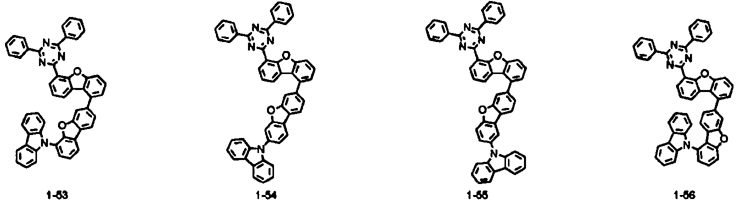

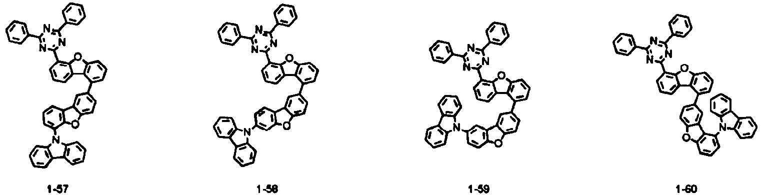

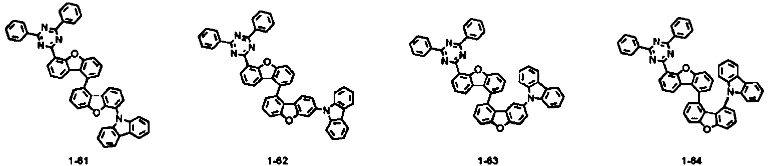

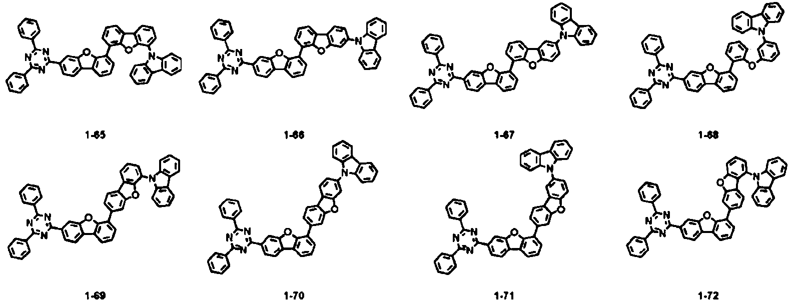

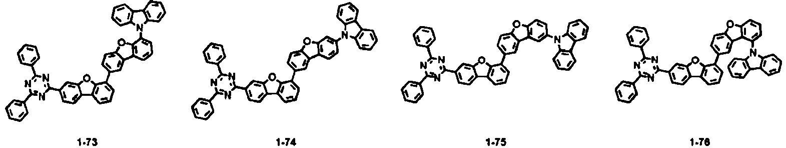

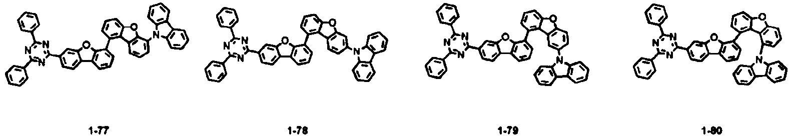

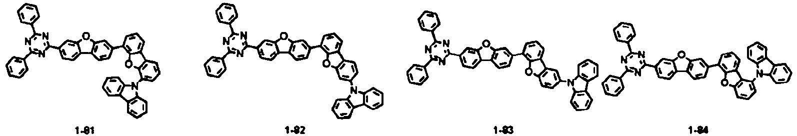

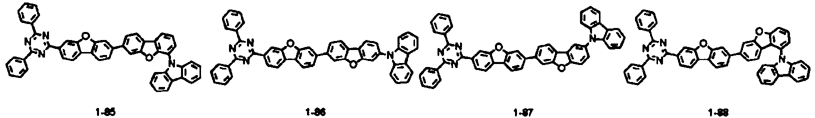

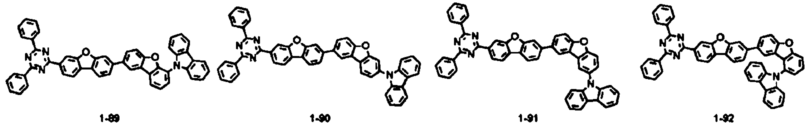

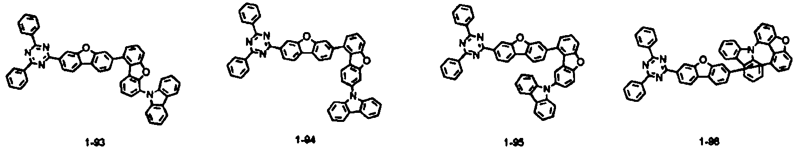

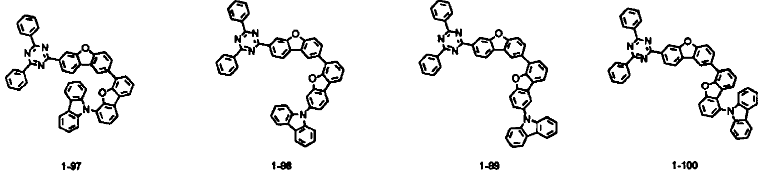

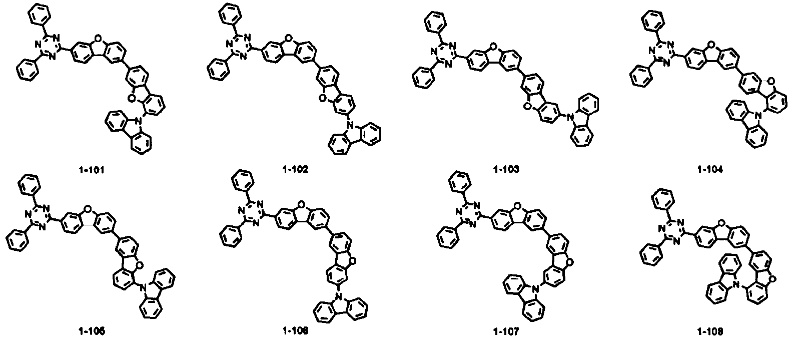

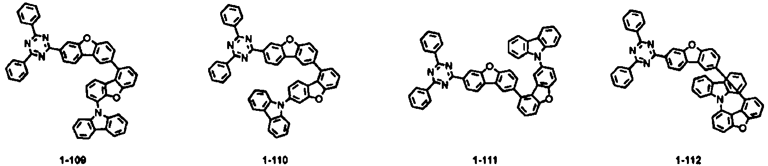

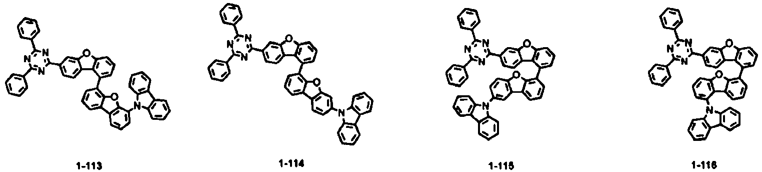

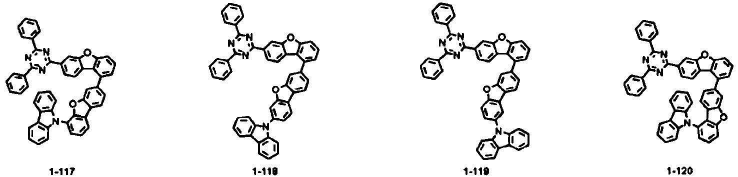

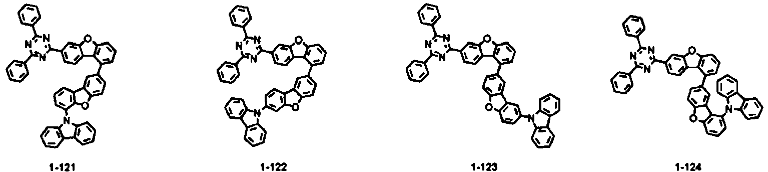

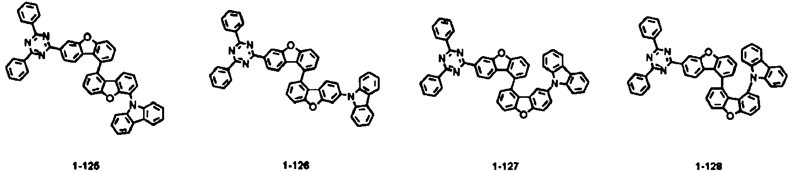

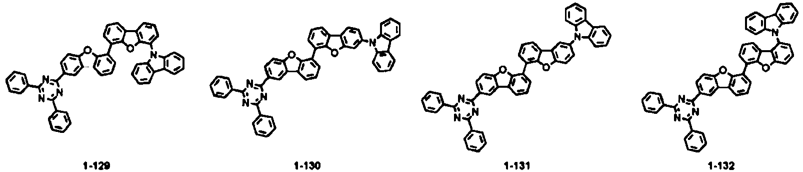

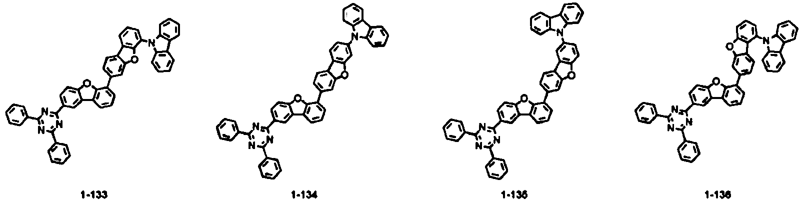

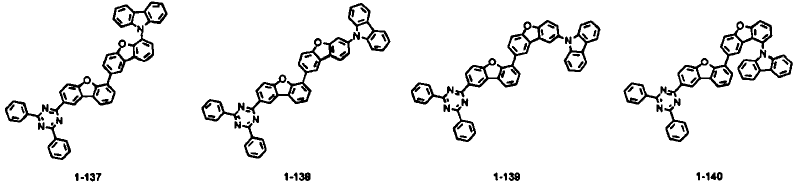

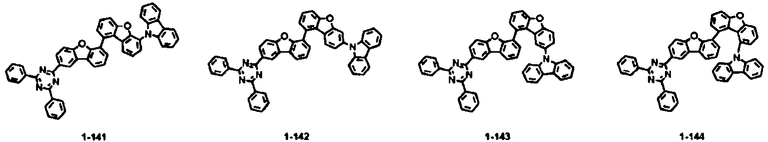

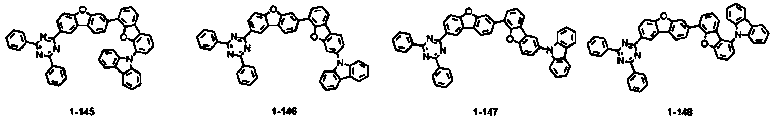

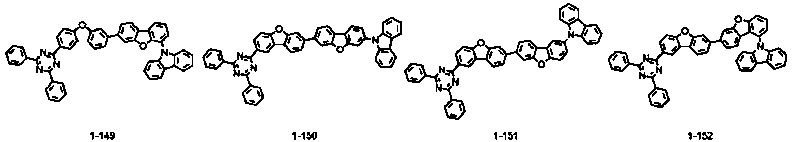

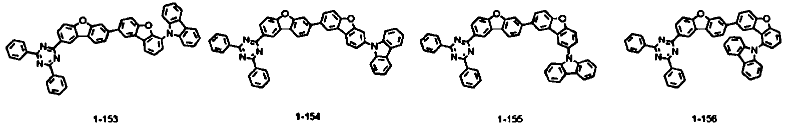

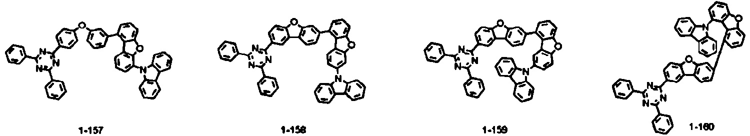

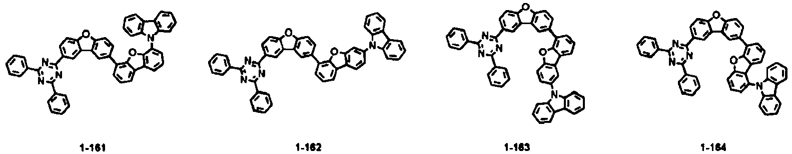

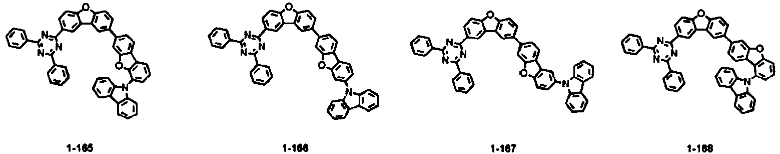

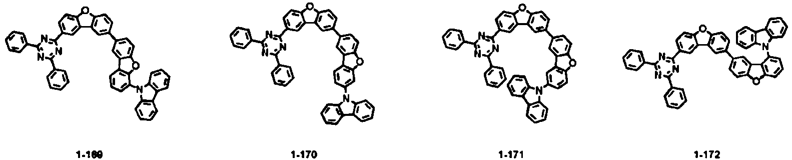

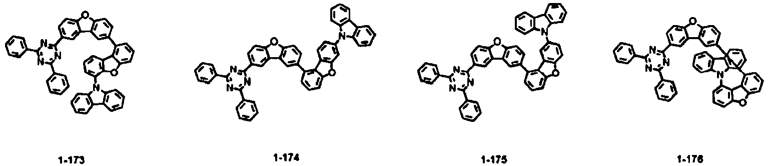

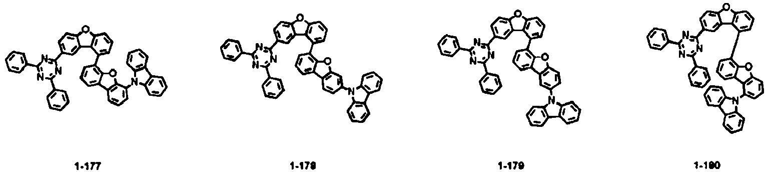

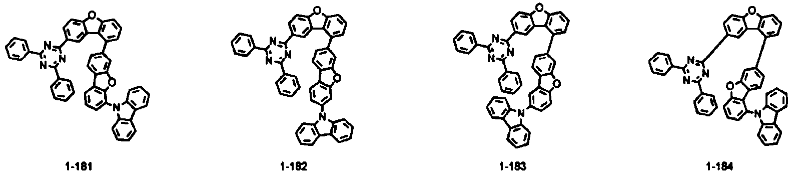

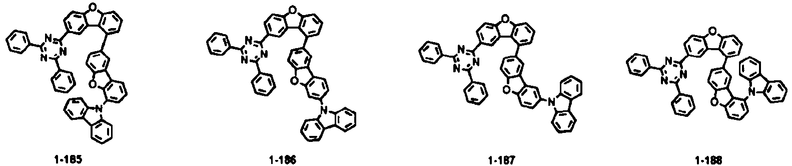

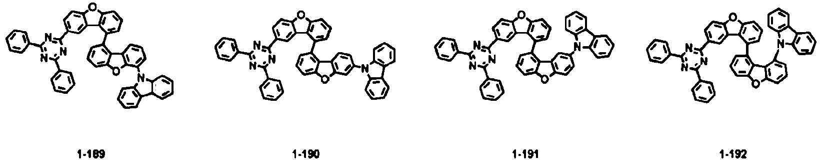

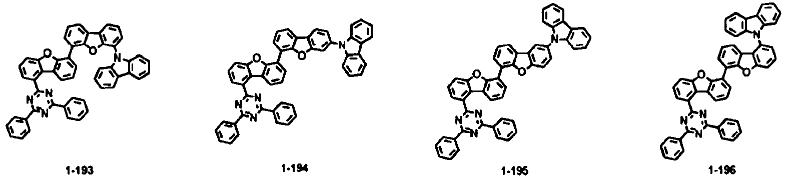

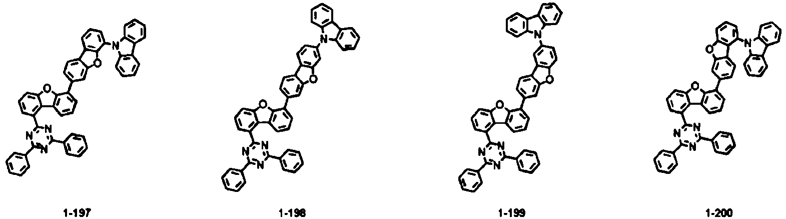

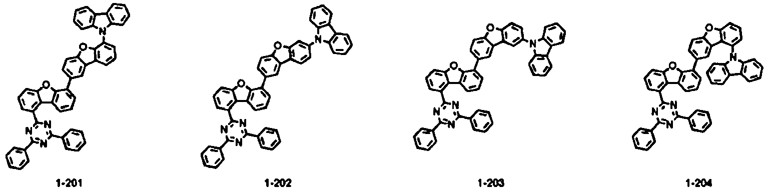

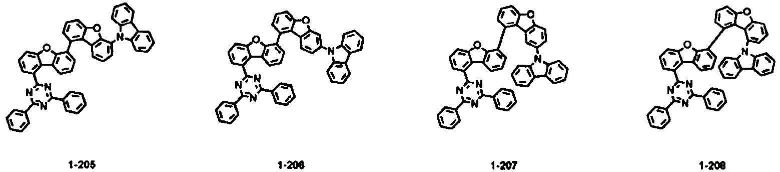

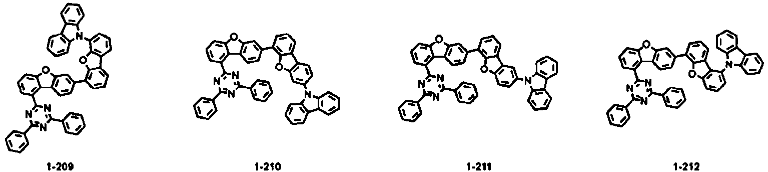

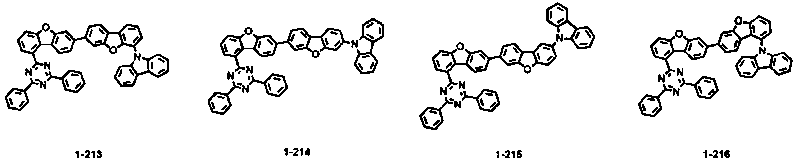

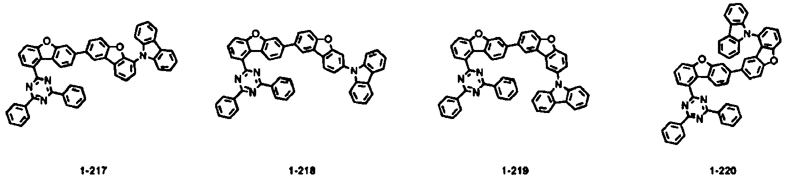

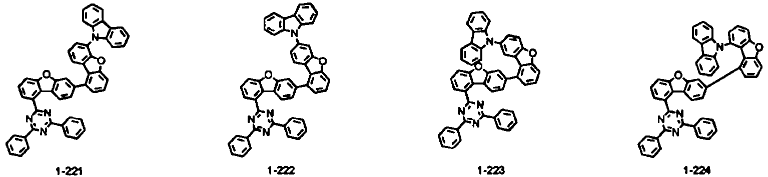

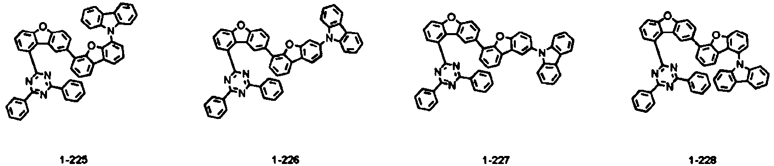

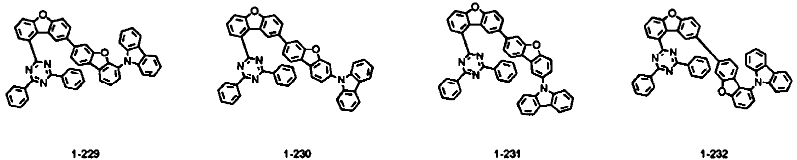

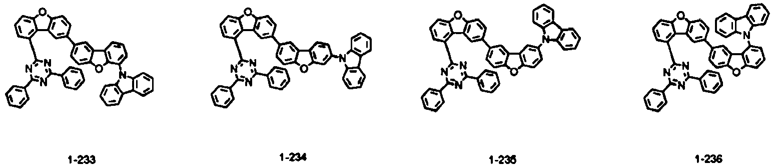

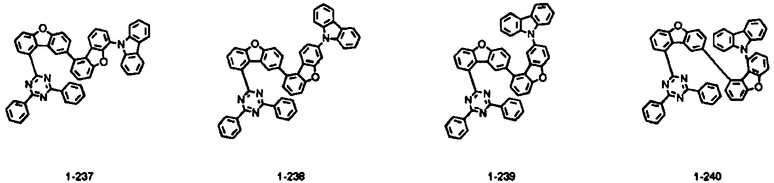

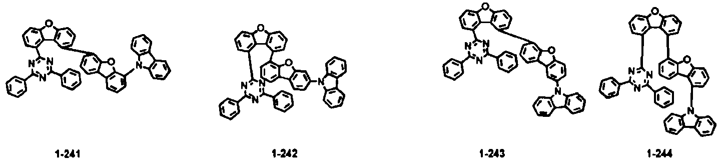

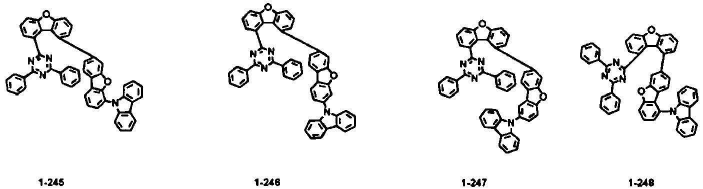

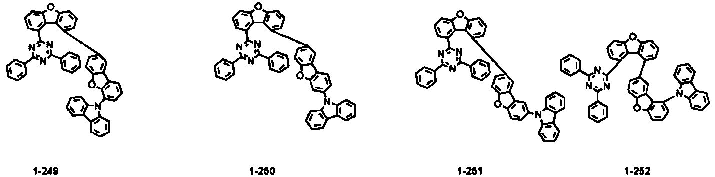

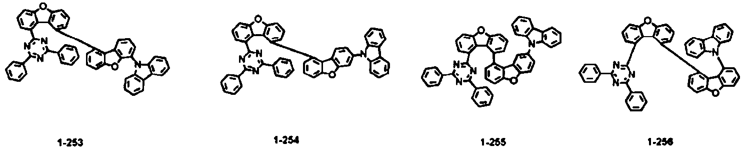

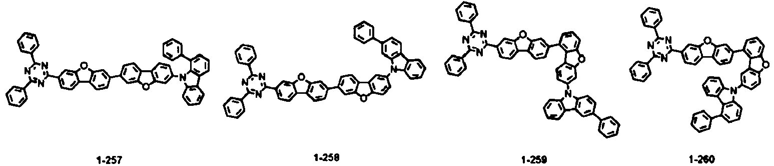

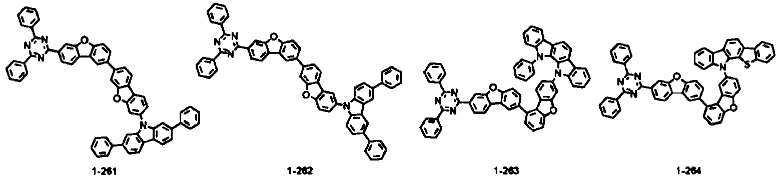

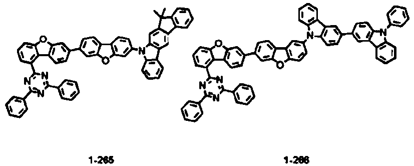

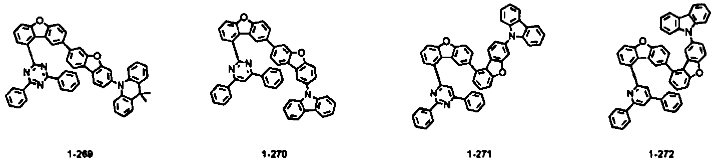

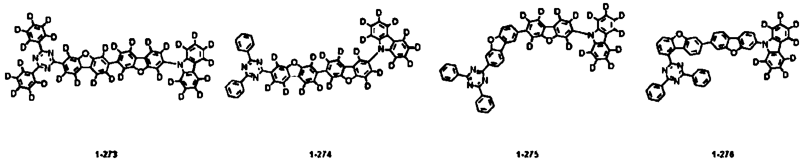

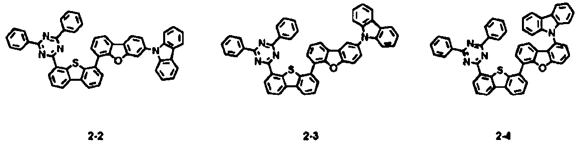

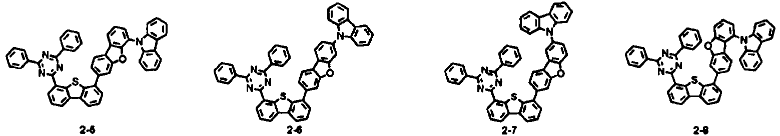

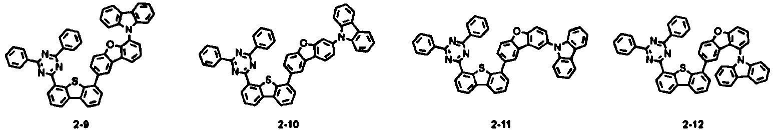

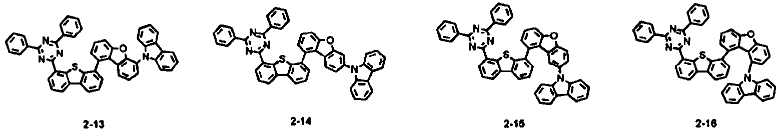

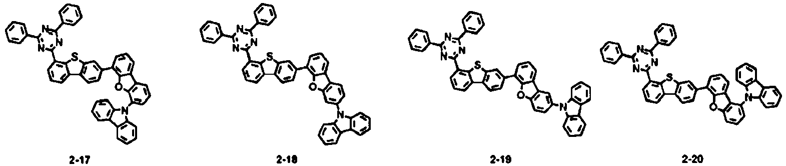

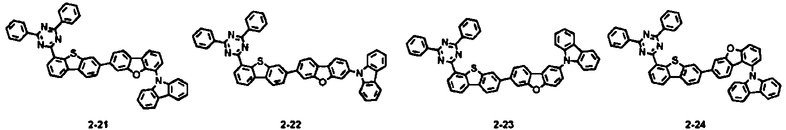

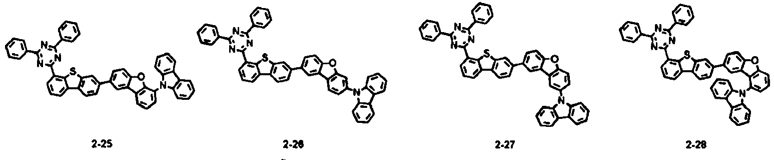

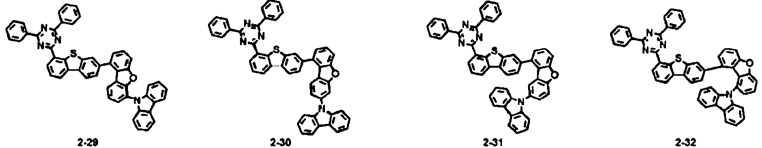

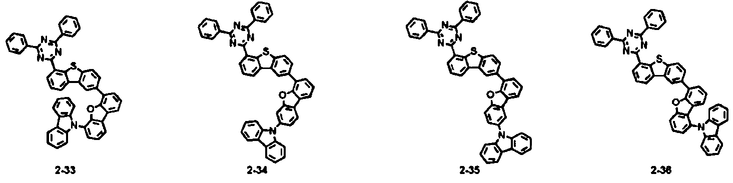

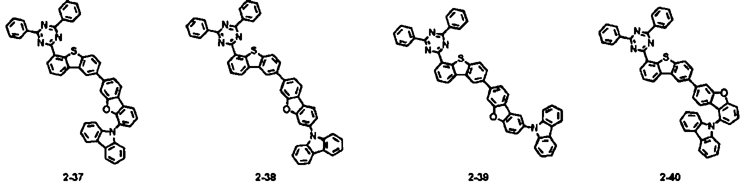

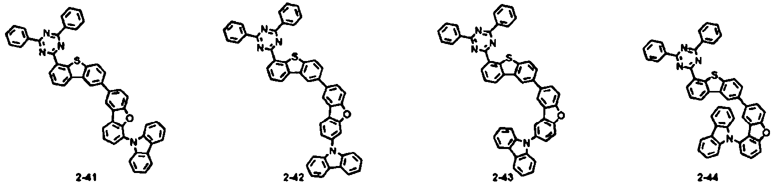

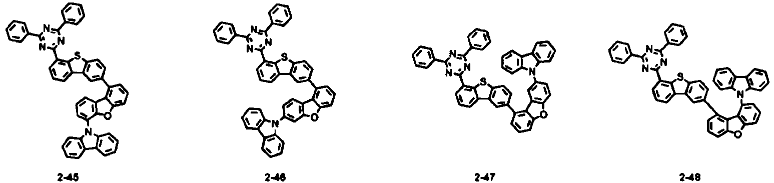

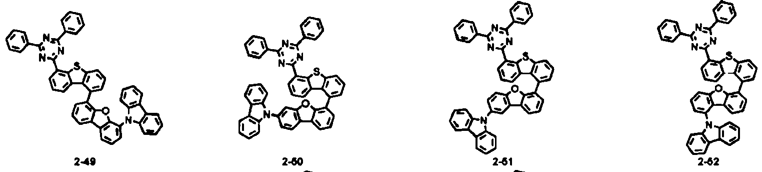

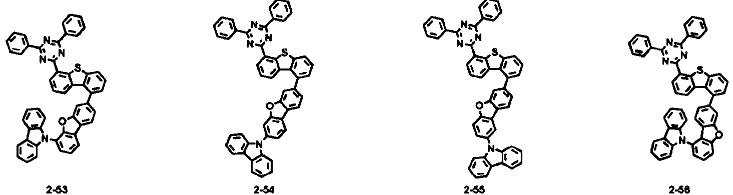

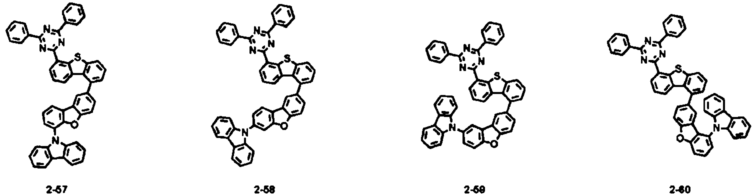

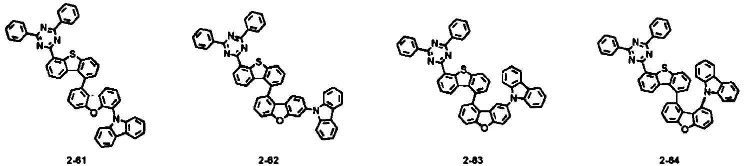

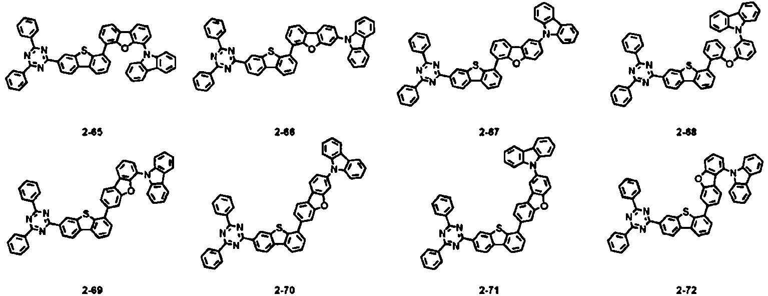

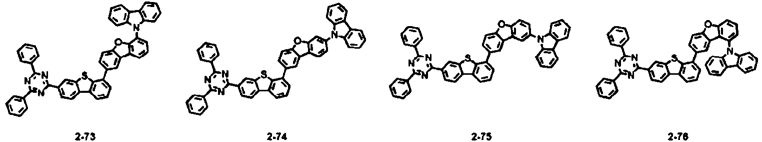

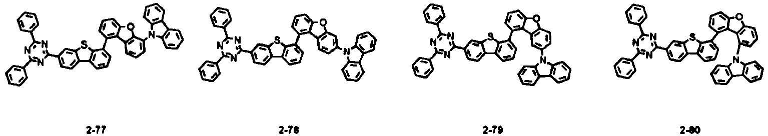

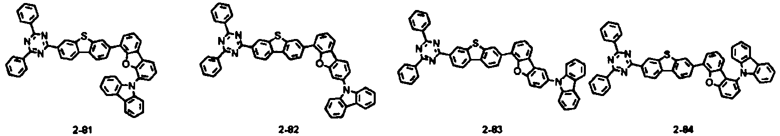

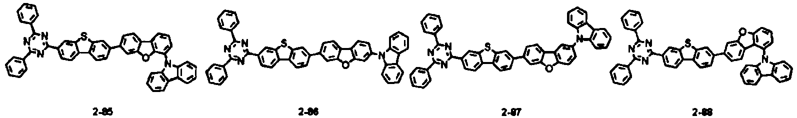

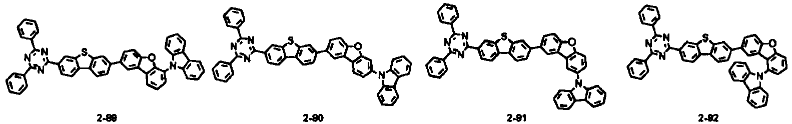

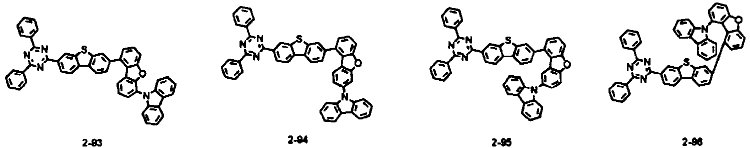

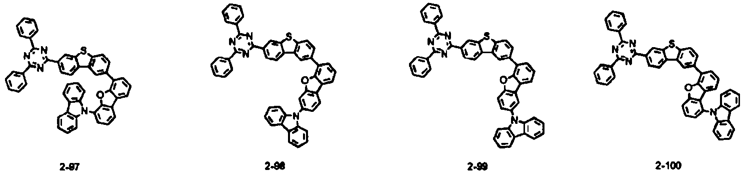

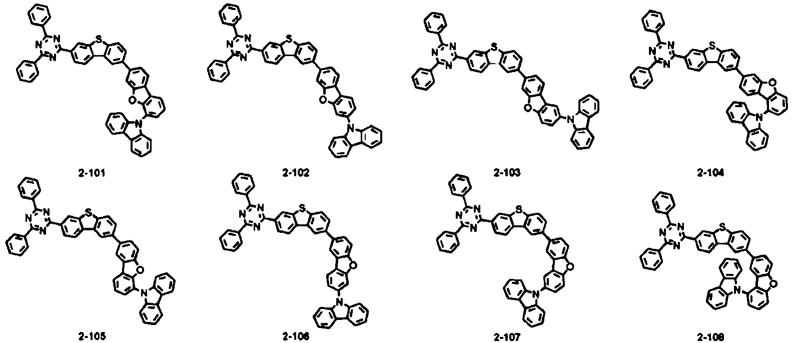

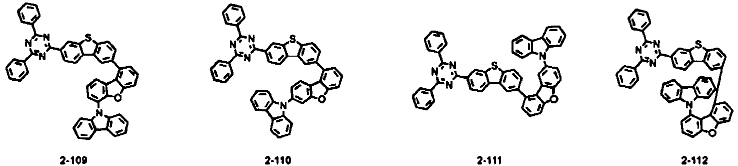

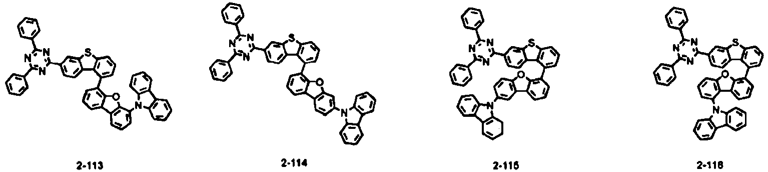

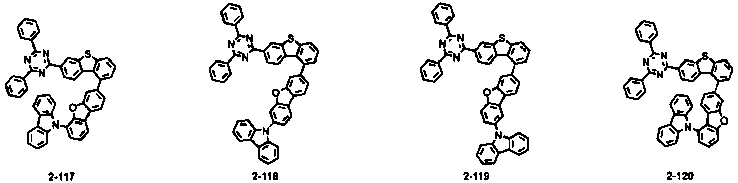

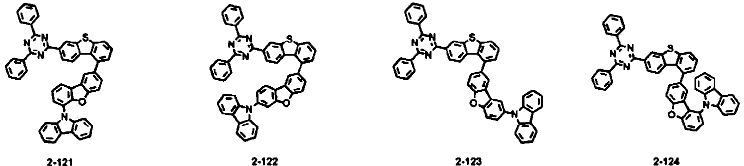

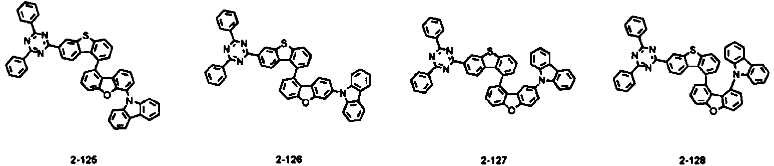

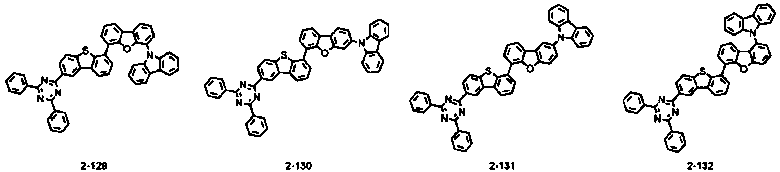

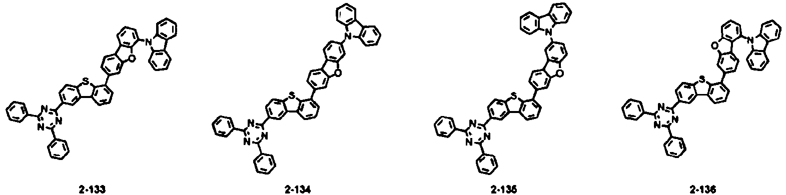

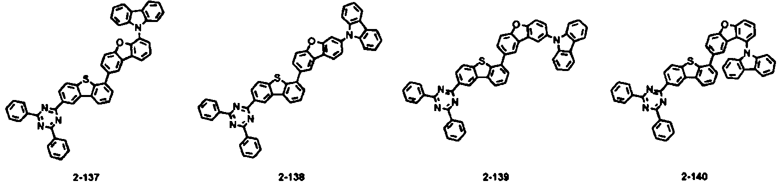

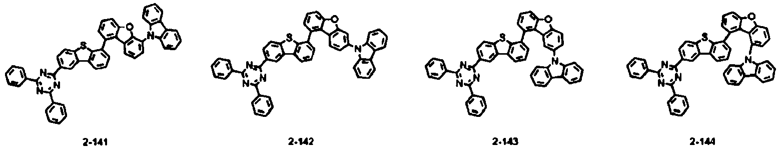

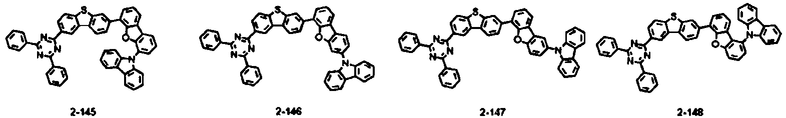

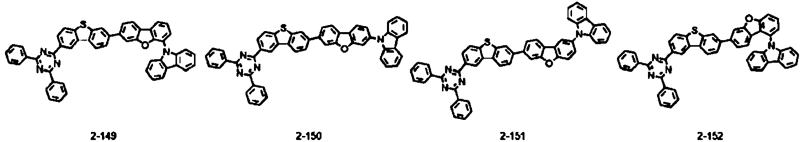

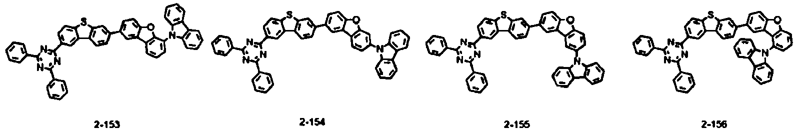

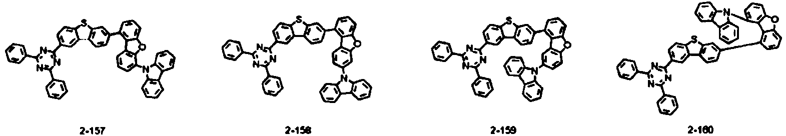

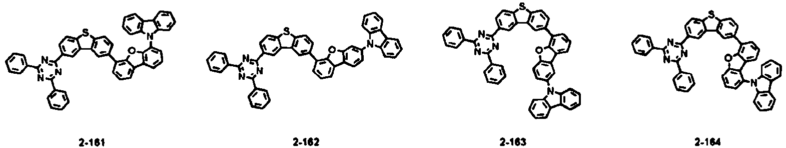

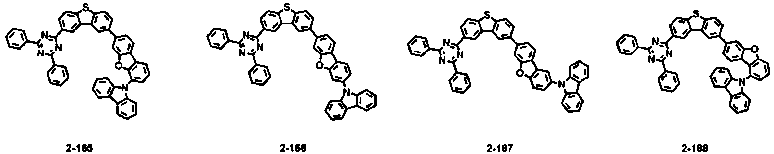

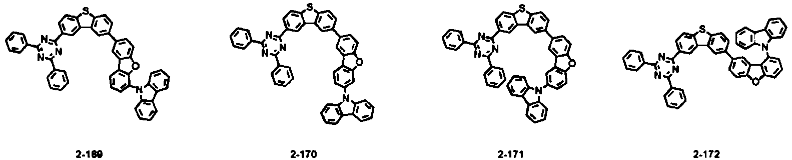

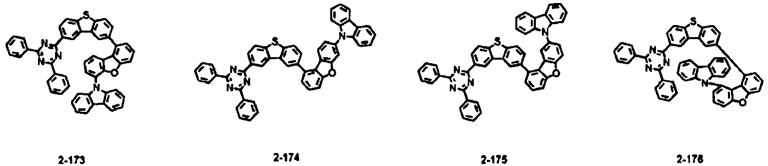

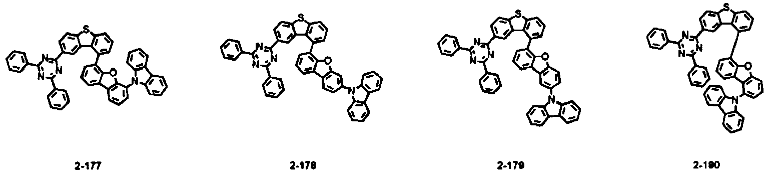

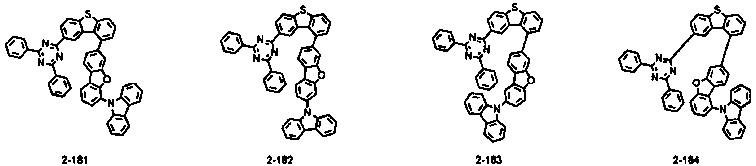

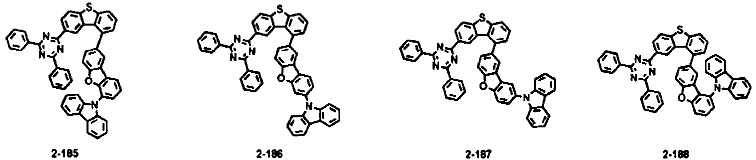

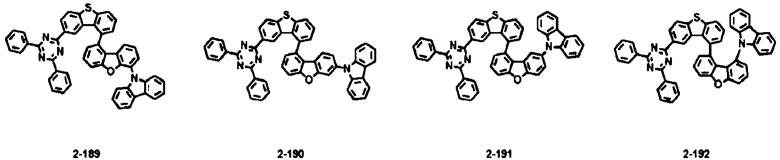

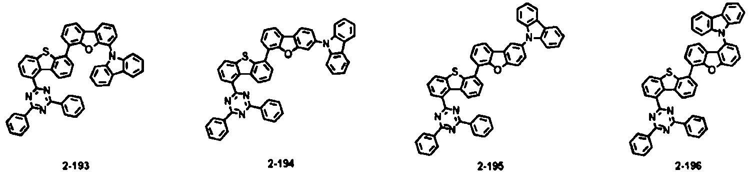

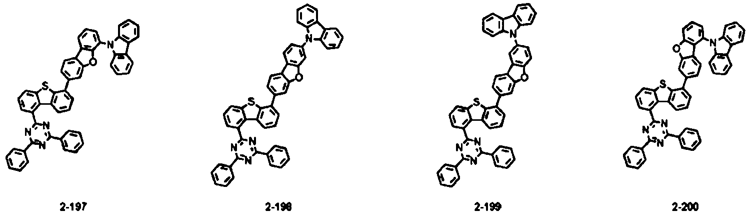

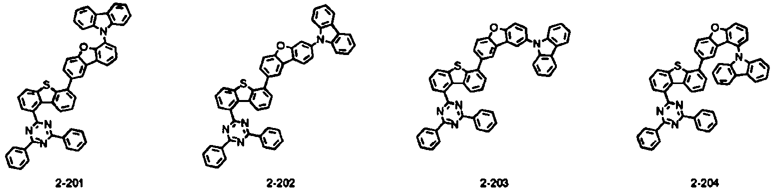

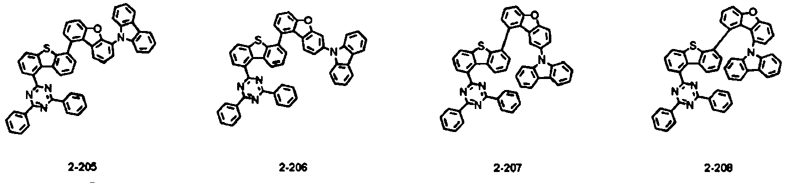

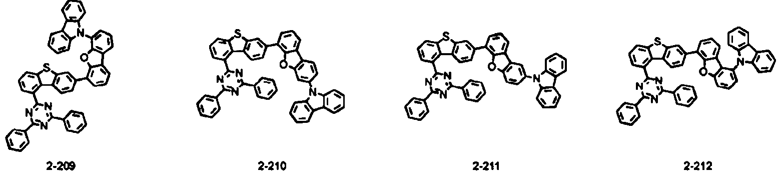

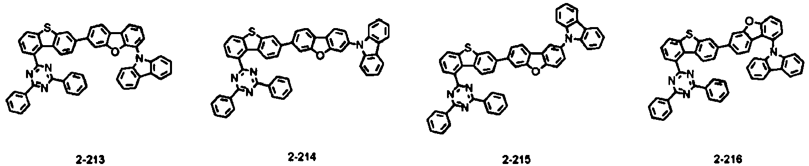

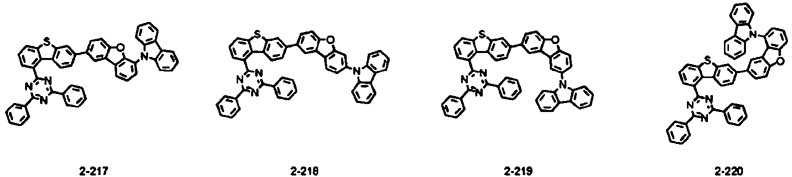

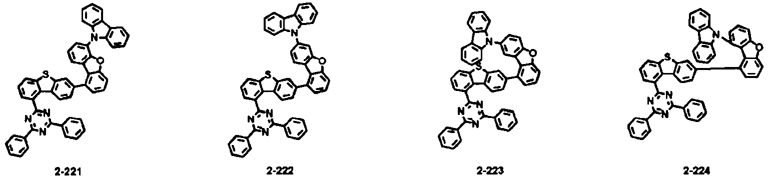

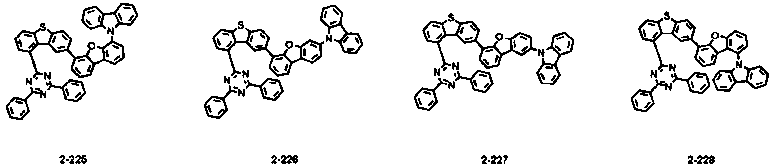

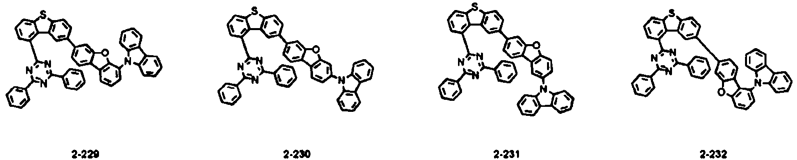

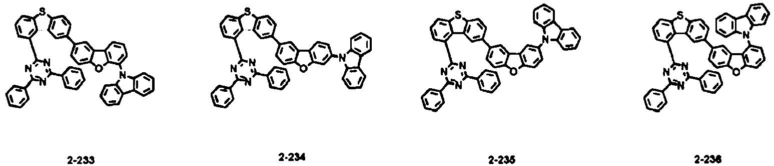

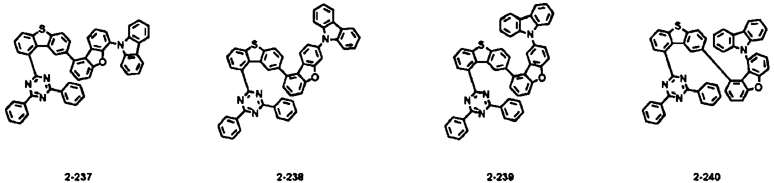

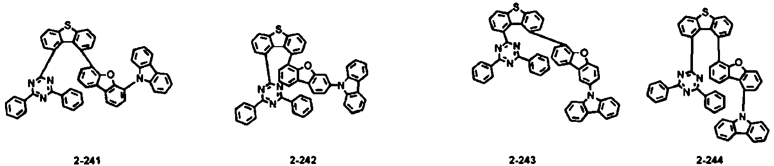

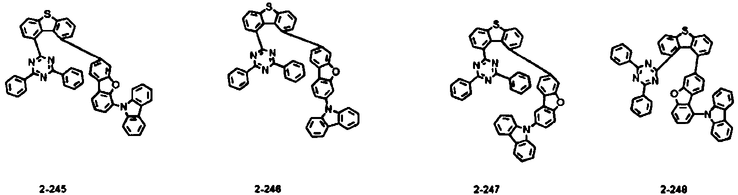

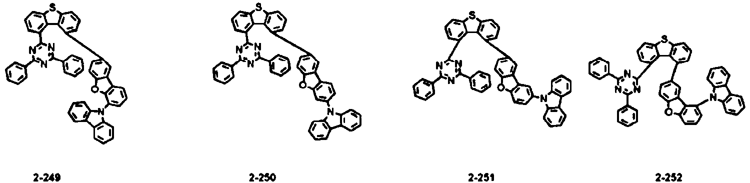

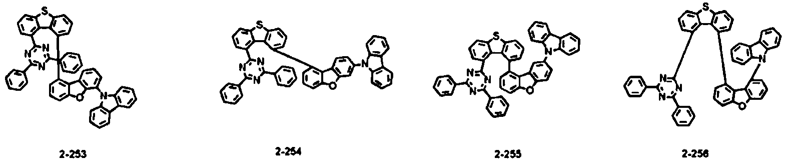

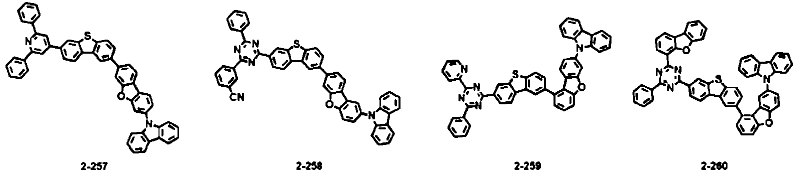

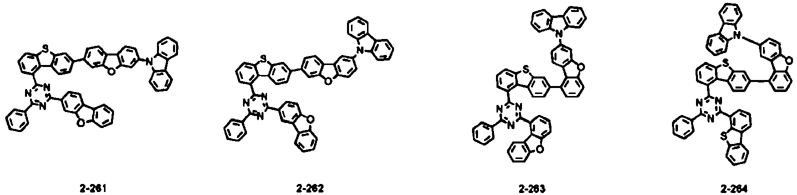

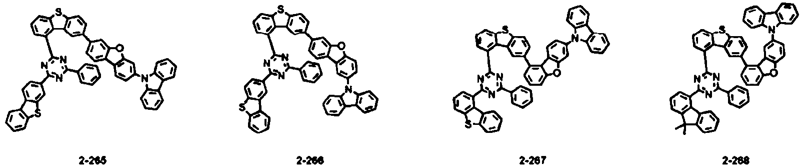

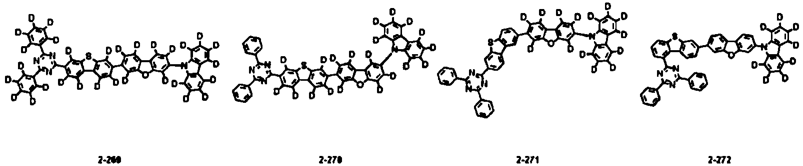

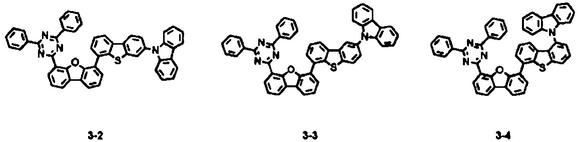

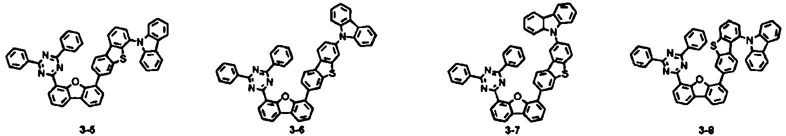

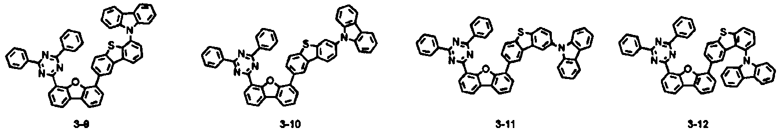

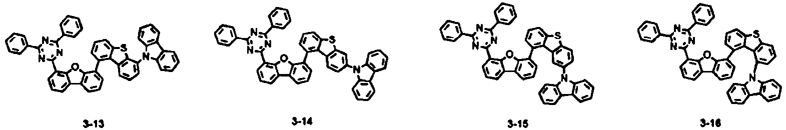

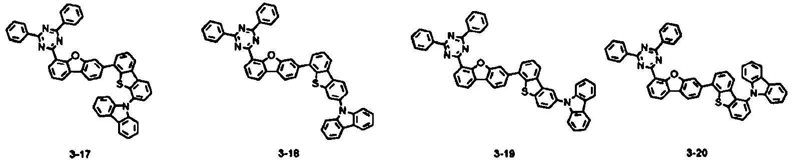

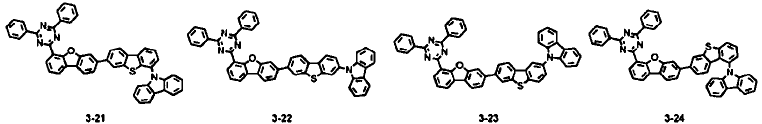

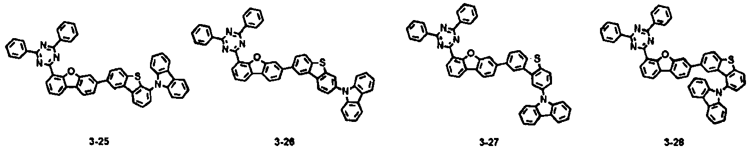

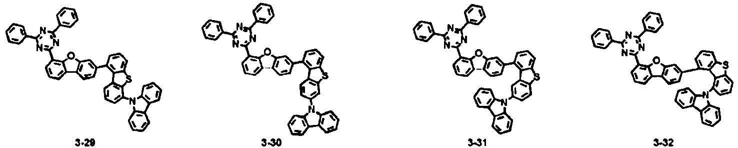

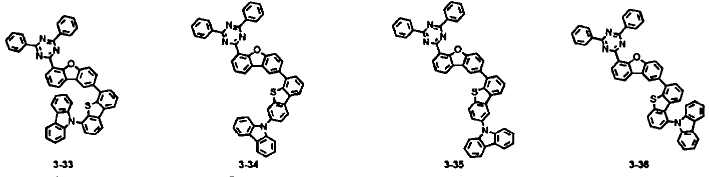

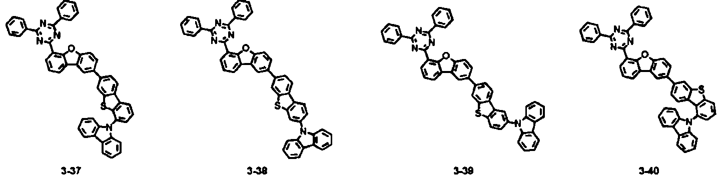

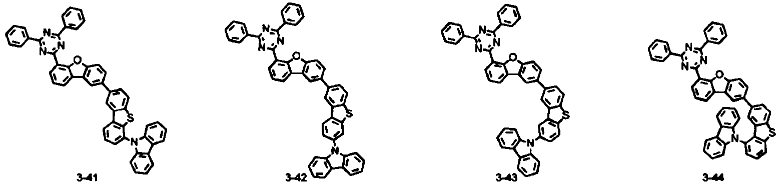

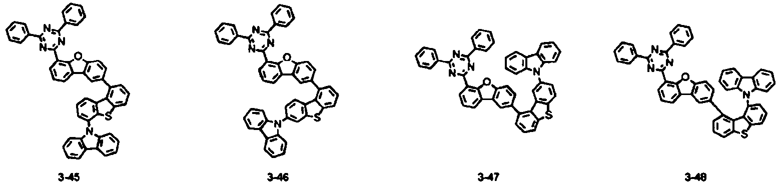

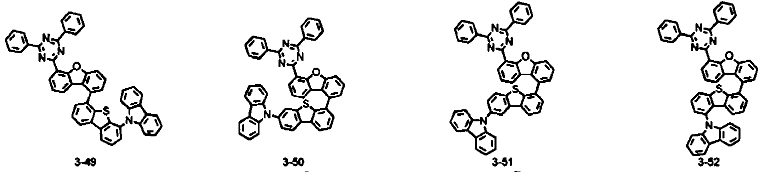

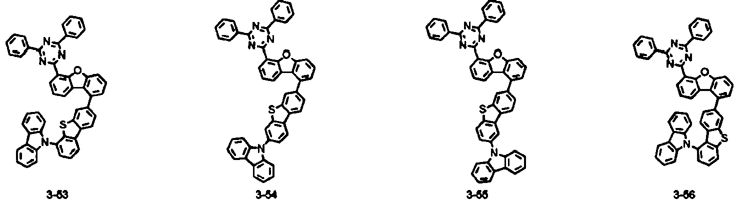

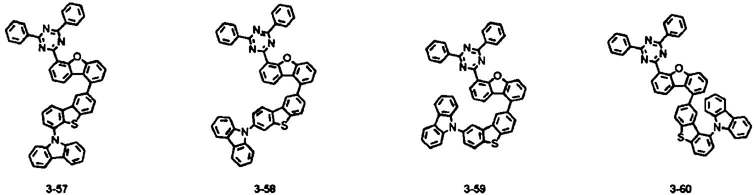

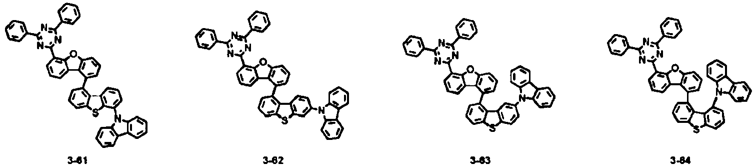

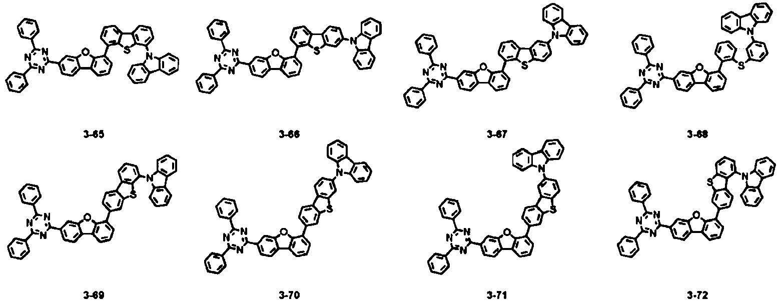

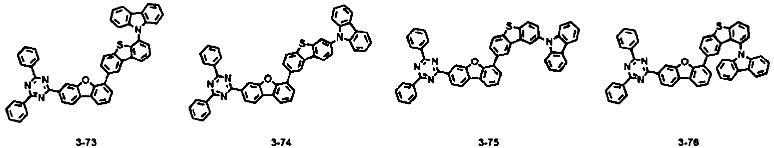

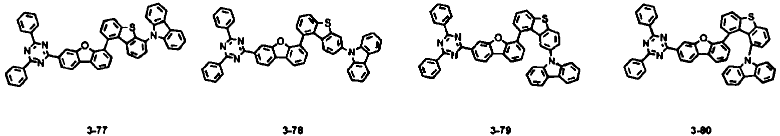

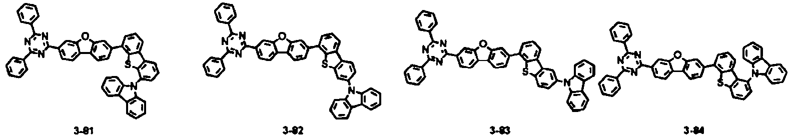

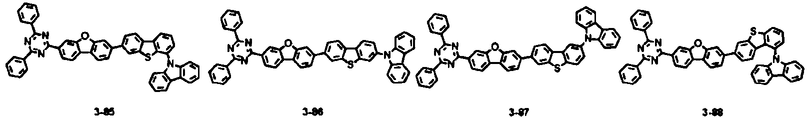

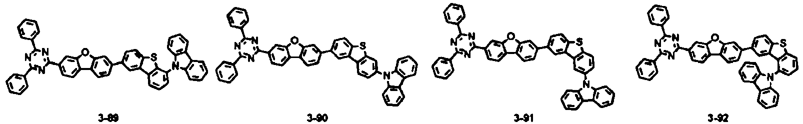

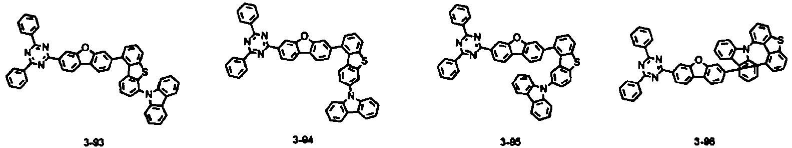

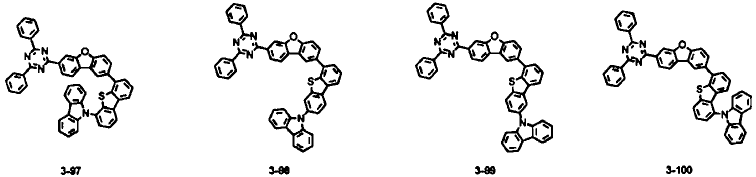

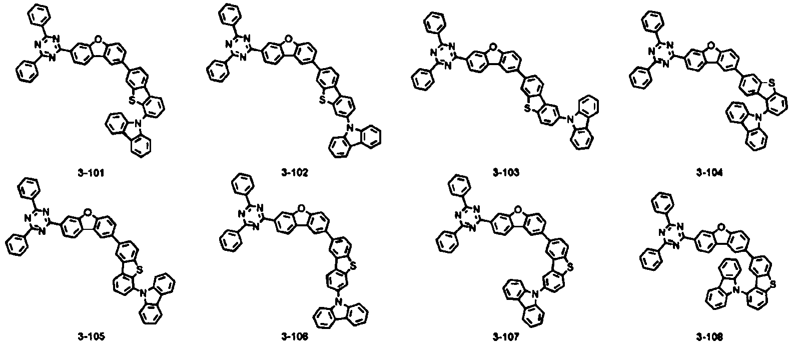

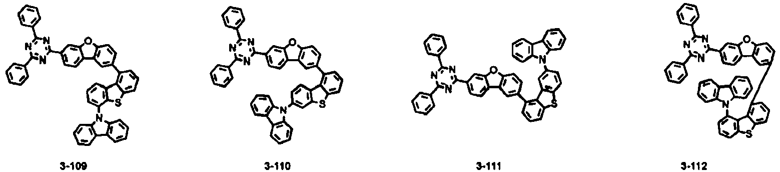

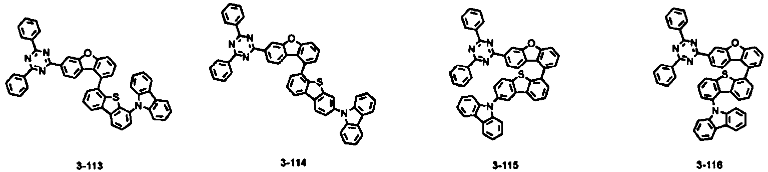

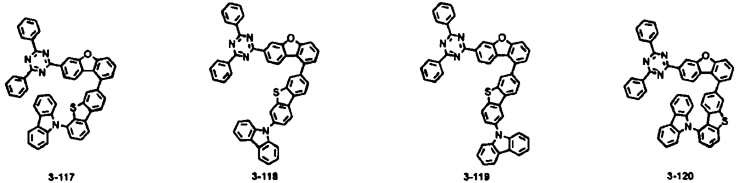

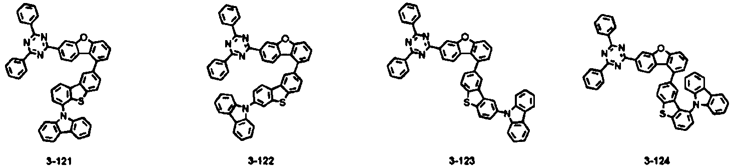

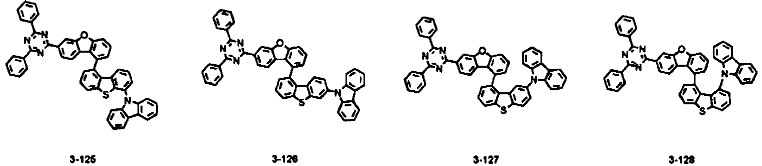

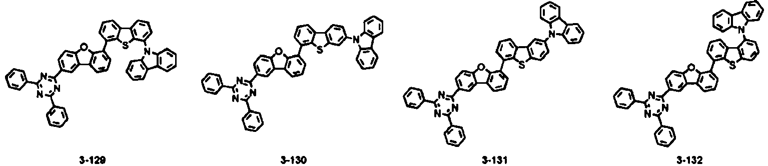

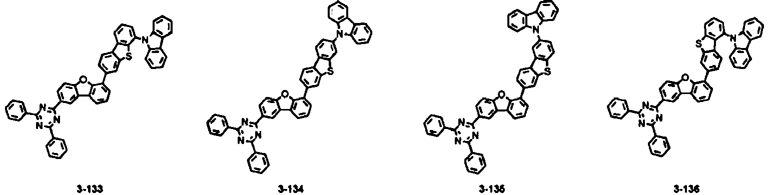

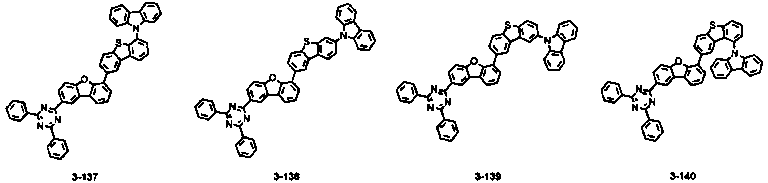

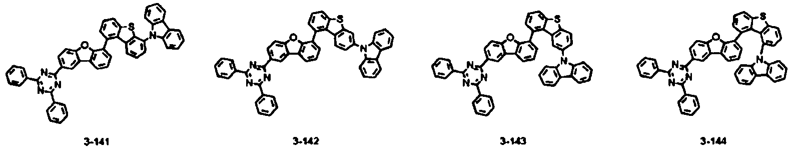

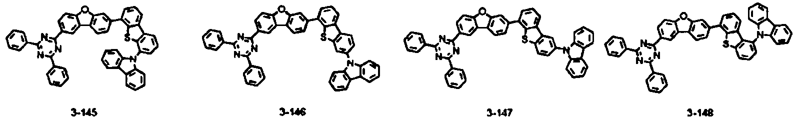

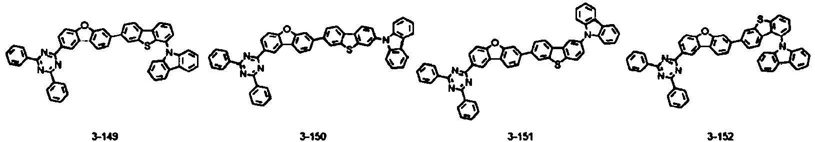

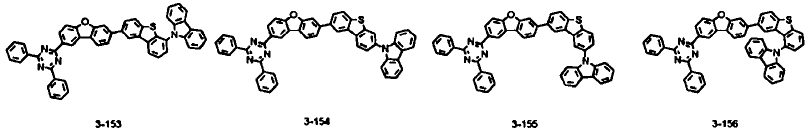

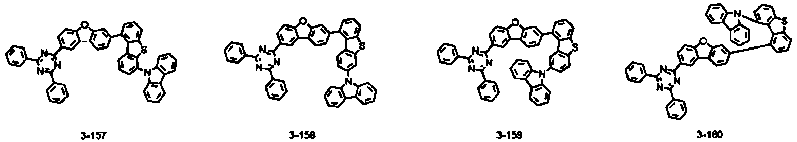

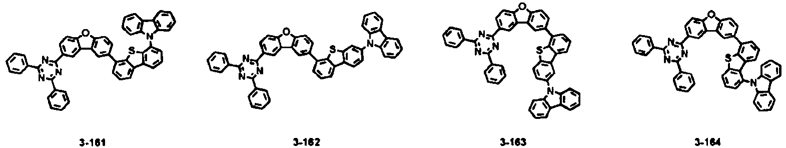

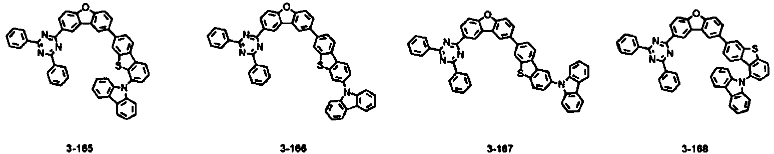

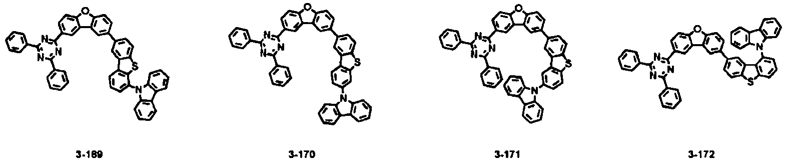

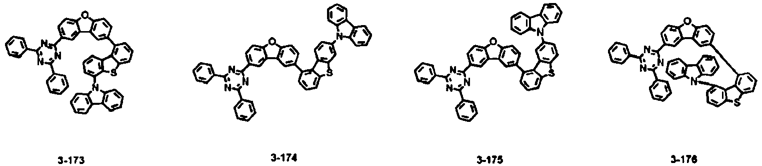

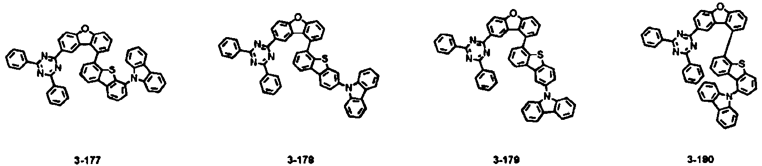

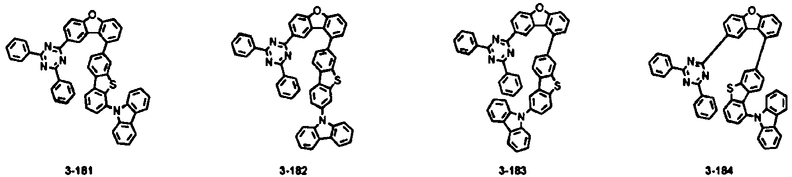

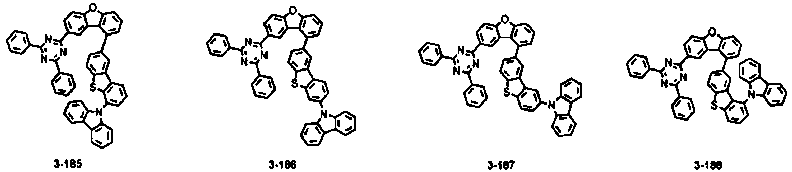

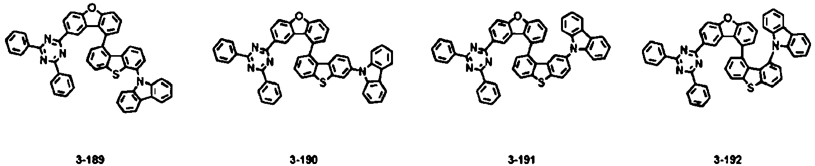

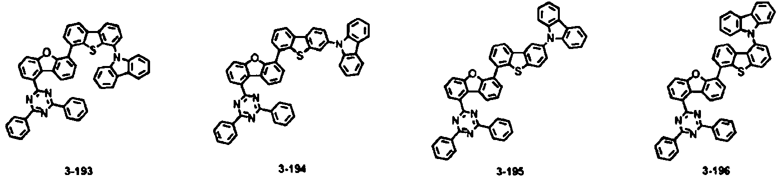

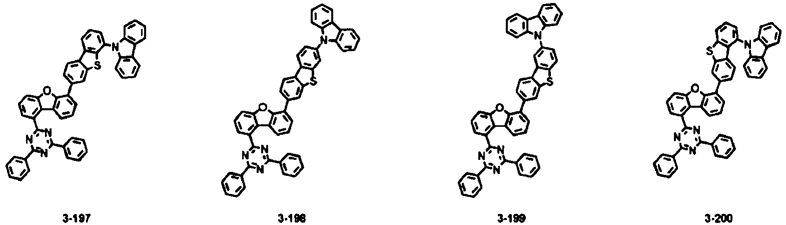

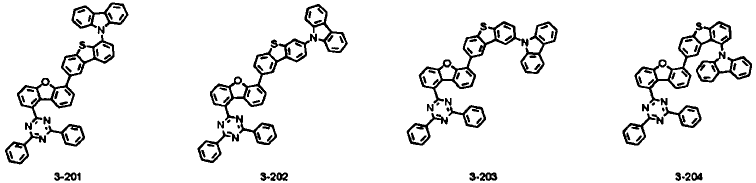

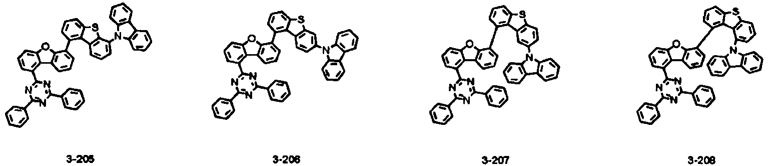

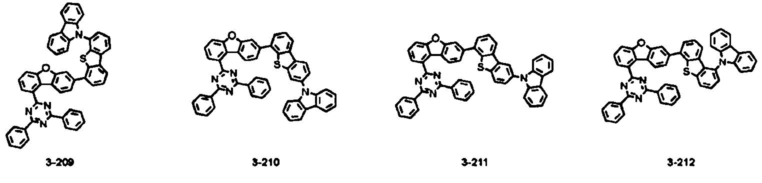

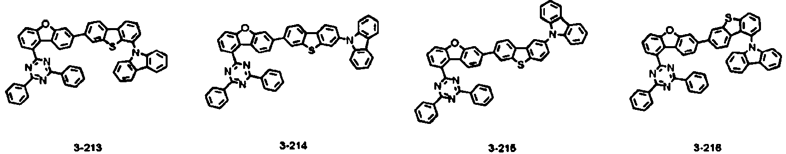

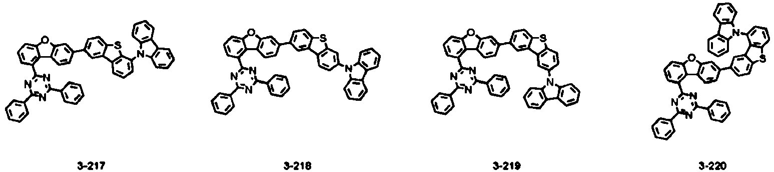

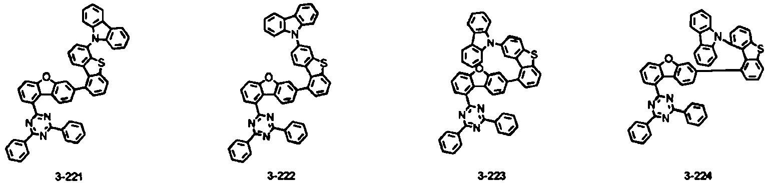

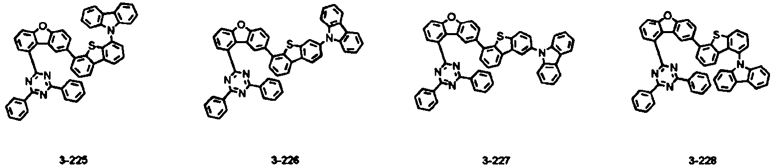

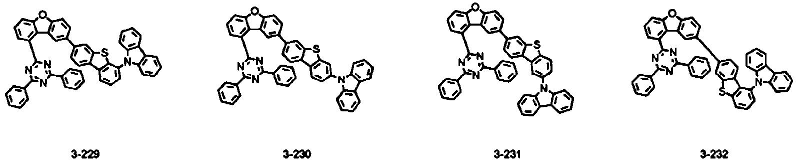

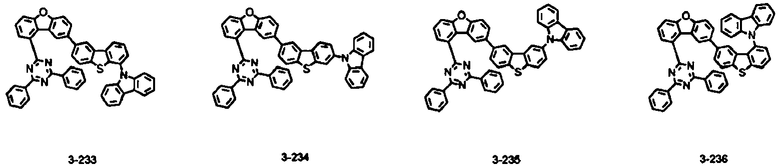

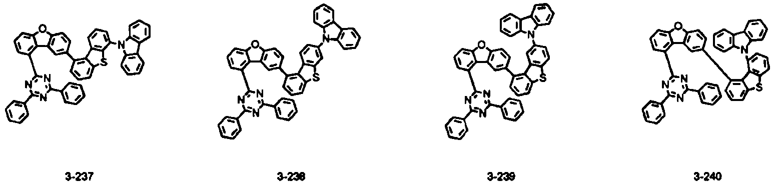

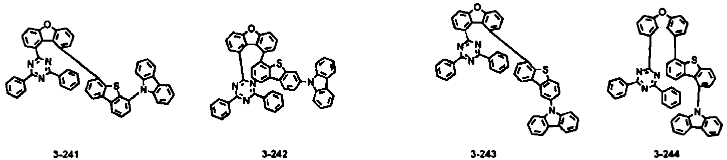

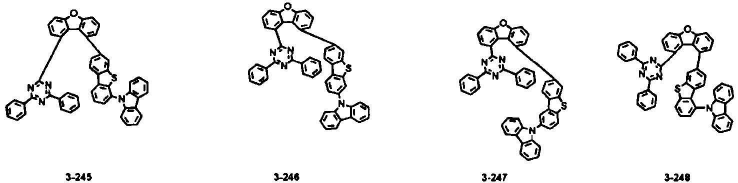

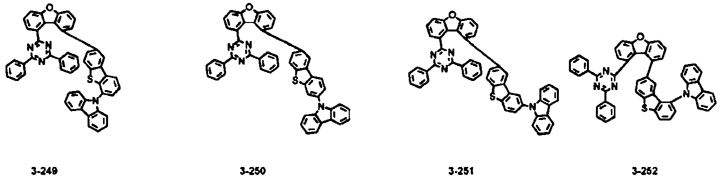

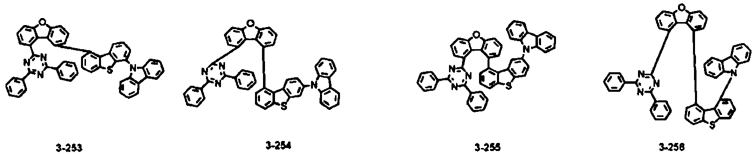

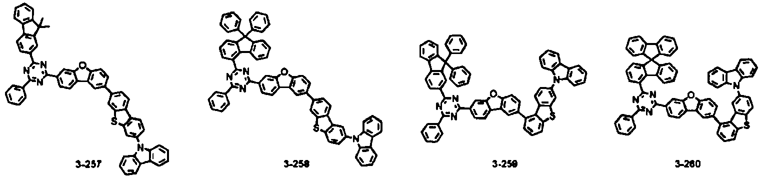

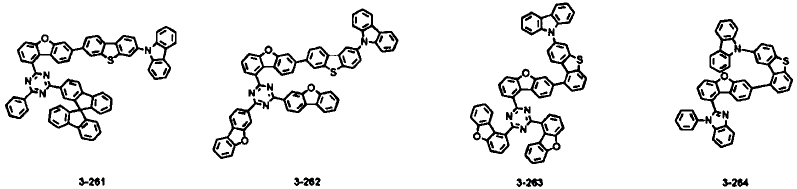

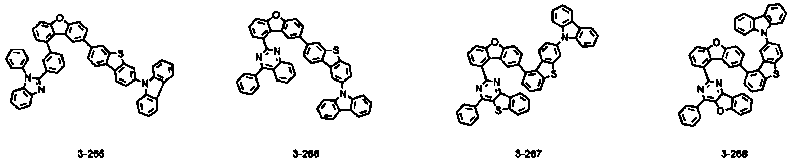

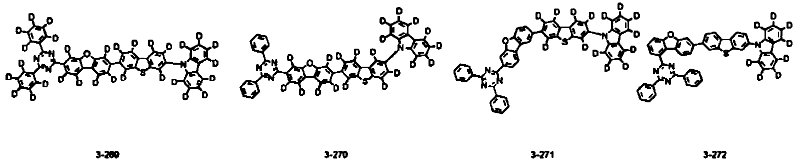

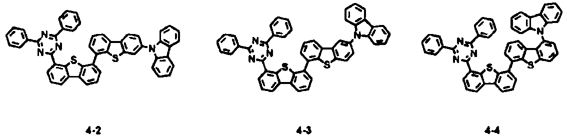

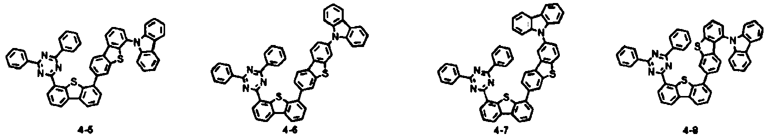

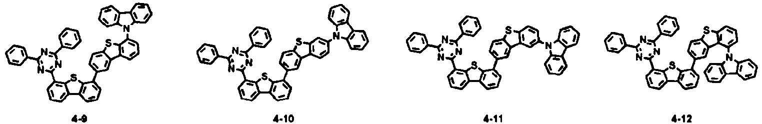

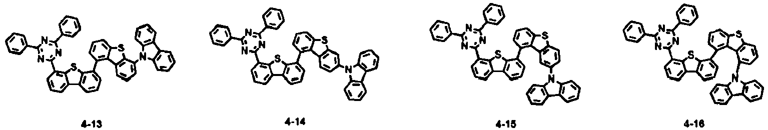

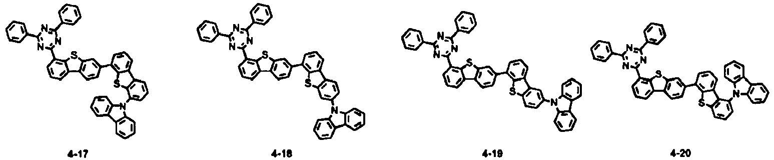

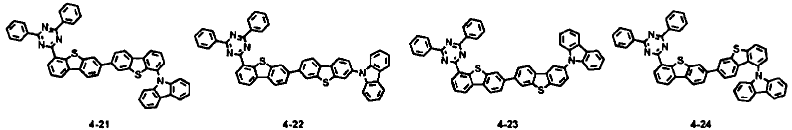

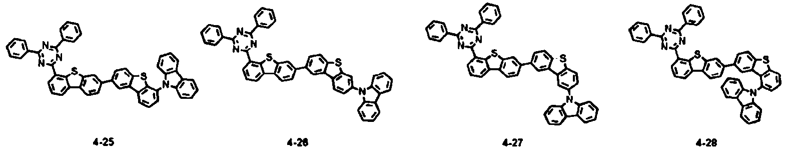

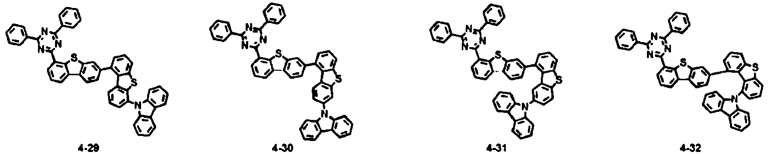

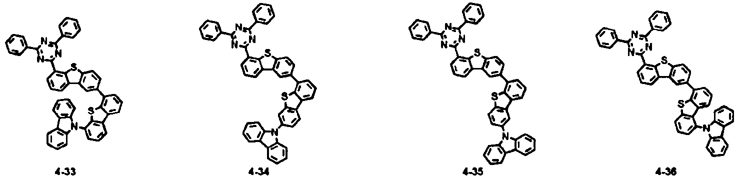

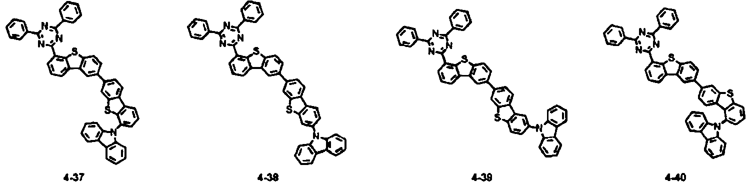

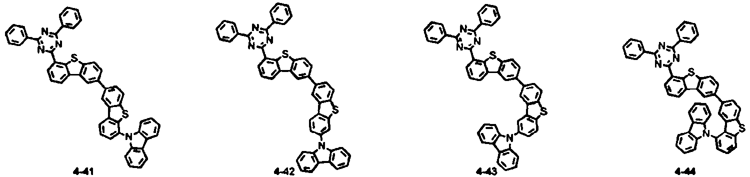

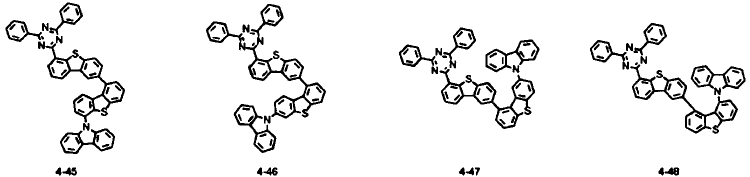

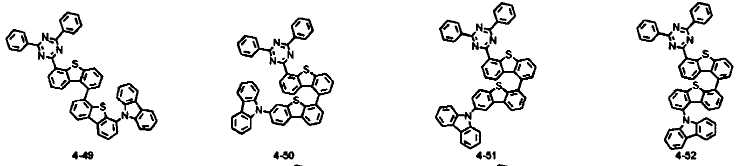

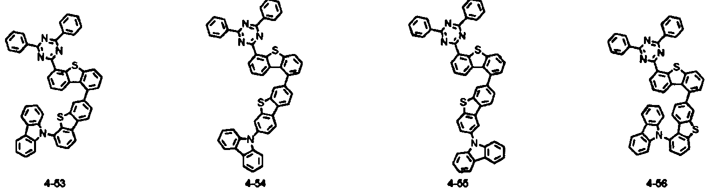

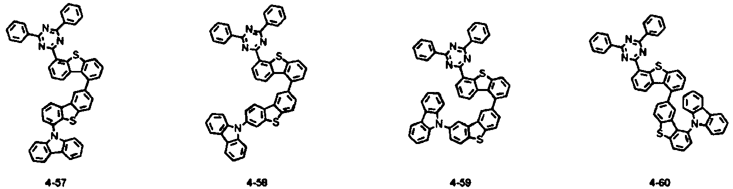

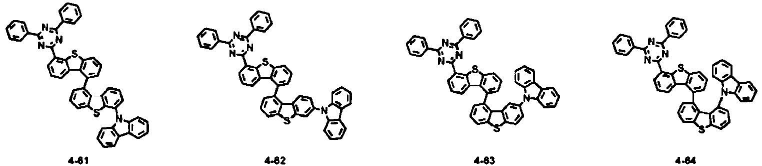

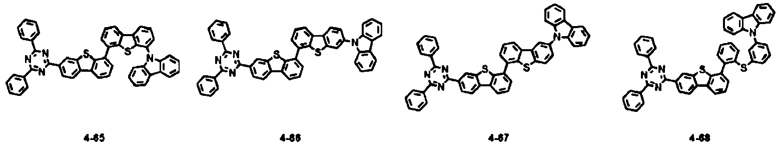

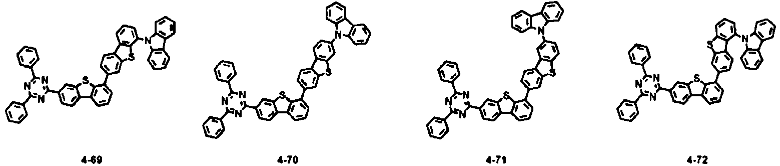

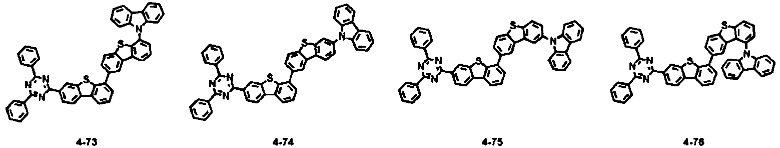

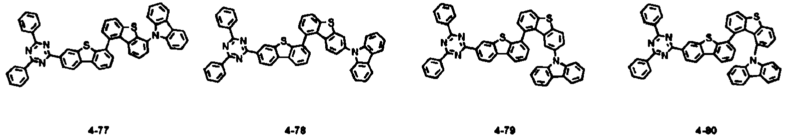

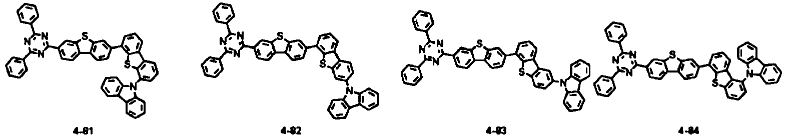

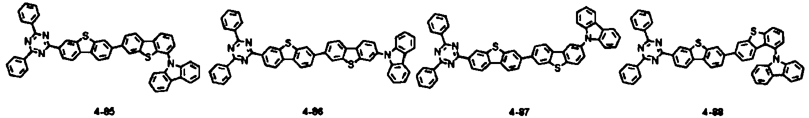

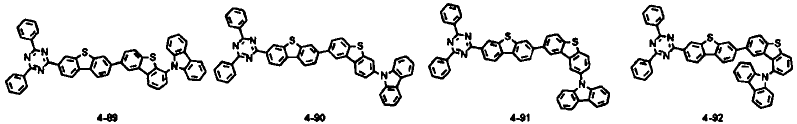

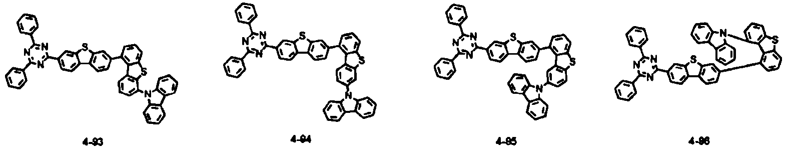

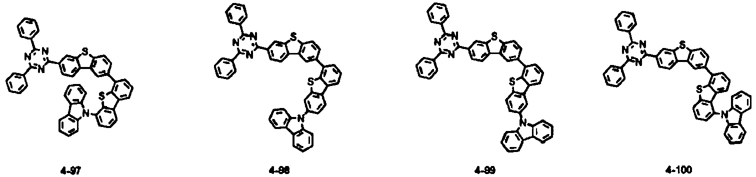

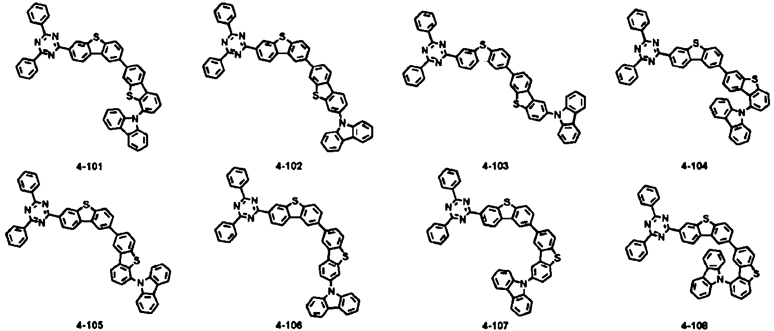

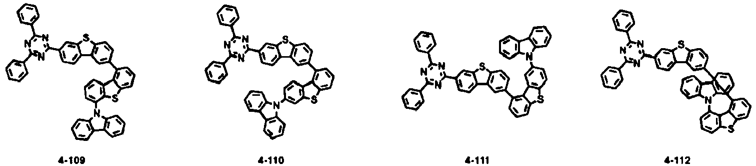

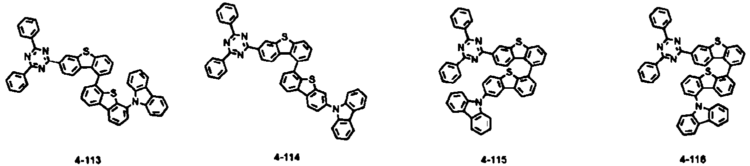

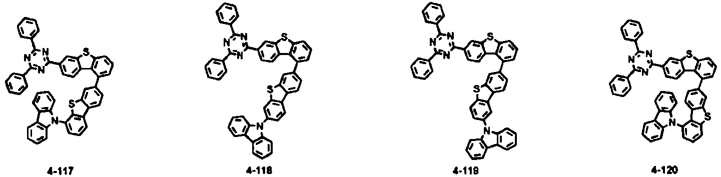

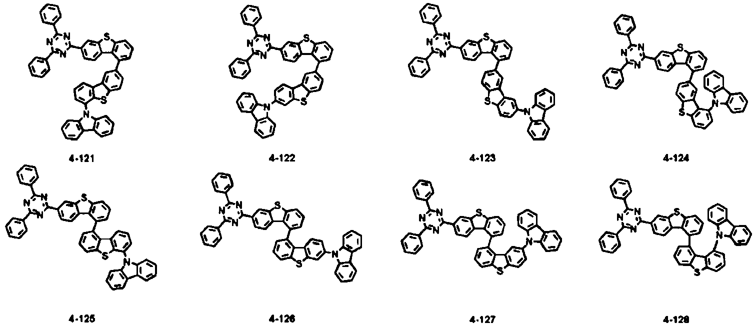

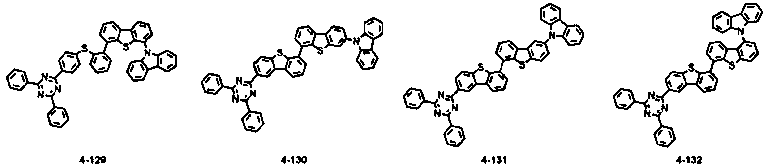

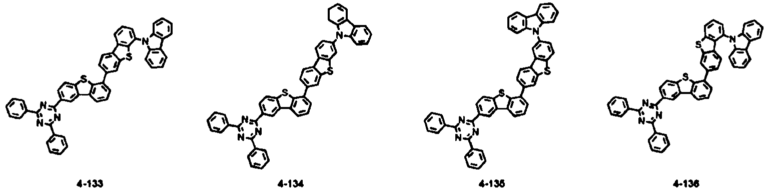

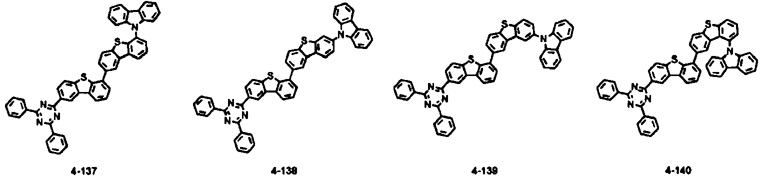

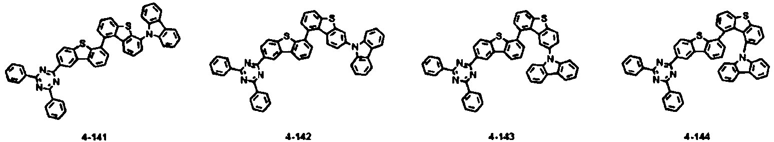

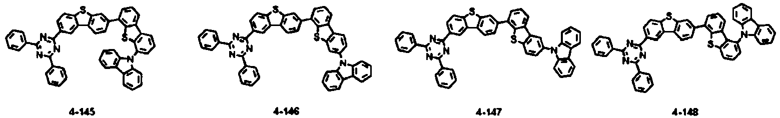

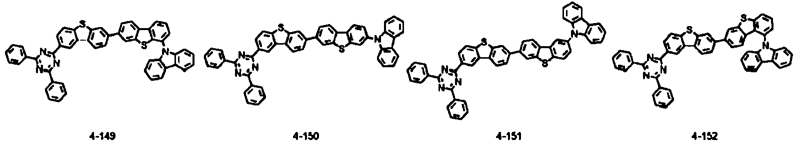

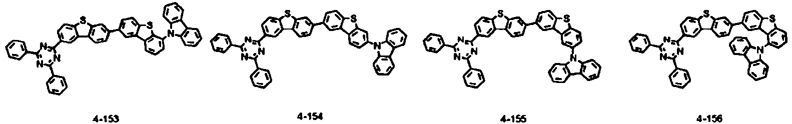

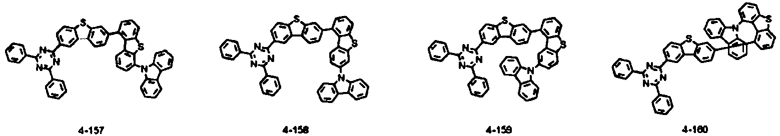

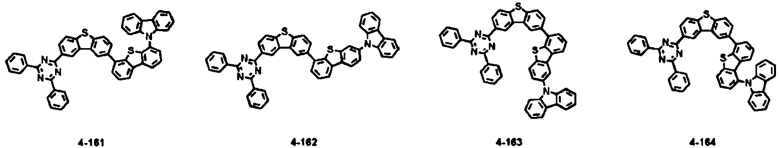

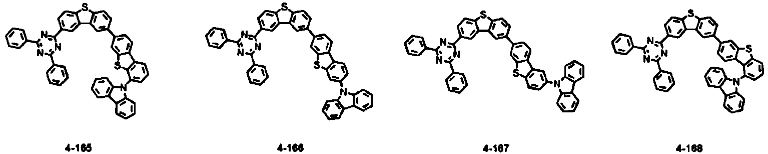

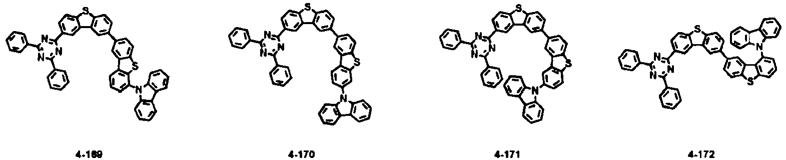

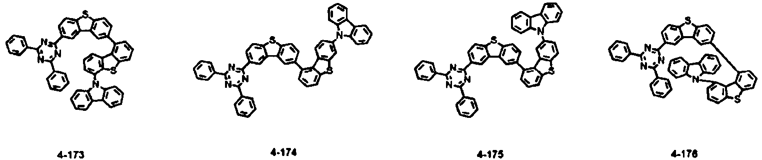

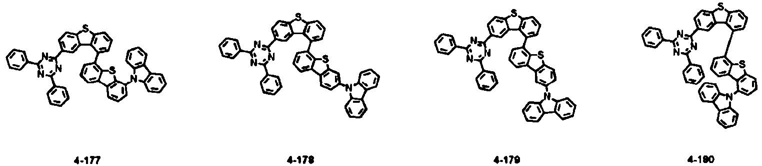

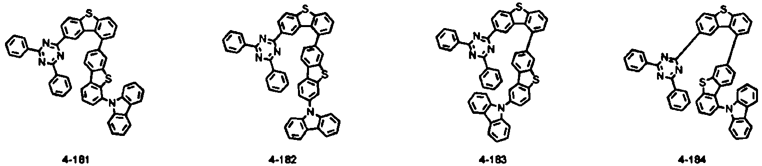

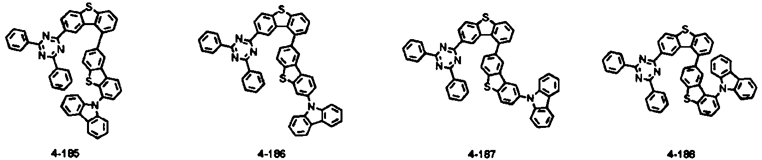

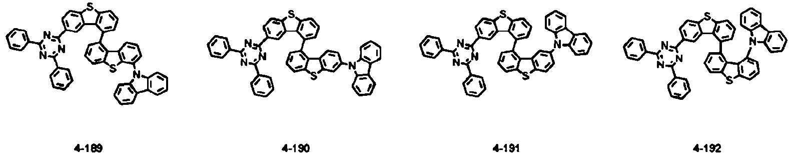

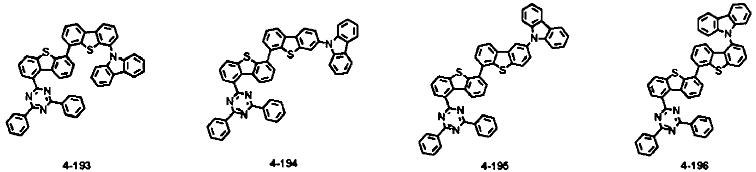

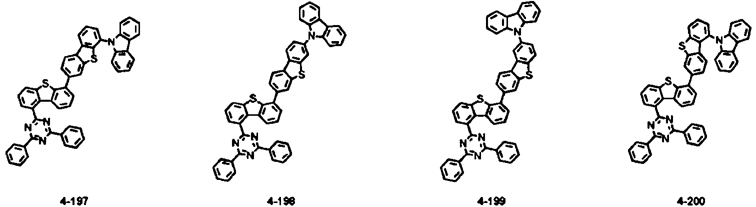

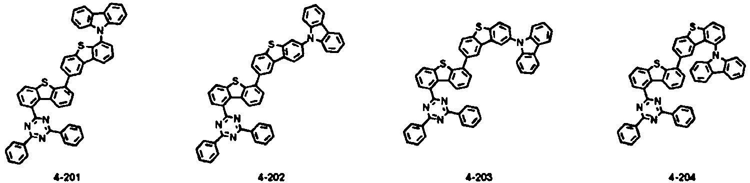

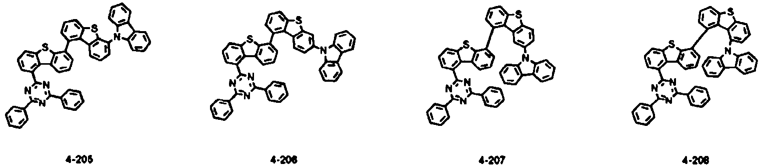

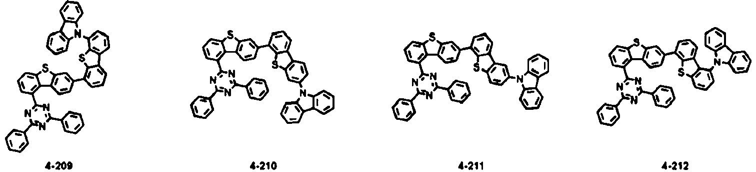

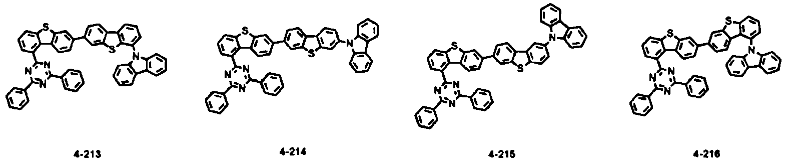

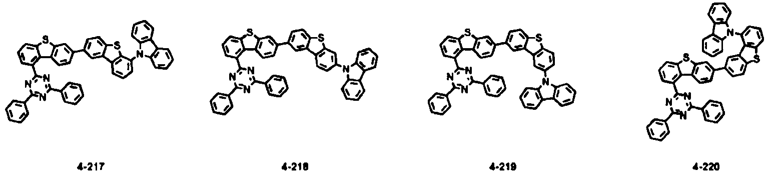

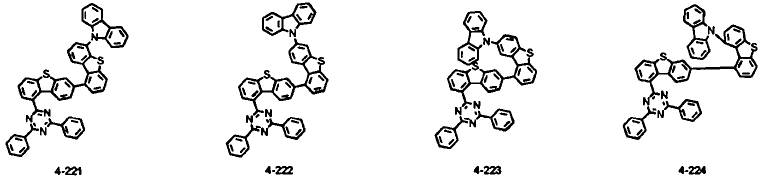

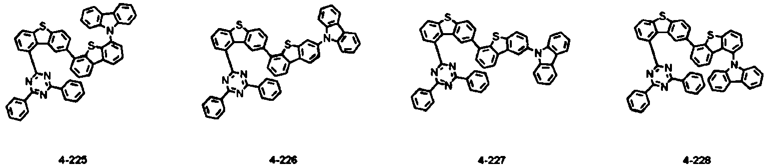

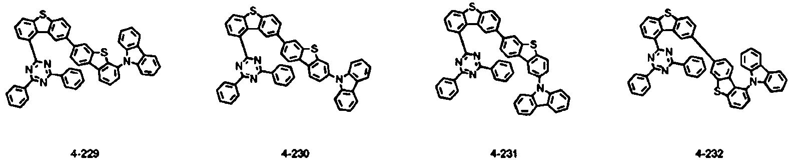

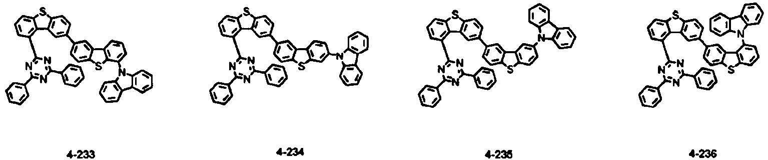

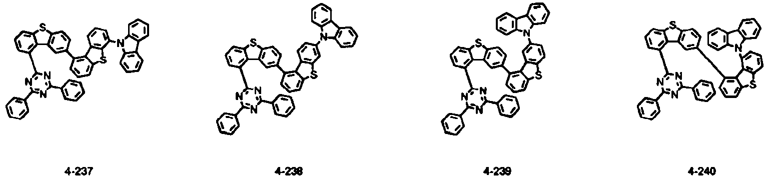

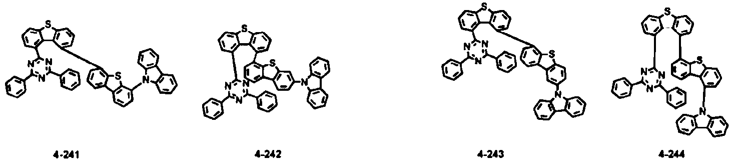

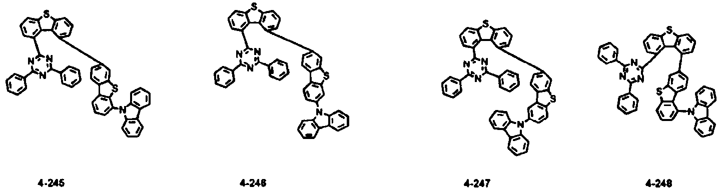

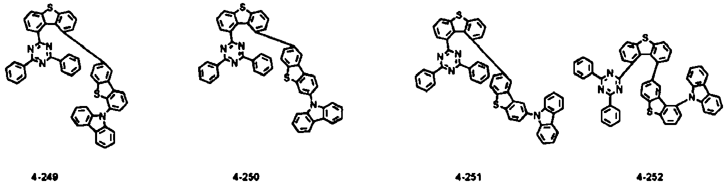

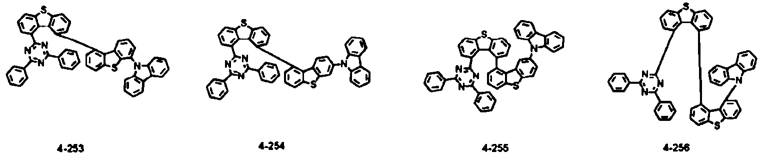

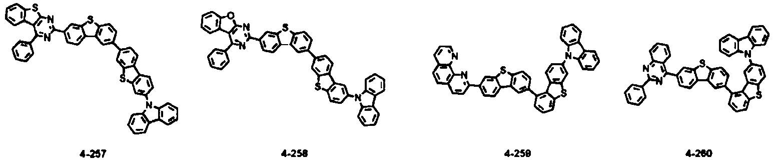

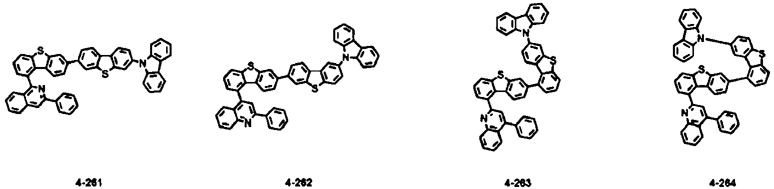

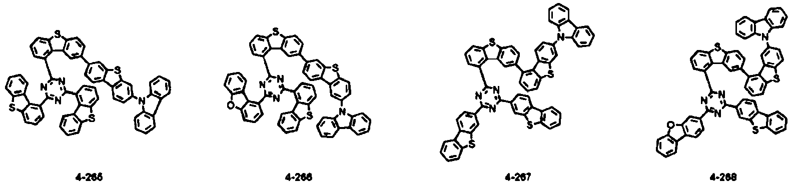

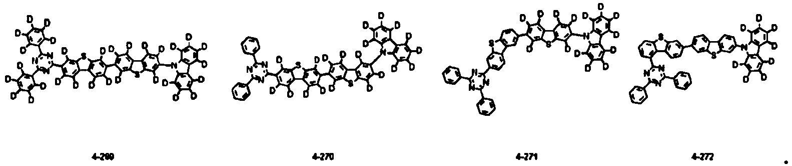

4. Dispositif émetteur de lumière organique comprenant :
une première électrode ;
une deuxième électrode ; et
une ou plusieurs couches de matière organique fournies entre la première électrode et la deuxième électrode,
dans lequel une ou plusieurs couches des couches de matière organique comprennent le composé hétérocyclique selon l'une quelconque des revendications 1 à 3.

5. Dispositif émetteur de lumière organique selon la revendication 4, dans lequel la couche de matière organique comprend une ou plusieurs couches émettrices de lumière, et la couche émettrice de lumière comprend le composé hétérocyclique.

6. Dispositif émetteur de lumière organique selon la revendication 5, dans lequel la couche électroluminescente comprend deux matériaux hôtes ou plus, et au moins l'un des matériaux hôtes comprend le composé hétérocyclique en tant que matériau hôte d'un matériau émetteur de lumière.

7. Dispositif émetteur de lumière organique selon la revendication 4, comprenant en outre une, deux ou plusieurs couches choisies dans le groupe constitué d'une couche émettrice de lumière, d'une couche d'injection de trous, d'une couche de transfert de trous, d'une couche d'injection d'électrons, d'une couche de transfert d'électrons, d'une couche auxiliaire de trous et d'une couche de blocage de trous.

8. Dispositif émetteur de lumière organique selon la revendication 4, dans lequel la couche de matière organique comprend le composé hétérocyclique selon l'une quelconque des revendications 1 à 3 en tant que premier composé, et comprend en outre un composé hétérocyclique représenté par la formule chimique suivante 13 ou 14 en tant que second composé : 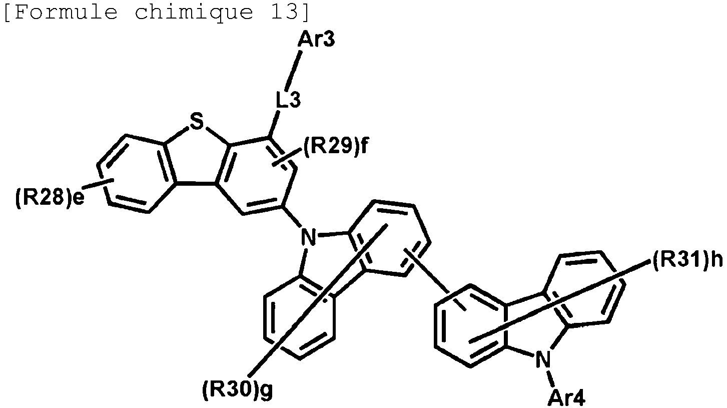 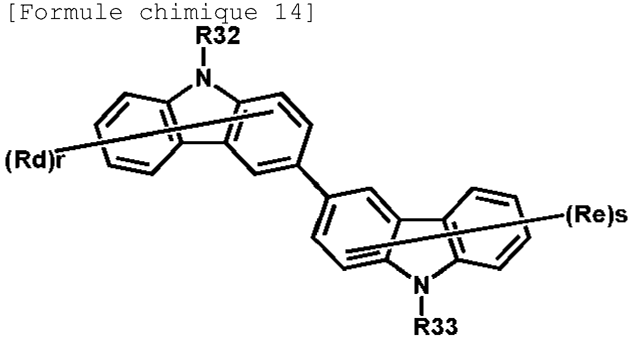 dans les formules chimiques 13 et 14,
L3 est une liaison directe ; un groupe arylène substitué ou non substitué ayant 6 à 60 atomes de carbone ; ou un groupe hétéroarylène substitué ou non substitué ayant 2 à 60 atomes de carbone ;
R28 à R31, Rd et Re sont identiques ou différents les uns des autres, et chacun choisi indépendamment dans le groupe constitué d'hydrogène ; de deutérium ; d'halogène ; d'un groupe cyano ; d'un groupe alkyle substitué ou non substitué ayant 1 à 60 atomes de carbone ; d'un groupe alcényle substitué ou non substitué ayant 2 à 60 atomes de carbone ; d'un groupe alcynyle substitué ou non substitué ayant 2 à 60 atomes de carbone ; d'un groupe alcoxy substitué ou non substitué ayant 2 à 60 atomes de carbone ; d'un groupe cycloalkyle substitué ou non substitué ayant 3 à 60 atomes de carbone ; d'un groupe hétérocycloalkyle substitué ou non substitué ayant 2 à 60 atomes de carbone ; d'un groupe aryle substitué ou non substitué ayant 6 à 60 atomes de carbone ; d'un groupe hétéroaryle substitué ou non substitué ayant 2 à 60 atomes de carbone ; d'un groupe oxyde de phosphine substitué ou non substitué ; et d'un groupe amine substitué ou non substitué, ou de deux groupes, ou plus, adjacents, liés l'un à l'autre pour former un cycle hydrocarboné aromatique substitué ou non substitué ayant 6 à 60 atomes de carbone ou un hétérocycle substitué ou non substitué ayant 2 à 60 atomes de carbone, r et s sont un entier de 0 à 7, et lorsque r est 2 ou plus, Rds sont identiques ou différents les uns des autres, lorsque s est 2 ou plus, Res sont identiques ou différents les uns des autres, e est un entier de 0 à 4, g et h sont chacun un entier de 0 à 7, f est un entier de 0 à 2, et lorsque e vaut 2 ou plus, R28 sont identiques ou différents les uns des autres, lorsque f est un entier de 2, R29 sont identiques ou différents les uns des autres, lorsque g vaut 2 ou plus, R30 sont identiques ou différents les uns des autres, et lorsque h vaut 2 ou plus, R31 sont identiques ou différents les uns des autres ;
Ar3 est un groupe aryle substitué ou non substitué ayant 6 à 60 atomes de carbone ; ou un groupe hétéroaryle substitué ou non substitué ayant 2 à 60 atomes de carbone ;
Ar4 est un groupe aryle substitué ou non substitué ayant 6 à 60 atomes de carbone ; ou un groupe hétéroaryle substitué ou non substitué ayant 2 à 60 atomes de carbone ; et
R32 et R33 sont identiques ou différents l'un de l'autre, et chacun indépendamment un groupe silyle substitué ou non substitué ; un groupe aryle substitué ou non substitué ayant 6 à 60 atomes de carbone ; ou un groupe hétéroaryle substitué ou non substitué ayant 2 à 60 atomes de carbone.

9. Dispositif émetteur de lumière organique selon la revendication 8, dans lequel le composé hétérocyclique représenté par la formule chimique 13 a une teneur en deutérium de 0 %, ou supérieure à 10 % et inférieure ou égale à 100 %.

10. Dispositif émetteur de lumière organique selon la revendication 8, dans lequel le composé hétérocyclique représenté par la formule chimique 14 a une teneur en deutérium de 0 %, ou supérieure à 10 % et inférieure ou égale à 100 %.

11. Dispositif émetteur de lumière organique selon la revendication 8, dans lequel la formule chimique 13 est représentée par l'un quelconque des composés suivants :

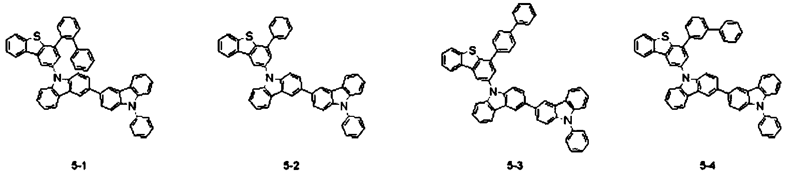

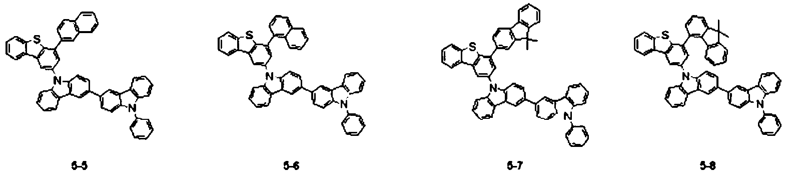

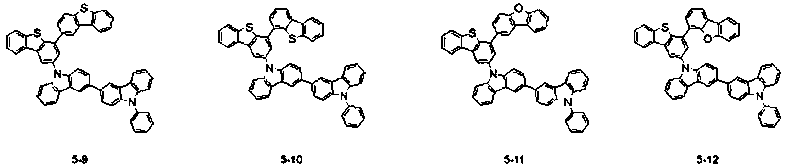

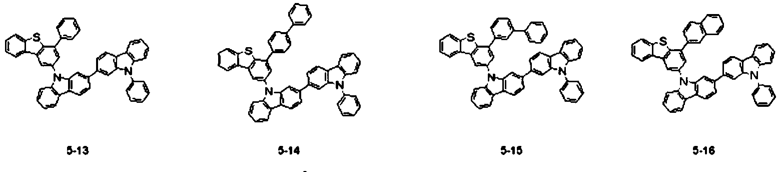

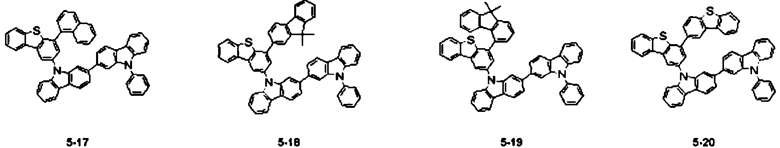

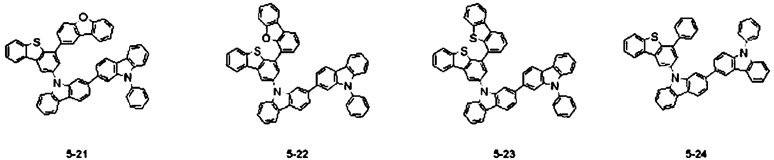

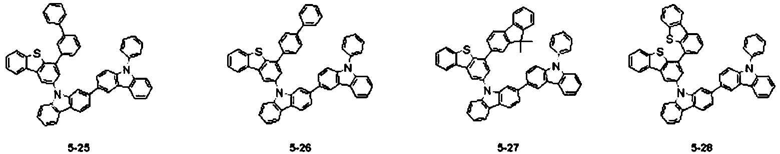

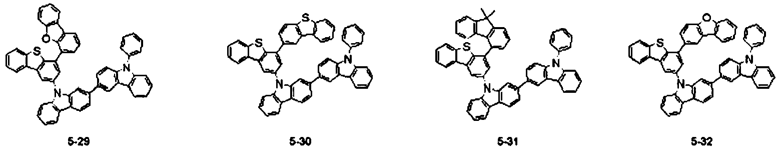

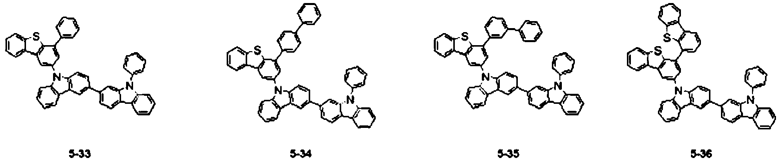

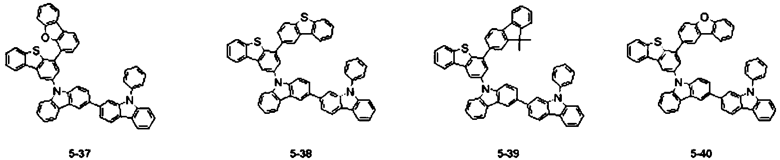

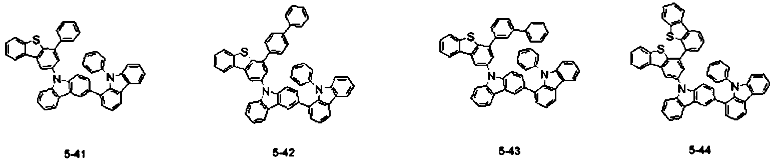

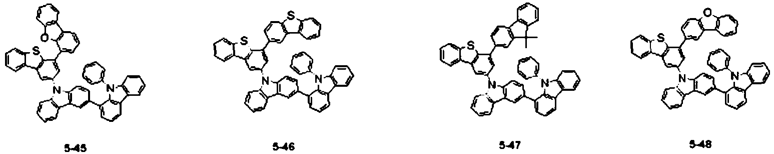

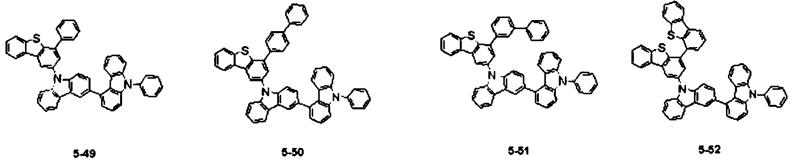

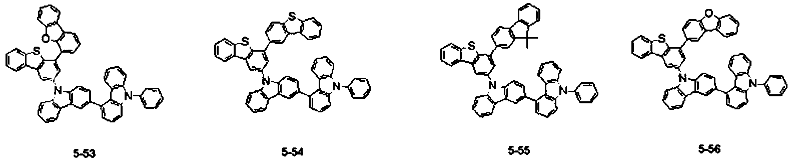

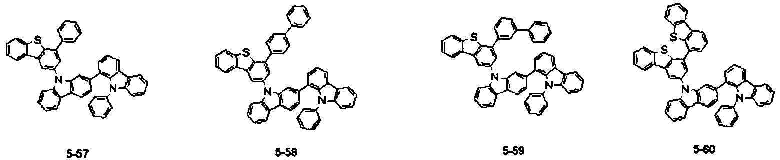

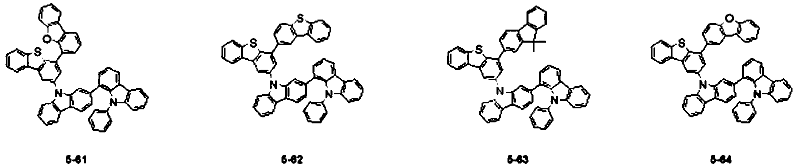

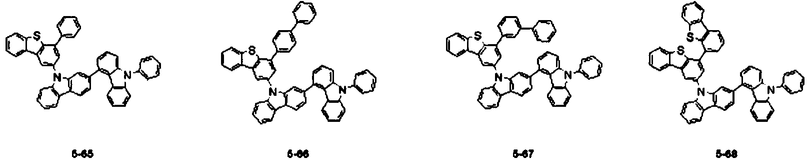

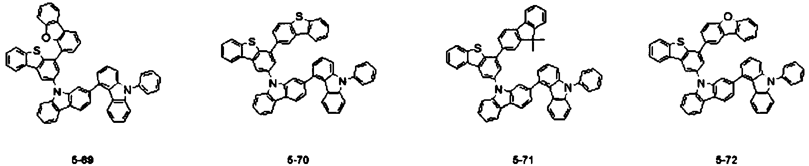

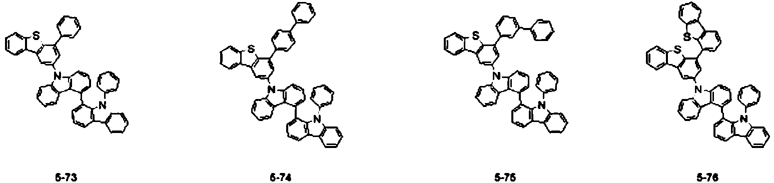

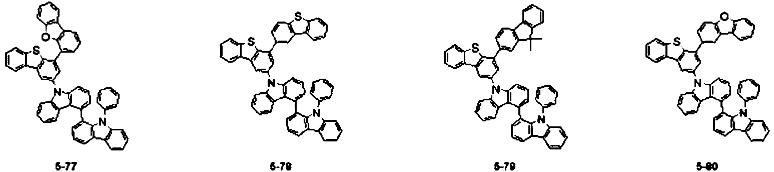

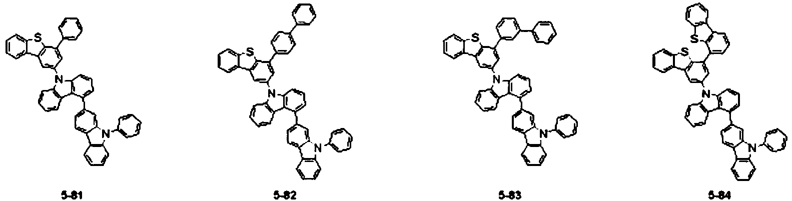

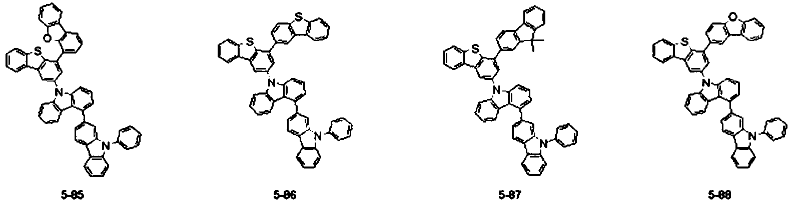

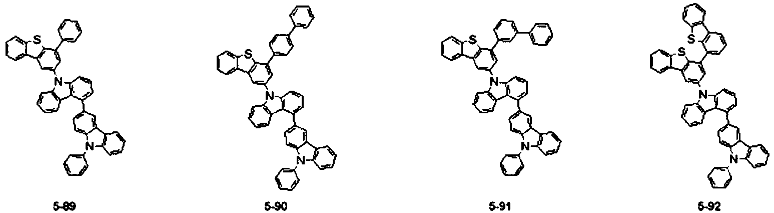

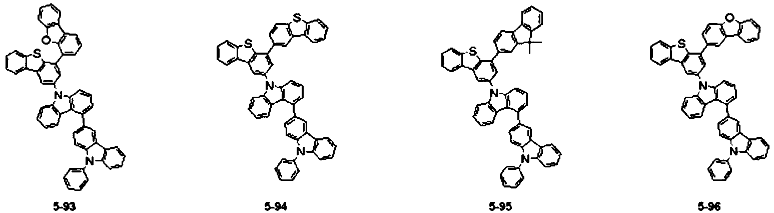

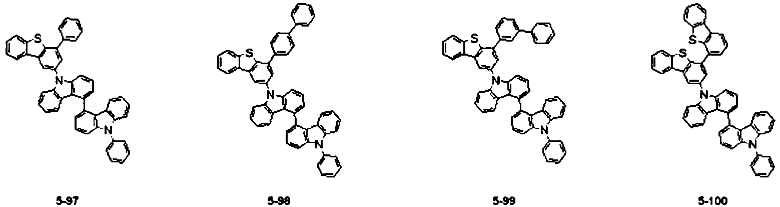

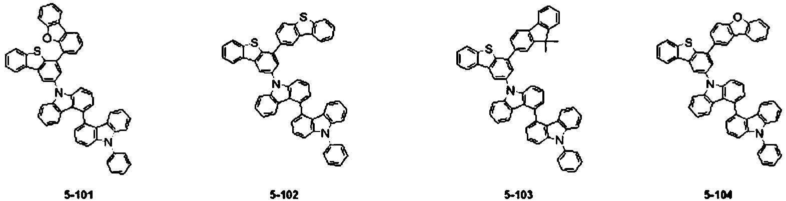

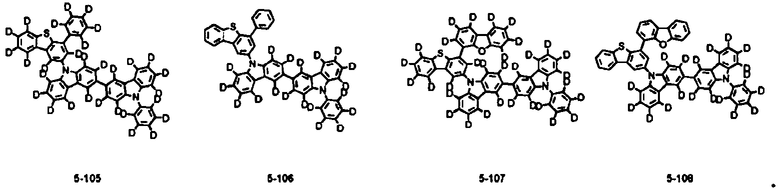

12. Dispositif émetteur de lumière organique selon la revendication 9, dans lequel la formule chimique 14 est représentée par l'un quelconque des composés suivants :

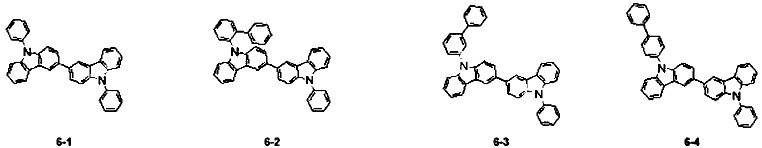

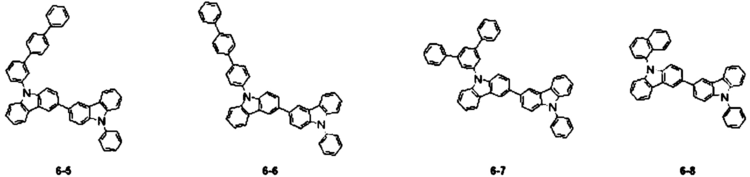

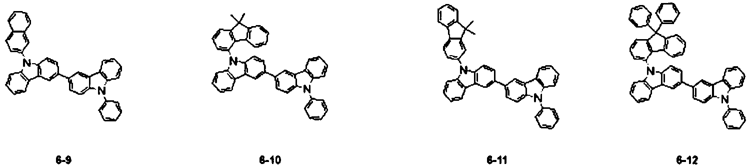

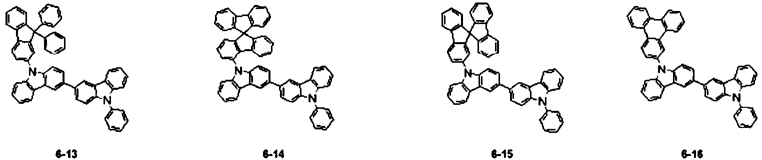

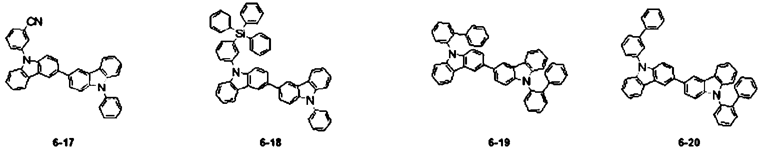

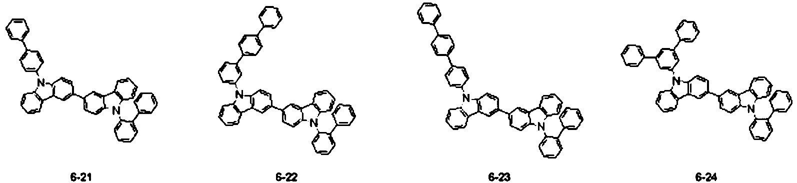

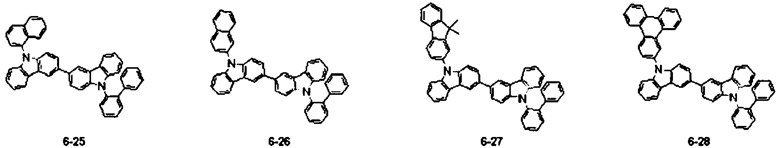

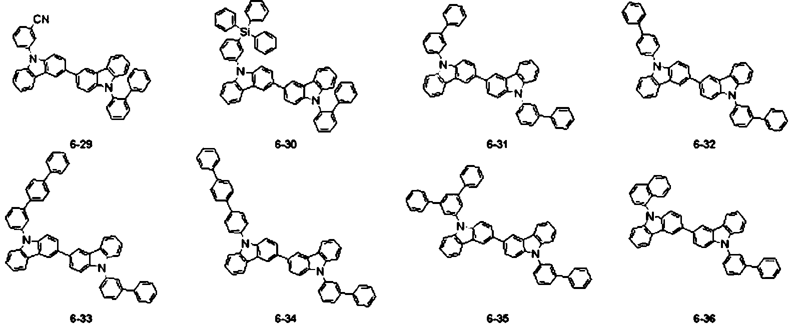

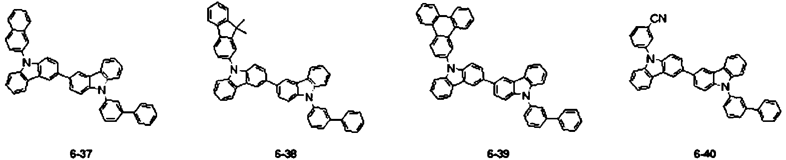

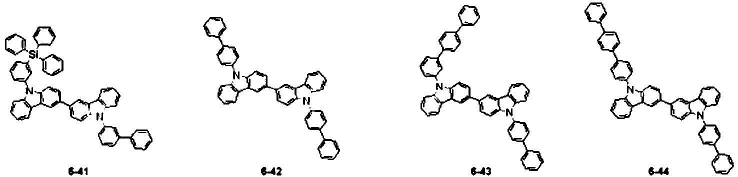

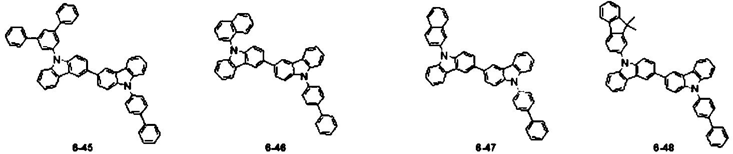

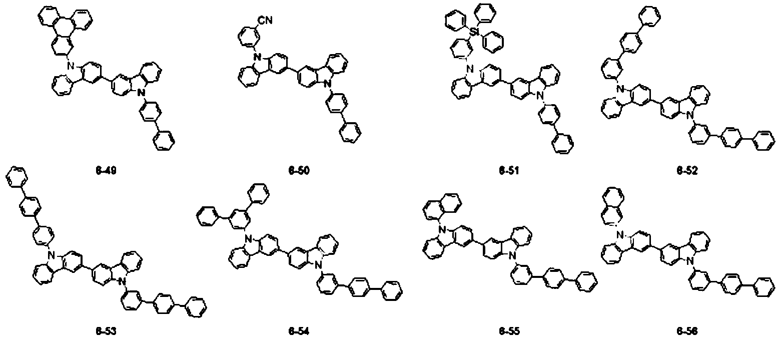

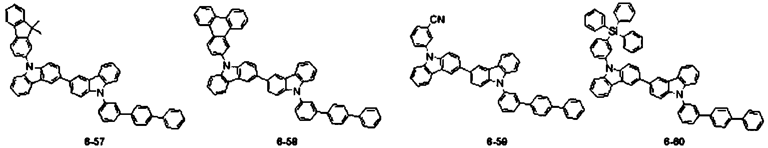

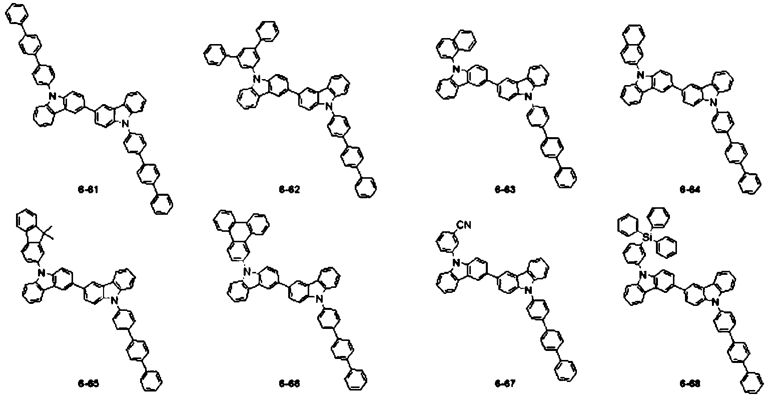

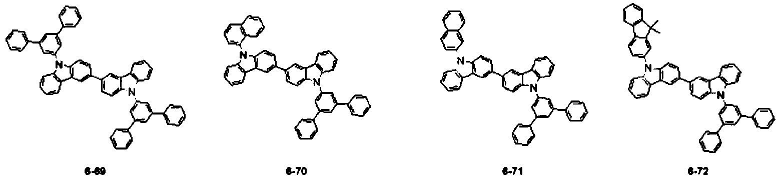

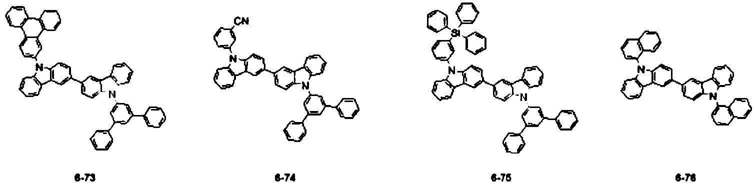

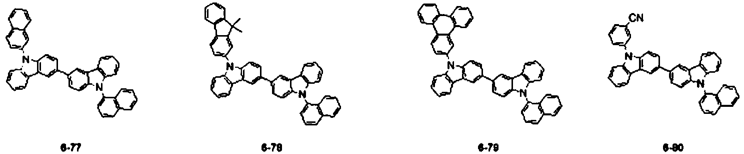

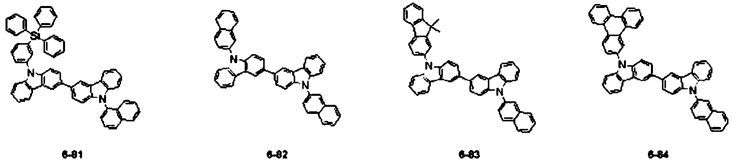

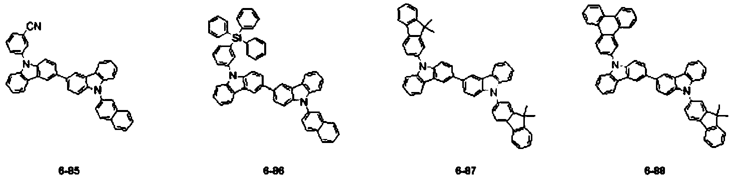

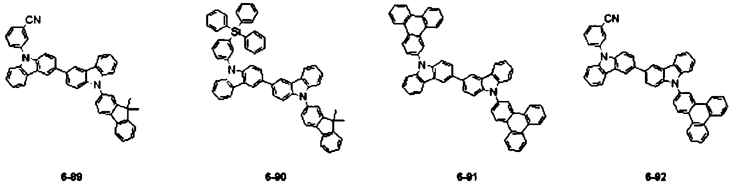

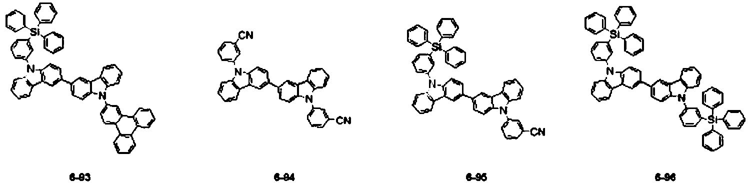

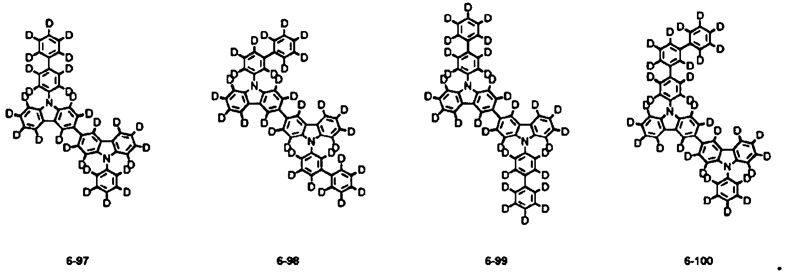

13. Composition pour une couche de matière organique d'un dispositif émetteur de lumière organique, la composition comprenant :
le composé hétérocyclique représenté par l'une quelconque des formules chimiques 1-2 à 1-4 de la revendication 1 ; et
un composé hétérocyclique représenté par la formule chimique 13 ou 14 suivante : 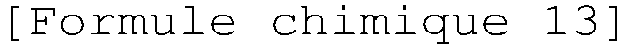 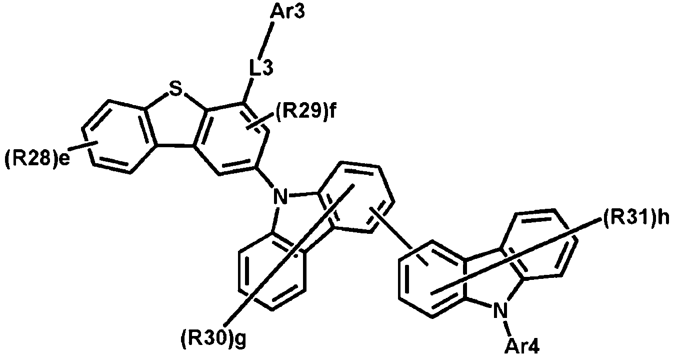 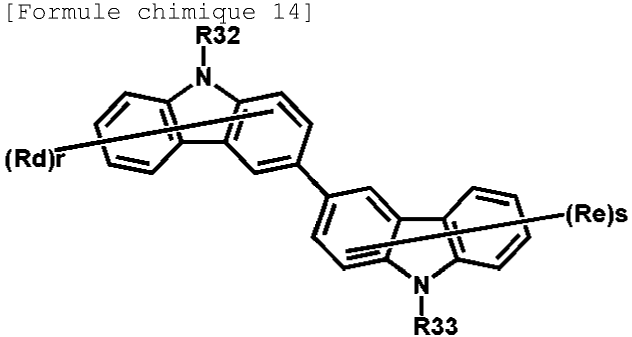 dans laquelle, dans les formules chimiques 13 et 14,
L3 est une liaison directe ; un groupe arylène substitué ou non substitué ayant 6 à 60 atomes de carbone ; ou un groupe hétéroarylène substitué ou non substitué ayant 2 à 60 atomes de carbone ;
R28 à R31, Rd et Re sont identiques ou différents les uns des autres, et chacun choisi indépendamment dans le groupe constitué d'hydrogène ; de deutérium ; d'halogène ; d'un groupe cyano ; d'un groupe alkyle substitué ou non substitué ayant 1 à 60 atomes de carbone ; d'un groupe alcényle substitué ou non substitué ayant 2 à 60 atomes de carbone ; d'un groupe alcynyle substitué ou non substitué ayant 2 à 60 atomes de carbone ; d'un groupe alcoxy substitué ou non substitué ayant 2 à 60 atomes de carbone ; d'un groupe cycloalkyle substitué ou non substitué ayant 3 à 60 atomes de carbone ; d'un groupe hétérocycloalkyle substitué ou non substitué ayant 2 à 60 atomes de carbone ; d'un groupe aryle substitué ou non substitué ayant 6 à 60 atomes de carbone ; d'un groupe hétéroaryle substitué ou non substitué ayant 2 à 60 atomes de carbone ; d'un groupe oxyde de phosphine substitué ou non substitué ; et d'un groupe amine substitué ou non substitué, ou de deux groupes, ou plus, adjacents, liés l'un à l'autre pour former un cycle hydrocarboné aromatique substitué ou non substitué ayant 6 à 60 atomes de carbone ou un hétérocycle substitué ou non substitué ayant 2 à 60 atomes de carbone, r et s sont un entier de 0 à 7, et lorsque r est 2 ou plus, Rds sont identiques ou différents les uns des autres, lorsque s est 2 ou plus, Res sont identiques ou différents les uns des autres, e est un entier de 0 à 4, g et h sont chacun un entier de 0 à 7, f est un entier de 0 à 2, et lorsque e vaut 2 ou plus, R28 sont identiques ou différents les uns des autres, lorsque f est un entier de 2, R29 sont identiques ou différents les uns des autres, lorsque g vaut 2 ou plus, R30 sont identiques ou différents les uns des autres, et lorsque h vaut 2 ou plus, R31 sont identiques ou différents les uns des autres ;
Ar3 est un groupe aryle substitué ou non substitué ayant 6 à 60 atomes de carbone ; ou un groupe hétéroaryle substitué ou non substitué ayant 2 à 60 atomes de carbone ;
Ar4 est un groupe aryle substitué ou non substitué ayant 6 à 60 atomes de carbone ; ou un groupe hétéroaryle substitué ou non substitué ayant 2 à 60 atomes de carbone ; et
R32 et R33 sont identiques ou différents l'un de l'autre, et chacun indépendamment un groupe silyle substitué ou non substitué ; un groupe aryle substitué ou non substitué ayant 6 à 60 atomes de carbone ; ou un groupe hétéroaryle substitué ou non substitué ayant 2 à 60 atomes de carbone.
